# EUROPEAN PATENT SPECIFICATION

(11) **EP 1 319 069 B1**
(45) Date of publication and mention of the grant of the patent: **21.05.2008**
(21) Application number: 01952390.1
(22) Date of filing: 28.06.2001
(51) Int. Cl.: C12N 15/12, C07K 14/47, C07K 16/30, G01N 33/574, C12Q 1/68, C12N 5/08, A61K 38/17, A61K 31/711, A61K 39/395, A61K 35/14

(54) **COMPOSITIONS AND METHODS FOR THE THERAPY AND DIAGNOSIS OF LUNG CANCER**
ZUSAMMENSETZUNGEN UND VERFAHREN FÜR THERAPIE UND DIAGNOSE VON LUNGENKREBS
COMPOSITIONS ET METHODES POUR LE TRAITEMENT ET LE DIAGNOSTIC DU CANCER DU POUMON

(30) Priority: 28.06.2000 US 606421; 02.08.2000 US 630940; 21.08.2000 US 643597; 15.09.2000 US 662786; 09.10.2000 US 685696; 12.12.2000 US 735705; 07.05.2001 US 850716
(43) Date of publication of application: 18.06.2003
(73) Proprietor: CORIXA CORPORATION, Seattle, WA 98104 (US)
(72) Inventor: WANG, Tongtong, Medina, WA 98039 (US); WANG, Aijun, Issaquah, WA 98029 (US); SKEIKY, Yasir, A., W., Bellevue, WA 98006 (US); LI, Samuel, X., Redmond, WA 98052 (US); KALOS, Michael, D., Seattle, WA 98103 (US); HENDERSON, Robert, A., Edmonds, WA 98026 (US); MCNEILL, Patricia, D., Federal Way, WA 98003 (US); FANGER, Neil, Seattle, WA 98103 (US); RETTER, Marc, W., Carnation, WA 98014 (US); DURHAM, Margarita, Seattle, WA 98199 (US); FANGER, Gary, Richard, Mill Creek, WA 98012 (US); VEDVICK, Thomas, S., Federal Way, WA 98003 (US); CARTER, Darrick, Seattle, WA 98102 (US); WATANABE, Yoshihiro, Mercer Island, WA 98040 (US); PECKHAM, David, W., Seattle, WA 98104 (US)
(74) Representative: Gowshall, Jonathan Vallance
(86) International application number: PCT/US2001/021065
(87) International publication number: WO 2002/000174

(56) References cited:
- US-A- 5 589 579
- SKEIKY Y A W ET AL: "Cloning, expression and immunological evaluation of two putative secreted serine protease antigens of Mycobacterium tuberculosis" INFECTION AND IMMUNITY, AMERICAN SOCIETY OF MICROBIOLOGY, WASHINGTON, DC, US, vol. 67, no. 8, August 1999 (1999-08), pages 3998-4007, XP002143392 ISSN: 0019-9567 cited in the application
- GÜRE A O ET AL: "Human lung cancer antigens recognized by autologous antibodies: definition of a novel cDNA derived from the tumor suppressor gene locus on chromosome 3p21.3" CANCER RESEARCH, AMERICAN ASSOCIATION FOR CANCER RESEARCH, BALTIMORE, MD, US, vol. 58, 1 March 1998 (1998-03-01), pages 1034-1041, XP002103188 ISSN: 0008-5472
- CHEN S-L ET AL: "Isolation and characterizaton of a novel gene expressed in multiple cancers" ONCOGENE, BASINGSTOKE, HANTS, GB, vol. 12, no. 4, 15 February 1996 (1996-02-15), pages 741-751, XP002106655 ISSN: 0950-9232

## Description

### TECHNICAL FIELD OF THE INVENTION

The present invention relates generally to therapy and diagnosis of cancer, such as lung cancer. The invention is more specifically related to polypeptides, comprising at least a portion of a lung tumor protein, and to polynucleotides encoding such polypeptides. Such polypeptides and polynucleotides are useful in pharmaceutical compositions, e.g., vaccines, and other compositions for the diagnosis and treatment of lung cancer.

### BACKGROUND OF THE INVENTION

Cancer is a significant health problem throughout the world. Although advances have been made in detection and therapy of cancer, no vaccine or other universally successful method for prevention and/or treatment is currently available. Current therapies, which are generally based on a combination of chemotherapy or surgery and radiation, continue to prove inadequate in many patients.

Lung cancer is the primary cause of cancer death among both men and women in the U.S., with an estimated 172,000 new cases being reported in 1994. The five-year survival rate among all lung cancer patients, regardless of the stage of disease at diagnosis, is only 13%. This contrasts with a five-year survival rate of 46% among cases detected while the disease is still localized. However, only 16% of lung cancers are discovered before the disease has spread.

In spite of considerable research into therapies for these and other cancers, lung cancer remains difficult to diagnose and treat effectively. Accordingly, there is a need in the art for improved methods for detecting and treating such cancers. The present invention fulfills these needs and further provides other related advantages.

The present invention provides an isolated antibody, or antigen-binding fragment thereof, that specifically binds to a polypeptide set forth in SEQ ID NO. 380. There is also provided the use of the antibody of the present invention in therapy and diagnosis, in particular, for use in the treatment of lung cancer in a patient and for the use in the diagnosis of lung cancer in a patient.

The present disclosure provides polynucleotide compositions comprising a sequence selected from the group consisting of:
(a) sequences provided in SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182,184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467;
(b) complements of the sequences provided in SEQ ID NOs:1-3, 6-8, 10-13, 15-27, 29, 30, 32, 34-49, 51; 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158; 160; 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467;
(c) sequences consisting of at least 10, 15, 20, 25, 30, 35, 40, 45, 50, 75 and 100 contiguous residues of a sequence provided in SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467;
(d) sequences that hybridize to a sequence provided in SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467, under moderate or highly stringent conditions;
(e) sequences having at least 75%, 80%, 85%, 90%, 95%, 96%, 97%, 98% or 99% identity to a sequence of SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467; and
(f) degenerate variants of a sequence provided in SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467.

In one preferred embodiment, the polynucleotide compositions of the disclosure are expressed in at least about 20%, more preferably in at least about 30%, and most preferably in at least about 50% of lung tumors samples tested, at a level that is at least about 2-fold, preferably at least about 5-fold, and most preferably at least about 10-fold higher than that for normal tissues.

The present disclosure in another aspect, provides polypeptide compositions comprising an amino acid sequence that is encoded by a polynucleotide sequence described above.

The present disclosure further provides polypeptide compositions comprising an amino acid sequence selected from the group consisting of sequences recited in SEQ ID NOs:152, 155, 156, 165, 166, 169, 170, 172, 174, 176, 226-252, 338-344, 346, 350, 357, 361, 363, 365, 367, 369, 376-382, 387-419, 423, 427, 430, 433, 441, 443, 446, 449 and 451-466,

In certain preferred embodiments, the polypeptides and/or polynucleotides of the present disclosure are immunogenic, *i*.*e*., they are capable of eliciting an immune response, particularly a humoral and/or cellular immune response, as further described herein.

The present disclosure further provides fragments, variants and/or derivatives of the disclosed polypeptide and/or polynucleotide sequences, wherein the fragments, variants and/or derivatives preferably have a level of immunogenic activity of at least about 50%, preferably at least about 70% and more preferably at least about 90% of the level of immunogenic activity of a polypeptide sequence set forth in SEQ ID NOs:152, 155, 156, 165, 166, 169, 170, 172, 174, 176, 226-252, 338-344, 346, 350, 357, 361, 363, 365, 367, 369, 376-382, 387-419, 423, 427, 430, 433, 441, 443, 446, 449 and 451-466, or a polypeptide sequence encoded by a polynucleotide sequence set forth in SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151,153, 154, 157, 158,160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467.

The present disclosure further provides polynucleotides that encode a polypeptide described above, expression vectors comprising such polynucleotides and host cells transformed or transfected with such expression vectors.

Within other aspects, the present disclosure provides pharmaceutical compositions comprising a polypeptide or polynucleotide as described above and a physiologically acceptable carrier.

Within a related aspect of the present disclosure, the pharmaceutical compositions, e.g., vaccine compositions, are provided for prophylactic or therapeutic applications. Such compositions generally comprise an immunogenic polypeptide or polynucleotide of the invention and an immunostimulant, such as an adjuvant.

The present invention further provides pharmaceutical compositions that comprise: (a) an antibody or antigen-binding fragment thereof that specifically binds to a polypeptide of the present invention set forth is SEQ ID NO 380, or a fragment thereof; and (b) a physiologically acceptable carrier.

Within further aspects, the present disclosure provides pharmaceutical compositions comprising: (a) an antigen presenting cell that expresses a polypeptide as described above and (b) a pharmaceutically acceptable carrier or excipient. Illustrative antigen presenting cells include dendritic cells, macrophages, monocytes, fibroblasts and B cells.

Within related aspects, pharmaceutical compositions are disclosures that comprise: (a) an antigen presenting cell that expresses a polypeptide as described above and (b) an immunostimulant.

The present disclosure further provides, in other aspects, fusion proteins that comprise at least one polypeptide as described above, as well as polynucleotides encoding such fusion proteins, typically in the form of pharmaceutical compositions, e.g., vaccine compositions, comprising a physiologically acceptable carrier and/or an immunostimulant. The fusions proteins may comprise multiple immunogenic polypeptides or portions/variants thereof, as described herein, and may further comprise one or more polypeptide segments for facilitating the expression, purification and/or immunogenicity of the polypeptide(s).

Within further aspects, the present disclosure provides methods for stimulating an immune response in a patient, preferably a T cell response in a human patient, comprising administering a pharmaceutical composition described herein. The patient may be afflicted with lung cancer, in which case the methods provide treatment for the disease, or patient considered at risk for such a disease may be treated prophylactically.

Within further aspects, the present invention provides methods for inhibiting the development of a cancer in a patient, comprising administering to a patient a pharmaceutical composition as recited above. The patient may be afflicted with lung cancer, in which case the methods provide treatment for the disease, or patient considered at risk for such a disease may be treated prophylactically.

The present disclosure further provides, within other aspects, methods for removing tumor cells from a biological sample, comprising contacting a biological sample with T cells that specifically react with a polypeptide of the present invention, wherein the step of contacting is performed under conditions and for a time sufficient to permit the removal of cells expressing the protein from the sample.

Within related aspects, methods are provided for inhibiting the development of a cancer in a patient, comprising administering to a patient a biological sample treated as described above.

Methods are further provided, within other aspects, for stimulating and/or expanding T cells specific for a polypeptide of the present invention, comprising contacting T cells with one or more of: (i) a polypeptide as described above; (ii) a polynucleotide encoding such a polypeptide; and/or (iii) an antigen presenting cell that expresses such a polypeptide; under conditions and for a time sufficient to permit the stimulation and/or expansion of T cells. Isolated T cell populations comprising T cells prepared as described above are also provided.

Within further aspects, the present disclosure provides methods for inhibiting the development of a cancer in a patient, comprising administering to a patient an effective amount of a T cell population as described above.

The present disclosure further provides methods for inhibiting the development of a cancer in a patient, comprising the steps of: (a) incubating CD4⁺ and/or CD8⁺ T cells isolated from a patient with one or more of: (i) a polypeptide comprising at least an immunogenic portion of polypeptide disclosed herein; (ii) a polynucleotide encoding such a polypeptide; and (iii) an antigen-presenting cell that expressed such a polypeptide; and (b) administering to the patient an effective amount of the proliferated T cells, and thereby inhibiting the development of a cancer in the patient. Proliferated cells may, but need not, be cloned prior to administration to the patient.

Within further aspects, the present invention provides methods for determining the presence or absence of a cancer, preferably a lung cancer, in a patient comprising: (a) contacting a biological sample obtained from a patient with the antibody of the present invention; (b) detecting in the sample an amount of polypeptide that binds to the binding agent; and (c) comparing the amount of polypeptide with a predetermined cut-off value, and therefrom determining the presence or absence of a cancer in the patient. Within preferred embodiments, the binding agent is an antibody, more preferably a monoclonal antibody.

The present invention also provides, within other aspects, methods for monitoring the progression of a cancer in a patient. Such methods comprise the steps of: (a) contacting a biological sample obtained from a patient at a first point in time with a binding agent that binds to a polypeptide as recited above; (b) detecting in the sample an amount of polypeptide that binds to the binding agent; (c) repeating steps (a) and (b) using a biological sample obtained from the patient at a subsequent point in time; and (d) comparing the amount of polypeptide detected in step (c) with the amount detected in step (b) and therefrom monitoring the progression of the cancer in the patient.

The present disclosure further provides, within other aspects, methods for determining the presence or absence of a cancer in a patient, comprising the steps of: (a) contacting a biological sample obtained from a patient with an oligonucleotide that hybridizes to a polynucleotide that encodes a polypeptide of the present invention; (b) detecting in the sample a level of a polynucleotide, preferably mRNA, that hybridizes to the oligonucleotide; and (c) comparing the level of polynucleotide that hybridizes to the oligonucleotide with a predetermined cut-off value, and therefrom determining the presence or absence of a cancer in the patient. Within certain embodiments, the amount of mRNA is detected via polymerase chain reaction using, for example, at least one oligonucleotide primer that hybridizes to a polynucleotide encoding a polypeptide as recited above, or a complement of such a polynucleotide. Within other embodiments, the amount of mRNA is detected using a hybridization technique, employing an oligonucleotide probe that hybridizes to a polynucleotide that encodes a polypeptide as recited above, or a complement of such a polynucleotide.

In related aspects, methods are provided for monitoring the progression of a cancer in a patient, comprising the steps of: (a) contacting a biological sample obtained from a patient with an oligonucleotide that hybridizes to a polynucleotide that encodes a polypeptide of the present invention; (b) detecting in the sample an amount of a polynucleotide that hybridizes to the oligonucleotide; (c) repeating steps (a) and (b) using a biological sample obtained from the patient at a subsequent point in time; and (d) comparing the amount of polynucleotide detected in step (c) with the amount detected in step (b) and therefrom monitoring the progression of the cancer in the patient.

Within further aspects, the present invention provides antibodies, such as monoclonal antibodies, that bind to a polypeptide as described above, as well as diagnostic kits comprising such antibodies. Diagnostic kits comprising one or more oligonucleotide probes or primers as described above are also disclosures.

These and other aspects of the present invention will become apparent upon reference to the following detailed description and attached drawings.

### SEQUENCE IDENTIFIERS

SEQ ID NO:1 is the determined cDNA sequence for LST-S1-2
SEQ ID NO:2 is the determined cDNA sequence for LST-S1-28
SEQ ID NO:3 is the determined cDNA sequence for LST-S1-90
SEQ ID NO:4 is the determined cDNA sequence for LST-S1-144
SEQ ID NO:5 is the determined cDNA sequence for LST-S1-133
SEQ ID NO:6 is the determined cDNA sequence for LST-S1-169
SEQ ID NO:7 is the determined cDNA sequence for LST-S2-6
SEQ ID NO:8 is the determined cDNA sequence for LST-S2-11
SEQ ID NO:9 is the determined cDNA sequence for LST-S2-17
SEQ ID NO:10 is the determined cDNA sequence for LST-S2-25
SEQ ID NO:11 is the determined cDNA sequence for LST-S2-39
SEQ ID NO:12 is a first determined cDNA sequence for LST-S2-43
SEQ ID NO:13 is a second determined cDNA sequence for LST-S2-43
SEQ ID NO:14 is the determined cDNA sequence for LST-S2-65
SEQ ID NO:15 is the determined cDNA sequence for LST-S2-68
SEQ ID NO:16 is the determined cDNA sequence for LST-S2-72
SEQ ID NO:17 is the determined cDNA sequence for LST-S2-74
SEQ ID NO:18 is the determined cDNA sequence for LST-S2-103
SEQ ID NO:19 is the determined cDNA sequence for LST-S2-N1-1F
SEQ ID NO:20 is the determined cDNA sequence for LST-S2-N1-2A
SEQ ID NO:21 is the determined cDNA sequence for LST-S2-N1-4H
SEQ ID NO:22 is the determined cDNA sequence for LST-S2-N1-5A
SEQ ID NO:23 is the determined cDNA sequence for LST-S2-N1-6B
SEQ ID NO:24 is the determined cDNA sequence for LST-S2-N1-7B
SEQ ID NO:25 is the determined cDNA sequence for LST-S2-N1-7H
SEQ ID NO:26 is the determined cDNA sequence for LST-S2-N1-8A
SEQ ID NO:27 is the determined cDNA sequence for LST-S2-N1-8D
SEQ ID NO:28 is the determined cDNA sequence for LST-S2-N1-9A
SEQ ID NO:29 is the determined cDNA sequence for LST-S2-N1-9E
SEQ ID NO:30 is the determined cDNA sequence for LST-S2-N1-10A
SEQ ID NO:31 is the determined cDNA sequence for LST-S2-N1-10G
SEQ ID NO:32 is the determined cDNA sequence for LST-S2-N1-11A
SEQ ID NO:33 is the determined cDNA sequence for LST-S2-N1-12C
SEQ ID NO:34 is the determined cDNA sequence for LST-S2-N1-12E
SEQ ID NO:35 is the determined cDNA sequence for LST-S2-B1-3D
SEQ ID NO:36 is the determined cDNA sequence for LST-S2-B1-6C
SEQ ID NO:37 is the determined cDNA sequence for LST-S2-B1-5D
SEQ ID NO:38 is the determined cDNA sequence for LST-S2-B1-5F
SEQ ID NO:39 is the determined cDNA sequence for LST-S2-B1-6G
SEQ ID NO:40 is the determined cDNA sequence for LST-S2-B1-8A
SEQ ID NO:41 is the determined cDNA sequence for LST-S2-B1-8D
SEQ ID NO:42 is the determined cDNA sequence for LST-S2-B1-10A
SEQ ID NO:43 is the determined cDNA sequence for LST-S2-B1-9B
SEQ ID NO:44 is the determined cDNA sequence for LST-S2-B1-9F
SEQ ID NO:45 is the determined cDNA sequence for LST-S2-B1-12D
SEQ ID NO:46 is the determined cDNA sequence for LST-S2-I2-2B
SEQ ID NO:47 is the determined cDNA sequence for LST-S2-I2-5F
SEQ ID NO:48 is the determined cDNA sequence for LST-S2-I2-6B
SEQ ID NO:49 is the determined cDNA sequence for LST-S2-I2-7F
SEQ ID NO:50 is the determined cDNA sequence for LST-S2-I2-8G
SEQ ID NO:51 is the determined cDNA sequence for LST-S2-I2-9E
SEQ ID NO:52 is the determined cDNA sequence for LST-S2-I2-12B
SEQ ID NO:53 is the determined cDNA sequence for LST-S2-H2-2C
SEQ ID NO:54 is the determined cDNA sequence for LST-S2-H2-1 G
SEQ ID NO:55 is the determined cDNA sequence for LST-S2-H2-4G
SEQ ID NO:56 is the determined cDNA sequence for LST-S2-H2-3H
SEQ ID NO:57 is the determined cDNA sequence for LST-S2-H2-5G
SEQ ID NO:58 is the determined cDNA sequence for LST-S2-H2-9B
SEQ ID NO:59 is the determined cDNA sequence for LST-S2-H2-10H
SEQ ID NO:60 is the determined cDNA sequence for LST-S2-H2-12D
SEQ ID NO: 61 is the determined cDNA sequence for LST-S3-2
SEQ ID NO: 62 is the determined cDNA sequence for LST-S3-4
SEQ ID NO: 63 is the determined cDNA sequence for LST-S3-7
SEQ ID NO: 64 is the determined cDNA sequence for LST-S3-8
SEQ ID NO: 65 is the determined cDNA sequence for LST-S3-12
SEQ ID NO: 66 is the determined cDNA sequence for LST-S3-13
SEQ ID NO: 67 is the determined cDNA sequence for LST-S3-14
SEQ ID NO: 68 is the determined cDNA sequence for LST-S3-16
SEQ ID NO: 69 is the determined cDNA sequence for LST-S3-21
SEQ ID NO: 70 is the determined cDNA sequence for LST-S3-22
SEQ ID NO: 71 is the determined cDNA sequence for LST-S1-7
SEQ ID NO: 72 is the determined cDNA sequence for LST-S1-A-1E
SEQ ID NO: 73 is the determined cDNA sequence for LST-S1-A-1G
SEQ ID NO: 74 is the determined cDNA sequence for LST-S1-A-3E
SEQ ID NO: 75 is the determined cDNA sequence for LST-S1-A-4E
SEQ ID NO: 76 is the determined cDNA sequence for LST-S1-A-6D
SEQ ID NO: 77 is the determined cDNA sequence for LST-S1-A-8D
SEQ ID NO: 78 is the determined cDNA sequence for LST-S1-A-10A
SEQ ID NO: 79 is the determined cDNA sequence for LST-S1-A-10C
SEQ ID NO: 80 is the determined cDNA sequence for LST-S1-A-9D
SEQ ID NO: 81 is the determined cDNA sequence for LST-S1-A-10D
SEQ ID NO: 82 is the determined cDNA sequence for LST-S1-A-9H
SEQ ID NO: 83 is the determined cDNA sequence for LST-S1-A-11D
SEQ ID NO: 84 is the determined cDNA sequence for LST-S1-A-12D
SEQ ID NO: 85 is the determined cDNA sequence for LST-S1-A-11E
SEQ ID NO: 86 is the determined cDNA sequence for LST-S1-A-12E
SEQ ID NO: 87 is the determined cDNA sequence for L513S (T3).
SEQ ID NO: 88 is the determined cDNA sequence for L513S contig 1.
SEQ ID NO: 89 is a first determined cDNA sequence for L514S.
SEQ ID NO: 90 is a second determined cDNA sequence for L514S.
SEQ ID NO: 91 is a first determined cDNA sequence for L516S.
SEQ ID NO: 92 is a second determined cDNA sequence for L516S.
SEQ ID NO: 93 is the determined cDNA sequence for L517S.
SEQ ID NO: 94 is the extended cDNA sequence for LST-S1-169 (also known as L519S).
SEQ ID NO: 95 is a first determined cDNA sequence for L520S.
SEQ ID NO: 96 is a second determined cDNA sequence for L520S.
SEQ ID NO: 97 is a first determined cDNA sequence for L521 S.
SEQ ID NO: 98 is a second determined cDNA sequence for L521 S.
SEQ ID NO: 99 is the determine cDNA sequence for L522S.
SEQ ID NO: 100 is the determined cDNA sequence for L523S.
SEQ ID NO: 101 is the determined cDNA sequence for L524S.
SEQ ID NO: 102 is the determined cDNA sequence for L525S.
SEQ ID NO: 103 is the determined cDNA sequence for L526S.
SEQ ID NO: 104 is the determined cDNA sequence for L527S.
SEQ ID NO: 105 is the determined cDNA sequence for L528S.
SEQ ID NO: 106 is the determined cDNA sequence for L529S.
SEQ ID NO: 107 is a first determined cDNA sequence for L530S.
SEQ ID NO: 108 is a second determined cDNA sequence for L530S.
SEQ ID NO: 109 is the determined full-length cDNA sequence for L531S short form
SEQ ID NO: 110 is the amino acid sequence encoded by SEQ ID NO: 109.
SEQ ID NO: 111 is the determined full-length cDNA sequence for L531S long form
SEQ ID NO: 112 is the amino acid sequence encoded by SEQ ID NO: 111.
SEQ ID NO: 113 is the determined full-length cDNA sequence for L520S.
SEQ ID NO: 114 is the amino acid sequence encoded by SEQ ID NO: 113.
SEQ ID NO: 115 is the determined cDNA sequence for contig 1.
SEQ ID NO: 116 is the determined cDNA sequence for contig 3.
SEQ ID NO: 117 is the determined cDNA sequence for contig 4.
SEQ ID NO: 118 is the determined cDNA sequence for contig 5.
SEQ ID NO: 119 is the determined cDNA sequence for contig 7.
SEQ ID NO: 120 is the determined cDNA sequence for contig 8.
SEQ ID NO: 121 is the determined cDNA sequence for contig 9.
SEQ ID NO: 122 is the determined cDNA sequence for contig 10.
SEQ ID NO: 123 is the determined cDNA sequence for contig 12.
SEQ ID NO: 124 is the determined cDNA sequence for contig 11.
SEQ ID NO: 125 is the determined cDNA sequence for contig 13 (also known as L761P).
SEQ ID NO: 126 is the determined cDNA sequence for contig 15.
SEQ ID NO: 127 is the determined cDNA sequence for contig 16.
SEQ ID NO: 128 is the determined cDNA sequence for contig 17.
SEQ ID NO: 129 is the determined cDNA sequence for contig 19.
SEQ ID NO: 130 is the determined cDNA sequence for contig 20.
SEQ ID NO: 131 is the determined cDNA sequence for contig 22.
SEQ ID NO: 132 is the determined cDNA sequence for contig 24.
SEQ ID NO: 133 is the determined cDNA sequence for contig 29.
SEQ ID NO: 134 is the determined cDNA sequence for contig 31.
SEQ ID NO: 135 is the determined cDNA sequence for contig 33.
SEQ ID NO: 136 is the determined cDNA sequence for contig 38.
SEQ ID NO: 137 is the determined cDNA sequence for contig 39.
SEQ ID NO: 138 is the determined cDNA sequence for contig 41.
SEQ ID NO: 139 is the determined cDNA sequence for contig 43.
SEQ ID NO: 140 is the determined cDNA sequence for contig 44.
SEQ ID NO: 141 is the determined cDNA sequence for contig 45.
SEQ ID NO: 142 is the determined cDNA sequence for contig 47.
SEQ ID NO: 143 is the determined cDNA sequence for contig 48.
SEQ ID NO: 144 is the determined cDNA sequence for contig 49.
SEQ ID NO: 145 is the determined cDNA sequence for contig 50.
SEQ ID NO: 146 is the determined cDNA sequence for contig 53.
SEQ ID NO: 147 is the determined cDNA sequence for contig 54.
SEQ ID NO: 148 is the determined cDNA sequence for contig 56.
SEQ ID NO: 149 is the determined cDNA sequence for contig 57.
SEQ ID NO: 150 is the determined cDNA sequence for contig 58.
SEQ ID NO: 151 is the full-length cDNA sequence for L530S.
SEQ ID NO: 152 is the amino acid sequence encoded by SEQ ID NO: 151
SEQ ID NO: 153 is the full-length cDNA sequence of a first variant of L514S
SEQ ID NO: 154 is the full-length cDNA sequence of a second variant of L514S
SEQ ID NO: 155 is the amino acid sequence encoded by SEQ ID NO: 153.
SEQ ID NO: 156 is the amino acid sequence encoded by SEQ ID NO: 154.
SEQ ID NO: 157 is the determined cDNA sequence for contig 59.
SEQ ID NO: 158 is the full-length cDNA sequence for L763P (also referred to as contig 22).
SEQ ID NO: 159 is the amino acid sequence encoded by SEQ ID NO: 158.
SEQ ID NO: 160 is the full-length cDNA sequence for L762P (also referred to as contig 17).
SEQ ID NO: 161 is the amino acid sequence encoded by SEQ ID NO: 160.
SEQ ID NO: 162 is the determined cDNA sequence for L515S.
SEQ ID NO: 163 is the full-length cDNA sequence of a first variant of L524S.
SEQ ID NO: 164 is the full-length cDNA sequence of a second variant of L524S.
SEQ ID NO: 165 is the amino acid sequence encoded by SEQ ID NO: 163.
SEQ ID NO: 166 is the amino acid sequence encoded by SEQ ID NO: 164.
SEQ ID NO: 167 is the full-length cDNA sequence of a first variant of L762P.
SEQ ID NO: 168 is the full-length cDNA sequence of a second variant of L762P.
SEQ ID NO: 169 is the amino acid sequence encoded by SEQ ID NO: 167.
SEQ ID NO: 170 is the amino acid sequence encoded by SEQ ID NO: 168.
SEQ ID NO: 171 is the full-length cDNA sequence for L773P (also referred to as contig 56).
SEQ ID NO: 172 is the amino acid sequence encoded by SEQ ID NO: 171.
SEQ ID NO: 173 is an extended cDNA sequence for L519S.
SEQ ID NO: 174 is the amino acid sequence encoded by SEQ ID NO: 174.
SEQ ID NO: 175 is the full-length cDNA sequence for L523S.
SEQ ID NO: 176 is the amino acid sequence encoded by SEQ ID NO: 175.
SEQ ID NO: 177 is the determined cDNA sequence for LST-sub5-7A.
SEQ ID NO: 178 is the determined cDNA sequence for LST-sub5-8G.
SEQ ID NO: 179 is the determined cDNA sequence for LST-sub5-8H.
SEQ ID NO: 180 is the determined cDNA sequence for LST-sub5-10B.
SEQ ID NO: 181 is the determined cDNA sequence for LST-sub5-10H.
SEQ ID NO: 182 is the determined cDNA sequence for LST-sub5-12B.
SEQ ID NO: 183 is the determined cDNA sequence for LST-sub5-11C.
SEQ ID NO: 184 is the determined cDNA sequence for LST-sub6-1c.
SEQ ID NO: 185 is the determined cDNA sequence for LST-sub6-2f.
SEQ ID NO: 186 is the determined cDNA sequence for LST-sub6-2G.
SEQ ID NO: 187 is the determined cDNA sequence for LST-sub6-4d.
SEQ ID NO: 188 is the determined cDNA sequence for LST-sub6-4e.
SEQ ID NO: 189 is the determined cDNA sequence for LST-sub6-4f.
SEQ ID NO: 190 is the determined cDNA sequence for LST-sub6-3h.
SEQ ID NO: 191 is the determined cDNA sequence for LST-sub6-5d.
SEQ ID NO: 192 is the determined cDNA sequence for LST-sub6-5h.
SEQ ID NO: 193 is the determined cDNA sequence for LST-sub6-6h.
SEQ ID NO: 194 is the determined cDNA sequence for LST-sub6-7a.
SEQ ID NO: 195 is the determined cDNA sequence for LST-sub6-8a.
SEQ ID NO: 196 is the determined cDNA sequence for LST-sub6-7d.
SEQ ID NO: 197 is the determined cDNA sequence for LST-sub6-7e.
SEQ ID NO: 198 is the detennined cDNA sequence for LST-sub6-8e.
SEQ ID NO: 199 is the determined cDNA sequence for LST-sub6-7g.
SEQ ID NO: 200 is the determined cDNA sequence for LST-sub6-9f.
SEQ ID NO: 201 is the determined cDNA sequence for LST-sub6-9h.
SEQ ID NO: 202 is the determined cDNA sequence for LST-sub6-11b.
SEQ ID NO: 203 is the determined cDNA sequence for LST-sub6-11c.
SEQ ID NO: 204 is the determined cDNA sequence for LST-sub6-12c.
SEQ ID NO: 205 is the determined cDNA sequence for LST-sub6-12e.
SEQ ID NO: 206 is the determined cDNA sequence for LST-sub6-12f.
SEQ ID NO: 207 is the determined cDNA sequence for LST-sub6-11g.
SEQ ID NO: 208 is the determined cDNA sequence for LST-sub6-12g.
SEQ ID NO: 209 is the determined cDNA sequence for LST-sub6-12h.
SEQ ID NO: 210 is the determined cDNA sequence for LST-sub6-II-1a.
SEQ ID NO: 211 is the determined cDNA sequence for LST-sub6-II-2b.
SEQ ID NO: 212 is the determined cDNA sequence for LST-sub6-II-2g.
SEQ ID NO: 213 is the determined cDNA sequence for LST-sub6-II-1h.
SEQ ID NO: 214 is the determined cDNA sequence for LST-sub6-II-4a.
SEQ ID NO: 215 is the determined cDNA sequence for LST-sub6-II-4b.
SEQ ID NO: 216 is the determined cDNA sequence for LST-sub6-II-3e.
SEQ ID NO: 217 is the determined cDNA sequence for LST-sub6-II-4f.
SEQ ID NO: 218 is the determined cDNA sequence for LST-sub6-II-4g.
SEQ ID NO: 219 is the determined cDNA sequence for LST-sub6-II-4h.
SEQ ID NO: 220 is the determined cDNA sequence for LST-sub6-II-5c.
SEQ ID NO: 221 is the determined cDNA sequence for LST-sub6-II-5e.
SEQ ID NO: 222 is the determined cDNA sequence for LST-sub6-II-6f.
SEQ ID NO: 223 is the determined cDNA sequence for LST-sub6-II-5g.
SEQ ID NO: 224 is the determined cDNA sequence for LST-sub6-II-6g.
SEQ ID NO: 225 is the amino acid sequence for L528S.
SEQ ID NO: 226-251 are synthetic peptides derived from L762P.
SEQ ID NO: 252 is the expressed amino acid sequence of L514S.
SEQ ID NO: 253 is the DNA sequence corresponding to SEQ ID NO: 252.
SEQ ID NO: 254 is the DNA sequence of a L762P expression construct.
SEQ ID NO: 255 is the determined cDNA sequence for clone 23785.
SEQ ID NO: 256 is the determined cDNA sequence for clone 23786.
SEQ ID NO: 257 is the determined cDNA sequence for clone 23788.
SEQ ID NO: 258 is the determined cDNA sequence for clone 23790.
SEQ ID NO: 259 is the determined cDNA sequence for clone 23793.
SEQ ID NO: 260 is the determined cDNA sequence for clone 23794. '
SEQ ID NO: 261 is the determined cDNA sequence for clone 23795.
SEQ ID NO: 262 is the determined cDNA sequence for clone 23796.
SEQ ID NO: 263 is the determined cDNA sequence for clone 23797.
SEQ ID NO: 264 is the determined cDNA sequence for clone 23798.
SEQ ID NO: 265 is the determined cDNA sequence for clone 23799.
SEQ ID NO: 266 is the determined cDNA sequence for clone 23800.
SEQ ID NO: 267 is the determined cDNA sequence for clone 23802.
SEQ ID NO: 268 is the determined cDNA sequence for clone 23803.
SEQ ID NO: 269 is the determined cDNA sequence for clone 23804.
SEQ ID NO: 270 is the determined cDNA sequence for clone 23805.
SEQ ID NO: 271 is the determined cDNA sequence for clone 23806.
SEQ ID NO: 272 is the determined cDNA sequence for clone 23807.
SEQ ID NO: 273 is the determined cDNA sequence for clone 23808.
SEQ ID NO: 274 is the determined cDNA sequence for clone 23809.
SEQ ID NO: 275 is the determined cDNA sequence for clone 23810.
SEQ ID NO: 276 is the determined cDNA sequence for clone 23811.
SEQ ID NO: 277 is the determined cDNA sequence for clone 23812.
SEQ ID NO: 278 is the determined cDNA sequence for clone 23813.
SEQ ID NO: 279 is the determined cDNA sequence for clone 23815.
SEQ ID NO: 280 is the determined cDNA sequence for clone 25298.
SEQ ID NO: 281 is the determined cDNA sequence for clone 25299.
SEQ ID NO: 282 is the determined cDNA sequence for clone 25300.
SEQ ID NO: 283 is the determined cDNA sequence for clone 25301
SEQ ID NO: 284 is the determined cDNA sequence for clone 25304
SEQ ID NO: 285 is the determined cDNA sequence for clone 25309.
SEQ ID NO: 286 is the determined cDNA sequence for clone 25312.
SEQ ID NO: 287 is the determined cDNA sequence for clone 25317.
SEQ ID NO:288 is the determined cDNA sequence for clone 25321.
SEQ ID NO:289 is the determined cDNA sequence for clone 25323.
SEQ ID NO:290 is the determined cDNA sequence for clone 25327.
SEQ ID NO:291 is the determined cDNA sequence for clone 25328.
SEQ ID NO:292 is the determined cDNA sequence for clone 25332.
SEQ ID NO:293 is the determined cDNA sequence for clone 25333.
SEQ ID NO:294 is the determined cDNA sequence for clone 25336.
SEQ ID NO:295 is the determined cDNA sequence for clone 25340.
SEQ ID NO:296 is the determined cDNA sequence for clone 25342.
SEQ ID NO:297 is the determined cDNA sequence for clone 25356.
SEQ ID NO:298 is the determined cDNA sequence for clone 25357.
SEQ ID NO:299 is the determined cDNA sequence for clone 25361.
SEQ ID NO:300 is the determined cDNA sequence for clone 25363.
SEQ ID NO:301 is the determined cDNA sequence for clone 25397.
SEQ ID NO:302 is the determined cDNA sequence for clone 25402.
SEQ ID NO:303 is the determined cDNA sequence for clone 25403.
SEQ ID NO:304 is the determined cDNA sequence for clone 25405.
SEQ ID NO:305 is the determined cDNA sequence for clone 25407.
SEQ ID NO:306 is the determined cDNA sequence for clone 25409.
SEQ ID NO:307 is the determined cDNA sequence for clone 25396.
SEQ ID NO:308 is the determined cDNA sequence for clone 25414.
SEQ ID NO:309 is the determined cDNA sequence for clone 25410.
SEQ ID NO:310 is the determined cDNA sequence for clone 25406.
SEQ ID NO:311 is the determined cDNA sequence for clone 25306.
SEQ ID NO:312 is the determined cDNA sequence for clone 25362.
SEQ ID NO:313 is the determined cDNA sequence for clone 25360.
SEQ ID NO:314 is the determined cDNA sequence for clone 25398.
SEQ ID NO:315 is the determined cDNA sequence for clone 25355.
SEQ ID NO:316 is the determined cDNA sequence for clone 25351.
SEQ ID NO:317 is the determined cDNA sequence for clone 25331.
SEQ ID NO:318 is the determined cDNA sequence for clone 25338.
SEQ ID NO:319 is the determined cDNA sequence for clone 25335.
SEQ ID NO:320 is the determined cDNA sequence for clone 25329.
SEQ ID NO:321 is the determined cDNA sequence for clone 25324.
SEQ ID NO:322 is the determined cDNA sequence for clone 25322.
SEQ ID NO:323 is the determined cDNA sequence for clone 25319.
SEQ ID NO:324 is the determined cDNA sequence for clone 25316.
SEQ ID NO:325 is the determined cDNA sequence for clone 25311.
SEQ ID NO:326 is the determined cDNA sequence for clone 25310.
SEQ ID NO:327 is the determined cDNA sequence for clone 25302.
SEQ ID NO:328 is the determined cDNA sequence for clone 25315.
SEQ ID NO:329 is the determined cDNA sequence for clone 25308.
SEQ ID NO:330 is the determined cDNA sequence for clone 25303.
SEQ ID NOs:331-337 are the cDNA sequences of isoforms of the p53 tumor suppressor homologue, p63 (also referred to as L530S).
SEQ ID NOs:338-344 are the amino acid sequences encoded by SEQ ID NOs:331-337, respectively
SEQ ID NO:345 is a second cDNA sequence for the antigen L763P.
SEQ ID NO:346 is the amino acid sequence encoded by the sequence of SEQ ID NO: 345.
SEQ ID NO:347 is a determined full-length cDNA sequence for L523S.
SEQ ID NO:348 is the amino acid sequence encoded by SEQ ID NO: 347.
SEQ ID NO:349 is the cDNA sequence encoding the N-terminal portion of L773P.
SEQ ID NO:350 is the amino acid sequence of the N-terminal portion of L773P.
SEQ ID NO:351 is the DNA sequence for a fusion of Ra12 and the N-terminal portion of L763P
SEQ ID NO:352 is the amino acid sequence of the fusion of Ra12 and the N-terminal portion of L763P
SEQ ID NO:353 is the DNA sequence for a fusion of Ra12 and the C-terminal portion of L763P
SEQ ID NO:354 is the amino acid sequence of the fusion of Ra12 and the C-tenninal portion of L763P
SEQ ID NO:355 is a primer.
SEQ ID NO:356 is a primer.
SEQ ID NO:357 is the protein sequence of expressed recombinant L762P.
SEQ ID NO:358 is the DNA sequence of expressed recombinant L762P.
SEQ ID NO:359 is a primer.
SEQ ID NO:360 is a primer.
SEQ ID NO:361 is the protein sequence of expressed recombinant L773P A.
SEQ ID NO:362 is the DNA sequence of expressed recombinant L773P A.
SEQ ID NO:363 is an epitope derived from clone L773P polypeptide.
SEQ ID NO:364 is a polynucleotide encoding the polypeptide of SEQ ID NO:363.
SEQ ID NO:365 is an epitope derived from clone L773P polypeptide.
SEQ ID NO:366 is a polynucleotide encoding the polypeptide of SEQ ID NO:365.
SEQ ID NO:367 is an epitope consisting of amino acids 571-590 of SEQ ID NO:161, clone L762P.
SEQ ID NO:368 is the full-length DNA sequence for contig 13 (SEQ ID NO:125), also referred to as L761 P.
SEQ ID NO:369 is the protein sequence encoded by the DNA sequence of SEQ ID NO:368.
SEQ ID NO:370 is an L762P DNA sequence from nucleotides 2071-2130.
SEQ ID NO:371 is an L762P DNA sequence from nucleotides 1441-1500.
SEQ ID NO:372 is an L762P DNA sequence from nucleotides 1936-1955.
SEQ ID NO:373 is an L762P DNA sequence from nucleotides 2620-2679.
SEQ ID NO:374 is an L762P DNA sequence from nucleotides 1801-1860.
SEQ ID NO:375 is an L762P DNA sequence from nucleotides 1531-1591.
SEQ ID NO:376 is the amino acid sequence of the L762P peptide encoded by SEQ ID NO:373.
SEQ ID NO:377 is the amino acid sequence of the L762P peptide encoded by SEQ ID NO:370.
SEQ ID NO:378 is the amino acid sequence of the L762P peptide encoded by SEQ ID NO:372.
SEQ ID NO:379 is the amino acid sequence of the L762P peptide encoded by SEQ ID NO:374.
SEQ ID NO:380 is the amino acid sequence of the L762P peptide encoded by SEQ ID NO:371.
SEQ ID NO:381 is the amino acid sequence of the L762P peptide encoded by SEQ ID NO:375.
SEQ ID NO:382 is the amino acid sequence of an epitope of L762P.
SEQ ID NOs:383-386 are PCR primers.
SEQ ID NOs:387-395 are the amino acid sequences of L773P peptides.
SEQ ID NOs:396-419 are the amino acid sequences of L523S peptides.
SEQ ID NO:420 is the determined cDNA sequence for clone #19014.
SEQ ID NO:421 is the forward primer PDM-278 for the L514S-13160 coding region.
SEQ ID NO:422 is the reverse primer PDM-278 for the L514S-13160 coding region.
SEQ ID NO:423 is the amino acid sequence for the expressed recombinant L514S.
SEQ ID NO:424 is the DNA coding sequence for the recombinant L514S.
SEQ ID NO:425 is the forward primer PDM-414 for the L523S coding region.
SEQ ID NO:426 is the reverse primer PDM-414 for the L523S coding region.
SEQ ID NO:427 is the amino acid sequence for the expressed recombinant L523S.
SEQ ID NO:428 is the DNA coding sequence for the recombinant L523S.
SEQ ID NO:429 is the reverse primer PDM-279 for the L762PA coding region.
SEQ ID NO:430 is the amino acid sequence for the expressed recombinant L762PA.
SEQ ID NO:431 is the DNA coding sequence for the recombinant L762PA.
SEQ ID NO:432 is the reverse primer PDM-300 for the L773P coding region.
SEQ ID NO:433 is the amino acid sequence of the expressed recombinant L773P.
SEQ ID NO:434 is the DNA coding sequence for the recombinant L773P.
SEQ ID NO:435 is the forward primer for TCR Valpha8.
SEQ ID NO:436 is the reverse primer for TCR Valpha8.
SEQ ID NO:437 is the forward primer for TCR Vbeta8.
SEQ ID NO:438 is the reverse primer for TCR Vbeta8.
SEQ ID NO:439 is the TCR Valpha DNA sequence of the TCR clone specific for the lung antigen L762P.
SEQ ID NO:440 is the TCR Vbeta DNA sequence of the TCR clone specific for the lung antigen L762P.
SEQ ID NO:441 is the amino acid sequence of L763 peptide #2684.
SEQ ID NO:442 is the predicted full-length cDNA for the cloned partial sequence of clone L529S (SEQ ID NO:106).
SEQ ID NO:443 is the deduced amino acid sequence encoded by SEQ ID NO:442
SEQ ID NO:444 is the forward primer PDM-734 for the coding region of clone L523S.
SEQ ID NO:445 is the reverse primer PDM-735 for the coding region of clone L523S.
SEQ ID NO:446 is the amino acid sequence for the expressed recombinant L523S.
SEQ ID NO:447 is the DNA coding sequence for the recombinant L523S.
SEQ ID NO:448 is another forward primer PDM-733 for the coding region of clone L523S.
SEQ ID NO:449 is the amino acid sequence for a second expressed recombinant L523S.
SEQ ID NO:450 is the DNA coding sequence for a second recombinant L523S.
SEQ ID NO:451 corresponds to amino acids 86-110, an epitope of L514S-specific in the generation of antibodies.
SEQ ID NO:452 corresponds to amino acids 21-45, an epitope of L514S-specific in the generation of antibodies.
SEQ ID NO:453 corresponds to amino acids 121-135, an epitope of L514S-specific in the generation of antibodies.
SEQ ID NO:454 corresponds to amino acids 440-460, an epitope of L523S-specific in the generation of antibodies.
SEQ ID NO:455 corresponds to amino acids 156-175, an epitope of L523S-specific in the generation of antibodies.
SEQ ID NO:456 corresponds to amino acids 326-345, an epitope of L523S-specific in the generation of antibodies.
SEQ ID NO:457 corresponds to amino acids 40-59, an epitope of L523S-specific in the generation of antibodies.
SEQ ID NO:458 corresponds to amino acids 80-99, an epitope of L523S-specific in the generation of antibodies.
SEQ ID NO:459 corresponds to amino acids 160-179, an epitope of L523S-specific in the generation of antibodies.
SEQ ID NO:460 corresponds to amino acids 180-199, an epitope of L523S-specific in the generation of antibodies.
SEQ ID NO:461 corresponds to amino acids 320-339, an epitope of L523S-specific in the generation of antibodies.
SEQ ID NO:462 corresponds to amino acids 340-359, an epitope of L523S-specific in the generation of antibodies.
SEQ ID NO:463 corresponds to amino acids 370-389, an epitope of L523S-specific in the generation of antibodies.
SEQ ID NO:464 corresponds to amino acids 380-399, an epitope of L523S-specific in the generation of antibodies.
SEQ ID NO:465 corresponds to amino acids 37-55, an epitope of L523S-recognized by the L523S-specific CTL line 6B1.
SEQ ID NO:466 corresponds to amino acids 41-51, the mapped antigenic epitope of L523S-recognized by the L523S-specific CTL line 6B1.
SEQ ID NO:467 corresponds to the DNA sequence which encodes SEQ ID NO:466.

### DETAILED DESCRIPTION OF THE INVENTION

The present invention is directed generally to compositions and their use in the therapy and diagnosis of cancer, particularly lung cancer. As described further below, illustrative compositions of the present invention include, but are not restricted to, polypeptides, particularly immunogenic polypeptides, polynucleotides encoding such polypeptides, antibodies and other binding agents, antigen presenting cells (APCs) and immune system cells (e.g., T cells).

The practice of the present invention will employ, unless indicated specifically to the contrary, conventional methods of virology, immunology, microbiology, molecular biology and recombinant DNA techniques within the skill of the art, many of which are described below for the purpose of illustration. Such techniques are explained fully in the literature. See, *e*.*g*., Sambrook, et al. Molecular Cloning: A Laboratory Manual (2nd Edition, 1989); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984).

All publications, patents and patent applications cited herein, whether supra or infra, are hereby incorporated by reference in their entirety.

As used in this specification and the appended claims, the singular forms "a," "an" and "the" include plural references unless the content clearly dictates otherwise.

### Polypeptide Compositions

As used herein, the term "polypeptide" " is used in its conventional meaning, *i*.*e*., as a sequence of amino acids. The polypeptides are not limited to a specific length of the product; thus, peptides, oligopeptides, and proteins are included within the definition of polypeptide, and such terms may be used interchangeably herein unless specifically indicated otherwise. This term also does not refer to or exclude post-expression modifications of the polypeptide, for example, glycosylations, acetylations, phosphorylations and the like, as well as other modifications known in the art, both naturally occurring and non-naturally occurring. A polypeptide may be an entire protein, or a subsequence thereof. Particular polypeptides of interest in the context of this invention are amino acid subsequences comprising epitopes, i.e., antigenic determinants substantially responsible for the immunogenic properties of a polypeptide and being capable of evoking an immune response.

Particularly illustrative polypeptides of the present invention comprise those encoded by a polynucleotide sequence set forth in any one of SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467, or a sequence that hybridizes under moderately stringent conditions, or, alternatively, under highly stringent conditions, to a polynucleotide sequence set forth in any one of SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467. Certain illustrative polypeptides of the invention comprise amino acid sequences as set forth in any one of SEQ ID NOs:152, 155, 156, 165, 166, 169, 170, 172, 174, 176, 226-252, 338-344, 346, 350, 357, 361, 363, 365, 367,369, 376-382,387-419,423,427,430,433,441,443,446,449 and 451-466.

The polypeptides of the present invention are sometimes herein referred to as lung tumor proteins or lung tumor polypeptides, as an indication that their identification has been based at least in part upon their increased levels of expression in lung tumor samples. Thus, a "lung tumor polypeptide" or "lung tumor protein," refers generally to a polypeptide sequence of the present invention, or a polynucleotide sequence encoding such a polypeptide, that is expressed in a substantial proportion of lung tumor samples, for example preferably greater than about 20%, more preferably greater than about 30%, and most preferably greater than about 50% or more of lung tumor samples tested, at a level that is at least two fold, and preferably at least five fold, greater than the level of expression in normal tissues, as determined using a representative assay provided herein. A lung tumor polypeptide sequence of the invention, based upon its increased level of expression in tumor cells, has particular utility both as a diagnostic marker as well as a therapeutic target, as further described below.

In certain preferred embodiments, the polypeptides of the invention are immunogenic, i.e., they react detectably within an immunoassay (such as an ELISA or T-cell stimulation assay) with antisera and/or T-cells from a patient with lung cancer. Screening for immunogenic activity can be performed using techniques well known to the skilled artisan. For example, such screens can be performed using methods such as those described in Harlow and Lane, Antibodies: A Laboratory Manual, Cold Spring Harbor Laboratory, 1988. In one illustrative example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As would be recognized by the skilled artisan, immunogenic portions of the polypeptides disclosed herein are also encompassed by the present invention. An "immunogenic portion," as used herein, is a fragment of an immunogenic polypeptide of the invention that itself is immunologically reactive (*i*.*e*., specifically binds) with the B-cells and/or T-cell surface antigen receptors that recognize the polypeptide. Immunogenic portions may generally be identified using well known techniques, such as those summarized in Paul, Fundamental Immunology, 3rd ed., 243-247 (Raven Press, 1993) and references cited therein. Such techniques include screening polypeptides for the ability to react with antigen-specific antibodies, antisera and/or T-cell lines or clones. As used herein, antisera and antibodies are "antigen-specific" if they specifically bind to an antigen (*i.e*., they react with the protein in an ELISA or other immunoassay, and do not react detectably with unrelated proteins). Such antisera and antibodies may be prepared as described herein, and using well-known techniques.

In one preferred embodiment, an immunogenic portion of a polypeptide of the present invention is a portion that reacts with antisera and/or T-cells at a level that is not substantially less than the reactivity of the full-length polypeptide (*e*.*g*., in an ELISA and/or T-cell reactivity assay). Preferably, the level of immunogenic activity of the immunogenic portion is at least about 50%, preferably at least about 70% and most preferably greater than about 90% of the immunogenicity for the full-length polypeptide. In some instances, preferred immunogenic portions will be identified that have a level of immunogenic activity greater than that of the corresponding full-length polypeptide, *e*.*g*., having greater than about 100% or 150% or more immunogenic activity.

In certain other embodiments, illustrative immunogenic portions may include peptides in which an N-terminal leader sequence and/or transmembrane domain have been deleted. Other illustrative immunogenic portions will contain a small N-and/or C-terminal deletion (*e*.*g*., 1-30 amino acids, preferably 5-15 amino acids), relative to the mature protein.

In another embodiment, a polypeptide composition of the invention may also comprise one or more polypeptides that are immunologically reactive with T cells and/or antibodies generated against a polypeptide of the invention, particularly a polypeptide having an amino acid sequence disclosed herein, or to an immunogenic fragment or variant thereof.

In another embodiment of the invention, polypeptides are provided that comprise one or more polypeptides that are capable of eliciting T cells and/or antibodies that are immunologically reactive with one or more polypeptides described herein, or one or more polypeptides encoded by contiguous nucleic acid sequences contained in the polynucleotide sequences disclosed herein, or immunogenic fragments or variants thereof, or to one or more nucleic acid sequences which hybridize to one or more of these sequences under conditions of moderate to high stringency.

The present invention, in another aspect, provides polypeptide fragments comprising at least about 5, 10, 15, 20, 25, 50, or 100 contiguous amino acids, or more, including all intermediate lengths, of a polypeptide compositions set forth herein, such as those set forth in SEQ ID NOs:152, 155, 156, 165, 166, 169, 170, 172, 174, 176, 226-252, 338-344, 346, 350, 357, 361, 363, 365, 367, 369, 376-382 and 387-419, 441, 443, 446, 449 and 451-466, or those encoded by a polynucleotide sequence set forth in a sequence of SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467.

In another aspect, the present invention provides variants of the polypeptide compositions described herein. Polypeptide variants generally encompassed by the present invention will typically exhibit at least about 70%, 75%, 80%, 85%, 90%, 91%, 92%, 93%, 94%, 95%, 96%, 97%, 98%, or 99% or more identity (determined as described below), along its length, to a polypeptide sequences set forth herein.

In one preferred embodiment, the polypeptide fragments and variants provide by the present invention are immunologically reactive with an antibody and/or T-cell that reacts with a full-length polypeptide specifically set for the herein.

In another preferred embodiment, the polypeptide fragments and variants provided by the present invention exhibit a level of immunogenic activity of at least about 50%, preferably at least about 70%, and most preferably at least about 90% or more of that exhibited by a full-length polypeptide sequence specifically set forth herein.

A polypeptide "variant," as the term is used herein, is a polypeptide that typically differs from a polypeptide specifically disclosed herein in one or more substitutions, deletions, additions and/or insertions. Such variants may be naturally occurring or may be synthetically generated, for example, by modifying one or more of the above polypeptide sequences of the invention and evaluating their immunogenic activity as described herein and/or using any of a number of techniques well known in the art.

For example, certain illustrative variants of the polypeptides of the invention include those in which one or more portions, such as an N-terminal leader sequence or transmembrane domain, have been removed. Other illustrative variants include variants in which a small portion (*e*.*g*., 1-30 amino acids, preferably 5-15 amino acids) has been removed from the N- and/or C-terminal of the mature protein.

In many instances, a variant will contain conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. As described above, modifications may be made in the structure of the polynucleotides and polypeptides of the present invention and still obtain a functional molecule that encodes a variant or derivative polypeptide with desirable characteristics, *e*.*g*., with immunogenic characteristics. When it is desired to alter the amino acid sequence of a polypeptide to create an equivalent, or even an improved, immunogenic variant or portion of a polypeptide of the invention, one skilled in the art will typically change one or more of the codons of the encoding DNA sequence according to Table 1.

For example, certain amino acids may be substituted for other amino acids in a protein structure without appreciable loss of interactive binding capacity with structures such as, for example, antigen-binding regions of antibodies or binding sites on substrate molecules. Since it is the interactive capacity and nature of a protein that defines that protein's biological functional activity, certain amino acid sequence substitutions can be made in a protein sequence, and, of course, its underlying DNA coding sequence, and nevertheless obtain a protein with like properties. It is thus contemplated that various changes may be made in the peptide sequences of the disclosed compositions, or corresponding DNA sequences which encode said peptides without appreciable loss of their biological utility or activity.

**TABLE 1**

| **Amino Acids** | | **Codons** | | | | | | |
|---|---|---|---|---|---|---|---|---|
| Alanine | Ala | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Lysine | Lys | K | AAA | AAG | | | | |
| Leucine | Leu | L | UUA | UUG | CUA | CUC | CUG | CUU |
| Methionine | Met | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | T | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

In making such changes, the hydropathic index of amino acids may be considered. The importance of the hydropathic amino acid index in conferring interactive biologic function on a protein is generally understood in the art (Kyte and Doolittle, 1982, incorporated herein by reference). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of its hydrophobicity and charge characteristics (Kyte and Doolittle, 1982). These values are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is known in the art that certain amino acids may be substituted by other amino acids having a similar hydropathic index or score and still result in a protein with similar biological activity, *i*.*e*. still obtain a biological functionally equivalent protein. In making such changes, the substitution of amino acids whose hydropathic indices are within ±2 is preferred, those within ±1 are particularly preferred, and those within ±0.5 are even more particularly preferred. It is also understood in the art that the substitution of like amino acids can be made effectively on the basis of hydrophilicity. U. S. Patent 4,554,101 (specifically incorporated herein by reference in its entirety), states that the greatest local average hydrophilicity of a protein, as governed by the hydrophilicity of its adjacent amino acids, correlates with a biological property of the protein.

As detailed in U. S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent protein. In such changes, the substitution of amino acids whose hydrophilicity values are within ±2 is preferred, those within ±1 are particularly preferred, and those within ±0.5 are even more particularly preferred.

As outlined above, amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine.

In addition, any polynucleotide may be further modified to increase stability *in vivo*. Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetylmethyl-, thio- and other modified forms of adenine, cytidine, guanine, thymine and uridine.

Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain non-conservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein, which co-translationally or post-translationally directs transfer of the protein. The polypeptide may also be conjugated to a linker or other sequence for ease of synthesis, purification or identification of the polypeptide (*e*.*g*., poly-His), or to enhance binding of the polypeptide to a solid support. For example, a polypeptide may be conjugated to an immunoglobulin Fc region.

When comparing polypeptide sequences, two sequences are said to be "identical" if the sequence of amino acids in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) CABIOS 5:151-153; Myers, E.W. and Muller W. (1988) CABIOS 4:11-17; Robinson, E.D. (1971) Comb. Theor 11:105; Santou, N. Nes, M. (1987) Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Proc. Natl. Acad., Sci. USA 80:726-730.

Alternatively, optimal alignment of sequences for comparison may be conducted by the local identity algorithm of Smith and Waterman (1981) Add. APL. Math 2:482, by the identity alignment algorithm of Needleman and Wunsch (1970) J. Mol. Biol. 48:443, by the search for similarity methods of Pearson and Lipman (1988) Proc. Natl. Acad. Sci. USA 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One preferred example of algorithms that are suitable for determining percent sequence identity and sequence similarity are the BLAST and BLAST 2.0 algorithms, which are described in Altschul et al. (1977) Nucl. Acids Res. 25:3389-3402 and Altschul et al. (1990) J. Mol. Biol. 215:403-410, respectively. BLAST and BLAST 2.0 can be used, for example with the parameters described herein, to determine percent sequence identity for the polynucleotides and polypeptides of the invention. Software for performing BLAST analyses is publicly available through the National Center for Biotechnology Information. For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment.

In one preferred approach, the "percentage of sequence identity" is determined by comparing two optimally aligned sequences over a window of comparison of at least 20 positions, wherein the portion of the polypeptide sequence in the comparison window may comprise additions or deletions (*i*.*e*., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the reference sequence (*i*.*e*., the window size) and multiplying the results by 100 to yield the percentage of sequence identity.

Within other illustrative embodiments, a polypeptide may be a fusion polypeptide that comprises multiple polypeptides as described herein, or that comprises at least one polypeptide as described herein and an unrelated sequence, such as a known tumor protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the polypeptide or to enable the polypeptide to be targeted to desired intracellular compartments. Still further fusion partners include affinity tags, which facilitate purification of the polypeptide.

Fusion polypeptides may generally be prepared using standard techniques, including chemical conjugation. Preferably, a fusion polypeptide is expressed as a recombinant polypeptide, allowing the production of increased levels, relative to a non-fused polypeptide, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion polypeptide that retains the biological activity of both component polypeptides.

A peptide linker sequence may be employed to separate the first and second polypeptide components by a distance sufficient to ensure that each polypeptide folds into its secondary and tertiary structures. Such a peptide linker sequence is incorporated into the fusion polypeptide using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

The fusion polypeptide can comprise a polypeptide as described herein together with an unrelated immunogenic protein, such as an immunogenic protein capable of eliciting a recall response. Examples of such proteins include tetanus, tuberculosis and hepatitis proteins (*see,* for example, Stoute et al. New Engl. J. Med., 336:86-91, 1997).

In one preferred embodiment, the immunological fusion partner is derived from a Mycobacterium sp., such as a Mycobacterium tuberculosis-derived Ra12 fragment. Ra12 compositions and methods for their use in enhancing the expression and/or immunogenicity of heterologous polynucleotide/polypeptide sequences is described in U.S. WO 2001/25401 Patent Application 60/158,585. Briefly, Ra12 refers to a polynucleotide region that is a subsequence of a *Mycobacterium tuberculosis* MTB32A nucleic acid. MTB32A is a serine protease of 32 KD molecular weight encoded by a gene in virulent and avirulent strains of *M. tuberculosis*. The nucleotide sequence and amino acid sequence of MTB32A have been described (for example, WO 2001/25401; see also, Skeiky et al., Infection and Immun. (1999) 67:3998-4007). C-terminal fragments of the MTB32A coding sequence express at high levels and remain as a soluble polypeptides throughout the purification process. Moreover, Ra12 may enhance the immunogenicity of heterologous immunogenic polypeptides with which it is fused. One preferred Ra12 fusion polypeptide comprises a 14 KD C-terminal fragment corresponding to amino acid residues 192 to 323 of MTB32A.

Other preferred Ra12 polynucleotides generally comprise at least about 15 consecutive nucleotides, at least about 30 nucleotides, at least about 60 nucleotides, at least about 100 nucleotides, at least about 200 nucleotides, or at least about 300 nucleotides that encode a portion of a Ra12 polypeptide.

Ra12 polynucleotides may comprise a native sequence (*i*.*e*., an endogenous sequence that encodes a Ra12 polypeptide or a portion thereof) or may comprise a variant of such a sequence. Ra12 polynucleotide variants may contain one or more substitutions, additions, deletions and/or insertions such that the biological activity of the encoded fusion polypeptide is not substantially diminished, relative to a fusion polypeptide comprising a native Ra12 polypeptide. Variants preferably exhibit at least about 70% identity, more preferably at least about 80% identity and most preferably at least about 90% identity to a polynucleotide sequence that encodes a native Ra12 polypeptide or a portion thereof.

Within other preferred embodiments, an immunological fusion partner is derived from protein D, a surface protein of the gram-negative bacterium Haemophilus influenza B (WO 91/18926). Preferably, a protein D derivative comprises approximately the first third of the protein (*e*.*g*., the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E*. *coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae*, which synthesizes an N-acetyl-L-alanine amidase known as amidase LYTA (encoded by the LytA gene; Gene 43:265-292, 1986). LYTA is an autolysin that specifically degrades certain bonds in the peptidoglycan backbone. The C-tenninal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described (*see Biotechnology 10*:795-798, 1992). Within a preferred embodiment, a repeat portion of LYTA may be incorporated into a fusion polypeptide. A repeat portion is found in the C-terminal region starting at residue 178. A particularly preferred repeat portion incorporates residues 188-305.

Yet another illustrative embodiment involves fusion polypeptides, and the polynucleotides encoding them, wherein the fusion partner comprises a targeting signal capable of directing a polypeptide to the endosomal/lysosomal compartment, as described in U.S. Patent No. 5,633,234. An immunogenic polypeptide of the invention, when fused with this targeting signal, will associate more efficiently with MHC class II molecules and thereby provide enhanced in vivo stimulation of CD4⁺ T-cells specific for the polypeptide.

Polypeptides of the invention are prepared using any of a variety of well known synthetic and/or recombinant techniques, the latter of which are further described below. Polypeptides, portions and other variants generally less than about 150 amino acids can be generated by synthetic means, using techniques well known to those of ordinary skill in the art. In one illustrative example, such polypeptides are synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Perkin Elmer/Applied BioSystems Division (Foster City, CA), and may be operated according to the manufacturer's instructions.

In general, polypeptide compositions (including fusion polypeptides) of the invention are isolated. An "isolated" polypeptide is one that is removed from its original environment. For example, a naturally-occurring protein or polypeptide is isolated if it is separated from some or all of the coexisting materials in the natural system. Preferably, such polypeptides are also purified, *e*.*g*., are at least about 90% pure, more preferably at least about 95% pure and most preferably at least about 99% pure.

### Polynucleotide Compositions

The present invention, in other aspects, provides polynucleotide compositions. The terms "DNA" and "polynucleotide" are used essentially interchangeably herein to refer to a DNA molecule that has been isolated free of total genomic DNA of a particular species. "Isolated," as used herein, means that a polynucleotide is substantially away from other coding sequences, and that the DNA molecule does not contain large portions of unrelated coding DNA, such as large chromosomal fragments or other functional genes or polypeptide coding regions. Of course, this refers to the DNA molecule as originally isolated, and does not exclude genes or coding regions later added to the segment by the hand of man.

As will be understood by those skilled in the art, the polynucleotide compositions of this invention can include genomic sequences, extra-genomic and plasmid-encoded sequences and smaller engineered gene segments that express, or may be adapted to express, proteins, polypeptides, peptides and the like. Such segments may be naturally isolated, or modified synthetically by the hand of man.

As will be also recognized by the skilled artisan, polynucleotides of the invention may be single-stranded (coding or antisense) or double-stranded, and may be DNA (genomic, cDNA or synthetic) or RNA molecules. RNA molecules may include HnRNA molecules, which contain introns and correspond to a DNA molecule in a one-to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (*i*.*e*., an endogenous sequence that encodes a polypeptide/protein of the invention or a portion thereof) or may comprise a sequence that encodes a variant or derivative, preferably and immunogenic variant or derivative, of such a sequence.

Therefore, according to another aspect of the present invention, polynucleotide compositions are provided that comprise some or all of a polynucleotide sequence set forth in any one of SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467, complements of a polynucleotide sequence set forth in any one of SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467, and degenerate variants of a polynucleotide sequence set forth in any one of SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467. In certain preferred embodiments, the polynucleotide sequences set forth herein encode immunogenic polypeptides, as described above.

In other related embodiments, the present invention provides polynucleotide variants having substantial identity to the sequences disclosed herein in SEQ ID NOs:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467, for example those comprising at least 70% sequence identity, preferably at least 75%, 80%, 85%, 90%, 95%, 96%, 97%, 98%, or 99% or higher, sequence identity compared to a polynucleotide sequence of this invention using the methods described herein, (*e*.*g*., BLAST analysis using standard parameters, as described below). One skilled in this art will recognize that these values can be appropriately adjusted to determine corresponding identity of proteins encoded by two nucleotide sequences by taking into account codon degeneracy, amino acid similarity, reading frame positioning and the like.

Typically, polynucleotide variants will contain one or more substitutions, additions, deletions and/or insertions, preferably such that the immunogenicity of the polypeptide encoded by the variant polynucleotide is not substantially diminished relative to a polypeptide encoded by a polynucleotide sequence specifically set forth herein). The term "variants" should also be understood to encompasses homologous genes of xenogenic origin.

In additional embodiments, the present invention provides polynucleotide fragments comprising various lengths of contiguous stretches of sequence identical to or complementary to one or more of the sequences disclosed herein. For example, polynucleotides are provided by this invention that comprise at least about 10, 15, 20, 30, 40, 50, 75, 100, 150, 200, 300, 400, 500 or 1000 or more contiguous nucleotides of one or more of the sequences disclosed herein as well as all intermediate lengths there between. It will be readily understood that "intermediate lengths", in this context, means any length between the quoted values, such as 16, 17, 18, 19, *etc*.; 21, 22, 23, *etc*.; 30, 31, 32, *etc.;* 50, 51, 52, 53, *etc*.; 100, 101, 102, 103, *etc*.; 150, 151, 152, 153, etc.; including all integers through 200-500; 500-1,000, and the like.

In another embodiment of the invention, polynucleotide compositions are provided that are capable of hybridizing under moderate to high stringency conditions to a polynucleotide sequence provided herein, or a fragment thereof, or a complementary sequence thereof. Hybridization techniques are well known in the art of molecular biology. For purposes of illustration, suitable moderately stringent conditions for testing the hybridization of a polynucleotide of this invention with other polynucleotides include prewashing in a solution of 5 X SSC, 0.5% SDS, 1.0 mM EDTA (pH 8.0); hybridizing at 50°C-60°C, 5 X SSC, overnight; followed by washing twice at 65°C for 20 minutes with each of 2X, 0.5X and 0.2X SSC containing 0.1% SDS. One skilled in the art will understand that the stringency of hybridization can be readily manipulated, such as by altering the salt content of the hybridization solution and/or the temperature at which the hybridization is performed. For example, in another embodiment, suitable highly stringent hybridization conditions include those described above, with the exception that the temperature of hybridization is increased, *e*.*g*., to 60-65°C or 65-70°C.

In certain preferred embodiments, the polynucleotides described above, *e*.*g*., polynucleotide variants, fragments and hybridizing sequences, encode polypeptides that are immunologically cross-reactive with a polypeptide sequence specifically set forth herein. In other preferred embodiments, such polynucleotides encode polypeptides that have a level of immunogenic activity of at least about 50%, preferably at least about 70%, and more preferably at least about 90% of that for a polypeptide sequence specifically set forth herein.

The polynucleotides of the present invention, or fragments thereof, regardless of the length of the coding sequence itself, may be combined with other DNA sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant DNA protocol. For example, illustrative polynucleotide segments with total lengths of about 10,000, about 5000, about 3000, about 2,000, about 1,000, about 500, about 200, about 100, about 50 base pairs in length, and the like, (including all intermediate lengths) are contemplated to be useful in many implementations of this invention.

When comparing polynucleotide sequences, two sequences are said to be "identical" if the sequence of nucleotides in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) CABIOS 5:151-153; Myers, E.W. and Muller W. (1988) CABIOS 4:11-17; Robinson, E.D. (1971) Comb. Theor 11:105; Santou, N. Nes, M. (1987) Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Proc. Natl. Acad., Sci. USA 80:726-730.

Alternatively, optimal alignment of sequences for comparison may be conducted by the local identity algorithm of Smith and Waterman (1981) Add. APL. Math 2:482, by the identity alignment algorithm of Needleman and Wunsch (1970) J. Mol. Biol. 48:443, by the search for similarity methods of Pearson and Lipman (1988) Proc. Natl. Acad. Sci. USA 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One preferred example of algorithms that are suitable for determining percent sequence identity and sequence similarity are the BLAST and BLAST 2.0 algorithms, which are described in Altschul et al. (1977) Nucl. Acids Res. 25:3389-3402 and Altschul et al. (1990) J. Mol. Biol. 215:403-410, respectively. BLAST and BLAST 2.0 can be used, for example with the parameters described herein, to determine percent sequence identity for the polynucleotides of the invention. Software for performing BLAST analyses is publicly available through the National Center for Biotechnology Information. In one illustrative example, cumulative scores can be calculated using, for nucleotide sequences, the parameters M (reward score for a pair of matching residues; always >0) and N (penalty score for mismatching residues; always <0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a wordlength (W) of 11, and expectation (E) of 10, and the BLOSUM62 scoring matrix (see Henikoff and Henikoff (1989) Proc. Natl. Acad. Sci. USA 89:10915) alignments, (B) of 50, expectation (E) of 10, M=5, N=-4 and a comparison of both strands.

Preferably, the "percentage of sequence identity" is determined by comparing two optimally aligned sequences over a window of comparison of at least 20 positions, wherein the portion of the polynucleotide sequence in the comparison window may comprise additions or deletions (*i.e*., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases occurs in both sequences to yield the number of matched positions, dividing the number of matched positions by the total number of positions in the reference sequence (*i*.*e*., the window size) and multiplying the results by 100 to yield the percentage of sequence identity.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Therefore, in another embodiment of the invention, a mutagenesis approach, such as site-specific mutagenesis, is employed for the preparation of immunogenic variants and/or derivatives of the polypeptides described herein. By this approach, specific modifications in a polypeptide sequence can be made through mutagenesis of the underlying polynucleotides that encode them. These techniques provides a straightforward approach to prepare and test sequence variants, for example, incorporating one or more of the foregoing considerations, by introducing one or more nucleotide sequence changes into the polynucleotide.

Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Mutations may be employed in a selected polynucleotide sequence to improve, alter, decrease, modify, or otherwise change the properties of the polynucleotide itself, and/or alter the properties, activity, composition, stability, or primary sequence of the encoded polypeptide.

In certain embodiments of the present invention, the inventors contemplate the mutagenesis of the disclosed polynucleotide sequences to alter one or more properties of the encoded polypeptide, such as the immunogenicity of a polypeptide vaccine. The techniques of site-specific mutagenesis are well-known in the art, and are widely used to create variants of both polypeptides and polynucleotides. For example, site-specific mutagenesis is often used to alter a specific portion of a DNA molecule. In such embodiments, a primer comprising typically about 14 to about 25 nucleotides or so in length is employed, with about 5 to about 10 residues on both sides of the junction of the sequence being altered.

As will be appreciated by those of skill in the art, site-specific mutagenesis techniques have often employed a phage vector that exists in both a single stranded and double stranded form. Typical vectors useful in site-directed mutagenesis include vectors such as the M13 phage. These phage are readily commercially-available and their use is generally well-known to those skilled in the art. Double-stranded plasmids are also routinely employed in site directed mutagenesis that eliminates the step of transferring the gene of,interest from a plasmid to a phage.

In general, site-directed mutagenesis in accordance herewith is performed by first obtaining a single-stranded vector or melting apart of two strands of a double-stranded vector that includes within its sequence a DNA sequence that encodes the desired peptide. An oligonucleotide primer bearing the desired mutated sequence is prepared, generally synthetically. This primer is then annealed with the single-stranded vector, and subjected to DNA polymerizing enzymes such as *E. coli* polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform appropriate cells, such as *E. coli* cells, and clones are selected which include recombinant vectors bearing the mutated sequence arrangement.

The preparation of sequence variants of the selected peptide-encoding DNA segments using site-directed mutagenesis provides a means of producing potentially useful species and is not meant to be limiting as there are other ways in which sequence variants of peptides and the DNA sequences encoding them may be obtained. For example, recombinant vectors encoding the desired peptide sequence may be treated with mutagenic agents, such as hydroxylamine, to obtain sequence variants. Specific details regarding these methods and protocols are found in the teachings of Maloy *et al*., 1994; Segal, 1976; Prokop and Bajpai, 1991; Kuby, 1994; and Maniatis *et al*., 1982, for that purpose.

As used herein, the term "oligonucleotide directed mutagenesis procedure" refers to template-dependent processes and vector-mediated propagation which result in an increase in the concentration of a specific nucleic acid molecule relative to its initial concentration, or in an increase in the concentration of a detectable signal, such as amplification. As used herein, the term "oligonucleotide directed mutagenesis procedure" is intended to refer to a process that involves the template-dependent extension of a primer molecule. The term template dependent process refers to nucleic acid synthesis of an RNA or a DNA molecule wherein the sequence of the newly synthesized strand of nucleic acid is dictated by the well-known rules of complementary base pairing (see, for example, Watson, 1987). Typically, vector mediated methodologies involve the introduction of the nucleic acid fragment into a DNA or RNA vector, the clonal amplification of the vector, and the recovery of the amplified nucleic acid fragment. Examples of such methodologies are provided by U. S. Patent No. 4,237,224.

In another approach for the production of polypeptide variants of the present invention, recursive sequence recombination, as described in U.S. Patent No. 5,837,458, may be employed. In this approach, iterative cycles of recombination and screening or selection are performed to "evolve" individual polynucleotide variants of the invention having, for example, enhanced immunogenic activity.

In other embodiments of the present invention, the polynucleotide sequences provided herein can be advantageously used as probes or primers for nucleic acid hybridization. As such, it is contemplated that nucleic acid segments that comprise a sequence region of at least about 15 nucleotide long contiguous sequence that has the same sequence as, or is complementary to, a 15 nucleotide long contiguous sequence disclosed herein will find particular utility. Longer contiguous identical or complementary sequences, *e*.*g*., those of about 20, 30, 40, 50, 100, 200, 500, 1000 (including all intermediate lengths) and even up to full length sequences will also be of use in certain embodiments.

The ability of such nucleic acid probes to specifically hybridize to a sequence of interest will enable them to be of use in detecting the presence of complementary sequences in a given sample. However, other uses are also envisioned, such as the use of the sequence information for the preparation of mutant species primers, or primers for use in preparing other genetic constructions.

Polynucleotide molecules having sequence regions consisting of contiguous nucleotide stretches of 10-14, 15-20, 30, 50, or even of 100-200 nucleotides or so (including intermediate lengths as well), identical or complementary to a polynucleotide sequence disclosed herein, are particularly contemplated as hybridization probes for use in, e.g., Southern and Northern blotting. This would allow a gene product, or fragment thereof, to be analyzed, both in diverse cell types and also in various bacterial cells. The total size of fragment, as well as the size of the complementary stretch(es), will ultimately depend on the intended use or application of the particular nucleic acid segment. Smaller fragments will generally find use in hybridization embodiments, wherein the length of the contiguous complementary region may be varied, such as between about 15 and about 100 nucleotides, but larger contiguous complementarity stretches may be used, according to the length complementary sequences one wishes to detect.

The use of a hybridization probe of about 15-25 nucleotides in length allows the formation of a duplex molecule that is both stable and selective. Molecules having contiguous complementary sequences over stretches greater than 15 bases in length are generally preferred, though, in order to increase stability and selectivity of the hybrid, and thereby improve the quality and degree of specific hybrid molecules obtained. One will generally prefer to design nucleic acid molecules having gene-complementary stretches of 15 to 25 contiguous nucleotides, or even longer where desired.

Hybridization probes may be selected from any portion of any of the sequences disclosed herein. All that is required is to review the sequences set forth herein, or to any continuous portion of the sequences, from about 15-25 nucleotides in length up to and including the full length sequence, that one wishes to utilize as a probe or primer. The choice of probe and primer sequences may be governed by various factors. For example, one may wish to employ primers from towards the termini of the total sequence.

Small polynucleotide segments or fragments may be readily prepared by, for example, directly synthesizing the fragment by chemical means, as is commonly practiced using an automated oligonucleotide synthesizer. Also, fragments may be obtained by application of nucleic acid reproduction technology, such as the PCR™ technology of U. S. Patent 4,683,202 (incorporated herein by reference), by introducing selected sequences into recombinant vectors for recombinant production, and by other recombinant DNA techniques generally known to those of skill in the art of molecular biology.

The nucleotide sequences of the invention may be used for their ability to selectively form duplex molecules with complementary stretches of the entire gene or gene fragments of interest. Depending on the application envisioned, one will typically desire to employ varying conditions of hybridization to achieve varying degrees of selectivity of probe towards target sequence. For applications requiring high selectivity, one will typically desire to employ relatively stringent conditions to form the hybrids, *e*.*g*., one will select relatively low salt and/or high temperature conditions, such as provided by a salt concentration of from about 0.02 M to about 0.15 M salt at temperatures of from about 50°C to about 70°C. Such selective conditions tolerate little, if any, mismatch between the probe and the template or target strand, and would be particularly suitable for isolating related sequences.

Of course, for some applications, for example, where one desires to prepare mutants employing a mutant primer strand hybridized to an underlying template, less stringent (reduced stringency) hybridization conditions will typically be needed in order to allow formation of the heteroduplex. In these circumstances, one may desire to employ salt conditions such as those of from about 0.15 M to about 0.9 M salt, at temperatures ranging from about 20°C to about 55°C. Cross-hybridizing species can thereby be readily identified as positively hybridizing signals with respect to control hybridizations. In any case, it is generally appreciated that conditions can be rendered more stringent by the addition of increasing amounts of formamide, which serves to destabilize the hybrid duplex in the same manner as increased temperature. Thus, hybridization conditions can be readily manipulated, and thus will generally be a method of choice depending on the desired results.

According to another embodiment of the present invention, polynucleotide compositions comprising antisense oligonucleotides are provided. Antisense oligonucleotides have been demonstrated to be effective and targeted inhibitors of protein synthesis, and, consequently, provide a therapeutic approach by which a disease can be treated by inhibiting the synthesis of proteins that contribute to the disease. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (U. S. Patent 5,739,119 and U. S. Patent 5,759,829). Further, examples of antisense inhibition have been demonstrated with the nuclear protein cyclin, the multiple drug resistance gene (MDG1), ICAM-1, E-selectin, STK-1, striatal GABA_{A} receptor and human EGF (Jaskulski et al., Science. 1988 Jun 10;240(4858):1544-6; Vasanthakumar and Ahmed, Cancer Commun. 1989;1(4):225-32; Peris et al., Brain Res Mol Brain Res. 1998 Jun 15;57(2):310-20; U. S. Patent 5,801,154; U.S. Patent 5,789,573; U. S. Patent 5,718,709 and U.S. Patent 5,610,288). Antisense constructs have also been described that inhibit and can be used to treat a variety of abnormal cellular proliferations, e.g. cancer (U. S. Patent 5,747,470; U. S. Patent 5,591,317 and U. S. Patent 5,783,683).

Therefore, in certain embodiments, the present invention provides oligonucleotide sequences that comprise all, or a portion of, any sequence that is capable of specifically binding to polynucleotide sequence described herein, or a complement thereof. In one embodiment, the antisense oligonucleotides comprise DNA or derivatives thereof. In another embodiment, the oligonucleotides comprise RNA or derivatives thereof. In a third embodiment, the oligonucleotides are modified DNAs comprising a phosphorothioated modified backbone. In a fourth embodiment, the oligonucleotide sequences comprise peptide nucleic acids or derivatives thereof. In each case, preferred compositions comprise a sequence region that is complementary, and more preferably substantially-complementary, and even more preferably, completely complementary to one or more portions of polynucleotides disclosed herein. Selection of antisense compositions specific for a given gene sequence is based upon analysis of the chosen target sequence and determination of secondary structure, Tₘ, binding energy, and relative stability. Antisense compositions may be selected based upon their relative inability to form dimers, hairpins, or other secondary structures that would reduce or prohibit specific binding to the target mRNA in a host cell. Highly preferred target regions of the mRNA, are those which are at or near the AUG translation initiation codon, and those sequences which are substantially complementary to 5' regions of the mRNA. These secondary structure analyses and target site selection considerations can be performed, for example, using v.4 of the OLIGO primer analysis software and/or the BLASTN 2.0.5 algorithm software (Altschul et al., Nucleic Acids Res. 1997, 25(17):3389-402).

The use of an antisense delivery method employing a short peptide vector, termed MPG (27 residues), is also contemplated. The MPG peptide contains a hydrophobic domain derived from the fusion sequence of HIV gp41 and a hydrophilic domain from the nuclear localization sequence of SV40 T-antigen (Morris et al., Nucleic Acids Res. 1997 Jul 15;25(14):2730-6). It has been demonstrated that several molecules of the MPG peptide coat the antisense oligonucleotides and can be delivered into cultured mammalian cells in less than 1 hour with relatively high efficiency (90%). Further, the interaction with MPG strongly increases both the stability of the oligonucleotide to nuclease and the ability to cross the plasma membrane.

According to another embodiment of the invention, the polynucleotide compositions described herein are used in the design and preparation of ribozyme molecules for inhibiting expression of the tumor polypeptides and proteins of the present invention in tumor cells. Ribozymes are RNA-protein complexes that cleave nucleic acids in a site-specific fashion. Ribozymes have specific catalytic domains that possess endonuclease activity (Kim and Cech, Proc Natl Acad Sci U S A. 1987 Dec;84(24):8788-92; Forster and Symons, Cell. 1987 Apr 24;49(2):211-20). For example, a large number of ribozymes accelerate phosphoester transfer reactions with a high degree of specificity, often cleaving only one of several phosphoesters in an oligonucleotide substrate (Cech et al., Cell. 1981 Dec;27(3 Pt 2):487-96; Michel and Westhof, J Mol Biol. 1990 Dec 5;216(3):585-610; Reinhold-Hurek and Shub, Nature. 1992 May 14;357(6374):173-6). This specificity has been attributed to the requirement that the substrate bind via specific base-pairing interactions to the internal guide sequence ("IGS") of the ribozyme prior to chemical reaction.

Six basic varieties of naturally-occurring enzymatic RNAs are known presently. Each can catalyze the hydrolysis of RNA phosphodiester bonds *in trans* (and thus can cleave other RNA molecules) under physiological conditions. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nature of a ribozyme is advantageous over many technologies, such as antisense technology (where a nucleic acid molecule simply binds to a nucleic acid target to block its translation) since the concentration of ribozyme necessary to affect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding to the target RNA, but also on the mechanism of target RNA cleavage. Single mismatches, or base-substitutions, near the site of cleavage can completely eliminate catalytic activity of a ribozyme. Similar mismatches in antisense molecules do not prevent their action (Woolf et al., Proc Natl Acad Sci U S A. 1992 Aug 15;89(16):7305-9). Thus, the specificity of action of a ribozyme is greater than that of an antisense oligonucleotide binding the same RNA site.

The enzymatic nucleic acid molecule may be formed in a hammerhead, hairpin, a hepatitis δ virus, group I intron or RNaseP RNA (in association with an RNA guide sequence) or Neurospora VS RNA motif. Examples of hammerhead motifs are described by Rossi et al. Nucleic Acids Res. 1992 Sep 11;20(17):4559-65. Examples of hairpin motifs are described by Hampel *et al*. (Eur. Pat. Appl. Publ. No. EP 0360257), Hampel and Tritz, Biochemistry 1989 Jun 13;28(12):4929-33; Hampel et al., Nucleic Acids Res. 1990 Jan 25;18(2):299-304 and U. S. Patent 5,631,359. An example of the hepatitis δ virus motif is described by Perrotta and Been, Biochemistry. 1992 Dec 1;31(47):11843-52; an example of the RNaseP motif is described by Guerrier-Takada et al., Cell. 1983 Dec;35(3 Pt 2):849-57; Neurospora VS RNA ribozyme motif is described by Collins (Saville and Collins, Cell. 1990 May 18;61(4):685-96; Saville and Collins, Proc Natl Acad Sci U S A. 1991 Oct 1;88(19):8826-30; Collins and Olive, Biochemistry. 1993 Mar 23;32(11):2795-9); and an example of the Group I intron is described in (U. S. Patent 4,987,071). All that is important in an enzymatic nucleic acid molecule of this invention is that it has a specific substrate binding site which is complementary to one or more of the target gene RNA regions, and that it have nucleotide sequences within or surrounding that substrate binding site which impart an RNA cleaving activity to the molecule. Thus the ribozyme constructs need not be limited to specific motifs mentioned herein.

Ribozymes may be designed as described in Int. Pat. Appl. Publ. No. WO 93/23569 and Int. Pat. Appl. Publ. No. WO 94/02595, each specifically incorporated herein by reference) and synthesized to be tested *in vitro* and *in vivo,* as described. Such ribozymes can also be optimized for delivery. While specific examples are provided, those in the art will recognize that equivalent RNA targets in other species can be utilized when necessary.

Ribozyme activity can be optimized by altering the length of the ribozyme binding arms, or chemically synthesizing ribozymes with modifications that prevent their degradation by serum ribonucleases (see e.g., Int. Pat. Appl. Publ. No. WO 92/07065; Int. Pat. Appl. Publ. No. WO 93/15187; Int. Pat. Appl. Publ. No. WO 91/03162; Eur. Pat. Appl. Publ. No. 92110298.4; U. S. Patent 5,334,711; and Int. Pat. Appl. Publ. No. WO 94/13688, which describe various chemical modifications that can be made to the sugar moieties of enzymatic RNA molecules), modifications which enhance their efficacy in cells, and removal of stem II bases to shorten RNA synthesis times and reduce chemical requirements.

Sullivan *et al*. (Int. Pat. Appl. Publ. No. WO 94/02595) describes the general methods for delivery of enzymatic RNA molecules. Ribozymes may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, ribozymes may be directly delivered *ex* vivo to cells or tissues with or without the aforementioned vehicles. Alternatively, the RNA/vehicle combination may be locally delivered by direct inhalation, by direct injection or by use of a catheter, infusion pump or stint. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of ribozyme delivery and administration are provided in Int. Pat. Appl. Publ. No. WO 94/02595 and Int. Pat. Appl. Publ. No. WO 93/23569, each specifically incorporated herein by reference.

Another means of accumulating high concentrations of a ribozyme(s) within cells is to incorporate the ribozyme-encoding sequences into a DNA expression vector. Transcription of the ribozyme sequences are driven from a promoter for eukaryotic RNA polymerase I (pol I), RNA polymerase II (pol II), or RNA polymerase III (pol III). Transcripts from pol II or pol III promoters will be expressed at high levels in all cells; the levels of a given pol II promoter in a given cell type will depend on the nature of the gene regulatory sequences (enhancers, silencers, *etc.*) present nearby. Prokaryotic RNA polymerase promoters may also be used, providing that the prokaryotic RNA polymerase enzyme is expressed in the appropriate cells. Ribozymes expressed from such promoters have been shown to function in mammalian cells. Such transcription units can be incorporated into a variety of vectors for introduction into mammalian cells, including but not restricted to, plasmid DNA vectors, viral DNA vectors (such as adenovirus or adeno-associated vectors), or viral RNA vectors (such as retroviral, semliki forest virus, sindbis virus vectors).

In another embodiment of the invention, peptide nucleic acids (PNAs) compositions are provided. PNA is a DNA mimic in which the nucleobases are attached to a pseudopeptide backbone (Good and Nielsen, Antisense Nucleic Acid Drug Dev. 1997 7(4) 431-37). PNA is able to be utilized in a number methods that traditionally have used RNA or DNA. Often PNA sequences perform better in techniques than the corresponding RNA or DNA sequences and have utilities that are not inherent to RNA or DNA. A review of PNA including methods of making, characteristics of, and methods of using, is provided by Corey (Trends Biotechnol 1997 Jun;15(6):224-9). As such, in certain embodiments, one may prepare PNA sequences that are complementary to one or more portions of the ACE mRNA sequence, and such PNA compositions may be used to regulate, alter, decrease, or reduce the translation of ACE-specific mRNA, and thereby alter the level of ACE activity in a host cell to which such PNA compositions have been administered.

PNAs have 2-aminoethyl-glycine linkages replacing the normal phosphodiester backbone of DNA (Nielsen et al., Science 1991 Dec 6;254(5037):1497-500; Hanvey et al., Science. 1992 Nov 27;258(5087):1481-5; Hyrup and Nielsen, Bioorg Med Chem. 1996 Jan;4(1):5-23). This chemistry has three important consequences: firstly, in contrast to DNA or phosphorothioate oligonucleotides, PNAs are neutral molecules; secondly, PNAs are achiral, which avoids the need to develop a stereoselective synthesis; and thirdly, PNA synthesis uses standard Boc or Fmoc protocols for solid-phase peptide synthesis, although other methods, including a modified Merrifield method, have been used.

PNA monomers or ready-made oligomers are commercially available from PerSeptive Biosystems (Framingham, MA). PNA syntheses by either Boc or Fmoc protocols are straightforward using manual or automated protocols (Norton et al., Bioorg Med Chem. 1995 Apr;3(4):437-45). The manual protocol lends itself to the production of chemically modified PNAs or the simultaneous synthesis of families of closely related PNAs.

As with peptide synthesis, the success of a particular PNA synthesis will depend on the properties of the chosen sequence. For example, while in theory PNAs can incorporate any combination of nucleotide bases, the presence of adjacent purines can lead to deletions of one or more residues in the product. In expectation of this difficulty, it is suggested that, in producing PNAs with adjacent purines, one should repeat the coupling of residues likely to be added inefficiently. This should be followed by the purification of PNAs by reverse-phase high-pressure liquid chromatography, providing yields and purity of product similar to those observed during the synthesis of peptides.

Modifications of PNAs for a given application may be accomplished by coupling amino acids during solid-phase synthesis or by attaching compounds that contain a carboxylic acid group to the exposed N-terminal amine. Alternatively, PNAs can be modified after synthesis by coupling to an introduced lysine or cysteine. The ease with which PNAs can be modified facilitates optimization for better solubility or for specific functional requirements. Once synthesized, the identity of PNAs and their derivatives can be confirmed by mass spectrometry. Several studies have made and utilized modifications of PNAs (for example, Norton et al., Bioorg Med Chem. 1995 Apr;3(4):437-45; Petersen et al., J Pept Sci. 1995 May-Jun;1(3):175-83; Orum et al., Biotechniques. 1995 Sep;19(3):472-80; Footer et al., Biochemistry. 1996 Aug 20;35(33):10673-9; Griffith et al., Nucleic Acids Res. 1995 Aug 11;23(15):3003-8; Pardridge et al., Proc Natl Acad Sci U S A. 1995 Jun 6;92(12):5592-6; Boffa et al., Proc Natl Acad Sci U S A. 1995 Mar 14;92(6):1901-5; Gambacorti-Passerini et al., Blood. 1996 Aug 15;88(4):1411-7; Armitage et al., Proc Natl Acad Sci U S A. 1997 Nov 11;94(23):12320-5; Seeger et al., Biotechniques. 1997 Sep;23(3):512-7). U.S. Patent No. 5,700,922 discusses PNA-DNA-PNA chimeric molecules and their uses in diagnostics, modulating protein in organisms, and treatment of conditions susceptible to therapeutics.

Methods of characterizing the antisense binding properties of PNAs are discussed in Rose (Anal Chem. 1993 Dec 15;65(24):3545-9) and Jensen et al. (Biochemistry. 1997 Apr 22;36(16):5072-7). Rose uses capillary gel electrophoresis to determine binding of PNAs to their complementary oligonucleotide, measuring the relative binding kinetics and stoichiometry. Similar types of measurements were made by Jensen *et al*. using BIAcore^{™} technology.

Other applications of PNAs that have been described and will be apparent to the skilled artisan include use in DNA strand invasion, antisense inhibition, mutational analysis, enhancers of transcription, nucleic acid purification, isolation of transcriptionally active genes, blocking of transcription factor binding, genome cleavage, biosensors, *in situ* hybridization, and the like.

### Polynucleotide Identification, Characterization and Expression

Polynucleotides compositions of the present invention may be identified, prepared and/or manipulated using any of a variety of well established techniques (see generally, Sambrook et al., Molecular Cloning: A Laboratory Manual, Cold Spring Harbor Laboratories, Cold Spring Harbor, NY, 1989, and other like references). For example, a polynucleotide may be identified, as described in more detail below, by screening a microarray of cDNAs for tumor-associated expression (*i*.*e*., expression that is at least two fold greater in a tumor than in normal tissue, as determined using a representative assay provided herein). Such screens may be performed, for example, using the microarray technology of Affymetrix, Inc. (Santa Clara, CA) according to the manufacturer's instructions (and essentially as described by Schena et al., Proc. Natl. Acad. Sci. USA 93:10614-10619, 1996 and Heller et al., Proc. Natl. Acad. Sci. USA 94:2150-2155, 1997). Alternatively, polynucleotides may be amplified from cDNA prepared from cells expressing the proteins described herein, such as tumor cells.

Many template dependent processes are available to amplify a target sequences of interest present in a sample. One of the best known amplification methods is the polymerase chain reaction (PCR^{™}) which is described in detail in U.S. Patent Nos. 4,683,195, 4,683,202 and 4,800,159. Briefly, in PCR^{™}, two primer sequences are prepared which are complementary to regions on opposite complementary strands of the target sequence. An excess of deoxynucleoside triphosphates is added to a reaction mixture along with a DNA polymerase (*e.g., Taq* polymerase). If the target sequence is present in a sample, the primers will bind to the target and the polymerase will cause the primers to be extended along the target sequence by adding on nucleotides. By raising and lowering the temperature of the reaction mixture, the extended primers will dissociate from the target to form reaction products, excess primers will bind to the target and to the reaction product and the process is repeated. Preferably reverse transcription and PCR^{™} amplification procedure may be performed in order to quantify the amount of mRNA amplified. Polymerase chain reaction methodologies are well known in the art.

Any of a number of other template dependent processes, many of which are variations of the PCR ^{™} amplification technique, are readily known and available in the art. Illustratively, some such methods include the ligase chain reaction (referred to as LCR), described, for example, in Eur. Pat. Appl. Publ. No. 320,308 and U.S. Patent No. 4,883,750; Qbeta Replicase, described in PCT Intl. Pat. Appl. Publ. No. PCT/US87/00880; Strand Displacement Amplification (SDA) and Repair Chain Reaction (RCR). Still other amplification methods are described in Great Britain Pat. Appl. No. 2 202 328, and in PCT Intl. Pat. Appl. Publ. No. PCT/US89/01025. Other nucleic acid amplification procedures include transcription-based amplification systems (TAS) (PCT Intl. Pat. Appl. Publ. No. WO 88/10315), including nucleic acid sequence based amplification (NASBA) and 3SR. Eur. Pat. Appl. Publ. No. 329,822 describes a nucleic acid amplification process involving cyclically synthesizing single-stranded RNA ("ssRNA"), ssDNA, and double-stranded DNA (dsDNA). PCT Intl. Pat. Appl. Publ. No. WO 89/06700 describes a nucleic acid sequence amplification scheme based on the hybridization of a promoter/primer sequence to a target single-stranded DNA ("ssDNA") followed by transcription of many RNA copies of the sequence. Other amplification methods such as "RACE" (Frohman, 1990), and "one-sided PCR" (Ohara, 1989) are also well-known to those of skill in the art.

An amplified portion of a polynucleotide of the present invention may be used to isolate a full length gene from a suitable library (*e*.*g*., a tumor cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (*e*.*g*., by nick-translation or end-labeling with ³²P) using well known techniques. A bacterial or bacteriophage library is then generally screened by hybridizing filters containing denatured bacterial colonies (or lawns containing phage plaques) with the labeled probe (*see* Sambrook et al., Molecular Cloning: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences can then assembled into a single contiguous sequence. A full length cDNA molecule can be generated by ligating suitable fragments, using well known techniques.

Alternatively, amplification techniques, such as those described above, can be useful for obtaining a full length coding sequence from a partial cDNA sequence. One such amplification technique is inverse PCR (*see* Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Another such technique is known as "rapid amplification of cDNA ends" or RACE. This technique involves the use of an internal primer and an external primer, which hybridizes to a polyA region or vector sequence, to identify sequences that are 5' and 3' of a known sequence. Additional techniques include capture PCR (Lagerstrom et al., PCR Methods Applic. 1:111-19, 1991) and walking PCR (Parker et al., Nucl. Acids. Res. 19:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (*e*.*g*., NCBI BLAST searches), and such ESTs may be used to generate a contiguous full length sequence. Full length DNA sequences may also be obtained by analysis of genomic fragments.

In other embodiments of the invention, polynucleotide sequences or fragments thereof which encode polypeptides of the invention, or fusion proteins or functional equivalents thereof, may be used in recombinant DNA molecules to direct expression of a polypeptide in appropriate host cells. Due to the inherent degeneracy of the genetic code, other DNA sequences that encode substantially the same or a functionally equivalent amino acid sequence may be produced and these sequences may be used to clone and express a given polypeptide.

As will be understood by those of skill in the art, it may be advantageous in some instances to produce polypeptide-encoding nucleotide sequences possessing non-naturally occurring codons. For example, codons preferred by a particular prokaryotic or eukaryotic host can be selected to increase the rate of protein expression or to produce a recombinant RNA transcript having desirable properties, such as a half-life which is longer than that of a transcript generated from the naturally occurring sequence.

Moreover, the polynucleotide sequences of the present invention can be engineered using methods generally known in the art in order to alter polypeptide encoding sequences for a variety of reasons, including but not limited to, alterations which modify the cloning, processing, and/or expression of the gene product. For example, DNA shuffling by random fragmentation and PCR reassembly of gene fragments and synthetic oligonucleotides may be used to engineer the nucleotide sequences. In addition, site-directed mutagenesis may be used to insert new restriction sites, alter glycosylation patterns, change codon preference, produce splice variants, or introduce mutations, and so forth.

In another embodiment of the invention, natural, modified, or recombinant nucleic acid sequences may be ligated to a heterologous sequence to encode a fusion protein. For example, to screen peptide libraries for inhibitors of polypeptide activity, it may be useful to encode a chimeric protein that can be recognized by a commercially available antibody. A fusion protein may also be engineered to contain a cleavage site located between the polypeptide-encoding sequence and the heterologous protein sequence, so that the polypeptide may be cleaved and purified away from the heterologous moiety.

Sequences encoding a desired polypeptide may be synthesized, in whole or in part, using chemical methods well known in the art (see Caruthers, M. H. et al. (1980) Nucl. Acids Res. Symp. Ser. 215-223, Horn, T. et al. (1980) Nucl. Acids Res. Symp. Ser. 225-232). Alternatively, the protein itself may be produced using chemical methods to synthesize the amino acid sequence of a polypeptide, or a portion thereof. For example, peptide synthesis can be performed using various solid-phase techniques (Roberge, J. Y. et al. (1995) Science 269:202-204) and automated synthesis may be achieved, for example, using the ABI 431A Peptide Synthesizer (Perkin Elmer, Palo Alto, CA).

A newly synthesized peptide may be substantially purified by preparative high performance liquid chromatography (*e*.*g*., Creighton, T. (1983) Proteins, Structures and Molecular Principles, WH Freeman and Co., New York, N.Y.) or other comparable techniques available in the art. The composition of the synthetic peptides may be confirmed by amino acid analysis or sequencing (*e*.*g*., the Edman degradation procedure). Additionally, the amino acid sequence of a polypeptide, or any part thereof, may be altered during direct synthesis and/or combined using chemical methods with sequences from other proteins, or any part thereof, to produce a variant polypeptide.

In order to express a desired polypeptide, the nucleotide sequences encoding the polypeptide, or functional equivalents, may be inserted into appropriate expression vector, *i*.*e*., a vector which contains the necessary elements for the transcription and translation of the inserted coding sequence. Methods which are well known to those skilled in the art may be used to construct expression vectors containing sequences encoding a polypeptide of interest and appropriate transcriptional and translational control elements. These methods include *in vitro* recombinant DNA techniques, synthetic techniques, and *in vivo* genetic recombination. Such techniques are described, for example, in Sambrook, J. et al. (1989) Molecular Cloning, A Laboratory Manual, Cold Spring Harbor Press, Plainview, N.Y., and Ausubel, F. M. et al. (1989) Current Protocols in Molecular Biology, John Wiley & Sons, New York. N.Y.

A variety of expression vector/host systems may be utilized to contain and express polynucleotide sequences. These include, but are not limited to, microorganisms such as bacteria transformed with recombinant bacteriophage, plasmid, or cosmid DNA expression vectors; yeast transformer with yeast expression vectors; insect cell systems infected with virus expression vectors (*e*.*g*., baculovirus); plant cell systems transformed with virus expression vectors (*e*.*g*., cauliflower mosaic virus, CaMV; tobacco mosaic virus, TMV) or with bacterial expression vectors (*e*.*g*., Ti or pBR322 plasmids); or animal cell systems.

The "control elements" or "regulatory sequences" present in an expression vector are those non-translated regions of the vector--enhancers, promoters, 5' and 3' untranslated regions--which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the PBLUESCRIPT phagemid (Stratagene, La Jolla, Calif.) or PSPORT1 plasmid (Gibco BRL, Gaithersburg, MD) and the like may be used. In mammalian cell systems, promoters from mammalian genes or from mammalian viruses are generally preferred. If it is necessary to generate a cell line that contains multiple copies of the sequence encoding a polypeptide, vectors based on SV40 or EBV may be advantageously used with an appropriate selectable marker.

In bacterial systems, any of a number of expression vectors may be selected depending upon the use intended for the expressed polypeptide. For example, when large quantities are needed, for example for the induction of antibodies, vectors which direct high level expression of fusion proteins that are readily purified may be used. Such vectors include, but are not limited to, the multifunctional *E*. *coli* cloning and expression vectors such as BLUESCRIPT (Stratagene), in which the sequence encoding the polypeptide of interest may be ligated into the vector in frame with sequences for the amino-terminal Met and the subsequent 7 residues of .beta.-galactosidase so that a hybrid protein is produced; pIN vectors (Van Heeke, G. and S. M. Schuster (1989) J. Biol. Chem. 264:5503-5509); and the like. pGEX Vectors (Promega, Madison, Wis.) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor XA protease cleavage sites so that the cloned polypeptide of interest can be released from the GST moiety at will.

In the yeast, Saccharomyces cerevisiae, a number of vectors containing constitutive or inducible promoters such as alpha factor, alcohol oxidase, and PGH may be used. For reviews, see Ausubel et al. (supra) and Grant et al. (1987) Methods Enzymol. 153:516-544.

In cases where plant expression vectors are used, the expression of sequences encoding polypeptides may be driven by any of a number of promoters. For example, viral promoters such as the 35S and 19S promoters of CaMV may be used alone or in combination with the omega leader sequence from TMV (Takamatsu, N. (1987) EMBO J. 6:307-311. Alternatively, plant promoters such as the small subunit of RUBISCO or heat shock promoters may be used (Coruzzi, G. et al. (1984) EMBO J. 3:1671-1680; Broglie, R. et al. (1984) Science 224:838-843; and Winter, J. et al. (1991) Results Probl. Cell Differ. 17:85-105). These constructs can be introduced into plant cells by direct DNA transformation or pathogen-mediated transfection. Such techniques are described in a number of generally available reviews (see, for example, Hobbs, S. or Murry, L. E. in McGraw Hill Yearbook of Science and Technology (1992) McGraw Hill, New York, N.Y.; pp. 191-196).

An insect system may also be used to express a polypeptide of interest. For example, in one such system, Autographa californica nuclear polyhedrosis virus (AcNPV) is used as a vector to express foreign genes in Spodoptera frugiperda cells or in Trichoplusia larvae. The sequences encoding the polypeptide may be cloned into a non-essential region of the virus, such as the polyhedrin gene, and placed under control of the polyhedrin promoter. Successful insertion of the polypeptide-encoding sequence will render the polyhedrin gene inactive and produce recombinant virus lacking coat protein. The recombinant viruses may then be used to infect, for example, S. frugiperda cells or Trichoplusia larvae in which the polypeptide of interest may be expressed (Engelhard, E. K. et al. (1994) Proc. Natl. Acad. Sci. 91 :3224-3227).

In mammalian host cells, a number of viral-based expression systems are generally available. For example, in cases where an adenovirus is used as an expression vector, sequences encoding a polypeptide of interest may be ligated into an adenovirus transcription/translation complex consisting of the late promoter and tripartite leader sequence. Insertion in a non-essential E1 or E3 region of the viral genome may be used to obtain a viable virus which is capable of expressing the polypeptide in infected host cells (Logan, J. and Shenk, T. (1984) Proc. Natl. Acad. Sci. 81:3655-3659). In addition, transcription enhancers, such as the Rous sarcoma virus (RSV) enhancer, may be used to increase expression in mammalian host cells.

Specific initiation signals may also be used to achieve more efficient translation of sequences encoding a polypeptide of interest. Such signals include the ATG initiation codon and adjacent sequences. In cases where sequences encoding the polypeptide, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) Results Probl. Cell Differ. 20:125-162).

In addition, a host cell strain may be chosen for its ability to modulate the expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding and/or function. Different host cells such as CHO, COS, HeLa, MDCK, HEK293, and WI38, which have specific cellular machinery and characteristic mechanisms for such post-translational activities, may be chosen to ensure the correct modification and processing of the foreign protein.

For long-term, high-yield production of recombinant proteins, stable expression is generally preferred. For example, cell lines which stably express a polynucleotide of interest may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformer cells may be proliferated using tissue culture techniques appropriate to the cell type.

Any number of selection systems may be used to recover transformed cell lines. These include, but are not limited to, the herpes simplex virus thymidine kinase (Wigler, M. et al. (1977) Cell 11:223-32) and adenine phosphoribosyltransferase (Lowy, I. et al. (1990) Cell 22:817-23) genes which can be employed in tk.sup.- or aprt.sup.- cells, respectively. Also, antimetabolite, antibiotic or herbicide resistance can be used as the basis for selection; for example, dhfr which confers resistance to methotrexate (Wigler, M. et al. (1980) Proc. Natl. Acad. Sci. 77:3567-70); npt, which confers resistance to the aminoglycosides, neomycin and G-418 (Colbere-Garapin, F. et al (1981) J. Mol. Biol. 150:1-14); and als or pat, which confer resistance to chlorsulfuron and phosphinotricin acetyltransferase, respectively (Murry, *supra*). Additional selectable genes have been described, for example, trpB, which allows cells to utilize indole in place of tryptophan, or hisD, which allows cells to utilize histinol in place of histidine (Hartman, S. C. and R. C. Mulligan (1988) Proc. Natl. Acad. Sci. 85:8047-51). The use of visible markers has gained popularity with such markers as anthocyanins, beta-glucuronidase and its substrate GUS, and luciferase and its substrate luciferin, being widely used not only to identify transformants, but also to quantify the amount of transient or stable protein expression attributable to a specific vector system (Rhodes, C. A. et al. (1995) Methods Mol. Biol. 55:121-131).

Although the presence/absence of marker gene expression suggests that the gene of interest is also present, its presence and expression may need to be confirmed. For example, if the sequence encoding a polypeptide is inserted within a marker gene sequence, recombinant cells containing sequences can be identified by the absence of marker gene function. Alternatively, a marker gene can be placed in tandem with a polypeptide-encoding sequence under the control of a single promoter. Expression of the marker gene in response to induction or selection usually indicates expression of the tandem gene as well.

Alternatively, host cells that contain and express a desired polynucleotide sequence may be identified by a variety of procedures known to those of skill in the art. These procedures include, but are not limited to, DNA-DNA or DNA-RNA hybridizations and protein bioassay or immunoassay techniques which include, for example, membrane, solution, or chip based technologies for the detection and/or quantification of nucleic acid or protein.

A variety of protocols for detecting and measuring the expression of polynucleotide-encoded products, using either polyclonal or monoclonal antibodies specific for the product are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on a given polypeptide may be preferred for some applications, but a competitive binding assay may also be employed. These and other assays are described, among other places, in Hampton, R. et al. (1990; Serological Methods, a Laboratory Manual, APS Press, St Paul. Minn.) and Maddox, D. E. et al. (1983; J. Exp. Med. 158:1211-1216).

A wide variety of labels and conjugation techniques are known by those skilled in the art and may be used in various nucleic acid and amino acid assays. Means for producing labeled hybridization or PCR probes for detecting sequences related to polynucleotides include oligolabeling, nick translation, end-labeling or PCR amplification using a labeled nucleotide. Alternatively, the sequences, or any portions thereof may be cloned into a vector for the production of an mRNA probe. Such vectors are known in the art, are commercially available, and may be used to synthesize RNA probes in vitro by addition of an appropriate RNA polymerase such as T7, T3, or SP6 and labeled nucleotides. These procedures may be conducted using a variety of commercially available kits. Suitable reporter molecules or labels, which may be used include radionuclides, enzymes, fluorescent, chemiluminescent, or chromogenic agents as well as substrates, cofactors, inhibitors, magnetic particles, and the like.

Host cells transformed with a polynucleotide sequence of interest may be cultured under conditions suitable for the expression and recovery of the protein from cell culture. The protein produced by a recombinant cell may be secreted or contained intracellularly depending on the sequence and/or the vector used. As will be understood by those of skill in the art, expression vectors containing polynucleotides of the invention may be designed to contain signal sequences which direct secretion of the encoded polypeptide through a prokaryotic or eukaryotic cell membrane. Other recombinant constructions may be used to join sequences encoding a polypeptide of interest to nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). The inclusion of cleavable linker sequences such as those specific for Factor XA or enterokinase (Invitrogen. San Diego, Calif.) between the purification domain and the encoded polypeptide may be used to facilitate purification. One such expression vector provides for expression of a fusion protein containing a polypeptide of interest and a nucleic acid encoding 6 histidine residues preceding a thioredoxin or an enterokinase cleavage site. The histidine residues facilitate purification on IMIAC (immobilized metal ion affinity chromatography) as described in Porath, J. et al. (1992, Prot. Exp. Purif. 3:263-281) while the enterokinase cleavage site provides a means for purifying the desired polypeptide from the fusion protein. A discussion of vectors which contain fusion proteins is provided in Kroll, D. J. et al. (1993; DNA Cell Biol. 12:441-453).

In addition to recombinant production methods, polypeptides of the invention, and fragments thereof, may be produced by direct peptide synthesis using solid-phase techniques (Merrifield J. (1963) J. Am. Chem. Soc. 85:2149-2154). Protein synthesis may be performed using manual techniques or by automation. Automated synthesis may be achieved, for example, using Applied Biosystems 431A Peptide Synthesizer (Perkin Elmer). Alternatively, various fragments may be chemically synthesized separately and combined using chemical methods to produce the full length molecule.

### Antibody Compositions, Fragments Thereof and Other Binding Agents

According to another aspect, the present invention further provides binding agents, such as antibodies and antigen-binding fragments thereof, that exhibit immunological binding to a tumor polypeptide disclosed herein, or to a portion, variant or derivative thereof. An antibody, or antigen-binding fragment thereof, is said to "specifically bind," "immunogically bind," and/or is "immunologically reactive" to a polypeptide of the invention if it reacts at a detectable level (within, for example, an ELISA assay) with the polypeptide, and does not react detectably with unrelated polypeptides under similar conditions.

Immunological binding, as used in this context, generally refers to the non-covalent interactions of the type which occur between an immunoglobulin molecule and an antigen for which the immunoglobulin is specific. The strength, or affinity of immunological binding interactions can be expressed in terms of the dissociation constant (K_{d}) of the interaction, wherein a smaller K_{d} represents a greater affinity. Immunological binding properties of selected polypeptides can be quantified using methods well known in the art. One such method entails measuring the rates of antigen-binding site/antigen complex formation and dissociation, wherein those rates depend on the concentrations of the complex partners, the affinity of the interaction, and on geometric parameters that equally influence the rate in both directions. Thus, both the "on rate constant" (Kₒₙ) and the "off rate constant" (K_{off}) can be determined by calculation of the concentrations and the actual rates of association and dissociation. The ratio of K_{off} /Kₒₙ enables cancellation of all parameters not related to affinity, and is thus equal to the dissociation constant K_{d}. See, generally, Davies et al. (1990) Annual Rev. Biochem. 59:439-473.

An "antigen-binding site," or "binding portion" of an antibody refers to the part of the immunoglobulin molecule that participates in antigen binding. The antigen binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FRs". Thus the term "FR" refers to amino acid sequences which are naturally found between and adjacent to hypervariable regions in immunoglobulins. In an antibody molecule, the three hypervariable regions of a light chain and the three hypervariable regions of a heavy chain are disposed relative to each other in three dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs."

Binding agents may be further capable of differentiating between patients with and without a cancer, such as lung cancer, using the representative assays provided herein. For example, antibodies or other binding agents that bind to a tumor protein will preferably generate a signal indicating the presence of a cancer in at least about 20% of patients with the disease, more preferably at least about 30% of patients. Alternatively, or in addition, the antibody will generate a negative signal indicating the absence of the disease in at least about 90% of individuals without the cancer. To determine whether a binding agent satisfies this requirement, biological samples (*e*.*g*., blood, sera, sputum, urine and/or tumor biopsies) from patients with and without a cancer (as determined using standard clinical tests) may be assayed as described herein for the presence of polypeptides that bind to the binding agent. Preferably, a statistically significant number of samples with and without the disease will be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See*, *e*.*g*., Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (*e*.*g*., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i*.*e*., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

A number of therapeutically useful molecules are known in the art which comprise antigen-binding sites that are capable of exhibiting immunological binding properties of an antibody molecule. The proteolytic enzyme papain preferentially cleaves IgG molecules to yield several fragments, two of which (the "F(ab)" fragments) each comprise a covalent heterodimer that includes an intact antigen-binding site. The enzyme pepsin is able to cleave IgG molecules to provide several fragments, including the "F(ab')₂ " fragment which comprises both antigen-binding sites. An "Fv" fragment can be produced by preferential proteolytic cleavage of an IgM, and on rare occasions IgG or IgA immunoglobulin molecule. Fv fragments are, however, more commonly derived using recombinant techniques known in the art. The Fv fragment includes a non-covalent V_{H}::V_{L} heterodimer including an antigen-binding site which retains much of the antigen recognition and binding capabilities of the native antibody molecule. Inbar et al. (1972) Proc. Nat. Acad. Sci. USA 69:2659-2662; Hochman et al. (1976) Biochem 15:2706-2710; and Ehrlich et al. (1980) Biochem 19:4091-4096.

A single chain Fv ("sFv") polypeptide is a covalently linked V_{H}::V_{L} heterodimer which is expressed from a gene fusion including V_{H}- and V_{L}-encoding genes linked by a peptide-encoding linker. Huston et al. (1988) Proc. Nat. Acad. Sci. USA 85(16):5879-5883. A number of methods have been described to discern chemical structures for converting the naturally aggregated--but chemically separated--light and heavy polypeptide chains from an antibody V region into an sFv molecule which will fold into a three dimensional structure substantially similar to the structure of an antigen-binding site. See, *e*.*g*., U.S. Pat. Nos. 5,091,513 and 5,132,405, to Huston et al.; and U.S. Pat. No. 4,946,778, to Ladner et al.

Each of the above-described molecules includes a heavy chain and a light chain CDR set, respectively interposed between a heavy chain and a light chain FR set which provide support to the CDRS and define the spatial relationship of the CDRs relative to each other. As used herein, the term "CDR set" refers to the three hypervariable regions of a heavy or light chain V region. Proceeding from the N-terminus of a heavy or light chain, these regions are denoted as "CDR1," "CDR2," and "CDR3" respectively. An antigen-binding site, therefore, includes six CDRs, comprising the CDR set from each of a heavy and a light chain V region. A polypeptide comprising a single CDR, (*e*.*g*., a CDR1, CDR2 or CDR3) is referred to herein as a "molecular recognition unit." Crystallographic analysis of a number of antigen-antibody complexes has demonstrated that the amino acid residues of CDRs form extensive contact with bound antigen, wherein the most extensive antigen contact is with the heavy chain CDR3. Thus, the molecular recognition units are primarily responsible for the specificity of an antigen-binding site.

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRS. Within FRs, certain amino residues and certain structural features are very highly conserved. In this regard, all V region sequences contain an internal disulfide loop of around 90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structures--regardless of the precise CDR amino acid sequence. Further, certain FR residues are known to participate in non-covalent interdomain contacts which stabilize the interaction of the antibody heavy and light chains.

A number of "humanized" antibody molecules comprising an antigen-binding site derived from a non-human immunoglobulin have been described, including chimeric antibodies having rodent V regions and their associated CDRs fused to human constant domains (Winter et al. (1991) Nature 349:293-299; Lobuglio et al. (1989) Proc. Nat. Acad. Sci. USA 86:4220-4224; Shaw et al. (1987) J Immunol. 138:4534-4538; and Brown et al. (1987) Cancer Res. 47:3577-3583), rodent CDRs grafted into a human supporting FR prior to fusion with an appropriate human antibody constant domain (Riechmann et al. (1988) Nature 332:323-327; Verhoeyen et al. (1988) Science 239:1534-1536; and Jones et al. (1986) Nature 321:522-525), and rodent CDRs supported by recombinantly veneered rodent FRs (European Patent Publication No. 519,596, published Dec. 23, 1992). These "humanized" molecules are designed to minimize unwanted immunological response toward rodent antihuman antibody molecules which limits the duration and effectiveness of therapeutic applications of those moieties in human recipients.

As used herein, the terms "veneered FRs" and "recombinantly veneered FRs" refer to the selective replacement of FR residues from, e.g., a rodent heavy or light chain V region, with human FR residues in order to provide a xenogeneic molecule comprising an antigen-binding site which retains substantially all of the native FR polypeptide folding structure. Veneering techniques are based on the understanding that the ligand binding characteristics of an antigen-binding site are determined primarily by the structure and relative disposition of the heavy and light chain CDR sets within the antigen-binding surface. Davies et al. (1990) Ann. Rev. Biochem. 59:439-473. Thus, antigen binding specificity can be preserved in a humanized antibody only wherein the CDR structures, their interaction with each other, and their interaction with the rest of the V region domains are carefully maintained. By using veneering techniques, exterior (e.g., solvent-accessible) FR residues which are readily encountered by the immune system are selectively replaced with human residues to provide a hybrid molecule that comprises either a weakly immunogenic, or substantially non-immunogenic veneered surface.

The process of veneering makes use of the available sequence data for human antibody variable domains compiled by Kabat et al., in Sequences of Proteins of Immunological Interest, 4th ed., (U.S. Dept. of Health and Human Services, U.S. Government Printing Office, 1987), updates to the Kabat database, and other accessible U.S. and foreign databases (both nucleic acid and protein). Solvent accessibilities of V region amino acids can be deduced from the known three-dimensional structure for human and murine antibody fragments. There are two general steps in veneering a murine antigen-binding site. Initially, the FRs of the variable domains of an antibody molecule of interest are compared with corresponding FR sequences of human variable domains obtained from the above-identified sources. The most homologous human V regions are then compared residue by residue to corresponding murine amino acids. The residues in the murine FR which differ from the human counterpart are replaced by the residues present in the human moiety using recombinant techniques well known in the art. Residue switching is only carried out with moieties which are at least partially exposed (solvent accessible), and care is exercised in the replacement of amino acid residues which may have a significant effect on the tertiary structure of V region domains, such as proline, glycine and charged amino acids.

In this manner, the resultant "veneered" murine antigen-binding sites are thus designed to retain the murine CDR residues, the residues substantially adjacent to the CDRs, the residues identified as buried or mostly buried (solvent inaccessible), the residues believed to participate in non-covalent (e.g., electrostatic and hydrophobic) contacts between heavy and light chain domains, and the residues from conserved structural regions of the FRs which are believed to influence the "canonical" tertiary structures of the CDR loops. These design criteria are then used to prepare recombinant nucleotide sequences which combine the CDRs of both the heavy and light chain of a murine antigen-binding site into human-appearing FRs that can be used to transfect mammalian cells for the expression of recombinant human antibodies which exhibit the antigen specificity of the murine antibody molecule.

In another embodiment of the invention, monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (e.g., covalently bonded) to a suitable monoclonal antibody either directly or indirectly (e.g., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (e.g., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e*.*g*., U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e*.*g*., U.S. Patent No. 4,489,710, to Spitler), by irradiation of a photolabile bond (*e*.*g*., U.S. Patent No. 4,625,014, to Senter et al.), by hydrolysis of derivatized amino acid side chains (*e*.*g*., U.S. Patent No. 4,638,045, to Kohn et al.), by serum complement-mediated hydrolysis (*e*.*g*., U.S. Patent No. 4,671,958, to Rodwell et al.), and acid-catalyzed hydrolysis (*e*.*g*., U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers that provide multiple sites for attachment can be used. Alternatively, a carrier can be used.

A carrier may bear the agents in a variety of ways, including covalent bonding either directly or via a linker group. Suitable carriers include proteins such as albumins (*e*.*g*., U.S. Patent No. 4,507,234, to Kato et al.), peptides and polysaccharides such as aminodextran (e.g., U.S. Patent No. 4,699,784, to Shih et al.). A carrier may also bear an agent by noncovalent bonding or by encapsulation, such as within a liposome vesicle (*e*.*g*., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

### T Cell Compositions

The present invention, in another aspect, provides T cells specific for a tumor polypeptide disclosed herein, or for a variant or derivative thereof. Such cells may generally be *prepared in vitro* or *ex vivo*, using standard procedures. For example, T cells may be isolated from bone marrow, peripheral blood, or a fraction of bone marrow or peripheral blood of a patient, using a commercially available cell separation system, such as the Isolex^{™} System, available from Nexell Therapeutics, Inc. (Irvine, CA; see also U.S. Patent No. 5,240,856; U.S. Patent No. 5,215,926; WO 89/06280; WO 91/16116 and WO 92/07243). Alternatively, T cells may be derived from related or unrelated humans, non-human mammals, cell lines or cultures.

T cells may be stimulated with a polypeptide, polynucleotide encoding a polypeptide and/or an antigen presenting cell (APC) that expresses such a polypeptide. Such stimulation is performed under conditions and for a time sufficient to permit the generation of T cells that are specific for the polypeptide of interest. Preferably, a tumor polypeptide or polynucleotide of the invention is present within a delivery vehicle, such as a microsphere, to facilitate the generation of specific T cells.

T cells are considered to be specific for a polypeptide of the present invention if the T cells specifically proliferate, secrete cytokines or kill target cells coated with the polypeptide or expressing a gene encoding the polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res. 54:1065-1070, 1994. Alternatively, detection of the proliferation of T cells may be accomplished by a variety of known techniques. For example, T cell proliferation can be detected by measuring an increased rate of DNA synthesis (*e*.*g*., by pulse-labeling cultures of T cells with tritiated thymidine and measuring the amount of tritiated thymidine incorporated into DNA). Contact with a tumor polypeptide (100 ng/ml - 100 µg/ml, preferably 200 ng/ml - 25 µg/ml) for 3 - 7 days will typically result in at least a two fold increase in proliferation of the T cells. Contact as described above for 2-3 hours should result in activation of the T cells, as measured using standard cytokine assays in which a two fold increase in the level of cytokine release (*e*.*g*., TNF or IFN-γ) is indicative of T cell activation *(see* Coligan et al., Current Protocols in Immunology, vol. 1, Wiley Interscience (Greene 1998)). T cells that have been activated in response to a tumor polypeptide, polynucleotide or polypeptide-expressing APC may be CD4⁺ and/or CD8⁺. Tumor polypeptide-specific T cells may be expanded using standard techniques. Within preferred embodiments, the T cells are derived from a patient, a related donor or an unrelated donor, and are administered to the patient following stimulation and expansion.

For therapeutic purposes, CD4⁺ or CD8⁺ T cells that proliferate in response to a tumor polypeptide, polynucleotide or APC can be expanded in number either *in vitro* or *in vivo.* Proliferation of such T cells *in vitro* may be accomplished in a variety of ways. For example, the T cells can be re-exposed to a tumor polypeptide, or a short peptide corresponding to an immunogenic portion of such a polypeptide, with or without the addition of T cell growth factors, such as interleukin-2, and/or stimulator cells that synthesize a tumor polypeptide. Alternatively, one or more T cells that proliferate in the presence of the tumor polypeptide can be expanded in number by cloning. Methods for cloning cells are well know in the art, and include limiting dilution.

### T Cell Receptor Compositions

The T cell receptor (TCR) consists of 2 different, highly variable polypeptide chains, termed the T-cell receptor α and β chains, that are linked by a disulfide bond (Janeway, Travers, Walport. Immunobiology. Fourth Ed., 148-159. Elsevier Science Ltd/Garland Publishing. 1999). The α/β heterodimer complexes with the invariant CD3 chains at the cell membrane. This complex recognizes specific antigenic peptides bound to MHC molecules. The enormous diversity of TCR specificities is generated much like immunoglobulin diversity, through somatic gene rearrangement. The β chain genes contain over 50 variable (V), 2 diversity (D), over 10 joining (J) segments, and 2 constant region segments (C). The α chain genes contain over 70 V segments, and over 60 J segments but no D segments, as well as one C segment. During T cell development in the thymus, the D to J gene rearrangement of the β chain occurs, followed by the V gene segment rearrangement to the DJ. This functional VDJ_{β} exon is transcribed and spliced to join to a C_{β}. For the α chain, a V_{α} gene segment rearranges to a J_{α} gene segment to create the functional exon that is then transcribed and spliced to the C_{α}. Diversity is further increased during the recombination process by the random addition of P and N-nucleotides between the V, D, and J segments of the β chain and between the V and J segments in the α chain (Janeway, Travers, Walport. Immunobiology. Fourth Ed., 98 and 150. Elsevier Science Ltd/Garland Publishing. 1999).

The present invention, in another aspect, provides TCRs specific for a polypeptide disclosed herein, or for a variant or derivative thereof. In accordance with the present invention, polynucleotide and amino acid sequences are provided for the V-J or V-D-J junctional regions or parts thereof for the alpha and beta chains of the T-cell receptor which recognize tumor polypeptides described herein. In general, this aspect of the invention relates to T-cell receptors which recognize or bind tumor polypeptides presented in the context of MHC. In a preferred embodiment the tumor antigens recognized by the T-cell receptors comprise a polypeptide of the present invention. For example, cDNA encoding a TCR specific for a _tumor peptide can be isolated from T cells specific for a tumor polypeptide using standard molecular biological and recombinant DNA techniques.

This invention further includes the T-cell receptors or analogs thereof having substantially the same function or activity as the T-cell receptors of this invention which recognize or bind tumor polypeptides. Such receptors include, but are not limited to, a fragment of the receptor, or a substitution, addition or deletion mutant of a T-cell receptor provided herein. This invention also encompasses polypeptides or peptides that are substantially homologous to the T-cell receptors provided herein or that retain substantially the same activity. The term "analog" includes any protein or polypeptide having an amino acid residue sequence substantially identical to the T-cell receptors provided herein in which one or more residues, preferably no more than 5 residues, more preferably no more than 25 residues have been conservatively substituted with a functionally similar residue and which displays the functional aspects of the T-cell receptor as described herein.

The present invention further provides for suitable mammalian host cells, for example, non-specific T cells, that are transfected with a polynucleotide encoding TCRs specific for a polypeptide described herein, thereby rendering the host cell specific for the polypeptide. The α and β chains of the TCR may be contained on separate expression vectors or alternatively, on a single expression vector that also contains an internal ribosome entry site (IRES) for cap-independent translation of the gene downstream of the IRES. Said host cells expressing TCRs specific for the polypeptide may be used, for example, for adoptive immunotherapy of lung cancer as discussed further below.

In further aspects of the present invention, cloned TCRs specific for a polypeptide recited herein may be used in a kit for the diagnosis of lung cancer. For example, the nucleic acid sequence or portions thereof, of tumor-specific TCRs can be used as probes or primers for the detection of expression of the rearranged genes encoding the specific TCR in a biological sample. Therefore, the present invention further provides for an assay for detecting messenger RNA or DNA encoding the TCR specific for a polypeptide.

### Pharmaceutical Compositions

In additional embodiments, the present invention concerns formulation of one or more of the polynucleotide, polypeptide, T-cell and/or antibody compositions disclosed herein in pharmaceutically-acceptable carriers for administration to a cell or an animal, either alone, or in combination with one or more other modalities of therapy.

It will be understood that, if desired, a composition as disclosed herein may be administered in combination with other agents as well, such as, e.g., other proteins or polypeptides or various pharmaceutically-active agents. In fact, there is virtually no limit to other components that may also be included, given that the additional agents do not cause a significant adverse effect upon contact with the target cells or host tissues. The compositions may thus be delivered along with various other agents as required in the particular instance. Such compositions may be purified from host cells or other biological sources, or alternatively may be chemically synthesized as described herein. Likewise, such compositions may further comprise substituted or derivatized RNA or DNA compositions.

Therefore, in another aspect of the present invention, pharmaceutical compositions are provided comprising one or more of the polynucleotide, polypeptide, antibody, and/or T-cell compositions described herein in combination with a physiologically acceptable carrier. In certain preferred embodiments, the pharmaceutical compositions of the invention comprise immunogenic polynucleotide and/or polypeptide compositions of the invention for use in prophylactic and theraputic vaccine applications. Vaccine preparation is generally described in, for example, M.F. Powell and M.J. Newman, eds., "Vaccine Design (the subunit and adjuvant approach)," Plenum Press (NY, 1995). Generally, such compositions will comprise one or more polynucleotide and/or polypeptide compositions of the present invention in combination with one or more immunostimulants.

It will be apparent that any of the pharmaceutical compositions described herein can contain pharmaceutically acceptable salts of the polynucleotides and polypeptides of the invention. Such salts can be prepared, for example, from pharmaceutically acceptable non-toxic bases, including organic bases (e.g., salts of primary, secondary and tertiary amines and basic amino acids) and inorganic bases (e.g., sodium, potassium, lithium, ammonium, calcium and magnesium salts).

In another embodiment, illustrative immunogenic compositions, e.g., vaccine compositions, of the present invention comprise DNA encoding one or more of the polypeptides as described above, such that the polypeptide is generated *in situ.* As noted above, the polynucleotide may be administered within any of a variety of delivery systems known to those of ordinary skill in the art. Indeed, numerous gene delivery techniques are well known in the art, such as those described by Rolland, Crit. Rev. Therap. Drug Carrier Systems 15:143-198, 1998, and references cited therein. Appropriate polynucleotide expression systems will, of course, contain the necessary regulatory DNA regulatory sequences for expression in a patient (such as a suitable promoter and terminating signal). Alternatively, bacterial delivery systems may involve the administration of a bacterium (such as *Bacillus-Calmette-Guerrin*) that expresses an immunogenic portion of the polypeptide on its cell surface or secretes such an epitope.

Therefore, in certain embodiments, polynucleotides encoding immunogenic polypeptides described herein are introduced into suitable mammalian host cells for expression using any of a number of known viral-based systems. In one illustrative embodiment, retroviruses provide a convenient and effective platform for gene delivery systems. A selected nucleotide sequence encoding a polypeptide of the present invention can be inserted into a vector and packaged in retroviral particles using techniques known in the art. The recombinant virus can then be isolated and delivered to a subject. A number of illustrative retroviral systems have been described (*e*.*g*., U.S. Pat. No. 5,219,740; Miller and Rosman (1989) BioTechniques 7:980-990; Miller, A. D. (1990) Human Gene Therapy 1:5-14; Scarpa et al. (1991) Virology 180:849-852; Bums et al. (1993) Proc. Natl. Acad. Sci. USA 90:8033-8037; and Boris-Lawrie and Temin (1993) Cur. Opin. Genet. Develop. 3:102-109.

In addition, a number of illustrative adenovirus-based systems have also been described. Unlike retroviruses which integrate into the host genome, adenoviruses persist extrachromosomally thus minimizing the risks associated with insertional mutagenesis (Haj-Ahmad and Graham (1986) J. Virol. 57:267-274; Bett et al. (1993) J. Virol. 67:5911-5921; Mittereder et al. (1994) Human Gene Therapy 5:717-729; Seth et al. (1994) J. Virol. 68:933-940; Barr et al. (1994) Gene Therapy 1:51-58; Berkner, K. L. (1988) BioTechniques 6:616-629; and Rich et al. (1993) Human Gene Therapy 4:461-476).

Various adeno-associated virus (AAV) vector systems have also been developed for polynucleotide delivery. AAV vectors can be readily constructed using techniques well known in the art. See, *e*.*g*., U.S. Pat. Nos. 5,173,414 and 5,139,941; International Publication Nos. WO 92/01070 and WO 93/03769; Lebkowski et al. (1988) Molec. Cell. Biol. 8:3988-3996; Vincent et al. (1990) Vaccines 90 (Cold Spring Harbor Laboratory Press); Carter, B. J. (1992) Current Opinion in Biotechnology 3:533-539; Muzyczka, N. (1992) Current Topics in Microbiol. and Immunol. 158:97-129; Kotin, R. M. (1994) Human Gene Therapy 5:793-801; Shelling and Smith (1994) Gene Therapy 1:165-169; and Zhou et al. (1994) J. Exp. Med. 179:1867-1875.

Additional viral vectors useful for delivering the polynucleotides encoding polypeptides of the present invention by gene transfer include those derived from the pox family of viruses, such as vaccinia virus and avian poxvirus. By way of example, vaccinia virus recombinants expressing the novel molecules can be constructed as follows. The DNA encoding a polypeptide is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the polypeptide of interest into the viral genome. The resulting TK.sup.(-) recombinant can be selected by culturing the cells in the presence of 5-bromodeoxyuridine and picking viral plaques resistant thereto.

A vaccinia-based infection/transfection system can be conveniently used to provide for inducible, transient expression or coexpression of one or more polypeptides described herein in host cells of an organism. In this particular system, cells are first infected in vitro with a vaccinia virus recombinant that encodes the bacteriophage T7 RNA polymerase. This polymerase displays exquisite specificity in that it only transcribes templates bearing T7 promoters. Following infection, cells are transfected with the polynucleotide or polynucleotides of interest, driven by a T7 promoter. The polymerase expressed in the cytoplasm from the vaccinia virus recombinant transcribes the transfected DNA into RNA which is then translated into polypeptide by the host translational machinery. The method provides for high level, transient, cytoplasmic production of large quantities of RNA and its translation products. See, *e*.*g*., Elroy-Stein and Moss, Proc. Natl. Acad. Sci. USA (1990) 87:6743-6747; Fuerst et al. Proc. Natl. Acad. Sci. USA (1986) 83:8122-8126.

Alternatively, avipoxviruses, such as the fowlpox and canarypox viruses, can also be used to deliver the coding sequences of interest. Recombinant avipox viruses, expressing immunogens from mammalian pathogens, are known to confer protective immunity when administered to non-avian species. The use of an Avipox vector is particularly desirable in human and other mammalian species since members of the Avipox genus can only productively replicate in susceptible avian species and therefore are not infective in mammalian cells. Methods for producing recombinant Avipoxviruses are known in the art and employ genetic recombination, as described above with respect to the production of vaccinia viruses. See, e.g., WO 91/12882; WO 89/03429; and WO 92/03545.

Any of a number of alphavirus vectors can also be used for delivery of polynucleotide compositions of the present invention, such as those vectors described in U.S. Patent Nos. 5,843,723; 6,015,686; 6,008,035 and 6,015,694. Certain vectors based on Venezuelan Equine Encephalitis (VEE) can also be used, illustrative examples of which can be found in U.S. Patent Nos. 5,505,947 and 5,643,576.

Moreover, molecular conjugate vectors, such as the adenovirus chimeric vectors described in Michael et al. J. Biol. Chem. (1993) 268:6866-6869 and Wagner et al. Proc. Natl. Acad. Sci. USA (1992) 89:6099-6103, can also be used for gene delivery under the invention.

Additional illustrative information on these and other known viral-based delivery systems can be found, for example, in Fisher-Hoch et al., Proc. Natl. Acad Sci. USA 86:317-321, 1989; Flexner et al., Ann. N.Y. Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad Sci. USA 91:215-219, 1994; Kass-Eisler et al., Proc. Natl. Acad. Sci. USA 90:11498-11502, 1993; Guzman et al., Circulation 88:2838-2848, 1993; and Guzman et al., Cir. Res. 73:1202-1207, 1993.

In certain embodiments, a polynucleotide may be integrated into the genome of a target cell. This integration may be in the specific location and orientation *via* homologous recombination (gene replacement) or it may be integrated in a random, non-specific location (gene augmentation). In yet further embodiments, the polynucleotide may be stably maintained in the cell as a separate, episomal segment of DNA. Such polynucleotide segments or "episomes" encode sequences sufficient to permit maintenance and replication independent of or in synchronization with the host cell cycle. The manner in which the expression construct is delivered to a cell and where in the cell the polynucleotide remains is dependent on the type of expression construct employed.

In another embodiment of the invention, a polynucleotide is administered/delivered as "naked" DNA, for example as described in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

In still another embodiment, a composition of the present invention can be delivered via a particle bombardment approach, many of which have been described. In one illustrative example, gas-driven particle acceleration can be achieved with devices such as those manufactured by Powderject Pharmaceuticals PLC (Oxford, UK) and Powderject Vaccines Inc. (Madison, WI), some examples of which are described in U.S. Patent Nos. 5,846,796; 6,010,478; 5,865,796; 5,584,807; and EP Patent No. 0500 799. This approach offers a needle-free delivery approach wherein a dry powder formulation of microscopic particles, such as polynucleotide or polypeptide particles, are accelerated to high speed within a helium gas jet generated by a hand held device, propelling the particles into a target tissue of interest.

In a related embodiment, other devices and methods that may be useful for gas-driven needle-less injection of compositions of the present invention include those provided by Bioject, Inc. (Portland, OR), some examples of which are described in U.S. Patent Nos. 4,790,824; 5,064,413; 5,312,335; 5,383,851; 5,399,163; 5,520,639 and 5,993,412.

According to another embodiment, the pharmaceutical compositions described herein will comprise one or more immunostimulants in addition to the immunogenic polynucleotide, polypeptide, antibody, T-cell and/or APC compositions of this invention. An immunostimulant refers to essentially any substance that enhances or potentiates an immune response (antibody and/or cell-mediated) to an exogenous antigen. One preferred type of immunostimulant comprises an adjuvant. Many adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Certain adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF, interleukin-2, -7, -12, and other like growth factors, may also be used as adjuvants.

Within certain embodiments of the invention, the adjuvant composition is preferably one that induces an immune response predominantly of the Th1 type. High levels of Th1-type cytokines (*e*.*g*., IFN-γ, TNFα, IL-2 and IL-12) tend to favor the induction of cell mediated immune responses to an administered antigen. In contrast, high levels of Th2-type cytokines (*e*.*g*., IL-4, IL-5, IL-6 and IL-10) tend to favor the induction of humoral immune responses. Following application of a vaccine as provided herein, a patient will support an immune response that includes Th1- and Th2- type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

Certain preferred adjuvants for eliciting a predominantly Th1-type response include, for example, a combination of monophosphoryl lipid A, preferably 3-de-O-acylated monophosphoryl lipid A, together with an aluminum salt. MPL^{®} adjuvants are available from Corixa Corporation (Seattle, WA; *see*, for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., Science 273:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins. Other preferred formulations include more than one saponin in the adjuvant combinations of the present invention, for example combinations of at least two of the following group comprising QS21, QS7, Quil A, β-escin, or digitonin.

Alternatively the saponin formulations may be combined with vaccine vehicles composed of chitosan or other polycationic polymers, polylactide and polylactide-co-glycolide particles, poly-N-acetyl glucosamine-based polymer matrix, particles composed of polysaccharides or chemically modified polysaccharides, liposomes and lipid-based particles, particles composed of glycerol monoesters, etc. The saponins may also be formulated in the presence of cholesterol to form particulate structures such as liposomes or ISCOMs. Furthermore, the saponins may be formulated together with a polyoxyethylene ether or ester, in either a non-particulate solution or suspension, or in a particulate structure such as a paucilamelar liposome or ISCOM. The saponins may also be formulated with excipients such as Carbopol^{R} to increase viscosity, or may be formulated in a dry powder form with a powder excipient such as lactose.

In one preferred embodiment, the adjuvant system includes the combination of a monophosphoryl lipid A and a saponin derivative, such as the combination of QS21 and 3D-MPL^{®} adjuvant, as described in WO 94/00153, or a less reactogenic composition where the QS21 is quenched with cholesterol, as described in WO 96/33739. Other preferred formulations comprise an oil-in-water emulsion and tocopherol. Another particularly preferred adjuvant formulation employing QS21, 3D-MPL^{®} adjuvant and tocopherol in an oil-in-water emulsion is described in WO 95/17210.

Another enhanced adjuvant system involves the combination of a CpG-containing oligonucleotide and a saponin derivative particularly the combination of CpG and QS21 is disclosed in WO 00/09159. Preferably the formulation additionally comprises an oil in water emulsion and tocopherol.

Additional illustrative adjuvants for use in the pharmaceutical compositions of the invention include Montanide ISA 720 (Seppic, France), SAF (Chiron, California, United States), ISCOMS (CSL), MF-59 (Chiron), the SBAS series of adjuvants (e.g., SBAS-2 or SBAS-4, available from SmithKline Beecham, Rixensart, Belgium), Detox (Enhanzyn^{®} (Corixa, Hamilton, MT), RC-529 (Corixa, Hamilton, MT) and other aminoalkyl glucosaminide 4-phosphates (AGPs), such as those described in pending U.S. Patent Application Serial Nos. 08/853,826 and 09/074,720, the disclosures of which are incorporated herein by reference in their entireties, and polyoxyethylene ether adjuvants such as those described in WO 99/52549A1.

Other preferred adjuvants include adjuvant molecules of the general formula

(I): HO(CH₂CH₂O)ₙ-A-R,

wherein, n is 1-50, A is a bond or -C(O)-, R is C₁₋₅₀ alkyl or Phenyl C₁₋₅₀ alkyl.

One embodiment of the present invention consists of a vaccine formulation comprising a polyoxyethylene ether of general formula (I), wherein *n* is between 1 and 50, preferably 4-24, most preferably 9; the *R* component is C₁₋₅₀, preferably C₄-C₂₀ alkyl and most preferably C₁₂ alkyl, and *A* is a bond. The concentration of the polyoxyethylene ethers should be in the range 0.1-20%, preferably from 0.1-10%, and most preferably in the range 0.1-1%. Preferred polyoxyethylene ethers are selected from the following group: polyoxyethylene-9-lauryl ether, polyoxyethylene-9-steoryl ether, polyoxyethylene-8-steoryl ether, polyoxyethylene-4-lauryl ether, polyoxyethylene-35-lauryl ether, and polyoxyethylene-23-lauryl ether. Polyoxyethylene ethers such as polyoxyethylene lauryl ether are described in the Merck index (12^{th} edition: entry 7717). These adjuvant molecules are described in WO 99/52549.

The polyoxyethylene ether according to the general formula (I) above may, if desired, be combined with another adjuvant. For example, a preferred adjuvant combination is preferably with CpG as described in the WO 99/52549.

According to another embodiment of this invention, an immunogenic composition described herein is delivered to a host via antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver (*i*.*e.*, matched HLA haplotype). APCs may generally be isolated from any of a variety of biological fluids and organs, including tumor and peritumoral tissues, and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, Nature 392:245-251, 1998) and have been shown to be effective as a physiological adjuvant for eliciting prophylactic or therapeutic antitumor immunity (*see* Timmerman and Levy, Ann. Rev. Med. 50:507-529, 1999). In general, dendritic cells may be identified based on their typical shape (stellate *in situ,* with marked cytoplasmic processes (dendrites) visible *in vitro*), their ability to take up, process and present antigens with high efficiency and their ability to activate naive T cell responses. Dendritic cells may, of course, be engineered to express specific cell-surface receptors or ligands that are not commonly found on dendritic cells *in vivo* or *ex vivo,* and such modified dendritic cells are contemplated by the present invention. As an alternative to dendritic cells, secreted vesicles antigen-loaded dendritic cells (called exosomes) may be used within a vaccine (*see* Zitvogel et al., Nature Med. 4:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e*.*g*., CD54 and CD11) and costimulatory molecules (*e*.*g*., CD40, CD80, CD86 and 4-1BB).

APCs may generally be transfected with a polynucleotide of the invention (or portion or other variant thereof) such that the encoded polypeptide, or an immunogenic portion thereof, is expressed on the cell surface. Such transfection may take place *ex vivo,* and a Pharmaceutical composition comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., Immunology and cell Biology 75:456-460, 1997. Antigen loading of dendritic cells may be achieved by incubating dendritic cells or progenitor cells with the tumor polypeptide, DNA (naked or within a plasmid vector) or RNA; or with antigen-expressing recombinant bacterium or viruses (*e*.*g*., vaccinia, fowlpox, adenovirus or lentivirus vectors). Prior to loading, the polypeptide may be covalently conjugated to an immunological partner that provides T cell help (*e*.*g*., a carrier molecule). Alternatively, a dendritic cell may be pulsed with a non-conjugated immunological partner, separately or in the presence of the polypeptide.

While any suitable carrier known to those of ordinary skill in the art may be employed in the pharmaceutical compositions of this invention, the type of carrier will typically vary depending on the mode of administration. Compositions of the present invention may be formulated for any appropriate manner of administration, including for example, topical, oral, nasal, mucosal, intravenous, intracranial, intraperitoneal, subcutaneous and intramuscular administration.

Carriers for use within such pharmaceutical compositions are biocompatible, and may also be biodegradable. In certain embodiments, the formulation preferably provides a relatively constant level of active component release. In other embodiments, however, a more rapid rate of release immediately upon administration may be desired. The formulation of such compositions is well within the level of ordinary skill in the art using known techniques. Illustrative carriers useful in this regard include microparticles of poly(lactide-co-glycolide), polyacrylate, latex, starch, cellulose, dextran and the like. Other illustrative delayed-release carriers include supramolecular biovectors, which comprise a non-liquid hydrophilic core (*e*.*g*., a cross-linked polysaccharide or oligosaccharide) and, optionally, an external layer comprising an amphiphilic compound, such as a phospholipid (*see e*.*g*., U.S. Patent No. 5,151,254 and PCT applications WO 94/20078, WO/94/23701 and WO 96/06638). The amount of active compound contained within a sustained release formulation depends upon the site of implantation, the rate and expected duration of release and the nature of the condition to be treated or prevented.

In another illustrative embodiment, biodegradable microspheres (*e*.*g*., polylactate polyglycolate) are employed as carriers for the compositions of this invention. Suitable biodegradable microspheres are disclosed, for example, in U.S. Patent Nos. 4,897,268; 5,075,109; 5,928,647; 5,811,128; 5,820,883; 5,853,763; 5,814,344, 5,407,609 and 5,942,252. Modified hepatitis B core protein carrier systems, such as described in WO/99 40934, and references cited therein, will also be useful for many applications. Another illustrative carrier/delivery system employs a carrier comprising particulate-protein complexes, such as those described in U.S. Patent No. 5,928,647, which are capable of inducing a class I-restricted cytotoxic T lymphocyte responses in a host.

The pharmaceutical compositions of the invention will often further comprise one or more buffers (*e*.*g*., neutral buffered saline or phosphate buffered saline), carbohydrates (*e*.*g*., glucose, mannose, sucrose or dextrans), mannitol, proteins, polypeptides or amino acids such as glycine, antioxidants, bacteriostats, chelating agents such as EDTA or glutathione, adjuvants (*e*.*g*., aluminum hydroxide), solutes that render the formulation isotonic, hypotonic or weakly hypertonic with the blood of a recipient, suspending agents, thickening agents and/or preservatives. Alternatively, compositions of the present invention may be formulated as a lyophilizate.

The pharmaceutical compositions described herein may be presented in unit-dose or multi-dose containers, such as sealed ampoules or vials. Such containers are typically sealed in such a way to preserve the sterility and stability of the formulation until use. In general, formulations may be stored as suspensions, solutions or emulsions in oily or aqueous vehicles. Alternatively, a pharmaceutical composition may be stored in a freeze-dried condition requiring only the addition of a sterile liquid carrier immediately prior to use.

The development of suitable dosing and treatment regimens for using the particular compositions described herein in a variety of treatment regimens, including e.g., oral, parenteral, intravenous, intranasal, and intramuscular administration and formulation, is well known in the art, some of which are briefly discussed below for general purposes of illustration.

In certain applications, the pharmaceutical compositions disclosed herein may be delivered *via* oral administration to an animal. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

The active compounds may even be incorporated with excipients and used in the form of ingestible tablets, buccal tables, troches, capsules, elixirs, suspensions, syrups, wafers, and the like (see, for example, Mathiowitz et al., Nature 1997 Mar 27;386(6623):410-4; Hwang et al., Crit Rev Ther Drug Carrier Syst 1998;15(3):243-84; U. S. Patent 5,641,515; U. S. Patent 5,580,579 and U. S. Patent 5,792,451). Tablets, troches, pills, capsules and the like may also contain any of a variety of additional components, for example, a binder, such as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. Of course, any material used in preparing any dosage unit form should be pharmaceutically pure and substantially non-toxic in the amounts employed. In addition, the active compounds may be incorporated into sustained-release preparation and formulations.

Typically, these formulations will contain at least about 0.1% of the active compound or more, although the percentage of the active ingredient(s) may, of course, be varied and may conveniently be between about 1 or 2% and about 60% or 70% or more of the weight or volume of the total formulation. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

For oral administration the compositions of the present invention may alternatively be incorporated with one or more excipients in the form of a mouthwash, dentifrice, buccal tablet, oral spray, or sublingual orally-administered formulation. Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively the compositions may be fashioned into a tablet or solution form that may be placed under the tongue or otherwise dissolved in the mouth.

In certain circumstances it will be desirable to deliver the pharmaceutical compositions disclosed herein parenterally, intravenously, intramuscularly, or even intraperitoneally. Such approaches are well known to the skilled artisan, some of which are further described, for example, in U. S. Patent 5,543,158; U. S. Patent 5,641,515 and U. S. Patent 5,399,363. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms.

Illustrative pharmaceutical forms suitable for injectable use include sterile aqueous solutions or dispersions and sterile powders for the extemporaneous preparation of sterile injectable solutions or dispersions (for example, see U. S. Patent 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (*e*.*g*., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In one embodiment, for parenteral administration in an aqueous solution, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. Moreover, for human administration, preparations will of course preferably meet sterility, pyrogenicity, and the general safety and purity standards as required by FDA Office of Biologies standards.

In another embodiment of the invention, the compositions disclosed herein may be formulated in a neutral or salt form. Illustrative pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective.

The carriers can further comprise any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically-acceptable" refers to molecular entities and compositions that do not produce an allergic or similar untoward reaction when administered to a human.

In certain embodiments, the pharmaceutical compositions may be delivered by intranasal sprays, inhalation, and/or other aerosol delivery vehicles. Methods for delivering genes, nucleic acids, and peptide compositions directly to the lungs *via* nasal aerosol sprays has been described, *e*.*g*., in U. S. Patent 5,756,353 and U. S. Patent 5,804,212. Likewise, the delivery of drugs using intranasal microparticle resins (Takenaga et al., J Controlled Release 1998 Mar 2;52(1-2):81-7) and lysophosphatidyl-glycerol compounds (U. S. Patent 5,725,871) are also well-known in the pharmaceutical arts. Likewise, illustrative transmucosal drug delivery in the form of a polytetrafluoroetheylene support matrix is described in U. S. Patent 5,780,045.

In certain embodiments, liposomes, nanocapsules, microparticles, lipid particles, vesicles, and the like, are used for the introduction of the compositions of the present invention into suitable host cells/organisms. In particular, the compositions of the present invention may be formulated for delivery either encapsulated in a lipid particle, a liposome, a vesicle, a nanosphere, or a nanoparticle or the like. Alternatively, compositions of the present invention can be bound, either covalently or non-covalently, to the surface of such carrier vehicles.

The formation and use of liposome and liposome-like preparations as potential drug carriers is generally known to those of skill in the art (see for example, Lasic, Trends Biotechnol 1998 Jul;16(7):307-21; Takakura, Nippon Rinsho 1998 Mar;56(3):691-5; Chandran et al., Indian J Exp Biol. 1997 Aug;35(8):801-9; Margalit, Crit Rev Ther Drug Carrier Syst. 1995;12(2-3):233-61; U.S. Patent 5,567,434; U.S. Patent 5,552,157; U.S. Patent 5,565,213; U.S. Patent 5,738,868 and U.S. Patent 5,795,587).

Liposomes have been used successfully with a number of cell types that are normally difficult to transfect by other procedures, including T cell suspensions, primary hepatocyte cultures and PC 12 cells (Renneisen et al., J Biol Chem. 1990 Sep 25;265(27):16337-42; Muller et al., DNA Cell Biol. 1990 Apr;9(3):221-9). In addition, liposomes are free of the DNA length constraints that are typical of viral-based delivery systems. Liposomes have been used effectively to introduce genes, various drugs, radiotherapeutic agents, enzymes, viruses, transcription factors, allosteric effectors and the like, into a variety of cultured cell lines and animals. Furthermore, he use of liposomes does not appear to be associated with autoimmune responses or unacceptable toxicity after systemic delivery.

In certain embodiments, liposomes are formed from phospholipids that are dispersed in an aqueous medium and spontaneously form multilamellar concentric bilayer vesicles (also termed multilamellar vesicles (MLVs).

Alternatively, in other embodiments, the invention provides for pharmaceutically-acceptable nanocapsule formulations of the compositions of the present invention. Nanocapsules can generally entrap compounds in a stable and reproducible way (see, for example, Quintanar-Guerrero et al., Drug Dev Ind Pharm. 1998 Dec;24(12):1113-28). To avoid side effects due to intracellular polymeric overloading, such ultrafine particles (sized around 0.1 µm) may be designed using polymers able to be degraded *in vivo.* Such particles can be made as described, for example, by Couvreur et al., Crit Rev Ther Drug Carrier Syst. 1988;5(1):1-20; zur Muhlen et al., Eur J Pharm Biopharm. 1998 Mar;45(2):149-55; Zambaux et al. J Controlled Release. 1998 Jan 2;50(1-3):31-40; and U. S. Patent 5,145,684.

### Cancer Therapeutic Methods

Immunologic approaches to cancer therapy are based on the recognition that cancer cells can often evade the body's defenses against aberrant or foreign cells and molecules, and that these defenses might be therapeutically stimulated to regain the lost ground, e.g. pgs. 623-648 in Klein, Immunology (Wiley-Interscience, New York, 1982). Numerous recent observations that various immune effectors can directly or indirectly inhibit growth of tumors has led to renewed interest in this approach to cancer therapy, e.g. Jager, et al., Oncology 2001;60(1):1-7; Renner, et al., Ann Hematol 2000 Dec;79(12):651-9.

Four-basic cell types whose function has been associated with antitumor cell immunity and the elimination of tumor cells from the body are: i) B-lymphocytes which secrete immunoglobulins into the blood plasma for identifying and labeling the nonself invader cells; ii) monocytes which secrete the complement proteins that are responsible for lysing and processing the immunoglobulin-coated target invader cells; iii) natural killer lymphocytes having two mechanisms for the destruction of tumor cells, antibody-dependent cellular cytotoxicity and natural killing; and iv) T-lymphocytes possessing antigen-specific receptors and having the capacity to recognize a tumor cell carrying complementary marker molecules (Schreiber, H., 1989, in Fundamental Immunology (ed). W. E. Paul, pp. 923-955).

Cancer immunotherapy generally focuses on inducing humoral immune responses, cellular immune responses, or both. Moreover, it is well established that induction of CD4⁺ T helper cells is necessary in order to secondarily induce either antibodies or cytotoxic CD8⁺ T cells. Polypeptide antigens that are selective or ideally specific for cancer cells, particularly lung cancer cells, offer a powerful approach for inducing immune responses against lung cancer, and are an important aspect of the present invention.

Therefore, in further aspects of the present invention, the pharmaceutical compositions described herein may be used for the treatment of cancer, particularly for the immunotherapy of lung cancer. Within such methods, the pharmaceutical compositions described herein are administered to a patient, typically a warm-blooded animal, preferably a human. A patient may or may not be afflicted with cancer. Accordingly, the above pharmaceutical compositions may be used to prevent the development of a cancer or to treat a patient afflicted with a cancer. Pharmaceutical compositions and vaccines may be administered either prior to or following surgical removal of primary tumors and/or treatment such as administration of radiotherapy or conventional chemotherapeutic drugs. As discussed above, administration of the pharmaceutical compositions may be by any suitable method, including administration by intravenous, intraperitoneal, intramuscular, subcutaneous, intranasal, intradermal, anal, vaginal, topical and oral routes.

Within certain embodiments, immunotherapy may be active immunotherapy, in which treatment relies on the *in vivo* stimulation of the endogenous host immune system to react against tumors with the administration of immune response-modifying agents (such as polypeptides and polynucleotides as provided herein).

Within other embodiments, immunotherapy may be passive immunotherapy, in which treatment involves the delivery of agents with established tumor-immune reactivity (such as effector cells or antibodies) that can directly or indirectly mediate antitumor effects and does not necessarily depend on an intact host immune system. Examples of effector cells include T cells as discussed above, T lymphocytes (such as CD8⁺ cytotoxic T lymphocytes and CD4⁺ T-helper tumor-infiltrating lymphocytes), killer cells (such as Natural Killer cells and lymphokine-activated killer cells), B cells and antigen-presenting cells (such as dendritic cells and macrophages) expressing a polypeptide provided herein. T cell receptors and antibody receptors specific for the polypeptides recited herein may be cloned, expressed and transferred into other vectors or effector cells for adoptive immunotherapy. The polypeptides provided herein may also be used to generate antibodies or anti-idiotypic antibodies (as described above and in U.S. Patent No. 4,918,164) for passive immunotherapy.

Monoclonal antibodies may be labeled with any of a variety of labels for desired selective usages in detection, diagnostic assays or therapeutic applications (as described in U.S. Patent Nos. 6,090,365; 6,015,542; 5,843,398; 5,595,721; and 4,708,930). In each case, the binding of the labelled monoclonal antibody to the determinant site of the antigen will signal detection or delivery of a particular therapeutic agent to the antigenic determinant on the non-normal cell. A further object of this invention is to provide the specific monoclonal antibody suitably labelled for achieving such desired selective usages thereof.

Effector cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells. As noted above, immunoreactive polypeptides as provided herein may be used to rapidly expand antigen-specific T cell cultures in order to generate a sufficient number of cells for immunotherapy. In particular, antigen-presenting cells, such as dendritic, macrophage, monocyte, fibroblast and/or B cells, may be pulsed with immunoreactive polypeptides or transfected with one or more polynucleotides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo.* Studies have shown that cultured effector cells can be induced to grow in vivo and to survive long term in substantial numbers by repeated stimulation with antigen supplemented with IL-2 (*see,* for example, Cheever et al., Immunological Reviews 157:177, 1997).

Alternatively, a vector expressing a polypeptide recited herein may be introduced into antigen presenting cells taken from a patient and clonally propagated *ex vivo* for transplant back into the same patient. Transfected cells may be reintroduced into the patient using any means known in the art, preferably in sterile form by intravenous, intracavitary, intraperitoneal or intratumor administration.

Routes and frequency of administration of the therapeutic compositions described herein, as well as dosage, will vary from individual to individual, and may be readily established using standard techniques. In general, the pharmaceutical compositions and vaccines may be administered by injection (*e*.*g*., intracutaneous, intramuscular, intravenous or subcutaneous), intranasally (*e*.*g*., by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (*i*.*e*., untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e*.*g*., more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 25 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (*e*.*g*., more frequent remissions, complete or partial, or longer disease-free survival) in treated patients as compared to non-treated patients. Increases in preexisting immune responses to a tumor protein generally correlate with an improved clinical outcome. Such immune responses may generally be evaluated using standard proliferation, cytotoxicity or cytokine assays, which may be performed using samples obtained from a patient before and after treatment.

### Cancer Detection and Diagnostic Compositions, Methods and Kits

In general, a cancer may be detected in a patient based on the presence of one or more lung tumor proteins and/or polynucleotides encoding such proteins in a biological sample (for example, blood, sera, sputum urine and/or tumor biopsies) obtained from the patient. In other words, such proteins may be used as markers to indicate the presence or absence of a cancer such as lung cancer. In addition, such proteins may be useful for the detection of other cancers. The binding agents provided herein generally permit detection of the level of antigen that binds to the agent in the biological sample.

Polynucleotide primers and probes may be used to detect the level of mRNA encoding a tumor protein, which is also indicative of the presence or absence of a cancer. In general, a tumor sequence should be present at a level that is at least two-fold, preferably three-fold, and more preferably five-fold or higher in tumor tissue than in normal tissue of the same type from which the tumor arose. Expression levels of a particular tumor sequence in tissue types different from that in which the tumor arose are irrelevant in certain diagnostic embodiments since the presence of tumor cells can be confirmed by observation of predetermined differential expression levels, e.g., 2-fold, 5-fold, etc, in tumor tissue to expression levels in normal tissue of the same type.

Other differential expression patterns can be utilized advantageously for diagnostic purposes. For example, in one aspect of the invention, overexpression of a tumor sequence in tumor tissue and normal tissue of the same type, but not in other normal tissue types, *e*.*g*. PBMCs, can be exploited diagnostically. In this case, the presence of metastatic tumor cells, for example in a sample taken from the circulation or some other tissue site different from that in which the tumor arose, can be identified and/or confirmed by detecting expression of the tumor sequence in the sample, for example using RT-PCR analysis. In many instances, it will be desired to enrich for tumor cells in the sample of interest, e.g., PBMCs, using cell capture or other like techniques.

There are a variety of assay formats known to those of ordinary skill in the art for using a binding agent to detect polypeptide markers in a sample. *See*, *e*.*g*., Harlow and Lane, Antibodies: A Laboratory Manual, Cold Spring Harbor Laboratory, 1988. In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

In a preferred embodiment, the assay involves the use of binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include full length lung tumor proteins and polypeptide portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (*see*, *e*.*g*., Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20™ (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i*.*e*., incubation time) is a period of time that is sufficient to detect the presence of polypeptide within a sample obtained from an individual with lung cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20^{™}. The second antibody, which contains a reporter group, may then be added to the solid support. Preferred reporter groups include those groups recited above.

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as lung cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-7. Briefly, in this embodiment, the cut-off value may be determined from a plot of pairs of true positive rates (*i*.*e*., sensitivity) and false positive rates (100%-specificity) that correspond to each possible cut-off value for the diagnostic test result. The cut-off value on the plot that is the closest to the upper left-hand corner (*i*.*e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

In a related embodiment, the assay is performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agent-polypeptide complex as a solution containing the second binding agent flows through the membrane. The detection of bound second binding agent may then be performed as described above. In the strip test format, one end of the membrane to which binding agent is bound is immersed in a solution containing the sample. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1µg, and more preferably from about 50 ng to about 500 ng. Such tests can typically be performed with a very small amount of biological sample.

Of course, numerous other assay protocols exist that are suitable for use with the tumor proteins or binding agents of the present invention. The above descriptions are intended to be exemplary only. For example, it will be apparent to those of ordinary skill in the art that the above protocols may be readily modified to use tumor polypeptides to detect antibodies that bind to such polypeptides in a biological sample. The detection of such tumor protein specific antibodies may correlate with the presence of a cancer.

A cancer may also, or alternatively, be detected based on the presence of T cells that specifically react with a tumor protein in a biological sample. Within certain methods, a biological sample comprising CD4⁺ and/or CD8⁺ T cells isolated from a patient is incubated with a tumor polypeptide, a polynucleotide encoding such a polypeptide and/or an APC that expresses at least an immunogenic portion of such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with polypeptide (e.g., 5 - 25 µg/ml). It may be desirable to incubate another aliquot of a T cell sample in the absence of tumor polypeptide to serve as a control. For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding a tumor protein in a biological sample. For example, at least two oligonucleotide primers may be employed in a polymerase chain reaction (PCR) based assay to amplify a portion of a tumor cDNA derived from a biological sample, wherein at least one of the oligonucleotide primers is specific for (*i*.*e*., hybridizes to) a polynucleotide encoding the tumor protein. The amplified cDNA is then separated and detected using techniques well known in the art, such as gel electrophoresis.

Similarly, oligonucleotide probes that specifically hybridize to a polynucleotide encoding a tumor protein may be used in a hybridization assay to detect the presence of polynucleotide encoding the tumor protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding a tumor protein of the invention that is at least 10 nucleotides, and preferably at least 20 nucleotides, in length. Preferably, oligonucleotide primers and/or probes hybridize to a polynucleotide encoding a polypeptide described herein under moderately stringent conditions, as defined above. Oligonucleotide primers and/or probes which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides, of a DNA molecule having a sequence as disclosed herein. Techniques for both PCR based assays and hybridization assays are well known in the art *(see,* for example, Mullis et al., Cold Spring Harbor Symp. Quant. Biol., 51:263, 1987; Erlich ed., PCR Technology, Stockton Press, NY, 1989).

One preferred assay employs RT-PCR, in which PCR is applied in conjunction with reverse transcription. Typically, RNA is extracted from a biological sample, such as biopsy tissue, and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

In another aspect of the present invention, cell capture technologies may be used in conjunction, with, for example, real-time PCR to provide a more sensitive tool for detection of metastatic cells expressing lung tumor antigens. Detection of lung cancer cells in biological samples, e.g., bone marrow samples, peripheral blood, and small needle aspiration samples is desirable for diagnosis and prognosis in lung cancer patients.

Immunomagnetic beads coated with specific monoclonal antibodies to surface cell markers, or tetrameric antibody complexes, may be used to first enrich or positively select cancer cells in a sample. Various commercially available kits may be used, including Dynabeads® Epithelial Enrich (Dynal Biotech, Oslo, Norway), StemSep^{™} (StemCell Technologies, Inc., Vancouver, BC), and RosetteSep (StemCell Technologies). A skilled artisan will recognize that other methodologies and kits may also be used to enrich or positively select desired cell populations. Dynabeads® Epithelial Enrich contains magnetic beads coated with mAbs specific for two glycoprotein membrane antigens expressed on normal and neoplastic epithelial tissues. The coated beads may be added to a sample and the sample then applied to a magnet, thereby capturing the cells bound to the beads. The unwanted cells are washed away and the magnetically isolated cells eluted from the beads and used in further analyses.

RosetteSep can be used to enrich cells directly from a blood sample and consists of a cocktail of tetrameric antibodies that targets a variety of unwanted cells and crosslinks them to glycophorin A on red blood cells (RBC) present in the sample, forming rosettes. When centrifuged over Ficoll, targeted cells pellet along with the free RBC. The combination of antibodies in the depletion cocktail determines which cells will be removed and consequently which cells will be recovered. Antibodies that are available include, but are not limited to: CD2, CD3, CD4, CD5, CD8, CD10, CD11b, CD14, CD15, CD16, CD19, CD20, CD24, CD25, CD29, CD33, CD34, CD36, CD38, CD41, CD45, CD45RA, CD45RO, CD56, CD66B, CD66e, HLA-DR, IgE, and TCRαβ.

Additionally, it is contemplated in the present invention that mAbs specific for lung tumor antigens can be generated and used in a similar manner. For example, mAbs that bind to tumor-specific cell surface antigens may be conjugated to magnetic beads, or formulated in a tetrameric antibody complex, and used to enrich or positively select metastatic lung tumor cells from a sample. Once a sample is enriched or positively selected, cells may be lysed and RNA isolated. RNA may then be subjected to RT-PCR analysis using lung tumor-specific primers in a real-time PCR assay as described herein. One skilled in the art will recognize that enriched or selected populations of cells may be analyzed by other methods (*e.g. in situ* hybridization or flow cytometry).

In another embodiment, the compositions described herein may be used as markers for the progression of cancer. In this embodiment, assays as described above for the diagnosis of a cancer may be performed over time, and the change in the level of reactive polypeptide(s) or polynucleotide(s) evaluated. For example, the assays may be performed every 24-72 hours for a period of 6 months to 1 year, and thereafter performed as needed. In general, a cancer is progressing in those patients in whom the level of polypeptide or polynucleotide detected increases over time. In contrast, the cancer is not progressing when the level of reactive polypeptide or polynucleotide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple tumor protein markers may be assayed within a given sample. It will be apparent that binding agents specific for different proteins provided herein may be combined within a single assay. Further, multiple primers or probes may be used concurrently. The selection of tumor protein markers may be based on routine experiments to determine combinations that results in optimal sensitivity. In addition, or alternatively, assays for tumor proteins provided herein may be combined with assays for other known tumor antigens.

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to a tumor protein. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

Alternatively, a kit may be designed to detect the level of mRNA encoding a tumor protein in a biological sample. Such kits generally comprise at least one oligonucleotide probe or primer, as described above, that hybridizes to a polynucleotide encoding a tumor protein. Such an oligonucleotide may be used, for example, within a PCR or hybridization assay. Additional components that may be present within such kits include a second oligonucleotide and/or a diagnostic reagent or container to facilitate the detection of a polynucleotide encoding a tumor protein.

The following Examples are offered by way of illustration and not by way of limitation.

### EXAMPLES

### EXAMPLE 1

### ISOLATION AND CHARACTERIZATION OF cDNA SEQUENCES ENCODING LUNG TUMOR POLYPEPTIDES

This example illustrates the isolation of cDNA molecules encoding lung tumor-specific polypeptides from lung tumor cDNA libraries.

### A. ISOLATION OF cDNA SEQUENCES FROM A LUNG SQUAMOUS CELL CARCINOMA LIBRARY

A human lung squamous cell carcinoma cDNA expression library was constructed from poly A⁺ RNA from a pool of two patient tissues using a Superscript Plasmid System for cDNA Synthesis and Plasmid Cloning kit (BRL Life Technologies, Gaithersburg, MD) following the manufacturer's protocol. Specifically, lung carcinoma tissues were homogenized with polytron (Kinematica, Switzerland) and total RNA was extracted using Trizol reagent (BRL Life Technologies) as directed by the manufacturer. The poly A⁺ RNA was then purified using an oligo dT cellulose column as described in Sambrook et al., Molecular Cloning: A Laboratory Manual, Cold Spring Harbor Laboratories, Cold Spring Harbor, NY, 1989. First-strand cDNA was synthesized using the NotI/Oligo-dT18 primer. Double-stranded cDNA was synthesized, ligated with BstXI/EcoRI adaptors (Invitrogen, San Diego, CA) and digested with NotI. Following size fractionation with cDNA size fractionation columns (BRL Life Technologies), the cDNA was ligated into the BstXI/NotI site of pcDNA3.1 (Invitrogen) and transformed into ElectroMax *E. coli* DH10B cells (BRL Life Technologies) by electroporation.

Using the same procedure, a normal human lung cDNA expression library was prepared from a pool of four tissue specimens. The cDNA libraries were characterized by determining the number of independent colonies, the percentage of clones that carried insert, the average insert size and by sequence analysis. The lung squamous cell carcinoma library contained 2.7 x 10⁶ independent colonies, with 100% of clones having an insert and the average insert size being 2100 base pairs. The normal lung cDNA library contained 1.4 x 10⁶ independent colonies, with 90% of clones having inserts and the average insert size being 1800 base pairs. For both libraries, sequence analysis showed that the majority of clones had a full length cDNA sequence and were synthesized from mRNA

cDNA library subtraction was performed using the above lung squamous cell carcinoma and normal lung cDNA libraries, as described by Hara et al. (Blood, 84:189-199, 1994) with some modifications. Specifically, a lung squamous cell carcinoma-specific subtracted cDNA library was generated as follows. Normal tissue cDNA library (80 µg) was digested with BamHI and XhoI, followed by a filling-in reaction with DNA polymerase Klenow fragment. After phenol-chloroform extraction and ethanol precipitation, the DNA was dissolved in 133 µl of H₂O, heat-denatured and mixed with 133 µl (133 µg) of Photoprobe biotin (Vector Laboratories, Burlingame, CA). As recommended by the manufacturer, the resulting mixture was irradiated with a 270 W sunlamp on ice for 20 minutes. Additional Photoprobe biotin (67 µl) was added and the biotinylation reaction was repeated. After extraction with butanol five times, the DNA was ethanol-precipitated and dissolved in 23 µl H₂O to form the driver DNA.

To form the tracer DNA, 10 µg lung squamous cell carcinoma cDNA library was digested with NotI and SpeI, phenol chloroform extracted and passed through Chroma spin-400 columns (Clontech, Palo Alto, CA). Typically, 5 µg of cDNA was recovered after the sizing column. Following ethanol precipitation, the tracer DNA was dissolved in 5 µl H₂O. Tracer DNA was mixed with 15 µl driver DNA and 20 µl of 2 x hybridization buffer (1.5 M NaCl/10 mM EDTA/50 mM HEPES pH 7.5/0.2% sodium dodecyl sulfate), overlaid with mineral oil, and heat-denatured completely. The sample was immediately transferred into a 68 °C water bath and incubated for 20 hours (long hybridization [LH]). The reaction mixture was then subjected to a streptavidin treatment followed by phenol/chloroform extraction. This process was repeated three more times. Subtracted DNA was precipitated, dissolved in 12 µl H₂O, mixed with 8 µl driver DNA and 20 µl of 2 x hybridization buffer, and subjected to a hybridization at 68°C for 2 hours (short hybridization [SH]). After removal of biotinylated double-stranded DNA, subtracted cDNA was ligated into NotI/SpeI site of chloramphenicol resistant pBCSK⁺ (Stratagene, La Jolla, CA) and transformed into ElectroMax E. *coli* DH10B cells by electroporation to generate a lung squamous cell carcinoma specific subtracted cDNA library (herein after referred to as "lung subtraction I").

A second lung squamous cell carcinoma specific subtracted cDNA library (referred to as "lung subtraction II") was generated in a similar way to the lung subtraction library I, except that eight frequently recovered genes from lung subtraction I were included in the driver DNA, and 24,000 independent clones were recovered.

To analyze the subtracted cDNA libraries, plasmid DNA was prepared from 320 independent clones, randomly picked from the subtracted lung squamous cell carcinoma specific libraries. Representative cDNA clones were further characterized by DNA sequencing with a Perkin Elmer/Applied Biosystems Division Automated Sequencer Model 373A and/or Model 377 (Foster City, CA). The cDNA sequences for sixty isolated clones are provided in SEQ ID NO: 1-60. These sequences were compared to known sequences in the gene bank using the EMBL and GenBank databases (release 96). No significant homologies were found to the sequences provided in SEQ ID NO: 2, 3, 19, 38 and 46. The sequences of SEQ ID NO: 1, 6-8, 10-13, 15, 17, 18, 20-27, 29, 30, 32, 34-37, 39-45, 47-49, 51, 52, 54, 55 and 57-59 were found to show some homology to previously identified expressed sequence tags (ESTs). The sequences of SEQ ID NO: 9, 28, 31 and 33 were found to show some homology to previously identified non-human gene sequences and the sequences of SEQ ID NO: 4, 5, 14, 50, 53, 56 and 60 were found to show some homology to gene sequences previously identified in humans.

The subtraction procedure described above was repeated using the above lung squamous cell carcinoma cDNA library as the tracer DNA, and the above normal lung tissue cDNA library and a cDNA library from normal liver and heart (constructed from a pool of one sample of each tissue as described above), plus twenty other cDNA clones that were frequently recovered in lung subtractions I and II, as the driver DNA (lung subtraction III). The normal liver and heart cDNA library contained 1.76 x 10⁶ independent colonies, with 100% of clones having inserts and the average insert size being 1600 base pairs. Ten additional clones were isolated (SEQ ID NO: 61-70). Comparison of these cDNA sequences with those in the gene bank as described above, revealed no significant homologies to the sequences provided in SEQ ID NO: 62 and 67. The sequences of SEQ ID NO: 61, 63-66, 68 and 69 were found to show some homology to previously isolated ESTs and the sequence provided in SEQ ID NO: 70 was found to show some homology to a previously identified rat gene.

In further studies, the subtraction procedure described above was repeated using the above lung squamous cell carcinoma cDNA library as the tracer DNA, and a cDNA library from a pool of normal lung, kidney, colon, pancreas, brain, resting PBMC, heart, skin and esophagus as the driver DNA, with esophagus cDNAs making up one third of the driver material. Since esophagus is enriched in normal epithelial cells, including differentiated squamous cells, this procedure is likely to enrich genes that are tumor specific rather than tissues specific. The cDNA sequences of 48 clones determined in this subtraction are provided in SEQ ID NO: 177-224. The sequences of SEQ ID NO: 177, 178, 180, 181, 183, 187, 192, 195-197, 208, 211, 212, 215, 216, 218 and 219 showed some homology to previously identified genes. The sequences of SEQ ID NO: 179, 182, 184-186, 188-191, 193, 194, 198-207, 209 210, 213, 214, 217, 220 and 224 showed some homology to previously determined ESTs. The sequence of SEQ ID NO: 221-223 showed no homology to any previously determined sequence.

### B. ISOLATION OF cDNA SEQUENCES FROM A LUNG ADENOCARCINOMA LIBRARY

A human lung adenocarcinoma cDNA expression library was constructed as described above. The library contained 3.2 x 10⁶ independent colonies, with 100% of clones having an insert and the average insert size being 1500 base pairs. Library subtraction was performed as described above using the normal lung and normal liver and heart cDNA expression libraries described above as the driver DNA. Twenty-six hundred independent clones were recovered.

Initial cDNA sequence analysis from 100 independent clones revealed many ribosomal protein genes. The cDNA sequences for fifteen clones isolated in this subtraction are provided in SEQ ID NO: 71-86. Comparison of these sequences with those in the gene bank as described above revealed no significant homologies to the sequence provided in SEQ ID NO: 84. The sequences of SEQ ID NO: 71, 73, 74, 77, 78 and 80-82 were found to show some homology to previously isolated ESTs, and the sequences of SEQ ID NO: 72, 75, 76, 79, 83 and 85 were found to show some homology to previously identified human genes.

In further studies, a cDNA library (referred to as mets3616A) was constructed from a metastatic lung adenocarcinoma. The determined cDNA sequences of 25 clones sequenced at random from this library are provided in SEQ ID NO: 255-279. The mets3616A cDNA library was subtracted against a cDNA library prepared from a pool of normal lung, liver, pancreas, skin, kidney, brain and resting PBMC. To increase the specificity of the subtraction, the driver was spiked with genes that were determined to be most abundant in the mets3616A cDNA library, such as EF1-alpha, integrin-beta and anticoagulant protein PP4, as well as with cDNAs that were previously found to be differentially expressed in subtracted lung adenocarcinoma cDNA libraries. The determined cDNA sequences of 51 clones isolated from the subtracted library (referred to as mets3616A-S1) are provided in SEQ ID NO: 280-330.

Comparison of the sequences of SEQ ID NO: 255-330 with those in the public databases revealed no significant homologies to the sequences of SEQ ID NO: 255-258, 260, 262-264, 270, 272, 275, 276, 279, 281, 287, 291, 296, 300 and 310. The sequences of SEQ ID NO: 259, 261, 265-269, 271, 273, 274, 277, 278, 282-285, 288-290, 292, 294, 297-299, 301, 303-309, 313, 314, 316, 320-324 and 326-330 showed some homology to previously identified gene sequences, while the sequences of SEQ ID NO: 280, 286, 293, 302, 310, 312, 315, 317-319 and 325 showed some homology to previously isolated expressed sequence tags (ESTs).

### EXAMPLE 2

### DETERMINATION OF TISSUE SPECIFICITY OF LUNG TUMOR POLYPEPTIDES

Using gene specific primers, mRNA expression levels for seven representative lung tumor polypeptides described in Example 1 were examined in a variety of normal and tumor tissues using RT-PCR.

Briefly, total RNA was extracted from a variety of normal and tumor tissues using Trizol reagent as described above. First strand synthesis was carried out using 2 µg of total RNA with SuperScript II reverse transcriptase (BRL Life Technologies) at 42°C for one hour. The cDNA was then amplified by PCR with gene-specific primers. To ensure the semi-quantitative nature of the RT-PCR, β-actin was used as an internal control for each of the tissues examined. 1 µl of 1:30 dilution of cDNA was employed to enable the linear range amplification of the β-actin template and was sensitive enough to reflect the differences in the initial copy numbers. Using these conditions, the β-actin levels were determined for each reverse transcription reaction from each tissue. DNA contamination was minimized by DNase treatment and by assuring a negative PCR result when using first strand cDNA that was prepared without adding reverse transcriptase.

mRNA Expression levels were examined in five different types of tumor tissue (lung squamous cell carcinoma from 3 patients, lung adenocarcinoma, colon tumor from 2 patients, breast tumor and prostate tumor), and thirteen different normal tissues (lung from 4 donors, prostate, brain, kidney, liver, ovary, skeletal muscle, skin, small intestine, stomach, myocardium, retina and testes). Using a 10-fold amount of cDNA, the antigen LST-S1-90 (SEQ ID NO: 3) was found to be expressed at high levels in lung squamous cell carcinoma and in breast tumor, and at low to undetectable levels in the other tissues examined.

The antigen LST-S2-68 (SEQ ID NO: 15) appears to be specific to lung and breast tumor, however, expression was also detected in normal kidney. Antigens LST-S1-169 (SEQ ID NO: 6) and LST-S1-133 (SEQ ID NO: 5) appear to be very abundant in lung tissues (both normal and tumor), with the expression of these two genes being decreased in most of the normal tissues tested. Both LST-S1-169 and LST-S1-133 were also expressed in breast and colon tumors. Antigens LST-S1-6 (SEQ ID NO: 7) and LST-S2-I2-5F (SEQ ID NO: 47) did not show tumor or tissue specific expression, with the expression of LST-S1-28 being rare and only detectable in a few tissues. The antigen LST-S3-7 (SEQ ID NO: 63) showed lung and breast tumor specific expression, with its message only being detected in normal testes when the PCR was performed for 30 cycles. Lower level expression was detected in some normal tissues when the cycle number was increased to 35. Antigen LST-S3-13 (SEQ ID NO: 66) was found to be expressed in 3 out of 4 lung tumors, one breast tumor and both colon tumor samples. Its expression in normal tissues was lower compared to tumors, and was only detected in 1 out of 4 normal lung tissues and in normal tissues from kidney, ovary and retina. Expression of antigens LST-S3-4 (SEQ ID NO: 62) and LST-S3-14 (SEQ ID NO: 67) was rare and did not show any tissue or tumor specificity. Consistent with Northern blot analyses, the RT-PCR results on antigen LAT-S1-A-10A (SEQ ID NO: 78) suggested that its expression is high in lung, colon, stomach and small intestine tissues, including lung and colon tumors, whereas its expression was low or undetectable in other tissues.

A total of 2002 cDNA fragments isolated in lung subtractions I, II and III, described above, were colony PCR amplified and their mRNA expression levels in lung tumor, normal lung, and various other normal and tumor tissues were determined using microarray technology (Synteni, Palo Alto, CA). Briefly, the PCR amplification products were dotted onto slides in an array format, with each product occupying a unique location in the array. mRNA was extracted from the tissue sample to be tested, reverse transcribed, and fluorescent-labeled cDNA probes were generated. The microarrays were probed with the labeled cDNA probes, the slides scanned and fluorescence intensity was measured. This intensity correlates with the hybridization intensity. Seventeen non-redundant cDNA clones showed over-expression in lung squamous tumors, with expression in normal tissues tested (lung, skin, lymph node, colon, liver, pancreas, breast, heart, bone marrow, large intestine, kidney, stomach, brain, small intestine, bladder and salivary gland) being either undetectable, or 10-fold less compared to lung squamous tumors. The determined cDNA sequences for the clone L513S are provided in SEQ ID NO: 87 and 88; those for L514S are provided in SEQ ID NO: 89 and 90; those for L516S in SEQ ID NO: 91 and 92; that for L517S in SEQ ID NO: 93; that for L519S in SEQ ID NO: 94; those for L520S in SEQ ID NO: 95 and 96; those for L521S in SEQ ID NO: 97 and 98; that for L522S in SEQ ID NO: 99; that for L523S in SEQ ID NO: 100; that for L524S in SEQ ID NO: 101; that for L525S in SEQ ID NO: 102; that for L526S in SEQ ID NO: 103; that for L527S in SEQ ID NO: 104; that for L528S in SEQ ID NO: 105; that for L529S in SEQ ID NO: 106; and those for L530S in SEQ ID NO: 107 and 108. Additionally, the full-length cDNA sequence for L530S is provided in SEQ ID NO: 151, with the corresponding amino acid sequence being provided in SEQ ID NO: 152. L530S shows homology to a splice variant of a p53 tumor suppressor homologue, p63. The cDNA sequences of 7 known isoforms of p63 are provided in SEQ ID NO: 331-337, with the corresponding amino acid sequences being provided in SEQ ID NO: 338-344, respectively.

Due to polymorphisms, the clone L531S appears to have two forms. A first determined full-length cDNA sequence for L531S is provided in SEQ ID NO: 109, with the corresponding amino acid sequence being provided in SEQ ID NO: 110. A second determined full-length cDNA sequence for L531 S is provided in SEQ ID NO: 111, with the corresponding amino acid sequence being provided in SEQ ID NO: 112. The sequence of SEQ ID NO: 111 is identical to that of SEQ ID NO: 109, except that it contains a 27 bp insertion. Similarly, L514S has two alternatively spliced forms; the first variant cDNA is listed as SEQ ID NO: 153, with the corresponding amino acid sequence being provided in SEQ ID NO: 155. The full-length cDNA for the second variant form of L514S is provided in SEQ ID NO: 154, with the corresponding amino acid sequence being provided in SEQ ID NO: 156.

Full length cloning for L524S (SEQ ID NO: 101) yielded two variants (SEQ ID NO: 163 and 164) with the corresponding amino acid sequences of SEQ ID NO: 165 and 166, respectively. Both variants have been shown to encode parathyroid hormone-related peptide.

Attempts to isolate the full-length cDNA for L519S, resulted in the isolation of the extended cDNA sequence provided in SEQ ID NO: 173, which contains a potential open reading frame. The amino acid sequence encoded by the sequence of SEQ ID NO: 173 is provided in SEQ ID NO: 174. Additionally, the full-length cDNA sequence for the clone of SEQ ID NO: 100 (known as L523S), a known gene, is provided in SEQ ID NO: 175, with the corresponding amino acid sequence being provided in SEQ ID NO: 176. In further studies, a full-length cDNA sequence for L523S was isolated from a L523S-positive tumor cDNA library by PCR amplification using gene specific primers designed from the sequence of SEQ ID NO: 175. The determined full-length cDNA sequence is provided in SEQ ID NO: 347. The amino acid sequence encoded by this sequence is provided in SEQ ID NO: 348. This protein sequence differs from the previously published protein sequence at two amino acid positions, namely at positions 158 and 410.

Comparison of the sequences of L514S and L531S (SEQ ID NO: 87 and 88, and 109, respectively) with those in the gene bank, as described above, revealed no significant homologies to known sequences. The sequences of L513S, L516S, L517S, L519S, L520S and L530S (SEQ ID NO: 87 and 88, 91 and 92, 93, 94, 95 and 96, 107 and 108, respectively) were found to show some homology to previously identified ESTs. The sequences of L521S, L522S, L523S, L524S, L525S, L526S, L527S, L528S and L529S (SEQ ID NO: 97 and 98, 99, 99, 101, 102, 103, 104, 105, and 106, respectively) were found to represent known genes. The determined full-length cDNA sequence for L520S is provided in SEQ ID NO: 113, with the corresponding amino acid sequence being provided in SEQ ID NO: 114. Subsequent microarray analysis showed L520S to be overexpressed in breast tumors in addition to lung squamous tumors.

Further analysis demonstrated that L529S (SEQ ID NO: 106 and 115), L525S (SEQ ID NO: 102 and 120) and L527S (SEQ ID NO: 104) are cytoskeletal components and potentially squamous cell specific proteins. L529S is connexin 26, a gap junction protein. It was found to be highly expressed in one lung squamous tumor, referred to as 9688T, and moderately over-expressed in two others. However, lower level expression of connexin 26 is also detectable in normal skin, colon, liver and stomach. The over-expression of connexin 26 in some breast tumors has been reported and a mutated form of L529S may result in over-expression in lung tumors. L525S is plakophilin 1, a desmosomal protein found in plaque-bearing adhering junctions of the skin. Expression levels for L525S mRNA was highly elevated in three out of four lung squamous tumors tested, and in normal skin. L527S has been identified as keratin 6 isoform, type II 58 Kd keratin and cytokeratin 13, and shows over-expression in squamous tumors and low expression in normal skin, breast and colon tissues. Keratin and keratin-related genes have been extensively documented as potential markers for lung cancer including CYFRA2.1 (Pastor, A., et al, Eur. Respir. J., 10:603-609, 1997). L513S (SEQ ID NO: 87 and 88) shows moderate over-expression in several tumor tissues tested, and encodes a protein that was first isolated as a pemphigus vulgaris antigen.

L520S (SEQ ID NO: 95 and 96) and L521S (SEQ ID NO: 97 and 98) are highly expressed in lung squamous tumors, with L520S being up-regulated in normal salivary gland and L521 S being over-expressed in normal skin. Both belong to a family of small proline rich proteins and represent markers for fully differentiated squamous cells. L521 S has been described as a specific marker for lung squamous tumor (Hu, R., et al, Lung Cancer, 20:25-30, 1998). L515S (SEQ ID NO: 162) encodes IGF-β2 and L516S is an aldose reductase homologue. Both are moderately expressed in lung squamous tumors and in normal colon. Notably, L516S (SEQ ID NO: 91 and 92) is up-regulated in metastatic tumors but not primary lung adenocarcinoma, an indication of its potential role in metatasis and a potential prognostic marker. L522S (SEQ ID NO: 99) is moderately over-expressed in lung squamous tumors with minimum expression in normal tissues. L522S has been shown to belong to a class IV alcohol dehydrogenase, ADH7, and its expression profile suggests it is a squamous cell specific antigen. L523S (SEQ ID NO: 100) is moderately over-expressed in lung squamous tumor, human pancreatic cancer cell lines and pancreatic cancer tissues, suggesting this gene may be a shared antigen between pancreatic and lung squamous cell cancer.

L524S (SEQ ID NO: 101) is over-expressed in the majority of squamous tumors tested and is homologous with parathyroid hormone-related peptide (PTHrP), which is best known to cause humoral hypercalcaemia associated with malignant tumors such as leukemia, prostate and breast cancer. It is also believed that PTHrP is most commonly associated with squamous carcinoma of lung and rarely with lung adenocarcinoma (Davidson, L.A., et al, J. Pathol., 178: 398-401, 1996). L528S (SEQ ID NO: 105) is highly over-expressed in two lung squamous tumors with moderate expression in two other squamous tumors, one lung adenocarcinoma and some normal tissues, including skin, lymph nodes, heart, stomach and lung. It encodes the NMB gene that is similar to the precursor of melanocyte specific gene Pmel17, which is reported to be preferentially expressed in low-metastatic potential melanoma cell lines. This suggests that L528S may be a shared antigen in both melanoma and lung squamous cell carcinoma. L526S (SEQ ID NO: 103) was overexpressed in all lung squamous cell tumor tissues tested and has been shown to share homology with a gene (ATM) in which a mutation causes ataxia telangiectasia, a genetic disorder in humans causing a predisposition to cancer, among other symptoms. ATM encodes a protein that activates a p53 mediated cell-cycle checkpoint through direct binding and phosphorylation of the p53 molecule. Approximately 40% of lung cancers are associated with p53 mutations, and it is speculated that over-expression of ATM is a result of compensation for loss of p53 function, but it is unknown whether over-expression is the cause of result of lung squamous cell carcinoma. Additionally, expression of L526S (ATM) is also detected in a metastatic but not lung adenocarcinoma, suggesting a role in metastasis.

Expression of L523S (SEQ ID NO: 175), was examined by real time RT-PCR as described above. In a first study using a panel of lung squamous tumors, L523S was found to be expressed in 4/7 lung squamous tumors, 2/3 head and neck squamous tumors and 2/2 lung adenocarcinomas, with low level expression being observed in skeletal muscle, soft palate and tonsil. In a second study using a lung adenocarcinoma panel, expression of L523S was observed in 4/9 primary adenocarcinomas, 2/2 lung plural effusions, 1/1 metastatic lung adenocarcinomas and 2/2 lung squamous tumors, with little expression being observed in normal tissues.

Expression of L523S in lung tumors and various normal tissues was also examined by Northern blot analysis, using standard techniques. In a first study, L523S was found to be expressed in a number of lung adenocarcinomas and squamous cell carcinomas, as well as normal tonsil. No expression was observed in normal lung. In a second study using a normal tissue blot (referred to as HB-12) from Clontech, no expression was observed in brain, skeletal muscle, colon, thymus, spleen, kidney, liver, small intestine, lung or PBMC, although there was strong expression in placenta.

### EXAMPLE 3

### ISOLATION AND CHARACTERIZATION OF LUNG TUMOR POLYPEPTIDES BY PCR-BASED SUBTRACTION

Eight hundred and fifty seven clones from a cDNA subtraction library, containing cDNA from a pool of two human lung squamous tumors subtracted against eight normal human tissue cDNAs including lung, PBMC, brain, heart, kidney, liver, pancreas, and skin, (Clontech, Palo Alto, CA) were derived and submitted to a first round of PCR amplification. This library was subjected to a second round of PCR amplification, following the manufacturer's protocol. The resulting cDNA fragments were subcloned into the P7-Adv vector (Clontech, Palo Alto, CA) and transformed into DH5α *E. coli* (Gibco, BRL). DNA was isolated from independent clones and sequenced using a Perkin Elmer/Applied Biosystems Division Automated Sequencer Model 373A.

One hundred and sixty two positive clones were sequenced. Comparison of the DNA sequences of these clones with those in the EMBL and GenBank databases, as described above, revealed no significant homologies to 13 of these clones, hereinafter referred to as Contigs 13, 16, 17, 19, 22, 24, 29, 47, 49, 56-59. The determined cDNA sequences for these clones are provided in SEQ ID NO: 125, 127-129, 131-133, 142, 144, 148-150, and 157, respectively. Contigs 1, 3-5, 7-10, 12, 11, 15, 20, 31, 33, 38, 39, 41, 43, 44, 45, 48, 50, 53, 54 (SEQ ID NO: 115-124, 126, 130, 134-141, 143, 145-147, respectively) were found to show some degree of homology to previously identified DNA sequences. Contig 57 (SEQ ID NO: 149) was found to represent the clone L519S (SEQ ID NO: 94) disclosed in US. Patent Application No. 09/123,912, filed July 27, 1998. To the best of the inventors' knowledge, none of these sequences have been previously shown to be differentially over-expressed in lung tumors.

mRNA expression levels for representative clones in lung tumor tissues, normal lung tissues (n=4), resting PBMC, salivary gland, heart, stomach, lymph nodes, skeletal muscle, soft palate, small intestine, large intestine, bronchial, bladder, tonsil, kidney, esophagus, bone marrow, colon, adrenal gland, pancreas, and skin (all derived from human) were determined by RT-PCR as described above. Expression levels using microarray technology, as described above, were examined in one sample of each tissue type unless otherwise indicated.

Contig 3 (SEQ ID NO: 116) was found to be highly expressed in all head and neck squamous cell tumors tested (17/17), and expressed in the majority (8/12) of lung squamous tumors, (high expression in 7/12, moderate in 2/12, and low in 2/12), while showing negative expression for 2/4 normal lung tissues and low expression in the remaining two samples. Contig 3 showed moderate expression in skin and soft palate, and lowered expression levels in resting PBMC, large intestine, salivary gland, tonsil, pancreas, esophagus, and colon. Contig 11 (SEQ ID NO: 124) was found to be expressed in all head and neck squamous cell tumors tested (17/17), with high levels of expression being seen in 14/17 tumors, and moderately levels of expression being seen in 3/17 tumors. Additionally, high expression was seen in 3/12 lung squamous tumors and moderate expression in 4/12 lung squamous tumors. Contig 11 was negative for 3/4 normal lung samples, with the remaining sample having only low expression. Contig 11 showed low to moderate reactivity to salivary gland, soft palate, bladder, tonsil, skin, esophagus, and large intestine. Contig 13 (SEQ ID NO: 125) was found to be expressed in all head and neck squamous cell tumors tested (17/17), with high expression in 12/17, and moderate expression in 5/17. Contig 13 was expressed in 7/12 lung squamous tumors, with high expression in 4/12 and moderate expression in three samples. Analysis of normal lung samples showed negative expression for 2/4 and low to moderate expression in the remaining two samples. Contig 13 showed low to moderate reactivity to resting PBMC, salivary gland, bladder, pancreas, tonsil, skin, esophagus, and large intestine, as well as high expression in soft palate. Subsequent full-length cloning efforts revealed that contig 13 (also known as L761P) maps to the 3' untranslated region of the hSec10p gene. The full-length sequence for this gene is set forth in SEQ ID NO: 368, and encodes the protein set forth in SEQ ID NO: 369.

Contig 16 (SEQ ID NO: 127) was found to be moderately expressed in several head and neck squamous cell tumors (6/17) and one lung squamous tumor, while showing no expression in any normal lung samples tested. Contig 16 showed low reactivity to resting PBMC, large intestine, skin, salivary gland, and soft palate. Contig 17 (SEQ ID NO: 128) was shown to be expressed in all head and neck squamous cell tumors tested (17/17) (highly expressed in 5/17, and moderately expressed in 12/17). Determination of expression levels in lung squamous tumors showed one tumor sample with high expression and 3/12 with moderate levels. Contig 17 was negative for 2/4 normal lung samples, with the remaining samples having only low expression. Additionally, low level expression was found in esophagus and soft palate. Contig 19 (SEQ ID NO: 129) was found to be expressed in most head and neck squamous cell tumors tested (11/17); with two samples having high expression levels, 6/17 showing moderate expression, and low expression being found in 3/17. Testing in lung squamous tumors revealed only moderate expression in 3/12 samples. Expression levels in 2/4 of normal lung samples were negative, the two other samples having only low expression. Contig 19 showed low expression levels in esophagus, resting PBMC, salivary gland, bladder, soft palate and pancreas.

Contig 22 (SEQ ID NO: 131), was shown to be expressed in most head and neck squamous cell tumors tested (13/17) with high expression in four of these samples, moderate expression in 6/17, and low expression in 3/17. Expression levels in lung squamous tumors were found to be moderate to high for 3/12 tissues tested, with negative expression in two normal lung samples and low expression in two other samples (n=4). Contig 22 showed low expression in skin, salivary gland and soft palate. Similarly, Contig 24 (SEQ ID NO: 132) was found to be expressed in most head and neck squamous cell tumors tested (13/17) with high expression in three of these samples, moderate expression in 6/17, and low expression in 4/17. Expression levels in lung squamous tumors were found to be moderate to high for 3/12 tissues tested, with negative expression for three normal lung samples and low expression in one sample (n=4). Contig 24 showed low expression in skin, salivary gland and soft palate. Contig 29 (SEQ ID NO: 133) was expressed in nearly all head and neck squamous cell tumors tested (16/17): highly expressed in 4/17, moderately expressed in 11/17, with low expression in one sample. Also, it was moderately expressed in 3/12 lung squamous tumors, while being negative for 2/4 normal lung samples. Contig 29 showed low to moderate expression in large intestine, skin, salivary gland, pancreas, tonsil, heart and soft palate. Contig 47 (SEQ ID NO: 142) was expressed in most head and neck squamous cell tumors tested (12/17): moderate expression in 10/17, and low expression in two samples. In lung squamous tumors, it was highly expressed in one sample and moderately expressed in two others (n=13). Contig 47 was negative for 2/4 normal lung samples, with the remaining two samples having moderate expression. Also, Contig 47 showed moderate expression in large intestine, and pancreas, and low expression in skin, salivary gland, soft palate, stomach, bladder, resting PBMC, and tonsil.

Contig 48 (SEQ ID NO: 143) was expressed in all head and neck squamous cell tumors tested (17/17): highly expressed in 8/17 and moderately expressed in 7/17, with low expression in two samples. Expression levels in lung squamous tumors were high to moderate in three samples (n=13). Contig 48 was negative for one out of four normal lung samples, the remaining showing low or moderate expression. Contig 48 showed moderate expression in soft palate, large intestine, pancreas, and bladder, and low expression in esophagus, salivary gland, resting PBMC, and heart. Contig 49 (SEQ ID NO: 144) was expressed at low to moderate levels in 6/17 head and neck squamous cell tumors tested. Expression levels in lung squamous tumors were moderate in three samples (n=13). Contig 49 was negative for 2/4 normal lung samples, the remaining samples showing low expression. Moderate expression levels in skin, salivary gland, large intestine, pancreas, bladder and resting PBMC were shown, as well as low expression in soft palate, lymph nodes, and tonsil. Contig 56 (SEQ ID NO: 148) was expressed in low to moderate levels in 3/17 head and neck squamous cell tumors tested, and in lung squamous tumors, showing low to moderate levels in three out of thirteen samples. Notably, low expression levels were detected in one adenocarcinoma lung tumor sample (n=2). Contig 56 was negative for 3/4 normal lung samples, and showed moderate expression levels in only large intestine, and low expression in salivary gland, soft palate, pancreas, bladder, and resting PBMC. Contig 58, also known as L769P, (SEQ ID NO: 150) was expressed at moderate levels in 11/17 head and neck squamous cell tumors tested and low expression in one additional sample. Expression in lung squamous tumors showed low to moderate levels in three out of thirteen samples. Contig 58 was negative for 3/4 normal lung samples, with one sample having low expression. Moderate expression levels in skin, large intestine, and resting PBMC were demonstrated, as well as low expression in salivary gland, soft palate, pancreas, and bladder. Contig 59 (SEQ ID NO: 157) was expressed in some head, neck, and lung squamous tumors. Low level expression of Contig 59 was also detected in salivary gland and large intestine.

The full-length cDNA sequence for Contig 22, also referred to as L763P, is provided in SEQ ID NO: 158, with the corresponding amino acid sequence being provided in SEQ ID NO: 159. Real-time RT-PCR analysis of L763P revealed that it is highly expressed in 3/4 lung squamous tumors as well as 4/4 head and neck squamous tumors, with low level expression being observed in normal brain, skin, soft pallet and trachea. Subsequent database searches revealed that the sequence of SEQ ID NO: 158 contains a mutation, resulting in a frameshift in the corresponding protein sequence. A second cDNA sequence for L763P is provided in SEQ ID NO: 345, with the corresponding amino acid sequence being provided in SEQ ID NO: 346. The sequences of SEQ ID NO: 159 and 346 are identical with the exception of the C-terminal 33 amino acids of SEQ ID NO: 159.

The full-length cDNA sequence incorporating Contigs 17, 19, and 24, referred to as L762P, is provided in SEQ ID NO: 160, with the corresponding amino acid sequence being provided in SEQ ID NO: 161. Further analysis of L762P has determined it to be a type I membrane protein and two additional variants have been sequenced. Variant 1 (SEQ ID NO: 167, with the corresponding amino acid sequence in SEQ ID NO: 169) is an alternatively spliced form of SEQ ID NO: 160 resulting in deletion of 503 nucleotides, as well as deletion of a short segment of the expressed protein. Variant 2 (SEQ ID NO: 168, with the corresponding amino acid sequence in SEQ ID NO: 170) has a two nucleotide deletion at the 3' coding region in comparison to SEQ ID NO: 160, resulting in a secreted form of the expressed protein. Real-time RT-PCR analysis of L762P revealed that is over-expressed in 3/4 lung squamous tumors and 4/4 head & neck tumors, with low level expression being observed in normal skin, soft pallet and trachea.

An epitope of L762P was identified as having the sequence KPGHWTYTLNNTHHSLQALK (SEQ ID NO: 382), which corresponds to amino acids 571-590 of SEQ ID NO:161.

The full-length cDNA sequence for contig 56 (SEQ ID NO: 148), also referred to as L773P, is provided in SEQ ID NO: 171, with the amino acid sequence in SEQ ID NO: 172. L773P was found to be identical to dihydroxyl dehydrogenase at the 3' portion of the gene, with divergent 5' sequence. As a result, the 69 N-terminal amino acids are unique. The cDNA sequence encoding the 69 N-terminal amino acids is provided in SEQ ID NO: 349, with the N-terminal amino acid sequence being provided in SEQ ID NO: 350. Real-time PCR revealed that L773P is highly expressed in lung squamous tumor and lung adenocarcinoma, with no detectable expression in normal tissues. Subsequent Northern blot analysis of L773P demonstrated that this transcript is differentially over-expressed in squamous tumors and detected at approximately 1.6 Kb in primary lung tumor tissue and approximately 1.3 Kb in primary head and neck tumor tissue.

Subsequent microarray analysis has shown Contig 58, also referred to as L769S (SEQ ID NO: 150), to be overexpressed in breast tumors in addition to lung squamous tumors.

### EXAMPLE 4

### ISOLATION AND CHARACTERIZATION OF LUNG TUMOR POLYPEPTIDES BY PCR-BASED SUBTRACTION

Seven hundred and sixty clones from a cDNA subtraction library, containing cDNA from a pool of two human lung primary adenocarcinomas subtracted against a pool of nine normal human tissue cDNAs including skin, colon, lung, esophagus, brain, kidney, spleen, pancreas and liver, (Clontech, Palo Alto, CA) were derived and submitted to a first round of PCR amplification. This library (referred to as ALT-1) was subjected to a second round of PCR amplification, following the manufacturer's protocol. The expression levels of these 760 cDNA clones in lung tumor, normal lung, and various other normal and tumor tissues, were examined using microarray technology (Incyte, Palo Alto, CA). Briefly, the PCR amplification products were dotted onto slides in an array format, with each product occupying a unique location in the array. mRNA was extracted from the tissue sample to be tested, reverse transcribed, and fluorescent-labeled cDNA probes were generated. The microarrays were probed with the labeled cDNA probes, the slides scanned and fluorescence intensity was measured. This intensity correlates with the hybridization intensity.. A total of 118 clones, of which 55 were unique, were found to be over-expressed in lung tumor tissue, with expression in normal tissues tested (lung, skin, lymph node, colon, liver, pancreas, breast, heart, bone marrow, large intestine, kidney, stomach, brain, small intestine, bladder and salivary gland) being either undetectable, or at significantly lower levels. One of these clones, having the sequence as provided in SEQ ID NO:420 (clone #19014), shows homology to a previously identified clone, L773P. Clone L773P has the full-length cDNA sequence provided in SEQ ID NO:171 and the amino acid sequence provided in SEQ ID NO:172 The isolation of clone #19014 is also described in US 2003 064947.

### EXAMPLE 5

### SYNTHESIS OF POLYPEPTIDES

Polypeptides may be synthesized on a Perkin Elmer/Applied Biosystems Division 430A peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation, binding to an immobilized surface, or labeling of the peptide. Cleavage of the peptides from the solid support is carried out using the following cleavage mixture: trifluoroacetic acid:ethanedithiol:thioanisole:water:phenol (40:1:2:2:3). After cleaving for 2 hours, the peptides are precipitated in cold methyl-t-butyl-ether. The peptide pellets are then dissolved in water containing 0.1% trifluoroacetic acid (TFA) and lyophilized prior to purification by C18 reverse phase HPLC. A gradient of 0%-60% acetonitrile (containing 0.1% TFA) in water (containing 0.1% TFA) may be used to elute the peptides. Following lyophilization of the pure fractions, the peptides are characterized using electrospray or other types of mass spectrometry and by amino acid analysis.

### EXAMPLE 6

### PREPARATION OF ANTIBODIES AGAINST LUNG CANCER ANTIGENS

Polyclonal antibodies against the lung cancer antigens L514S, L528S, L531S, L523 and L773P (SEQ ID NO: 155, 225, 112, 176 and 171, respectively) were prepared as follows.

Rabbits were immunized with recombinant protein expressed in and purified from *E*. *coli* as described below. For the initial immunization, 400 µg of antigen combined with muramyl dipeptide (MDP) was injected subcutaneously (S.C.). Animals were boosted S.C. 4 weeks later with 200 µg of antigen mixed with incomplete Freund's Adjuvant (IFA). Subsequent boosts of 100 µg of antigen mixed with IFA were injected S.C. as necessary to induce high antibody titer responses. Serum bleeds from immunized rabbits were tested for antigen-specific reactivity using ELISA assays with purified protein. Polyclonal antibodies against L514S, L528S, L531S, L523S and L773P were affinity purified from high titer polyclonal sera using purified protein attached to a solid support.

Immunohistochemical analysis using polyclonal antibodies against L514S was performed on a panel of 5 lung tumor samples, 5 normal lung tissue samples and normal colon, kidney, liver, brain and bone marrow. Specifically, tissue samples were fixed in formalin solution for 24 hours and embedded in paraffin before being sliced into 10 micron sections. Tissue sections were permeabilized and incubated with antibody for 1 hr. HRP-labeled anti-mouse followed by incubation with DAB chromogen was used to visualize L514S immunoreactivity. L514S was found to be highly expressed in lung tumor tissue with little or no expression being observed in normal lung, brain or bone marrow. Light staining was observed in colon (epithelial crypt cells positive) and kidney (tubules positive). Staining was seen in normal liver but no mRNA has been detected in this tissue making this result suspect.

Using the same procedure, immunohistochemical analysis using polyclonal antibodies against L528S demonstrated staining in lung tumor and normal lung samples, light staining in colon and kidney, and no staining in liver and heart.

Immunohistochemical analysis using polyclonal antibodies against L531S demonstrated staining in lung tumor samples, light membrane staining in most normal lung samples, epithelial staining in colon, tubule staining in kidney, ductal epithelial staining in liver and no staining in heart.

Immunohistochemical analysis using polyclonal antibodies against L523S demonstrated staining in all lung cancer samples tested but no staining in normal lung, kidney, liver, colon, bone marrow or cerebellum.

Generation of polyclonal anti-sera against L762P (SEQ ID NO: 169 and 170) was performed as follows. 400 micrograms of lung antigen was combined with 100 micrograms of muramyldipeptide (MDP). An equal volume of Incomplete Freund's Adjuvant (IFA) was added and then mixed until an emulsion was formed. Rabbits were injected subcutaneously (S.C.). After four weeks the animals were injected S.C. with 200 micrograms of antigen mixed with an equal volume of IFA. Every four weeks animals were boosted with 100 micrograms of antigen. Seven days following each boost the animal was bled. Sera was generated by incubating the blood at 4°C for 12-24 hours followed by centrifugation.

Characterization of polyclonal antisera was carried out as follows. Ninety-six well plates were coated with antigen by incubing with 50 microliters (typically 1 microgram) at 4°C for 20 hrs. 250 microliters of BSA blocking buffer was added to the wells and incubated at room temperature for 2 hrs. Plates were washed 6 times with PBS/0.01% Tween. Rabbit sera was diluted in PBSand 50 microliters of diluted sera was added to each well and incubated at room temperature for 30 min. Plates were washed as described above before addition of 50 microliters of goat anti-rabbit horse radish peroxidase (HRP) at a 1:10000 dilution and incubation at room temperature for 30 min. Plates were washed as described above and 100µl of TMB Microwell Peroxidase Substrate was added to each well. Following a 15 minute incubation in the dark at room temperature, the colorimetric reaction was stopped with 100µl 1N H₂SO₄ and read immediately at 450 nm. Antisera showed strong reactivity to antigen L762P.

Immunohistochemical analysis using polyclonal antibodies against L762P demonstrated staining in all lung cancer samples tested, some light staining in the bronchiole epithelium of normal lung, tubule staining in kidney, light epithelial staining in colon and no staining in heart or liver.

In order to evaluate L773P protein expression in various tissues, immunohistochemistry (IHC) analysis was performed using an affinity purified L773P polyclonal antibody. Briefly, tissue samples were fixed in formalin solution for 12-24 hrs and embedded in paraffin before being sliced into 8 micron sections. Steam heat induced epitope retrieval (SHIER) in 0.1 M sodiuym citrate buffer (pH 6.0) was used for optimal staining conditions. Sections were incubated with 10% serum/PBS for 5 minutes. Primary antibody was added to each section for 25 minutes at indicated concentrations followed by 25 minute incubation with either anti-rabbit or anti-mouse biotinylated antibody. Endogenous peroxidase activitiy was blocked by three 1.5 minute incubations with hydrogen peroxidase. The avidin biotin complex/horse radish peroxidase (ABC/HRP) system was used along with DAB chromogen to visualize L773P expression. Slides were counterstainied with hematoxylin to visualize cell nuclei. Using this approach, L773P protein was detected in 6/8 lung tumors, 4/6 normal lung samples (very light staining in some cases), 1/1 kidney samples (very light staining), 0/1 heart samples, 1/1 colon samples (very light staining) and 0/1 liver samples.

### EXAMPLE 7

### PEPTIDE PRIMING OF MICE AND PROPAGATION OF CTL LINES

Immunogenic peptides from the lung cancer antigen L762P (SEQ ID NO: 161) for HLA-A2/K^{b}-restricted CD8+ T cells were identified as follows.

The location of HLA-A2 binding peptides within the lung cancer antigen L762P (SEQ ID NO: 161) was predicted using a computer program which predicts peptides sequences likely to being to HLA-A*0201 by fitting to the known peptide binding motif for HLA-A*0201 (Rupert et al. (1993) Cell 74:929; Rammensee et al. (1995) Immunogenetics 41:178-228). A series of 19 synthetic peptides corresponding to a selected subset of the predicted HLA-A*0201 binding peptides was prepared as described above.

Mice expressing the transgene for human HLA A2/K^{b} (provided by Dr L. Sherman, The Scripps Research Institute, La Jolla, CA) were immunized with the synthetic peptides, as described by Theobald et al., Proc. Natl. Acad. Sci. USA 92:11993-11997, 1995, with the following modifications. Mice were immunized with 50µg of L726P peptide and 120µg of an I-A^{b} binding peptide derived from hepatitis B virus protein emulsified in incomplete Freund's adjuvant. Three weeks later these mice were sacrificed and single cell suspensions prepared. Cells were then resuspended at 7 x 10⁶ cells/ml in complete media (RPMI-1640; Gibco BRL, Gaithersburg, MD) containing 10% FCS, 2mM Glutamine (Gibco BRL), sodium pyruvate (Gibco BRL), non-essential amino acids (Gibco BRL), 2 x 10⁻⁵ M 2-mercaptoethanol, 50U/ml penicillin and streptomycin, and cultured in the presence of irradiated (3000 rads) L762P peptide- (5µg/ml) and 10mg/ml B₂-microglobulin- (3 µg/ml) LPS blasts (A2 transgenic spleens cells cultured in the presence of 7µg/ml dextran sulfate and 25µg/ml LPS for 3 days). After six days, cells (5 x 10⁵/ml) were restimulated with 2.5 x 10⁶/ml peptide-pulsed irradiated (20,000 rads) EL4A2Kb cells (Sherman et al, Science 258:815-818, 1992) and 5 x 10⁶/ml irradiated (3000 rads) A2/K^{b}-transgenic spleen feeder cells. Cells were cultured in the presence of 10U/ml IL-2. Cells were restimulated on a weekly basis as described, in preparation for cloning the line.

Peptide-specific cell lines were cloned by limiting dilution analysis with irradiated (20,000 rads) L762P peptide-pulsed EL4 A2Kb tumor cells (1 x 10⁴ cells/well) as stimulators and irradiated (3000 rads) A2/K^{b}-transgenic spleen cells as feeders (5 x 10⁵ cells/ well) grown in the presence of 10U/ml IL-2. On day 7, cells were restimulated as before. On day 14, clones that were growing were isolated and maintained in culture.

Cell lines specific for the peptides L762P-87 (SEQ ID NO: 226; corresponding to amino acids 87-95 of SEQ ID NO: 161), L762P-145 (SEQ ID NO: 227; corresponding to amino acids 145-153 of SEQ ID NO: 161), L762P-585 (SEQ ID NO: 228; corresponding to amino acids 585-593 of SEQ ID NO: 161), L762P-425 (SEQ ID NO: 229; corresponding to amino acids 425-433 of SEQ ID NO: 161), L762P(10)-424 (SEQ ID NO: 230; corresponding to amino acids 424-433 of SEQ ID NO: 161) and L762P(10)-458 (SEQ ID NO: 231; corresponding to amino acids 458-467 of SEQ ID NO: 161) demonstrated significantly higher reactivity (as measured by percent specific lysis) against L762P peptide-pulsed EL4-A2/K^{b} tumor target cells than control peptide-pulsed EL4-A2/K^{b} tumor target cells.

### EXAMPLE 8

### IDENTIFICATION OF CD4 IMMUNOGENIC T CELL EPITOPES DERIVED FROM THE LUNG CANCER ANTIGEN L762P

CD4 T cell lines specific for the antigen L762P (SEQ ID NO: 161) were generated as follows.

A series of 28 overlapping peptides were synthesized that spanned approximately 50% of the L762P sequence. For priming, peptides were combined into pools of 4-5 peptides, pulsed at 20 micrograms/ml into dendritic cells for 24 hours. The dendritic cells were then washed and mixed with positively selected CD4+ T cells in 96 well U-bottomed plates. Forty cultures were generated for each peptide pool. Cultures were restimulated weekly with fresh dendritic cells loaded with peptide pools. Following a total of 3 stimulation cycles, cells were rested for an additional week and tested for specificity to antigen presenting cells (APC) pulsed with peptide pools using interferon-gamma ELISA and proliferation assays. For these assays, adherent monocytes loaded with either the relevant peptide pool or an irrelevant peptide were used as APC. T cell lines that appeared to specifically recognize L762P peptide pools both by cytokine release and proliferation were identified for each pool. Emphasis was placed on identifying T cells with proliferative responses. T cell lines that demonstrated either both L762P-specific cytokine secretion and proliferation, or strong proliferation alone were further expanded to be tested for recognition of individual peptides from the pools, as well as for recognition of recombinant L762P. The source of recombinant L762P was *E. coli,* and the material was partially purified and endotoxin positive. These studies employed 10 micrograms of individual peptides, 10 or 2 micrograms of an irrelevant peptide, and 2 or 0.5 micrograms of either L762P protein or an irrelevant, equally impure, *E. coli* generated recombinant protein. Significant interferon-gamma production and CD4 T cell proliferation was induced by a number of L762P-derived peptides in each pool. The amino acid sequences for these peptides are provided in SEQ ID NO: 232-251. These peptides correspond to amino acids 661-680, 676-696, 526-545, 874-893, 811-830, 871-891, 856-875, 826-845, 795-815, 736-755, 706-725, 706-725, 691-710, 601-620, 571-590, 556-575, 616-635, 646-665, 631-650, 541-560 and 586-605, respectively, of SEQ ID NO: 161.

CD4 T cell lines that demonstrated specificity for individual L762P-derived peptides were further expanded by stimulation with the relevant peptide at 10 micrograms/ml. Two weeks post-stimulation, T cell lines were tested using both proliferation and IFN-gamma ELISA assays for recognition of the specific peptide. A number of previously identified T cells continued to demonstrate L762P-peptide specific activity. Each of these lines was further expanded on the relevant peptide and, following two weeks of expansion, tested for specific recognition of the L762P-peptide in titration experiments, as well as for recognition of recombinant E. coli-derived L762P protein. For these experiments, autologous adherent monocytes were pulsed with either the relevant L762P-derived peptide, an irrelevant mammaglobin-derived peptide, recombinant E. coli-derived L762P (approx. 50% pure), or an irrelevant E. coli-derived protein. The majority of T cell lines were found to show low affinity for the relevant peptide, since specific proliferation and IFN-gamma ratios dramatically decreased as L762P peptide was diluted. However, four lines were identified that demonstrated significant activity even at 0.1 micrograms/ml peptide. Each of these lines (referred to as A/D5, D/F5, E/A7 and E/B6) also appeared to specifically proliferate in response to the E. coli-derived L762P protein preparation, but not in response to the irrelevant protein preparation. The amino acid sequences of the L762P-derived peptides recognized by these lines are provided in SEQ ID NO: 234, 249, 236 and 245, respectively. No protein specific IFN-gamma was detected for any of the lines. Lines A/D5, E/A7 and E/B6 were cloned on autologous adherent monocytes pulsed with the relevant peptide at 0.1 (A/D5 and E/A7) or 1 (D/F5) microgram/ml. Following growth, clones were tested for specificity for the relevant peptide. Numerous clones specific for the relevant peptide were identified for lines A/D5 and E/A7.

### EXAMPLE 9

### PROTEIN EXPRESSION OF LUNG TUMOR-SPECIFIC ANTIGENS

### a) Expression of L514S in E. coli

The lung tumor antigen L514S (SEQ ID NO: 89) was subcloned into the expression vector pE32b at NcoI and NotI sites, and transformed into E. *coli* using standard techniques. The protein was expressed from residues 3-153 of SEQ ID NO: 89. The expressed amino acid sequence and the corresponding DNA sequence are provided in SEQ ID NO: 252 and 253, respectively.

### b) Expression of L762P

Amino acids 32-944 of the lung tumor antigen L762P (SEQ ID NO: 161), with a 6X His Tag, were subcloned into a modified pET28 expression vector, using kanamycin resistance, and transformed into BL21 CodonPlus using standard techniques. Low to moderate levels of expression were observed. The determined DNA sequence of the L762P expression construct is provided in SEQ ID NO: 254.

### EXAMPLE 10

### IDENTIFICATION OF MHC CLASS II RESTRICTING ALLELE FOR L762P PEPTIDE-SPECIFIC RESPONSES

A panel of HLA mismatched antigen presenting cells (APC) were used to identify the MHC class II restricting allele for the L762P-peptide specific responses of CD4 T cell clones derived from lines that recognized L762P peptide and recombinant protein. Clones from two lines, AD-5 and EA-7, were tested as described below. The AD-5 derived clones were found to be restricted by the HLA-DRB-1101 allele, and an EA-7 derived clone was found to be restricted by the HLA DRB-0701 or DQB1-0202 allele. Identification of the restriction allele allows targeting of vaccine therapies using the defined peptide to individuals that express the relevant class II allele. Knowing the relevant restricting allele will also enable clinical monitoring for responses to the defined peptide since only individuals that express the relevant allele will be monitored.

CD4 T cell clones derived from line AD-5 and EA-7 were stimulated on autologous APC pulsed with the specific peptide at 10 µg/ml, and tested for recognition of autologous APC (from donor D72) as well as against a panel of APC partially matched with D72 at class II alleles. Table 2 shows the HLA class typing of the APC tested. Adherent monocytes (generated by 2 hour adherence) from four different donors, referred to as D45, D187, D208, and D326, were used as APC in these experiments. Autologous APC were not included in the experiment. Each of the APC were pulsed with the relevant peptide (5a for AD-5 and 3e for 3A-7) or the irrelevant mammoglobin peptide at 10 µg/ml, and cultures were established for 10,000 T cells and about 20,000 APC/well. As shown in Table 3, specific proliferation and cytokine production could be detected only when partially matched donor cells were used as APC. Based on the MHC typing analysis, these results strongly suggest that the restricting allele for the L762-specific response of the AD-5 derived clones is HLA-DRB-1101 and for the EA-7 derived clone the restricting allele is HLA DRB-0701 or DQB1-0202.

**Table 2 - HLA Typing of APC**

| DONOR | DR | DR | DQ | DQ |
|---|---|---|---|---|
| D72 | B1-1101 | B1-0701 | B1-0202 | B1-0301 |
| D45 | -3 | -15 | B1-0201 | B1-0602 |
| D187 | -4 | -15 | -1 | -7 |
| D208 | B1-1101 | B1-0407 | -3 | -3 |
| D326 | B1-0301 | B1-0701 | B1-0202 | B1-0201 |

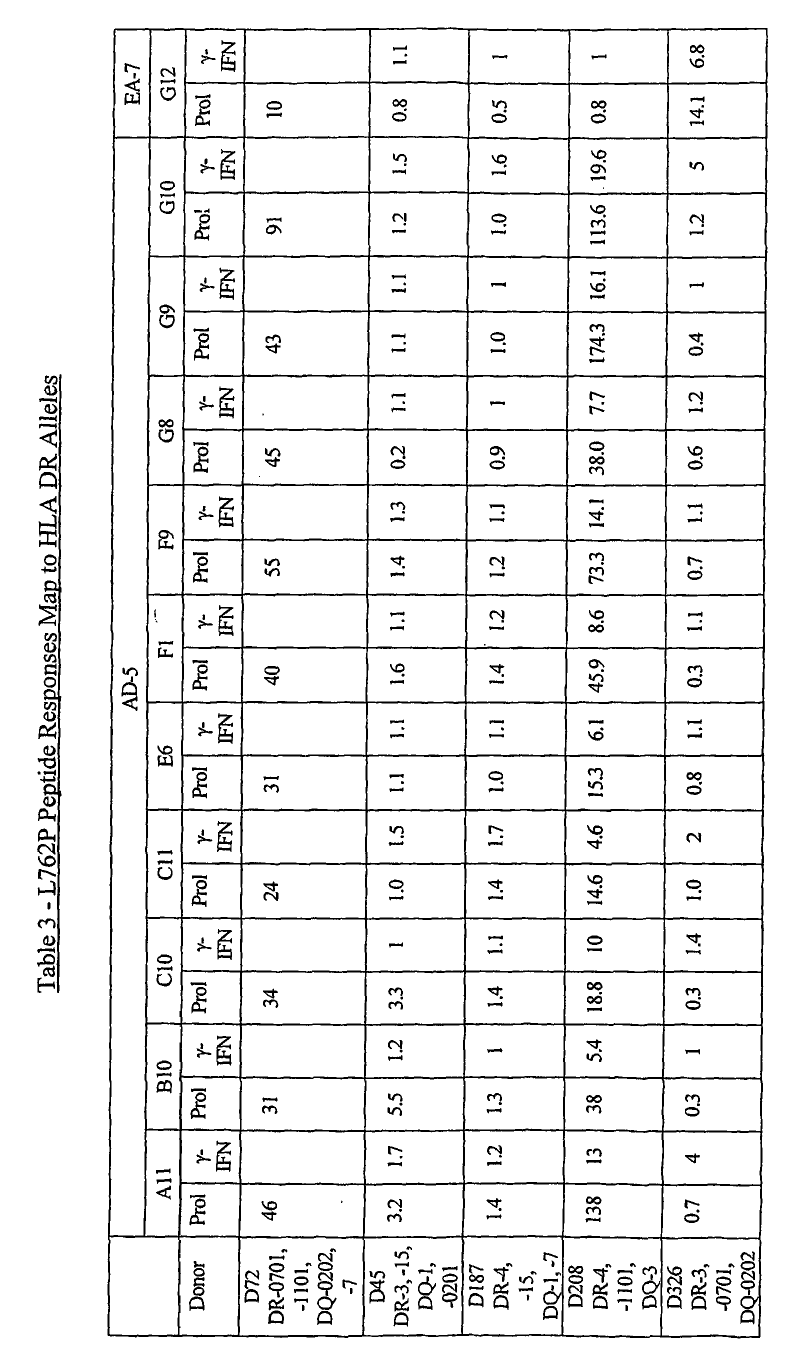

### EXAMPLE 11

### FUSION PROTEINS OF N-TERMINAL AND C-TERMINAL PORTIONS OF L763P

In another embodiment, a *Mycobacterium tuberculosis-*derived polynucleotide, referred to as Ra12, is linked to at least an immunogenic portion of a polynucleotide of this invention. Ra12 compositions and methods for their use in enhancing expression of heterologous polynucleotide sequences are described in U.S. Patent Application 60/158,585, the disclosure of which is incorporated herein by reference in its entirety. Briefly, Ra12 refers to a polynucleotide region that is a subsequence of a *Mycobacterium tuberculosis* MTB32A nucleic acid. MTB32A is a serine protease of 32 KD molecular weight encoded by a gene in virulent and avirulent strains of *M. tuberculosis.* The nucleotide sequence and amino acid sequence of MTB32A have been described (for example, WO 2001/25401; see also, Skeiky et a/., Infection and Immun. (1999) 67:3998-4007). Surprisingly, it was discovered that a 14 KD C-terminal fragment of the MTB32A coding sequence expresses at high levels on its own and remains as a soluble protein throughout the purification process. Moreover, this fragment may enhance the immunogenicity of heterologous antigenic polypeptides with which it is fused. This 14 KD C-terminal fragment of the MTB32A is referred to herein as Ra12 and represents a fragment comprising some or all of amino acid residues 192 to 323 of MTB32A.

Recombinant nucleic acids which encode a fusion polypeptide comprising a Ra12 polypeptide and a heterologous lung tumor polypeptide of interest, can be readily constructed by conventional genetic engineering techniques. Recombinant nucleic acids are constructed so that, preferably, a Ra12 polynucleotide sequence is located 5' to a selected heterologous lung tumor polynucleotide sequence. It may also be appropriate to place a Ra12 polynucleotide sequence 3' to a selected heterologous polynucleotide sequence or to insert a heterologous polynucleotide sequence into a site within a Ra12 polynucleotide sequence.

In addition, any suitable polynucleotide that encodes a Ra12 or a portion or other variant thereof can be used in constructing recombinant fusion polynucleotides comprising Ra12 and one or more lung tumor polynucleotides disclosed herein. Preferred Ra12 polynucleotides generally comprise at least about 15 consecutive nucleotides, at least about 30 nucleotides, at least about 60 nucleotides, at least about 100 nucleotides, at least about 200 nucleotides, or at least about 300 nucleotides that encode a portion of a Ra12 polypeptide.

Ra12 polynucleotides may comprise a native sequence (*i.e*., an endogenous sequence that encodes a Ra12 polypeptide or a portion thereof) or may comprise a variant of such a sequence. Ra12 polynucleotide variants may contain one or more substitutions, additions, deletions and/or insertions such that the biological activity of the encoded fusion polypeptide is not substantially diminished, relative to a fusion polypeptide comprising a native Ra12 polypeptide. Variants preferably exhibit at least about 70% identity, more preferably at least about 80% identity and most preferably at least about 90% identity to a polynucleotide sequence that encodes a native Ra12 polypeptide or a portion thereof.

Two specific embodiments of fusions between Ra12 and antigens of the present invention are described in this example.

### A. N-Terminal Portion of L763P

A fusion protein of full-length Ra12 and the N-terminal portion of L763P (referred to as L763P-N; amino acid residues 1-130 of SEQ ID NO: 159) was expressed as a single recombinant protein in E. coli. The cDNA for the N-terminal portion was obtained by PCR with a cDNA for the full length L763P and primers L763F3 (5' CGGCGAATTCATGGATTGGGGGACGCTGC; SEQ ID NO: 383) and 1763RV3 (5' CGGCCTCGAGTCACCCCTCTATCCGAACCTTCTGC; SEQ ID NO: 384). The PCR product with expected size was recovered from agarose gel, digested with restriction enzymes EcoRI and XhoI, and cloned into the corresponding sites in the expression vector pCRX1. The sequence for the fusion of full-length of Ra12 and L763P-N was confirmed by DNA sequencing. The determined cDNA sequence is provided in SEQ ID NO:351, with the corresponding amino acid sequence being provided in SEQ ID NO: 352).

### B. C-Terminal Portion of L763P

A fusion protein of full-length Ra12 and the C-terminal portion of L763P (referred to as L763P-C; amino acid residues 100-262 of SEQ ID NO: 159) was expressed as a single recombinant protein in E. *coli.* The cDNA of the C-terminal portion of L763P was obtained by PCR with a cDNA for the full length of L763P and primers L763F4 (5' CGGCGAATTCCACGAACCACTCGCAAGTTCAG; SEQ ID NO: 385) and L763RV4 (5' CGGCTCGAG-TTAGCTTGGGCCTGTGATTGC; SEQ ID NO: 386). The PCR product with expected size was recovered from agarose gel, digested with restriction enzymes EcoRI and XhoI, and cloned into the corresponding sites in the expression vector pCRX1. The sequence for the fusion of full-length Ra12 and L763P-C was confirmed by DNA sequencing. The determined DNA sequence is provided in SEQ ID NO:353, with the corresponding amino acid sequence being provided in SEQ ID NO: 354.

The recombinant proteins described in this example are useful for the preparation of vaccines, for antibody therapeutics, and for diagnosis of lung tumors.

### EXAMPLE 12

### EXPRESSION IN E. COLI OF L762P HIS TAG FUSION PROTEIN

PCR was performed on the L762P coding region with the following primers:
Forward primer starting at amino acid 32.
PDM-278, 5'ggagtacagcttcaagacaatggg 3' (SEQ ID NO:355) Tm 57°C.
Reverse primer including natural stop codon after amino acid 920, creating EcoRI site
PDM-280 5'ccatgggaattcattataataattttgttcc 3' (SEQ ID NO:356) TM55°C.

The PCR product was digested with EcoRI restriction enzyme, gel purified and then cloned into pPDM His, a modified pET28 vector with a His tag in frame, which had been digested with Eco72I and EcoRI restriction enzymes. The correct construct was confirmed by DNA sequence analysis and then transformed into BL21 (DE3) pLys S and BL21 (DE3) CodonPlus RIL expression hosts.

The protein sequence of expressed recombinant L762P is shown in SEQ ID NO:357, and the DNA sequence is shown in SEQ ID NO:358.

### EXAMPLE 13

### EXPRESSION IN E. COLI OF A L773PA HIS TAG FUSION PROTEIN

The L773PA coding region (encoding amino acids 2-71 of SEQ ID NO: 172) was PCR amplified using the following primers:
Forward primer for L773PA starting at amino acid 2:
   PDM-299 5'tggcagcccctcttcttcaagtggc 3' (SEQ ID NO:359) Tm63°C.
   Reverse primer for L773PA creating artificial stop codon after amino acid 70:
      PDM-355 5'cgccagaattcatcaaacaaatctgttagcacc 3' (SEQ ID NO:360) Tm62°C.

The resulting PCR product was digested with EcoRI restriction enzyme, gel purified and then cloned into pPDM His, a modified pET28 vector with a His tag in frame, which had been digested with Eco72I and EcoRI restriction enzymes. The correct construct was confirmed by DNA sequence analysis and transformed into BL21 (DE3) pLys S and BL21 (DE3) CodonPlus RIL expression hosts.

The protein sequence of expressed recombinant L773PA is shown in SEQ ID NO:361, and the DNA sequence is shown in SEQ ID NO:362.

### EXAMPLE 14

### IDENTIFICATION OF EPITOPES DERIVED FROM LUNG TUMOR SPECIFIC POLYPEPTIDES

A series of peptides from the L773P amino acid sequence (SEQ ID NO: 172) were synthesized and used in *in vitro* priming experiments to generate peptide-specific CD4 T cells. These peptides were 20-mers that overlapped by 15 amino acids and corresponded to amino acids 1-69 of the L773P protein. This region has been demonstrated to be tumor-specific. Following three *in vitro* stimulations, CD4 T cell lines were identified that produced IFNγ in response to the stimulating peptide but not the control peptide. Some of these T cell lines demonstrated recognition of recombinant L773P and L773PA (tumor-specific region) proteins.

To perform the experiments, a total of eleven 20-mer peptides (SEQ ID NOs: 363, 365 and 387-395) overlapping by 15 amino acids and derived from the N-terminal tumor-specific region of L773P (corresponding to amino acids 1-69 of SEQ ID NO:172) were generated by standard procedures. Dendritic cells were derived from PBMC of a normal donor using GMCSF and IL-4 by standard protocol. Purified CD4 T cells were generated from the same donor as the dendritic cells using MACS beads and negative selection of PBMCs. Dendritic cells were pulsed overnight with the individual 20-mer peptides at a concentration of 10 µg/ml. Pulsed dendritic cells were washed and plated at 1 x 10⁴/well of a 96-well U-bottom plates, and purified CD4 cells were added at 1 x 10⁵ well. Cultures were supplemented with 10 ng/ml IL-6 and 5 ng/ml IL-12, and incubated at 37°C. Cultures were re-stimulated as above on a weekly basis using as APC dendritic cells generated and pulsed as above, supplemented with 5 ng/ml IL-7 and 10 µg/ml IL-2. Following 3 *in vitro* stimulation cycles, cell lines (each corresponding to one well) were tested for cytokine production in response to the stimulating peptide vs. an irrelevant peptide.

A small number of individual CD4 T cell lines (9/528) demonstrated cytokine release (IFNγ) in response to the stimulating peptide but not to control peptide. The CD4 T cell lines that demonstrated specific activity were restimulated on the appropriate L773P peptide and reassayed using autologous dendritic cells pulsed with 10 µg/ml of the appropriate L773P peptide, an irrelevant control peptide, recombinant L773P protein (amino acids 2-364, made in E. *coli*), recombinant L773PA (amino acids 2-71, made in *E. coli*), or an appropriate control protein (L3E, made in *E. coli*). Three of the nine lines tested (1-3C, 1-6G, and 4-12B) recognized the appropriate L773P peptide as well as recombinant L773P and L773PA. Four of the lines tested (4-8A, 4-8E, 4-12D, and 4-12E) recognized the appropriate L773P peptide only. Two of the lines tested (5-6F and 9-3B) demonstrated non-specific activity.

These results demonstrate that the peptide sequences MWQPLFFKWLLSCCPGSSQI (amino acids 1-20 of SEQ ID NO: 172; SEQ ID NO:363) and GSSQIAAAASTQPEDDINTQ (amino acids 16-35 of SEQ ID NO: 172; SEQ ID NO: 365) may represent naturally processed epitopes of L773P, which are capable of stimulating human class II MHC-restricted CD4 T cell responses.

In subsequent studies, the above epitope mapping experiment was repeated using a different donor. Again, some of the resulting T cell lines were found to respond to peptide and recombinant protein. An additional peptide was found to be naturally processed. Specifically, purified CD4 cells were stimulated on a total of eleven 20-mer peptides overlapping by 15 amino acids (SEQ ID NO: 363, 387, 388, 365 and 389-395, respectively). The priming was carried out as described above, except that a peptide concentration of 0.5 ug/mL rather than 10 ug/mL was employed. In the initial screen of the cell lines 9 of the 528 lines released at least a three-fold greater level of IFN-gamma with stimulating peptide vs. control peptide. These 9 lines were restimulated on the appropriate peptide and then tested on dendritic cells pulsed with a titration of appropriate peptide (10 ug/mL, 1 ug/mL and 0.1 ug/mL), and 10 ug/mL of a control peptide. Six of the 9 lines recognized recombinant L773P as well as peptide. The six lines referred to as 1-1E, 1-2E, 1-4H, 1-6A, 1-6G and 2-12B recognized L773PA and the appropriate peptide. These results demonstrate that the peptides of SEQ ID NO: 363 and 387 represent naturally processed epitopes of L773P.

Using the procedures described above, CD4+ T cell responses were generated from PBMC of normal donors using dendritic cells pulsed with overlapping 20-mer peptides (SEQ ID NO: 396-419) spanning the L523S polypeptide sequence (SEQ ID NO: 176). A number of CD4+ T cells demonstrated reactivity with the priming peptides as well as with L523S recombinant protein, with the dominant reactivity of these lines being within the peptides 4, 7 and 21 (SEQ ID NO: 399, 402 and 416; corresponding to amino acids 30-39, 60-79 and 200-219, respectively, of SEQ ID NO: 176).

Epitopes within the scope of the invention include epitopes restricted by other class II MHC molecules. In addition, variants of the peptide can be produced wherein one or more amino acids are altered such that there is no effect on the ability of the peptides to bind to MHC molecules, no effect on their ability to elicit T cell responses, and no effect on the ability of the elicited T cells to recognize recombinant protein.

### EXAMPLE 15

### SURFACE EXPRESSION OF L762P AND ANTIBODY EPITOPES THEREOF

Rabbits were immunized with full-length histidine-tagged L762P protein generated in *E. coli*. Sera was isolated from rabbits and screened for specific recognition of L762P in ELISA assays. One polyclonal serum, referred to as 2692L, was identified that specifically recognized recombinant L762P protein. The 2692L anti-L762P polyclonal antibodies were purified from the serum by affinity purification using L762P affinity columns. Although L762P is expressed in a subset of primary lung tumor samples, expression appears to be lost in established lung tumor cell lines. Therefore, to characterize surface expression of L762P, a retrovirus construct that expresses L762P was used to transduce primary human fibroblasts as well as 3 lung tumor cell lines (522-23, HTB, and 343T). Transduced lines were selected and expanded to examine L762P surface expression by FACS analysis. For this analysis, non-transduced and transduced cells were harvested using cell dissociation medium, and incubated with 10-50 micrograms/ml of either affinity purified anti-L762P or irrelevant antisera. Following a 30 minute incubation on ice, cells were washed and incubated with a secondary, FITC conjugated, anti rabbit IgG antibody as above. Cells were washed, resuspended in buffer with Propidium Iodide (PI) and examined by FACS using an Excalibur fluorescence activated cell sorter. For FACS analysis, PI-positive (i.e. dead/permeabilized cells) were excluded. The polyclonal anti-L762P sera specifically recognized and bound to the surface of L762P-transduced cells but not the non-transduced counterparts. These results demonstrate that L762P is localized to the cell surface of both fibroblasts as well as lung tumor cells.

To identify the peptide epitopes recognized by 2692L, an epitope mapping approach was pursued. A series of overlapping 19-21 mers (5 amino acid overlap) was synthesized that spanned the C terminal portion of L762P (amino acids 481-894 of SEQ ID NO: 161). In an initial experiment peptides were tested in pools. Specific reactivity with the L762P antiserum was observed with pools A, B, C, and E. To identify the specific peptides recognized by the antiserum, flat bottom 96 well microtiter plates were coated with individual peptides at 10 microgram/ml for 2 hours at 37 °C. Wells were then aspirated and blocked with phosphate buffered saline containing 5% (w/v) milk for 2 hours at 37 °C, and subsequently washed in PBS containing 0.1% Tween 20 (PBST). Purified rabbit anti-L762P serum 2692L was added at 200 or 20 ng/well to triplicate wells in PBST and incubated overnight at room temperature. This was followed by washing 6 times with PBST and subsequently incubating with HRP-conjugated donkey anti rabbit IgG (H+L)Affinipure F(ab') fragment at 1:2,000 for 60 minutes. Plates were then washed, and incubated in tetramethyl benzidine substrate. Reactions were stopped by the addition of 1N sulfuric acid and plates were read at 450/570 nm using an ELISA plate reader.

The resulting data, presented in Table 4 below, demonstrates that the L762P antisera recognized at least 6 distinct peptide epitopes from the 3' half of L762P.

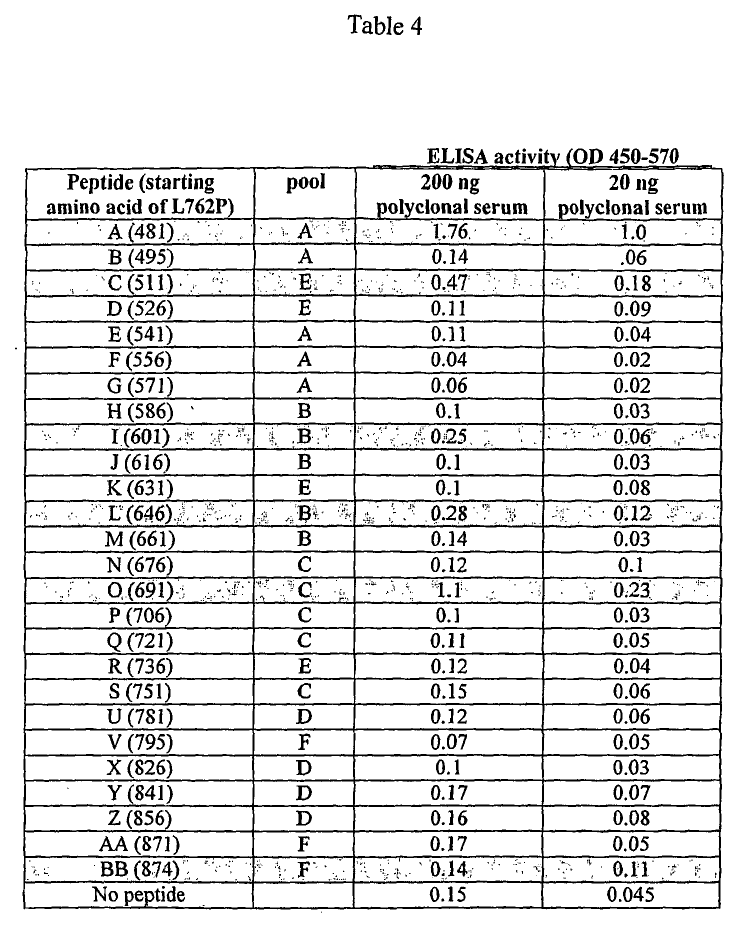

Individual peptides were identified from each of the pools, and additionally a weak reactivity was identified with peptide BB from pool F. The relevant peptide epitopes are summarized in the Table 5 below The amino acid sequences for peptides BB, O, L, I, A and C are provided in SEQ ID NO: 376-381, respectively, with the corresponding cDNA sequences being provided in SEQ ID NO: 373, 370, 372, 374, 371 and 375, respectively.

**Table 5**

| **ELISA activity** | | | | | | |
|---|---|---|---|---|---|---|
| **(OD 450-570)** | | | | | | |
| **Peptide** | **Nucleotides of L762P** | **Amino acids of L762P** | **Sequence** | **pool** | **200 ng** | **20 ng** |
| A | 1441-1500 | 481-500 | SRISSGTGDIFQQHIQLEST | A | 1.76 | 1.0 |
| C | 1531-1590 | 511-530 | KNTVTVDNTVGNDTMFLVTW | E | 0.47 | 0.18 |
| I | 1801-1860 | 601-620 | AVPPATVEAFVERDSLHFPH | B | 0.25 | 0.06 |
| L | 1936-1955 | 646-665 | PETGDPVTLRLLDDGAGADV | B | 0.28 | 0.12 |
| O | 2071-2130 | 691-710 | VNHSPSISTPAHSIPGSHAMIL | C | 1.1 | 0.23 |
| BB | 2620-2679 | 874-893 | LQSAVSNIAQAPLFIPPNSD | F | 0.14 | 0.11 |
| None | - | - | - | - | 0.15 | 0.05 |

### EXAMPLE 16

### DETECTION OF ANTIBODIES AGAINST LUNG TUMOR ANTIGENS IN PATIENT SERA

Antibodies specific for the lung tumor antigens L773PA (SEQ ID NO:361), L514S (SEQ ID NO:155 and 156), L523S (SEQ ID NO:176), L762P (SEQ ID NO:161) and L763P (SEQ ID NO:159) were shown to be present in effusion fluid or sera of lung cancer patients but not in normal donors. More specifically, the presence of antibodies against L773PA, L514S, L523S, L762P and L763P in effusion fluid obtained from lung cancer patients and in sera from normal donors was detected by ELISA using recombinant proteins and HRP-conjugated anti-human Ig. Briefly, each protein (100 ng) was coated in 96-well plate at pH 9.5. In parallel, BSA (bovine serum albumin) was also coated as a control protein. The signals ([S], absorbance measured at 405 nm) against BSA ([N]) were determined. The results of these studies are shown in Table 6, wherein - represents [S]/[N] < 2; +/- represents [S]/[N] >2; ++ represents [S]/[N] >3; and +++ represents [S]/[N] >5.

**Table 6 - Detection of Antibodies against Lung Tumor Antigens**

| | L514S | L523S | L762P | L763P | L773PA |
|---|---|---|---|---|---|
| Effusion fluid | | | | | |
| #1 | +++ | ++ | ++ | - | ++ |
| #2 | - | - | +/- | ++ | +/- |
| #3 | - | - | - | - | +/- |
| #4 | +/- | ++ | +/- | - | +/- |
| #5 | +/- | +++ | +/- | +/- | ++ |
| #7 | - | +/- | - | - | +/- |
| #8 | - | +++ | - | - | ++ |
| #10 | - | ++ | +/- | +/- | - |
| #11 | +/- | ++ | ++ | - | ++ |
| #12 | +++ | +/- | - | +/- | +/- |
| #13 | - | +/- | - | - | +/- |
| #14 | - | +++ | +/- | +/- | ++ |
| #15 | +/- | ++ | +/- | - | ++ |
| #17 | - | +/- | - | - | +/- |
| #18 | - | ++ | - | - | - |
| #19 | - | +/- | - | - | +/- |
| #20 | +/- | +/- | +/- | - | +/- |

| Normal sera | | | | | |
|---|---|---|---|---|---|
| #21 | - | +/- | - | - | - |
| #22 | - | - | - | - | - |
| #23 | - | - | - | - | +/- |
| #24 | - | +/- | - | - | - |
| #25 | +/- | +/- | - | - | +/- |

Using Western blot analyses, antibodies against L523S were found to be preset in 3 out of 4 samples of effusion fluid from lung cancer patients, with no L523S antibodies being detected in the three samples of normal sera tested.

### EXAMPLE 17

### EXPRESSION IN E. COLI OF A L514S HIS TAG FUSION PROTEIN

PCR was performed on the L514S-13160 coding region with the following primers:
Forward primer PDM-278 5' cacactagtgtccgcgtggcggcctac 3' (SEQ ID NO:421) Tm 67°C.
Reverse primer PDM-280 5' catgagaattcatcacatgcccttgaaggctccc 3' (SEQ ID NO:422) TM 66°C.

The PCR conditions were as follows:
10µl 10X Pfu buffer
1.0µl 10mM dNTPs
2.0µl 10µM each primer
83µl sterile water
1.5µl Pfu DNA polymerase (Stratagene, La Jolla, CA)
50ηg DNA
96°C for 2 minutes, 96°C for 20 seconds, 66°C for 15 seconds, 72°C for 1 minute with 40 cycles and then 72°C for 4 minutes.

The PCR product was digested with EcoRI restriction enzyme, gel purified and then cloned into pPDM His, a modified pET28 vector with a His tag in frame, which had been digested with Eco72I and EcoRI restriction enzymes. The correct construct was confirmed by DNA sequence analysis and then transformed into BL21 CodonPlus (Stratagene, La Jolla, CA) cells for expression.

The amino acid sequence of expressed recombinant L514S is shown in SEQ ID NO:423, and the DNA coding region sequence is shown in SEQ ID NO:424.

### EXAMPLE 18

### EXPRESSION IN E. COLI OF A L523S HIS TAG FUSION PROTEIN

PCR was performed on the L523S coding region with the following primers:
Forward primer PDM-414 5' aacaaactgtatatcggaaacctcagcgagaa 3' (SEQ ID NO:425) Tm 62°C.
Reverse primer PDM-415 5' ccatagaattcattacttccgtcttgactgagg 3' (SEQ ID NO:426) TM 62°C.

The PCR conditions were as follows:
10µl 10X Pfu buffer
1.0µl 10mM dNTPs
2.0µl 10µM each primer
83µl sterile water
1.5µl Pfu DNA polymerase (Stratagene, La Jolla, CA)
50ηg DNA
96°C for 2 minutes, 96°C for 20 seconds, 62°C for 15 seconds, 72°C for 4 minutes with 40 cycles and then 72°C for 4 minutes.

The PCR product was digested with EcoRI restriction enzyme, gel purified and then cloned into pPDM His, a modified pET28 vector with a His tag in frame, which had been digested with Eco72I and EcoRI restriction enzymes. The correct construct was confirmed by DNA sequence analysis and then transformed into BL21 CodonPlus (Stratagene, La Jolla, CA) cells for expression.

The amino acid sequence of expressed recombinant L523S is shown in SEQ ID NO:427, and the DNA coding region sequence is shown in SEQ ID NO:428.

### EXAMPLE 19

### EXPRESSION IN E. COLI OF A L762PA HIS TAG FUSION PROTEIN

PCR was performed on the L762PA coding region (L762PA is missing the signal sequence, the C-terminal transmembrane domain and the cytoplasmic tail) with the following primers:
Forward primer PDM-278 5'ggagtacagcttcaagacaatggg 3' (SEQ ID NO:355) Tm 57°C.
Reverse primer PDM-279 5'ccatggaattcattatttcaatataagataatctc 3' (SEQ ID NO:429) TM56°C.

The PCR conditions were as follows:
10µl 10X Pfu buffer
1.0µl 10mM dNTPs
2.0µl 10µM each primer
83µl sterile water
1.5µl Pfu DNA polymerase (Stratagene, La Jolla, CA)
50ηg DNA
96°C for 2 minutes, 96°C for 20 seconds, 55°C for 15 seconds, 72°C for 5 minutes with 40 cycles and then 72°C for 4 minutes.

The PCR product was digested with EcoRI restriction enzyme, gel purified and then cloned into pPDM His, a modified pET28 vector with a His tag in frame, which had been digested with Eco72I and EcoRI restriction enzymes. The correct construct was confirmed by DNA sequence analysis and then transformed into BL21 pLys S (Novagen, Madison, WI) cells for expression.

The amino acid sequence of expressed recombinant L762PA is shown in SEQ ID NO:430, and the DNA coding region sequence is shown in SEQ ID NO:431.

### EXAMPLE 20

### EXPRESSION IN E. COLI OF A L773P HIS TAG FUSION PROTEIN

PCR was performed on the L773P coding region with the following primers:
Forward primer PDM-299 5' tggcagcccctcttcttcaagtggc 3' (SEQ ID NO:359) Tm 63°C.
Reverse primer PDM-300 5' cgcctgctcgagtcattaatattcatcagaaaatgg 3' (SEQ ID NO:432) TM 63°C.

The PCR conditions were as follows:
10µl 10X Pfu buffer
1.0µl 10mM dNTPs
2.0µl 10µM each primer
83µl sterile water
1.5µl Pfu DNA polymerase (Stratagene, La Jolla, CA)
50µg DNA
96°C for 2 minutes, 96°C for 20 seconds, 63°C for 15 seconds, 72°C for 2 minutes 15 seconds with 40 cycles and then 72°C for 4 minutes.

The PCR product was digested with EcoRI restriction enzyme, gel purified and then cloned into pPDM His, a modified pET28 vector with a His tag in frame, which had been digested with Eco72I and EcoRI restriction enzymes. The correct construct was confined by DNA sequence analysis and then transformed into BL21 pLys S (Novagen, Madison, WI) and BL21 CodonPlus (Stratagene, La Jolla, CA) cells for expression.

The amino acid sequence of expressed recombinant L773P is shown in SEQ ID NO:433, and the DNA coding region sequence is shown in SEQ ID NO:434.

### EXAMPLE 21

### CLONING AND SEQUENCING OF A T-CELL RECEPTOR CLONE FOR THE LUNG SPECIFIC ANTIGEN L762P

T cell receptor (TCR) alpha and beta chains from a CD4 T cell clone specific for the lung specific antigen L762P were cloned and sequence. Basically, total mRNA from 2 X 10⁶ cells from CTL clone 4H6 was isolated using Trizol reagent and cDNA was synthesized using Ready-to go kits (Pharmacia). To determine Valpha and Vbeta sequences of this clone, a panel of Valpha and Vbeta subtype specific primers was synthesized and used in RT-PCR reactions with cDNA generated from each of the clones. The RT-PCR reactions demonstrated that each of the clones expressed a common Vbeta sequence that corresponded to the Vbeta8 subfamily and a Valpha sequence that corresponded to the Valpha8 subfamily. To clone the full TCR alpha and beta chains from clone 4H6, primers were designed that spanned the initiator and terminator-coding TCR nucleotides. The primers were as follows:
forward primer for TCR Valpha8 5' ggatccgccgccaccatgacatccattcgagctgta 3' (SEQ ID NO:435; has a BamHI site inserted);
Kozak reverse primer for TCR Valpha8 (antisense) 5' gtcgactcagctggaccacagccgcag 3' (SEQ ID NO:436; has a SalI site inserted plus the TCR alpha constant sequence);
forward primer for TCR Vbeta8 (sense) 5' ggatccgccgccaccatggactcctggaccttctgct 3' (SEQ ID NO:437; has a BamHI site inserted); and
Kozak reverse primer for TCR Vbeta 5' gtcgactcagaaatcctttctcttgac 3' (SEQ ID NO:438; has a SalI site inserted plus the TCR beta constant sequence).
Standard 35 cycle RT-PCR reactions were established using the cDNA synthesized from the CTL clone and the above primers utilizing the proofreading thermostable polymerase, PWO (Roche). The resultant PCR band, about 850 bp for Valpha and about 950 for Vbeta, was ligated into a PCR blunt vector (Invitrogen) and transformed into *E. coli. E. coli* transformed with plasmids having full-length alpha and beta chains were identified.. Large scale preparations of the corresponding plasmids were generated, and these plasmids were sequenced. The Valpha sequence (SEQ ID NO:439) was shown by nucleotide sequence alignment to be homologous to Valpha8.1, while the Vbeta sequence (SEQ ID NO:440) was shown by nucleotide sequence alignment to be homologous to Vbeta8.2.

### EXAMPLE 22

### RECOMBINANT EXPRESSION OF FULL LENGTH L762P IN MAMMALAIN CELLS

Full length L762P cDNA was subcloned into the mammalian expression vectors VR1012 and pCEP4 (Invitrogen). Both expression vectors had previously been modified to contain a FLAG epitope tag. These constructs were transfected into HEK293 and CHL-1 cells (ATCC) using Lipofectamine 2000 reagent (Gibco). Briefly, both the HEK and CHL-1 cells were plated at a density of 100,000 cells/ml in DMEM (Gibco) containing 10% FBS (Hyclone) and grown overnight. The following day, 4µl of Lipofectamine 2000 was added to 100µl of DMEM containing no FBS and incubated for 5 minutes at room temperature. The Lipofectamine/DMEM mixture was then added to 1µg of L762P Flag/pCEP4 or L762P Flag/VR1012 plasmid DNA resuspended in 100µl DMEM and incubated for 15 minutes at room temperature. The Lipofectamine/DNA mix was then added to the HEK293 and CHL-1 cells and incubated for 48-72 hours at 37°C with 7% CO₂. Cells were rinsed with PBS, then collected and pelleted by centrifugation. L672P expression was detected in the transfected HEK293 and CHL-1 cell lysates by Western blot analysis and was detected on the surface of transfected HEK cells by flow cytometry analysis.

For Western blot analysis, whole cell lysates were generated by incubating the cells in Triton-X100 containing lysis buffer for 30 minutes on ice. Lysates were then cleared by centrifugation at 10,000 rpm for 5 minutes at 4°C. Samples were diluted with SDS-PAGE loading buffer containing beta-mercaptoethanol, then boiled for 10 minutes prior to loading the SDS-PAGE gel. The protein was transferred to nitrocellulose and probed using 1 µg/ml purified anti-L762P rabbit polyclonal sera (lot #690/73) or non-diluted anti-L762P mAb 153.20.1 supernatant. Blots were revealed using either goat anti-rabbit Ig coupled to HRP or goat anti-mouse Ig coupled to HRP followed by incubation in ECL substrate.

For flow cytometric analysis, cells were washed further with ice cold staining buffer (PBS+1%BSA +Azide). Next, the cells were incubated for 30 minutes on ice with 10ug/ml of purified anti-L762P polyclonal sera (lot #690/73) or a 1:2 dilution of anti-L762P mAb 153.20.1 supernatant. The cells were washed 3 times with staining buffer and then incubated with a 1:100 dilution of goat anti-rabbit Ig(H+L)-FITC or goat anti-mouse Ig(H+L)-FITC reagent (Southern Biotechnology) for 30 minutes on ice. After 3 washes, the cells were resuspended in staining buffer containing propidium iodide (PI), a vital stain that allows for the exclusion of permeable cells, and analyzed by flow cytometry.

### EXAMPLE 23

### GENERATION OF POLYCLONAL ANTIBODIES TO LUNG TUMOR ANTIGENS

Three lung antigens, L523S (SEQ ID NO:176), L763P (SEQ ID NO:159) and L763 peptide #2684 (SEQ ID NO:441), were expressed and purified for use in antibody generation.

L523S and L763P were expressed in an *E. coli* recombinant expression system and grown overnight in LB Broth with the appropriate antibiotics at 37°C in a shaking incubator. The next morning, 10 ml of the overnight culture was added to 500 ml of 2x YT with the appropriate antibiotics in a 2L-baffled Erlenmeyer flask. When the optical density of the culture reached 0.4-0.6 at 560 nanometers, the cells were induced with IPTG (1 mM). Four hours after induction with IPTG, the cells were harvested by centrifugation.

The cells were then washed with phosphate buffered saline and centrifuged again. The supernatant was discarded and the cells were either frozen for future use or immediately processed. Twenty milliliters of lysis buffer was added to the cell pellets and vortexed. To break open the *E. coli* cells, this mixture was then run through a french press at a pressure of 16,000 psi. The cells were then centrifuged again and the supernatant and pellet were checked by SDS-PAGE for the partitioning of the recombinant protein.

For proteins that localized to the cell pellet, the pellet was resuspended in 10 mM Tris pH 8.0, 1% CHAPS and the inclusion body pellet was washed and centrifuged again. This procedure was repeated twice more. The washed inclusion body pellet was solubilized with either 8M urea or 6M guanidine HCl containing 10 mM Tris pH 8.0 plus 10 mM imidazole. The solubilized protein was added to 5 ml of nickel-chelate resin (Qiagen) and incubated for 45 minutes to 1 hour at room temperature with continuous agitation.

After incubation, the resin and protein mixture was poured through a disposable column and the flow through was collected. The column was then washed with 10-20 column volumes of the solubilization buffer. The antigen was then eluted from the column using 8M urea, 10 mM Tris pH 8.0 and 300 mM imidazole and collected in 3 ml fractions. A SDS-PAGE gel was run to determine which fractions to pool for further purification.

As a final purification step, a strong anion exchange resin, in this case Hi-Prep Q (Biorad), was equilibrated with the appropriate buffer and the pooled fractions from above were loaded onto the column. Each antigen was eluted off the column with an increasing salt gradient. Fractions were collected as the column was run and another SDS-PAGE gel was run to determine which fractions from the column to pool.

The pooled fractions were dialyzed against 10 mM Tris pH 8.0. The release criteria were purity as determined by SDS-PAGE or HPLC, concentration as determined by Lowry assay or Amino Acid Analysis, identity as determined by amino terminal protein sequence, and endotoxin level was determined by the Limulus (LAL) assay. The proteins were then put in vials after filtration through a 0.22-micron filter and the antigens were frozen until needed for immunization.

The L763 peptide #2684 was synthesized and conjugated to KLH and froze until needed for immunization.

The polyclonal antisera were generated using 400 micrograms of each lung antigen combined with 100 micrograms of muramyldipeptide (MDP). An equal volume of Incomplete Freund's Adjuvant (IFA) was added and then mixed and injected subcutaneously (S.C.) into a rabbit. After four weeks, the rabbit was S.C. boosted with 200 micrograms of antigen mixed with an equal volume of IFA. Thereafter the rabbit was I.V. boosted with 100 micrograms of antigen. The animal was bled seven days following each boost. The blood was then incubated at 4°C for 12-24 hours followed by centrifugation to generate the sera.

The polyclonal antisera were characterized using 96 well plates coated with antigen and incubated with 50 microliters (typically 1 microgram/microliter) of the polyclonal antisera at 4°C for 20 hours. Basically, 250 microliters of BSA blocking buffer was added to the wells and incubated at room temperature for 2 hours. Plates were washed 6 times with PBS/0.1% Tween. The rabbit sera were diluted in PBS/0.1% Tween/0.1%BSA. 50 microliters of diluted sera was added to each well and incubated at room temperature for 30 minutes. The plates were washed as described above, and then 50 microliters of goat anti-rabbit horseradish peroxidase (HRP) at a 1:10000 dilution was added and incubated at room temperature for 30 minutes.

The plates were washed as described above, and 100 microliters of TMB Microwell Peroxidase Substrate was added to each well. Following a 15-minute incubation in the dark at room temperature, the colorimetric reaction was stopped with 100 microliters of IN H₂SO₄ and read immediately at 450 nm. All the polyclonal antibodies showed immunoreactivity to the appropriate antigen.Tables 7-9 show the antibody reactivity of rabbit antisera in serial dilution to the three lung antigens, L523S, L763P and L763 peptide #2684. The first column shows the antibody dilutions. The columns "Pre-immune sera" indicate ELISA data for two experiments using pre-immune sera. These results are averaged in the fourth column. The columns "anti-L523S, L763P or #2684" indicate ELISA data for two experiments using sera from rabbits immunized as described in this Example, using the respective antigen, referred to as either L523S, L763P or #2684 in the tables.

**Table 7**

| **Antibody dilution** | **Pre-immune sera (1)** | **Pre-immune sera (2)** | **Average** | **Anti-L523S (1)** | **Anti-L523S (2)** | **Average** |
|---|---|---|---|---|---|---|
| **1:1000** | 0.14 | 0.14 | 0.14 | 2.36 | 2.37 | 2.37 |
| **1:2000** | 0.12 | 0.10 | 0.11 | 2.29 | 2.23 | 2.26 |
| **1:4000** | 0.10 | 0.09 | 0.10 | 2.11 | 2.17 | 2.14 |
| **1:8000** | 0.09 | 0.09 | 0.09 | 1.98 | 2.00 | 1.99 |
| **1:16000** | 0.09 | 0.09 | 0.09 | 1.73 | 1.76 | 1.75 |
| **1:32000** | 0.09 | 0.09 | 0.09 | 1.35 | 1.40 | 1.37 |
| **1:64000** | 0.09 | 0.11 | 0.10 | 0.94 | 0.98 | 0.96 |
| **1:128000** | 0.09 | 0.08 | 0.08 | 0.61 | 0.61 | 0.61 |
| **1:256000** | 0.08 | 0.08 | 0.08 | 0.38 | 0.38 | 0.38 |
| **1:512000** | 0.09 | 0.08 | 0.08 | 0.24 | 0.25 | 0.25 |
| **1:1024000** | 0.08 | 0.08 | 0.08 | 0.17 | 0.17 | 0.17 |
| **1:2048000** | 0.08 | 0.08 | 0.08 | 0.14 | 0.13 | 0.13 |

**Table 8**

| **Antibody dilution** | **Pre-immune sera (1)** | **Pre-immune sera (2)** | **Average** | **Anti-L763P (1)** | **Anti-L763P (2)** | **Average** |
|---|---|---|---|---|---|---|
| **1:1000** | 0.09 | 0.11 | 0.10 | 1.97 | 1.90 | 1.93 |
| **1:2000** | 0.07 | 0.07 | 0.07 | 1.86 | 1.84 | 1.85 |
| **1:4000** | 0.06 | 0.06 | 0.06 | 1.82 | 1.81 | 1.81 |
| **1:8000** | 0.06 | 0.06 | 0.06 | 1.83 | 1.81 | 1.82 |
| **1:16000** | 0.06 | 0.05 | 0.06 | 1.79 | 1.74 | 1.76 |
| **1:32000** | 0.06 | 0.06 | 0.06 | 1.56 | 1.51 | 1.53 |
| **1:64000** | 0.06 | 0.05 | 0.05 | 1.35 | 1.34 | 1.35 |
| **1:128000** | 0.05 | 0.05 | 0.05 | 1.01 | 0.98 | 0.99 |
| **1:256000** | 0.06 | 0.05 | 0.05 | 0.69 | 0.70 | 0.70 |
| **1:512000** | 0.06 | 0.05 | 0.05 | 0.47 | 0.44 | 0.46 |
| **1:1024000** | 0.06 | 0.05 | 0.06 | 0.27 | 0.27 | 0.27 |
| **1:2048000** | 0.05 | 0.05 | 0.05 | 0.16 | 0.15 | 0.16 |

**Table 9**

| **Antibody dilution** | **Pre-immune sera (1)** | **Pre-immune sera (2)** | **Average** | **Anti-#2684 (1)** | **Anti-#2684 (2)** | **Average** |
|---|---|---|---|---|---|---|
| **1:1000** | 0.07 | 0.07 | 0.07 | 2.10 | 2.00 | 2.05 |
| **1:2000** | 0.07 | 0.06 | 0.06 | 1.95 | 1.96 | 1.95 |
| **1:4000** | 0.06 | 0.06 | 0.06 | 1.77 | 1.82 | 1.79 |
| **1:8000** | 0.06 | 0.06 | 0.06 | 1.79 | 1.81 | 1.80 |
| **1:16000** | 0.06 | 0.06 | 0.06 | 1.54 | 1.50 | 1.52 |
| **1:32000** | 0.06 | 0.06 | 0.06 | 1.27 | 1.20 | 1.24 |
| **1:64000** | 0.06 | 0.06 | 0.06 | 0.85 | 0.82 | 0.83 |
| **0** | 0.06 | 0.06 | 0.06 | 0.06 | 0.06 | 0.06 |

Tables 10-12 show the affinity purification of the respective antibodies to the three lung antigens, L523S, L763P and L763 peptide #2684.

**Table 10**

| **Antibody conc. (µg/ml)** | **Affinity pure (salt peak)** | **Affinity pure (salt peak)** | **Average** | **Affinity pure (acid peak)** | **Affinity pure (acid peak)** | **Average** |
|---|---|---|---|---|---|---|
| **1.0** | 2.38 | 2.35 | 2.36 | 2.25 | 2.31 | 2.28 |
| **0.5** | 2.24 | 2.22 | 2.23 | 2.19 | 2.18 | 2.18 |
| **0.25** | 2.05 | 2.09 | 2.07 | 2.01 | 2.03 | 2.02 |
| **0.13** | 1.70 | 1.81 | 1.75 | 1.74 | 1.74 | 1.74 |
| **0.063** | 1.44 | 1.44 | 1.44 | 1.43 | 1.38 | 1.40 |
| **0.031** | 1.05 | 1.05 | 1.05 | 0.99 | 0.99 | 0.99 |
| **0.016** | 0.68 | 0.67 | 0.68 | 0.65 | 0.64 | 0.64 |
| **0.0078** | 0.43 | 0.42 | 0.42 | 0.39 | 0.39 | 0.39 |
| **0.0039** | 0.27 | 0.26 | 0.27 | 0.24 | 0.26 | 0.25 |
| **0.0020** | 0.18 | 0.20 | 0.19 | 0.19 | 0.18 | 0.19 |
| **0.0010** | 0.13 | 0.14 | 0.13 | 0.13 | 0.14 | 0.13 |
| **0.00** | 0.11 | 0.12 | 0.11 | 0.10 | 0.12 | 0.11 |

**Table 11**

| **Antibody dilution** | **Affinity pure** | **Affinity pure** | **Average** |
|---|---|---|---|
| **1:1000** | 1.64 | 1.77 | 1.70 |
| **1:2000** | 1.59 | 1.76 | 1.68 |
| **1:4000** | 1.48 | 1.62 | 1.55 |
| **1:8000** | 1.35 | 1.43 | 1.39 |
| **1:16000** | 1.09 | 1.19 | 1.14 |
| **1:32000** | 0.81 | 0.89 | 0.85 |
| **1:64000** | 0.55 | 0.58 | 0.56 |
| **1:128000** | 0.31 | 0.35 | 0.33 |
| **1:256000** | 0.18 | 0.20 | 0.19 |
| **1:512000** | 0.11 | 0.12 | 0.11 |
| **1:1024000** | 0.07 | 0.07 | 0.07 |
| **1:2048000** | 0.06 | 0.06 | 0.06 |

**Table 12**

| **Antibody conc. (µg/ml)** | **Affinity pure** | **Affinity pure** | **Average** |
|---|---|---|---|
| **1.0** | 2.00 | 2.02 | 2.01 |
| **0.5** | 2.01 | 1.93 | 1.97 |
| **0.25** | 1.84 | 1.83 | 1.84 |
| **0.13** | 1.80 | 1.83 | 1.81 |
| **0.06** | 1.39 | 1.60 | 1.50 |
| **0.03** | 1.33 | 1.35 | 1.34 |
| **0.02** | 0.94 | 0.93 | 0.94 |
| **0.00** | 0.06 | 0.06 | 0.06 |

### EXAMPLE 24

### FULL-LENGTH cDNA SEQUENCE ENCODING L529S

The isolation of a partial sequence (SEQ ID NO:106) for lung antigen L529S was previously provided in Example 2. This partial sequence was used as a query to identify potential full length cDNA and protein sequences by searching against publicly available databases. The predicted full-length cDNA sequence for the isolated cloned sequence of SEQ ID NO:106 is provided in SEQ ID NO:442. The deduced amino acid sequence of the antigen encoded by SEQ ID NO:442 is provided in SEQ ID NO:443. It was previously disclosed in Example 2 that L529S shows similarity to connexin 26, a gap junction protein.

### EXAMPLE 25

### EXPRESSION IN MEGATERIUM OF A HISTIDINE TAG-FREE LS23S FUSION PROTEIN

PCR was performed on the L523S coding region with the following primers:
Forward primer PDM-734 5' caatcaggcatgcacaacaaactgtatatcggaaac 3' (SEQ ID NO:444) Tm 63°C.
Reverse primer PDM-735 5' cgtcaagatcttcattacttccgtcttgac 3' (SEQ ID NO:445) TM 60°C.

The PCR conditions were as follows:
10µl 10X Pfu buffer
1.0µl 10mM dNTPs
2.0µl 10µM each primer
83µl sterile water
1.5µl Pfu DNA polymerase (Stratagene, La Jolla, CA)
50µg DNA
96°C for 2 minutes, 96°C for 20 seconds, 62°C for 15 seconds, 72°C for 4 minute with 40 cycles and then 72°C for 4 minutes.

The PCR product was digested with SphI and BglII restriction enzymes, gel purified and then cloned into pMEG-3, which had been digested with SphI and BglII restriction enzymes. The correct construct was confirmed by DNA sequence analysis and then transformed into Megaterium cells for expression.

The amino acid sequence of expressed recombinant L523S is shown in SEQ ID NO:446, and the DNA coding region sequence is shown in SEQ ID NO:447.

### EXAMPLE 26

### EXPRESSION IN E. COLI OF A HISTIDINE TAG-FREE L523S FUSION PROTEIN

PCR was performed on the L552S coding region with the following primers:
Forward primer PDM-733 5' cgtactagcatatgaacaaactgtatatcggaaac 3' (SEQ ID NO:448) Tm 64°C.
Reverse primer PDM-415 5' ccatagaattcattacttccgtcttgactgagg 3' (SEQ ID NO:426) TM 62°C.

The PCR conditions were as follows:
10µl 10X Pfu buffer
1.0µl 10mM dNTPs
2.0µl 10µM each primer
83µl sterile water
1.5µl Pfu DNA polymerase (Stratagene, La Jolla, CA)
50ηg DNA
96°C for 2 minutes, 96°C for 20 seconds, 62°C for 15 seconds, 72°C for 4 minute with 40 cycles and then 72°C for 4 minutes.

The PCR product was digested with NdeI and EcoRI restriction enzymes, gel purified and then cloned into pPDM, a modified pET28 vector, which had been digested with NdeI and EcoRI restriction enzymes. The correct construct was confirmed by DNA sequence analysis and then transformed into BLR pLys S and HMS 174 pLys S cells for expression.

The amino acid sequence of expressed recombinant L523S is shown in SEQ ID NO:449, and the DNA coding region sequence is shown in SEQ ID NO:450.

### EXAMPLE 27

### EPITOPE-ANALYSIS OF L514S AND L523S-SPECIFIC ANTIBODIES

Peptides of candidate antigens can be used for the evaluation of antibody responses in both preclinical and clinical studies. These data allow one to further confirm the antibody response against a certain candidate antigen. Protein-based ELISA with and without competitive peptides and peptide-based ELISA can be used to evaluate these antibody responses. Peptide ELISA is especially useful since it can further exclude the false positive of the antibody titer observed in protein-based ELISA as well as to provide the simplest assay system to test antibody responses to candidate antigens. In this example, data was obtained using both L514S- and L523S-peptides that show that individual cancer patients produce L514S- and L523S-specific antibodies. The L514S-specific antibodies-recognize primarily the following epitope of L514S:
aa86-110: LGKEVRDAKITPEAFEKLGFPAAKE (SED ID NO:451).

This epitope is the common epitope in humans. A rabbit antibody specific for L514S recognizes two addition epitopes of L514S:
(1) aa21-45: KASDGDYYTLAVPMGDVPMDGISVA (SEQ ID NO:452)
(2) aa121-135: PDRDVNLTHQLNPKVK (SED ID NO:453)

It was further found that the SEQ ID NO:452 is common to both L514S isoforms, L514S-13160 and L514S-13166, whereas the other epitopes, SEQ ID NO:451 and SEQ ID NO:453, are probably specific to the isoform, L514S-13160.

The L523S-specific antibodies recognize primarily the following epitope of L523S:
aa440-460: KIAPAEAPDAKVRMVIITGP (SEQ ID NO:454).

This epitope is the common epitope in humans. A rabbit antibody specific for L523S recognizes two other epitopes:

| | | |
|---|---|---|
| (1) | aa156-175 PDGAAQQNNNPLQQPRG | (SEQ ID NO:455) |
| (2) | aa326-345: RTITVKGNVETCAKAEEEIM | (SED ID NO:456) |

In further studies, it was determined by peptide based ELISAs that eight additional epitopes of L523S were recognized by L523S-specific antibodies:

| | | |
|---|---|---|
| (1) | aa40-59 AFVDCPDESWALKAIEALS | (SEQ ID NO:457) |
| (2) | aa80-99: IRKLQIRNIPPHLQWEVLDS | (SED ID NO:458) |
| (3) | aa160-179: AQQNPLQQPRGRRGLGQRGS | (SEQ ID NO:459) |
| (4) | aa180-199: DVHRKENAGAAEKSITILST | (SED ID NO:460) |
| (5) | aa320-339: LYNPERTITVKGNVETCAKA | (SEQ ID NO:461) |
| (6) | aa340-359: EEEIMKKIRESYENDIASMN | (SED ID NO:462) |
| (7) | aa370-389: LNALGLFPPTSGMPPPTSGP | (SEQ ID NO:463) |
| (8) | aa380-399: KIAPAEAPDAKVRMVIITGP | (SED ID NO:464) |

Out of these, six epitopes are common in both lung plural effusion fluid samples and in sera of lung patients. Of these six, SEQ ID NO:459 and SEQ ID NO:463 have no homology to other L523S-family proteins such as IGF-II mRNA-binding proteins 1 and 2. Accordingly, this indicates that these two peptides can be used as an assay system to determine the antibody response to L523S.

### EXAMPLE 28

### GENERATION OF LS23S-SPECIFIC CTL LINES USING IN VITRO WHOLE-GENE PRIMING

To determine if L523S is capable of generating a CD8⁺ T cell immune response, CTLs were generated using *in vitro* whole-gene priming methodologies with tumor antigen-vaccinia infected DC (Yee et al, The Journal of Immunology, 157(9):4079-86, 1996), human CTL lines were derived that specifically recognize autologous fibroblasts transduced with the L552S tumor antigen, as determined by interferon-gamma ELISPOT analysis. Specifically, dendritic cells (DC) were differentiated from Percoll-purified monocytes derived from PBMC of normal human donors by plastic adherence and growing for five days in RPMI medium containing 10% human serum, 50 ng/ml human GM-CSF and 30 ng/ml human IL-4. Following the five days of culture, the DC were infected overnight with a recombinant adenovirus that expresses L523S at a multiplicity of infection (M.O.I) of 33, 66 and 100, and matured overnight by the addition of 2 µg/ml CD40 ligand. The virus was then inactivated by UV irradiation. In order to generate a CTL line, autologous PBMC were isolated and CD8+ T cells were enriched for by the negative selection using magnetic beads conjugated to CD4+, CD14+, CD16+, CD19+, CD34+ and CD56+ cells. CD8+ T cells specific for L523S were established in round bottom 96-well plates using 10,000 L523S expressing DCs and 100,000 CD8+ T cells per well in RPMI supplemented with 10% human serum, 10ng/ml of IL-6 and 5ng/ml of IL-12. The cultures were restimulated every 7-10 days using autologous primary fibroblasts retrovirally transduced with L523S, and the costimulatory molecule CD80 in the presence of IL-2. The cells were also stimulated with IFN-gamma to upregulate MHC Class I. The media was supplemented with 10U/ml of IL-2 at the time of stimulation as well as on days 2 and 5 following stimulation. Following three stimulation cycles, ten L523S specific CD8+ T cell lines were identified using interferon-gamma ELISPOT analysis that specifically produce interferon-gamma when stimulated with the L523S tumor antigen-transduced autologous fibroblasts, but not with a control antigen.

One line, 6B1, was cloned using anti-CD3 and feeder cells. The clones were tested for specificity on L523S-transduced fibroblasts. In addition, using a panel of HLA-mismatched lines transduced with a vector expressing L523S and measuring interferon-gamma production by this CTL line in an ELISPOT assay, it was determined that this clone 6B1.4B8 is restricted by HLA-A0201.

Also using transfected Cos cells, it was shown that clone 6B1.4B8 recognizes Cos cells transfected with pcDNA3 HLA A0201/L523S in an HLA-restricted and antigen specific manner.

An epitope mapping study demonstrated the clone 6B1.4B8 recognizes HLA-A201 LCL loaded with peptide pool 3 (a polypeptide corresponding to amino acid positions 33-59 of L523S.

A peptide pool breakdown study demonstrated that clone 6B1.4B8 recognizes autologous B-LCL loaded with 15-mer peptides from amino acid positions 37-55 of L523S, TGYAFVCPDESWALKAIE (SEQ ID NO:465). A further peptide breakdown study demonstrated that clone 6B1.4B8 recognizes T2 cells loaded with the same 15-mer peptides.

A peptide recognition study demonstrated that clone 6B1.4B8 prefers T2 cells loaded with the peptide FVDCPESWAL (SEQ ID NO:466) which is corresponds to the amino acid sequence at positions 41-51 of L523S and is encoded by the DNA sequence of SEQ ID NO:467.

### EXAMPLE 29

### L523S EXPRESSION IN OTHER HUMAN CANCERS

It was previously disclosed in Example 2 that L523S is expressed in lung cancers including squamous, adenocarcinoma and small cell carcinoma. EST profiling analyisis of L523S further indicates that this protein may also be expressed in a number other tumor types, inclduing colon adenocarcinomas, prostate adenocarcinomas, CML, AML, Burkitt's Lymphoma, brain tumors, retinoblastomas, ovarian tumors, teratocarcinomas, uterus myosarcomas, germ cell tumors as well as pancreatic and cervical tumor cell lines.

## Claims

1. An isolated antibody, or antigen-binding fragment thereof, that specifically binds to a polypeptide set forth in SEQ ID NO:380.

2. The isolated antibody or antigen-binding fragment thereof of claim 1, wherein the antibody is a monoclonal antibody.

3. The isolated antibody or antigen-binding fragment thereof of claim 1, wherein the antibody is a polyclonal antibody.

4. A composition comprising a first component selected from the group consisting of physiologically acceptable carriers and immunostimulants, and a second component comprising the antibody of claims 1, 2 or 3.

5. The composition of claim 4 for use in therapy.

6. The composition of claim 4 for use in stimulating an immune response in a patient.

7. The use of the composition of claim 4 for the manufacture of a medicament for use in stimulating an immune response.

8. The composition of claim 4 for use in treating lung cancer in a patient.

9. The use of the composition of claim 4 for the manufacture of a medicament for use in treating lung cancer in a patient.

10. A diagnostic kit comprising at least one antibody according to claims 1, 2 or 3 and a detection reagent, wherein the detection reagent comprises a reporter group.

11. The isolated antibody, or antigen-binding fragment thereof, of any one of claims 1 to 3 for use in therapy.

12. The isolated antibody, or antigen-binding fragment thereof, of any one of claims 1 to 3 for use in treating lung cancer in a patient.

13. The use of the antibody, or antigen binding fragment thereof, of any one of claims 1 to 3 for the manufacture of a medicament for treating lung cancer in a patient.

14. The use of the isolated antibody, or antigen-binding fragment thereof, of any one of claims 1 to 3 for detecting lung cancer in a patient from a biological sample obtained from the patient.

## Patentansprüche

1. Ein isolierter Antikörper oder ein Antigen-bindendes Fragment davon, der/das spezifisch an ein Polypeptid bindet, das in SEQ ID NO:380 dargelegt ist.

2. Der isolierte Antikörper oder das Antigen-bindende Fragment davon nach Anspruch 1, wobei der Antikörper ein monoklonaler Antikörper ist.

3. Der isolierte Antikörper oder das Antigen-bindende Fragment davon nach Anspruch 1, wobei der Antikörper ein polyklonaler Antikörper ist.

4. Eine Zusammensetzung, die eine erste Komponente, die aus der Gruppe, die aus physiologisch geeigneten Trägern und Immunostimulantien besteht, ausgewählt wird, und eine zweite Komponente, die den Antikörper nach den Ansprüchen 1, 2 oder 3 umfasst, umfasst.

5. Die Zusammensetzung nach Anspruch 4 für die Verwendung in der Therapie.

6. Die Zusammensetzung nach Anspruch 4 für die Verwendung bei der Stimulierung einer Immunantwort bei einem Patienten.

7. Die Verwendung der Zusammensetzung nach Anspruch 4 für die Herstellung eines Medikaments für die Verwendung bei der Stimulierung einer Immunantwort.

8. Die Zusammensetzung nach Anspruch 4 für die Verwendung bei der Behandlung von Lungenkrebs bei einem Patienten.

9. Die Verwendung der Zusammensetzung nach Anspruch 4 für die Herstellung eines Medikaments für die Verwendung bei der Behandlung von Lungenkrebs bei einem Patienten.

10. Ein diagnostisches Kit, das mindestens einen Antikörper gemäß den Ansprüchen 1, 2 oder 3 und ein Detektionsreagenz umfasst, wobei das Detektionsreagenz eine Reportergruppe umfasst.

11. Der isolierte Antikörper oder das Antigen-bindende Fragment davon nach einem der Ansprüche 1 bis 3 für die Verwendung in der Therapie.

12. Der isolierte Antikörper oder das Antigen-bindende Fragment davon nach einem der Ansprüche 1 bis 3 für die Verwendung bei der Behandlung von Lungenkrebs bei einem Patienten.

13. Die Verwendung des Antikörpers oder des Antigen-bindenden Fragments davon nach einem der Ansprüche 1 bis 3 für die Herstellung eines Medikaments zur Behandlung von Lungenkrebs bei einem Patienten.

14. Die Verwendung des isolierten Antikörpers oder des Antigen-bindenden Fragments davon nach einem der Ansprüche 1 bis 3 zur Detektion von Lungenkrebs bei einem Patienten in einer biologischen Probe, die von dem Patienten erhalten wurde.

## Revendications

1. Un anticorps isolé, ou un fragment antigène - liaison de celui-ci, qui lie spécifiquement un polypeptide présenté dans la SEQ ID NO : 380.

2. Un anticorps isolé ou un fragment antigène - liaison de celui-ci selon la revendication 1, dans lequel l'anticorps est un anticorps monoclonal.

3. Un anticorps isolé ou un fragment antigène - liaison de celui-ci selon la revendication 1, dans lequel l'anticorps est un anticorps polyclonal.

4. Une composition comportant une première composante choisie à partir du groupe constitué par des transporteurs acceptables sur le plan physiologique et des immunostimulants, et une seconde composante constituée de l'anticorps selon les revendications 1, 2 ou 3.

5. Une composition selon la revendication 4 pour l'utilisation en thérapie.

6. Une composition selon la revendication 4 pour l'utilisation dans la stimulation de la réponse immune chez un patient.

7. L'utilisation de la composition selon la revendication 4 pour la fabrication d'un médicament ayant la propriété de stimuler la réponse immune.

8. La composition selon la revendication 4 pour une utilisation dans le traitement du cancer pulmonaire chez un patient.

9. L'utilisation de la composition selon la revendication 4 pour la fabrication d'un médicament dont l'utilisation est le traitement du cancer pulmonaire chez un patient.

10. Un kit diagnostique comportant au moins un anticorps selon les revendications 1, 2 ou 3 et un agent réactif de détection, dans lequel l'agent réactif de détection comporte un groupe reporteur.

11. Un anticorps isolé, ou un fragment antigène - liaison de celui-ci, selon l'une quelconque des revendications de 1 à 3 pour une utilisation thérapeutique.

12. Un anticorps isolé, ou un fragment antigène - liaison de celui-ci, selon l'une quelconque des revendications de 1 à 3 pour une utilisation thérapeutique dans le cancer pulmonaire chez un patient.

13. L'utilisation d'un anticorps, ou d'un fragment antigène - liaison de celui-ci, selon l'une quelconque des revendications de 1 à 3 pour la fabrication d'une médicament pour le traitement du cancer pulmonaire chez un patient.

14. L'utilisation d'un anticorps isolé, ou d'un fragment antigène - liaison de celui-ci, selon l'une quelconque des revendications de 1 à 3 pour détecter la présence d'un cancer pulmonaire chez un patient à partir d'un échantillon biologique obtenu à partir du patient.


### SEQUENCE LISTING

<110> Corixa Corporation
   Wang, Tongtong
   Marnerakis, Margarita
   Fanger, Gary R.
   Vedvick, Thomas S.
   Carter, Darrick
   Watanabe, Yoshihiro
   Henderson, Robert A.
   Peckham, David W.
   Fanger, Neil
<120> COMPOSITIONS AND METHODS FOR THE THERAPY AND DIAGNOSIS OF LUNG CANCER
<130> 210121.45502PC
<140> PCT
   <141> 2001-06-28
<160> 467
<170> FastSEQ for Windows Version 4.0
<210> 1
   <211> 315
   <212> DNA
   <213> Homo sapiens
<220>
   <221> misc_feature
   <222> 236, 241
   <223> n = A,T,C or G
<400> 1
   gcagagacag actggtggtt gaacctggag gtgccaaaaa agccagctgc gggcccagga 60
   cagctgccgt gagactcccg atgtcacagg cagtctgtgt ggttacagcg cccctcagtg 120
   ttcatctcca gcagagacaa cggaggaggc tcccaccagg acggttctca ttatttatat 180
   gttaatatgt ttgtaaactc atgtacagtt ttttttgggg gggaagcaat gggaanggta 240
   naaattacaa atagaatcat ttgctgtaat ccttaaatgg caaacggtca ggccacgtga 300
   aaaaaaaaaa aaaaa                                                  315
<210> 2
   <211> 380
   <212> DNA
   <213> Homo sapiens
<400> 2
   atttaggctt aagattttgt ttacccttgt tactaaggag caaattagta ttaaagtata 60
   atatatataa acaaatacaa aaagttttga gtggttcagc ttttttattt tttttaatgg 120
   cataactttt aacaacactg ctctgtaatg ggttgaactg tggtactcag actgagataa 180
   ctgaaatgag tggatgtata gtgttattgc ataattatcc cactatgaag caaagggact 240
   ggataaattc ccagtctaga ttattagcct ttgttaacca tcaagcacct agaagaagaa 300
   ttattggaaa ttttgtcctc tgtaactggc actttggggt gtgacttatc ttttgccttt 360
   gtaaaaaaaa aaaaaaaaaa                                             380
<210> 3
   <211> 346
   <212> DNA
   <213> Homo sapiens
<220>
   <221> misc_feature
   <222> 316, 317, 318, 322, 323, 326, 329, 330, 331, 336, 337, 339, 340, 342, 343
   <223> n = A,T,C or G
<400> 3
   ttgtaagtat acaattttag aaaggattaa atgttattga tcattttact gaatactgca 60
   catcctcacc atacaccatc cactttccaa taacatttaa tcctttctaa aattgtaagt 120
   atacaattgt actttctttg gattttcata acaaatatac catagactgt taattttatt 180
   gaagtttcct taatggaatg agtcattttt gtcttgtgct tttgaggtta cctttgcttt 240
   gacttccaac aatttgatca tatagtgttg agctgtggaa atctttaagt ttattctata 300
   gcaataattt ctattnnnag annccnggnn naaaannann annaaa                 346
<210> 4
   <211> 372
   <212> DNA
   <213> Homo sapiens
<220>
   <221> misc_feature
   <222> 297, 306, 332
   <223> n = A,T,C or G
<400> 4
   actagtctca ttactccaga attatgctct tgtacctgtg tggctgggtt tcttagtcgt 60
   tggtttggtt tggttttttg aactggtatg tagggtggtt cacagttcta atgtaagcac 120
   tctcttctcc aagttgtgct ttgtggggac aatcattctt tgaacattag agaggaaggc 180
   agttcaagct gttgaaaaga ctattgctta tttttgtttt taaagaccta cttgacgtca 240
   tgtggacagt gcacgtgcct tacgctacat cttgttttct aggaagaagg ggatgcnggg 300
   aaggantggg tgctttgtga tggataaaac gnctaaataa cacaccttta cattttgaaa 360
   aaaacaaaac aa                                                     372
<210> 5
   <211> 698
   <212> DNA
   <213> Homo sapiens
<220>
   <221> misc_feature
   <222> 8, 345, 422, 430, 433, 436, 438, 472, 481, 486, 515, 521, 536, 549, 553, 556, 557, 559, 568, 593, 597, 605, 611, 613, 616, 618, 620, 628, 630, 632, 634, 635, 639, 643, 647, 648, 649, 652, 654, 658, 664, 690
   <223> n = A,T,C or G
<400> 5 <210> 6
   <211> 740
   <212> DNA
   <213> Homo sapiens
<220>
   <221> misc_feature
   <222> 82, 406, 426, 434, 462, 536, 551, 558, 563, 567, 582, 584, 592, 638, 651, 660, 664, 673, 675, 697, 706, 711, 715, 716, 717, 723, 724, 725, 733
   <223> n = A,T,C or G
<400> 6
<210> 7
   <211> 670
   <212> DNA
   <213> Homo sapiens
<220>
   <221> misc_feature
   <222> 265, 268, 457, 470, 485, 546, 553, 566, 590, 596, 613, 624, 639, 653, 659, 661
   <223> n = A,T,C or G
<400> 7
   gctggggagc tcggcatggc ggtccccgct gcagccatgg ggccctcggc gttgggccag 60
   agcggccccg gctcgatggc cccgtggtgc tcagtgagca gcggcccgtc gcgctacgtg 120
   cttgggatgc aggagctgtt ccggggccac agcaagaccg cgagttcctg gcgcacagcg 180
   ccaaggtgca ctcggtggcc tggagttgcg acgggcgtcg cctacctcgg ggtcttcgac 240
   aagacgccac gtcttcttgc tgganaanga ccgttggtca aagaaaacaa ttatcgggga 300
   catggggata gtgtggacca ctttgttggc atccaagtaa tcctgaccta tttgttacgg 360
   cgtctggaga taaaaccatt cgcatctggg atgtgaggac tacaaaatgc attgccactg 420
   tgaacactaa aggggagaac attaatatct gctggantcc tgatgggcan accattgctg 480
   tagcnacaag gatgatgtgg tgactttatt gatgccaaga aaccccgttc caaagcaaaa 540
   aaacanttcc aanttcgaag tcaccnaaat ctcctggaac aatgaacatn aatatnttct 600
   tcctgacaat ggnccttggg tgtntcacat cctcagctnc cccaaaactg aancctgtnc 660
   natccacccc                                                        670
<210> 8
   <211> 689
   <212> DNA
   <213> Homo sapiens
<220>
   <221> misc_feature
   <222> 253, 335, 410, 428, 448, 458, 466, 479, 480, 482, 483, 485, 488, 491, 492, 495, 499, 500, 502, 503, 512, 516, 524, 525, 526, 527, 530, 540, 546, 550, 581, 593, 594, 601, 606, 609, 610, 620, 621, 622, 628, 641, 646, 656, 673
   <223> n = A,T,C or G
<400> 8
   actagtatct aggaatgaac agtaaaagag gagcagttgg ctacttgatt acaacagagt 60
   aaatgaagta ctggatttgg gaaaacctgg ttttattaga acatatggaa tgaaagccta 120
   cacctagcat tgcctactta gccccctgaa ttaacagagc ccaattgaga caaacccctg 180
   gcaacaggaa attcaaggga gaaaaagtaa gcaacttggg ctaggatgag ctgactccct 240
   tagagcaaag ganagacagc ccccattacc aaataccatt tttgcctggg gcttgtgcag 300
   ctggcagtgt tcctgcccca gcatggcacc ttatngtttt gatagcaact tcgttgaatt 360
   ttcaccaact tattacttga aattataata tagcctgtcc gtttgctgtn tccaggctgt 420
   gatatatntt cctagtggtt tgactttnaa aataaatnag gtttantttt ctccccccnn 480
   cnntnctncc nntcnctcnn cnntcccccc cnctcngtcc tccnnnnttn gggggggccn 540
   cccccncggn ggacccccct ttggtccctt agtggaggtt natggcccct ggnnttatcc 600
   nggccntann tttccccgtn nnaaatgntt ccccctccca ntcccnccac ctcaanccgg 660
   aagcctaagt ttntaccctg ggggtcccc                                   689
<210> 9
   <211> 674
   <212> DNA
   <213> Homo sapiens
<220>
   <221> misc_feature
   <222> 602, 632, 639, 668
   <223> n = A,T,C or G
<400> 9
   gtccactctc ctttgagtgt actgtcttac tgtgcactct gtttttcaac tttctagata 60
   taaaaaatgc ttgttctata gtggagtaag agctcacaca cccaaggcag caagataact 120
   gaaaaaagcg aggctttttt gccaccttgg taaaggccag ttcactgcta tagaactgct 180
   ataagcctga agggaagtag ctatgagact ttccattttt cttagttctc ccaataggct 240
   ccttcatgga aaaaggcttc ctgtaataat tttcacctaa tgaattagca gtgtgattat 300
   ttctgaaata agagacaaat tgggccgcag agtcttcctg tgatttaaaa taaacaaccc 360
   aaagttttgt ttggtcttca ccaaaggaca tactctaggg ggtatgttgt tgaagacatt 420
   caaaaacatt agctgttctg tctttcaatt tcaagttatt ttggagactg cctccatgtg 480
   agttaattac tttgctctgg aactagcatt attgtcatta tcatcacatt ctgtcatcat 540
   catctgaata atattgtgga tttccccctc tgcttgcatc ttcttttgac tcctctggga 600
   anaaatgtca aaaaaaaagg tcgatctact cngcaaggnc catctaatca ctgcgctgga 660
   aggacccnct gccc                                                   674
<210> 10
   <211> 346
   <212> DNA
   <213> Homo sapiens
<220>
   <221> misc_feature
   <222> 320, 321, 322, 325, 326, 328, 329, 330, 332, 333, 334, 335, 342
   <223> n = A,T,C or G
<400> 10
   actagtctgc tgatagaaag cactatacat cctattgttt ctttctttcc aaaatcagcc 60
   ttctgtctgt aacaaaaatg tactttatag agatggagga aaaggtctaa tactacatag 120
   ccttaagtgt ttctgtcatt gttcaagtgt attttctgta acagaaacat atttggaatg 180
   tttttctttt ccccttataa attgtaattc ctgaaatact gctgctttaa aaagtcccac 240
   tgtcagatta tattatctaa caattgaata ttgtaaatat acttgtctta cctctcaata 300
   aaagggtact tttctattan nnagnngnnn gnnnnataaa anaaaa                346
<210> 11
   <211> 602
   <212> DNA
   <213> Homo sapiens
<400> 11
   actagtaaaa agcagcattg ccaaataatc cctaattttc cactaaaaat ataatgaaat 60
   gatgttaagc tttttgaaaa gtttaggtta aacctactgt tgttagatta atgtatttgt 120
   tgcttccctt tatctggaat gtggcattag ctttttattt ttaaccctct ttaattctta 180
   ttcaattcca tgacttaagg ttggagagct aaacactggg atttttggat aacagactga 240
   cagttttgca taattataat cggcattgta catagaaagg atatggctac cttttgttaa 300
   atctgcactt tctaaatatc aaaaaaggga aatgaagtta taaatcaatt tttgtataat 360
   ctgtttgaaa catgagtttt atttgcttaa tattagggct ttgccccttt tctgtaagtc 420
   tcttgggatc ctgtgtagaa ctgttctcat taaacaccaa acagttaagt ccattctctg 480
   gtactagcta caaattcggt ttcatattct acttaacaat ttaaataaac tgaaatattt 540
   ctagatggtc tacttctgtt catataaaaa caaaacttga tttccaaaaa aaaaaaaaaa 600
   aa                                                                602
<210> 12
   <211> 685
   <212> DNA
   <213> Homo sapiens
<220>
   <221> misc_feature
   <222> 170, 279, 318, 321, 322, 422, 450, 453, 459, 467, 468, 470, 473, 475, 482, 485, 486, 491, 498, 503, 506, 509, 522, 526, 527, 528, 538, 542, 544, 551, 567, 568, 569, 574, 576, 582, 587, 588, 589, 590, 592, 593, 598, 599, 603, 605, 608
   <223> n = A,T,C or G
<221> misc_feature
   <222> 633, 634, 635, 644, 646, 648, 651, 655, 660, 662, 663, 672, 674, 675, 682, 683
   <223> n = A,T,C or G
<400> 12
   actagtcctg tgaaagtaca actgaaggca gaaagtgtta ggattttgca tctaatgttc 60
   attatcatgg tattgatgga cctaagaaaa taaaaattag actaagcccc caaataagct 120
   gcatgcattt gtaacatgat tagtagattt gaatatatag atgtagtatn ttgggtatct 180
   aggtgtttta tcattatgta aaggaattaa agtaaaggac tttgtagttg tttttattaa 240
   atatgcatat agtagagtgc aaaaatatag caaaaatana aactaaaggt agaaaagcat 300
   tttagatatg ccttaatnta nnaactgtgc caggtggccc tcggaataga tgccaggcag 360
   agaccagtgc ctgggtggtg cctccccttg tctgcccccc tgaagaactt ccctcacgtg 420
   angtagtgcc ctcgtaggtg tcacgtggan tantgggaac aggccgnncn gtnanaagaa 480
   ancanngtga nagtttcncc gtngangcng aactgtccct gngccnnnac gctcccanaa 540
   cntntccaat ngacaatcga gtttccnnnc tccngnaacc tngccgnnnn cnngcccnnc 600
   cantntgnta accccgcgcc cggatcgctc tcnnntcgtt ctcncncnaa ngggntttcn 660
   cnnccgccgt cncnnccccg cnncc                                       685
<210> 13
   <211> 694
   <212> DNA
   <213> Homo sapiens
<220>
   <221> misc_feature
   <222> 503, 546, 599, 611, 636, 641, 643, 645, 656, 658, 662, 676, 679, 687
   <223> n = A,T,C or G
<400> 13
   cactagtcac tcattagcgt tttcaatagg gctcttaagt ccagtagatt acgggtagtc 60
   agttgacgaa gatctggttt acaagaacta attaaatgtt tcattgcatt tttgtaagaa 120
   cagaataatt ttataaaatg tttgtagttt ataattgccg aaaataattt aaagacactt 180
   tttctctgtg tgtgcaaatg tgtgtttgtg atccattttt tttttttttt taggacacct 240
   gtttactagc tagctttaca atatgccaaa aaaggatttc tccctgaccc catccgtggt 300
   tcaccctctt ttccccccat gctttttgcc ctagtttata acaaaggaat gatgatgatt 360
   taaaaagtag ttctgtatct tcagtatctt ggtcttccag aaccctctgg ttgggaaggg 420
   gatcattttt tactggtcat ttccctttgg agtgtactac tttaacagat ggaaagaact 480
   cattggccat ggaaacagcc gangtgttgg gagccagcag tgcatggcac cgtccggcat 540
   ctggcntgat tggtctggct gccgtcattg tcagcacagt gccatgggac atggggaana 600
   ctgactgcac ngccaatggt tttcatgaag aatacngcat ncncngtgat cacgtnancc 660
   angacgctat gggggncana gggccanttg cttc                             694
<210> 14
   <211> 679
   <212> DNA
   <213> Homo sapiens
<220>
   <221> misc_feature
   <222> 29, 68, 83, 87, 94, 104, 117, 142, 145, 151, 187, 201, 211, 226, 229, 239, 241, 245, 252, 255, 259, 303, 309, 359, 387, 400, 441, 446, 461, 492, 504, 505, 512, 525, 527, 533, 574, 592, 609, 610, 618, 620, 626, 627, 633, 639, 645, 654
   <223> n = A,T,C or G
<400> 14
   cagccgcctg catctgtatc cagcgccang tcccgccagt cccagctgcg cgcgcccccc 60
   agtcccgnac ccgttcggcc cangctnagt tagncctcac catnccggtc aaaggangca 120
   ccaagtgcat caaatacctg cngtncggat ntaaattcat cttctggctt gccgggattg 180
   ctgtccntgc cattggacta nggctccgat ncgactctca gaccanganc atcttcganc 240
   naganactaa tnatnattnt tccagcttct acacaggagt ctatattctg atcggatccg 300
   gcnccctcnt gatgctggtg ggcttcctga gctgctgcgg ggctgtgcaa gagtcccant 360
   gcatgctggg actgttcttc ggcttcntct tggtgatatn cgccattgaa atacctgcgg 420
   ccatctgggg atattccact ncgatnatgt gattaaggaa ntccacggag ttttacaagg 480
   acacgtacaa cnacctgaaa accnnggatg anccccaccg ggaancnctg aangccatcc 540
   actatgcgtt gaactgcaat ggtttggctg gggnccttga acaatttaat cncatacatc 600
   tggccccann aaaggacntn ctcganncct tcnccgtgna attcngttct gatnccatca 660
   cagaagtctc gaacaatcc 679
<210> 15
   <211> 695
   <212> DNA
   <213> Homo sapiens
<220>
   <221> misc_feature
   <222> 105, 172, 176, 179, 189, 203, 212, 219, 221, 229, 231, 238, 242, 261, 266, 270, 278, 285, 286, 298, 311, 324, 337, 350, 363, 384, 391, 395, 405, 411, 424, 427, 443, 448, 453, 455, 458, 463, 467, 470, 479, 482, 484, 493, 499, 505, 518
   <223> n = A,T,C or G
<221> misc_feature
   <222> 520, 523, 531, 540, 584, 595, 597, 609, 611, 626, 628, 651, 652, 657, 661, 665, 669, 672, 681, 683, 691, 693
   <223> n = A,T,C or G
<400> 15
   actagtggat aaaggccagg gatgctgctc aacctcctac catgtacagg gacgtctccc 60
   cattacaact acccaatccg aagtgtcaac tgtgtcagga ctaanaaacc ctggttttga 120
   ttaaaaaagg gcctgaaaaa aggggagcca caaatctgtc tgcttcctca cnttantcnt 180
   tggcaaatna gcattctgtc tcnttggctg cngcctcanc ncaaaaaanc ngaactcnat 240
   cnggcccagg aatacatctc ncaatnaacn aaattganca aggcnntggg aaatgccnga 300
   tgggattatc ntccgcttgt tgancttcta agtttcnttc ccttcattcn accctgccag 360
   ccnagttctg ttagaaaaat gccngaattc naacnccggt tttcntactc ngaatttaga 420
   tctncanaaa cttcctggcc acnattcnaa ttnanggnca cgnacanatn ccttccatna 480
   ancncacccc acntttgana gccangacaa tgactgcntn aantgaaggc ntgaaggaan 540
   aactttgaaa ggaaaaaaaa ctttgtttcc ggccccttcc aacncttctg tgttnancac 600
   tgccttctng naaccctgga agcccngnga cagtgttaca tgttgttcta nnaaacngac 660
   ncttnaatnt cnatcttccc nanaacgatt ncncc 695
<210> 16
   <211> 669
   <212> DNA
   <213> Homo sapiens
<220>
   <221> misc_feature
   <222> 299, 354, 483, 555, 571, 573, 577, 642, 651, 662, 667
   <223> n = A,T,C or G
<400> 16
   cgccgaagca gcagcgcagg ttgtccccgt ttcccctccc ccttcccttc tccggttgcc 60
   ttcccgggcc ccttacactc cacagtcccg gtcccgccat gtcccagaaa caagaagaag 120
   agaaccctgc ggaggagacc ggcgaggaga agcaggacac gcaggagaaa gaaggtattc 180
   tgcctgagag agctgaagag gcaaagctaa aggccaaata cccaagccta ggacaaaagc 240
   ctggaggctc cgacttcctc atgaagagac tccagaaagg gcaaaagtac tttgactcng 300
   gagactacaa catggccaaa gccaacatga agaataagca gctgccaagt gcangaccag 360
   acaagaacct ggtgactggt gatcacatcc ccaccccaca ggatctgccc agagaaagtc 420
   ctcgctcgtc accagcaagc ttgcgggtgg ccaagttgaa tgatgctgcc ggggctctgc 480
   canatctgag acgcttccct ccctgcccca cccgggtcct gtgctggctc ctgcccttcc 540
   tgcttttgca gccangggtc aggaagtggc ncnggtngtg gctggaaagc aaaacccttt 600
   cctgttggtg tcccacccat ggagcccctg gggcgagccc angaacttga ncctttttgt 660
   tntcttncc                                                         669
<210> 17
   <211> 697
   <212> DNA
   <213> Homo sapiens
<220>
   <221> misc_feature
   <222> 33, 48, 50, 55, 59, 60, 76, 77, 78, 90, 113, 118, 130, 135, 141, 143, 150, 156, 166, 167, 170, 172, 180, 181, 190, 192, 194, 199, 201, 209, 212, 224, 225, 226, 230, 233, 234, 236, 242, 244, 251, 253, 256, 268, 297, 305, 308, 311, 314
   <223> n = A, T, C or G
<221> misc_feature
   <222> 315, 317, 322, 324, 327, 333, 337, 343, 362, 364, 367, 368, 373, 384, 388, 394, 406, 411, 413, 423, 429, 438, 449, 450, 473, 476, 479, 489, 491, 494, 499, 505, 507, 508, 522, 523, 527, 530, 533, 535, 538, 539, 545, 548, 550, 552, 555
   <223> n = A,T,C or G
<221> misc_feature
   <222> 562, 563, 566, 568, 572, 577, 578, 580, 581, 591, 594, 622, 628, 632, 638, 642, 644, 653, 658, 662, 663, 665, 669, 675, 680, 686, 689
   <223> n = A,T,C or G
<400> 17
   gcaagatatg gacaactaag tgagaaggta atnctctact gctctagntn ctccnggcnn 60
   gacgcgctga ggagannnac gctggcccan ctgccggcca cacacgggga tcntggtnat 120
   gcctgcccan gggancccca ncnctcggan cccatntcac acccgnnccn tncgcccacn 180
   ncctggctcn cncngcccng nccagctcnc gnccccctcc gccnnnctcn ttnncntctc 240
   cncnccctcc ncnacnacct cctacccncg gctccctccc cagccccccc ccgcaancct 300
   ccacnacncc ntcnncncga ancnccnctc gcnctcngcc ccngccccct gccccccgcc 360
   cncnacnncg cgntcccccg cgcncgcngc ctcnccccct cccacnacag ncncacccgc 420
   agncacgcnc tccgcccnct gacgccccnn cccgccgcgc tcaccttcat ggnccnacng 480
   ccccgctcnc nccnctgcnc gccgncnngg cgccccgccc cnnccgngtn ccncncgnng 540
   ccccngcngn angcngtgcg cnncangncc gngccgnncn ncaccctccg nccnccgccc 600
   cgcccgctgg gggctcccgc cncgcggntc antccccncc cntncgccca ctntccgntc 660
   cnncnctcnc gctcngcgcn cgcccnccnc ccccccc                          697
<210> 18
   <211> 670
   <212> DNA
   <213> Homo sapiens
<220>
   <221> misc_feature
   <222> 234, 292, 329, 437, 458, 478, 487, 524, 542, 549, 550, 557, 576, 597, 603, 604, 646, 665
   <223> n = A,T,C or G
<400> 18
   ctcgtgtgaa gggtgcagta cctaagccgg agcggggtag aggcgggccg gcaccccctt 60
   ctgacctcca gtgccgccgg cctcaagatc agacatggcc cagaacttga acgacttggc 120
   gggacggctg cccgccgggc cccggggcat gggcacggcc ctgaagctgt tgctgggggc 180
   cggcgccgtg gcctacggtg tgcgcgaatc tgtgttcacc gtggaaggcg ggcncagagc 240
   catcttcttc aatcggatcg gtggagtgca caggacacta tcctgggccg anggccttca 300
   cttcaggatc cttggttcca gtaccccanc atctatgaca ttcgggccag acctcgaaaa 360
   aatctcctcc ctacaggctc caaagaccta cagatggtga atatctccct gcgagtgttg 420
   tctcgaccaa tgctcangaa cttcctaaca tgttccancg cctaagggct ggactacnaa 480
   gaacgantgt tgccgtccat tgtcacgaag tgctcaagaa tttnggtggc caagttcaat 540
   gncctcacnn ctgatcnccc agcggggcca agttanccct ggttgatccc cgggganctg 600
   acnnaaaagg gccaaggact tcccctcatc ctggataatg tggccntcac aaagctcaac 660
   tttanccacc                                                        670
<210> 19
   <211> 606
   <212> DNA
   <213> Homo sapiens
<220>
   <221> misc_feature
   <222> 506
   <223> n = A,T,C or G
<400> 19
   actagtgcca acctcagctc ccaggccagt tctctgaatg tcgaggagtt ccaggatctc 60
   tggcctcagt tgtccttggt tattgatggg ggacaaattg gggatggcca gagccccgag 120
   tgtcgccttg gctcaactgt ggttgatttg tctgtgcccg gaaagtttgg catcattcgt 180
   ccaggctgtg ccctggaaag tactacagcc atcctccaac agaagtacgg actgctcccc 240
   tcacatgcgt cctacctgtg aaactctggg aagcaggaag gcccaagacc tggtgctgga 300
   tactatgtgt ctgtccactg acgactgtca aggcctcatt tgcagaggcc accggagcta 360
   gggcactagc ctgactttta aggcagtgtg tctttctgag cactgtagac caagcccttg 420
   gagctgctgg tttagccttg cacctgggga aaggatgtat ttatttgtat tttcatatat 480
   cagccaaaag ctgaatggaa aagttnagaa cattcctagg tggccttatt ctaataagtt 540
   tcttctgtct gttttgtttt tcaattgaaa agttattaaa taacagattt agaatctagt 600
   gagacc                                                            606
<210> 20
   <211> 449
   <212> DNA
   <213> Homo sapiens
<400> 20
   actagtaaac aacagcagca gaaacatcag tatcagcagc gtcgccagca ggagaatatg 60
   cagcgccaga gccgaggaga acccccgctc cctgaggagg acctgtccaa actcttcaaa 120
   ccaccacagc cgcctgccag gatggactcg ctgctcattg caggccagat aaacacttac 180
   tgccagaaca tcaaggagtt cactgcccaa aacttaggca agctcttcat ggcccaggct 240
   cttcaagaat acaacaacta agaaaaggaa gtttccagaa aagaagttaa catgaactct 300
   tgaagtcaca ccagggcaac tcttggaaga aatatatttg catattgaaa agcacagagg 360
   atttctttag tgtcattgcc gattttggct ataacagtgt ctttctagcc ataataaaat 420
   aaaacaaaat cttgactgct tgctcaaaa 449
<210> 21
   <211> 409
   <212> DNA
   <213> Homo sapiens
<400> 21
   tatcaatcaa ctggtgaata attaaacaat gtgtggtgtg atcatacaaa gggtaccact 60
   caatgataaa aggaacaagc tgcctatatg tggaacaaca tggatgcatt tcagaaactt 120
   tatgttgagt gaaagaacaa acacggagaa catactatgt ggttctcttt atgtaacatt 180
   acagaaataa aaacagaggc aaccaccttt gaggcagtat ggagtgagat agactggaaa 240
   aaggaaggaa ggaaactcta cgctgatgga aatgtctgtg tcttcattgg gtggtagtta 300
   tgtggggata tacatttgtc aaaatttatt gaactatata ctaaagaact ctgcatttta 360
   ttgggatgta aataatacct caattaaaaa gacaaaaaaa aaaaaaaaa 409
<210> 22
   <211> 649
   <212> DNA
   <213> Homo sapiens
<220>
   <221> misc_feature
   <222> 263, 353, 610, 635, 646
   <223> n = A,T,C or G
<400> 22
   acaattttca ttatcttaag cacattgtac atttctacag aacctgtgat tattctcgca 60
   tgataaggat ggtacttgca tatggtgaat tactactgtt gacagtttcc gcagaaatcc 120
   tatttcagtg gaccaacatt gtggcatggc agcaaatgcc aacattttgt ggaatagcag 180
   caaatctaca agagaccctg gttggttttt cgttttgttt tctttgtttt ttcccccttc 240
   tcctgaatca gcagggatgg aangagggta gggaagttat gaattactcc ttccagtagt 300
   agctctgaag tgtcacattt aatatcagtt ttttttaaac atgattctag ttnaatgtag 360
   aagagagaag aaagaggaag tgttcacttt tttaatacac tgatttagaa atttgatgtc 420
   ttatatcagt agttctgagg tattgatagc ttgctttatt tctgccttta cgttgacagt 480
   gttgaagcag ggtgaataac taggggcata tatatttttt ttttttgtaa gctgtttcat 540
   gatgttttct ttggaatttc cggataagtt caggaaaaca tctgcatgtt gttatctagt 600
   ctgaagttcn tatccatctc attacaacaa aaacncccag aacggnttg 649
<210> 23
   <211> 669
   <212> DNA
   <213> Homo sapiens
<220>
   <221> misc_feature
   <222> 642, 661
   <223> n = A,T,C or G
<400> 23
   actagtgccg tactggctga aatccctgca ggaccaggaa gagaaccagt tcagactttg 60
   tactctcagt caccagctct ggaattagat aaattccttg aagatgtcag gaatgggatc 120
   tatcctctga cagcctttgg gctgcctcgg ccccagcagc cacagcagga ggaggtgaca 180
   tcacctgtcg tgcccccctc tgtcaagact ccgacacctg aaccagctga ggtggagact 240
   cgcaaggtgg tgctgatgca gtgcaacatt gagtcggtgg aggagggagt caaacaccac 300
   ctgacacttc tgctgaagtt ggaggacaaa ctgaaccggc acctgagctg tgacctgatg 360
   ccaaatgaga atatccccga gttggcggct gagctggtgc agctgggctt cattagtgag 420
   gctgaccaga gccggttgac ttctctgcta gaagagactt gaacaagttc aattttgcca 480
   ggaacagtac cctcaactca gccgctgtca ccgtctcctc ttagagctca ctcgggccag 540
   gccctgatct gcgctgtggc tgtcctggac gtgctgcacc ctctgtcctt ccccccagtc 600
   agtattacct gtgaagccct tccctccttt attattcagg anggctgggg gggctccttg 660
   nttctaacc                                                         669
<210> 24
   <211> 442
   <212> DNA
   <213> Homo sapiens
<400> 24
   actagtacca tcttgacaga ggatacatgc tcccaaaacg tttgttacca cacttaaaaa 60
   tcactgccat cattaagcat cagtttcaaa attatagcca ttcatgattt actttttcca 120
   gatgactatc attattctag tcctttgaat ttgtaagggg aaaaaaaaca aaaacaaaaa 180
   cttacgatgc acttttctcc agcacatcag atttcaaatt gaaaattaaa gacatgctat 240
   ggtaatgcac ttgctagtac tacacacttt ggtacaacaa aaaacagagg caagaaacaa 300
   cggaaagaga aaagccttcc tttgttggcc cttaaactga gtcaagatct gaaatgtaga 360
   gatgatctct gacgatacct gtatgttctt attgtgtaaa taaaattgct ggtatgaaat 420
   gacctaaaaa aaaaaaaaga aa                                          442
<210> 25
   <211> 656
   <212> DNA
   <213> Homo sapiens
<220>
   <221> misc_feature
   <222> 330, 342, 418, 548, 579, 608
   <223> n = A,T,C or G
<400> 25
   tgcaagtacc acacactgtt tgaattttgc acaaaaagtg actgtaggat caggtgatag 60
   ccccggaatg tacagtgtct tggtgcacca agatgccttc taaaggctga cataccttgg 120
   accctaatgg ggcagagagt atagccctag cccagtggtg acatgaccac tccctttggg 180
   aggcctgagg tagaggggag tggtatgtgt tttctcagtg gaagcagcac atgagtgggt 240
   gacaggatgt tagataaagg ctctagttag ggtgtcattg tcatttgaga gactgacaca 300
   ctcctagcag ctggtaaagg ggtgctggan gccatggagg anctctagaa acattagcat 360
   gggctgatct gattacttcc tggcatcccg ctcactttta tgggaagtct tattagangg 420
   atgggacagt tttccatatc cttgctgtgg agctctggaa cactctctaa atttccctct 480
   attaaaaatc actgccctaa ctacacttcc tccttgaagg aatagaaatg gaactttctc 540
   tgacatantt cttggcatgg ggagccagcc acaaatgana atctgaacgt gtccaggttt 600
   ctcctganac tcatctacat agaattggtt aaaccctccc ttggaataag gaaaaa     656
<210> 26
   <211> 434
   <212> DNA
   <213> Homo sapiens
<220>
   <221> misc_feature
   <222> 395
   <223> n = A,T,C or G
<400> 26
   actagttcag actgccacgc caaccccaga aaatacccca catgccagaa aagtgaagtc 60
   ctaggtgttt ccatctatgt ttcaatctgt ccatctacca ggcctcgcga taaaaacaaa 120
   acaaaaaaac gctgccaggt tttagaagca gttctggtct caaaaccatc aggatcctgc 180
   caccagggtt cttttgaaat agtaccacat gtaaaaggga atttggcttt cacttcatct 240
   aataactgaa ttgtcaggct ttgattgata attgtagaaa taagtagcct tctgttgtgg 300
   gaataagtta taatcagtat tcatctcttt gttttttgtc actcttttct ctctaattgt 360
   gtcatttgta ctgtttgaaa aatatttctt ctatnaaatt aaactaacct gccttaaaaa 420
   aaaaaaaaaa aaaa                                                   434
<210> 27
   <211> 654
   <212> DNA
   <213> Homo sapiens
<220>
   <221> misc_feature
   <222> 505, 533, 563, 592, 613, 635, 638
   <223> n = A,T,C or G
<400> 27
   actagtccaa cacagtcaga aacattgttt tgaatcctct gtaaaccaag gcattaatct 60
   taataaacca ggatccattt aggtaccact tgatataaaa aggatatcca taatgaatat 120
   tttatactgc atcctttaca ttagccacta aatacgttat tgcttgatga agacctttca 180
   cagaatccta tggattgcag catttcactt ggctacttca tacccatgcc ttaaagaggg 240
   gcagtttctc aaaagcagaa acatgccgcc agttctcaag ttttcctcct aactccattt 300
   gaatgtaagg gcagctggcc cccaatgtgg ggaggtccga acattttctg aattcccatt 360
   ttcttgttcg cggctaaatg acagtttctg tcattactta gattccgatc tttcccaaag 420
   gtgttgattt acaaagaggc cagctaatag cagaaatcat gaccctgaaa gagagatgaa 480
   attcaagctg tgagccaggc agganctcag tatggcaaag gtcttgagaa tcngccattt 540
   ggtacaaaaa aaattttaaa gcntttatgt tataccatgg aaccatagaa anggcaaggg 600
   aattgttaag aanaatttta agtgtccaga cccanaanga aaaaaaaaaa aaaa       654
<210> 28
   <211> 670
   <212> DNA
   <213> Homo sapiens
<220>
   <221> misc_feature
   <222> 101, 226, 274, 330, 385, 392, 397, 402, 452, 473, 476, 532, 534, 538, 550, 583, 595, 604, 613, 622, 643, 669
   <223> n = A,T,C or G
<400> 28
   cgtgtgcaca tactgggagg atttccacag ctgcacggtc acagccctta cggattgcca 60
   ggaaggggcg aaagatatgt gggataaact gagaaaagaa nccaaaaacc tcaacatcca 120
   aggcagctta ttcgaactct gcggcagcgg caacggggcg gcggggtccc tgctcccggc 180
   gttcccggtg ctcctggtgt ctctctcggc agctttagcg acctgncttt ccttctgagc 240
   gtggggccag ctccccccgc ggcgcccacc cacnctcact ccatgctccc ggaaatcgag 300
   aggaagatca ttagttcttt ggggacgttn gtgattctct gtgatgctga aaaacactca 360
   tatagggaat gtgggaaatc ctganctctt tnttatntcg tntgatttct tgtgttttat 420
   ttgccaaaat gttaccaatc agtgaccaac cnagcacagc caaaaatcgg acntcngctt 480
   tagtccgtct tcacacacag aataagaaaa cggcaaaccc accccacttt tnantttnat 540
   tattactaan ttttttctgt tgggcaaaag aatctcagga acngccctgg ggccnccgta 600
   ctanagttaa ccnagctagt tncatgaaaa atgatgggct ccncctcaat gggaaagcca 660
   agaaaaagnc                                                        670
<210> 29
   <211> 551
   <212> DNA
   <213> Homo sapiens
<220>
   <221> misc_feature
   <222> 336, 474, 504, 511, 522, 523, 524, 540, 547
   <223> n = A,T,C or G
<400> 29
   actagtcctc cacagcctgt gaatccccct agacctttca agcatagtga gcggagaaga 60
   agatctcagc gtttagccac cttacccatg cctgatgatt ctgtagaaaa ggtttcttct 120
   ccctctccag ccactgatgg gaaagtattc tccatcagtt ctcaaaatca gcaagaatct 180
   tcagtaccag aggtgcctga tgttgcacat ttgccacttg agaagctggg accctgtctc 240
   cctcttgact taagtcgtgg ttcagaagtt acagcaccgg tagcctcaga ttcctcttac 300
   cgtaatgaat gtcccagggc agaaaaagag gatacncaga tgcttccaaa tccttcttcc 360
   aaagcaatag ctgatgggaa gaggagctcc agcagcagca ggaatatcga aaacagaaaa 420
   aaaagtgaaa ttgggaagac aaaagctcaa cagcatttgg taaggagaaa aganaagatg 480
   aggaaggaag agagaagaga gacnaagatc nctacggacc gnnncggaag aagaagaagn 540
   aaaaaanaaa a                                                      551
<210> 30
   <211> 684
   <212> DNA
   <213> Homo sapiens
<220>
   <221> misc_feature
   <222> 545, 570, 606, 657, 684
   <223> n = A,T,C or G
<400> 30
   actagttcta tctggaaaaa gcccgggttg gaagaagctg tggagagtgc gtgtgcaatg 60
   cgagactcat ttcttggaag catccctggc aaaaatgcag ctgagtacaa ggttatcact 120
   gtgatagaac ctggactgct ttttgagata atagagatgc tgcagtctga agagacttcc 180
   agcacctctc agttgaatga attaatgatg gcttctgagt caactttact ggctcaggaa 240
   ccacgagaga tgactgcaga tgtaatcgag cttaaaggga aattcctcat caacttagaa 300
   ggtggtgata ttcgtgaaga gtcttcctat aaagtaattg tcatgccgac tacgaaagaa 360
   aaatgccccc gttgttggaa gtatacagcg ggagtcttca gatacactgt gtcctcgatg 420
   tgcagaagtt gtcagtggga aaatagtatt aacagctcac tcgagcaaga accctcctga 480
   cagtactggg ctagaagttt ggatggatta tttacaatat aggaaagaaa gccaagaatt 540
   aggtnatgag tggatgagta aatggtggan gatggggaat tcaaatcaga attatggaag 600
   aagttnttcc tgttactata gaaaggaatt atgtttattt acatgcagaa aatatanatg 660
   tgtggtgtgt accgtggatg gaan                                        684
<210> 31
   <211> 654
   <212> DNA
   <213> Homo sapiens
<220>
   <221> misc_feature
   <222> 326, 582, 651
   <223> n = A,T,C or G
<400> 31
   gcgcagaaaa ggaaccaata tttcagaaac aagcttaata ggaacagctg cctgtacatc 60
   aacatcttct cagaatgacc cagaagttat catcgtggga gctggcgtgc ttggctctgc 120
   tttggcagct gtgctttcca gagatggaag aaaggtgaca gtcattgaga gagacttaaa 180
   agagcctgac agaatagttg gagaattcct gcagccgggt ggttatcatg ttctcaaaga 240
   ccttggtctt ggagatacag tggaaggtct tgatgcccag gttgtaaatg gttacatgat 300
   tcatgatcag ggaaagcaaa tcagangttc agattcctta ccctctgtca gaaaacaatc 360
   aagtgcagag tggaagagct ttccatcacg gaagattcat catgagtctc cggaaagcag 420
   ctatggcaga gcccaatgca aagtttattg aaggtgttgt gttacagtta ttagaggaag 480
   atgatgttgt gatgggagtt cagtacaagg ataaagagac tgggagatat caaggaactc 540
   catgctccac tgactgttgt tgcagatggg cttttctcca anttcaggaa aagcctggtc 600
   tcaataaagt ttctgtatca ctcatttggt tggcttctta tgaagaatgc nccc       654
<210> 32
   <211> 673
   <212> DNA
   <213> Homo sapiens
<220>
   <221> misc_feature
   <222> 376, 545, 627
   <223> n = A,T,C or G
<400> 32
   actagtgaag aaaaagaaat tctgatacgg gacaaaaatg ctcttcaaaa catcattctt 60
   tatcacctga caccaggagt tttcattgga aaaggatttg aacctggtgt tactaacatt 120
   ttaaagacca cacaaggaag caaaatcttt ctgaaagaag taaatgatac acttctggtg 180
   aatgaattga aatcaaaaga atctgacatc atgacaacaa atggtgtaat tcatgttgta 240
   gataaactcc tctatccagc agacacacct gttggaaatg atcaactgct ggaaatactt 300
   aataaattaa tcaaatacat ccaaattaag tttgttcgtg gtagcacctt caaagaaatc 360
   cccgtgactg tctatnagcc aattattaaa aaatacacca aaatcattga tgggagtgcc 420
   tgtgggaaat aactgaaaaa gagaccgaga agaacgaatc attacaggtc ctgaaataaa 480
   atacctagga tttctactgg aggtggagaa acagaagaac tctgaagaaa ttgttacaag 540
   aagangtccc aaggtcacca aattcattga aggtggtgat ggtctttatt tgaagatgaa 600
   gaaattaaaa gacgcttcag ggagacnccc catgaaggaa ttgccagcca caaaaaaatt 660
   cagggattag aaa                                                    673
<210> 33
   <211> 673
   <212> DNA
   <213> Homo sapiens
<220>
   <221> misc_feature
   <222> 325, 419, 452, 532, 538, 542, 571, 600, 616, 651, 653, 672
   <223> n = A,T,C or G
<400> 33
   actagttatt tactttcctc cgcttcagaa ggtttttcag actgagagcc taagcatact 60
   ggatctgttg tttcttttgg gtctcacctc atcagtgtgc atagtggcag aaattataaa 120
   gaaggttgaa aggagcaggg aaaagatcca gaagcatgtt agttcgacat catcatcttt 180
   tcttgaagta tgatgcatat tgcattattt tatttgcaaa ctaggaattg cagtctgagg 240
   atcatttaga agggcaagtt caagaggata tgaagatttg agaacttttt aactattcat 300
   tgactaaaaa tgaacattaa tgttnaagac ttaagacttt aacctgctgg cagtcccaaa 360
   tgaaattatg caactttgat atcatattcc ttgatttaaa ttgggctttt gtgattgant 420
   gaaactttat aaagcatatg gtcagttatt tnattaaaaa ggcaaaacct gaaccacctt 480
   ctgcacttaa agaagtctaa cagtacaaat acctatctat cttagatgga tntatttntt 540
   tntattttta aatattgtac tatttatggt nggtggggct ttcttactaa tacacaaatn 600
   aatttatcat ttcaanggca ttctatttgg gtttagaagt tgattccaag nantgcatat 660
   ttcgctactg tnt                                                    673
<210> 34
   <211> 684
   <212> DNA
   <213> Homo sapiens
<220>
   <221> misc_feature
   <222> 414, 472, 480, 490, 503, 507, 508, 513, 523, 574, 575, 598, 659, 662, 675
   <223> n = A,T,C or G
<400> 34
   actagtttat tcaagaaaag aacttactga ttcctctgtt cctaaagcaa gagtggcagg 60
   tgatcagggc tggtgtagca tccggttcct ttagtgcagc taactgcatt tgtcactgat 120
   gaccaaggag gaaatcacta agacatttga gaagcagtgg tatgaacgtt cttggacaag 180
   ccacagttct gagccttaac cctgtagttt gcacacaaga acgagctcca cctccccttc 240
   ttcaggagga atctgtgcgg atagattggc tggacttttc aatggttctg ggttgcaagt 300
   gggcactgtt atggctgggt atggagcgga cagccccagg aatcagagcc tcagcccggc 360
   tgcctggttg gaaggtacag gtgttcagca ccttcggaaa aagggcataa agtngtgggg 420
   gacaattctc agtccaagaa gaatgcattg accattgctg gctatttgct tncctagtan 480
   gaattggatn catttttgac cangatnntt ctnctatgct ttnttgcaat gaaatcaaat 540
   cccgcattat ctacaagtgg tatgaagtcc tgcnnccccc agagaggctg ttcaggcnat 600
   gtcttccaag ggcagggtgg gttacaccat tttacctccc ctctcccccc agattatgna 660
   cncagaagga atttntttcc tccc                                        684
<210> 35
   <211> 614
   <212> DNA
   <213> Homo sapiens
<220>
   <221> misc_feature
   <222> 17, 20, 152, 223, 267, 287, 304, 306, 316, 319, 321, 355, 365, 382, 391, 407, 419, 428, 434, 464, 467, 477, 480, 495, 499, 505, 515, 516, 522, 524, 527, 542, 547, 549, 567, 572, 576, 578
   <223> n = A,T,C or G
<400> 35
   actagtccaa cgcgttngcn aatattcccc tggtagccta cttccttacc cccgaatatt 60
   ggtaagatcg agcaatggct tcaggacatg ggttctcttc tcctgtgatc attcaagtgc 120
   tcactgcatg aagactggct tgtctcagtg tntcaacctc accagggctg tctcttggtc 180
   cacacctcgc tccctgttag tgccgtatga cagcccccat canatgacct tggccaagtc 240
   acggtttctc tgtggtcaat gttggtnggc tgattggtgg aaagtanggt ggaccaaagg 300
   aagncncgtg agcagncanc nccagttctg caccagcagc gcctccgtcc tactngggtg 360
   ttccngtttc tcctggccct gngtgggcta nggcctgatt cgggaanatg cctttgcang 420
   gaaggganga taantgggat ctaccaattg attctggcaa aacnatntct aagattnttn 480
   tgctttatgt ggganacana tctanctctc atttnntgct gnanatnaca ccctactcgt 540
   gntcgancnc gtcttcgatt ttcgganaca cnccantnaa tactggcgtt ctgttgttaa 600
   aaaaaaaaaa aaaa                                                  614
<210> 36
   <211> 686
   <212> DNA
   <213> Homo sapiens
<220>
   <221> misc_feature
   <222> 222, 224, 237, 264, 285, 548, 551, 628, 643, 645, 665, 674
   <223> n = A,T,C or G
<400> 36
   gtggctggcc cggttctccg cttctcccca tcccctactt tcctccctcc ctccctttcc 60
   ctccctcgtc gactgttgct tgctggtcgc agactccctg acccctccct cacccctccc 120
   taacctcggt gccaccggat tgcccttctt ttcctgttgc ccagcccagc cctagtgtca 180
   gggcgggggc ctggagcagc ccgaggcact gcagcagaag ananaaaaga cacgacnaac 240
   ctcagctcgc cagtccggtc gctngcttcc cgccgcatgg caatnagaca gacgccgctc 300
   acctgctctg ggcacacgcg acccgtggtt gatttggcct tcagtggcat cacccttatg 360
   ggtatttctt aatcagcgct tgcaaagatg gttaacctat gctacgccag ggagatacag 420
   gagactggat tggaacattt ttggggtcta aaggtctgtt tggggtgcaa cactgaataa 480
   ggatgccacc aaagcagcta cagcagctgc agatttcaca gcccaagtgt gggatgctgt 540
   ctcagganat naattgataa cctggctcat aacacattgt caagaatgtg gatttcccca 600
   ggatattatt atttgtttac cggggganag gataactgtt tcncntattt taattgaaca 660
   aactnaaaca aaanctaagg aaatcc                                     686
<210> 37
   <211> 681
   <212> DNA
   <213> Homo sapiens
<220>
   <221> misc_feature
   <222> 7, 10, 11, 19, 25, 32, 46, 53, 77, 93, 101, 103, 109, 115, 123, 128, 139, 157, 175, 180, 192, 193, 194, 212, 218, 226, 227, 233, 240, 241, 259, 260, 267, 289, 296, 297, 298, 312, 313, 314, 320, 325, 330, 337, 345, 346, 352, 353, 356
   <223> n = A,T,C or G
<221> misc_feature
   <222> 382, 385, 400, 427, 481, 484, 485, 491, 505, 515, 533, 542, 544, 554, 557, 560, 561, 564, 575, 583, 589, 595, 607, 619, 628, 634, 641, 645, 658, 670
   <223> n = A,T,C or G
<400> 37
   gagacanacn naacgtcang agaanaaaag angcatggaa cacaanccag gcncgatggc 60
   caccttccca ccagcancca gcgcccccca gcngccccca ngnccggang accangactc 120
   cancctgnat caatctganc tctattcctg gcccatncct acctcggagg tggangccgn 180
   aaaggtcgca cnnncagaga agctgctgcc ancaccancc gccccnnccc tgncgggctn 240
   nataggaaac tggtgaccnn gctgcanaat tcatacagga gcacgcgang ggcacnnnct 300
   cacactgagt tnnngatgan gcctnaccan ggacctnccc cagcnnattg annacnggac 360
   tgcggaggaa ggaagacccc gnacnggatc ctggccggcn tgccaccccc ccacccctag 420
   gattatnccc cttgactgag tctctgaggg gctacccgaa cccgcctcca ttccctacca 480
   natnntgctc natcgggact gacangctgg ggatnggagg ggctatcccc cancatcccc 540
   tnanaccaac agcnacngan natnggggct ccccngggtc ggngcaacnc tcctncaccc 600
   cggcgcnggc cttcggtgnt gtcctccntc aacnaattcc naaanggcgg gccccccngt 660
   ggactcctcn ttgttccctc c 681
<210> 38
   <211> 687
   <212> DNA
   <213> Homo sapiens
<220>
   <221> misc_feature
   <222> 3, 30, 132, 151, 203, 226, 228, 233, 252, 264, 279, 306, 308, 320, 340, 347, 380, 407, 429, 437, 440, 445, 448, 491, 559, 567, 586, 589, 593, 596, 603, 605, 606, 609, 626, 639, 655, 674, 682
   <223> n = A,T,C or G
<400> 38
   canaaaaaaa aaaacatggc cgaaaccagn aagctgcgcg atggcgccac ggcccctctt 60
   ctcccggcct gtgtccggaa ggtttccctc cgaggcgccc cggctcccgc aagcggagga 120
   gagggcggga cntgccgggg ccggagctca naggccctgg ggccgctctg ctctcccgcc 180
   atcgcaaggg cggcgctaac ctnaggcctc cccgcaaagg tccccnangc ggnggcggcg 240
   gggggctgtg anaaccgcaa aaanaacgct gggcgcgcng cgaacccgtc cacccccgcg 300
   aaggananac ttccacagan gcagcgtttc cacagcccan agccacnttt ctagggtgat 360
   gcaccccagt aagttcctgn cggggaagct caccgctgtc aaaaaanctc ttcgctccac 420
   cggcgcacna aggggangan ggcangangc tgccgcccgc acaggtcatc tgatcacgtc 480
   gcccgcccta ntctgctttt gtgaatctcc actttgttca accccacccg ccgttctctc 540
   ctccttgcgc cttcctctna ccttaanaac cagcttcctc tacccnatng tanttnctct 600
   gcncnngtng aaattaattc ggtccnccgg aacctcttnc ctgtggcaac tgctnaaaga 660
   aactgctgtt ctgnttactg cngtccc 687
<210> 39
   <211> 695
   <212> DNA
   <213> Homo sapiens
<220>
   <221> misc_feature
   <222> 300, 401, 423, 429, 431, 437, 443, 448, 454, 466, 492, 515, 523, 524, 536, 538, 541, 552, 561, 566, 581, 583, 619, 635, 636, 641, 649, 661, 694
   <223> n = A,T,C or G
<400> 39
   actagtctgg cctacaatag tgtgattcat gtaggacttc tttcatcaat tcaaaacccc 60
   tagaaaaacg tatacagatt atataagtag ggataagatt tctaacattt ctgggctctc 120
   tgacccctgc gctagactgt ggaaagggag tattattata gtatacaaca ctgctgttgc 180
   cttattagtt ataacatgat aggtgctgaa ttgtgattca caatttaaaa acactgtaat 240
   ccaaactttt ttttttaact gtagatcatg catgtgaatg ttaatgttaa tttgttcaan 300
   gttgttatgg gtagaaaaaa ccacatgcct taaaatttta aaaagcaggg cccaaactta 360
   ttagtttaaa attaggggta tgtttccagt ttgttattaa ntggttatag ctctgtttag 420
   aanaaatcna ngaacangat ttngaaantt aagntgacat tatttnccag tgacttgtta 480
   atttgaaatc anacacggca ccttccgttt tggtnctatt ggnntttgaa tccaancngg 540
   ntccaaatct tnttggaaac ngtccnttta acttttttac nanatcttat ttttttattt 600
   tggaatggcc ctatttaang ttaaaagggg ggggnnccac naccattcnt gaataaaact 660
   naatatatat ccttggtccc ccaaaattta aggng 695
<210> 40
   <211> 674
   <212> DNA
   <213> Homo sapiens
<220>
   <221> misc_feature
   <222> 403, 428, 432, 507, 530, 543, 580, 583, 591, 604, 608, 621, 624, 626, 639, 672
   <223> n = A,T,C or G
<400> 40
   actagtagtc agttgggagt ggttgctata ccttgacttc atttatatga atttccactt 60
   tattaaataa tagaaaagaa aatcccggtg cttgcagtag agttatagga cattctatgc 120
   ttacagaaaa tatagccatg attgaaatca aatagtaaag gctgttctgg ctttttatct 180
   tcttagctca tcttaaataa gtagtacact tgggatgcag tgcgtctgaa gtgctaatca 240
   gttgtaacaa tagcacaaat cgaacttagg atgtgtttct tctcttctgt gtttcgattt 300
   tgatcaattc tttaattttg ggaacctata atacagtttt cctattcttg gagataaaaa 360
   ttaaatggat cactgatatt taagtcattc tgcttctcat ctnaatattc catattctgt 420
   attagganaa antacctccc agcacagccc cctctcaaac cccacccaaa accaagcatt 480
   tggaatgagt ctcctttatt tccgaantgt ggatggtata acccatatcn ctccaatttc 540
   tgnttgggtt gggtattaat ttgaactgtg catgaaaagn ggnaatcttt nctttgggtc 600
   aaantttncc ggttaatttg nctngncaaa tccaatttnc tttaagggtg tctttataaa 660
   atttgctatt cngg 674
<210> 41
   <211> 657
   <212> DNA
   <213> Homo sapiens
<220>
   <221> misc_feature
   <222> 243, 247, 251, 261, 267, 272, 298, 312, 315, 421, 432, 434, 501, 524, 569, 594, 607, 650
   <223> n = A,T,C or G
<400> 41
   gaaacatgca agtaccacac actgtttgaa ttttgcacaa aaagtgactg tagggatcag 60
   gtgatagccc cggaatgtac agtgtcttgg tgcaccaaga tgccttctaa aggctgacat 120
   accttgggac cctaatgggg cagagagtat agccctagcc cagtggtgac atgaccactc 180
   cctttgggag gctgaagtta aagggaatgg tatgtgtttt ctcatggaag cagcacatga 240
   atnggtnaca ngatgttaaa ntaaggntct antttgggtg tcttgtcatt tgaaaaantg 300
   acacactcct ancanctggt aaaggggtgc tggaagccat ggaagaactc taaaaacatt 360
   agcatgggct gatctgatta cttcctggca tcccgctcac ttttatggga agtcttatta 420
   naaggatggg ananttttcc atatccttgc tgttggaact ctggaacact ctctaaattt 480
   ccctctatta aaaatcactg nccttactac acttcctcct tganggaata gaaatggacc 540
   tttctctgac ttagttcttg gcatggganc cagcccaaat taaaatctga cttntccggt 600
   ttctccngaa ctcacctact tgaattggta aaacctcctt tggaattagn aaaaacc 657
<210> 42
   <211> 389
   <212> DNA
   <213> Homo sapiens
<220>
   <221> misc_feature
   <222> 179, 317, 320
   <223> n = A, T, C or G
<400> 42
   actagtgctg aggaatgtaa acaagtttgc tgggccttgc gagacttcac caggttgttt 60
   cgatagctca cactcctgca ctgtgcctgt cacccaggaa tgtctttttt aattagaaga 120
   caggaagaaa acaaaaacca gactgtgtcc cacaatcaga aacctccgtt gtggcagang 180
   ggccttcacc gccaccaggg tgtcccgcca gacagggaga gactccagcc ttctgaggcc 240
   atcctgaaga attcctgttt gggggttgtg aaggaaaatc acccggattt aaaaagatgc 300
   tgttgcctgc ccgcgtngtn gggaagggac tggtttcctg gtgaatttct taaaagaaaa 360
   atattttaag ttaagaaaaa aaaaaaaaa 389
<210> 43
   <211> 279
   <212> DNA
   <213> Homo sapiens
<400> 43
   actagtgaca agctcctggt cttgagatgt cttctcgtta aggagatggg ccttttggag 60
   gtaaaggata aaatgaatga gttctgtcat gattcactat tctagaactt gcatgacctt 120
   tactgtgtta gctctttgaa tgttcttgaa attttagact ttctttgtaa acaaataata 180
   tgtccttatc attgtataaa agctgttatg tgcaacagtg tggagatcct tgtctgattt 240
   aataaaatac ttaaacactg aaaaaaaaaa aaaaaaaaa 279
<210> 44
   <211> 449
   <212> DNA
   <213> Homo sapiens
<220>
   <221> misc_feature
   <222> 245, 256, 264, 266, 273, 281, 323, 325, 337, 393
   <223> n = A,T,C or G
<400> 44
   actagtagca tcttttctac aacgttaaaa ttgcagaagt agcttatcat taaaaaacaa 60
   caacaacaac aataacaata aatcctaagt gtaaatcagt tattctaccc cctaccaagg 120
   atatcagcct gttttttccc ttttttctcc tgggaataat tgtgggcttc ttcccaaatt 180
   tctacagcct ctttcctctt ctcatgcttg agcttccctg tttgcacgca tgcgttgtgc 240
   aagantgggc tgtttngctt ggantncggt ccnagtggaa ncatgctttc ccttgttact 300
   gttggaagaa actcaaacct tcnancccta ggtgttncca ttttgtcaag tcatcactgt 360
   atttttgtac tggcattaac aaaaaaagaa atnaaatatt gttccattaa actttaataa 420
   aactttaaaa gggaaaaaaa aaaaaaaaa                                449
<210> 45
   <211> 559
   <212> DNA
   <213> Homo sapiens
<220>
   <221> misc_feature
   <222> 263
   <223> n = A,T,C or G
<400> 45
   actagtgtgg gggaatcacg gacacttaaa gtcaatctgc gaaataattc ttttattaca 60
   cactcactga agtttttgag tcccagagag ccattctatg tcaaacattc caagtactct 120
   ttgagagccc agcattacat caacatgccc gtgcagttca aaccgaagtc cgcaggcaaa 180
   tttgaagctt tgcttgtcat tcaaacagat gaaggcaaga gtattgctat tcgactaatt 240
   ggtgaagctc ttggaaaaaa ttnactagaa tactttttgt gttaagttaa ttacataagt 300
   tgtattttgt taactttatc tttctacact acaattatgc ttttgtatat atattttgta 360
   tgatggatat ctataattgt agattttgtt tttacaagct aatactgaag actcgactga 420
   aatattatgt atctagccca tagtattgta cttaactttt acagggtgaa aaaaaaattc 480
   tgtgtttgca ttgattatga tattctgaat aaatatggga atatatttta atgtgggtaa 540
   aaaaaaaaaa aaaaaggaa                                           559
<210> 46
   <211> 731
   <212> DNA
   <213> Homo sapiens
<220>
   <221> misc_feature
   <222> 270, 467, 477, 502, 635, 660, 671, 688, 695, 697, 725
   <223> n = A,T,C or G
<400> 46
   actagttcta gtaccatggc tgtcatagat gcaaccatta tattccattt agtttcttcc 60
   tcaggttccc taacaattgt ttgaaactga atatatatgt ttatgtatgt gtgtgtgttc 120
   actgtcatgt atatggtgta tatgggatgt gtgcagtttt cagttatata tatattcata 180
   tatacatatg catatatatg tataatatac atatatacat gcatacactt gtataatata 240
   catatatata cacatatatg cacacatatn atcactgagt tccaaagtga gtctttattt 300
   ggggcaattg tattctctcc ctctgtctgc tcactgggcc tttgcaagac atagcaattg 360
   cttgatttcc tttggataag agtcttatct tcggcactct tgactctagc cttaacttta 420
   gatttctatt ccagaatacc tctcatatct atcttaaaac ctaaganggg taaagangtc 480
   ataagattgt agtatgaaag antttgctta gttaaattat atctcaggaa actcattcat 540
   ctacaaatta aattgtaaaa tgatggtttg ttgtatctga aaaaatgttt agaacaagaa 600
   atgtaactgg gtacctgtta tatcaaagaa cctcnattta ttaagtctcc tcatagccan 660
   atccttatat ngccctctct gacctgantt aatananact tgaataatga atagttaatt 720
   taggnttggg c                                                      731
<210> 47
   <211> 640
   <212> DNA
   <213> Homo sapiens
<220>
   <221> misc_feature
   <222> 5, 28, 106, 153, 158, 173, 176, 182, 189, 205, 210, 214, 225, 226, 229, 237, 260, 263, 269, 277, 281, 282, 322, 337, 338, 354, 365, 428, 441, 443, 456, 467, 476, 484, 503, 508, 554, 567, 575, 579, 588, 601, 606, 609, 611, 621, 636
   <223> n = A,T,C or G
<400> 47
   tgcgngccgg tttggccctt ctttgtanga cactttcatc cgccctgaaa tcttcccgat 60
   cgttaataac tcctcaggtc cctgcctgca cagggttttt tcttantttg ttgcctaaca 120
   gtacaccaaa tgtgacatcc tttcaccaat atngattnct tcataccaca tcntcnatgg 180
   anacgactnc aacaattttt tgatnacccn aaanactggg ggctnnaana agtacantct 240
   ggagcagcat ggacctgtcn gcnactaang gaacaanagt nntgaacatt tacacaacct 300
   ttggtatgtc ttactgaaag anagaaacat gcttctnncc ctagaccacg aggncaaccg 360
   caganattgc caatgccaag tccgagcggt tagatcaggt aatacattcc atggatgcat 420
   tacatacntt gtccccgaaa nanaagatgc cctaanggct tcttcanact ggtccngaaa 480
   acanctacac ctggtgcttg ganaacanac tctttggaag atcatctggc acaagttccc 540
   cccagtgggt tttnccttgg cacctanctt accanatcna ttcggaancc attctttgcc 600
   ntggcnttnt nttgggacca ntcttctcac aactgnaccc                       640
<210> 48
   <211> 257
   <212> DNA
   <213> Homo sapiens
<400> 48
   actagtatat gaaaatgtaa atatcacttg tgtactcaaa caaaagttgg tcttaagctt 60
   ccaccttgag cagccttgga aacctaacct gcctctttta gcataatcac attttctaaa 120
   tgattttctt tgttcctgaa aaagtgattt gtattagttt tacatttgtt ttttggaaga 180
   ttatatttgt atatgtatca tcataaaata tttaaataaa aagtatcttt agagtgaaaa 240
   aaaaaaaaaa aaaaaaa                                                 257
<210> 49
   <211> 652
   <212> DNA
   <213> Homo sapiens
<220>
   <221> misc_feature
   <222> 410, 428, 496, 571, 647
   <223> n = A,T,C or G
<400> 49
   actagttcag atgagtggct gctgaagggg cccccttgtc attttcatta taacccaatt 60
   tccacttatt tgaactctta agtcataaat gtataatgac ttatgaatta gcacagttaa 120
   gttgacacta gaaactgccc atttctgtat tacactatca aataggaaac attggaaaga 180
   tggggaaaaa aatcttattt taaaatggct tagaaagttt tcagattact ttgaaaattc 240
   taaacttctt tctgtttcca aaacttgaaa atatgtagat ggactcatgc attaagactg 300
   ttttcaaagc tttcctcaca tttttaaagt gtgattttcc ttttaatata catatttatt 360
   ttctttaaag cagctatatc ccaacccatg actttggaga tatacctatn aaaccaatat 420
   aacagcangg ttattgaagc agctttctca aatgttgctt cagatgtgca agttgcaaat 480
   tttattgtat ttgtanaata caatttttgt tttaaactgt atttcaatct atttctccaa 540
   gatgcttttc atatagagtg aaatatccca ngataactgc ttctgtgtcg tcgcatttga 600
   cgcataactg cacaaatgaa cagtgtatac ctcttggttg tgcattnacc cc          652
<210> 50
   <211> 650
   <212> DNA
   <213> Homo sapiens
<220>
   <221> misc_feature
   <222> 237, 270, 311, 443, 454, 488, 520, 535, 539, 556, 567, 594, 603, 634
   <223> n = A,T,C or G
<400> 50
   ttgcgctttg atttttttag ggcttgtgcc ctgtttcact tatagggtct agaatgcttg 60
   tgttgagtaa aaaggagatg cccaatattc aaagctgcta aatgttctct ttgccataaa 120
   gactccgtgt aactgtgtga acacttggga tttttctcct ctgtcccgag gtcgtcgtct 180
   gctttctttt ttgggttctt tctagaagat tgagaaatgc atatgacagg ctgagancac 240
   ctccccaaac acacaagctc tcagccacan gcagcttctc cacagcccca gcttcgcaca 300
   ggctcctgga nggctgcctg ggggaggcag acatgggagt gccaaggtgg ccagatggtt 360
   ccaggactac aatgtcttta tttttaactg tttgccactg ctgccctcac ccctgcccgg 420
   ctctggagta ccgtctgccc canacaagtg ggantgaaat gggggtgggg gggaacactg 480
   attcccantt agggggtgcc taactgaaca gtagggatan aaggtgtgaa cctgngaant 540
   gcttttataa attatnttcc ttgttanatt tattttttaa tttaatctct gttnaactgc 600
   ccngggaaaa ggggaaaaaa aaaaaaaaat tctntttaaa cacatgaaca            650
<210> 51
   <211> 545
   <212> DNA
   <213> Homo sapiens
<220>
   <221> misc_feature
   <222> 66, 159, 195, 205, 214, 243, 278, 298, 306, 337, 366, 375, 382, 405, 446, 477, 492, 495, 503, 507, 508, 521, 537
   <223> n = A,T,C or G
<400> 51
   tggcgtgcaa ccagggtagc tgaagtttgg gtctgggact ggagattggc cattaggcct 60
   cctganattc cagctccctt ccaccaagcc cagtcttgct acgtggcaca gggcaaacct 120
   gactcccttt gggcctcagt ttcccctccc cttcatgana tgaaaagaat actacttttt 180
   cttgttggtc taacnttgct ggacncaaag tgtngtcatt attgttgtat tgggtgatgt 240
   gtncaaaact gcagaagctc actgcctatg agaggaanta agagagatag tggatganag 300
   ggacanaagg agtcattatt tggtatagat ccacccntcc caacctttct ctcctcagtc 360
   cctgcncctc atgtntctgg tntggtgagt cctttgtgcc accanccatc atgctttgca 420
   ttgctgccat cctgggaagg gggtgnatcg tctcacaact tgttgtcatc gtttganatg 480
   catgctttct tnatnaaaca aanaaannaa tgtttgacag ngtttaaaat aaaaaanaaa 540
   caaaa                                                             545
<210> 52
   <211> 678
   <212> DNA
   <213> Homo sapiens
<220>
   <221> misc_feature
   <222> 98, 119, 121, 131, 136, 139, 140, 142, 143, 163, 168, 172, 176, 184, 189, 190, 191, 200, 201, 205, 207, 221, 223, 229, 230, 237, 240, 241, 255, 264, 266, 267, 276, 280, 288, 289, 291, 297, 301, 306, 308, 314, 315, 326, 332, 335, 337
   <223> n = A,T,C or G
<221> misc_feature
   <222> 339, 341, 343, 344, 345, 347, 350, 355, 356, 358, 362, 363, 372, 379, 395, 397, 398, 400, 403, 412, 414, 421, 423, 431, 435, 438, 439, 450, 457, 463, 467, 471, 474, 480, 483, 484, 487, 490, 491, 492, 493, 499, 500, 504, 508, 518, 536
   <223> n = A,T,C or G
<221> misc_feature
   <222> 538, 549, 551, 552, 554, 556, 557, 562, 563, 567, 571, 572, 576, 579, 590, 592, 595, 598, 606, 609, 613, 620, 622, 624, 626, 631, 634, 638, 641, 647, 654, 660, 661, 674
   <223> n = A,T,C or G
<400> 52
   actagtagaa gaactttgcc gcttttgtgc ctctcacagg cgcctaaagt cattgccatg 60
   ggaggaagac gatttggggg gggagggggg gggggcangg tccgtggggc tttccctant 120
   ntatctccat ntccantgnn cnntgtcgcc tcttccctcg tcncattnga anttantccc 180
   tggnccccnn nccctctccn ncctncncct cccccctccg ncncctccnn ctttttntan 240
   ncttccccat ctccntcccc cctnanngtc ccaacnccgn cagcaatnnc ncacttnctc 300
   nctccncncc tccnnccgtt cttctnttct cnacntntnc ncnnntnccn tgccnntnaa 360
   annctctccc cnctgcaanc gattctctcc ctccncnnan ctntccactc cntncttctc 420
   ncncgctcct nttcntcnnc ccacctctcn ccttcgnccc cantacnctc nccncccttn 480
   cgnntcnttn nnntcctcnn accncccncc tcccttcncc cctcttctcc ccggtntntc 540
   tctctcccnc nncncnncct cnncccntcc nngcgnccnt ttccgccccn cnccnccntt 600
   ccttcntcnc cantccatcn cntntnccat nctncctncc nctcacnccc gctncccccn 660
   ntctctttca cacngtcc                                               678
<210> 53
   <211> 502
   <212> DNA
   <213> Homo sapiens
<220>
   <221> misc_feature
   <222> 139, 146, 215, 217, 257, 263, 289, 386, 420, 452, 457, 461, 466, 482, 486
   <223> n = A,T,C or G
<400> 53
   tgaagatcct ggtgtcgcca tgggccgccg ccccgcccgt tgttaccggt attgtaagaa 60
   caagccgtac ccaaagtctc gcttctgccg aggtgtccct gatgccaaaa ttcgcatttt 120
   tgacctgggg cggaaaaang caaaantgga tgagtctccg ctttgtggcc acatggtgtc 180
   agatcaatat gagcagctgt cctctgaagc cctgnangct gcccgaattt gtgccaataa 240
   gtacatggta aaaagtngtg gcnaagatgc ttccatatcc gggtgcggnt ccaccccttc 300
   cacgtcatcc gcatcaacaa gatgttgtcc tgtgctgggg ctgacaggct cccaacaggc 360
   atgcgaagtg cctttggaaa acccanggca ctgtggccag ggttcacatt gggccaattn 420
   atcatgttca tccgcaccaa ctgcagaaca angaacntgt naattnaagc cctgcccagg 480
   gncaanttca aatttcccgg cc                                          502
<210> 54
   <211> 494
   <212> DNA
   <213> Homo sapiens
<220>
   <221> misc_feature
   <222> 431, 442, 445
   <223> n = A,T,C or G
<400> 54
   actagtccaa gaaaaatatg cttaatgtat attacaaagg ctttgtatat gttaacctgt 60
   tttaatgcca aaagtttgct ttgtccacaa tttccttaag acctcttcag aaagggattt 120
   gtttgcctta atgaatactg ttgggaaaaa acacagtata atgagtgaaa agggcagaag 180
   caagaaattt ctacatctta gcgactccaa gaagaatgag tatccacatt tagatggcac 240
   attatgagga ctttaatctt tccttaaaca caataatgtt ttcttttttc ttttattcac 300
   atgatttcta agtatatttt tcatgcagga cagtttttca accttgatgt acagtgactg 360
   tgttaaattt ttctttcagt ggcaacctct ataatcttta aaatatggtg agcatcttgt 420
   ctgttttgaa ngggatatga cnatnaatct atcagatggg aaatcctgtt tccaagttag 480
   aaaaaaaaaa aaaa                                                   494
<210> 55
   <211> 606
   <212> DNA
   <213> Homo sapiens
<220>
   <221> misc_feature
   <222> 375, 395, 511, 542, 559, 569, 578, 581
   <223> n = A,T,C or G
<400> 55
   actagtaaaa agcagcattg ccaaataatc cctaattttc cactaaaaat ataatgaaat 60
   gatgttaagc tttttgaaaa gtttaggtta aacctactgt tgttagatta atgtatttgt 120
   tgcttccctt tatctggaat gtggcattag cttttttatt ttaaccctct ttaattctta 180
   ttcaattcca tgacttaagg ttggagagct aaacactggg atttttggat aacagactga 240
   cagttttgca taattataat cggcattgta catagaaagg atatggctac cttttgttaa 300
   atctgcactt tctaaatatc aaaaaaggga aatgaagtat aaatcaattt ttgtataatc 360
   tgtttgaaac atgantttta tttgcttaat attanggctt tgcccttttc tgttagtctc 420
   ttgggatcct gtgtaaaact gttctcatta aacaccaaac agttaagtcc attctctggt 480
   actagctaca aattccgttt catattctac ntaacaattt aaattaactg aaatatttct 540
   anatggtcta cttctgtcnt ataaaaacna aacttgantt nccaaaaaaa aaaaaaaaaa 600
   aaaaaa                                                             606
<210> 56
   <211> 183
   <212> DNA
   <213> Homo sapiens
<400> 56
   actagtatat ttaaacttac aggcttattt gtaatgtaaa ccaccatttt aatgtactgt 60
   aattaacatg gttataatac gtacaatcct tccctcatcc catcacacaa ctttttttgt 120
   gtgtgataaa ctgattttgg tttgcaataa aaccttgaaa aataaaaaaa aaaaaaaaaa 180
   aaa                                                                183
<210> 57
   <211> 622
   <212> DNA
   <213> Homo sapiens
<220>
   <221> misc_feature
   <222> 358, 368, 412, 414, 425, 430, 453, 455, 469, 475, 495, 499, 529, 540, 564, 575, 590
   <223> n = A,T,C or G
<400> 57
   actagtcact actgtcttct ccttgtagct aatcaatcaa tattcttccc ttgcctgtgg 60
   gcagtggaga gtgctgctgg gtgtacgctg cacctgccca ctgagttggg gaaagaggat 120
   aatcagtgag cactgttctg ctcagagctc ctgatctacc ccacccccta ggatccagga 180
   ctgggtcaaa gctgcatgaa accaggccct ggcagcaacc tgggaatggc tggaggtggg 240
   agagaacctg acttctcttt ccctctccct cctccaacat tactggaact ctatcctgtt 300
   agggatcttc tgagcttgtt tccctgctgg gtgggacaga agacaaagga gaagggangg 360
   tctacaanaa gcagcccttc tttgtcctct ggggttaatg agcttgacct ananttcatg 420
   gaganaccan aagcctctga tttttaattt ccntnaaatg tttgaagtnt atatntacat 480
   atatatattt ctttnaatnt ttgagtcttt gatatgtctt aaaatccant ccctctgccn 540
   gaaacctgaa ttaaaaccat gaanaaaaat gtttncctta aagatgttan taattaattg 600
   aaacttgaaa aaaaaaaaaa aa                                           622
<210> 58
   <211> 433
   <212> DNA
   <213> Homo sapiens
<400> 58
   gaacaaattc tgattggtta tgtaccgtca aaagacttga agaaatttca tgattttgca 60
   gtgtggaagc gttgaaaatt gaaagttact gcttttccac ttgctcatat agtaaaggga 120
   tcctttcagc tgccagtgtt gaataatgta tcatccagag tgatgttatc tgtgacagtc 180
   accagcttta agctgaacca ttttatgaat accaaataaa tagacctctt gtactgaaaa 240
   catatttgtg actttaatcg tgctgcttgg atagaaatat ttttactggt tcttctgaat 300
   tgacagtaaa cctgtccatt atgaatggcc tactgttcta ttatttgttt tgacttgaat 360
   ttatccacca aagacttcat ttgtgtatca tcaataaagt tgtatgtttc aactgaaaaa 420
   aaaaaaaaaa aaa 433
<210> 59
   <211> 649
   <212> DNA
   <213> Homo sapiens
<220>
   <221> misc_feature
   <222> 22, 190, 217, 430, 433, 484, 544, 550, 577, 583, 594
   <223> n = A,T,C or G
<400> 59
   actagttatt atctgacttt cnggttataa tcattctaat gagtgtgaag tagcctctgg 60
   tgtcatttgg atttgcattt ctctgatgag tgatgctatc aagcaccttt gctggtgctg 120
   ttggccatat gtgtatgttc cctggagaag tgtctgtgct gagccttggc ccacttttta 180
   attaggcgtn tgtcttttta ttactgagtt gtaaganttc tttatatatt ctggattcta 240
   gacccttatc agatacatgg tttgcaaata ttttctccca ttctgtgggt tgtgttttca 300
   ctttatcgat aatgtcctta gacatataat aaatttgtat tttaaaagtg acttgatttg 360
   ggctgtgcaa ggtgggctca cgcttgtaat cccagcactt tgggagactg aggtgggtgg 420
   atcatatgan gangctagga gttcgaggtc agcctggcca gcatagcgaa aacttgtctc 480
   tacnaaaaat acaaaaatta gtcaggcatg gtggtgcacg tctgtaatac cagcttctca 540
   ggangctgan gcacaaggat cacttgaacc ccagaangaa gangttgcag tganctgaag 600
   atcatgccag ggcaacaaaa atgagaactt gtttaaaaaa aaaaaaaaa 649
<210> 60
   <211> 423
   <212> DNA
   <213> Homo sapiens
<220>
   <221> misc_feature
   <222> 209, 222, 277, 389, 398
   <223> n = A,T,C or G
<400> 60
   actagttcag gccttccagt tcactgacaa acatggggaa gtgtgcccag ctggctggaa 60
   acctggcagt gataccatca agcctgatgt ccaaaagagc aaagaatatt tctccaagca 120
   gaagtgagcg ctgggctgtt ttagtgccag gctgcggtgg gcagccatga gaacaaaacc 180
   tcttctgtat tttttttttc cattagtana acacaagact cngattcagc cgaattgtgg 240
   tgtcttacaa ggcagggctt tcctacaggg ggtgganaaa acagcctttc ttcctttggt 300
   aggaatggcc tgagttggcg ttgtgggcag gctactggtt tgtatgatgt attagtagag 360
   caacccatta atcttttgta gtttgtatna aacttganct gagaccttaa acaaaaaaaa 420
   aaa 423
<210> 61
   <211> 423
   <212> DNA
   <213> Homo sapiens
<220>
   <221> misc_feature
   <222> 195, 285, 295, 329, 335, 340, 347, 367, 382, 383, 391, 396, 418
   <223> n = A,T,C or G
<400> 61
   cgggactgga atgtaaagtg aagttcggag ctctgagcac gggctcttcc cgccgggtcc 60
   tccctcccca gaccccagag ggagaggccc accccgccca gccccgcccc agcccctgct 120
   caggtctgag tatggctggg agtcgggggc cacaggcctc tagctgtgct gctcaagaag 180
   actggatcag ggtanctaca agtggccggg ccttgccttt gggattctac cctgttccta 240
   atttggtgtt ggggtgcggg gtccctggcc cccttttcca cactncctcc ctccngacag 300
   caacctccct tggggcaatt gggcctggnt ctccncccgn tgttgcnacc ctttgttggt 360
   ttaaggnctt taaaaatgtt annttttccc ntgccngggt taaaaaagga aaaaactnaa 420
   aaa                                                                423
<210> 62
   <211> 683
   <212> DNA
   <213> Homo sapiens
<220>
   <221> misc_feature
   <222> 218, 291, 305, 411, 416, 441, 443, 453, 522, 523, 536, 542, 547, 566, 588, 592, 595, 603, 621, 628, 630, 632, 644, 645, 648, 655, 660, 672, 674, 676, 677, 683
   <223> n = A,T,C or G
<400> 62
   gctggagagg ggtacggact ttcttggagt tgtcccaggt tggaatgaga ctgaactcaa 60
   gaagagaccc taagagactg gggaatggtt cctgccttca ggaaagtgaa agacgcttag 120
   gctgtcaaca cttaaaggaa gtccccttga agcccagagt ggacagacta gacccattga 180
   tggggccact ggccatggtc cgtggacaag acattccngt gggccatggc acaccggggg 240
   ggatcaaaat gtgtacttgt ggggtctcgc cccttgccaa aaccaaacca ntcccactcc 300
   tgtcnttgga ctttcttccc attccctcct ccccaaatgc acttcccctc ctccctctgc 360
   ccctcctgtg tttttggaat tctgtttccc tcaaaattgt taatttttta nttttngacc 420
   atgaacttat gtttggggtc nangttcccc ttnccaatgc atactaatat attaatggtt 480
   atttattttt gaaatatttt ttaatgaact tggaaaaaat tnntggaatt tccttncttc 540
   cnttttnttt ggggggggtg gggggntggg ttaaaatttt tttggaancc cnatnggaaa 600
   ttnttacttg gggcccccct naaaaaantn anttccaatt cttnnatngc ccctnttccn 660
   ctaaaaaaaa ananannaaa aan                                          683
<210> 63
   <211> 731
   <212> DNA
   <213> Homo sapiens
<220>
   <221> misc_feature
   <222> 237, 249, 263, 288, 312, 317, 323, 326, 337, 352, 362, 370, 377, 400, 411, 414, 434, 436, 446, 457, 473, 486, 497, 498, 502, 512, 531, 546, 554, 563, 565, 566, 588, 597, 608, 611, 613, 615, 627, 632, 640, 641, 644, 654, 660, 663, 665
   <223> n = A,T,C or G
<221> misc_feature
   <222> 671, 678, 692, 697, 698, 699, 704, 705, 712, 714, 717, 718, 719, 723, 725, 730, 731
   <223> n = A,T,C or G
<400> 63
   actagtcata aagggtgtgc gcgtcttcga cgtggcggtc ttggcgccac tgctgcgaga 60
   cccggccctg gacctcaagg tcatccactt ggtgcgtgat ccccgcgcgg tggcgagttc 120
   acggatccgc tcgcgccacg gcctcatccg tgagagccta caggtggtgc gcagccgaga 180
   ccgcgagctc accgcatgcc cttcttggag gccgcgggcc acaagcttgg cgcccanaaa 240
   gaaggcgtng ggggcccgca aantaccacg ctctgggcgc tatggaangt cctcttgcaa 300
   taatattggt tnaaaanctg canaanagcc cctgcanccc cctgaactgg gntgcagggc 360
   cncttacctn gtttggntgc ggttacaaag aacctgtttn ggaaaaccct nccnaaaacc 420
   ttccgggaaa attntncaaa tttttnttgg ggaattnttg ggtaaacccc ccnaaaatgg 480
   gaaacntttt tgccctnnaa antaaaccat tnggttccgg gggccccccc ncaaaaccct 540
   tttttntttt tttntgcccc cantnncccc ccggggcccc tttttttngg ggaaaanccc 600
   cccccctncc nanantttta aaagggnggg anaatttttn nttncccccc gggncccccn 660
   ggngntaaaa nggtttcncc cccccgaggg gnggggnnnc ctcnnaaacc cntntcnnna 720
   ccncnttttn n                                                        731
<210> 64
   <211> 313
   <212> DNA
   <213> Homo sapiens
<220>
   <221> misc_feature
   <222> 240
   <223> n = A,T,C or G
<400> 64
   actagttgtg caaaccacga ctgaagaaag acgaaaagtg ggaaataact tgcaacgtct 60
   gttagagatg gttgctacac atgttgggtc tgtagagaaa catcttgagg agcagattgc 120
   taaagttgat agagaatatg aagaatgcat gtcagaagat ctctcggaaa atattaaaga 180
   gattagagat aagtatgaga agaaagctac tctaattaag tcttctgaag aatgaagatn 240
   aaatgttgat catgtatata tatccatagt gaataaaatt gtctcagtaa agttgtaaaa 300
   aaaaaaaaaa aaa                                                      313
<210> 65
   <211> 420
   <212> DNA
   <213> Homo sapiens
<220>
   <221> misc_feature
   <222> 400, 402, 403, 404, 405, 406, 409, 411, 412, 414, 415, 416
   <223> n = A,T,C or G
<400> 65
   actagttccc tggcaggcaa gggcttccaa ctgaggcagt gcatgtgtgg cagagagagg 60
   caggaagctg gcagtggcag cttctgtgtc tagggagggg tgtggctccc tccttccctg 120
   tctgggaggt tggagggaag aatctaggcc ttagcttgcc ctcctgccac ccttcccctt 180
   gtagatactg ccttaacact ccctcctctc tcagctgtgg ctgccaccca agccaggttt 240
   ctccgtgctc actaatttat ttccaggaaa ggtgtgtgga agacatgagc cgtgtataat 300
   atttgtttta acattttcat tgcaagtatt gaccatcatc cttggttgtg tatcgttgta 360
   acacaaatta atgatattaa aaagcatcca aacaaagccn annnnnaana nnannngaaa 420
<210> 66
   <211> 676
   <212> DNA
   <213> Homo sapiens
<220>
   <221> misc_feature
   <222> 328, 454, 505, 555, 586, 612, 636, 641
   <223> n = A,T,C or G
<400> 66
   actagtttcc tatgatcatt aaactcattc tcagggttaa gaaaggaatg taaatttctg 60
   cctcaatttg tacttcatca ataagttttt gaagagtgca gatttttagt caggtcttaa 120
   aaataaactc acaaatctgg atgcatttct aaattctgca aatgtttcct ggggtgactt 180
   aacaaggaat aatcccacaa tatacctagc tacctaatac atggagctgg ggctcaaccc 240
   actgttttta aggatttgcg cttacttgtg gctgaggaaa aataagtagt tccgagggaa 300
   gtagttttta aatgtgagct tatagatngg aaacagaata tcaacttaat tatggaaatt 360
   gttagaaacc tgttctcttg ttatctgaat cttgattgca attactattg tactggatag 420
   actccagccc attgcaaagt ctcagatatc ttanctgtgt agttgaattc cttggaaatt 480
   ctttttaaga aaaaattgga gtttnaaaga aataaacccc tttgttaaat gaagcttggc 540
   tttttggtga aaaanaatca tcccgcaggg cttattgttt aaaaanggaa ttttaagcct 600
   ccctggaaaa anttgttaat taaatgggga aaatgntggg naaaaattat ccgttagggt 660
   ttaaagggaa aactta 676
<210> 67
   <211> 620
   <212> DNA
   <213> Homo sapiens
<220>
   <221> misc_feature
   <222> 419, 493, 519, 568, 605, 610
   <223> n = A,T,C or G
<400> 67
   caccattaaa gctgcttacc aagaacttcc ccagcatttt gacttccttg tttgatagct 60
   gaattgtgag caggtgatag aagagccttt ctagttgaac atacagataa tttgctgaat 120
   acattccatt taatgaaggg gttacatctg ttacgaagct actaagaagg agcaagagca 180
   taggggaaaa aaatctgatc agaacgcatc aaactcacat gtgccccctc tactacaaac 240
   agattgtagt gctgtggtgg tttattccgt tgtgcagaac ttgcaagctg agtcactaaa 300
   cccaaagaga ggaaattata ggttagttaa acattgtaat cccaggaact aagtttaatt 360
   cacttttgaa gtgttttgtt ttttattttt ggtttgtctg atttactttg ggggaaaang 420
   ctaaaaaaaa agggatatca atctctaatt cagtgcccac taaaagttgt ccctaaaaag 480
   tctttactgg aanttatggg actttttaag ctccaggtnt tttggtcctc caaattaacc 540
   ttgcatgggc cccttaaaat tgttgaangg cattcctgcc tctaagtttg gggaaaattc 600
   ccccnttttn aaaatttgga 620
<210> 68
   <211> 551
   <212> DNA
   <213> Homo sapiens
<220>
   <221> misc_feature
   <222> 286, 464, 480, 501, 502, 518, 528, 533, 536, 537, 538, 539, 540, 541, 543, 544, 545, 547, 548, 549
   <223> n = A,T,C or G
<400> 68
   actagtagct ggtacataat cactgaggag ctatttctta acatgctttt atagaccatg 60
   ctaatgctag accagtattt aagggctaat ctcacacctc cttagctgta agagtctggc 120
   ttagaacaga cctctctgtg caataacttg tggccactgg aaatccctgg gccggcattt 180
   gtattggggt tgcaatgact cccaagggcc aaaagagtta aaggcacgac tgggatttct 240
   tctgagactg tggtgaaact ccttccaagg ctgagggggt cagtangtgc tctgggaggg 300
   actcggcacc actttgatat tcaacaagcc acttgaagcc caattataaa attgttattt 360
   tacagctgat ggaactcaat ttgaaccttc aaaactttgt tagtttatcc tattatattg 420
   ttaaacctaa ttacatttgt ctagcattgg atttggttcc tgtngcatat gtttttttcn 480
   cctatgtgct cccctccccc nnatcttaat ttaaaccnca attttgcnat tcnccnnnnn 540
   nannnannna a 551
<210> 69
   <211> 396
   <212> DNA
   <213> Homo sapiens
<220>
   <221> misc_feature
   <222> 235, 310, 323, 381
   <223> n = A,T,C or G
<400> 69
   cagaaatgga aagcagagtt ttcatttctg tttataaacg tctccaaaca aaaatggaaa 60
   gcagagtttt cattaaatcc ttttaccttt tttttttctt ggtaatcccc tcaaataaca 120
   gtatgtggga tattgaatgt taaagggata tttttttcta ttatttttat aattgtacaa 180
   aattaagcaa atgttaaaag ttttatatgc tttattaatg ttttcaaaag gtatnataca 240
   tgtgatacat tttttaagct tcagttgctt gtcttctggt actttctgtt atgggctttt 300
   ggggagccan aaaccaatct acnatctctt tttgtttgcc aggacatgca ataaaattta 360
   aaaaataaat aaaaactatt nagaaattga aaaaaa 396
<210> 70
   <211> 536
   <212> DNA
   <213> Homo sapiens
<220>
   <221> misc_feature
   <222> 388, 446, 455
   <223> n = A, T, C or G
<400> 70
   actagtgcaa aagcaaatat aaacatcgaa aaggcgttcc tcacgttagc tgaagatatc 60
   cttcgaaaga cccctgtaaa agagcccaac agtgaaaatg tagatatcag cagtggagga 120
   ggcgtgacag gctggaagag caaatgctgc tgagcattct cctgttccat cagttgccat 180
   ccactacccc gttttctctt cttgctgcaa aataaaccac tctgtccatt tttaactcta 240
   aacagatatt tttgtttctc atcttaacta tccaagccac ctattttatt tgttctttca 300
   tctgtgactg cttgctgact ttatcataat tttcttcaaa caaaaaaatg tatagaaaaa 360
   tcatgtctgt gacttcattt ttaaatgnta cttgctcagc tcaactgcat ttcagttgtt 420
   ttatagtcca gttcttatca acattnaaac ctatngcaat catttcaaat ctattctgca 480
   aattgtataa gaataaaagt tagaatttaa caattaaaaa aaaaaaaaaa aaaaaa     536
<210> 71
   <211> 865
   <212> DNA
   <213> Homo sapiens
<220>
   <221> misc_feature
   <222> 22, 35, 39, 56, 131, 138, 146, 183, 194, 197, 238, 269, 277, 282, 297, 316, 331, 336, 340, 341, 346, 349, 370, 376, 381, 382, 392, 396, 397, 401, 433, 444, 445, 454, 455, 469, 472, 477, 480, 482, 489, 497, 499, 511, 522, 526, 527
   <223> n = A,T,C or G
<221> misc_feature
   <222> 545, 553, 556, 567, 574, 580, 610, 613, 634, 638, 639, 663, 672, 689, 693, 694, 701, 704, 713, 723, 729, 732, 743, 744, 749, 761, 765, 767, 769, 772, 774, 780, 783, 788, 792, 803, 810, 824, 840, 848
   <223> n = A,T,C or G
<400> 71
   gacaaagcgt taggagaaga anagaggcag ggaanactnc ccaggcacga tggccncctt 60
   cccaccagca accagcgccc cccaccagcc cccaggcccg gacgacgaag actccatcct 120
   ggattaatct nacctctntc gcctgnccca ttcctacctc ggaggtggag gccggaaagg 180
   tcncaccaag aganaanctg ctgccaacac caaccgcccc agccctggcg ggcacganag 240
   gaaactggtg accaatctgc agaattctna gaggaanaag cnaggggccc cgcgctnaga 300
   cagagctgga tatgangcca gaccatggac nctacncccn ncaatncana cgggactgcg 360
   gaagatggan gacccncgac nngatcaggc cngctnncca nccccccacc cctatgaatt 420
   attcccgctg aangaatctc tganngcctt ccannaaagc gcctccccnc cnaacgnaan 480
   tncaacatng ggattanang ctgggaactg naaggggcaa ancctnnaat atccccagaa 540
   acaanctctc ccnaanaaac tggggcncct catnggtggn accaactatt aactaaaccg 600
   cacgccaagn aantataaaa ggggggcccc tccncggnng accccctttt gtcccttaat 660
   ganggttatc cnccttgcgt accatggtnc ccnnttctgt ntgnatgttt ccnctcccct 720
   ccncctatnt cnagccgaac tcnnatttnc ccgggggtgc natcnantng tncncctttn 780
   ttngttgncc cngccctttc cgncggaacn cgtttccccg ttantaacgg cacccggggn 840
   aagggtgntt ggccccctcc ctccc                                       865
<210> 72
   <211> 560
   <212> DNA
   <213> Homo sapiens
<220>
   <221> misc_feature
   <222> 83, 173, 183, 186, 209, 211, 215, 255, 321, 322, 323, 335, 344, 357, 361, 368, 394, 412, 415, 442, 455, 469, 472, 475, 487, 513, 522, 528, 531, 534, 546
   <223> n = A,T,C or G
<400> 72
   cctggacttg tcttggttcc agaacctgac gacccggcga cggcgacgtc tcttttgact 60
   aaaagacagt gtccagtgct ccngcctagg agtctacggg gaccgcctcc cgcgccgcca 120
   ccatgcccaa cttctctggc aactggaaaa tcatccgatc ggaaaacttc gangaattgc 180
   tcnaantgct gggggtgaat gtgatgctna ngaanattgc tgtggctgca gcgtccaagc 240
   cagcagtgga gatcnaacag gagggagaca ctttctacat caaaacctcc accaccgtgc 300
   gcaccacaaa gattaacttc nnngttgggg aggantttga ggancaaact gtggatngga 360
   ngcctgtnaa aacctggtga aatgggagaa tganaataaa atggtctgtg ancanaaact 420
   cctgaaagga gaaggccccc anaactcctg gaccngaaaa actgacccnc cnatngggga 480
   actgatnctt gaaccctgaa cgggcgggat ganccttttt tnttgccncc naangggttc 540
   tttccntttc cccaaaaaaa                                             560
<210> 73
   <211> 379
   <212> DNA
   <213> Homo sapiens
<220>
   <221> misc_feature
   <222> 8, 17, 18, 21, 26, 29, 30, 32, 53, 56, 67, 71, 81, 102, 104, 111, 112, 114, 119, 122, 124, 125, 134, 144, 146, 189, 190, 214, 215, 219, 220, 235, 237, 246, 280, 288, 302, 310, 313, 319, 322, 343, 353, 354
   <223> n = A,T,C or G
<400> 73
   ctggggancc ggcggtnngc nccatntcnn gncgcgaagg tggcaataaa aanccnctga 60
   aaccgcncaa naaacatgcc naagatatgg acgaggaaga tngngctttc nngnacaanc 120
   gnanngagga acanaacaaa ctcnangagc tctcaagcta atgccgcggg gaaggggccc 180
   ttggccacnn gtggaattaa gaaatctggc aaanngtann tgttccttgt gcctnangag 240
   ataagngacc ctttatttca tctgtattta aacctctctn ttccctgnca taacttcttt 300
   tnccacgtan agntggaant anttgttgtc ttggactgtt gtncatttta gannaaactt 360
   ttgttcaaaa aaaaaataa                                              379
<210> 74
   <211> 437
   <212> DNA
   <213> Homo sapiens
<220>
   <221> misc_feature
   <222> 145, 355
   <223> n = A,T,C or G
<400> 74
   actagttcag actgccacgc caaccccaga aaatacccca catgccagaa aagtgaagtc 60
   ctaggtgttt ccatctatgt ttcaatctgt ccatctacca ggcctcgcga taaaaacaaa 120
   acaaaaaaac gctgccaggt tttanaagca gttctggtct caaaaccatc aggatcctgc 180
   caccagggtt cttttgaaat agtaccacat gtaaaaggga atttggcttt cacttcatct 240
   aatcactgaa ttgtcaggct ttgattgata attgtagaaa taagtagcct tctgttgtgg 300
   gaataagtta taatcagtat tcatctcttt gttttttgtc actcttttct ctctnattgt 360
   gtcatttgta ctgtttgaaa aatatttctt ctataaaatt aaactaacct gccttaaaaa 420
   aaaaaaaaaa aaaaaaa 437
<210> 75
   <211> 579
   <212> DNA
   <213> Homo sapiens
<220>
   <221> misc_feature
   <222> 440, 513, 539, 551
   <223> n = A,T,C or G
<400> 75
   ctccgtcgcc gccaagatga tgtgcggggc gccctccgcc acgcagccgg ccaccgccga 60
   gacccagcac atcgccgacc aggtgaggtc ccagcttgaa gagaaagaaa acaagaagtt 120
   ccctgtgttt aaggccgtgt cattcaagag ccaggtggtc gcggggacaa actacttcat 180
   caaggtgcac gtcggcgacg aggacttcgt acacctgcga gtgttccaat ctctccctca 240
   tgaaaacaag cccttgacct tatctaacta ccagaccaac aaagccaagc atgatgagct 300
   gacctatttc tgatcctgac tttggacaag gcccttcagc cagaagactg acaaagtcat 360
   cctccgtcta ccagagcgtg cacttgtgat cctaaaataa gcttcatctc cgggctgtgc 420
   ccttggggtg gaaggggcan gatctgcact gcttttgcat ttctcttcct aaatttcatt 480
   gtgttgattc tttccttcca ataggtgatc ttnattactt tcagaatatt ttccaaatna 540
   gatatatttt naaaatcctt aaaaaaaaaa aaaaaaaaa 579
<210> 76
   <211> 666
   <212> DNA
   <213> Homo sapiens
<220>
   <221> misc_feature
   <222> 411, 470, 476, 491, 506, 527, 560, 570, 632, 636, 643, 650, 654, 658
   <223> n = A,T,C or G
<400> 76
   gtttatccta tctctccaac cagattgtca gctccttgag ggcaagagcc acagtatatt 60
   tccctgtttc ttccacagtg cctaataata ctgtggaact aggttttaat aattttttaa 120
   ttgatgttgt tatgggcagg atggcaacca gaccattgtc tcagagcagg tgctggctct 180
   ttcctggcta ctccatgttg gctagcctct ggtaacctct tacttattat cttcaggaca 240
   ctcactacag ggaccaggga tgatgcaaca tccttgtctt tttatgacag gatgtttgct 300
   cagcttctcc aacaataaaa agcacgtggt aaaacacttg cggatattct ggactgtttt 360
   taaaaaatat acagtttacc gaaaatcata ttatcttaca atgaaaagga ntttatagat 420
   cagccagtga acaacctttt cccaccatac aaaaattcct tttcccgaan gaaaanggct 480
   ttctcaataa ncctcacttt cttaanatct tacaagatag ccccganatc ttatcgaaac 540
   tcattttagg caaatatgan ttttattgtn cgttacttgt ttcaaaattt ggtattgtga 600
   atatcaatta ccacccccat ctcccatgaa anaaanggga aanggtgaan ttcntaancg 660
   cttaaa 666
<210> 77
   <211> 396
   <212> DNA
   <213> Homo sapiens
<220>
   <221> misc_feature
   <222> 31, 54, 125, 128, 136, 163, 168, 198
   <223> n = A,T,C or G
<400> 77
   ctgcagcccg ggggatccac taatctacca nggttatttg gcagctaatt ctanatttgg 60
   atcattgccc aaagttgcac ttgctggtct cttgggattt ggccttggaa aggtatcata 120
   catanganta tgccanaata aattccattt ttttgaaaat canctccntg gggctggttt 180
   tggtccacag cataacangc actgcctcct tacctgtgag gaatgcaaaa taaagcatgg 240
   attaagtgag aagggagact ctcagccttc agcttcctaa attctgtgtc tgtgactttc 300
   gaagtttttt aaacctctga atttgtacac atttaaaatt tcaagtgtac tttaaaataa 360
   aatacttcta atgggaacaa aaaaaaaaaa aaaaaa 396
<210> 78
   <211> 793
   <212> DNA
   <213> Homo sapiens
<220>
   <221> misc_feature
   <222> 309, 492, 563, 657, 660, 703, 708, 710, 711, 732, 740, 748, 758, 762, 765, 787
   <223> n = A,T,C or G
<400> 78
   gcatcctagc cgccgactca cacaaggcag gtgggtgagg aaatccagag ttgccatgga 60
   gaaaattcca gtgtcagcat tcttgctcct tgtggccctc tcctacactc tggccagaga 120
   taccacagtc aaacctggag ccaaaaagga cacaaaggac tctcgaccca aactgcccca 180
   gaccctctcc agaggttggg gtgaccaact catctggact cagacatatg aagaagctct 240
   atataaatcc aagacaagca acaaaccctt gatgattatt catcacttgg atgagtgccc 300
   acacagtcna gctttaaaga aagtgtttgc tgaaaataaa gaaatccaga aattggcaga 360
   gcagtttgtc ctcctcaatc tggtttatga aacaactgac aaacaccttt ctcctgatgg 420
   ccagtatgtc ccaggattat gtttgttgac ccatctctga cagttgaagc cgatatcctg 480
   ggaagatatt cnaaccgtct ctatgcttac aaactgcaga tacgctctgt tgcttgacac 540
   atgaaaaagc tctcaagttg ctnaaaatga attgtaagaa aaaaaatctc cagccttctg 600
   tctgtcggct tgaaaattga aaccagaaaa atgtgaaaaa tggctattgt ggaacanatn 660
   gacacctgat taggttttgg ttatgttcac cactattttt aanaaaanan nttttaaaat 720
   ttggttcaat tntctttttn aaacaatntg tttctacntt gnganctgat ttctaaaaaa 780
   aataatnttt ggc 793
<210> 79
   <211> 456
   <212> DNA
   <213> Homo sapiens
<220>
   <221> misc_feature
   <222> 89, 195, 255, 263, 266, 286, 353, 384, 423, 425, 436, 441
   <223> n = A,T,C or G
<400> 79
   actagtatgg ggtgggaggc cccacccttc tcccctaggc gctgttcttg ctccaaaggg 60
   ctccgtggag agggactggc agagctgang ccacctgggg ctggggatcc cactcttctt 120
   gcagctgttg agcgcaccta accactggtc atgcccccac ccctgctctc cgcacccgct 180
   tcctcccgac cccangacca ggctacttct cccctcctct tgcctccctc ctgcccctgc 240
   tgcctctgat cgtangaatt gangantgtc ccgccttgtg gctganaatg gacagtggca 300
   ggggctggaa atgggtgtgt gtgtgtgtgt gtgtgtgtgt gtgtgtgtgt gcnccccccc 360
   tgcaagaccg agattgaggg aaancatgtc tgctgggtgt gaccatgttt cctctccata 420
   aantncccct gtgacnctca naaaaaaaaa aaaaaa 456
<210> 80
   <211> 284
   <212> DNA
   <213> Homo sapiens
<220>
   <221> misc_feature
   <222> 283
   <223> n = A,T,C or G
<400> 80
   ctttgtacct ctagaaaaga taggtattgt gtcatgaaac ttgagtttaa attttatata 60
   taaaactaaa agtaatgctc actttagcaa cacatactaa aattggaacc atactgagaa 120
   gaatagcatg acctccgtgc aaacaggaca agcaaatttg tgatgtgttg attaaaaaga 180
   aataaataaa tgtgtatatg tgtaacttgt atgtttatgt ggaatacaga ttgggaaata 240
   aaatgtattt cttactgtga aaaaaaaaaa aaaaaaaaaa aana 284
<210> 81
   <211> 671
   <212> DNA
   <213> Homo sapiens
<220>
   <221> misc_feature
   <222> 388, 505, 600, 603, 615, 642, 644, 660
   <223> n = A,T,C or G
<400> 81
   gccaccaaca ttccaagcta ccctgggtac ctttgtgcag tagaagctag tgagcatgtg 60
   agcaagcggt gtgcacacgg agactcatcg ttataattta ctatctgcca agagtagaaa 120
   gaaaggctgg ggatatttgg gttggcttgg ttttgatttt ttgcttgttt gtttgttttg 180
   tactaaaaca gtattatctt ttgaatatcg tagggacata agtatataca tgttatccaa 240
   tcaagatggc tagaatggtg cctttctgag tgtctaaaac ttgacacccc tggtaaatct 300
   ttcaacacac ttccactgcc tgcgtaatga agttttgatt catttttaac cactggaatt 360
   tttcaatgcc gtcattttca gttagatnat tttgcacttt gagattaaaa tgccatgtct 420
   atttgattag tcttattttt ttatttttac aggcttatca gtctcactgt tggctgtcat 480
   tgtgacaaag tcaaataaac ccccnaggac aacacacagt atgggatcac atattgtttg 540
   acattaagct ttggccaaaa aatgttgcat gtgttttacc tcgacttgct aaatcaatan 600
   canaaaggct ggctnataat gttggtggtg aaataattaa tnantaacca aaaaaaaaan 660
   aaaaaaaaaa a                                                      671
<210> 82
   <211> 217
   <212> DNA
   <213> Homo sapiens
<220>
   <221> misc_feature
   <222> 35
   <223> n = A,T,C or G
<400> 82
   ctgcagatgt ttcttgaatg ctttgtcaaa ttaanaaagt taaagtgcaa taatgtttga 60
   agacaataag tggtggtgta tcttgtttct aataagataa acttttttgt ctttgcttta 120
   tcttattagg gagttgtatg tcagtgtata aaacatactg tgtggtataa caggcttaat 180
   aaattcttta aaaggaaaaa aaaaaaaaaa aaaaaaa                          217
<210> 83
   <211> 460
   <212> DNA
   <213> Homo sapiens
<220>
   <221> misc_feature
   <222> 104, 118, 172, 401, 422, 423, 444, 449
   <223> n = A,T,C or G
<400> 83
   cgcgagtggg agcaccagga tctcgggctc ggaacgagac tgcacggatt gttttaagaa 60
   aatggcagac aaaccagaca tgggggaaat cgccagcttc gatnaggcca agctgaanaa 120
   aacggagacg caggagaaga acaccctgcc gaccaaagag accattgagc angagaagcg 180
   gagtgaaatt tcctaagatc ctggaggatt tcctaccccc gtcctcttcg agaccccagt 240
   cgtgatgtgg aggaagagcc acctgcaaga tggacacgag ccacaagctg cactgtgaac 300
   ctgggcactc cgcgccgatg ccaccggcct gtgggtctct gaagggaccc cccccaatcg 360
   gactgccaaa ttctccggtt tgccccggga tattatacaa nattatttgt atgaataatg 420
   annataaaac acacctcgtg gcancaaana aaaaaaaaaa 460
<210> 84
   <211> 323
   <212> DNA
   <213> Homo sapiens
<220>
   <221> misc_feature
   <222> 70, 138, 178, 197, 228, 242, 244, 287, 311
   <223> n = A,T,C or G
<400> 84
   tggtggatct tggctctgtg gagctgctgg gacgggatct aaaagactat tctggaagct 60
   gtggtccaan gcattttgct ggcttaacgg gtcccggaac aaaggacacc agctctctaa 120
   aattgaagtt tacccganat aacaatcttt tgggcagaga tgcctatttt aacaaacncc 180
   gtccctgcgc aacaacnaac aatctctggg aaataccggc catgaacntg ctgtctcaat 240
   cnancatctc tctagctgac cgatcatatc gtcccagatt actacanatc ataataattg 300
   atttcctgta naaaaaaaaa aaa 323
<210> 85
   <211> 771
   <212> DNA
   <213> Homo sapiens
<220>
   <221> misc_feature
   <222> 63, 426, 471, 497, 521, 554, 583, 586, 606, 609, 615, 652, 686, 691, 694, 695, 706, 713, 730, 732, 743, 751
   <223> n = A,T,C or G
<400> 85
   aaactgggta ctcaacactg agcagatctg ttctttgagc taaaaaccat gtgctgtacc 60
   aanagtttgc tcctggctgc tttgatgtca gtgctgctac tccacctctg cggcgaatca 120
   gaagcaagca actttgactg ctgtcttgga tacacagacc gtattcttca tcctaaattt 180
   attgtgggct tcacacggca gctggccaat gaaggctgtg acatcaatgc tatcatcttt 240
   cacacaaaga aaaagttgtc tgtgtgcgca aatccaaaac agacttgggt gaaatatatt 300
   gtgcgtctcc tcagtaaaaa agtcaagaac atgtaaaaac tgtggctttt ctggaatgga 360
   attggacata gcccaagaac agaaagaact tgctggggtt ggaggtttca cttgcacatc 420
   atgganggtt tagtgcttat cttatttgtg cctcctggac ttgtccaatt natgaagtta 480
   atcatattgc atcatanttt gctttgttta acatcacatt naaattaaac tgtattttat 540
   gttatttata gctntaggtt ttctgtgttt aactttttat acnaantttc ctaaactatt 600
   ttggtntant gcaanttaaa aattatattt ggggggggaa taaatattgg antttctgca 660
   gccacaagct ttttttaaaa aaccantaca nccnngttaa atggtnggtc ccnaatggtt 720
   tttgcttttn antagaaaat ttnttagaac natttgaaaa aaaaaaaaaa a 771
<210> 86
   <211> 628
   <212> DNA
   <213> Homo sapiens
<220>
   <221> misc_feature
   <222> 162, 249, 266, 348, 407, 427, 488, 518, 545, 566, 569, 597, 598, 611, 617, 621, 624
   <223> n = A,T,C or G
<400> 86
   actagtttgc tttacatttt tgaaaagtat tatttttgtc caagtgctta tcaactaaac 60
   cttgtgttag gtaagaatgg aatttattaa gtgaatcagt gtgacccttc ttgtcataag 120
   attatcttaa agctgaagcc aaaatatgct tcaaaagaaa angactttat tgttcattgt 180
   agttcataca ttcaaagcat ctgaactgta gtttctatag caagccaatt acatccataa 240
   gtggagaang aaatagatta atgtcnaagt atgattggtg gagggagcaa ggttgaagat 300
   aatctggggt tgaaattttc tagttttcat tctgtacatt tttagttnga catcagattt 360
   gaaatattaa tgtttacctt tcaatgtgtg gtatcagctg gactcantaa cacccctttc 420
   ttccctnggg gatggggaat ggattattgg aaaatggaaa gaaaaaagta cttaaagcct 480
   tcctttcnca gtttctggct cctaccctac tgatttancc agaataagaa aacattttat 540
   catcntctgc tttattccca ttaatnaant tttgatgaat aaatctgctt ttatgcnnac 600
   ccaaggaatt nagtggnttc ntcnttgt                                   628
<210> 87
   <211> 518
   <212> DNA
   <213> Homo sapiens
<220>
   <221> misc_feature
   <222> 384, 421, 486
   <223> n = A,T,C or G
<400> 87
   tttttatttt tttttagaga gtagttcagc ttttatttat aaatttattg cctgttttat 60
   tataacaaca ttatactgtt tatggtttaa tacatatggt tcaaaatgta taatacatca 120
   agtagtacag ttttaaaatt ttatgcttaa aacaagtttt gtgtaaaaaa tgcagataca 180
   ttttacatgg caaatcaatt tttaagtcat cctaaaaatt gatttttttt tgaaatttaa 240
   aaacacattt aatttcaatt tctctcttat ataaccttta ttactatagc atggtttcca 300
   ctacagttta acaatgcagc aaaattccca tttcacggta aattgggttt taagcggcaa 360
   ggttaaaatg ctttgaggat cctnaatacc ctttgaactt caaatgaagg ttatggttgt 420
   naatttaacc ctcatgccat aagcagaagc acaagtttag ctgcattttg ctctaaactg 480
   taaaancgag ccccccgttg aaaaagcaaa agggaccc                        518
<210> 88
   <211> 1844
   <212> DNA
   <213> Homo sapiens
<400> 88
   gagacagtga atcctagtat caaaggattt ttggcctcag aaaaagttgt tgattatttt 60
   tattttattt tatttttcga gactccgtct caaaaaaaaa aaaaaaaaaa agaatcacaa 120
   ggtatttgct aaagcatttt gagctgcttg gaaaaaggga agtagttgca gtagagtttc 180
   ttccatcttc ttggtgctgg gaagccatat atgtgtcttt tactcaagct aaggggtata 240
   agcttatgtg ttgaatttgc tacatctata tttcacatat tctcacaata agagaatttt 300
   gaaatagaaa tatcatagaa catttaagaa agtttagtat aaataatatt ttgtgtgttt 360
   taatcccttt gaagggatct atccaaagaa aatattttac actgagctcc ttcctacacg 420
   tctcagtaac agatcctgtg ttagtctttg aaaatagctc attttttaaa tgtcagtgag 480
   tagatgtagc atacatatga tgtataatga cgtgtattat gttaacaatg tctgcagatt 540
   ttgtaggaat acaaaacatg gcctttttta taagcaaaac gggccaatga ctagaataac 600
   acatagggca atctgtgaat atgtattata agcagcattc cagaaaagta gttggtgaaa 660
   taattttcaa gtcaaaaagg gatatggaaa gggaattatg agtaacctct attttttaag 720
   ccttgctttt aaattaaacg ctacagccat ttaagccttg aggataataa agcttgagag 780
   taataatgtt aggttagcaa aggtttagat gtatcacttc atgcatgcta ccatgatagt 840
   aatgcagctc ttcgagtcat ttctggtcat tcaagatatt cacccttttg cccatagaaa 900
   gcaccctacc tcacctgctt actgacattg tcttagctga tcacaagatc attatcagcc 960
   tccattattc cttactgtat ataaaataca gagttttata ttttcctttc ttcgtttttc 1020
   accatattca aaacctaaat ttgtttttgc agatggaatg caaagtaatc aagtgttcgt 1080
   gctttcacct agaagggtgt ggtcctgaag gaaagaggtc cctaaatatc ccccaccctg 1140
   ggtgctcctc cttccctggt accctgacta ccagaagtca ggtgctagag cagctggaga 1200
   agtgcagcag cctgtgcttc cacagatggg ggtgctgctg caacaaggct ttcaatgtgc 1260
   ccatcttagg gggagaagct agatcctgtg cagcagcctg gtaagtcctg aggaggttcc 1320
   attgctcttc ctgctgctgt cctttgcttc tcaacggggc tcgctctaca gtctagagca 1380
   catgcagcta acttgtgcct ctgcttatgc atgagggtta aattaacaac cataaccttc 1440
   atttgaagtt caaaggtgta ttcaggatcc tcaaagcatt ttaaccttgc cgcttaaaac 1500
   ccaatttacc gtgaaatggg aattttgctg cattgttaaa ctgtagtgga aaccatgcta 1560
   tagtaataaa ggttatataa gagagaaatt gaaattaaat gtgtttttaa atttcaaaaa 1620
   aaaatcaatc tttaggatga cttaaaaatt gatttgccat gtaaaatgta tctgcatttt 1680
   ttacacaaaa cttgttttaa gcataaaatt ttaaaactgt actacttgat gtattataca 1740
   ttttgaacca tatgtattaa accataaaca gtataatgtt gttataataa aacaggcaat 1800
   aaatttataa ataaaagctg aaaaaaaaaa aaaaaaaaaa aaaa                  1844
<210> 89
   <211> 523
   <212> DNA
   <213> Homo sapiens
<220>
   <221> misc_feature
   <222> 288, 352, 369, 398, 475, 511, 513
   <223> n = A,T,C or G
<400> 89
   tttttttttt tttttttagt caatccacat ttattgatca cttattatgt accaggcact 60
   gggataaaga tgactgttag tcactcacag taaggaagaa aactagcaaa taagacgatt 120
   acaatatgat gtagaaaatg ctaagccaga gatatagaaa ggtcctattg ggtccttctg 180
   tcaccttgtc tttccacatc cctacccttc acaggccttc cctccagctt cctgcccccg 240
   ctccccactg cagatcccct gggattttgc ctagagctaa acgagganat gggccccctg 300
   gccctggcat gacttgaacc caaccacaga ctgggaaagg gagcctttcg anagtggatc 360
   actttgatna gaaaacacat agggaattga agagaaantc cccaaatggc cacccgtgct 420
   ggtgctcaag aaaagtttgc agaatggata aatgaaggat caagggaatt aatanatgaa 480
   taattgaatg gtggctcaat aagaatgact ncnttgaatg acc                   523
<210> 90
   <211> 604
   <212> DNA
   <213> Homo sapiens
<220>
   <221> misc_feature
   <222> 563
   <223> n = A,T,C or G
<400> 90
   ccagtgtggt ggaatgcaaa gattaccccg gaagctttcg agaagctggg attccctgca 60
   gcaaaggaaa tagccaatat gtgtcgtttc tatgaaatga agccagaccg agatgtcaat 120
   ctcacccacc aactaaatcc caaagtcaaa agcttcagcc agtttatctc agagaaccag 180
   gggagccttc aagggcatgt agaaaatcag ctgttcagat aggcctctgc accacacagc 240
   ctctttcctc tctgatcctt ttcctcttta cggcacaaca ttcatgtttg acagaacatg 300
   ctggaatgca attgtttgca acaccgaagg atttcctgcg gtcgcctctt cagtaggaag 360
   cactgcattg gtgataggac acggtaattt gattcacatt taacttgcta gttagtgata 420
   aggggtggta cacctgtttg gtaaaatgag aagcctcgga aacttgggag cttctctcct 480
   accactaatg gggagggcag attattactg ggatttctcc tggggtgaat taatttcaag 540
   ccctaattgc tgaaattccc ctnggcaggc tccagttttc tcaactgcat tgcaaaattc 600
   cccc                                                               604
<210> 91
   <211> 858
   <212> DNA
   <213> Homo sapiens
<220>
   <221> misc_feature
   <222> 570, 591, 655, 664, 667, 683, 711, 759, 760, 765, 777, 787, 792, 794, 801, 804, 809, 817, 820
   <223> n = A,T,C or G
<400> 91
   tttttttttt ttttttttta tgattattat tttttttatt gatctttaca tcctcagtgt 60
   tggcagagtt tctgatgctt aataaacatt tgttctgatc agataagtgg aaaaaattgt 120
   catttcctta ttcaagccat gcttttctgt gatattctga tcctagttga acatacagaa 180
   ataaatgtct aaaacagcac ctcgattctc gtctataaca ggactaagtt cactgtgatc 240
   ttaaataagc ttggctaaaa tgggacatga gtggaggtag tcacacttca gcgaagaaag 300
   agaatctcct gtataatctc accaggagat tcaacgaatt ccaccacact ggactagtgg 360
   atcccccggg ctgcaggaat tcgatatcaa gcttatcgat accgtcgacc tcgagggggg 420
   gcccggtacc caattcgccc tatagtgagt cgtattacgc gcgctcactg gccgtcgttt 480
   tacaacgtcg tgactgggaa aaccctggcg ttacccaact taatcgcctt gcagcacatc 540
   cccctttcgc cagctggcgt aatagcgaan agcccgcacc gatcgccctt ncaacagttg 600
   cgcagcctga atggcgaatg ggacgcgccc tgtagcggcg cattaaagcg cggcngggtg 660
   tggnggntcc cccacgtgac cgntacactt ggcagcgcct tacgccggtc nttcgctttc 720
   ttcccttcct ttctcgcacc gttcgccggg tttccccgnn agctnttaat cgggggnctc 780
   cctttanggg tncnaattaa nggnttacng gaccttngan cccaaaaact ttgattaggg 840
   ggaaggtccc cgaagggg                                                858
<210> 92
   <211> 585
   <212> DNA
   <213> Homo sapiens
<220>
   <221> misc_feature
   <222> 317, 319, 320, 321, 325, 327, 328, 330, 331, 332, 460, 462, 483, 485, 487, 523, 538, 566, 584
   <223> n = A,T,C or G
<400> 92
   gttgaatctc ctggtgagat tatacaggag attctctttc ttcgctgaag tgtgactacc 60
   tccactcatg tcccatttta gccaagctta tttaagatca cagtgaactt agtcctgtta 120
   tagacgagaa tcgaggtgct gttttagaca tttatttctg tatgttcaac taggatcaga 180
   atatcacaga aaagcatggc ttgaataagg aaatgacaat tttttccact tatctgatca 240
   gaacaaatgt ttattaagca tcagaaactc tgccaacact gaggatgtaa agatcaataa 300
   aaaaaataat aatcatnann naaanannan nngaagggcg gccgccaccg cggtggagct 360
   ccagcttttg ttccctttag tgagggttaa ttgcgcgctt ggcgttaatc atggtcatag 420
   ctgtttcctg tgtgaaattg ttatccggct cacaattccn cncaacatac gagccgggaa 480
   gcntnangtg taaaagcctg gggtgcctaa attgagtgag ctnactcaca ttaattgngt 540
   tgcgctccac ttgcccgctt ttccantccg ggaaacctgt tcgnc                 585
<210> 93
   <211> 567
   <212> DNA
   <213> Homo sapiens
<220>
   <221> misc_feature
   <222> 82, 158, 230, 232, 253, 266, 267, 268, 269, 270, 271, 272, 273, 274, 275, 276, 277, 278, 279, 280, 281, 282, 283, 284, 285, 286, 287, 295, 303, 307, 314, 349, 352, 354, 356, 366, 369, 379, 382, 386, 393, 404, 427, 428, 446, 450, 452
   <223> n = A,T,C or G
<221> misc_feature
   <222> 453, 454, 459, 462, 480, 481, 483, 488, 493, 501, 509, 511, 512, 518, 520, 525, 526, 532, 541, 557
   <223> n = A,T,C or G
<400> 93
   cggcagtgtt gctgtctgcg tgtccacctt ggaatctggc tgaactggct gggaggacca 60
   agactgcggc tggggtgggc anggaaggga accgggggct gctgtgaagg atcttggaac 120
   ttccctgtac ccaccttccc cttgcttcat gtttgtanag gaaccttgtg ccggccaagc 180
   ccagtttcct tgtgtgatac actaatgtat ttgctttttt tgggaaatan anaaaaatca 240
   attaaattgc tantgtttct ttgaannnnn nnnnnnnnnn nnnnnnnggg ggggncgccc 300
   ccncggngga aacnccccct tttgttccct ttaattgaaa ggttaattng cncncntggc 360
   gttaanccnt gggccaaanc tngttncccg tgntgaaatt gttnatcccc tcccaaattc 420
   ccccccnncc ttccaaaccc ggaaancctn annntgttna ancccggggg gttgcctaan 480
   ngnaattnaa ccnaaccccc ntttaaatng nntttgcncn ccacnngccc cnctttccca 540
   nttcggggaa aaccctntcc gtgccca                                     567
<210> 94
   <211> 620
   <212> DNA
   <213> Homo sapiens
<220>
   <221> misc_feature
   <222> 169, 171, 222, 472, 528, 559, 599
   <223> n = A,T,C or G
<400> 94
   actagtcaaa aatgctaaaa taatttggga gaaaatattt tttaagtagt gttatagttt 60
   catgtttatc ttttattatg ttttgtgaag ttgtgtcttt tcactaatta cctatactat 120
   gccaatattt ccttatatct atccataaca tttatactac atttgtaana naatatgcac 180
   gtgaaactta acactttata aggtaaaaat gaggtttcca anatttaata atctgatcaa 240
   gttcttgtta tttccaaata gaatggactt ggtctgttaa gggctaagga gaagaggaag 300
   ataaggttaa aagttgttaa tgaccaaaca ttctaaaaga aatgcaaaaa aaaagtttat 360
   tttcaagcct tcgaactatt taaggaaagc aaaatcattt cctaaatgca tatcatttgt 420
   gagaatttct cattaatatc ctgaatcatt catttcacta aggctcatgt tnactccgat 480
   atgtctctaa gaaagtacta tttcatggtc caaacctggt tgccatantt gggtaaaggc 540
   tttcccttaa gtgtgaaant atttaaaatg aaattttcct ctttttaaaa attctttana 600
   agggttaagg gtgttgggga                                              620
<210> 95
   <211> 470
   <212> DNA
   <213> Homo sapiens
<220>
   <221> misc_feature
   <222> 61, 67, 79, 89, 106, 213, 271, 281, 330, 354, 387, 432, 448
   <223> n = A,T,C or G
<400> 95
   ctcgaccttc tctgcacagc ggatgaaccc tgagcagctg aagaccagaa aagccactat 60
   nactttntgc ttaattcang agcttacang attcttcaaa gagtgngtcc agcatccttt 120
   gaaacatgag ttcttaccag cagaagcaga cctttacccc accacctcag cttcaacagc 180
   agcaggtgaa acaacccatc cagcctccac ctnaggaaat atttgttccc acaaccaagg 240
   agccatgcca ctcaaaggtt ccacaacctg naaacacaaa nattccagag ccaggctgta 300
   ccaaggtccc tgagccaggg ctgtaccaan gtccctgagc caggttgtac caangtccct 360
   gagccaggat gtaccaaggt ccctgancca ggttgtccaa ggtccctgag ccaggctaca 420
   ccaagggcct gngccaggca gcatcaangt ccctgaccaa ggcttatcaa             470
<210> 96
   <211> 660
   <212> DNA
   <213> Homo sapiens
<220>
   <221> misc_feature
   <222> 299, 311, 360, 426, 538, 540, 542, 553, 563, 565, 592, 603, 604, 618, 633, 647, 649, 651, 653
   <223> n = A,T,C or G
<400> 96
   tttttttttt tttttttttt ggaattaaaa gcaatttaat gagggcagag caggaaacat 60
   gcatttcttt tcattcgaat cttcagatga accctgagca gccgaagacc agaaaagcca 120
   tgaagacttt ctgcttaatt caggggctta caggattctt cagagtgtgt gtgaacaaaa 180
   gctttatagt acgtattttt aggatacaaa taagagagag actatggctt ggggtgagaa 240
   tgtactgatt acaaggtcta cagacaatta agacacagaa acagatggga agagggtgnc 300
   cagcatctgg nggttggctt ctcaagggct tgtctgtgca ccaaattact tctgcttggn 360
   cttctgctga gctgggcctg gagtgaccgt tgaaggacat ggctctggta cctttgtgta 420
   gcctgncaca ggaactttgg tgtatccttg ctcaggaact ttgatggcac ctggctcagg 480
   aaacttgatg aagccttggt caagggacct tgatgcttgc tggctcaggg accttggngn 540
   ancctgggct canggacctt tgncncaacc ttggcttcaa gggacccttg gnacatcctg 600
   gcnnagggac ccttgggncc aaccctgggc ttnagggacc ctttggntnc nanccttggc 660
<210> 97
   <211> 441
   <212> DNA
   <213> Homo sapiens
<220>
   <221> misc_feature
   <222> 12, 308
   <223> n = A,T,C or G
<400> 97
   gggaccatac anagtattcc tctcttcaca ccaggaccag ccactgttgc agcatgagtt 60
   cccagcagca gaagcagccc tgcatcccac cccctcagct tcagcagcag caggtgaaac 120
   agccttgcca gcctccacct caggaaccat gcatccccaa aaccaaggag ccctgccacc 180
   ccaaggtgcc tgagccctgc caccccaaag tgcctgagcc ctgccagccc aaggttccag 240
   agccatgcca ccccaaggtg cctgagccct gcccttcaat agtcactcca gcaccagccc 300
   agcagaanac caagcagaag taatgtggtc cacagccatg cccttgagga gccggccacc 360
   agatgctgaa tcccctatcc cattctgtgt atgagtccca tttgccttgc aattagcatt 420
   ctgtctcccc caaaaaaaaa a 441
<210> 98
   <211> 600
   <212> DNA
   <213> Homo sapiens
<220>
   <221> misc_feature
   <222> 295, 349, 489, 496, 583
   <223> n = A,T,C or G
<400> 98
   gtattcctct cttcacacca ggaccagcca ctgttgcagc atgagttccc agcagcagaa 60
   gcagccctgc atcccacccc ctcagcttca gcagcagcag gtgaaacagc cttgccagcc 120
   tccacctcag gaaccatgca tccccaaaac caaggagccc tgccacccca aggtgcctga 180
   gccctgccac cccaaagtgc ctgagccctg ccagcccaag gttccagagc catgccaccc 240
   caaggtgcct gagccctgcc cttcaatagt cactccagca ccagcccagc agaanaccaa 300
   gcagaagtaa tgtggtccac agccatgccc ttgaggagcc ggccaccana tgctgaatcc 360
   cctatcccat tctgtgtatg agtcccattt gccttgcaat tagcattctg tctcccccaa 420
   aaaagaatgt gctatgaagc tttctttcct acacactctg agtctctgaa tgaagctgaa 480
   ggtcttaant acaganctag ttttcagctg ctcagaattc tctgaagaaa agatttaaga 540
   tgaaaggcaa atgattcagc tccttattac cccattaaat tcnctttcaa ttccaaaaaa 600
<210> 99
   <211> 667
   <212> DNA
   <213> Homo sapiens
<220>
   <221> misc_feature
   <222> 345, 562, 635
   <223> n = A,T,C or G
<400> 99
   actagtgact gagttcctgg caaagaaatt tgacctggac cagttgataa ctcatgtttt 60
   accatttaaa aaaatcagtg aaggatttga gctgctcaat tcaggacaaa gcattcgaac 120
   ggtcctgacg ttttgagatc caaagtggca ggaggtctgt gttgtcatgg tgaactggag 180
   tttctcttgt gagagttccc tcatctgaaa tcatgtatct gtctcacaaa tacaagcata 240
   agtagaagat ttgttgaaga catagaaccc ttataaagaa ttattaacct ttataaacat 300
   ttaaagtctt gtgagcacct gggaattagt ataataacaa tgttnatatt tttgatttac 360
   attttgtaag gctataattg tatcttttaa gaaaacatac cttggatttc tatgttgaaa 420
   tggagatttt taagagtttt aaccagctgc tgcagatata ttactcaaaa cagatatagc 480
   gtataaagat atagtaaatg catctcctag agtaatattc acttaacaca ttggaaacta 540
   ttatttttta gatttgaata tnaatgttat tttttaaaca cttgttatga gttacttggg 600
   attacatttt gaaatcagtt cattccatga tgcanattac tgggattaga ttaagaaaga 660
   cggaaaa                                                           667
<210> 100
   <211> 583
   <212> DNA
   <213> Homo sapiens
<220>
   <221> misc_feature
   <222> 404, 506, 514, 527, 528, 538, 548, 556, 568, 569
   <223> n = A,T,C or G
<400> 100
   gttttgtttg taagatgatc acagtcatgt tacactgatc taaaggacat atatataacc 60
   ctttaaaaaa aaaatcactg cctcattctt atttcaagat gaatttctat acagactaga 120
   tgtttttctg aagatcaatt agacattttg aaaatgattt aaagtgtttt ccttaatgtt 180
   ctctgaaaac aagtttcttt tgtagtttta accaaaaaag tgcccttttt gtcactggat 240
   tctcctagca ttcatgattt ttttttcata caatgaaatt aaaattgcta aaatcatgga 300
   ctggctttct ggttggattt caggtaagat gtgtttaagg ccagagcttt tctcagtatt 360
   tgattttttt ccccaatatt tgatttttta aaaatataca catnggtgct gcatttatat 420
   ctgctggttt aaaattctgt catatttcac ttctagcctt ttagttatgg caaatcatat 480
   tttactttta cttaaagcat ttggtnattt ggantatctg gttctannct aaaaaaanta 540
   attctatnaa ttgaantttt ggtactcnnc catatttgga tcc                   583
<210> 101
   <211> 592
   <212> DNA
   <213> Homo sapiens
<220>
   <221> misc_feature
   <222> 218, 497, 502, 533, 544, 546, 548, 550, 555
   <223> n = A,T,C or G
<400> 101
   gtggagacgt acaaagagca gccgctcaag acacctggga agaaaaagaa aggcaagccc 60
   gggaaacgca aggagcagga aaagaaaaaa cggcgaactc gctctgcctg gttagactct 120
   ggagtgactg ggagtgggct agaaggggac cacctgtctg acacctccac aacgtcgctg 180
   gagctcgatt cacggaggca ttgaaatttt cagcaganac cttccaagga catattgcag 240
   gattctgtaa tagtgaacat atggaaagta ttagaaatat ttattgtctg taaatactgt 300
   aaatgcattg gaataaaact gtctccccca ttgctctatg aaactgcaca ttggtcattg 360
   tgaatatttt tttttttgcc aaggctaatc caattattat tatcacattt accataattt 420
   attttgtcca ttgatgtatt tattttgtaa atgtatcttg gtgctgctga atttctatat 480
   tttttgtaca taatgcnttt anatatacct atcaagtttg ttgataaatg acncaatgaa 540
   gtgncncnan ttggnggttg aatttaatga atgcctaatt ttattatccc aa         592
<210> 102
   <211> 587
   <212> DNA
   <213> Homo sapiens
<220>
   <221> misc_feature
   <222> 91, 131, 256, 263, 332, 392, 400, 403, 461, 496, 497, 499, 510, 511, 518, 519, 539, 554, 560, 576
   <223> n = A,T,C or G
<400> 102
   cgtcctaagc acttagacta catcagggaa gaacacagac cacatccctg tcctcatgcg 60
   gcttatgttt tctggaagaa agtggagacc nagtccttgg ctttagggct ccccggctgg 120
   gggctgtgca ntccggtcag ggcgggaagg gaaatgcacc gctgcatgtg aacttacagc 180
   ccaggcggat gccccttccc ttagcactac ctggcctcct gcatcccctc gcctcatgtt 240
   cctcccacct tcaaanaatg aanaacccca tgggcccagc cccttgccct ggggaaccaa 300
   ggcagccttc caaaactcag gggctgaagc anactattag ggcaggggct gactttgggt 360
   gacactgccc attccctctc agggcagctc angtcacccn ggnctcttga acccagcctg 420
   ttcctttgaa aaagggcaaa actgaaaagg gcttttccta naaaaagaaa aaccagggaa 480
   ctttgccagg gcttcnntnt taccaaaacn ncttctcnng gatttttaat tccccattng 540
   gcctccactt accnggggcn atgccccaaa attaanaatt tcccatc 587
<210> 103
   <211> 496
   <212> DNA
   <213> Homo sapiens
<220>
   <221> misc_feature
   <222> 2, 17, 66, 74, 82, 119, 164, 166, 172, 200, 203, 228, 232, 271, 273, 415, 423, 445, 446, 473
   <223> n = A,T,C or G
<400> 103
   anaggactgg ccctacntgc tctctctcgt cctacctatc aatgcccaac atggcagaac 60
   ctgcanccct tggncactgc anatggaaac ctctcagtgt cttgacatca ccctacccnt 120
   gcggtgggtc tccaccacaa ccactttgac tctgtggtcc ctgnanggtg gnttctcctg 180
   actggcagga tggaccttan ccnacatatc cctctgttcc ctctgctnag anaaagaatt 240
   cccttaacat gatataatcc acccatgcaa ntngctactg gcccagctac catttaccat 300
   ttgcctacag aatttcattc agtctacact ttggcattct ctctggcgat agagtgtggc 360
   tgggctgacc gcaaaaggtg ccttacacac tggcccccac cctcaaccgt tgacncatca 420
   gangcttgcc tcctccttct gattnncccc catgttggat atcagggtgc tcnagggatt 480
   ggaaaagaaa caaaac 496
<210> 104
   <211> 575
   <212> DNA
   <213> Homo sapiens
<220>
   <221> misc_feature
   <222> 18, 19, 45, 68, 77, 132, 155, 174, 219, 226, 238, 259, 263, 271, 273, 306, 323, 339, 363, 368, 370, 378, 381, 382, 436, 440, 449, 450, 456, 481, 485, 496, 503, 510, 512, 515, 528, 542, 552
   <223> n = A,T,C or G
<400> 104
   gcacctgctc tcaatccnnc tctcaccatg atcctccgcc tgcanaaact cctctgccaa 60
   ctatggangt ggtttcnggg gtggctcttg ccaactggga agaagccgtg gtgtctctac 120
   ctgttcaact cngtttgtgt ctgggggatc aactnggggc tatggaagcg gctnaactgt 180
   tgttttggtg gaagggctgg taattggctt tgggaagtng cttatngaag ttggcctngg 240
   gaagttgcta ttgaaagtng ccntggaagt ngntttggtg gggggttttg ctggtggcct 300
   ttgttnaatt tgggtgcttt gtnaatggcg gccccctcnc ctgggcaatg aaaaaaatca 360
   ccnatgcngn aaacctcnac nnaacagcct gggcttccct cacctcgaaa aaagttgctc 420
   cccccccaaa aaaggncaan cccctcaann tggaangttg aaaaaatcct cgaatgggga 480
   ncccnaaaac aaaaancccc ccntttcccn gnaanggggg aaataccncc cccccactta 540
   cnaaaaccct tntaaaaaac cccccgggaa aaaaa 575
<210> 105
   <211> 619
   <212> DNA
   <213> Homo sapiens
<220>
   <221> misc_feature
   <222> 260, 527, 560, 564, 566, 585, 599
   <223> n = A,T,C or G
<400> 105
   cactagtagg atagaaacac tgtgtcccga gagtaaggag agaagctact attgattaga 60
   gcctaaccca ggttaactgc aagaagaggc gggatacttt cagctttcca tgtaactgta 120
   tgcataaagc caatgtagtc cagtttctaa gatcatgttc caagctaact gaatcccact 180
   tcaatacaca ctcatgaact cctgatggaa caataacagg cccaagcctg tggtatgatg 240
   tgcacacttg ctagactcan aaaaaatact actctcataa atgggtggga gtattttggt 300
   gacaacctac tttgcttggc tgagtgaagg aatgatattc atatattcat ttattccatg 360
   gacatttagt tagtgctttt tatataccag gcatgatgct gagtgacact cttgtgtata 420
   tttccaaatt tttgtacagt cgctgcacat atttgaaatc atatattaag acttccaaaa 480
   aatgaagtcc ctggtttttc atggcaactt gatcagtaaa ggattcncct ctgtttggta 540
   cttaaaacat ctactatatn gttnanatga aattcctttt ccccncctcc cgaaaaaana 600
   aagtggtggg gaaaaaaaa                                           619
<210> 106
   <211> 506
   <212> DNA
   <213> Homo sapiens
<220>
   <221> misc_feature
   <222> 8, 21, 31, 32, 58, 75, 89, 96, 99, 103, 122, 126, 147, 150, 158, 195, 210, 212, 219, 226, 246, 248, 249, 255, 258, 261, 263, 265, 275, 304, 317, 321, 331, 337, 340, 358, 371, 377, 380, 396, 450, 491
   <223> n = A,T,C or G
<400> 106
   cattggtnct ttcatttgct ntggaagtgt nnatctctaa cagtggacaa agttcccngt 60
   gccttaaact ctgtnacact tttgggaant gaaaanttng tantatgata ggttattctg 120
   angtanagat gttctggata ccattanatn tgcccccngt gtcagaggct catattgtgt 180
   tatgtaaatg gtatntcatt cgctactatn antcaattng aaatanggtc tttgggttat 240
   gaatantnng cagcncanct nanangctgt ctgtngtatt cattgtggtc atagcacctc 300
   acancattgt aacctcnatc nagtgagaca nactagnaan ttcctagtga tggctcanga 360
   ttccaaatgg nctcatntcn aatgtttaaa agttanttaa gtgtaagaaa tacagactgg 420
   atgttccacc aactagtacc tgtaatgacn ggcctgtccc aacacatctc ccttttccat 480
   gactgtggta ncccgcatcg gaaaaa                                    506
<210> 107
   <211> 452
   <212> DNA
   <213> Homo sapiens
<220>
   <221> misc_feature
   <222> 289, 317, 378
   <223> n = A,T,C or G
<400> 107
   gttgagtctg tactaaacag taagatatct caatgaacca taaattcaac tttgtaaaaa 60
   tcttttgaag catagataat attgtttggt aaatgtttct tttgtttggt aaatgtttct 120
   tttaaagacc ctcctattct ataaaactct gcatgtagag gcttgtttac ctttctctct 180
   ctaaggttta caataggagt ggtgatttga aaaatataaa attatgagat tggttttcct 240
   gtggcataaa ttgcatcact gtatcatttt cttttttaac cggtaagant ttcagtttgt 300
   tggaaagtaa ctgtganaac ccagtttccc gtccatctcc cttagggact acccatagaa 360
   catgaaaagg tccccacnga agcaagaaga taagtctttc atggctgctg gttgcttaaa 420
   ccactttaaa accaaaaaat tccccttgga aa                              452
<210> 108
   <211> 502
   <212> DNA
   <213> Homo sapiens
<220>
   <221> misc_feature
   <222> 22, 31, 126, 168, 183, 205, 219, 231, 236, 259, 283, 295, 296, 298, 301, 340, 354, 378, 383, 409, 433, 446, 455, 466, 488
   <223> n = A,T,C or G
<400> 108
   atcttcttcc cttaattagt tnttatttat ntattaaatt ttattgcatg tcctggcaaa 60
   caaaaagaga ttgtagattg gcttctggct ccccaaaagc ccataacaga aagtaccaca 120
   agaccncaac tgaagcttaa aaaatctatc acatgtataa tacctttnga agaacattaa 180
   tanagcatat aaaactttta acatntgctt aatgttgtnc aattataaaa ntaatngaaa 240
   aaaatgtccc tttaacatnc aatatcccac atagtgttat ttnaggggat taccnngnaa 300
   naaaaaaagg gtagaaggga tttaatgaaa actctgcttn ccatttctgt ttanaaacgt 360
   ctccagaaca aaaacttntc aantctttca gctaaccgca tttgagctna ggccactcaa 420
   aaactccatt agncccactt tctaanggtc tctanagctt actaancctt ttgacccctt 480
   accctggnta ctcctgccct ca                                         502
<210> 109
   <211> 1308
   <212> DNA
   <213> Homo sapiens
<400> 109
   acccgaggtc tcgctaaaat catcatggat tcacttggcg ccgtcagcac tcgacttggg 60
   tttgatcttt tcaaagagct gaagaaaaca aatgatggca acatcttctt ttcccctgtg 120
   ggcatcttga ctgcaattgg catggtcctc ctggggaccc gaggagccac cgcttcccag 180
   ttggaggagg tgtttcactc tgaaaaagag acgaagagct caagaataaa ggctgaagaa 240
   aaagaggtga ttgagaacac agaagcagta catcaacaat tccaaaagtt tttgactgaa 300
   ataagcaaac tcactaatga ttatgaactg aacataacca acaggctgtt tggagaaaaa 360
   acatacctct tccttcaaaa atacttagat tatgttgaaa aatattatca tgcatctctg 420
   gaacctgttg attttgtaaa tgcagccgat gaaagtcgaa agaagattaa ttcctgggtt 480
   gaaagcaaaa caaatgaaaa aatcaaggac ttgttcccag atggctctat tagtagctct 540
   accaagctgg tgctggtgaa catggtttat tttaaagggc aatgggacag ggagtttaag 600
   aaagaaaata ctaaggaaga gaaattttgg atgaataaga gcacaagtaa atctgtacag 660
   atgatgacac agagccattc ctttagcttc actttcctgg aggacttgca ggccaaaatt 720
   ctagggattc catataaaaa caacgaccta agcatgtttg tgcttctgcc caacgacatc 780
   gatggcctgg agaagataat agataaaata agtcctgaga aattggtaga gtggactagt 840
   ccagggcata tggaagaaag aaaggtgaat ctgcacttgc cccggtttga ggtggaggac 900
   agttacgatc tagaggcggt cctggctgcc atggggatgg gcgatgcctt cagtgagcac 960
   aaagccgact actcgggaat gtcgtcaggc tccgggttgt acgcccagaa gttcctgcac 1020
   agttcctttg tggcagtaac tgaggaaggc accgaggctg cagctgccac tggcataggc 1080
   tttactgtca catccgcccc aggtcatgaa aatgttcact gcaatcatcc cttcctgttc 1140
   ttcatcaggc acaatgaatc caacagcatc ctcttcttcg gcagattttc ttctccttaa 1200
   gatgatcgtt gccatggcat tgctgctttt agcaaaaaac aactaccagt gttactcata 1260
   tgattatgaa aatcgtccat tcttttaaat ggtggctcac ttgcattt             1308
<210> 110
   <211> 391
   <212> PRT
   <213> Homo sapiens
<400> 110
<210> 111
   <211> 1419
   <212> DNA
   <213> Homo sapiens
<400> 111
   ggagaactat aaattaagga tcccagctac ttaattgact tatgcttcct agttcgttgc 60
   ccagccacca ccgtctctcc aaaaacccga ggtctcgcta aaatcatcat ggattcactt 120
   ggcgccgtca gcactcgact tgggtttgat cttttcaaag agctgaagaa aacaaatgat 180
   ggcaacatct tcttttcccc tgtgggcatc ttgactgcaa ttggcatggt cctcctgggg 240
   acccgaggag ccaccgcttc ccagttggag gaggtgtttc actctgaaaa agagacgaag 300
   agctcaagaa taaaggctga agaaaaagag gtggtaagaa taaaggctga aggaaaagag 360
   attgagaaca cagaagcagt acatcaacaa ttccaaaagt tttgactgaa aataagcaaa 420
   ctcactaatg attatgaact gaacataacc aacaggctgt ttggagaaaa aacatacctc 480
   ttccttcaaa aatacttaga ttatgttgaa aaatattatc atgcatctct ggaacctgtt 540
   gattttgtaa atgcagccga tgaaagtcga aagaagatta attcctgggt tgaaagcaaa 600
   acaaatgaaa aaatcaagga cttgttccca gatggctcta ttagtagctc taccaagctg 660
   gtgctggtga acatggttta ttttaaaggg caatgggaca gggagtttaa gaaagaaaat 720
   actaaggaag agaaattttg gatgaataag agcacaagta aatctgtaca gatgatgaca 780
   cagagccatt cctttagctt cactttcctg gaggacttgc aggccaaaat tctagggatt 840
   ccatataaaa acaacgacct aagcatgttt gtgcttctgc ccaacgacat cgatggcctg 900
   gagaagataa tagataaaat aagtcctgag aaattggtag agtggactag tccagggcat 960
   atggaagaaa gaaaggtgaa tctgcacttg ccccggtttg aggtggagga cagttacgat 1020
   ctagaggcgg tcctggctgc catggggatg ggcgatgcct tcagtgagca caaagccgac 1080
   tactcgggaa tgtcgtcagg ctccgggttg tacgcccaga agttcctgca cagttccttt 1140
   gtggcagtaa ctgaggaagg caccgaggct gcagctgcca ctggcatagg ctttactgtc 1200
   acatccgccc caggtcatga aaatgttcac tgcaatcatc ccttcctgtt cttcatcagg 1260
   cacaatgaat ccaacagcat cctcttcttc ggcagatttt cttctcctta agatgatcgt 1320
   tgccatggca ttgctgcttt tagcaaaaaa caactaccag tgttactcat atgattatga 1380
   aaatcgtcca ttcttttaaa tggtggctca cttgcattt                        1419
<210> 112
   <211> 400
   <212> PRT
   <213> Homo sapiens
<400> 112
<210> 113
   <211> 957
   <212> DNA
   <213> Homo sapiens
<400> 113
   ctcgaccttc tctgcacagc ggatgaaccc tgagcagctg aagaccagaa aagccactat 60
   gactttctgc ttaattcagg agcttacagg attcttcaaa gagtgtgtcc agcatccttt 120
   gaaacatgag ttcttaccag cagaagcaga cctttacccc accacctcag cttcaacagc 180
   agcaggtgaa acaacccagc cagcctccac ctcaggaaat atttgttccc acaaccaagg 240
   agccatgcca ctcaaaggtt ccacaacctg gaaacacaaa gattccagag ccaggctgta 300
   ccaaggtccc tgagccaggc tgtaccaagg tccctgagcc aggttgtacc aaggtccctg 360
   agccaggatg taccaaggtc cctgagccag gttgtaccaa ggtccctgag ccaggctaca 420
   ccaaggtccc tgagccaggc agcatcaagg tccctgacca aggcttcatc aagtttcctg 480
   agccaggtgc catcaaagtt cctgagcaag gatacaccaa agttcctgtg ccaggctaca 540
   caaaggtacc agagccatgt ccttcaacgg tcactccagg cccagctcag cagaagacca 600
   agcagaagta atttggtgca cagacaagcc cttgagaagc caaccaccag atgctggaca 660
   ccctcttccc atctgtttct gtgtcttaat tgtctgtaga ccttgtaatc agtacattct 720
   caccccaagc catagtctct ctcttatttg tatcctaaaa atacggtact ataaagcttt 780
   tgttcacaca cactctgaag aatcctgtaa gcccctgaat taagcagaaa gtcttcatgg 840
   cttttctggt cttcggctgc tcagggttca tctgaagatt cgaatgaaaa gaaatgcatg 900
   tttcctgctc tgccctcatt aaattgcttt taattccaaa aaaaaaaaaa aaaaaaa     957
<210> 114
   <211> 161
   <212> PRT
   <213> Homo sapiens
<400> 114
   Met Ser Ser Tyr Gln Gln Lys Gln Thr Phe Thr Pro Pro Pro Gln Leu
   1               5                   10                  15
   Gln Gln Gln Gln Val Lys Gln Pro Ser Gln Pro Pro Pro Gln Glu Ile
               20                  25                  30
   Phe Val Pro Thr Thr Lys Glu Pro Cys His Ser Lys Val Pro Gln Pro
           35                  40                  45
   Gly Asn Thr Lys Ile Pro Glu Pro Gly Cys Thr Lys Val Pro Glu Pro
       50                  55                  60
   Gly Cys Thr Lys Val Pro Glu Pro Gly Cys Thr Lys Val Pro Glu Pro
   65                  70                  75                  80
   Gly Cys Thr Lys Val Pro Glu Pro Gly Cys Thr Lys Val Pro Glu Pro
                   85                  90                  95
   Gly Tyr Thr Lys Val Pro Glu Pro Gly Ser Ile Lys Val Pro Asp Gln
               100                 105                 110
   Gly Phe Ile Lys Phe Pro Glu Pro Gly Ala Ile Lys Val Pro Glu Gln
           115                 120                 125
   Gly Tyr Thr Lys Val Pro Val Pro Gly Tyr Thr Lys Val Pro Glu Pro
       130                 135                 140
   Cys Pro Ser Thr Val Thr Pro Gly Pro Ala Gln Gln Lys Thr Lys Gln
   145                 150                 155                 160
   Lys
<210> 115
   <211> 506
   <212> DNA
   <213> Homo sapiens
<220>
   <221> misc_feature
   <222> 8, 21, 31, 32, 58, 75, 89, 96, 99, 103, 122, 126, 147, 150, 158, 195, 210, 212, 219, 226, 246, 248, 249, 255, 258, 261, 263, 265, 275, 304, 317, 321, 331, 337, 340, 358, 371, 377, 380, 396, 450, 491
   <223> n = A,T,C or G
<400> 115
   cattggtnct ttcatttgct ntggaagtgt nnatctctaa cagtggacaa agttcccngt 60
   gccttaaact ctgtnacact tttgggaant gaaaanttng tantatgata ggttattctg 120
   angtanagat gttctggata ccattanatn tgcccccngt gtcagaggct catattgtgt 180
   tatgtaaatg gtatntcatt cgctactatn antcaattng aaatanggtc tttgggttat 240
   gaatantnng cagcncanct nanangctgt ctgtngtatt cattgtggtc atagcacctc 300
   acancattgt aacctcnatc nagtgagaca nactagnaan ttcctagtga tggctcanga 360
   ttccaaatgg nctcatntcn aatgtttaaa agttanttaa gtgtaagaaa tacagactgg 420
   atgttccacc aactagtacc tgtaatgacn ggcctgtccc aacacatctc ccttttccat 480
   gactgtggta ncccgcatcg gaaaaa                                      506
<210> 116
   <211> 3079
   <212> DNA
   <213> Homo sapiens
<400> 116
   ggatccccgg gtttcctaaa ccccccacag agtcctgccc aggccaaaga gcaaggaaaa 60
   ggtcaaaggg cagaaaaaat gctgagttag gaggagctat ggaaggataa acctggcctt 120
   aaagaggtca aagtggttta tagggggcgc tgagggcttc ccacattctc tggcctaaac 180
   cttgcaggca gatctgccca gtgggctctg ggatagctgt gccttcccta acaaaaaaat 240
   tgtgcacaaa aggatgaaac tctattttcc ctctagcaca taaccaagaa tataaggcta 300
   cagattgcct ttcccagagg gaaaaccctg cagcaacctg ctgcctggaa aagtgtaaga 360
   gcagatcact ggggaatcgt ttgccccccg ctgatggaca gcttccccaa gctccaaggg 420
   caggtgctca gcatgtaccg tactgggatg gttgtcaata ctcctggtcc tgtaagagtc 480
   ccaggacact gccatgccaa tgccccctca gttcctggca tcctttttgg gctgctcaca 540
   gccccagcct ctatggtgaa gacatacttg ctagcagcgt caccaacttg ttgccaagag 600
   atcagtgctc gaaggcaagg ttatttctaa ctgagcagag cctgccagga agaaagcgtt 660
   tgcaccccac accactgtgc aggtgtgacc ggtgagctca cagctgcccc ccaggcatgc 720
   ccagcccact taatcatcac agctcgacag ctctctcgcc cagcccagtt ctggaaggga 780
   taaaaagggg catcaccgtt cctgggtaac agagccacct tctgcgtcct gctgagctct 840
   gttctctcca gcacctccca acccactagt gcctggttct cttgctccac caggaacaag 900
   ccaccatgtc tcgccagtca agtgtgtctt ccggagcggg gggcagtcgt agcttcagca 960
   ccgcctctgc catcaccccg tctgtctccc gcaccagctt cacctccgtg tcccggtccg 1020
   ggggtggcgg tggtggtggc ttcggcaggg tcagccttgc gggtgcttgt ggagtgggtg 1080
   gctatggcag ccggagcctc tacaacctgg ggggctccaa gaggatatcc atcagcacta 1140
   gtggtggcag cttcaggaac cggtttggtg ctggtgctgg aggcggctat ggctttggag 1200
   gtggtgccgg tagtggattt ggtttcggcg gtggagctgg tggtggcttt gggctcggtg 1260
   gcggagctgg ctttggaggt ggcttcggtg gccctggctt tcctgtctgc cctcctggag 1320
   gtatccaaga ggtcactgtc aaccagagtc tcctgactcc cctcaacctg caaatcgacc 1380
   ccagcatcca gagggtgagg accgaggagc gcgagcagat caagaccctc aacaataagt 1440
   ttgcctcctt catcgacaag gtgcggttcc tggagcagca gaacaaggtt ctggaaacaa 1500
   agtggaccct gctgcaggag cagggcacca agactgtgag gcagaacctg gagccgttgt 1560
   tcgagcagta catcaacaac ctcaggaggc agctggacag catcgtgggg gaacggggcc 1620
   gcctggactc agagctgaga aacatgcagg acctggtgga agacttcaag aacaagtatg 1680
   aggatgaaat caacaagcgt accactgctg agaatgagtt tgtgatgctg aagaaggatg 1740
   tagatgctgc ctacatgaac aaggtggagc tggaggccaa ggttgatgca ctgatggatg 1800
   agattaactt catgaagatg ttctttgatg cggagctgtc ccagatgcag acgcatgtct 1860
   ctgacacctc agtggtcctc tccatggaca acaaccgcaa cctggacctg gatagcatca 1920
   tcgctgaggt caaggcccag tatgaggaga ttgccaaccg cagccggaca gaagccgagt 1980
   cctggtatca gaccaagtat gaggagctgc agcagacagc tggccggcat ggcgatgacc 2040
   tccgcaacac caagcatgag atctctgaga tgaaccggat gatccagagg ctgagagccg 2100
   agattgacaa tgtcaagaaa cagtgcgcca atctgcagaa cgccattgcg gatgccgagc 2160
   agcgtgggga gctggccctc aaggatgcca ggaacaagct ggccgagctg gaggaggccc 2220
   tgcagaaggc caagcaggac atggcccggc tgctgcgtga gtaccaggag ctcatgaaca 2280
   ccaagctggc cctggacgtg gagatcgcca cttaccgcaa gctgctggag ggcgaggaat 2340
   gcagactcag tggagaagga gttggaccag tcaacatctc tgttgtcaca agcagtgttt 2400
   cctctggata tggcagtggc agtggctatg gcggtggcct cggtggaggt cttggcggcg 2460
   gcctcggtgg aggtcttgcc ggaggtagca gtggaagcta ctactccagc agcagtgggg 2520
   gtgtcggcct aggtggtggg ctcagtgtgg ggggctctgg cttcagtgca agcagtagcc 2580
   gagggctggg ggtgggcttt ggcagtggcg ggggtagcag ctccagcgtc aaatttgtct 2640
   ccaccacctc ctcctcccgg aagagcttca agagctaaga acctgctgca agtcactgcc 2700
   ttccaagtgc agcaacccag cccatggaga ttgcctcttc taggcagttg ctcaagccat 2760
   gttttatcct tttctggaga gtagtctaga ccaagccaat tgcagaacca cattctttgg 2820
   ttcccaggag agccccattc ccagcccctg gtctcccgtg ccgcagttct atattctgct 2880
   tcaaatcagc cttcaggttt cccacagcat ggcccctgct gacacgagaa cccaaagttt 2940
   tcccaaatct aaatcatcaa aacagaatcc ccaccccaat cccaaatttt gttttggttc 3000
   taactacctc cagaatgtgt tcaataaaat gttttataat ataagctggt gtgcagaatt 3060
   gttttttttt tctacccaa                                                 3079
<210> 117
   <211> 6921
   <212> DNA
   <213> Homo sapiens
<400> 117
   gaattctgac tgtccactca aaacttctat tccgatcaaa gctatctgtg actacagaca 60
   aattgagata accatttaca aagacgatga atgtgttttg gcgaataact ctcatcgtgc 120
   taaatggaag gtcattagtc ctactgggaa tgaggctatg gtcccatctg tgtgcttcac 180
   cgttcctcca ccaaacaaag aagcggtgga ccttgccaac agaattgagc aacagtatca 240
   gaatgtcctg actctttggc atgagtctca cataaacatg aagagtgtag tatcctggca 300
   ttatctcatc aatgaaattg atagaattcg agctagcaat gtggcttcaa taaagacaat 360
   gctacctggt gaacatcagc aagttctaag taatctacaa tctcgttttg aagattttct 420
   ggaagatagc caggaatccc aagtcttttc aggctcagat ataacacaac tggaaaagga 480
   ggttaatgta tgtaagcagt attatcaaga acttcttaaa tctgcagaaa gagaggagca 540
   agaggaatca gtttataatc tctacatctc tgaagttcga aacattagac ttcggttaga 600
   gaactgtgaa gatcggctga ttagacagat tcgaactccc ctggaaagag atgatttgca 660
   tgaaagtgtg ttcagaatca cagaacagga gaaactaaag aaagagctgg aacgacttaa 720
   agatgatttg ggaacaatca caaataagtg tgaggagttt ttcagtcaag cagcagcctc 780
   ttcatcagtc cctaccctac gatcagagct taatgtggtc cttcagaaca tgaaccaagt 840
   ctattctatg tcttccactt acatagataa gttgaaaact gttaacttgg tgttaaaaaa 900
   cactcaagct gcagaagccc tcgtaaaact ctatgaaact aaactgtgtg aagaagaagc 960
   agttatagct gacaagaata atattgagaa tctaataagt actttaaagc aatggagatc 1020
   tgaagtagat gaaaagagac aggtattcca tgccttagag gatgagttgc agaaagctaa 1080
   agccatcagt gatgaaatgt ttaaaacgta taaagaacgg gaccttgatt ttgactggca 1140
   caaagaaaaa gcagatcaat tagttgaaag gtggcaaaat gttcatgtgc agattgacaa 1200
   caggttacgg gacttagagg gcattggcaa atcactgaag tactacagag acacttacca 1260
   tcctttagat gattggatcc agcaggttga aactactcag agaaagattc aggaaaatca 1320
   gcctgaaaat agtaaaaccc tagccacaca gttgaatcaa cagaagatgc tggtgtccga 1380
   aatagaaatg aaacagagca aaatggacga gtgtcaaaaa tatgcagaac agtactcagc 1440
   tacagtgaag gactatgaat tacaaacaat gacctaccgg gccatggtag attcacaaca 1500
   aaaatctcca gtgaaacgcc gaagaatgca gagttcagca gatctcatta ttcaagagtt 1560
   catggaccta aggactcgat atactgccct ggtcactctc atgacacaat atattaaatt 1620
   tgctggtgat tcattgaaga ggctggaaga ggaggagatt aaaaggtgta aggagacttc 1680
   tgaacatggg gcatattcag atctgcttca gcgtcagaag gcaacagtgc ttgagaatag 1740
   caaacttaca ggaaagataa gtgagttgga aagaatggta gctgaactaa agaaacaaaa 1800
   gtcccgagta gaggaagaac ttccgaaggt cagggaggct gcagaaaatg aattgagaaa 1860
   gcagcagaga aatgtagaag atatctctct gcagaagata agggctgaaa gtgaagccaa 1920
   gcagtaccgc agggaacttg aaaccattgt gagagagaag gaagccgctg aaagagaact 1980
   ggagcgggtg aggcagctca ccatagaggc cgaggctaaa agagctgccg tggaagagaa 2040
   cctcctgaat tttcgcaatc agttggagga aaacaccttt accagacgaa cactggaaga 2100
   tcatcttaaa agaaaagatt taagtctcaa tgatttggag caacaaaaaa ataaattaat 2160
   ggaagaatta agaagaaaga gagacaatga ggaagaactc ttgaagctga taaagcagat 2220
   ggaaaaagac cttgcatttc agaaacaggt agcagagaaa cagttgaaag aaaagcagaa 2280
   aattgaattg gaagcaagaa gaaaaataac tgaaattcag tatacatgta gagaaaatgc 2340
   attgccagtg tgtccgatca cacaggctac atcatgcagg gcagtaacgg gtctccagca 2400
   agaacatgac aagcagaaag cagaagaact caaacagcag gtagatgaac taacagctgc 2460
   caatagaaag gctgaacaag acatgagaga gctgacatat gaacttaatg ccctccagct 2520
   tgaaaaaacg tcatctgagg aaaaggctcg tttgctaaaa gataaactag atgaaacaaa 2580
   taatacactc agatgcctta agttggagct ggaaaggaag gatcaggcgg agaaagggta 2640
   ttctcaacaa ctcagagagc ttggtaggca attgaatcaa accacaggta aagctgaaga 2700
   agccatgcaa gaagctagtg atctcaagaa aataaagcgc aattatcagt tagaattaga 2760
   atctcttaat catgaaaaag ggaaactaca aagagaagta gacagaatca caagggcaca 2820
   tgctgtagct gagaagaata ttcagcattt aaattcacaa attcattctt ttcgagatga 2880
   gaaagaatta gaaagactac aaatctgcca gagaaaatca gatcatctaa aagaacaatt 2940
   tgagaaaagc catgagcagt tgcttcaaaa tatcaaagct gaaaaagaaa ataatgataa 3000
   aatccaaagg ctcaatgaag aattggagaa aagtaatgag tgtgcagaga tgctaaaaca 3060
   aaaagtagag gagcttacta ggcagaataa tgaaaccaaa ttaatgatgc agagaattca 3120
   ggcagaatca gagaatatag ttttagagaa acaaactatc cagcaaagat gtgaagcact 3180
   gaaaattcag gcagatggtt ttaaagatca gctacgcagc acaaatgaac acttgcataa 3240
   acagacaaaa acagagcagg attttcaaag aaaaattaaa tgcctagaag aagacctggc 3300
   gaaaagtcaa aatttggtaa gtgaatttaa gcaaaagtgt gaccaacaga acattatcat 3360
   ccagaatacc aagaaagaag ttagaaatct gaatgcggaa ctgaatgctt ccaaagaaga 3420
   gaagcgacgc ggggagcaga aagttcagct acaacaagct caggtgcaag agttaaataa 3480
   caggttgaaa aaagtacaag acgaattaca cttaaagacc atagaggagc agatgaccca 3540
   cagaaagatg gttctgtttc aggaagaatc tggtaaattc aaacaatcag cagaggagtt 3600
   tcggaagaag atggaaaaat taatggagtc caaagtcatc actgaaaatg atatttcagg 3660
   cattaggctt gactttgtgt ctcttcaaca agaaaactct agagcccaag aaaatgctaa 3720
   gctttgtgaa acaaacatta aagaacttga aagacagctt caacagtatc gtgaacaaat 3780
   gcagcaaggg cagcacatgg aagcaaatca ttaccaaaaa tgtcagaaac ttgaggatga 3840
   gctgatagcc cagaagcgtg aggttgaaaa cctgaagcaa aaaatggacc aacagatcaa 3900
   agagcatgaa catcaattag ttttgctcca gtgtgaaatt caaaaaaaga gcacagccaa 3960
   agactgtacc ttcaaaccag attttgagat gacagtgaag gagtgccagc actctggaga 4020
   gctgtcctct agaaacactg gacaccttca cccaacaccc agatcccctc tgttgagatg 4080
   gactcaagaa ccacagccat tggaagagaa gtggcagcat cgggttgttg aacagatacc 4140
   caaagaagtc caattccagc caccaggggc tccactcgag aaagagaaaa gccagcagtg 4200
   ttactctgag tacttttctc agacaagcac cgagttacag ataacttttg atgagacaaa 4260
   ccccattaca agactgtctg aaattgagaa gataagagac caagccctga acaattctag 4320
   accacctgtt aggtatcaag ataacgcatg tgaaatggaa ctggtgaagg ttttgacacc 4380
   cttagagata gctaagaaca agcagtatga tatgcataca gaagtcacaa cattaaaaca 4440
   agaaaagaac ccagttccca gtgctgaaga atggatgctt gaagggtgca gagcatctgg 4500
   tggactcaag aaaggggatt tccttaagaa gggcttagaa ccagagacct tccagaactt 4560
   tgatggtgat catgcatgtt cagtcaggga tgatgaattt aaattccaag ggcttaggca 4620
   cactgtgact gccaggcagt tggtggaagc taagcttctg gacatgagaa caattgagca 4680
   gctgcgactc ggtcttaaga ctgttgaaga agttcagaaa actcttaaca agtttctgac 4740
   gaaagccacc tcaattgcag ggctttacct agaatctaca aaagaaaaga tttcatttgc 4800
   ctcagcggcc gagagaatca taatagacaa aatggtggct ttggcatttt tagaagctca 4860
   ggctgcaaca ggttttataa ttgatcccat ttcaggtcag acatattctg ttgaagatgc 4920
   agttcttaaa ggagttgttg accccgaatt cagaattagg cttcttgagg cagagaaggc 4980
   agctgtggga tattcttatt cttctaagac attgtcagtg tttcaagcta tggaaaatag 5040
   aatgcttgac agacaaaaag gtaaacatat cttggaagcc cagattgcca gtgggggtgt 5100
   cattgaccct gtgagaggca ttcgtgttcc tccagaaatt gctctgcagc aggggttgtt 5160
   gaataatgcc atcttacagt ttttacatga gccatccagc aacacaagag ttttccctaa 5220
   tcccaataac aagcaagctc tgtattactc agaattactg cgaatgtgtg tatttgatgt 5280
   agagtcccaa tgctttctgt ttccatttgg ggagaggaac atttccaatc tcaatgtcaa 5340
   gaaaacacat agaatttctg tagtagatac taaaacagga tcagaattga ccgtgtatga 5400
   ggctttccag agaaacctga ttgagaaaag tatatatctt gaactttcag ggcagcaata 5460
   tcagtggaag gaagctatgt tttttgaatc ctatgggcat tcttctcata tgctgactga 5520
   tactaaaaca ggattacact tcaatattaa tgaggctata gagcagggaa caattgacaa 5580
   agccttggtc aaaaagtatc aggaaggcct catcacactt acagaacttg ctgattcttt 5640
   gctgagccgg ttagtcccca agaaagattt gcacagtcct gttgcagggt attggctgac 5700
   tgctagtggg gaaaggatct ctgtactaaa agcctcccgt agaaatttgg ttgatcggat 5760
   tactgccctc cgatgccttg aagcccaagt cagtacaggg ggcataattg atcctcttac 5820
   tggcaaaaag taccgggtgg ccgaagcttt gcatagaggc ctggttgatg aggggtttgc 5880
   ccagcagctg cgacagtgtg aattagtaat cacagggatt ggccatccca tcactaacaa 5940
   aatgatgtca gtggtggaag ctgtgaatgc aaatattata aataaggaaa tgggaatccg 6000
   atgtttggaa tttcagtact tgacaggagg gttgatagag ccacaggttc actctcggtt 6060
   atcaatagaa gaggctctcc aagtaggtat tatagatgtc ctcattgcca caaaactcaa 6120
   agatcaaaag tcatatgtca gaaatataat atgccctcag acaaaaagaa agttgacata 6180
   taaagaagcc ttagaaaaag ctgattttga tttccacaca ggacttaaac tgttagaagt 6240
   atctgagccc ctgatgacag gaatttctag cctctactat tcttcctaat gggacatgtt 6300
   taaataactg tgcaaggggt gatgcaggct ggttcatgcc actttttcag agtatgatga 6360
   tatcggctac atatgcagtc tgtgaattat gtaacatact ctatttcttg agggctgcaa 6420
   attgctaagt gctcaaaata gagtaagttt taaattgaaa attacataag atttaatgcc 6480
   cttcaaatgg tttcatttag ccttgagaat ggttttttga aacttggcca cactaaaatg 6540
   tttttttttt tttacgtaga atgtgggata aacttgatga actccaagtt cacagtgtca 6600
   tttcttcaga actccccttc attgaatagt gatcatttat taaatgataa attgcactcg 6660
   ctgaaagagc acgtcatgaa gcaccatgga atcaaagaga aagatataaa ttcgttccca 6720
   cagccttcaa gctgcagtgt tttagattgc ttcaaaaaat gaaaaagttt tgcctttttc 6780
   gatatagtga ccttctttgc atattaaaat gtttaccaca atgtcccatt tctagttaag 6840
   tcttcgcact tgaaagctaa cattatgaat attatgtgtt ggaggagggg aaggattttc 6900
   ttcattctgt gtattttccg g 6921
<210> 118
   <211> 946
   <212> DNA
   <213> Homo sapiens
<400> 118
   cttctgactg ggctcaggct gacaggtaga gctcaccatg gcttcttgtg tccttgtccc 60
   ctccccatca cagctgtggt gcagtccacc gtctccagtg gctatggcgg tgccagtggt 120
   gtcggcagtg gcttaggcct gggtggagga agcagctact cctatggcag tggtcttggc 180
   gttggaggtg gcttcagttc cagcagtggc agagccattg ggggtggcct cagctctgtt 240
   ggaggcggca gttccaccat caagtacacc accacctcct cctccagcag gaagagctat 300
   aagcactaaa gtgcgtctgc tagctctcgg tcccacagtc ctcaggcccc tctctggctg 360
   cagagccctc tcctcaggtt gcctgtcctc tcctggcctc cagtctcccc tgctgtccca 420
   ggtagagctg gggatgaatg cttagtgccc tcacttcttc tctctctctc tataccatct 480
   gagcacccat tgctcaccat cagatcaacc tctgatttta catcatgatg taatcaccac 540
   tggagcttca ctgttactaa attattaatt tcttgcctcc agtgttctat ctctgaggct 600
   gagcattata agaaaatgac ctctgctcct tttcattgca gaaaattgcc aggggcttat 660
   ttcagaacaa cttccactta ctttccactg gctctcaaac tctctaactt ataagtgttg 720
   tgaaccccca cccaggcagt atccatgaaa gcacaagtga ctagtcctat gatgtacaaa 780
   gcctgtatct ctgtgatgat ttctgtgctc ttcactgttt gcaattgcta aataaagcag 840
   atttataata catatattct tttactttgc cttgctttgg ggccaaagtt ttgggcttaa 900
   acttttttat ctgataagtg aatagttgtt tttaaaagat aatcta 946
<210> 119
   <211> 8948
   <212> DNA
   <213> Homo sapiens
<400> 119
   tcaacagccc ctgctccttg ggcccctcca tgccatgccg taatctctcc cacccgacca 60
   acaccaacac ccagctccga cgcagctcct ctgcgccctt gccgccctcc gagccacagc 120
   tttcctcccg ctcctgcccc cggcccgtcg ccgtctccgc gctcgcagcg gcctcgggag 180
   ggcccaggta gcgagcagcg acctcgcgag ccttccgcac tcccgcccgg ttccccggcc 240
   gtccgcctat ccttggcccc ctccgctttc tccgcgccgg cccgcctcgc ttatgcctcg 300
   gcgctgagcc gctctcccga ttgcccgccg acatgagctg caacggaggc tcccacccgc 360
   ggatcaacac tctgggccgc atgatccgcg ccgagtctgg cccggacctg cgctacgagg 420
   tgaccagcgg cggcgggggc accagcagga tgtactattc tcggcgcggc gtgatcaccg 480
   accagaactc ggacggctac tgtcaaaccg gcacgatgtc caggcaccag aaccagaaca 540
   ccatccagga gctgctgcag aactgctccg actgcttgat gcgagcagag ctcatcgtgc 600
   agcctgaatt gaagtatgga gatggaatac aactgactcg gagtcgagaa ttggatgagt 660
   gttttgccca ggccaatgac caaatggaaa tcctcgacag cttgatcaga gagatgcggc 720
   agatgggcca gccctgtgat gcttaccaga aaaggcttct tcagctccaa gagcaaatgc 780
   gagcccttta taaagccatc agtgtccctc gagtccgcag ggccagctcc aagggtggtg 840
   gaggctacac ttgtcagagt ggctctggct gggatgagtt caccaaacat gtcaccagtg 900
   aatgtttggg gtggatgagg cagcaaaggg cggagatgga catggtggcc tggggtgtgg 960
   acctggcctc agtggagcag cacattaaca gccaccgggg catccacaac tccatcggcg 1020
   actatcgctg gcagctggac aaaatcaaag ccgacctgcg cgagaaatct gcgatctacc 1080
   agttggagga ggagtatgaa aacctgctga aagcgtcctt tgagaggatg gatcacctgc 1140
   gacagctgca gaacatcatt caggccacgt ccagggagat catgtggatc aatgactgcg 1200
   aggaggagga gctgctgtac gactggagcg acaagaacac caacatcgct cagaaacagg 1260
   aggccttctc catacgcatg agtcaactgg aagttaaaga aaaagagctc aataagctga 1320
   aacaagaaag tgaccaactt gtcctcaatc agcatccagc ttcagacaaa attgaggcct 1380
   atatggacac tctgcagacg cagtggagtt ggattcttca gatcaccaag tgcattgatg 1440
   ttcatctgaa agaaaatgct gcctactttc agttttttga agaggcgcag tctactgaag 1500
   catacctgaa ggggctccag gactccatca ggaagaagta cccctgcgac aagaacatgc 1560
   ccctgcagca cctgctggaa cagatcaagg agctggagaa agaacgagag aaaatccttg 1620
   aatacaagcg tcaggtgcag aacttggtaa acaagtctaa gaagattgta cagctgaagc 1680
   ctcgtaaccc agactacaga agcaataaac ccattattct cagagctctc tgtgactaca 1740
   aacaagatca gaaaatcgtg cataaggggg atgagtgtat cctgaaggac aacaacgagc 1800
   gcagcaagtg gtacgtgacg ggcccgggag gcgttgacat gcttgttccc tctgtggggc 1860
   tgatcatccc tcctccgaac ccactggccg tggacctctc ttgcaagatt gagcagtact 1920
   acgaagccat cttggctctg tggaaccagc tctacatcaa catgaagagc ctggtgtcct 1980
   ggcactactg catgattgac atagagaaga tcagggccat gacaatcgcc aagctgaaaa 2040
   caatgcggca ggaagattac atgaagacga tagccgacct tgagttacat taccaagagt 2100
   tcatcagaaa tagccaaggc tcagagatgt ttggagatga tgacaagcgg aaaatacagt 2160
   ctcagttcac cgatgcccag aagcattacc agaccctggt cattcagctc cctggctatc 2220
   cccagcacca gacagtgacc acaactgaaa tcactcatca tggaacctgc caagatgtca 2280
   accataataa agtaattgaa accaacagag aaaatgacaa gcaagaaaca tggatgctga 2340
   tggagctgca gaagattcgc aggcagatag agcactgcga gggcaggatg actctcaaaa 2400
   acctccctct agcagaccag gggtcttctc accacatcac agtgaaaatt aacgagctta 2460
   agagtgtgca gaatgattca caagcaattg ctgaggttct caaccagctt aaagatatgc 2520
   ttgccaactt cagaggttct gaaaagtact gctatttaca gaatgaagta tttggactat 2580
   ttcagaaact ggaaaatatc aatggtgtta cagatggcta cttaaatagc ttatgcacag 2640
   taagggcact gctccaggct attctccaaa cagaagacat gttaaaggtt tatgaagcca 2700
   ggctcactga ggaggaaact gtctgcctgg acctggataa agtggaagct taccgctgtg 2760
   gactgaagaa aataaaaaat gacttgaact tgaagaagtc gttgttggcc actatgaaga 2820
   cagaactaca gaaagcccag cagatccact ctcagacttc acagcagtat ccactttatg 2880
   atctggactt gggcaagttc ggtgaaaaag tcacacagct gacagaccgc tggcaaagga 2940
   tagataaaca gatcgacttt agattatggg acctggagaa acaaatcaag caattgagga 3000
   attatcgtga taactatcag gctttctgca agtggctcta tgatcgtaaa cgccgccagg 3060
   attccttaga atccatgaaa tttggagatt ccaacacagt catgcggttt ttgaatgagc 3120
   agaagaactt gcacagtgaa atatctggca aacgagacaa atcagaggaa gtacaaaaaa 3180
   ttgctgaact ttgcgccaat tcaattaagg attatgagct ccagctggcc tcatacacct 3240
   caggactgga aactctgctg aacataccta tcaagaggac catgattcag tccccttctg 3300
   gggtgattct gcaagaggct gcagatgttc atgctcggta cattgaacta cttacaagat 3360
   ctggagacta ttacaggttc ttaagtgaga tgctgaagag tttggaagat ctgaagctga 3420
   aaaataccaa gatcgaagtt ttggaagagg agctcagact ggcccgagat gccaactcgg 3480
   aaaactgtaa taagaacaaa ttcctggatc agaacctgca gaaataccag gcagagtgtt 3540
   cccagttcaa agcgaagctt gcgagcctgg aggagctgaa gagacaggct gagctggatg 3600
   ggaagtcggc taagcaaaat ctagacaagt gctacggcca aataaaagaa ctcaatgaga 3660
   agatcacccg actgacttat gagattgaag atgaaaagag aagaagaaaa tctgtggaag 3720
   acagatttga ccaacagaag aatgactatg accaactgca gaaagcaagg caatgtgaaa 3780
   aggagaacct tggttggcag aaattagagt ctgagaaagc catcaaggag aaggagtacg 3840
   agattgaaag gttgagggtt ctactgcagg aagaaggcac ccggaagaga gaatatgaaa 3900
   atgagctggc aaaggtaaga aaccactata atgaggagat gagtaattta aggaacaagt 3960
   atgaaacaga gattaacatt acgaagacca ccatcaagga gatatccatg caaaaagagg 4020
   atgattccaa aaatcttaga aaccagcttg atagactttc aagggaaaat cgagatctga 4080
   aggatgaaat tgtcaggctc aatgacagca tcttgcaggc cactgagcag cgaaggcgag 4140
   ctgaagaaaa cgcccttcag caaaaggcct gtggctctga gataatgcag aagaagcagc 4200
   atctggagat agaactgaag caggtcatgc agcagcgctc tgaggacaat gcccggcaca 4260
   agcagtccct ggaggaggct gccaagacca ttcaggacaa aaataaggag atcgagagac 4320
   tcaaagctga gtttcaggag gaggccaagc gccgctggga atatgaaaat gaactgagta 4380
   aggtaagaaa caattatgat gaggagatca ttagcttaaa aaatcagttt gagaccgaga 4440
   tcaacatcac caagaccacc atccaccagc tcaccatgca gaaggaagag gataccagtg 4500
   gctaccgggc tcagatagac aatctcaccc gagaaaacag gagcttatct gaagaaataa 4560
   agaggctgaa gaacactcta acccagacca cagagaatct caggagggtg gaagaagaca 4620
   tccaacagca aaaggccact ggctctgagg tgtctcagag gaaacagcag ctggaggttg 4680
   agctgagaca agtcactcag atgcgaacag aggagagcgt aagatataag caatctcttg 4740
   atgatgctgc caaaaccatc caggataaaa acaaggagat agaaaggtta aaacaactga 4800
   tcgacaaaga aacaaatgac cggaaatgcc tggaagatga aaacgcgaga ttacaaaggg 4860
   tccagtatga cctgcagaaa gcaaacagta gtgcgacgga gacaataaac aaactgaagg 4920
   ttcaggagca agaactgaca cgcctgagga tcgactatga aagggtttcc caggagagga 4980
   ctgtgaagga ccaggatatc acgcggttcc agaactctct gaaagagctg cagctgcaga 5040
   agcagaaggt ggaagaggag ctgaatcggc tgaagaggac cgcgtcagaa gactcctgca 5100
   agaggaagaa gctggaggaa gagctggaag gcatgaggag gtcgctgaag gagcaagcca 5160
   tcaaaatcac caacctgacc cagcagctgg agcaggcatc cattgttaag aagaggagtg 5220
   aggatgacct ccggcagcag agggacgtgc tggatggcca cctgagggaa aagcagagga 5280
   cccaggaaga gctgaggagg ctctcttctg aggtcgaggc cctgaggcgg cagttactcc 5340
   aggaacagga aagtgtcaaa caagctcact tgaggaatga gcatttccag aaggcgatag 5400
   aagataaaag cagaagctta aatgaaagca aaatagaaat tgagaggctg cagtctctca 5460
   cagagaacct gaccaaggag cacttgatgt tagaagaaga actgcggaac ctgaggctgg 5520
   agtacgatga cctgaggaga ggacgaagcg aagcggacag tgataaaaat gcaaccatct 5580
   tggaactaag gagccagctg cagatcagca acaaccggac cctggaactg caggggctga 5640
   ttaatgattt acagagagag agggaaaatt tgagacagga aattgagaaa ttccaaaagc 5700
   aggctttaga ggcatctaat aggattcagg aatcaaagaa tcagtgtact caggtggtac 5760
   aggaaagaga gagccttctg gtgaaaatca aagtcctgga gcaagacaag gcaaggctgc 5820
   agaggctgga ggatgagctg aatcgtgcaa aatcaactct agaggcagaa accagggtga 5880
   aacagcgcct ggagtgtgag aaacagcaaa ttcagaatga cctgaatcag tggaagactc 5940
   aatattcccg caaggaggag gctattagga agatagaatc ggaaagagaa aagagtgaga 6000
   gagagaagaa cagtcttagg agtgagatcg aaagactcca agcagagatc aagagaattg 6060
   aagagaggtg caggcgtaag ctggaggatt ctaccaggga gacacagtca cagttagaaa 6120
   cagaacgctc ccgatatcag agggagattg ataaactcag acagcgccca tatgggtccc 6180
   atcgagagac ccagactgag tgtgagtgga ccgttgacac ctccaagctg gtgtttgatg 6240
   ggctgaggaa gaaggtgaca gcaatgcagc tctatgagtg tcagctgatc gacaaaacaa 6300
   ccttggacaa actattgaag gggaagaagt cagtggaaga agttgcttct gaaatccagc 6360
   cattccttcg gggtgcagga tctatcgctg gagcatctgc ttctcctaag gaaaaatact 6420
   ctttggtaga ggccaagaga aagaaattaa tcagcccaga atccacagtc atgcttctgg 6480
   aggcccaggc agctacaggt ggtataattg atccccatcg gaatgagaag ctgactgtcg 6540
   acagtgccat agctcgggac ctcattgact tcgatgaccg tcagcagata tatgcagcag 6600
   aaaaagctat cactggtttt gatgatccat tttcaggcaa gacagtatct gtttcagaag 6660
   ccatcaagaa aaatttgatt gatagagaaa ccggaatgcg cctgctggaa gcccagattg 6720
   cttcaggggg tgtagtagac cctgtgaaca gtgtcttttt gccaaaagat gtcgccttgg 6780
   cccgggggct gattgataga gatttgtatc gatccctgaa tgatccccga gatagtcaga 6840
   aaaactttgt ggatccagtc accaaaaaga aggtcagtta cgtgcagctg aaggaacggt 6900
   gcagaatcga accacatact ggtctgctct tgctttcagt acagaagaga agcatgtcct 6960
   tccaaggaat cagacaacct gtgaccgtca ctgagctagt agattctggt atattgagac 7020
   cgtccactgt caatgaactg gaatctggtc agatttctta tgacgaggtt ggtgagagaa 7080
   ttaaggactt cctccagggt tcaagctgca tagcaggcat atacaatgag accacaaaac 7140
   agaagcttgg catttatgag gccatgaaaa ttggcttagt ccgacctggt actgctctgg 7200
   agttgctgga agcccaagca gctactggct ttatagtgga tcctgttagc aacttgaggt 7260
   taccagtgga ggaagcctac aagagaggtc tggtgggcat tgagttcaaa gagaagctcc 7320
   tgtctgcaga acgagctgtc actgggtata atgatcctga aacaggaaac atcatctctt 7380
   tgttccaagc catgaataag gaactcatcg aaaagggcca cggtattcgc ttattagaag 7440
   cacagatcgc aaccgggggg atcattgacc caaaggagag ccatcgttta ccagttgaca 7500
   tagcatataa gaggggctat ttcaatgagg aactcagtga gattctctca gatccaagtg 7560
   atgataccaa aggatttttt gaccccaaca ctgaagaaaa tcttacctat ctgcaactaa 7620
   aagaaagatg cattaaggat gaggaaacag ggctctgtct tctgcctctg aaagaaaaga 7680
   agaaacaggt gcagacatca caaaagaata ccctcaggaa gcgtagagtg gtcatagttg 7740
   acccagaaac caataaagaa atgtctgttc aggaggccta caagaagggc ctaattgatt 7800
   atgaaacctt caaagaactg tgtgagcagg aatgtgaatg ggaagaaata accatcacgg 7860
   gatcagatgg ctccaccagg gtggtcctgg tagatagaaa gacaggcagt cagtatgata 7920
   ttcaagatgc tattgacaag ggccttgttg acaggaagtt ctttgatcag taccgatccg 7980
   gcagcctcag cctcactcaa tttgctgaca tgatctcctt gaaaaatggt gtcggcacca 8040
   gcagcagcat gggcagtggt gtcagcgatg atgtttttag cagctcccga catgaatcag 8100
   taagtaagat ttccaccata tccagcgtca ggaatttaac cataaggagc agctcttttt 8160
   cagacaccct ggaagaatcg agccccattg cagccatctt tgacacagaa aacctggaga 8220
   aaatctccat tacagaaggt atagagcggg gcatcgttga cagcatcacg ggtcagaggc 8280
   ttctggaggc tcaggcctgc acaggtggca tcatccaccc aaccacgggc cagaagctgt 8340
   cacttcagga cgcagtctcc cagggtgtga ttgaccaaga catggccacc agcgtgaagc 8400
   ctgctcagaa agccttcata ggcttcgagg gtgtgaaggg aaagaagaag atgtcagcag 8460
   cagaggcagt gaaagaaaaa tggctcccgt atgaggctgg ccagcgcttc ctggagttcc 8520
   agtacctcac gggaggtctt gttgacccgg aagtgcatgg gaggataagc accgaagaag 8580
   ccatccggaa ggggttcata gatggccgcg ccgcacagag gctgcaagac accagcagct 8640
   atgccaaaat cctgacctgc cccaaaacca aattaaaaat atcctataag gatgccataa 8700
   atcgctccat ggtagaagat atcactgggc tgcgccttct ggaagccgcc tccgtgtcgt 8760
   ccaagggctt acccagccct tacaacatgt cttcggctcc ggggtcccgc tccggctccc 8820
   gctcgggatc tcgctccgga tctcgctccg ggtcccgcag tgggtcccgg agaggaagct 8880
   ttgacgccac agggaattct tcctactctt attcctactc atttagcagt agttctattg 8940
   ggcactag                                                          8948
<210> 120
   <211> 587
   <212> DNA
   <213> Homo sapiens
<220>
   <221> misc_feature
   <222> 91, 131, 256, 263, 332, 392, 400, 403, 461, 496, 497, 499, 510, 511, 518, 519, 539, 554, 560, 576
   <223> n = A,T,C or G
<400> 120
   cgtcctaagc acttagacta catcagggaa gaacacagac cacatccctg tcctcatgcg 60
   gcttatgttt tctggaagaa agtggagacc nagtccttgg ctttagggct ccccggctgg 120
   gggctgtgca ntccggtcag ggcgggaagg gaaatgcacc gctgcatgtg aacttacagc 180
   ccaggcggat gccccttccc ttagcactac ctggcctcct gcatcccctc gcctcatgtt 240
   cctcccacct tcaaanaatg aanaacccca tgggcccagc cccttgccct ggggaaccaa 300
   ggcagccttc caaaactcag gggctgaagc anactattag ggcaggggct gactttgggt 360
   gacactgccc attccctctc agggcagctc angtcacccn ggnctcttga acccagcctg 420
   ttcctttgaa aaagggcaaa actgaaaagg gcttttccta naaaaagaaa aaccagggaa 480
   ctttgccagg gcttcnntnt taccaaaacn ncttctcnng gatttttaat tccccattng 540
   gcctccactt accnggggcn atgccccaaa attaanaatt tcccatc               587
<210> 121
   <211> 619
   <212> DNA
   <213> Homo sapiens
<220>
   <221> misc_feature
   <222> 260, 527, 560, 564, 566, 585, 599
   <223> n = A,T,C or G
<400> 121
   cactagtagg atagaaacac tgtgtcccga gagtaaggag agaagctact attgattaga 60
   gcctaaccca ggttaactgc aagaagaggc gggatacttt cagctttcca tgtaactgta 120
   tgcataaagc caatgtagtc cagtttctaa gatcatgttc caagctaact gaatcccact 180
   tcaatacaca ctcatgaact cctgatggaa caataacagg cccaagcctg tggtatgatg 240
   tgcacacttg ctagactcan aaaaaatact actctcataa atgggtggga gtattttggt 300
   gacaacctac tttgcttggc tgagtgaagg aatgatattc atatattcat ttattccatg 360
   gacatttagt tagtgctttt tatataccag gcatgatgct gagtgacact cttgtgtata 420
   tttccaaatt tttgtacagt cgctgcacat atttgaaatc atatattaag acttccaaaa 480
   aatgaagtcc ctggtttttc atggcaactt gatcagtaaa ggattcncct ctgtttggta 540
   cttaaaacat ctactatatn gttnanatga aattcctttt ccccncctcc cgaaaaaana 600
   aagtggtggg gaaaaaaaa                                              619
<210> 122
   <211> 1475
   <212> DNA
   <213> Homo sapiens
<400> 122
   tccacctgtc cccgcagcgc cggctcgcgc cctcctgccg cagccaccga gccgccgtct 60
   agcgccccga cctcgccacc atgagagccc tgctggcgcg cctgcttctc tgcgtcctgg 120
   tcgtgagcga ctccaaaggc agcaatgaac ttcatcaagt tccatcgaac tgtgactgtc 180
   taaatggagg aacatgtgtg tccaacaagt acttctccaa cattcactgg tgcaactgcc 240
   caaagaaatt cggagggcag cactgtgaaa tagataagtc aaaaacctgc tatgagggga 300
   atggtcactt ttaccgagga aaggccagca ctgacaccat gggccggccc tgcctgccct 360
   ggaactctgc cactgtcctt cagcaaacgt accatgccca cagatctgat gctcttcagc 420
   tgggcctggg gaaacataat tactgcagga acccagacaa ccggaggcga ccctggtgct 480
   atgtgcaggt gggcctaaag ccgcttgtcc aagagtgcat ggtgcatgac tgcgcagatg 540
   gaaaaaagcc ctcctctcct ccagaagaat taaaatttca gtgtggccaa aagactctga 600
   ggccccgctt taagattatt gggggagaat tcaccaccat cgagaaccag ccctggtttg 660
   cggccatcta caggaggcac cgggggggct ctgtcaccta cgtgtgtgga ggcagcctca 720
   tcagcccttg ctgggtgatc agcgccacac actgcttcat tgattaccca aagaaggagg 780
   actacatcgt ctacctgggt cgctcaaggc ttaactccaa cacgcaaggg gagatgaagt 840
   ttgaggtgga aaacctcatc ctacacaagg actacagcgc tgacacgctt gctcaccaca 900
   acgacattgc cttgctgaag atccgttcca aggagggcag gtgtgcgcag ccatcccgga 960
   ctatacagac catctgcctg ccctcgatgt ataacgatcc ccagtttggc acaagctgtg 1020
   agatcactgg ctttggaaaa gagaattcta ccgactatct ctatccggag cagctgaaga 1080
   tgactgttgt gaagctgatt tcccaccggg agtgtcagca gccccactac tacggctctg 1140
   aagtcaccac caaaatgctg tgtgctgctg acccacagtg gaaaacagat tcctgccagg 1200
   gagactcagg gggacccctc gtctgttccc tccaaggccg catgactttg actggaattg 1260
   tgagctgggg ccgtggatgt gccctgaagg acaagccagg cgtctacacg agagtctcac 1320
   acttcttacc ctggatccgc agtcacacca aggaagagaa tggcctggcc ctctgagggt 1380
   ccccagggag gaaacgggca ccacccgctt tcttgctggt tgtcattttt gcagtagagt 1440
   catctccatc agctgtaaga agagactggg aagat                            1475
<210> 123
   <211> 2294
   <212> DNA
   <213> Homo sapiens
<400> 123
   cagcgccggc tcgcgccctc ctgccgcagc caccgagccg ccgtctagcg ccccgacctc 60
   gccaccatga gagccctgct ggcgcgcctg cttctctgcg tcctggtcgt gagcgactcc 120
   aaaggcagca atgaacttca tcaagttcca tcgaactgtg actgtctaaa tggaggaaca 180
   tgtgtgtcca acaagtactt ctccaacatt cactggtgca actgcccaaa gaaattcgga 240
   gggcagcact gtgaaataga taagtcaaaa acctgctatg aggggaatgg tcacttttac 300
   cgaggaaagg ccagcactga caccatgggc cggccctgcc tgccctggaa ctctgccact 360
   gtccttcagc aaacgtacca tgcccacaga tctgatgctc ttcagctggg cctggggaaa 420
   cataattact gcaggaaccc agacaaccgg aggcgaccct ggtgctatgt gcaggtgggc 480
   ctaaagccgc ttgtccaaga gtgcatggtg catgactgcg cagatggaaa aaagccctcc 540
   tctcctccag aagaattaaa atttcagtgt ggccaaaaga ctctgaggcc ccgctttaag 600
   attattgggg gagaattcac caccatcgag aaccagccct ggtttgcggc catctacagg 660
   aggcaccggg ggggctctgt cacctacgtg tgtggaggca gcctcatcag cccttgctgg 720
   gtgatcagcg ccacacactg cttcattgat tacccaaaga aggaggacta catcgtctac 780
   ctgggtcgct caaggcttaa ctccaacacg caaggggaga tgaagtttga ggtggaaaac 840
   ctaatcctac acaaggacta cagcgctgac acgcttgctc accacaacga cattgccttg 900
   ctgaagatcc gttccaagga gggcaggtgt gcgcagccat cccggactat acagaccatc 960
   tgcctgccct cgatgtataa cgatccccag tttggcacaa gctgtgagat cactggcttt 1020
   ggaaaagaga attctaccga ctatctctat ccggagcagc tgaaaatgac tgttgtgaag 1080
   ctgatttccc accgggagtg tcagcagccc cactactacg gctctgaagt caccaccaaa 1140
   atgctgtgtg ctgctgaccc acagtggaaa acagattcct gccagggaga ctcaggggga 1200
   cccctcgtct gttccctcca aggccgcatg actttgactg gaattgtgag ctggggccgt 1260
   ggatgtgccc tgaaggacaa gccaggcgtc tacacgagag tctcacactt cttaccctgg 1320
   atccgcagtc acaccaagga agagaatggc ctggccctct gagggtcccc agggaggaaa 1380
   cgggcaccac ccgctttctt gctggttgct attttgcagt agagtcatct ccatcagctg 1440
   taagaagagc tgggaatata ggctctgcac agatggattt gcctgtgcca ccaccagggc 1500
   gaacgacaat agctttaccc tcaggcatag gcctgggtgc tggctgccca gacccctctg 1560
   gccaggatgg aggggtggtc ctgactcaac atgttactga ccagcaactt gtctttttct 1620
   ggactgaagc ctgcaggagt taaaaagggc agggcatctc ctgtgcatgg gctcgaaggg 1680
   agagccagct cccccgaccg gtgggcattt gtgaggccca tggttgagaa atgaataatt 1740
   tcccaattag gaagtgtaag cagctgaggt ctcttgaggg agcttagcca atgtgggagc 1800
   agcggtttgg ggagcagaga cactaacgac ttcagggcag ggctctgata ttccatgaat 1860
   gtatcaggaa atatatatgt gtgtgtatgt ttgcacactt gtgtgtgggc tgtgagtgta 1920
   agtgtgagta agagctggtg tctgattgtt aagtctaaat atttccttaa actgtgtgga 1980
   ctgtgatgcc acacagagtg gtctttctgg agaggttata ggtcactcct ggggcctctt 2040
   gggtccccca cgtgacagtg cctgggaatg tattattctg cagcatgacc tgtgaccagc 2100
   actgtctcag tttcactttc acatagatgt ccctttcttg gccagttatc ccttcctttt 2160
   agcctagttc atccaatcct cactgggtgg ggtgaggacc actcctgtac actgaatatt 2220
   tatatttcac tatttttatt tatatttttg taattttaaa taaaagtgat caataaaatg 2280
   tgatttttct gatg                                                   2294
<210> 124
   <211> 956
   <212> DNA
   <213> Homo sapiens
<400> 124
   gatgagttcc gcaccaagtt tgagacagac caggccctgc gcctgagtgt ggaggccgac 60
   atcaatggcc tgcgcagggt gctggatgag ctgaccctgg ccagagccga cctggagatg 120
   cagattgaga acctcaagga ggagctggcc tacctgaaga agaaccacga ggaggagatg 180
   aacgccctgc gaggccaggt gggtggtgag atcaatgtgg agatggacgc tgccccaggc 240
   gtggacctga gccgcatcct caacgagatg cgtgaccagt atgagaagat ggcagagaag 300
   aaccgcaagg atgccgagga ttggttcttc agcaagacag aggaactgaa ccgcgaggtg 360
   gccaccaaca gtgagctggt gcagagtggc aagagtgaga tctcggagct ccggcgcacc 420
   atgcaggcct tggagataga gctgcagtcc cagctcagca tgaaagcatc cctggagggc 480
   aacctggcgg agacagagaa ccgctactgc gtgcagctgt cccagatcca ggggctgatt 540
   ggcagcgtgg aggagcagct ggcccagctt cgctgcgaga tggagcagca gaaccaggaa 600
   tacaaaatcc tgctggatgt gaagacgcgg ctggagcagg agattgccac ctaccgccgc 660
   ctgctggagg gagaggatgc ccacctgact cagtacaaga aagaaccggt gaccacccgt 720
   caggtgcgta ccattgtgga agaggtccag gatggcaagg tcatctcctc ccgcgagcag 780
   gtccaccaga ccacccgctg aggactcagc taccccggcc ggccacccag gaggcaggga 840
   cgcagccgcc ccatctgccc cacagtctcc ggcctctcca gcctcagccc cctgcttcag 900
   tcccttcccc atgcttcctt gcctgatgac aataaaagct tgttgactca gctatg     956
<210> 125
   <211> 486
   <212> DNA
   <213> Homo sapiens
<220>
   <221> misc_feature
   <222> 16
   <223> n = A,T,C or G
<400> 125
   aaattatata tagtgnttca gctcccattg tggtgttcat agtcttctag gaacagataa 60
   acttaagtat tcaattcact cttggcattt tttctttaat ataggctttt tagcctattt 120
   ttggaaaact gcttttcttc tgagaacctt attctgaatg tcatcaactt taccaaacct 180
   tctaagtcca gagctaactt agtactgttt aagttactat tgactgaatt ttcttcattt 240
   tctgtttagc cagtgttacc aaggtaagct ggggaatgaa gtataccaac ttctttcaga 300
   gcattttagg acattatggc agctttagaa ggctgtcttg tttctagcca agggagagcc 360
   agcgcaggtt ttggatacta gagaaagtca tttgcttgta ctattgccat tttagaaagc 420
   tctgatgtga attcaaattt tacctctgtt acttaaagcc aacaatttta aggcagtagt 480
   tttact                                                            486
<210> 126
   <211> 3552
   <212> DNA
   <213> Homo sapiens
<400> 126
   cggcaggcag gtctcgtctc ggcaccctcc cggcgcccgc gttctcctgg ccctgcccgg 60
   catcccgatg gccgccgctg ggccccggcg ctccgtgcgc ggagccgtct gcctgcatct 120
   gctgctgacc ctcgtgatct tcagtcgtgc tggtgaagcc tgcaaaaagg tgatacttaa 180
   tgtaccttct aaactagagg cagacaaaat aattggcaga gttaatttgg aagagtgctt 240
   caggtctgca gacctcatcc ggtcaagtga tcctgatttc agagttctaa atgatgggtc 300
   agtgtacaca gccagggctg ttgcgctgtc tgataagaaa agatcattta ccatatggct 360
   ttctgacaaa aggaaacaga cacagaaaga ggttactgtg ctgctagaac atcagaagaa 420
   ggtatcgaag acaagacaca ctagagaaac tgttctcagg cgtgccaaga ggagatgggc 480
   acctattcct tgctctatgc aagagaattc cttgggccct ttcccattgt ttcttcaaca 540
   agttgaatct gatgcagcac agaactatac tgtcttctac tcaataagtg gacgtggagt 600
   tgataaagaa cctttaaatt tgttttatat agaaagagac actggaaatc tattttgcac 660
   tcggcctgtg gatcgtgaag aatatgatgt ttttgatttg attgcttatg cgtcaactgc 720
   agatggatat tcagcagatc tgcccctccc actacccatc agggtagagg atgaaaatga 780
   caaccaccct gttttcacag aagcaattta taattttgaa gttttggaaa gtagtagacc 840
   tggtactaca gtgggggtgg tttgtgccac agacagagat gaaccggaca caatgcatac 900
   gcgcctgaaa tacagcattt tgcagcagac accaaggtca cctgggctct tttctgtgca 960
   tcccagcaca ggcgtaatca ccacagtctc tcattatttg gacagagagg ttgtagacaa 1020
   gtactcattg ataatgaaag tacaagacat ggatggccag ttttttggat tgataggcac 1080
   atcaacttgt atcataacag taacagattc aaatgataat gcacccactt tcagacaaaa 1140
   tgcttatgaa gcatttgtag aggaaaatgc attcaatgtg gaaatcttac gaatacctat 1200
   agaagataag gatttaatta acactgccaa ttggagagtc aattttacca ttttaaaggg 1260
   aaatgaaaat ggacatttca aaatcagcac agacaaagaa actaatgaag gtgttctttc 1320
   tgttgtaaag ccactgaatt atgaagaaaa ccgtcaagtg aacctggaaa ttggagtaaa 1380
   caatgaagcg ccatttgcta gagatattcc cagagtgaca gccttgaaca gagccttggt 1440
   tacagttcat gtgagggatc tggatgaggg gcctgaatgc actcctgcag cccaatatgt 1500
   gcggattaaa gaaaacttag cagtggggtc aaagatcaac ggctataagg catatgaccc 1560
   cgaaaataga aatggcaatg gtttaaggta caaaaaattg catgatccta aaggttggat 1620
   caccattgat gaaatttcag ggtcaatcat aacttccaaa atcctggata gggaggttga 1680
   aactcccaaa aatgagttgt ataatattac agtcctggca atagacaaag atgatagatc 1740
   atgtactgga acacttgctg tgaacattga agatgtaaat gataatccac cagaaatact 1800
   tcaagaatat gtagtcattt gcaaaccaaa aatggggtat accgacattt tagctgttga 1860
   tcctgatgaa cctgtccatg gagctccatt ttatttcagt ttgcccaata cttctccaga 1920
   aatcagtaga ctgtggagcc tcaccaaagt taatgataca gctgcccgtc tttcatatca 1980
   gaaaaatgct ggatttcaag aatataccat tcctattact gtaaaagaca gggccggcca 2040
   agctgcaaca aaattattga gagttaatct gtgtgaatgt actcatccaa ctcagtgtcg 2100
   tgcgacttca aggagtacag gagtaatact tggaaaatgg gcaatccttg caatattact 2160
   gggtatagca ctgctctttt ctgtattgct aactttagta tgtggagttt ttggtgcaac 2220
   taaagggaaa cgttttcctg aagatttagc acagcaaaac ttaattatat caaacacaga 2280
   agcacctgga gacgatagag tgtgctctgc caatggattt atgacccaaa ctaccaacaa 2340
   ctctagccaa ggtttttgtg gtactatggg atcaggaatg aaaaatggag ggcaggaaac 2400
   cattgaaatg atgaaaggag gaaaccagac cttggaatcc tgccgggggg ctgggcatca 2460
   tcataccctg gactcctgca ggggaggaca cacggaggtg gacaactgca gatacactta 2520
   ctcggagtgg cacagtttta ctcaaccccg tctcggtgaa aaattgcatc gatgtaatca 2580
   gaatgaagac cgcatgccat cccaagatta tgtcctcact tataactatg agggaagagg 2640
   atctccagct ggttctgtgg gctgctgcag tgaaaagcag gaagaagatg gccttgactt 2700
   tttaaataat ttggaaccca aatttattac attagcagaa gcatgcacaa agagataatg 2760
   tcacagtgct acaattaggt ctttgtcaga cattctggag gtttccaaaa ataatattgt 2820
   aaagttcaat ttcaacatgt atgtatatga tgattttttt ctcaattttg aattatgcta 2880
   ctcaccaatt tatattttta aagcaagttg ttgcttatct tttccaaaaa gtgaaaaatg 2940
   ttaaaacaga caactggtaa atctcaaact ccagcactgg aattaaggtc tctaaagcat 3000
   ctgctctttt ttttttttac agatatttta gtaataaata tgctggataa atattagtcc 3060
   aacaatagct aagttatgct aatatcacat tattatgtat tcactttaag tgatagttta 3120
   aaaaataaac aagaaatatt gagtatcact atgtgaagaa agttttggaa aagaaacaat 3180
   gaagactgaa ttaaattaaa aatgttgcag ctcataaaga attggactca cccctactgc 3240
   actaccaaat tcatttgact ttggaggcaa aatgtgttga agtgccctat gaagtagcaa 3300
   ttttctatag gaatatagtt ggaaataaat gtgtgtgtgt atattattat taatcaatgc 3360
   aatatttaaa tgaaatgaga acaaagagga aaatggtaaa aacttgaaat gaggctgggg 3420
   tatagtttgt cctacaatag aaaaaagaga gagcttccta ggcctgggct cttaaatgct 3480
   gcattataac tgagtctatg aggaaatagt tcctgtccaa tttgtgtaat ttgtttaaaa 3540
   ttgtaaataa at                                                      3552
<210> 127
   <211> 754
   <212> DNA
   <213> Homo sapiens
<400> 127
   tttttttttt ttgtcattgt tcattgattt taatgagaaa gctaagagag gaaataagta 60
   gcctttcaaa ggtcacacag aagtaagtga cagatccagg attcatatcc aagcattctg 120
   gctctagtgt ccatgcttct caaccattat gacccaatat tcaaccaaat caatactgaa 180
   ggacacgtga aatgtatccg gtattttact attacaaaca aaaatccaat gaacattctt 240
   gaagacatac acaaaaataa tggttacaat agaagttact ggaattgaaa ttttggttca 300
   acctatatta aaatgtaagg cttttgatat agctaataga tttttgaaat gatcagtctt 360
   aacgtttgta ggggagcaca ctcctgcatg gggaaaagat tcactgtgaa gcacagagca 420
   cctttatggt tggatcatct tgtcattaaa gttcaggcgt tatctatcct gtaagtggca 480
   gaatcaagac tgcaatatcg cctgcttttc tttttaactc atgttttccc ttgactacac 540
   tggtcctcaa agtaaaaccc ctgtgtcagt gtactattca tggaatactc tgcaattata 600
   accaccttct aatactttta atacccaatc aaaatttatt atacatatgt atcatagata 660
   ctcatctgta aagctgtgct tcaaaatagt gatctcttcc caacattaca atatatatta 720
   atgatgtcga acctgcccgg gcggccgctc gaag                             754
<210> 128
   <211> 374
   <212> DNA
   <213> Homo sapiens
<400> 128
   aggttttgat taaaaaggca aatgatttta ttgttcgata atcttttaaa aaaataagag 60
   gaaggagtaa aattaaagat gaaagatgat ttttatttcc ttgtgacctc tatatccccc 120
   ttcccctgcc cttggtaagt aactcttgat ggagaaagga ttaaagactc ttatttaacc 180
   aaaaaacaga gccagctaat catttccaaa ggttagtatc tccctgctga cctcttcttt 240
   ggtttaattg aataaaacta tatgttcata tatgtattaa aacaactcag aataacatct 300
   tttcttcctt agttaaggca ttataagggc tatactatca tccataataa ccaaggcaat 360
   aacttaaaaa gctg                                                   374
<210> 129
   <211> 546
   <212> DNA
   <213> Homo sapiens
<400> 129
   agtgtgatgg atatctgcag aattcgggct aagcgtggtc gcggcccgag gtctggaact 60
   tcccagcacy tgaaaaggag cctcctgagc tgactcggct aaagccccac tttcgctcct 120
   cctcatttct gcctactgat ttccttggag cattcatctg aatattaccg tttgctgtgt 180
   aacctggtac atacatagca tgactccctg gaatagagtg ggctggggtg cttatgctgg 240
   gagagtgatt gacatgcact ttcaagctat atctaccatt tgcagcaaag gagaaaaaat 300
   acctcgagta aattccatca ttttttataa catcagcacc tgctccatca tcaaggagtc 360
   tcagcgtaac aggatctcca gtctctggct caactgtggc agtgacagtg gcattaagaa 420
   tgggataaaa tccctgtttc acattggcat aaatcatcac aggatgagga aaatggaggc 480
   tgtctctttc cacaaaggct tccacagtgg ctgggggcac agacctgccc gggcggccgc 540
   tcgaaa 546
<210> 130
   <211> 5156
   <212> DNA
   <213> Homo sapiens
<400> 130
   accaaccgag gcgccgggca gcgacccctg cagcggagac agagactgag cggcccggca 60
   ccgccatgcc tgcgctctgg ctgggctgct gcctctgctt gtcgctcctc ctgcccgcag 120
   cccgggccac ctccaggagg gaagtctgtg attgcaatgg gaagtccagg cagtgtatct 180
   ttgatcggga acttcacaga caaactggta atggattccg ctgcctcaac tgcaatgaca 240
   acactgatgg cattcactgc gagaagtgca agaatggctt ttaccggcac agagaaaggg 300
   accgctgttt gccctgcaat tgtaactcca aaggttctct tagtgctcga tgtgacaact 360
   ccggacggtg cagctgtaaa ccaggtgtga caggagccag atgcgaccga tgtctgccag 420
   gcttccacat gctcacggat gcggggtgca cccaagacca gagactgcta gactccaagt 480
   gtgactgtga cccagctggc atcgcagggc cctgtgacgc gggccgctgt gtctgcaagc 540
   cagctgtcac tggagaacgc tgtgataggt gtcgatcagg ttactataat ctggatgggg 600
   ggaaccctga gggctgtacc cagtgtttct gctatgggca ttcagccagc tgccgcagct 660
   ctgcagaata cagtgtccat aagatcacct ctacctttca tcaagatgtt gatggctgga 720
   aggctgtcca acgaaatggg tctcctgcaa agctccaatg gtcacagcgc catcaagatg 780
   tgtttagctc agcccaacga ctagaccctg tctattttgt ggctcctgcc aaatttcttg 840
   ggaatcaaca ggtgagctat ggtcaaagcc tgtcctttga ctaccgtgtg gacagaggag 900
   gcagacaccc atctgcccat gatgtgattc tggaaggtgc tggtctacgg atcacagctc 960
   ccttgatgcc acttggcaag acactgcctt gtgggctcac caagacttac acattcaggt 1020
   taaatgagca tccaagcaat aattggagcc cccagctgag ttactttgag tatcgaaggt 1080
   tactgcggaa tctcacagcc ctccgcatcc gagctacata tggagaatac agtactgggt 1140
   acattgacaa tgtgaccctg atttcagccc gccctgtctc tggagcccca gcaccctggg 1200
   ttgaacagtg tatatgtcct gttgggtaca aggggcaatt ctgccaggat tgtgcttctg 1260
   gctacaagag agattcagcg agactggggc cttttggcac ctgtattcct tgtaactgtc 1320
   aagggggagg ggcctgtgat ccagacacag gagattgtta ttcaggggat gagaatcctg 1380
   acattgagtg tgctgactgc ccaattggtt tctacaacga tccgcacgac ccccgcagct 1440
   gcaagccatg tccctgtcat aacgggttca gctgctcagt gatgccggag acggaggagg 1500
   tggtgtgcaa taactgccct cccggggtca ccggtgcccg ctgtgagctc tgtgctgatg 1560
   gctactttgg ggaccccttt ggtgaacatg gcccagtgag gccttgtcag ccctgtcaat 1620
   gcaacaacaa tgtggacccc agtgcctctg ggaattgtga ccggctgaca ggcaggtgtt 1680
   tgaagtgtat ccacaacaca gccggcatct actgcgacca gtgcaaagca ggctacttcg 1740
   gggacccatt ggctcccaac ccagcagaca agtgtcgagc ttgcaactgt aaccccatgg 1800
   gctcagagcc tgtaggatgt cgaagtgatg gcacctgtgt ttgcaagcca ggatttggtg 1860
   gccccaactg tgagcatgga gcattcagct gtccagcttg ctataatcaa gtgaagattc 1920
   agatggatca gtttatgcag cagcttcaga gaatggaggc cctgatttca aaggctcagg 1980
   gtggtgatgg agtagtacct gatacagagc tggaaggcag gatgcagcag gctgagcagg 2040
   cccttcagga cattctgaga gatgcccaga tttcagaagg tgctagcaga tcccttggtc 2100
   tccagttggc caaggtgagg agccaagaga acagctacca gagccgcctg gatgacctca 2160
   agatgactgt ggaaagagtt cgggctctgg gaagtcagta ccagaaccga gttcgggata 2220
   ctcacaggct catcactcag atgcagctga gcctggcaga aagtgaagct tccttgggaa 2280
   acactaacat tcctgcctca gaccactacg tggggccaaa tggctttaaa agtctggctc 2340
   aggaggccac aagattagca gaaagccacg ttgagtcagc cagtaacatg gagcaactga 2400
   caagggaaac tgaggactat tccaaacaag ccctctcact ggtgcgcaag gccctgcatg 2460
   aaggagtcgg aagcggaagc ggtagcccgg acggtgctgt ggtgcaaggg cttgtggaaa 2520
   aattggagaa aaccaagtcc ctggcccagc agttgacaag ggaggccact caagcggaaa 2580
   ttgaagcaga taggtcttat cagcacagtc tccgcctcct ggattcagtg tctcggcttc 2640
   agggagtcag tgatcagtcc tttcaggtgg aagaagcaaa gaggatcaaa caaaaagcgg 2700
   attcactctc aagcctggta accaggcata tggatgagtt caagcgtaca cagaagaatc 2760
   tgggaaactg gaaagaagaa gcacagcagc tcttacagaa tggaaaaagt gggagagaga 2820
   aatcagatca gctgctttcc cgtgccaatc ttgctaaaag cagagcacaa gaagcactga 2880
   gtatgggcaa tgccactttt tatgaagttg agagcatcct taaaaacctc agagagtttg 2940
   acctgcaggt ggacaacaga aaagcagaag ctgaagaagc catgaagaga ctctcctaca 3000
   tcagccagaa ggtttcagat gccagtgaca agacccagca agcagaaaga gccctgggga 3060
   gcgctgctgc tgatgcacag agggcaaaga atggggccgg ggaggccctg gaaatctcca 3120
   gtgagattga acaggagatt gggagtctga acttggaagc caatgtgaca gcagatggag 3180
   ccttggccat ggaaaaggga ctggcctctc tgaagagtga gatgagggaa gtggaaggag 3240
   agctggaaag gaaggagctg gagtttgaca cgaatatgga tgcagtacag atggtgatta 3300
   cagaagccca gaaggttgat accagagcca agaacgctgg ggttacaatc caagacacac 3360
   tcaacacatt agacggcctc ctgcatctga tggaccagcc tctcagtgta gatgaagagg 3420
   ggctggtctt actggagcag aagctttccc gagccaagac ccagatcaac agccaactgc 3480
   ggcccatgat gtcagagctg gaagagaggg cacgtcagca gaggggccac ctccatttgc 3540
   tggagacaag catagatggg attctggctg atgtgaagaa cttggagaac attagggaca 3600
   acctgccccc aggctgctac aatacccagg ctcttgagca acagtgaagc tgccataaat 3660
   atttctcaac tgaggttctt gggatacaga tctcagggct cgggagccat gtcatgtgag 3720
   tgggtgggat ggggacattt gaacatgttt aatgggtatg ctcaggtcaa ctgacctgac 3780
   cccattcctg atcccatggc caggtggttg tcttattgca ccatactcct tgcttcctga 3840
   tgctgggcaa tgaggcagat agcactgggt gtgagaatga tcaaggatct ggaccccaaa 3900
   gaatagactg gatggaaaga caaactgcac aggcagatgt ttgcctcata atagtcgtaa 3960
   gtggagtcct ggaatttgga caagtgctgt tgggatatag tcaacttatt ctttgagtaa 4020
   tgtgactaaa ggaaaaaact ttgactttgc ccaggcatga aattcttcct aatgtcagaa 4080
   cagagtgcaa cccagtcaca ctgtggccag taaaatacta ttgcctcata ttgtcctctg 4140
   caagcttctt gctgatcaga gttcctccta cttacaaccc agggtgtgaa catgttctcc 4200
   attttcaagc tggaagaagt gagcagtgtt ggagtgagga cctgtaaggc aggcccattc 4260
   agagctatgg tgcttgctgg tgcctgccac cttcaagttc tggacctggg catgacatcc 4320
   tttcttttaa tgatgccatg gcaacttaga gattgcattt ttattaaagc atttcctacc 4380
   agcaaagcaa atgttgggaa agtatttact ttttcggttt caaagtgata gaaaagtgtg 4440
   gcttgggcat tgaaagaggt aaaattctct agatttatta gtcctaattc aatcctactt 4500
   ttagaacacc aaaaatgatg cgcatcaatg tattttatct tattttctca atctcctctc 4560
   tctttcctcc acccataata agagaatgtt cctactcaca cttcagctgg gtcacatcca 4620
   tccctccatt catccttcca tccatctttc catccattac ctccatccat ccttccaaca 4680
   tatatttatt gagtacctac tgtgtgccag gggctggtgg gacagtggtg acatagtctc 4740
   tgccctcata gagttgattg tctagtgagg aagacaagca tttttaaaaa ataaatttaa 4800
   acttacaaac tttgtttgtc acaagtggtg tttattgcaa taaccgcttg gtttgcaacc 4860
   tctttgctca acagaacata tgttgcaaga ccctcccatg gggcgacttg agttttggca 4920
   aggctgacag agctctgggt tgtgcacatt tctttgcatt ccagctgtca ctctgtgcct 4980
   ttctacaact gattgcaaca gactgttgag ttatgataac accagtggga attgctggag 5040
   gaaccagagg cacttccacc ttggctggga agactatggt gctgccttgc ttctgtattt 5100
   ccttggattt tcctgaaagt gtttttaaat aaagaacaat tgttagaaaa aaaaaa     5156
<210> 131
   <211> 671
   <212> DNA
   <213> Homo sapiens
<400> 131
   aggtctggag ggcccacagc cggatgtggg acaccgggaa aaagtggtca tagcacacat 60
   ttttgcatcc cggttgcagt gtgttgcaga cgaagtcctc ttgctcgtca ccccacactt 120
   cctgggcagc caycacgagg atcatgactc ggaaaataaa gatgactgtg atccacacct 180
   tcccgatgct ggtggagtgt ttgttgacac ccccgatgaa agtgtgcagc gtcccccaat 240
   ccattgcgct ggtttatccc tgagtcctgt ttccaacgac tgccagtgtt tcagacccaa 300
   agaatgaggg caagatccct ctgcgagggt ttcagacctc cttctcctac cccactggag 360
   tgcctagaag ccaatgggtg cacagtgatg atacgaatgt caatctttgc tcggtcagtg 420
   aggatgtcgc ctggaatatt caaattgaat tacagatgca tgaagagggc gtacaagtta 480
   gaatttttct ttcgccatac agaaattgtt tagccagatc ttctgtactt cttttccttc 540
   cctgaccctt cctgctcccc aggaagggag gtcagccccg tttgcaaaac acaggatgcc 600
   cgtgacaccg gagacaggtc ttcttcaccg acaggaagtg ccttctggtg cctgcacgtt 660
   ttaactgcta t                                                      671
<210> 132
   <211> 590
   <212> DNA
   <213> Homo sapiens
<400> 132
   ctgaatggaa aagcttatgg ctctgtgatg atattagtga ccagcggaga tgataagctt 60
   cttggcaatt gcttacccac tgtgctcagc agtggttcaa caattcactc cattgccctg 120
   ggttcatctg cagccccaaa tctggaggaa ttatcacgtc ttacaggagg tttaaagttc 180
   tttgttccag atatatcaaa ctccaatagc atgattgatg ctttcagtag aatttcctct 240
   ggaactggag acattttcca gcaacatatt cagcttgaaa gtacaggtga aaatgtcaaa 300
   cctcaccatc aattgaaaaa cacagtgact gtggataata ctgtgggcaa cgacactatg 360
   tttctagtta cgtggcaggc cagtggtcct cctgagatta tattatttga tcctgatgga 420
   cgaaaatact acacaaataa ttttatcacc aatctaactt ttcggacagc tagtctttgg 480
   attccaggaa cagctaagcc tgggcactgg acttacaccc tgaacaatac ccatcattct 540
   ctgcaagccc tgaaagtgac agtgacctct cgcgcctcca actcagacct            590
<210> 133
   <211> 581
   <212> DNA
   <213> Homo sapiens
<400> 133
   aggtcctgtc cgggggcact gagaactccc tctggaattc ttggggggtg ttggggagag 60
   actgtgggcc tggagataaa acttgtctcc tctaccacca ccctgtaccc tagcctgcac 120
   ctgtcctcat ctctgcaaag ttcagcttcc ttccccaggt ctctgtgcac tctgtcttgg 180
   atgctctggg gagctcatgg gtggaggagt ctccaccaga gggaggctca ggggactggt 240
   tgggccaggg atgaatattt gagggataaa aattgtgtaa gagccaaaga attggtagta 300
   gggggagaac agagaggagc tgggctatgg gaaatgattt gaataatgga gctgggaata 360
   tggctggata tctggtacta aaaaagggtc tttaagaacc tacttcctaa tctcttcccc 420
   aatccaaacc atagctgtct gtccagtgct ctcttcctgc ctccagctct gccccaggct 480
   cctcctagac tctgtccctg ggctagggca ggggaggagg gagagcaggg ttgggggaga 540
   ggctgaggag agtgtgacat gtggggagag gaccagacct c                     581
<210> 134
   <211> 4797
   <212> DNA
   <213> Homo sapiens
<220>
   <221> misc_feature
   <222> 135, 501, 4421, 4467, 4468, 4698
   <223> n = A,T,C or G
<400> 134
   cctgggacca aagtgctgcc cagagctgag ggtcctggag ccacatgaga aggcttctcc 60
   ctgtgtacct gtgcagcaca gggtagggtg agtccactca gctgtctagg agaggaccca 120
   ggagcagcag agacncgcca agcctttact cataccatat tctgatcctt ttccagcaaa 180
   ttgtggctac taatttgccc cctgaagatc aagatggctc tggggatgac tctgacaact 240
   tctccggctc aggtgcaggt gaggttgtca tgggggcccc ccccacccaa gacggcaaca 300
   ggtcatgcct gggggcagtg gtcaggcagt ctcctgtgtt tactgagcat gtactgagtg 360
   caccctgcct gccctgtctc cacccagctg gctccaaagg gcaatgctga ggagaggaat 420
   ggggtcgtga gctgctgtta aggagagctc atgcttggag gtgaggtgaa ggctgtgagc 480
   tccagaaggc cccagggcgc nctgctgcac gcaggctcat attcactagg aatagcttta 540
   ctcactaaga aacctctgga acccccttca gaaggttatt tgactcctga gcctctattt 600
   tctcatctgc aaaatgggaa taataccttg acctgataag cttgtggagc tgtaaggcag 660
   cacagagcca gctggggtgt agctcttcca tccaagctcc cttccttact tcccctttcc 720
   tgtggggact gggggagaga agtccctgag ctggaggtgg tcagggaagc ttcacagagg 780
   aggtggctct tgagtggacc tcaggaagag gggtgagaga gctaaggaag gaggctgagg 840
   tcatccctgg ggaagtgacc tagcggaggc ctgagagctg caaggtagga tatctgttgt 900
   tggaagtgtc tgttgttgga agtgggggcc tttttttcag ggagggtggg gccagagaag 960
   tgtgtgccct gggataagta ggataaccac agtagttatg cccctaaggg atgcccaccc 1020
   cacccctgtg gtcacagaaa agctttccca ggtggcctag gcacctgtct cgtggctcca 1080
   gagacaggct gcacctgaca cacacaatgg aaggacagct ctccttgtcc attttccaag 1140
   gagcttagcc tcagctgcct tgtccaggta ctagcctccc tcatagcctg agcttggcca 1200
   gcccaggtgc tctggagcct cccccgaccc acccaacaca ctctgcttct ggtcctcccc 1260
   accccccacc tccccaacac actctgcttc tggtcctgca ggtgctttgc aagatatcac 1320
   cttgtcacag cagaccccct ccacttggaa ggacacgcag ctcctgacgg ctattcccac 1380
   gtctccagaa cccaccggcc tggaggctac agctgcctcc acctccaccc tgccggctgg 1440
   agaggggccc aaggagggag aggctgtagt cctgccagaa gtggagcctg gcctcaccgc 1500
   ccgggagcag gaggccaccc cccgacccag ggagaccaca cagctcccga ccactcatca 1560
   ggcctcaacg accacagcca ccacggccca ggagcccgcc acctcccacc cccacaggga 1620
   catgcagcct ggccaccatg agacctcaac ccctgcagga cccagccaag ctgaccttca 1680
   cactccccac acagaggatg gaggtccttc tgccaccgag agggctgctg aggatggagc 1740
   ctccagtcag ctcccagcag cagagggctc tggggagcag gtgagtggcc tctgcattcc 1800
   ttgggaaatt gagtgggttg gtcctaatgc ctggcacttg gcaggcccta cacctgtgcc 1860
   ctgcgcgatc tcgtattcct caccaggaag acagggcaca ggggccgcct tcccctaccc 1920
   ccagggcctc gcagagcagg acagactaac tatgagatca gagcagaagc acccttaaag 1980
   atcacccaag agagggctcc caaactcaca atccaaactt gcagccctcg tcgaagagtg 2040
   aacgttatac cagtcatttt atttatagct tcgtggattt acgcttacac taaatagtct 2100
   gctattcata caaaatgtgt gctttgtatc actttttgtg atatccatgc catggtccag 2160
   ccagggtccg gagttgatgt ggcaagaagg cctggctttc gggccctgtg cgatcctggt 2220
   ttgggtgcat ctgagtgggt ggtggcaaag atcagggagg caggagctgc ttctgggtct 2280
   gtagtggagc tggttgctgc tgctggcggt gacctggcca acccaatctg cccctgccct 2340
   cccacaggac ttcacctttg aaacctcggg ggagaatacg gctgtagtgg ccgtggagcc 2400
   tgaccgccgg aaccagtccc cagtggatca gggggccacg ggggcctcac agggcctcct 2460
   ggacaggaaa gaggtgctgg gaggtgagtt ttctttcagg ggggtagttt ggggtgaatt 2520
   gctgctgtgg ggtcagggtg gggctgacca cagccaaggc cactgctttg ggagggtctg 2580
   cacgagagcc caaggagccg ctgagctgag ctggccccgt ctacctgccc taggggtcat 2640
   tgccggaggc ctcgtggggc tcatctttgc tgtgtgcctg gtgggtttca tgctgtaccg 2700
   catgaagaag aaggacgaag gcagctactc cttggaggag ccgaaacaag ccaacggcgg 2760
   ggcctaccag aagcccacca aacaggagga attctatgcc tgacgcggga gccatgcgcc 2820
   ccctccgccc tgccactcac taggccccca cttgcctctt ccttgaagaa ctgcaggccc 2880
   tggcctcccc tgccaccagg ccacctcccc agcattccag cccctctggt cgctcctgcc 2940
   cacggagtcg tgggtgtgct gggagctcca ctctgcttct ctgacttctg cctggagact 3000
   tagggcacca ggggtttctc gcataggacc tttccaccac agccagcacc tggcatcgca 3060
   ccattctgac tcggtttctc caaactgaag cagcctctcc ccaggtccag ctctggaggg 3120
   gagggggatc cgactgcttt ggacctaaat ggcctcatgt ggctggaaga tcctgcgggt 3180
   ggggcttggg gctcacacac ctgtagcact tactggtagg accaagcatc ttgggggggt 3240
   ggccgctgag tggcagggga caggagtcac tttgtttcgt ggggaggtct aatctagata 3300
   tcgacttgtt tttgcacatg tttcctctag ttctttgttc atagcccagt agaccttgtt 3360
   acttctgagg taagttaagt aagttgattc ggtatccccc catcttgctt ccctaatcta 3420
   tggtcgggag acagcatcag ggttaagaag actttttttt ttttttttaa actaggagaa 3480
   ccaaatctgg aagccaaaat gtaggcttag tttgtgtgtt gtctcttgag tttgtcgctc 3540
   atgtgtgcaa cagggtatgg actatctgtc tggtggcccc gttctggtgg tctgttggca 3600
   ggctggccag tccaggctgc cgtggggccg ccgcctcttt caagcagtcg tgcctgtgtc 3660
   catgcgctca gggccatgct gaggcctggg ccgctgccac gttggagaag cccgtgtgag 3720
   aagtgaatgc tgggactcag ccttcagaca gagaggactg tagggagggc ggcaggggcc 3780
   tggagatcct cctgcaggct cacgcccgtc ctcctgtggc gccgtctcca ggggctgctt 3840
   cctcctggaa attgacgagg ggtgtcttgg gcagagctgg ctctgagcgc ctccatccaa 3900
   ggccaggttc tccgttagct cctgtggccc caccctgggc cctgggctgg aatcaggaat 3960
   attttccaaa gagtgatagt cttttgcttt tggcaaaact ctacttaatc caatgggttt 4020
   ttccctgtac agtagatttt ccaaatgtaa taaactttaa tataaagtag tctgtgaatg 4080
   ccactgcctt cgcttcttgc ctctgtgctg tgtgtgacgt gaccggactt ttctgcaaac 4140
   accaacatgt tgggaaactt ggctcgaatc tctgtgcctt cgtctttccc atggggaggg 4200
   attctggttc cagggtccct ctgtgtattt gcttttttgt tttggctgaa attctcctgg 4260
   aggtcggtag gttcagccaa ggttttataa ggctgatgtc aatttctgtg ttgccaagct 4320
   ccaagcccat cttctaaatg gcaaaggaag gtggatggcc ccagcacagc ttgacctgag 4380
   gctgtggtca cagcggaggt gtggagccga ggcctacccc ncagacacct tggacatcct 4440
   cctcccaccc ggctgcagag gccaganncc agcccagggt cctgcactta cttgcttatt 4500
   tgacaacgtt tcagcgactc cgttggccac tccgagagtg ggccagtctg tggatcagag 4560
   atgcaccacc aagccaaggg aacctgtgtc cggtattcga tactgcgact ttctgcctgg 4620
   agtgtatgac tgcacatgac tcgggggtgg ggaaaggggt cggctgacca tgctcatctg 4680
   ctggtccgtg ggacggtncc caagccagag gtgggttcat ttgtgtaacg acaataaacg 4740
   gtacttgtca tttcgggcaa cggctgctgt ggtggtggtt gagtctcttc ttggcct 4797
<210> 135
   <211> 2856
   <212> DNA
   <213> Homo sapiens
<400> 135
   tagtcgcggg tccccgagtg agcacgccag ggagcaggag accaaacgac gggggtcgga 60
   gtcagagtcg cagtgggagt ccccggaccg gagcacgagc ctgagcggga gagcgccgct 120
   cgcacgcccg tcgccacccg cgtacccggc gcagccagag ccaccagcgc agcgctgcca 180
   tggagcccag cagcaagaag ctgacgggtc gcctcatgct ggctgtggga ggagcagtgc 240
   ttggctccct gcagtttggc tacaacactg gagtcatcaa tgccccccag aaggtgatcg 300
   aggagttcta caaccagaca tgggtccacc gctatgggga gagcatcctg cccaccacgc 360
   tcaccacgct ctggtccctc tcagtggcca tcttttctgt tgggggcatg attggctcct 420
   tctctgtggg ccttttcgtt aaccgctttg gccggcggaa ttcaatgctg atgatgaacc 480
   tgctggcctt cgtgtccgcc gtgctcatgg gcttctcgaa actgggcaag tcctttgaga 540
   tgctgatcct gggccgcttc atcatcggtg tgtactgcgg cctgaccaca ggcttcgtgc 600
   ccatgtatgt gggtgaagtg tcacccacag cctttcgtgg ggccctgggc accctgcacc 660
   agctgggcat cgtcgtcggc atcctcatcg cccaggtgtt cggcctggac tccatcatgg 720
   gcaacaagga cctgtggccc ctgctgctga gcatcatctt catcccggcc ctgctgcagt 780
   gcatcgtgct gcccttctgc cccgagagtc cccgcttcct gctcatcaac cgcaacgagg 840
   agaaccgggc caagagtgtg ctaaagaagc tgcgcgggac agctgacgtg acccatgacc 900
   tgcaggagat gaaggaagag agtcggcaga tgatgcggga gaagaaggtc accatcctgg 960
   agctgttccg ctcccccgcc taccgccagc ccatcctcat cgctgtggtg ctgcagctgt 1020
   cccagcagct gtctggcatc aacgctgtct tctattactc cacgagcatc ttcgagaagg 1080
   cgggggtgca gcagcctgtg tatgccacca ttggctccgg tatcgtcaac acggccttca 1140
   ctgtcgtgtc gctgtttgtg gtggagcgag caggccggcg gaccctgcac ctcataggcc 1200
   tcgctggcat ggcgggttgt gccatactca tgaccatcgc gctagcactg ctggagcagc 1260
   taccctggat gtcctatctg agcatcgtgg ccatctttgg ctttgtggcc ttctttgaag 1320
   tgggtcctgg ccccatccca tggttcatcg tggctgaact cttcagccag ggtccacgtc 1380
   cagctgccat tgccgttgca ggcttctcca actggacctc aaatttcatt gtgggcatgt 1440
   gcttccagta tgtggagcaa ctgtgtggtc cctacgtctt catcatcttc actgtgctcc 1500
   tggttctgtt cttcatcttc acctacttca aagttcctga gactaaaggc cggaccttcg 1560
   atgagatcgc ttccggcttc cggcaggggg gagccagcca aagtgataag acacccgagg 1620
   agctgttcca tcccctgggg gctgattccc aagtgtgagt cgccccagat caccagcccg 1680
   gcctgctccc agcagcccta aggatctctc aggagcacag gcagctggat gagacttcca 1740
   aacctgacag atgtcagccg agccgggcct ggggctcctt tctccagcca gcaatgatgt 1800
   ccagaagaat attcaggact taacggctcc aggattttaa caaaagcaag actgttgctc 1860
   aaatctattc agacaagcaa caggttttat aattttttta ttactgattt tgttattttt 1920
   atatcagcct gagtctcctg tgcccacatc ccaggcttca ccctgaatgg ttccatgcct 1980
   gagggtggag actaagccct gtcgagacac ttgccttctt cacccagcta atctgtaggg 2040
   ctggacctat gtcctaagga cacactaatc gaactatgaa ctacaaagct tctatcccag 2100
   gaggtggcta tggccacccg ttctgctggc ctggatctcc ccactctagg ggtcaggctc 2160
   cattaggatt tgccccttcc catctcttcc tacccaacca ctcaaattaa tctttcttta 2220
   cctgagacca gttgggagca ctggagtgca gggaggagag gggaagggcc agtctgggct 2280
   gccgggttct agtctccttt gcactgaggg ccacactatt accatgagaa gagggcctgt 2340
   gggagcctgc aaactcactg ctcaagaaga catggagact cctgccctgt tgtgtataga 2400
   tgcaagatat ttatatatat ttttggttgt caatattaaa tacagacact aagttatagt 2460
   atatctggac aagccaactt gtaaatacac cacctcactc ctgttactta cctaaacaga 2520
   tataaatggc tggtttttag aaacatggtt ttgaaatgct tgtggattga gggtaggagg 2580
   tttggatggg agtgagacag aagtaagtgg ggttgcaacc actgcaacgg cttagacttc 2640
   gactcaggat ccagtccctt acacgtacct ctcatcagtg tcctcttgct caaaaatctg 2700
   tttgatccct gttacccaga gaatatatac attctttatc ttgacattca aggcatttct 2760
   atcacatatt tgatagttgg tgttcaaaaa aacactagtt ttgtgccagc cgtgatgctc 2820
   aggcttgaaa tcgcattatt ttgaatgtga agggaa                           2856
<210> 136
   <211> 356
   <212> DNA
   <213> Homo sapiens
<400> 136
   ggtggagcca aatgaagaaa atgaagatga aagagacaga cacctcagtt tttctggatc 60
   aggcattgat gatgatgaag attttatctc cagcaccatt tcaaccacac cacgggcttt 120
   tgaccacaca aaacagaacc aggactggac tcagtggaac ccaagccatt caaatccgga 180
   agtgctactt cagacaacca caaggatgac tgatgtagac agaaatggca ccactgctta 240
   tgaaggaaac tggaacccag aagcacaccc tcccctcatt caccatgagc atcatgagga 300
   agaagagacc ccacattcta caagcacaat ccaggcaact cctagtagta caacgg 356
<210> 137
   <211> 356
   <212> DNA
   <213> Homo sapiens
<220>
   <221> misc_feature
   <222> 254, 264, 279, 281, 290, 328, 342
   <223> n = A,T,C or G
<400> 137
   gcaggtggag aagacatttt attgttcctg gggtctctgg aggcccattg gtggggctgg 60
   gtcactggct gcccccggaa cagggcgctg ctccatggct ctgcttgtgg tagtctgtgg 120
   ctatgtctcc cagcaaggac agaaactcag aaaaatcaat cttcttatcc tcattcttgt 180
   cctttttctc aaagacatcg gcgaggtaat ttgtgccctt tttacctcgg cccgcgacca 240
   cgctaaggcc aaanttccag acanayggcc gggccggtnc nataggggan cccaacttgg 300
   ggacccaaac tctggcgcgg aaacacangg gcataagctt gnttcctgtg gggaaa 356
<210> 138
   <211> 353
   <212> DNA
   <213> Homo sapiens
<400> 138
   aggtccagtc ctccacttgg cctgatgaga gtggggagtg gcaagggacg tttctcctgc 60
   aatagacact tagatttctc tcttgtggga agaaaccacc tgtccatcca ctgactcttc 120
   tacattgatg tggaaattgc tgctgctacc accacctcct gaagaggctt ccctgatgcc 180
   aatgccagcc atcttggcat cctggccctc gagcaggctg cggtaagtag cgatctcctg 240
   ctccagccgt gtctttatgt caagcagcat cttgtactcc tggttctgag cctccatctc 300
   gcatcggagc tcactcagac ctcgsccgsg mssmcgctam gccgaattcc agc 353
<210> 139
   <211> 371
   <212> DNA
   <213> Homo sapiens
<400> 139
   agcgtggtcg cggccgaggt ccatccgaag caagattgca gatggcagtg tgaagagaga 60
   agacatattc tacacttcaa agctttggtg caattcccat cgaccagagt tggtccgacc 120
   agccttggaa aggtcactga aaaatcttca attggattat gttgacctct accttattca 180
   ttttccagtg tctgtaaagc caggtgagga agtgatccca aaagatgaaa atggaaaaat 240
   actatttgac acagtggatc tctgtgccac gtgggaggcc gtggagaagt gtaaagatgc 300
   aggattggac ctgcccgggc ggccgctcga aagccgaatt ccagcacact ggcggccgtt 360
   actagtggat c 371
<210> 140
   <211> 370
   <212> DNA
   <213> Homo sapiens
<400> 140
   tagcgtggtc gcggccgagg tccatctccc tttgggaact agggggctgc tggtgggaaa 60
   tgggagccag ggcagatgtt gcattccttt gtgtccctgt aaatgtggga ctacaagaag 120
   aggagctgcc tgagtggtac tttctcttcc tggtaatcct ctggcccagc ctcatggcag 180
   aatagaggta tttttaggct atttttgtaa tatggcttct ggtcaaaatc cctgtgtagc 240
   tgaattccca agccctgcat tgtacagccc cccactcccc tcaccaccta ataaaggaat 300
   agttaacact caaaaaaaaa aaaaaacctg cccgggcggc cgctcgaaag ccgaattcca 360
   gcacactggc 370
<210> 141
   <211> 371
   <212> DNA
   <213> Homo sapiens
<400> 141
   tagcgtggtc gcggccgagg tcctctgtgc tgcctgtcac agcccgatgg taccagcgca 60
   gggtgtaggc agtgcaggag ccctcatcca gtggcaggga acaggggtca tcactatccc 120
   aaggagcttc agggtcctgg tactcctcca cagaatactc ggagtattca gagtactcat 180
   catcctcagg gggtacccgc tcttcctcct ctgcatgaga gacgcggagc acaggcacag 240
   catggagctg ggagccggca gtgtctgcag cataactagg gaggggtcgt gatccagatg 300
   cgatgaactg gccctggcag gcacagtgct gactcatctc ttggcgacct gcccgggcgg 360
   ccgctcgaag c 371
<210> 142
   <211> 343
   <212> DNA
   <213> Homo sapiens
<400> 142
   gcgttttgag gccaatggtg taaaaggaaa tatcttcaca taaaaactag atggaagcat 60
   tgtcagaaac ctctttgtga tgtttgcttt caactcacag agttgaacat tccttttcat 120
   agagcagttt tgaaacactc ttttgtagaa tttgcaagcg gatgattgga tcgctatgag 180
   gtcttcattg gaaacgggat acctttacat aaaaactaga cagtagcatt ctcagaaatt 240
   tctttgggat gtgggcattc aacccacaga ggagaacttc atttgataga gcagttttga 300
   aacacccttt ttgtagaatc tacaggtgga catttagagt gct 343
<210> 143
   <211> 354
   <212> DNA
   <213> Homo sapiens
<400> 143
   aggtctgatg gcagaaaaac tcagactgtc tgcaacttta cagatggtgc attggttcag 60
   catcaggagt gggatgggaa ggaaagcaca ataacaagaa aattgaaaga tgggaaatta 120
   gtggtggagt gtgtcatgaa caatgtcacc tgtactcgga tctatgaaaa agtagaataa 180
   aaattccatc atcactttgg acaggagtta attaagagaa tgaccaagct cagttcaatg 240
   agcaaatctc catactgttt ctttcttttt tttttcatta ctgtgttcaa ttatctttat 300
   cataaacatt ttacatgcag ctatttcaaa gtgtgttgga ttaattagga tcat       354
<210> 144
   <211> 353
   <212> DNA
   <213> Homo sapiens
<400> 144
   ggtcaaggac ctgggggacc cccaggtcca gcagccacat gattctgcag cagacaggga 60
   cctagagcac atctggatct cagccccacc cctggcaacc tgcctgccta gagaactccc 120
   aagatgacag actaagtagg attctgccat ttagaataat tctggtatcc tgggcgttgc 180
   gttaagttgc ttaactttca ttctgtctta cgatagtctt cagaggtggg aacagatgaa 240
   gaaaccatgc cccagagaag gttaagtgac ttcctcttta tggagccagt gttccaacct 300
   aggtttgcct gataccagac ctgtggcccc acctcccatg caggtctctg tgg        353
<210> 145
   <211> 371
   <212> DNA
   <213> Homo sapiens
<400> 145
   caggtctgtc ataaactggt ctggagtttc tgacgactcc ttgttcacca aatgcaccat 60
   ttcctgagac ttgctggcct ctccgttgag tccacttggc tttctgtcct ccacagctcc 120
   attgccactg ttgatcacta gctttttctt ctgcccacac cttcttcgac tgttgactgc 180
   aatgcaaact gcaagaatca aagccaaggc caagagggat gccaagatga tcagccattc 240
   tggaatttgg ggtgtcctta taggaccaga ggttgtgttt gctccacctt cttgactccc 300
   atgtgagacc tcggccgcga ccacgctaag ccgaattcca gcacactggc ggcccgttac 360
   tagtggatcc g                                                      371
<210> 146
   <211> 355
   <212> DNA
   <213> Homo sapiens
<400> 146
   ggtcctccgt cctcttccca gaggtgtcgg ggcttggccc cagcctccat cttcgtctct 60
   caggatggcg agtagcagcg gctccaaggc tgaattcatt gtcggaggga aatataaact 120
   ggtacggaag atcgggtctg gctccttcgg ggacatctat ttggcgatca acatcaccaa 180
   cggcgaggaa gtggcagtga agctagaatc tcagaaggcc aggcatcccc agttgctgta 240
   cgagagcaag ctctataaga ttcttcaagg tggggttggc atcccccaca tacggtggta 300
   tggtcaggaa aaagactaca atgtactagt catggatctt ctgggaccta gcctc      355
<210> 147
   <211> 355
   <212> DNA
   <213> Homo sapiens
<400> 147
   ggtctgttac aaaatgaaga cagacaacac aacatttact ctgtggagat atcctactca 60
   tactatgcac gtgctgtgat tttgaacata actcgtccca aaaacttgtc acgatcatcc 120
   tgacttttta ggttggctga tccatcaatc ttgcactcaa ctgttacttc tttcccagtg 180
   ttgttaggag caaagctgac ctgaacagca accaatggct gtagataccc aacatgcagt 240
   tttttcccat aatatgggaa atattttaag tctatcattc cattatgagg ataaactgct 300
   acatttggta tatcttcatt ctttgaaaca caatctatcc ttggcactcc ttcag      355
<210> 148
   <211> 369
   <212> DNA
   <213> Homo sapiens
<400> 148
   aggtctctct ccccctctcc ctctcctgcc agccaagtga agacatgctt acttcccctt 60
   caccttcctt catgatgtgg gaagagtgct gcaacccagc cctagccaac accgcatgag 120
   agggagtgtg ccgagggctt ctgagaaggt ttctctcaca tctagaaaga agcgcttaag 180
   atgtggcagc ccctcttctt caagtggctc ttgtcctgtt gccctgggag ttctcaaatt 240
   gctgcagcag cctccatcca gcctgaggat gacatcaata cacagaggaa gaagagtcag 300
   gaaaagatga gagaagttac agactctcct gggcgacccc gagagcttac cattcctcag 360
   acttcttca                                                         369
<210> 149
   <211> 620
   <212> DNA
   <213> Homo sapiens
<220>
   <221> misc_feature
   <222> 169, 171, 222, 472, 528, 559, 599
   <223> n = A,T,C or G
<400> 149
   actagtcaaa aatgctaaaa taatttggga gaaaatattt tttaagtagt gttatagttt 60
   catgtttatc ttttattatg ttttgtgaag ttgtgtcttt tcactaatta cctatactat 120
   gccaatattt ccttatatct atccataaca tttatactac atttgtaana naatatgcac 180
   gtgaaactta acactttata aggtaaaaat gaggtttcca anatttaata atctgatcaa 240
   gttcttgtta tttccaaata gaatggactt ggtctgttaa gggctaagga gaagaggaag 300
   ataaggttaa aagttgttaa tgaccaaaca ttctaaaaga aatgcaaaaa aaaagtttat 360
   tttcaagcct tcgaactatt taaggaaagc aaaatcattt cctaaatgca tatcatttgt 420
   gagaatttct cattaatatc ctgaatcatt catttcacta aggctcatgt tnactccgat 480
   atgtctctaa gaaagtacta tttcatggtc caaacctggt tgccatantt gggtaaaggc 540
   tttcccttaa gtgtgaaant atttaaaatg aaattttcct ctttttaaaa attctttana 600
   agggttaagg gtgttgggga 620
<210> 150
   <211> 371
   <212> DNA
   <213> Homo sapiens
<400> 150
   ggtccgatca aaacctgcta cctccccaag actttactag tgccgataaa ctttctcaaa 60
   gagcaaccag tatcacttcc ctgtttataa aacctctaac catctctttg ttctttgaac 120
   atgctgaaaa ccacctggtc tgcatgtatg cccgaatttg yaattctttt ctctcaaatg 180
   aaaatttaat tttagggatt catttctata ttttcacata tgtagtatta ttatttcctt 240
   atatgtgtaa ggtgaaattt atggtatttg agtgtgcaag aaaatatatt tttaaagctt 300
   tcatttttcc cccagtgaat gatttagaat tttttatgta aatatacaga atgttttttc 360
   ttacttttat a 371
<210> 151
   <211> 4655
   <212> DNA
   <213> Homo sapiens
<400> 151
   gggacttgag ttctgttatc ttcttaagta gattcatatt gtaagggtct cggggtgggg 60
   gggttggcaa aatcctggag ccagaagaaa ggacagcagc attgatcaat cttacagcta 120
   acatgttgta cctggaaaac aatgcccaga ctcaatttag tgagccacag tacacgaacc 180
   tggggctcct gaacagcatg gaccagcaga ttcagaacgg ctcctcgtcc accagtccct 240
   ataacacaga ccacgcgcag aacagcgtca cggcgccctc gccctacgca cagcccagct 300
   ccaccttcga tgctctctct ccatcacccg ccatcccctc caacaccgac tacccaggcc 360
   cgcacagttt cgacgtgtcc ttccagcagt cgagcaccgc caagtcggcc acctggacgt 420
   attccactga actgaagaaa ctctactgcc aaattgcaaa gacatgcccc atccagatca 480
   aggtgatgac cccacctcct cagggagctg ttatccgcgc catgcctgtc tacaaaaaag 540
   ctgagcacgt cacggaggtg gtgaagcggt gccccaacca tgagctgagc cgtgaattca 600
   acgagggaca gattgcccct yctagtcatt tgattcgagt agaggggaac agccatgccc 660
   agtatgtaga agatcccatc acaggaagac agagtgtgct ggtaccttat gagccacccc 720
   aggttggcac tgaattcacg acagtcttgt acaatttcat gtgtaacagc agttgtgttg 780
   gagggatgaa ccgccgtcca attttaatca ttgttactct ggaaaccaga gatgggcaag 840
   tcctgggccg acgctgcttt gaggcccgga tctgtgcttg cccaggaaga gacaggaagg 900
   cggatgaaga tagcatcaga aagcagcaag tttcggacag tacaaagaac ggtgatggta 960
   cgaagcgccc gtttcgtcag aacacacatg gtatccagat gacatccatc aagaaacgaa 1020
   gatccccaga tgatgaactg gtatacttac cagtgagggg ccgtgagact tatgaaatgc 1080
   tggtgaagat caaagagtcc ctggaactca tgcagtacct tcttcagcac acaattgaaa 1140
   cgtacaggca acagcaacag cagcagcacc agcacttact tcagaaacag acctcaatac 1200
   agtctccatc ttcatatggt aacagctccc cacctctgaa caaaatgaac agcatgaaca 1260
   agctgccttc tgtgagccag cttatcaacc ctcagcagcg caacgccctc actcctacaa 1320
   ccattcctga tggcatggga gccaacattc ccatgatggg cacccacatg ccaatggctg 1380
   gagacatgaa tggactcagc cccacccagg cactccctcc cccactctcc atgccatcca 1440
   cctcccactg cacaccccca cctccgtatc ccacagattg cagcattgtc agtttcttag 1500
   cgaggttggg ctgttcatca tgtctggact atttcacgac ccaggggctg accaccatct 1560
   atcagattga gcattactcc atggatgatc tggcaagtct gaaaatccct gagcaatttc 1620
   gacatgcgat ctggaagggc atcctggacc accggcagct ccacgaattc tcctcccctt 1680
   ctcatctcct gcggacccca agcagtgcct ctacagtcag tgtgggctcc agtgagaccc 1740
   ggggtgagcg tgttattgat gctgtgcgat tcaccctccg ccagaccatc tctttcccac 1800
   cccgagatga gtggaatgac ttcaactttg acatggatgc tcgccgcaat aagcaacagc 1860
   gcatcaaaga ggagggggag tgagcctcac catgtgagct cttcctatcc ctctcctaac 1920
   tgccagcccc ctaaaagcac tcctgcttaa tcttcaaagc cttctcccta gctcctcccc 1980
   ttcctcttgt ctgatttctt aggggaagga gaagtaagag gcttacttct taccctaacc 2040
   atctgacctg gcatctaatt ctgattctgg ctttaagcct tcaaaactat agcttgcaga 2100
   actgtagctt gccatggcta ggtagaagtg agcaaaaaag agttgggtgt ctccttaagc 2160
   tgcagagatt tctcattgac ttttataaag catgttcacc cttatagtct aagactatat 2220
   atataaatgt ataaatatac agtatagatt tttgggtggg gggcattgag tattgtttaa 2280
   aatgtaattt aaatgaaaga aaattgagtt gcacttattg accatttttt aatttacttg 2340
   ttttggatgg cttgtctata ctccttccct taaggggtat catgtatggt gataggtatc 2400
   tagagcttaa tgctacatgt gagtgacgat gatgtacaga ttctttcagt tctttggatt 2460
   ctaaatacat gccacatcaa acctttgagt agatccattt ccattgctta ttatgtaggt 2520
   aagactgtag atatgtattc ttttctcagt gttggtatat tttatattac tgacatttct 2580
   tctagtgatg atggttcacg ttggggtgat ttaatccagt tataagaaga agttcatgtc 2640
   caaacgtcct ctttagtttt tggttgggaa tgaggaaaat tcttaaaagg cccatagcag 2700
   ccagttcaaa aacacccgac gtcatgtatt tgagcatatc agtaaccccc ttaaatttaa 2760
   taccagatac cttatcttac aatattgatt gggaaaacat ttgctgccat tacagaggta 2820
   ttaaaactaa atttcactac tagattgact aactcaaata cacatttgct actgttgtaa 2880
   gaattctgat tgatttgatt gggatgaatg ccatctatct agttctaaca gtgaagtttt 2940
   actgtctatt aatattcagg gtaaatagga atcattcaga aatgttgagt ctgtactaaa 3000
   cagtaagata tctcaatgaa ccataaattc aactttgtaa aaatcttttg aagcatagat 3060
   aatattgttt ggtaaatgtt tcttttgttt ggtaaatgtt tcytttaaag accctcctat 3120
   tctataaaac tctgcatgta gaggcttgtt tacctttctc tctctaaggt ttacaatagg 3180
   agtggtgatt tgaaaaatat aaaattatga gattggtttt cctgtggcat aaattgcatc 3240
   actgtatcat tttctttttt aaccggtaag agtttcagtt tgttggaaag taactgtgag 3300
   aacccagttt cccgtccatc tcccttaggg actacccata gacatgaaag gtccccacag 3360
   agcaagagat aagtctttca tggctgctgt tgcttaaacc acttaaacga agagttccct 3420
   tgaaactttg ggaaaacatg ttaatgacaa tattccagat ctttcagaaa tataacacat 3480
   ttttttgcat gcatgcaaat gagctctgaa atcttcccat gcattctggt caagggctgt 3540
   cattgcacat aagcttccat tttaatttta aagtgcaaaa gggccagcgt ggctctaaaa 3600
   ggtaatgtgt ggattgcctc tgaaaagtgt gtatatattt tgtgtgaaat tgcatacttt 3660
   gtattttgat tatttttttt ttcttcttgg gatagtggga tttccagaac cacacttgaa 3720
   accttttttt atcgtttttg tattttcatg aaaataccat ttagtaagaa taccacatca 3780
   aataagaaat aatgctacaa ttttaagagg ggagggaagg gaaagttttt ttttttatta 3840
   tttttttaaa attttgtatg ttaaagagaa tgagtccttg atttcaaagt tttgttgtac 3900
   ttaaatggta ataagcactg taaacttctg caacaagcat gcagctttgc aaacccatta 3960
   aggggaagaa tgaaagctgt tccttggtcc tagtaagaag acaaactgct tcccttactt 4020
   tgctgagggt ttgaataaac ctaggacttc cgagctatgt cagtactatt caggtaacac 4080
   tagggccttg gaaatccctg tactgtgtct catggatttg gcactagcca aagcgaggca 4140
   ccccttactg gcttacctcc tcatggcagc ctactctcct tgagtgtatg agtagccagg 4200
   gtaaggggta aaaggatagt aagcatagaa accactagaa agtgggctta atggagttct 4260
   tgtggcctca gctcaatgca gttagctgaa gaattgaaaa gtttttgttt ggagacgttt 4320
   ataaacagaa atggaaagca gagttttcat taaatccttt tacctttttt ttttcttggt 4380
   aatcccctaa aataacagta tgtgggatat tgaatgttaa agggatattt ttttctatta 4440
   tttttataat tgtacaaaat taagcaaatg ttaaaagttt tatatgcttt attaatgttt 4500
   tcaaaaggta ttatacatgt gatacatttt ttaagcttca gttgcttgtc ttctggtact 4560
   ttctgttatg ggcttttggg gagccagaag ccaatctaca atctcttttt gtttgccagg 4620
   acatgcaata aaatttaaaa aataaataaa aacta 4655
<210> 152
   <211> 586
   <212> PRT
   <213> Homo sapiens
<400> 152
   Met Leu Tyr Leu Glu Asn Asn Ala Gln Thr Gln Phe Ser Glu Pro Gln
   1 5 10 15
   Tyr Thr Asn Leu Gly Leu Leu Asn Ser Met Asp Gln Gln Ile Gln Asn
   20 25 30
   Gly Ser Ser Ser Thr Ser Pro Tyr Asn Thr Asp His Ala Gln Asn Ser
   35 40 45
   Val Thr Ala Pro Ser Pro Tyr Ala Gln Pro Ser Ser Thr Phe Asp Ala
   50 55 60
   Leu Ser Pro Ser Pro Ala Ile Pro Ser Asn Thr Asp Tyr Pro Gly Pro
   65 70 75 80
   His Ser Phe Asp Val Ser Phe Gln Gln Ser Ser Thr Ala Lys Ser Ala
   85 90 95
   Thr Trp Thr Tyr Ser Thr Glu Leu Lys Lys Leu Tyr Cys Gln Ile Ala
   100 105 110
   Lys Thr Cys Pro Ile Gln Ile Lys Val Met Thr Pro Pro Pro Gln Gly
   115 120 125
   Ala Val Ile Arg Ala Met Pro Val Tyr Lys Lys Ala Glu His Val Thr
   130 135 140
   Glu Val Val Lys Arg Cys Pro Asn His Glu Leu Ser Arg Glu Phe Asn
   145 150 155 160
   Glu Gly Gln Ile Ala Pro Ser Ser His Leu Ile Arg Val Glu Gly Asn
   165 170 175
   Ser His Ala Gln Tyr Val Glu Asp Pro Ile Thr Gly Arg Gln Ser Val
   180 185 190
   Leu Val Pro Tyr Glu Pro Pro Gln Val Gly Thr Glu Phe Thr Thr Val
   195 200 205
   Leu Tyr Asn Phe Met Cys Asn Ser Ser Cys Val Gly Gly Met Asn Arg
   210 215 220
   Arg Pro Ile Leu Ile Ile Val Thr Leu Glu Thr Arg Asp Gly Gln Val
   225 230 235 240
   Leu Gly Arg Arg Cys Phe Glu Ala Arg Ile Cys Ala Cys Pro Gly Arg
   245 250 255
   Asp Arg Lys Ala Asp Glu Asp Ser Ile Arg Lys Gln Gln Val Ser Asp
                   260                 265                 270
   Ser Thr Lys Asn Gly Asp Gly Thr Lys Arg Pro Phe Arg Gln Asn Thr
               275                 280                 285
   His Gly Ile Gln Met Thr Ser Ile Lys Lys Arg Arg Ser Pro Asp Asp
       290                 295                 300
   Glu Leu Val Tyr Leu Pro Val Arg Gly Arg Glu Thr Tyr Glu Met Leu
   305                 310                 315                 320
   Val Lys Ile Lys Glu Ser Leu Glu Leu Met Gln Tyr Leu Leu Gln His
                   325                 330                 335
   Thr Ile Glu Thr Tyr Arg Gln Gln Gln Gln Gln Gln His Gln His Leu
               340                 345                 350
   Leu Gln Lys Gln Thr Ser Ile Gln Ser Pro Ser Ser Tyr Gly Asn Ser
           355                 360                 365
   Ser Pro Pro Leu Asn Lys Met Asn Ser Met Asn Lys Leu Pro Ser Val
       370                 375                 380
   Ser Gln Leu Ile Asn Pro Gln Gln Arg Asn Ala Leu Thr Pro Thr Thr
   385                 390                 395                 400
   Ile Pro Asp Gly Met Gly Ala Asn Ile Pro Met Met Gly Thr His Met
                   405                 410                 415
   Pro Met Ala Gly Asp Met Asn Gly Leu Ser Pro Thr Gln Ala Leu Pro
               420                 425                 430
   Pro Pro Leu Ser Met Pro Ser Thr Ser His Cys Thr Pro Pro Pro Pro
           435                 440                 445
   Tyr Pro Thr Asp Cys Ser Ile Val Ser Phe Leu Ala Arg Leu Gly Cys
       450                 455                 460
   Ser Ser Cys Leu Asp Tyr Phe Thr Thr Gln Gly Leu Thr Thr Ile Tyr
   465                 470                 475                 480
   Gln Ile Glu His Tyr Ser Met Asp Asp Leu Ala Ser Leu Lys Ile Pro
                   485                 490                 495
   Glu Gln Phe Arg His Ala Ile Trp Lys Gly Ile Leu Asp His Arg Gln
               500                 505                 510
   Leu His Glu Phe Ser Ser Pro Ser His Leu Leu Arg Thr Pro Ser Ser
           515                 520                 525
   Ala Ser Thr Val Ser Val Gly Ser Ser Glu Thr Arg Gly Glu Arg Val
       530                 535                 540
   Ile Asp Ala Val Arg Phe Thr Leu Arg Gln Thr Ile Ser Phe Pro Pro
   545                 550                 555                 560
   Arg Asp Glu Trp Asn Asp Phe Asn Phe Asp Met Asp Ala Arg Arg Asn
                   565                 570                 575
   Lys Gln Gln Arg Ile Lys Glu Glu Gly Glu
               580                 585

<210> 153
   <211> 2007
   <212> DNA
   <213> Homo sapiens
<400> 153
<210> 154
   <211> 2148
   <212> DNA
   <213> Homo sapiens
<400> 154
<210> 155
   <211> 153
   <212> PRT
   <213> Homo sapiens
<400> 155
   Met Thr Ser Val Arg Val Ala Ala Tyr Phe Glu Asn Phe Leu Ala Ala
    1               5                  10                  15
   Trp Arg Pro Val Lys Ala Ser Asp Gly Asp Tyr Tyr Thr Leu Ala Val
               20                  25                  30
   Pro Met Gly Asp Val Pro Met Asp Gly Ile Ser Val Ala Asp Ile Gly
           35                  40                  45
   Ala Ala Val Ser Ser Ile Phe Asn Ser Pro Glu Glu Phe Leu Gly Lys
       50                  55                  60
   Ala Val Gly Leu Ser Ala Glu Ala Leu Thr Ile Gln Gln Tyr Ala Asp
   65                  70                  75                  80
   Val Leu Ser Lys Ala Leu Gly Lys Glu Val Arg Asp Ala Lys Ile Thr
                   85                  90                  95
   Pro Glu Ala Phe Glu Lys Leu Gly Phe Pro Ala Ala Lys Glu Ile Ala
               100                 105                 110
   Asn Met Cys Arg Phe Tyr Glu Met Lys Pro Asp Arg Asp Val Asn Leu
           115                 120                 125
   Thr His Gln Leu Asn Pro Lys Val Lys Ser Phe Ser Gln Phe Ile Ser
       130                 135                 140
   Glu Asn Gln Gly Ala Phe Lys Gly Met
   145                 150
<210> 156
   <211> 128
   <212> PRT
   <213> Homo sapiens
<400> 156
   Met Thr Ser Val Arg Val Ala Ala Tyr Phe Glu Asn Phe Leu Ala Ala
    1               5                  10                  15
   Trp Arg Pro Val Lys Ala Ser Asp Gly Asp Tyr Tyr Thr Leu Ala Val
               20                  25                  30
   Pro Met Gly Asp Val Pro Met Asp Gly Ile Ser Val Ala Asp Ile Gly
           35                  40                  45
   Ala Ala Val Ser Ser Ile Phe Asn Ser Pro Glu Glu Phe Leu Gly Lys
       50                  55                  60
   Ala Val Gly Leu Ser Ala Glu Ala Leu Thr Ile Gln Gln Tyr Ala Asp
   65                  70                  75                  80
   Val Leu Ser Lys Ala Leu Gly Lys Glu Val Arg Asp Ala Lys Thr Ile
                   85                  90                  95
   Cys Ala Ile Asp Asp Gln Lys Thr Val Glu Glu Gly Phe Met Glu Asp
               100                 105                 110
   Val Gly Leu Ser Trp Ser Leu Arg Glu His Asp His Val Ala Gly Ala
           115                 120                 125
<210> 157
   <211> 424
   <212> DNA
   <213> Homo sapiens
<220>
   <221> misc_feature
   <222> 320, 322
   <223> n = A,T,C or G
<400> 157
   ctgcagcccg ggggatccac tagtccagtg tggtggaatt cattggtctt tacaagactt 60
   ggatacatta cagcagacat ggaaatataa ttttaaaaaa tttctctcca acctccttca 120
   aattcagtca ccactgttat attaccttct ccaggaaccc tccagtgggg aaggctgcga 180
   tattagattt ccttgtatgc aaagtttttg ttgaaagctg tgctcagagg aggtgagagg 240
   agaggaagga gaaaactgca tcataacttt acagaattga atctagagtc ttccccgaaa 300
   agcccagaaa cttctctgcn gnatctggct tgtccatctg gtctaaggtg gctgcttctt 360
   ccccagccat cgagtcagtt tgtgcccatg aataatacac gacctgctat ttcccatgac 420
   tgct                                                              424
<210> 158
   <211> 2099
   <212> DNA
   <213> Homo sapiens
<400> 158
<210> 159
   <211> 291
   <212> PRT
   <213> Homo sapiens
<400> 159
   Met Asp Trp Gly Thr Leu His Thr Phe Ile Gly Gly Val Asn Lys His
   1               5                   10                  15
   Ser Thr Ser Ile Gly Lys Val Trp Ile Thr Val Ile Phe Ile Phe Arg
               20                  25                  30
   Val Met Ile Leu Val Val Ala Ala Gln Glu Val Trp Gly Asp Glu Gln
           35                  40                  45
   Glu Asp Phe Val Cys Asn Thr Leu Gln Pro Gly Cys Lys Asn Val Cys
       50                  55                  60
   Tyr Asp His Phe Phe Pro Val Ser His Ile Arg Leu Trp Ala Leu Gln
   65                  70                  75                  80
   Leu Ile Phe Val Ser Thr Pro Ala Leu Leu Val Ala Met His Val Ala
                   85                  90                  95
   Tyr Tyr Arg His Glu Thr Thr Arg Lys Phe Arg Arg Gly Glu Lys Arg
               100                 105                 110
   Asn Asp Phe Lys Asp Ile Glu Asp Ile Lys Lys Gln Lys Val Arg Ile
           115                 120                 125
   Glu Gly Ser Leu Trp Trp Thr Tyr Thr Ser Ser Ile Phe Phe Arg Ile
       130                 135                 140
   Ile Phe Glu Ala Ala Phe Met Tyr Val Phe Tyr Phe Leu Tyr Asn Gly
   145                 150                 155                 160
   Tyr His Leu Pro Trp Val Leu Lys Cys Gly Ile Asp Pro Cys Pro Asn
                   165                 170                 175
   Leu Val Asp Cys Phe Ile Ser Arg Pro Thr Glu Lys Thr Val Phe Thr
               180                 185                 190
   Ile Phe Met Ile Ser Ala Ser Val Ile Cys Met Leu Leu Asn Val Ala
           195                 200                 205
   Glu Leu Cys Tyr Leu Leu Leu Lys Val Cys Phe Arg Arg Ser Lys Arg
       210                 215                 220
   Ala Gln Thr Gln Lys Asn His Pro Asn His Ala Leu Lys Glu Ser Lys
   225                 230                 235                 240
   Gln Asn Glu Met Asn Glu Leu Ile Ser Asp Ser Gly Gln Asn Ala Ile
                   245                 250                 255
   Thr Gly Ser Gln Ala Lys His Phe Lys Val Lys Cys Ser Cys Val Ile
               260                 265                 270
   Arg Arg Leu Leu Ser Ser Pro Glu Gly Asn Thr Asn Leu Lys Val Pro
           275                 280                 285
   Ser Val Ala
       290
<210> 160
   <211> 3951
   <212> DNA
   <213> Homo sapiens
<400> 160
   tctgcatcca tattgaaaac ctgacacaat gtatgcagca ggctcagtgt gagtgaactg 60
   gaggcttctc tacaacatga cccaaaggag cattgcaggt cctatttgca acctgaagtt 120
   tgtgactctc ctggttgcct taagttcaga actcccattc ctgggagctg gagtacagct 180
   tcaagacaat gggtataatg gattgctcat tgcaattaat cctcaggtac ctgagaatca 240
   gaacctcatc tcaaacatta aggaaatgat aactgaagct tcattttacc tatttaatgc 300
   taccaagaga agagtatttt tcagaaatat aaagatttta atacctgcca catggaaagc 360
   taataataac agcaaaataa aacaagaatc atatgaaaag gcaaatgtca tagtgactga 420
   ctggtatggg gcacatggag atgatccata caccctacaa tacagagggt gtggaaaaga 480
   gggaaaatac attcatttca cacctaattt cctactgaat gataacttaa cagctggcta 540
   cggatcacga ggccgagtgt ttgtccatga atgggcccac ctccgttggg gtgtgttcga 600
   tgagtataac aatgacaaac ctttctacat aaatgggcaa aatcaaatta aagtgacaag 660
   gtgttcatct gacatcacag gcatttttgt gtgtgaaaaa ggtccttgcc cccaagaaaa 720
   ctgtattatt agtaagcttt ttaaagaagg atgcaccttt atctacaata gcacccaaaa 780
   tgcaactgca tcaataatgt tcatgcaaag tttatcttct gtggttgaat tttgtaatgc 840
   aagtacccac aaccaagaag caccaaacct acagaaccag atgtgcagcc tcagaagtgc 900
   atgggatgta atcacagact ctgctgactt tcaccacagc tttcccatga acgggactga 960
   gcttccacct cctcccacat tctcgcttgt agaggctggt gacaaagtgg tctgtttagt 1020
   gctggatgtg tccagcaaga tggcagaggc tgacagactc cttcaactac aacaagccgc 1080
   agaattttat ttgatgcaga ttgttgaaat tcataccttc gtgggcattg ccagtttcga 1140
   cagcaaagga gagatcagag cccagctaca ccaaattaac agcaatgatg atcgaaagtt 1200
   gctggtttca tatctgccca ccactgtatc agctaaaaca gacatcagca tttgttcagg 1260
   gcttaagaaa ggatttgagg tggttgaaaa actgaatgga aaagcttatg gctctgtgat 1320
   gatattagtg accagcggag atgataagct tcttggcaat tgcttaccca ctgtgctcag 1380
   cagtggttca acaattcact ccattgccct gggttcatct gcagccccaa atctggagga 1440
   attatcacgt cttacaggag gtttaaagtt ctttgttcca gatatatcaa actccaatag 1500
   catgattgat gctttcagta gaatttcctc tggaactgga gacattttcc agcaacatat 1560
   tcagcttgaa agtacaggtg aaaatgtcaa acctcaccat caattgaaaa acacagtgac 1620
   tgtggataat actgtgggca acgacactat gtttctagtt acgtggcagg ccagtggtcc 1680
   tcctgagatt atattatttg atcctgatgg acgaaaatac tacacaaata attttatcac 1740
   caatctaact tttcggacag ctagtctttg gattccagga acagctaagc ctgggcactg 1800
   gacttacacc ctgaacaata cccatcattc tctgcaagcc ctgaaagtga cagtgacctc 1860
   tcgcgcctcc aactcagctg tgcccccagc cactgtggaa gcctttgtgg aaagagacag 1920
   cctccatttt cctcatcctg tgatgattta tgccaatgtg aaacagggat tttatcccat 1980
   tcttaatgcc actgtcactg ccacagttga gccagagact ggagatcctg ttacgctgag 2040
   actccttgat gatggagcag gtgctgatgt tataaaaaat gatggaattt actcgaggta 2100
   ttttttctcc tttgctgcaa atggtagata tagcttgaaa gtgcatgtca atcactctcc 2160
   cagcataagc accccagccc actctattcc agggagtcat gctatgtatg taccaggtta 2220
   cacagcaaac ggtaatattc agatgaatgc tccaaggaaa tcagtaggca gaaatgagga 2280
   ggagcgaaag tggggcttta gccgagtcag ctcaggaggc tccttttcag tgctgggagt 2340
   tccagctggc ccccaccctg atgtgtttcc accatgcaaa attattgacc tggaagctgt 2400
   aaaagtagaa gaggaattga ccctatcttg gacagcacct ggagaagact ttgatcaggg 2460
   ccaggctaca agctatgaaa taagaatgag taaaagtcta cagaatatcc aagatgactt 2520
   taacaatgct attttagtaa atacatcaaa gcgaaatcct cagcaagctg gcatcaggga 2580
   gatatttacg ttctcacccc aaatttccac gaatggacct gaacatcagc caaatggaga 2640
   aacacatgaa agccacagaa tttatgttgc aatacgagca atggatagga actccttaca 2700
   gtctgctgta tctaacattg cccaggcgcc tctgtttatt ccccccaatt ctgatcctgt 2760
   acctgccaga gattatctta tattgaaagg agttttaaca gcaatgggtt tgataggaat 2820
   catttgcctt attatagttg tgacacatca tactttaagc aggaaaaaga gagcagacaa 2880
   gaaagagaat ggaacaaaat tattataaat aaatatccaa agtgtcttcc ttcttagata 2940
   taagacccat ggccttcgac tacaaaaaca tactaacaaa gtcaaattaa catcaaaact 3000
   gtattaaaat gcattgagtt tttgtacaat acagataaga tttttacatg gtagatcaac 3060
   aaattctttt tgggggtaga ttagaaaacc cttacacttt ggctatgaac aaataataaa 3120
   aattattctt taaagtaatg tctttaaagg caaagggaag ggtaaagtcg gaccagtgtc 3180
   aaggaaagtt tgttttattg aggtggaaaa atagccccaa gcagagaaaa ggagggtagg 3240
   tctgcattat aactgtctgt gtgaagcaat catttagtta ctttgattaa tttttctttt 3300
   ctccttatct gtgcagaaca ggttgcttgt ttacaactga agatcatgct atatttcata 3360
   tatgaagccc ctaatgcaaa gctctttacc tcttgctatt ttgttatata tattacagat 3420
   gaaatctcac tgctaatgct cagagatctt ttttcactgt aagaggtaac ctttaacaat 3480
   atgggtatta cctttgtctc ttcataccgg ttttatgaca aaggtctatt gaatttattt 3540
   gtttgtaagt ttctactccc atcaaagcag ctttttaagt tattgccttg gttattatgg 3600
   atgatagtta tagcccttat aatgccttaa ctaaggaaga aaagatgtta ttctgagttt 3660
   gttttaatac atatatgaac atatagtttt attcaattaa accaaagaag aggtcagcag 3720
   ggagatacta acctttggaa atgattagct ggctctgttt tttggttaaa taagagtctt 3780
   taatcctttc tccatcaaga gttacttacc aagggcaggg gaagggggat atagaggtcc 3840
   caaggaaata aaaatcatct ttcatcttta attttactcc ttcctcttat ttttttaaaa 3900
   gattatcgaa caataaaatc atttgccttt ttaattaaaa acataaaaaa a          3951
<210> 161
   <211> 943
   <212> PRT
   <213> Homo sapiens
<400> 161
<210> 162
   <211> 498
   <212> DNA
   <213> Homo sapiens
<400> 162
   tggagaacca cgtggacagc accatgaaca tgttgggcgg gggaggcagt gctggccgga 60
   agcccctcaa gtcgggtatg aaggagctgg ccgtgttccg ggagaaggtc actgagcagc 120
   accggcagat gggcaagggt ggcaagcatc accttggcct ggaggagccc aagaagctgc 180
   gaccaccccc tgccaggact ccctgccaac aggaactgga ccaggtcctg gagcggatct 240
   ccaccatgcg ccttccggat gagcggggcc ctctggagca cctctactcc ctgcacatcc 300
   ccaactgtga caagcatggc ctgtacaacc tcaaacagtg gcaagatgtc tctgaacggg 360
   cagcgtgggg agtgctggtg tgtgaacccc aacaccggga agctgatcca gggagccccc 420
   accatccggg gggaccccga gtgtcatctc ttctacaatg agcagcagga ggctcgcggg 480
   gtgcacaccc cagcggat                                               498
<210> 163
   <211> 1128
   <212> DNA
   <213> Homo sapiens
<400> 163
   gccacctggc cctcctgatc gacgacacac gcacttgaaa cttgttctca gggtgtgtgg 60
   aatcaacttt ccggaagcaa ccagcccacc agaggaggtc ccgagcgcga gcggagacga 120
   tgcagcggag actggttcag cagtggagcg tcgcggtgtt cctgctgagc tacgcggtgc 180
   cctcctgcgg gcgctcggtg gagggtctca gccgccgcct caaaagagct gtgtctgaac 240
   atcagctcct ccatgacaag gggaagtcca tccaagattt acggcgacga ttcttccttc 300
   accatctgat cgcagaaatc cacacagctg aaatcagagc tacctcggag gtgtccccta 360
   actccaagcc ctctcccaac acaaagaacc accccgtccg atttgggtct gatgatgagg 420
   gcagatacct aactcaggaa actaacaagg tggagacgta caaagagcag ccgctcaaga 480
   cacctgggaa gaaaaagaaa ggcaagcccg ggaaacgcaa ggagcaggaa aagaaaaaac 540
   ggcgaactcg ctctgcctgg ttagactctg gagtgactgg gagtgggcta gaaggggacc 600
   acctgtctga cacctccaca acgtcgctgg agctcgattc acggaggcat tgaaattttc 660
   agcagagacc ttccaaggac atattgcagg attctgtaat agtgaacata tggaaagtat 720
   tagaaatatt tattgtctgt aaatactgta aatgcattgg aataaaactg tctcccccat 780
   tgctctatga aactgcacat tggtcattgt gaatattttt ttttttgcca aggctaatcc 840
   aattattatt atcacattta ccataattta ttttgtccat tgatgtattt attttgtaaa 900
   tgtatcttgg tgctgctgaa tttctatatt ttttgtaaca taatgcactt tagatataca 960
   tatcaagtat gttgataaat gacacaatga agtgtctcta ttttgtggtt gattttaatg 1020
   aatgcctaaa tataattatc caaattgatt ttcctttgtg catgtaaaaa taacagtatt 1080
   ttaaatttgt aaagaatgtc taataaaata taatctaatt acatcatg             1128
<210> 164
   <211> 1310
   <212> DNA
   <213> Homo sapiens
<400> 164
   gggcctggtt cgcaaagaag ctgacttcag agggggaaac tttcttcttt taggaggcgg 60
   ttagccctgt tccacgaacc caggagaact gctggccaga ttaattagac attgctatgg 120
   gagacgtgta aacacactac ttatcattga tgcatatata aaaccatttt attttcgcta 180
   ttatttcaga ggaagcgcct ctgatttgtt tcttttttcc ctttttgctc tttctggctg 240
   tgtggtttgg agaaagcaca gttggagtag ccggttgcta aataagtccc gagcgcgagc 300
   ggagacgatg cagcggagac tggttcagca gtggagcgtc gcggtgttcc tgctgagcta 360
   cgcggtgccc tcctgcgggc gctcggtgga gggtctcagc cgccgcctca aaagagctgt 420
   gtctgaacat cagctcctcc atgacaaggg gaagtccatc caagatttac ggcgacgatt 480
   cttccttcac catctgatcg cagaaatcca cacagctgaa atcagagcta cctcggaggt 540
   gtcccctaac tccaagccct ctcccaacac aaagaaccac cccgtccgat ttgggtctga 600
   tgatgagggc agatacctaa ctcaggaaac taacaaggtg gagacgtaca aagagcagcc 660
   gctcaagaca cctgggaaga aaaagaaagg caagcccggg aaacgcaagg agcaggaaaa 720
   gaaaaaacgg cgaactcgct ctgcctggtt agactctgga gtgactggga gtgggctaga 780
   aggggaccac ctgtctgaca cctccacaac gtcgctggag ctcgattcac ggaggcattg 840
   aaattttcag cagagacctt ccaaggacat attgcaggat tctgtaatag tgaacatatg 900
   gaaagtatta gaaatattta ttgtctgtaa atactgtaaa tgcattggaa taaaactgtc 960
   tcccccattg ctctatgaaa ctgcacattg gtcattgtga atattttttt ttttgccaag 1020
   gctaatccaa ttattattat cacatttacc ataatttatt ttgtccattg atgtatttat 1080
   tttgtaaatg tatcttggtg ctgctgaatt tctatatttt ttgtaacata atgcacttta 1140
   gatatacata tcaagtatgt tgataaatga cacaatgaag tgtctctatt ttgtggttga 1200
   ttttaatgaa tgcctaaata taattatcca aattgatttt cctttgtgcc cgtaaaaata 1260
   acagtatttt aaatttgtaa agaatgtcta ataaaatata atctaattac          1310
<210> 165
   <211> 177
   <212> PRT
   <213> Homo sapiens
<400> 165
<210> 166
   <211> 177
   <212> PRT
   <213> Homo sapiens
<400> 166
<210> 167
   <211> 3362
   <212> DNA
   <213> Homo sapiens
<400> 167
   cacaatgtat gcagcaggct cagtgtgagt gaactggagg cttctctaca acatgaccca 60
   aaggagcatt gcaggtccta tttgcaacct gaagtttgtg actctcctgg ttgccttaag 120
   ttcagaactc ccattcctgg gagctggagt acagcttcaa gacaatgggt ataatggatt 180
   gctcattgca attaatcctc aggtacctga gaatcagaac ctcatctcaa acattaagga 240
   aatgataact gaagcttcat tttacctatt taatgctacc aagagaagag tatttttcag 300
   aaatataaag attttaatac ctgccacatg gaaagctaat aataacagca aaataaaaca 360
   agaatcatat gaaaaggcaa atgtcatagt gactgactgg tatggggcac atggagatga 420
   tccatacacc ctacaataca gagggtgtgg aaaagaggga aaatacattc atttcacacc 480
   taatttccta ctgaatgata acttaacagc tggctacgga tcacgaggcc gagtgtttgt 540
   ccatgaatgg gcccacctcc gttggggtgt gttcgatgag tataacaatg acaaaccttt 600
   ctacataaat gggcaaaatc aaattaaagt gacaaggtgt tcatctgaca tcacaggcat 660
   ttttgtgtgt gaaaaaggtc cttgccccca agaaaactgt attattagta agctttttaa 720
   agaaggatgc acctttatct acaatagcac ccaaaatgca actgcatcaa taatgttcat 780
   gcaaagttta tcttctgtgg ttgaattttg taatgcaagt acccacaacc aagaagcacc 840
   aaacctacag aaccagatgt gcagcctcag aagtgcatgg gatgtaatca cagactctgc 900
   tgactttcac cacagctttc ccatgaacgg gactgagctt ccacctcctc ccacattctc 960
   gcttgtagag gctggtgaca aagtggtctg tttagtgctg gatgtgtcca gcaagatggc 1020
   agaggctgac agactccttc aactacaaca agccgcagaa ttttatttga tgcagattgt 1080
   tgaaattcat accttcgtgg gcattgccag tttcgacagc aaaggagaga tcagagccca 1140
   gctacaccaa attaacagca atgatgatcg aaagttgctg gtttcatatc tgcccaccac 1200
   tgtatcagct aaaacagaca tcagcatttg ttcagggctt aagaaaggat ttgaggtggt 1260
   tgaaaaactg aatggaaaag cttatggctc tgtgatgata ttagtgacca gcggagatga 1320
   taagcttctt ggcaattgct tacccactgt gctcagcagt ggttcaacaa ttcactccat 1380
   tgccctgggt tcatctgcag ccccaaatct ggaggaatta tcacgtctta caggaggttt 1440
   aaagttcttt gttccagata tatcaaactc caatagcatg attgatgctt tcagtagaat 1500
   ttcctctgga actggagaca ttttccagca acatattcag cttgaaagta caggtgaaaa 1560
   tgtcaaacct caccatcaat tgaaaaacac agtgactgtg gataatactg tgggcaacga 1620
   cactatgttt ctagttacgt ggcaggccag tggtcctcct gagattatat tatttgatcc 1680
   tgatggacga aaatactaca caaataattt tatcaccaat ctaacttttc ggacagctag 1740
   tctttggatt ccaggaacag ctaagcctgg gcactggact tacaccctga tgtgtttcca 1800
   ccatgcaaaa ttattgacct ggaagctgta aaagtagaag aggaattgac cctatcttgg 1860
   acagcacctg gagaagactt tgatcagggc caggctacaa gctatgaaat aagaatgagt 1920
   aaaagtctac agaatatcca agatgacttt aacaatgcta ttttagtaaa tacatcaaag 1980
   cgaaatcctc agcaagctgg catcagggag atatttacgt tctcacccca aatttccacg 2040
   aatggacctg aacatcagcc aaatggagaa acacatgaaa gccacagaat ttatgttgca 2100
   atacgagcaa tggataggaa ctccttacag tctgctgtat ctaacattgc ccaggcgcct 2160
   ctgtttattc cccccaattc tgatcctgta cctgccagag attatcttat attgaaagga 2220
   gttttaacag caatgggttt gataggaatc atttgcctta ttatagttgt gacacatcat 2280
   actttaagca ggaaaaagag agcagacaag aaagagaatg gaacaaaatt attataaata 2340
   aatatccaaa gtgtcttcct tcttagatat aagacccatg gccttcgact acaaaaacat 2400
   actaacaaag tcaaattaac atcaaaactg tattaaaatg cattgagttt ttgtacaata 2460
   cagataagat ttttacatgg tagatcaaca aattcttttt gggggtagat tagaaaaccc 2520
   ttacactttg gctatgaaca aataataaaa attattcttt aaagtaatgt ctttaaaggc 2580
   aaagggaagg gtaaagtcgg accagtgtca aggaaagttt gttttattga ggtggaaaaa 2640
   tagccccaag cagagaaaag gagggtaggt ctgcattata actgtctgtg tgaagcaatc 2700
   atttagttac tttgattaat ttttcttttc tccttatctg tgcagaacag gttgcttgtt 2760
   tacaactgaa gatcatgcta tatttcatat atgaagcccc taatgcaaag ctctttacct 2820
   cttgctattt tgttatatat attacagatg aaatctcact gctaatgctc agagatcttt 2880
   tttcactgta agaggtaacc tttaacaata tgggtattac ctttgtctct tcataccggt 2940
   tttatgacaa aggtctattg aatttatttg tttgtaagtt tctactccca tcaaagcagc 3000
   tttctaagtt attgccttgg ttattatgga tgatagttat agcccttata atgccttaac 3060
   taaggaagaa aagatgttat tctgagtttg ttttaataca tatatgaaca tatagtttta 3120
   ttcaattaaa ccaaagaaga ggtcagcagg gagatactaa cctttggaaa tgattagctg 3180
   gctctgtttt ttggttaaat aagagtcttt aatcctttct ccatcaagag ttacttacca 3240
   agggcagggg aagggggata tagaggtcac aaggaaataa aaatcatctt tcatctttaa 3300
   ttttactcct tcctcttatt tttttaaaag attatcgaac aataaaatca tttgcctttt 3360
   tt                                                                3362
<210> 168
   <211> 2784
   <212> DNA
   <213> Homo sapiens
<400> 168
   tctgcatcca tattgaaaac ctgacacaat gtatgcagca ggctcagtgt gagtgaactg 60
   gaggcttctc tacaacatga cccaaaggag cattgcaggt cctatttgca acctgaagtt 120
   tgtgactctc ctggttgcct taagttcaga actcccattc ctgggagctg gagtacagct 180
   tcaagacaat gggtataatg gattgctcat tgcaattaat cctcaggtac ctgagaatca 240
   gaacctcatc tcaaacatta aggaaatgat aactgaagct tcattttacc tatttaatgc 300
   taccaagaga agagtatttt tcagaaatat aaagatttta atacctgcca catggaaagc 360
   taataataac agcaaaataa aacaagaatc atatgaaaag gcaaatgtca tagtgactga 420
   ctggtatggg gcacatggag atgatccata caccctacaa tacagagggt gtggaaaaga 480
   gggaaaatac attcatttca cacctaattt cctactgaat gataacttaa cagctggcta 540
   cggatcacga ggccgagtgt ttgtccatga atgggcccac ctccgttggg gtgtgttcga 600
   tgagtataac aatgacaaac ctttctacat aaatgggcaa aatcaaatta aagtgacaag 660
   gtgttcatct gacatcacag gcatttttgt gtgtgaaaaa ggtccttgcc cccaagaaaa 720
   ctgtattatt agtaagcttt ttaaagaagg atgcaccttt atctacaata gcacccaaaa 780
   tgcaactgca tcaataatgt tcatgcaaag tttatcttct gtggttgaat tttgtaatgc 840
   aagtacccac aaccaagaag caccaaacct acagaaccag atgtgcagcc tcagaagtgc 900
   atgggatgta atcacagact ctgctgactt tcaccacagc tttcccatga acgggactga 960
   gcttccacct cctcccacat tctcgcttgt agaggctggt gacaaagtgg tctgtttagt 1020
   gctggatgtg tccagcaaga tggcagaggc tgacagactc cttcaactac aacaagccgc 1080
   agaattttat ttgatgcaga ttgttgaaat tcataccttc gtgggcattg ccagtttcga 1140
   cagcaaagga gagatcagag cccagctaca ccaaattaac agcaatgatg atcgaaagtt 1200
   gctggtttca tatctgccca ccactgtatc agctaaaaca gacatcagca tttgttcagg 1260
   gcttaagaaa ggatttgagg tggttgaaaa actgaatgga aaagcttatg gctctgtgat 1320
   gatattagtg accagcggag atgataagct tcttggcaat tgcttaccca ctgtgctcag 1380
   cagtggttca acaattcact ccattgccct gggttcatct gcagccccaa atctggagga 1440
   attatcacgt cttacaggag gtttaaagtt ctttgttcca gatatatcaa actccaatag 1500
   catgattgat gctttcagta gaatttcctc tggaactgga gacattttcc agcaacatat 1560
   tcagcttgaa agtacaggtg aaaatgtcaa acctcaccat caattgaaaa acacagtgac 1620
   tgtggataat actgtgggca acgacactat gtttctagtt acgtggcagg ccagtggtcc 1680
   tcctgagatt atattatttg atcctgatgg acgaaaatac tacacaaata attttatcac 1740
   caatctaact tttcggacag ctagtctttg gattccagga acagctaagc ctgggcactg 1800
   gacttacacc ctgaacaata cccatcattc tctgcaagcc ctgaaagtga cagtgacctc 1860
   tcgcgcctcc aactcagctg tgcccccagc cactgtggaa gcctttgtgg aaagagacag 1920
   cctccatttt cctcatcctg tgatgattta tgccaatgtg aaacagggat tttatcccat 1980
   tcttaatgcc actgtcactg ccacagttga gccagagact ggagatcctg ttacgctgag 2040
   actccttgat gatggagcag gtgctgatgt tataaaaaat gatggaattt actcgaggta 2100
   ttttttctcc tttgctgcaa atggtagata tagcttgaaa gtgcatgtca atcactctcc 2160
   cagcataagc accccagccc actctattcc agggagtcat gctatgtatg taccaggtta 2220
   cacagcaaac ggtaatattc agatgaatgc tccaaggaaa tcagtaggca gaaatgagga 2280
   ggagcgaaag tggggcttta gccgagtcag ctcaggaggc tccttttcag tgctgggagt 2340
   tccagctggc ccccaccctg atgtgtttcc accatgcaaa attattgacc tggaagctgt 2400
   aaatagaaga ggaattgacc ctatcttgga cagcacctgg agaagacttt gatcagggcc 2460
   aggctacaag ctatgaaata agaatgagta aaagtctaca gaatatccaa gatgacttta 2520
   acaatgctat tttagtaaat acatcaaagc gaaatcctca gcaagctggc atcagggaga 2580
   tatttacgtt ctcaccccaa atttccacga atggacctga acatcagcca aatggagaaa 2640
   cacatgaaag ccacagaatt tatgttgcaa tacgagcaat ggataggaac tccttacagt 2700
   ctgctgtatc taacattgcc caggcgcctc tgtttattcc ccccaattct gatcctgtac 2760
   ctgccagaga ttatcttata ttga                                        2784
<210> 169
   <211> 592
   <212> PRT
   <213> Homo sapiens
<400> 169
   Met Thr Gln Arg Ser Ile Ala Gly Pro Ile Cys Asn Leu Lys Phe Val
    1               5                  10                  15
   Thr Leu Leu Val Ala Leu Ser Ser Glu Leu Pro Phe Leu Gly Ala Gly
               20                  25                  30
   Val Gln Leu Gln Asp Asn Gly Tyr Asn Gly Leu Leu Ile Ala Ile Asn
           35                  40                  45
   Pro Gln Val Pro Glu Asn Gln Asn Leu Ile Ser Asn Ile Lys Glu Met
       50                  55                  60
   Ile Thr Glu Ala Ser Phe Tyr Leu Phe Asn Ala Thr Lys Arg Arg Val
   65                  70                  75                  80
   Phe Phe Arg Asn Ile Lys Ile Leu Ile Pro Ala Thr Trp Lys Ala Asn
                   85                  90                  95
   Asn Asn Ser Lys Ile Lys Gln Glu Ser Tyr Glu Lys Ala Asn Val Ile
               100                 105                 110
   Val Thr Asp Trp Tyr Gly Ala His Gly Asp Asp Pro Tyr Thr Leu Gln
           115                 120                 125
   Tyr Arg Gly Cys Gly Lys Glu Gly Lys Tyr Ile His Phe Thr Pro Asn
       130                 135                 140
   Phe Leu Leu Asn Asp Asn Leu Thr Ala Gly Tyr Gly Ser Arg Gly Arg
   145                 150                 155                 160
   Val Phe Val His Glu Trp Ala His Leu Arg Trp Gly Val Phe Asp Glu
                   165                 170                 175
   Tyr Asn Asn Asp Lys Pro Phe Tyr Ile Asn Gly Gln Asn Gln Ile Lys
               180                 185                 190
   Val Thr Arg Cys Ser Ser Asp Ile Thr Gly Ile Phe Val Cys Glu Lys
           195                 200                 205
   Gly Pro Cys Pro Gln Glu Asn Cys Ile Ile Ser Lys Leu Phe Lys Glu
       210                 215                 220
   Gly Cys Thr Phe Ile Tyr Asn Ser Thr Gln Asn Ala Thr Ala Ser Ile
   225                 230                 235                 240
   Met Phe Met Gln Ser Leu Ser Ser Val Val Glu Phe Cys Asn Ala Ser
                   245                 250                 255
   Thr His Asn Gln Glu Ala Pro Asn Leu Gln Asn Gln Met Cys Ser Leu
               260                 265                 270
   Arg Ser Ala Trp Asp Val Ile Thr Asp Ser Ala Asp Phe His His Ser
           275                 280                 285
   Phe Pro Met Asn Gly Thr Glu Leu Pro Pro Pro Pro Thr Phe Ser Leu
       290                 295                 300
   Val Glu Ala Gly Asp Lys Val Val Cys Leu Val Leu Asp Val Ser Ser
   305                 310                 315                 320
   Lys Met Ala Glu Ala Asp Arg Leu Leu Gln Leu Gln Gln Ala Ala Glu
                   325                 330                 335
   Phe Tyr Leu Met Gln Ile Val Glu Ile His Thr Phe Val Gly Ile Ala
               340                 345                 350
   Ser Phe Asp Ser Lys Gly Glu Ile Arg Ala Gln Leu His Gln Ile Asn
           355                 360                 365
   Ser Asn Asp Asp Arg Lys Leu Leu Val Ser Tyr Leu Pro Thr Thr Val
       370                 375                 380
   Ser Ala Lys Thr Asp Ile Ser Ile Cys Ser Gly Leu Lys Lys Gly Phe
   385                 390                 395                 400
   Glu Val Val Glu Lys Leu Asn Gly Lys Ala Tyr Gly Ser Val Met Ile
                   405                 410                 415
   Leu Val Thr Ser Gly Asp Asp Lys Leu Leu Gly Asn Cys Leu Pro Thr
               420                 425                 430
   Val Leu Ser Ser Gly Ser Thr Ile His Ser Ile Ala Leu Gly Ser Ser
           435                 440                 445
   Ala Ala Pro Asn Leu Glu Glu Leu Ser Arg Leu Thr Gly Gly Leu Lys
       450                 455                 460
   Phe Phe Val Pro Asp Ile Ser Asn Ser Asn Ser Met Ile Asp Ala Phe
   465                 470                 475                 480
   Ser Arg Ile Ser Ser Gly Thr Gly Asp Ile Phe Gln Gln His Ile Gln
                   485                 490                 495
   Leu Glu Ser Thr Gly Glu Asn Val Lys Pro His His Gln Leu Lys Asn
               500                 505                 510
   Thr Val Thr Val Asp Asn Thr Val Gly Asn Asp Thr Met Phe Leu Val
           515                 520                 525
   Thr Trp Gln Ala Ser Gly Pro Pro Glu Ile Ile Leu Phe Asp Pro Asp
       530                     535                     540
   Gly Arg Lys Tyr Tyr Thr Asn Asn Phe Ile Thr Asn Leu Thr Phe Arg
   545                     550                     555                     560
   Thr Ala Ser Leu Trp Ile Pro Gly Thr Ala Lys Pro Gly His Trp Thr
                   565                     570                     575
   Tyr Thr Leu Met Cys Phe His His Ala Lys Leu Leu Thr Trp Lys Leu
               580                     585                     590
<210> 170
   <211> 791
   <212> PRT
   <213> Homo sapiens
<400> 170
   Met Thr Gln Arg Ser Ile Ala Gly Pro Ile Cys Asn Leu Lys Phe Val
    1               5                  10                  15
   Thr Leu Leu Val Ala Leu Ser Ser Glu Leu Pro Phe Leu Gly Ala Gly
               20                  25                  30
   Val Gln Leu Gln Asp Asn Gly Tyr Asn Gly Leu Leu Ile Ala Ile Asn
           35                  40                  45
   Pro Gln Val Pro Glu Asn Gln Asn Leu Ile Ser Asn Ile Lys Glu Met
       50                  55                  60
   Ile Thr Glu Ala Ser Phe Tyr Leu Phe Asn Ala Thr Lys Arg Arg Val
   65                  70                  75                  80
   Phe Phe Arg Asn Ile Lys Ile Leu Ile Pro Ala Thr Trp Lys Ala Asn
                   85                  90                  95
   Asn Asn Ser Lys Ile Lys Gln Glu Ser Tyr Glu Lys Ala Asn Val Ile
               100                 105                 110
   Val Thr Asp Trp Tyr Gly Ala His Gly Asp Asp Pro Tyr Thr Leu Gln
           115                 120                 125
   Tyr Arg Gly Cys Gly Lys Glu Gly Lys Tyr Ile His Phe Thr Pro Asn
       130                 135                 140
   Phe Leu Leu Asn Asp Asn Leu Thr Ala Gly Tyr Gly Ser Arg Gly Arg
   145                 150                 155                 160
   Val Phe Val His Glu Trp Ala His Leu Arg Trp Gly Val Phe Asp Glu
                   165                 170                 175
   Tyr Asn Asn Asp Lys Pro Phe Tyr Ile Asn Gly Gln Asn Gln Ile Lys
               180                 185                 190
   Val Thr Arg Cys Ser Ser Asp Ile Thr Gly Ile Phe Val Cys Glu Lys
           195                 200                 205
   Gly Pro Cys Pro Gln Glu Asn Cys Ile Ile Ser Lys Leu Phe Lys Glu
       210                 215                 220
   Gly Cys Thr Phe Ile Tyr Asn Ser Thr Gln Asn Ala Thr Ala Ser Ile
   225                 230                 235                 240
   Met Phe Met Gln Ser Leu Ser Ser Val Val Glu Phe Cys Asn Ala Ser
                   245                 250                 255
   Thr His Asn Gln Glu Ala Pro Asn Leu Gln Asn Gln Met Cys Ser Leu
               260                 265                 270
   Arg Ser Ala Trp Asp Val Ile Thr Asp Ser Ala Asp Phe His His Ser
           275                 280                 285
   Phe Pro Met Asn Gly Thr Glu Leu Pro Pro Pro Pro Thr Phe Ser Leu
       290                 295                 300
   Val Glu Ala Gly Asp Lys Val Val Cys Leu Val Leu Asp Val Ser Ser
   305                 310                 315                 320
   Lys Met Ala Glu Ala Asp Arg Leu Leu Gln Leu Gln Gln Ala Ala Glu
                   325                 330                 335
   Phe Tyr Leu Met Gln Ile Val Glu Ile His Thr Phe Val Gly Ile Ala
                   340                 345                 350
   Ser Phe Asp Ser Lys Gly Glu Ile Arg Ala Gln Leu His Gln Ile Asn
               355                 360                 365
   Ser Asn Asp Asp Arg Lys Leu Leu Val Ser Tyr Leu Pro Thr Thr Val
           370                 375                 380
   Ser Ala Lys Thr Asp Ile Ser Ile Cys Ser Gly Leu Lys Lys Gly Phe
   385                 390                 395                 400
   Glu Val Val Glu Lys Leu Asn Gly Lys Ala Tyr Gly Ser Val Met Ile
                   405                 410                 415
   Leu Val Thr Ser Gly Asp Asp Lys Leu Leu Gly Asn Cys Leu Pro Thr
               420                 425                 430
   Val Leu Ser Ser Gly Ser Thr Ile His Ser Ile Ala Leu Gly Ser Ser
           435                 440                 445
   Ala Ala Pro Asn Leu Glu Glu Leu Ser Arg Leu Thr Gly Gly Leu Lys
       450                 455                 460
   Phe Phe Val Pro Asp Ile Ser Asn Ser Asn Ser Met Ile Asp Ala Phe
   465                 470                 475                 480
   Ser Arg Ile Ser Ser Gly Thr Gly Asp Ile Phe Gln Gln His Ile Gln
                   485                 490                 495
   Leu Glu Ser Thr Gly Glu Asn Val Lys Pro His His Gln Leu Lys Asn
               500                 505                 510
   Thr Val Thr Val Asp Asn Thr Val Gly Asn Asp Thr Met Phe Leu Val
           515                 520                 525
   Thr Trp Gln Ala Ser Gly Pro Pro Glu Ile Ile Leu Phe Asp Pro Asp
       530                 535                 540
   Gly Arg Lys Tyr Tyr Thr Asn Asn Phe Ile Thr Asn Leu Thr Phe Arg
   545                 550                 555                 560
   Thr Ala Ser Leu Trp Ile Pro Gly Thr Ala Lys Pro Gly His Trp Thr
                   565                 570                 575
   Tyr Thr Leu Asn Asn Thr His His Ser Leu Gln Ala Leu Lys Val Thr
               580                 585                 590
   Val Thr Ser Arg Ala Ser Asn Ser Ala Val Pro Pro Ala Thr Val Glu
           595                 600                 605
   Ala Phe Val Glu Arg Asp Ser Leu His Phe Pro His Pro Val Met Ile
       610                 615                 620
   Tyr Ala Asn Val Lys Gln Gly Phe Tyr Pro Ile Leu Asn Ala Thr Val
   625                 630                 635                 640
   Thr Ala Thr Val Glu Pro Glu Thr Gly Asp Pro Val Thr Leu Arg Leu
                   645                 650                 655
   Leu Asp Asp Gly Ala Gly Ala Asp Val Ile Lys Asn Asp Gly Ile Tyr
               660                 665                 670
   Ser Arg Tyr Phe Phe Ser Phe Ala Ala Asn Gly Arg Tyr Ser Leu Lys
           675                 680                 685
   Val His Val Asn His Ser Pro Ser Ile Ser Thr Pro Ala His Ser Ile
       690                 695                 700
   Pro Gly Ser His Ala Met Tyr Val Pro Gly Tyr Thr Ala Asn Gly Asn
   705                 710                 715                 720
   Ile Gln Met Asn Ala Pro Arg Lys Ser Val Gly Arg Asn Glu Glu Glu
                   725                 730                 735
   Arg Lys Trp Gly Phe Ser Arg Val Ser Ser Gly Gly Ser Phe Ser Val
               740                 745                 750
   Leu Gly Val Pro Ala Gly Pro His Pro Asp Val Phe Pro Pro Cys Lys
           755                 760                 765
   Ile Ile Asp Leu Glu Ala Val Asn Arg Arg Gly Ile Asp Pro Ile Leu
       770                 775                 780
   Asp Ser Thr Trp Arg Arg Leu
   785                 790
<210> 171
   <211> 1491
   <212> DNA
   <213> Homo sapiens
<400> 171
   cctcctgcca gccaagtgaa gacatgctta cttccccttc accttccttc atgatgtggg 60
   aagagtgctg caacccagcc ctagccaacg ccgcatgaga gggagtgtgc cgagggcttc 120
   tgagaaggtt tctctcacat ctagaaagaa gcgcttaaga tgtggcagcc cctcttcttc 180
   aagtggctct tgtcctgttg ccctgggagt tctcaaattg ctgcagcagc ctccacccag 240
   cctgaggatg acatcaatac acagaggaag aagagtcagg aaaagatgag agaagttaca 300
   gactctcctg ggcgaccccg agagcttacc attcctcaga cttcttcaca tggtgctaac 360
   agatttgttc ctaaaagtaa agctctagag gccgtcaaat tggcaataga agccgggttc 420
   caccatattg attctgcaca tgtttacaat aatgaggagc aggttggact ggccatccga 480
   agcaagattg cagatggcag tgtgaagaga gaagacatat tctacacttc aaagctttgg 540
   agcaattccc atcgaccaga gttggtccga ccagccttgg aaaggtcact gaaaaatctt 600
   caattggact atgttgacct ctatcttatt cattttccag tgtctgtaaa gccaggtgag 660
   gaagtgatcc caaaagatga aaatggaaaa atactatttg acacagtgga tctctgtgcc 720
   acatgggagg ccatggagaa gtgtaaagat gcaggattgg ccaagtccat cggggtgtcc 780
   aacttcaacc acaggctgct ggagatgatc ctcaacaagc cagggctcaa gtacaagcct 840
   gtctgcaacc aggtggaatg tcatccttac ttcaaccaga gaaaactgct ggatttctgc 900
   aagtcaaaag acattgttct ggttgcctat agtgctctgg gatcccatcg agaagaacca 960
   tgggtggacc cgaactcccc ggtgctcttg gaggacccag tcctttgtgc cttggcaaaa 1020
   aagcacaagc gaaccccagc cctgattgcc ctgcgctacc agctgcagcg tggggttgtg 1080
   gtcctggcca agagctacaa tgagcagcgc atcagacaga acgtgcaggt gtttgaattc 1140
   cagttgactt cagaggagat gaaagccata gatggcctaa acagaaatgt gcgatatttg 1200
   acccttgata tttttgctgg cccccctaat tatccatttt ctgatgaata ttaacatgga 1260
   gggcattgca tgaggtctgc cagaaggccc tgcgtgtgga tggtgacaca gaggatggct 1320
   ctatgctggt gactggacac atcgcctctg gttaaatctc tcctgcttgg cgacttcagt 1380
   aagctacagc taagcccatc ggccggaaaa gaaagacaat aattttgttt ttcattttga 1440
   aaaaattaaa tgctctctcc taaagattct tcacctaaaa aaaaaaaaaa a          1491
<210> 172
   <211> 364
   <212> PRT
   <213> Homo sapiens
<400> 172
   Met Trp Gln Pro Leu Phe Phe Lys Trp Leu Leu Ser Cys Cys Pro Gly
   1               5                   10                  15
   Ser Ser Gln Ile Ala Ala Ala Ala Ser Thr Gln Pro Glu Asp Asp Ile
               20                  25                  30
   Asn Thr Gln Arg Lys Lys Ser Gln Glu Lys Met Arg Glu Val Thr Asp
           35                  40                  45
   Ser Pro Gly Arg Pro Arg Glu Leu Thr Ile Pro Gln Thr Ser Ser His
       50                  55                  60
   Gly Ala Asn Arg Phe Val Pro Lys Ser Lys Ala Leu Glu Ala Val Lys
   65                  70                  75                  80
   Leu Ala Ile Glu Ala Gly Phe His His Ile Asp Ser Ala His Val Tyr
                   85                  90                  95
   Asn Asn Glu Glu Gln Val Gly Leu Ala Ile Arg Ser Lys Ile Ala Asp
               100                 105                 110
   Gly Ser Val Lys Arg Glu Asp Ile Phe Tyr Thr Ser Lys Leu Trp Ser
           115                 120                 125
   Asn Ser His Arg Pro Glu Leu Val Arg Pro Ala Leu Glu Arg Ser Leu
       130                 135                 140
   Lys Asn Leu Gln Leu Asp Tyr Val Asp Leu Tyr Leu Ile His Phe Pro
   145                 150                 155                 160
   Val Ser Val Lys Pro Gly Glu Glu Val Ile Pro Lys Asp Glu Asn Gly
                   165                 170                 175
   Lys Ile Leu Phe Asp Thr Val Asp Leu Cys Ala Thr Trp Glu Ala Met
               180                 185                 190
   Glu Lys Cys Lys Asp Ala Gly Leu Ala Lys Ser Ile Gly Val Ser Asn
           195                 200                 205
   Phe Asn His Arg Leu Leu Glu Met Ile Leu Asn Lys Pro Gly Leu Lys
       210                 215                 220
   Tyr Lys Pro Val Cys Asn Gln Val Glu Cys His Pro Tyr Phe Asn Gln
   225                 230                 235                 240
   Arg Lys Leu Leu Asp Phe Cys Lys Ser Lys Asp Ile Val Leu Val Ala
                   245                 250                 255
   Tyr Ser Ala Leu Gly Ser His Arg Glu Glu Pro Trp Val Asp Pro Asn
               260                 265                 270
   Ser Pro Val Leu Leu Glu Asp Pro Val Leu Cys Ala Leu Ala Lys Lys
           275                 280                 285
   His Lys Arg Thr Pro Ala Leu Ile Ala Leu Arg Tyr Gln Leu Gln Arg
       290                 295                 300
   Gly Val Val Val Leu Ala Lys Ser Tyr Asn Glu Gln Arg Ile Arg Gln
   305                 310                 315                 320
   Asn Val Gln Val Phe Glu Phe Gln Leu Thr Ser Glu Glu Met Lys Ala
                   325                 330                 335
   Ile Asp Gly Leu Asn Arg Asn Val Arg Tyr Leu Thr Leu Asp Ile Phe
               340                 345                 350
   Ala Gly Pro Pro Asn Tyr Pro Phe Ser Asp Glu Tyr
           355                 360
<210> 173
   <211> 1988
   <212> DNA
   <213> Homo sapiens
<400> 173
   cgggagccgc ctccccgcgg cctcttcgct tttgtggcgg cgcccgcgct cgcaggccac 60
   tctctgctgt cgcccgtccc gcgcgctcct ccgacccgct ccgctccgct ccgctcggcc 120
   ccgcgccgcc cgtcaacatg atccgctgcg gcctggcctg cgagcgctgc cgctggatcc 180
   tgcccctgct cctactcagc gccatcgcct tcgacatcat cgcgctggcc ggccgcggct 240
   ggttgcagtc tagcgaccac ggccagacgt cctcgctgtg gtggaaatgc tcccaagagg 300
   gcggcggcag cgggtcctac gaggagggct gtcagagcct catggagtac gcgtggggta 360
   gagcagcggc tgccatgctc ttctgtggct tcatcatcct ggtgatctgt ttcatcctct 420
   ccttcttcgc cctctgtgga ccccagatgc ttgtcttcct gagagtgatt ggaggtctcc 480
   ttgccttggc tgctgtgttc cagatcatct ccctggtaat ttaccccgtg aagtacaccc 540
   agaccttcac ccttcatgcc aaccctgctg tcacttacat ctataactgg gcctacggct 600
   ttgggtgggc agccacgatt atcctgatcg gctgtgcctt cttcttctgc tgcctcccca 660
   actacgaaga tgaccttctg ggcaatgcca agcccaggta cttctacaca tctgcctaac 720
   ttgggaatga atgtgggaga aaatcgctgc tgctgagatg gactccagaa gaagaaactg 780
   tttctccagg cgactttgaa cccatttttt ggcagtgttc atattattaa actagtcaaa 840
   aatgctaaaa taatttggga gaaaatattt tttaagtagt gttatagttt catgtttatc 900
   ttttattatg ttttgtgaag ttgtgtcttt tcactaatta cctatactat gccaatattt 960
   ccttatatct atccataaca tttatactac atttgtaaga gaatatgcac gtgaaactta 1020
   acactttata aggtaaaaat gaggtttcca agatttaata atctgatcaa gttcttgtta 1080
   tttccaaata gaatggactt ggtctgttaa gggctaagga gaagaggaag ataaggttaa 1140
   aagttgttaa tgaccaaaca ttctaaaaga aatgcaaaaa aaaagtttat tttcaagcct 1200
   tcgaactatt taaggaaagc aaaatcattt cctaaatgca tatcatttgt gagaatttct 1260
   cattaatatc ctgaatcatt catttcagct aaggcttcat gttgactcga tatgtcatct 1320
   aggaaagtac tatttcatgg tccaaacctg ttgccatagt tggtaaggct ttcctttaag 1380
   tgtgaaatat ttagatgaaa ttttctcttt taaagttctt tatagggtta gggtgtggga 1440
   aaatgctata ttaataaatc tgtagtgttt tgtgtttata tgttcagaac cagagtagac 1500
   tggattgaaa gatggactgg gtctaattta tcatgactga tagatctggt taagttgtgt 1560
   agtaaagcat taggagggtc attcytgtca caaaagtgcc actaaaacag cctcaggaga 1620
   ataaatgact tgcttttcta aatctcaggt ttatctgggc tctatcatat agacaggctt 1680
   ctgatagttt gcarctgtaa gcagaaacct acatatagtt aaaatcctgg tctttcttgg 1740
   taaacagatt ttaaatgtct gatataaaac atgccacagg agaattcggg gatttgagtt 1800
   tctctgaata gcatatatat gatgcatcgg ataggtcatt atgatttttt accatttcga 1860
   cttacataat gaaaaccaat tcattttaaa tatcagatta ttattttgta agttgtggaa 1920
   aaagctaatt gtagttttca ttatgaagtt ttcccaataa accaggtatt ctaaaaaaaa 1980
   aaaaaaaa                                                          1988
<210> 174
   <211> 238
   <212> PRT
   <213> Homo sapiens
<400> 174
   Gly Ala Ala Ser Pro Arg Pro Leu Arg Phe Cys Gly Gly Ala Arg Ala
    1               5                  10                  15
   Arg Arg Pro Leu Ser Ala Val Ala Arg Pro Ala Arg Ser Ser Asp Pro
               20                  25                  30
   Leu Arg Ser Ala Pro Leu Gly Pro Ala Pro Pro Val Asn Met Ile Arg
           35                  40                  45
   Cys Gly Leu Ala Cys Glu Arg Cys Arg Trp Ile Leu Pro Leu Leu Leu
       50                  55                  60
   Leu Ser Ala Ile Ala Phe Asp Ile Ile Ala Leu Ala Gly Arg Gly Trp
   65                  70                  75                  80
   Leu Gln Ser Ser Asp His Gly Gln Thr Ser Ser Leu Trp Trp Lys Cys
                   85                  90                  95
   Ser Gln Glu Gly Gly Gly Ser Gly Ser Tyr Glu Glu Gly Cys Gln Ser
               100                 105                 110
   Leu Met Glu Tyr Ala Trp Gly Arg Ala Ala Ala Ala Met Leu Phe Cys
           115                 120                 125
   Gly Phe Ile Ile Leu Val Ile Cys Phe Ile Leu Ser Phe Phe Ala Leu
       130                 135                 140
   Cys Gly Pro Gln Met Leu Val Phe Leu Arg Val Ile Gly Gly Leu Leu
   145                 150                 155                 160
   Ala Leu Ala Ala Val Phe Gln Ile Ile Ser Leu Val Ile Tyr Pro Val
                   165                 170                 175
   Lys Tyr Thr Gln Thr Phe Thr Leu His Ala Asn Pro Ala Val Thr Tyr
               180                 185                 190
   Ile Tyr Asn Trp Ala Tyr Gly Phe Gly Trp Ala Ala Thr Ile Ile Leu
           195                 200                 205
   Ile Gly Cys Ala Phe Phe Phe Cys Cys Leu Pro Asn Tyr Glu Asp Asp
       210                 215                 220
   Leu Leu Gly Asn Ala Lys Pro Arg Tyr Phe Tyr Thr Ser Ala
   225                 230                 235
<210> 175
   <211> 4181
   <212> DNA
   <213> Homo sapiens
<220>
   <221> misc_feature
   <222> 3347, 3502, 3506, 3520, 3538, 3549, 3646, 3940, 3968, 3974, 4036, 4056, 4062, 4080, 4088, 4115
   <223> n = A,T,C or G
<400> 175
   ggtggatgcg tttgggttgt agctaggctt tttcttttct ttctctttta aaacacatct 60
   agacaaggaa aaaacaagcc tcggatctga tttttcactc ctcgttcttg tgcttggttc 120
   ttactgtgtt tgtgtatttt aaaggcgaga agacgagggg aacaaaacca gctggatcca 180
   tccatcaccg tgggtggttt taatttttcg ttttttctcg ttattttttt ttaaacaacc 240
   actcttcaca atgaacaaac tgtatatcgg aaacctcagc gagaacgccg ccccctcgga 300
   cctagaaagt atcttcaagg acgccaagat cccggtgtcg ggacccttcc tggtgaagac 360
   tggctacgcg ttcgtggact gcccggacga gagctgggcc ctcaaggcca tcgaggcgct 420
   ttcaggtaaa atagaactgc acgggaaacc catagaagtt gagcactcgg tcccaaaaag 480
   gcaaaggatt cggaaacttc agatacgaaa tatcccgcct catttacagt gggaggtgct 540
   ggatagttta ctagtccagt atggagtggt ggagagctgt gagcaagtga acactgactc 600
   ggaaactgca gttgtaaatg taacctattc cagtaaggac caagctagac aagcactaga 660
   caaactgaat ggatttcagt tagagaattt caccttgaaa gtagcctata tccctgatga 720
   aatggccgcc cagcaaaacc ccttgcagca gccccgaggt cgccgggggc ttgggcagag 780
   gggctcctca aggcaggggt ctccaggatc cgtatccaag cagaaaccat gtgatttgcc 840
   tctgcgcctg ctggttccca cccaatttgt tggagccatc ataggaaaag aaggtgccac 900
   cattcggaac atcaccaaac agacccagtc taaaatcgat gtccaccgta aagaaaatgc 960
   gggggctgct gagaagtcga ttactatcct ctctactcct gaaggcacct ctgcggcttg 1020
   taagtctatt ctggagatta tgcataagga agctcaagat ataaaattca cagaagagat 1080
   ccccttgaag attttagctc ataataactt tgttggacgt cttattggta aagaaggaag 1140
   aaatcttaaa aaaattgagc aagacacaga cactaaaatc acgatatctc cattgcagga 1200
   attgacgctg tataatccag aacgcactat tacagttaaa ggcaatgttg agacatgtgc 1260
   caaagctgag gaggagatca tgaagaaaat cagggagtct tatgaaaatg atattgcttc 1320
   tatgaatctt caagcacatt taattcctgg attaaatctg aacgccttgg gtctgttccc 1380
   acccacttca gggatgccac ctcccacctc agggccccct tcagccatga ctcctcccta 1440
   cccgcagttt gagcaatcag aaacggagac tgttcatcag tttatcccag ctctatcagt 1500
   cggtgccatc atcggcaagc agggccagca catcaagcag ctttctcgct ttgctggagc 1560
   ttcaattaag attgctccag cggaagcacc agatgctaaa gtgaggatgg tgattatcac 1620
   tggaccacca gaggctcagt tcaaggctca gggaagaatt tatggaaaaa ttaaagaaga 1680
   aaactttgtt agtcctaaag aagaggtgaa acttgaagct catatcagag tgccatcctt 1740
   tgctgctggc agagttattg gaaaaggagg caaaacggtg aatgaacttc agaatttgtc 1800
   aagtgcagaa gttgttgtcc ctcgtgacca gacacctgat gagaatgacc aagtggttgt 1860
   caaaataact ggtcacttct atgcttgcca ggttgcccag agaaaaattc aggaaattct 1920
   gactcaggta aagcagcacc aacaacagaa ggctctgcaa agtggaccac ctcagtcaag 1980
   acggaagtaa aggctcagga aacagcccac cacagaggca gatgccaaac caaagacaga 2040
   ttgcttaacc aacagatggg cgctgacccc ctatccagaa tcacatgcac aagtttttac 2100
   ctagccagtt gtttctgagg accaggcaac ttttgaactc ctgtctctgt gagaatgtat 2160
   actttatgct ctctgaaatg tatgacaccc agctttaaaa caaacaaaca aacaaacaaa 2220
   aaaagggtgg gggagggagg gaaagagaag agctctgcac ttccctttgt tgtagtctca 2280
   cagtataaca gatattctaa ttcttcttaa tattccccca taatgccaga aattggctta 2340
   atgatgcttt cactaaattc atcaaataga ttgctcctaa atccaattgt taaaattgga 2400
   tcagaataat tatcacagga acttaaatgt taagccatta gcatagaaaa actgttctca 2460
   gttttatttt tacctaacac taacatgagt aacctaaggg aagtgctgaa tggtgttggc 2520
   aggggtatta aacgtgcatt tttactcaac tacctcaggt attcagtaat acaatgaaaa 2580
   gcaaaattgt tccttttttt tgaaaatttt atatacttta taatgataga agtccaaccg 2640
   ttttttaaaa aataaattta aaatttaaca gcaatcagct aacaggcaaa ttaagatttt 2700
   tacttctggc tggtgacagt aaagctggaa aattaatttc agggtttttt gaggcttttg 2760
   acacagttat tagttaaatc aaatgttcaa aaatacggag cagtgcctag tatctggaga 2820
   gcagcactac catttattct ttcatttata gttgggaaag tttttgacgg tactaacaaa 2880
   gtggtcgcag gagattttgg aacggctggt ttaaatggct tcaggagact tcagtttttt 2940
   gtttagctac atgattgaat gcataataaa tgctttgtgc ttctgactat caatacctaa 3000
   agaaagtgca tcagtgaaga gatgcaagac tttcaactga ctggcaaaaa gcaagcttta 3060
   gcttgtctta taggatgctt agtttgccac tacacttcag accaatggga cagtcataga 3120
   tggtgtgaca gtgtttaaac gcaacaaaag gctacatttc catggggcca gcactgtcat 3180
   gagcctcact aagctatttt gaagattttt aagcactgat aaattaaaaa aaaaaaaaaa 3240
   aaattagact ccaccttaag tagtaaagta taacaggatt tctgtatact gtgcaatcag 3300
   ttctttgaaa aaaaagtcaa aagatagaga atacaagaaa agttttnggg atataatttg 3360
   aatgactgtg aaaacatatg acctttgata acgaactcat ttgctcactc cttgacagca 3420
   aagcccagta cgtacaattg tgttgggtgt gggtggtctc caaggccacg ctgctctctg 3480
   aattgatttt ttgagttttg gnttgnaaga tgatcacagn catgttacac tgatcttnaa 3540
   ggacatatnt tataaccctt taaaaaaaaa atcccctgcc tcattcttat ttcgagatga 3600
   atttcgatac agactagatg tctttctgaa gatcaattag acattntgaa aatgatttaa 3660
   agtgttttcc ttaatgttct ctgaaaacaa gtttcttttg tagttttaac caaaaaagtg 3720
   ccctttttgt cactggtttc tcctagcatt catgattttt ttttcacaca atgaattaaa 3780
   attgctaaaa tcatggactg gctttctggt tggatttcag gtaagatgtg tttaaggcca 3840
   gagcttttct cagtatttga tttttttccc caatatttga ttttttaaaa atatacacat 3900
   aggagctgca tttaaaacct gctggtttaa attctgtcan atttcacttc tagcctttta 3960
   gtatggcnaa tcanaattta cttttactta agcatttgta atttggagta tctggtacta 4020
   gctaagaaat aattcnataa ttgagttttg tactcnccaa anatgggtca ttcctcatgn 4080
   ataatgtncc cccaatgcag cttcattttc caganacctt gacgcaggat aaattttttc 4140
   atcatttagg tccccaaaaa aaaaaaaaaa aaaaaaaaaa a                     4181
<210> 176
   <211> 579
   <212> PRT
   <213> Homo sapiens
<400> 176
   Met Asn Lys Leu Tyr Ile Gly Asn Leu Ser Glu Asn Ala Ala Pro Ser
    1               5                  10                  15
   Asp Leu Glu Ser Ile Phe Lys Asp Ala Lys Ile Pro Val Ser Gly Pro
               20                  25                  30
   Phe Leu Val Lys Thr Gly Tyr Ala Phe Val Asp Cys Pro Asp Glu Ser
           35                  40                  45
   Trp Ala Leu Lys Ala Ile Glu Ala Leu Ser Gly Lys Ile Glu Leu His
       50                  55                  60
   Gly Lys Pro Ile Glu Val Glu His Ser Val Pro Lys Arg Gln Arg Ile
   65                  70                  75                  80
   Arg Lys Leu Gln Ile Arg Asn Ile Pro Pro His Leu Gln Trp Glu Val
                   85                  90                  95
   Leu Asp Ser Leu Leu Val Gln Tyr Gly Val Val Glu Ser Cys Glu Gln
               100                 105                 110
   Val Asn Thr Asp Ser Glu Thr Ala Val Val Asn Val Thr Tyr Ser Ser
           115                 120                 125
   Lys Asp Gln Ala Arg Gln Ala Leu Asp Lys Leu Asn Gly Phe Gln Leu
       130                 135                 140
   Glu Asn Phe Thr Leu Lys Val Ala Tyr Ile Pro Asp Glu Met Ala Ala
   145                 150                 155                 160
   Gln Gln Asn Pro Leu Gln Gln Pro Arg Gly Arg Arg Gly Leu Gly Gln
                   165                 170                 175
   Arg Gly Ser Ser Arg Gln Gly Ser Pro Gly Ser Val Ser Lys Gln Lys
               180                 185                 190
   Pro Cys Asp Leu Pro Leu Arg Leu Leu Val Pro Thr Gln Phe Val Gly
           195                 200                 205
   Ala Ile Ile Gly Lys Glu Gly Ala Thr Ile Arg Asn Ile Thr Lys Gln
       210                 215                 220
   Thr Gln Ser Lys Ile Asp Val His Arg Lys Glu Asn Ala Gly Ala Ala
   225                 230                 235                 240
   Glu Lys Ser Ile Thr Ile Leu Ser Thr Pro Glu Gly Thr Ser Ala Ala
                   245                 250                 255
   Cys Lys Ser Ile Leu Glu Ile Met His Lys Glu Ala Gln Asp Ile Lys
               260                 265                 270
   Phe Thr Glu Glu Ile Pro Leu Lys Ile Leu Ala His Asn Asn Phe Val
               275                 280                 285
   Gly Arg Leu Ile Gly Lys Glu Gly Arg Asn Leu Lys Lys Ile Glu Gln
       290                 295                 300
   Asp Thr Asp Thr Lys Ile Thr Ile Ser Pro Leu Gln Glu Leu Thr Leu
   305                 310                 315                 320
   Tyr Asn Pro Glu Arg Thr Ile Thr Val Lys Gly Asn Val Glu Thr Cys
                   325                 330                 335
   Ala Lys Ala Glu Glu Glu Ile Met Lys Lys Ile Arg Glu Ser Tyr Glu
               340                 345                 350
   Asn Asp Ile Ala Ser Met Asn Leu Gln Ala His Leu Ile Pro Gly Leu
           355                 360                 365
   Asn Leu Asn Ala Leu Gly Leu Phe Pro Pro Thr Ser Gly Met Pro Pro
       370                 375                 380
   Pro Thr Ser Gly Pro Pro Ser Ala Met Thr Pro Pro Tyr Pro Gln Phe
   385                 390                 395                 400
   Glu Gln Ser Glu Thr Glu Thr Val His Gln Phe Ile Pro Ala Leu Ser
                   405                 410                 415
   Val Gly Ala Ile Ile Gly Lys Gln Gly Gln His Ile Lys Gln Leu Ser
               420                 425                 430
   Arg Phe Ala Gly Ala Ser Ile Lys Ile Ala Pro Ala Glu Ala Pro Asp
           435                 440                 445
   Ala Lys Val Arg Met Val Ile Ile Thr Gly Pro Pro Glu Ala Gln Phe
       450                 455                 460
   Lys Ala Gln Gly Arg Ile Tyr Gly Lys Ile Lys Glu Glu Asn Phe Val
   465                 470                 475                 480
   Ser Pro Lys Glu Glu Val Lys Leu Glu Ala His Ile Arg Val Pro Ser
                   485                 490                 495
   Phe Ala Ala Gly Arg Val Ile Gly Lys Gly Gly Lys Thr Val Asn Glu
               500                 505                 510
   Leu Gln Asn Leu Ser Ser Ala Glu Val Val Val Pro Arg Asp Gln Thr
           515                 520                 525
   Pro Asp Glu Asn Asp Gln Val Val Val Lys Ile Thr Gly His Phe Tyr
       530                 535                 540
   Ala Cys Gln Val Ala Gln Arg Lys Ile Gln Glu Ile Leu Thr Gln Val
   545                 550                 555                 560
   Lys Gln His Gln Gln Gln Lys Ala Leu Gln Ser Gly Pro Pro Gln Ser
                   565                 570                 575
   Arg Arg Lys
<210> 177
   <211> 401
   <212> DNA
   <213> Homo sapiens
<400> 177
   atgccccgta aatgtcttca gtgttcttca gggtagttgg gatctcaaaa gatttggttc 60
   agatccaaac aaatacacat tctgtgtttt agctcagtgt tttctaaaaa aagaaactgc 120
   cacacagcaa aaaattgttt actttgttgg acaaaccaaa tcagttctca aaaaatgacc 180
   ggtgcttata aaaagttata aatatcgagt agctctaaaa caaaccacct gaccaagagg 240
   gaagtgagct tgtgcttagt atttacattg gatgccagtt ttgtaatcac tgacttatgt 300
   gcaaactggt gcagaaattc tataaactct ttgctgtttt tgatacctgc tttttgtttc 360
   attttgtttt gttttgtaaa aatgataaaa cttcagaaaa t                    401
<210> 178
   <211> 561
   <212> DNA
   <213> Homo sapiens
<400> 178
   acgcctttca agggtgtacg caaagcactc attgataccc ttttggatgg ctatgaaaca 60
   gcccgctatg ggacaggggt ctttggccag aatgagtacc tacgctatca ggaggccctg 120
   agtgagctgg ccactgcggt taaagcacga attgggagct ctcagcgaca tcaccagtca 180
   gcagccaaag acctaactca gtcccctgag gtctccccaa caaccatcca ggtgacatac 240
   ctcccctcca gtcagaagag taaacgtgcc aagcacttcc ttgaattgaa gagctttaag 300
   gataactata acacattgga gagtactctg tgacggagct gaaggactct tgccgtagat 360
   taagccagtc agttgcaatg tgcaagacag gctgcttgcc gggccgccct cggaacatct 420
   ggcccagcag gcccagactg tatccatcca agttcccgtt gtatccagag ttcttagagc 480
   ttgtgtctaa agggtaattc cccaaccctt ccttatgagc atttttagaa cattggctaa 540
   gactattttc ccccagtagc g                                           561
<210> 179
   <211> 521
   <212> DNA
   <213> Homo sapiens
<400> 179
   cccaacgcgt ttgcaaatat tcccctggta gcctacttcc ttacccccga atattggtaa 60
   gatcgagcaa tggcttcagg acatgggttc tcttctcctg tgatcattca agtgctcact 120
   gcatgaagac tggcttgtct cagtgtttca acctcaccag ggctgtctct tggtccacac 180
   ctcgctccct gttagtgccg tatgacagcc cccatcaaat gaccttggcc aagtcacggt 240
   ttctctgtgg tcaaggttgg ttggctgatt ggtggaaagt agggtggacc aaaggaggcc 300
   acgtgagcag tcagcaccag ttctgcacca gcagcgcctc cgtcctagtg ggtgttcctg 360
   tttctcctgg ccctgggtgg gctagggcct gattcgggaa gatgcctttg cagggagggg 420
   aggataagtg ggatctacca attgattctg gcaaaacaat ttctaagatt tttttgcttt 480
   atgtgggaaa cagatctaaa tctcatttta tgctgtattt t                     521
<210> 180
   <211> 417
   <212> DNA
   <213> Homo sapiens
<400> 180
   ggtggaattc gccgaagatg gcggaggtgc aggtcctggt gcttgatggt cgaggccatc 60
   tcctgggccg cctggcggcc atcgtggcta aacaggtact gctgggccgg aaggtggtgg 120
   tcgtacgctg tgaaggcatc aacatttctg gcaatttcta cagaaacaag ttgaagtacc 180
   tggctttcct ccgcaagcgg atgaacacca acccttcccg aggcccctac cacttccggg 240
   cccccagccg catcttctgg cggaccgtgc gaggtatgct gccccacaaa accaagcgag 300
   gccaggccgc tctggaccgt ctcaaggtgt ttgacggcat cccaccgccc tacgacaaga 360
   aaaagcggat ggtggttcct gctgccctca aggtcgtgcg tctgaagcct acaagaa    417
<210> 181
   <211> 283
   <212> DNA
   <213> Homo sapiens
<220>
   <221> misc_feature
   <222> 35
   <223> n = A,T,C or G
<400> 181
   gatttcttct aaataggatg taaaacttct ttcanattac tcttcctcag tcctgcctgc 60
   caagaactca agtgtaactg tgataaaata acctttccca ggtatattgg caggtatgtg 120
   tgtaatctca gaatacacag gtgacataga tatgatatga caactggtaa tggtggattc 180
   atttacattg tttacacttc tatgaccagg ccttaaggga aggtcagttt tttaaaaaac 240
   caagtagtgt cttcctacct atctccagat acatgtcaaa aaa                   283
<210> 182
   <211> 401
   <212> DNA
   <213> Homo sapiens
<400> 182
   atattcttgc tgcttatgca gctgacattg ttgccctccc taaagcaacc aagtagcctt 60
   tatttcccac agtgaaagaa aacgctggcc tatcagttac attacaaaag gcagatttca 120
   agaggattga gtaagtagtt ggatggcttt cataaaaaca agaattcaag aagaggattc 180
   atgctttaag aaacatttgt tatacattcc tcacaaatta tacctgggat aaaaactatg 240
   tagcaggcag tgtgttttcc ttccatgtct ctctgcacta cctgcagtgt gtcctctgag 300
   gctgcaagtc tgtcctatct gaattcccag cagaagcact aagaagctcc accctatcac 360
   ctagcagata aaactatggg gaaaacttaa atctgtgcat a                     401
<210> 183
   <211> 366
   <212> DNA
   <213> Homo sapiens
<220>
   <221> misc_feature
   <222> 325
   <223> n = A,T,C or G
<400> 183
   accgtgtcca agtttttaga acccttgtta gccagaccga ggtgtcctgg tcaccgtttc 60
   accatcatgc tttgatgttc ccctgtcttt ctctcttctg ctctcaagag caaaggttaa 120
   tttaaggaca aagatgaagt cactgtaaac taatctgtca ttgtttttac cttccttttc 180
   tttttcagtg cagaaattaa aagtaagtat aaagcaccgt gattgggagt gtttttgcgt 240
   gtgtcggaat cactggtaaa tgttggctga gaacaatccc tccccttgca cttgtgaaaa 300
   cactttgagc gctttaagag attancctga gaaataatta aatatctttt ctcttcaaaa 360
   aaaaaa                                                            366
<210> 184
   <211> 370
   <212> DNA
   <213> Homo sapiens
<400> 184
   tcttacttca aaagaaaaat aaacataaaa aataagttgc tggttcctaa caggaaaaat 60
   tttaataatt gtactgagag aaactgctta cgtacacatt gcagatcaaa tatttggagt 120
   taaaatgtta gtctacatag atgggtgatt gtaactttat tgccattaaa agatttcaaa 180
   ttgcattcat gcttctgtgt acacataatg aaaaatgggc aaataatgaa gatctctcct 240
   tcagtctgct ctgtttaatt ctgctgtctg ctcttctcta atgctgcgtc cctaattgta 300
   cacagtttag tgatatctag gagtataaag ttgtcgccca tcaataaaaa tcacaaagtt 360
   ggtttaaaaa                                                        370
<210> 185
   <211> 107
   <212> DNA
   <213> Homo sapiens
<400> 185
   ctcatattat tttccttttg agaaattgga aactctttct gttgctatta tattaataaa 60
   gttggtgttt attttctggt agtcaccttc cccatttaaa aaaaaaa                107
<210> 186
   <211> 309
   <212> DNA
   <213> Homo sapiens
<400> 186
   gaaaggatgg ctctggttgc cacagagctg ggacttcatg ttcttctaga gagggccaca 60
   agagggccac aggggtggcc gggagttgtc agctgatgcc tgctgagagg caggaattgt 120
   gccagtgagt gacagtcatg agggagtgtc tcttcttggg gaggaaagaa ggtagagcct 180
   ttctgtctga atgaaaggcc aaggctacag tacagggccc cgccccagcc agggtgttaa 240
   tgcccacgta gtggaggcct ctggcagatc ctgcattcca aggtcactgg actgtacgtt 300
   tttatggtt                                                         309
<210> 187
   <211> 477
   <212> DNA
   <213> Homo sapiens
<400> 187
   ttcagtccta gcaagaagcg agaattctga gatcctccag aaagtcgagc agcacccacc 60
   tccaacctcg ggccagtgtc ttcaggcttt actggggacc tgcgagctgg cctaatgtgg 120
   tggcctgcaa gccaggccat ccctgggcgc cacagacgag ctccgagcca ggtcaggctt 180
   cggaggccac aagctcagcc tcaggcccag gcactgattg tggcagaggg gccactaccc 240
   aaggtctagc taggcccaag acctagttac ccagacagtg agaagcccct ggaaggcaga 300
   aaagttggga gcatggcaga cagggaaggg aaacattttc agggaaaaga catgtatcac 360
   atgtcttcag aagcaagtca ggtttcatgt aaccgagtgt cctcttgcgt gtccaaaagt 420
   agcccagggc tgtagcacag gcttcacagt gattttgtgt tcagccgtga gtcacac    477
<210> 188
   <211> 220
   <212> DNA
   <213> Homo sapiens
<400> 188
   taaatatggt agatattaat attcctctta gatgaccagt gattccaatt gtcccaagtt 60
   ttaaataagt accctgtgag tatgagataa attagtgaca atcagaacaa gtttcagtat 120
   cagatgttca agaggaagtt gctattgcat tgattttaat atttgtacat aaacactgat 180
   ttttttgagc attattttgt atttgttgta ctttaatacc 220
<210> 189
   <211> 417
   <212> DNA
   <213> Homo sapiens
<220>
   <221> misc_feature
   <222> 76, 77
   <223> n = A,T,C or G
<400> 189
   accatcttga cagaggatac atgctcccaa aacgtttgtt accacactta aaaatcactg 60
   ccatcattaa gcatcnnttt caaaattata gccattcatg atttactttt tccagatgac 120
   tatcattatt ctagtccttt gaatttgtaa ggggaaaaaa aacaaaaaca aaaacttacg 180
   atgcactttt ctccagcaca tcagatttca aattgaaaat taaagacatg ctatggtaat 240
   gcacttgcta gtactacaca ctttgtacaa caaaaaacag aggcaagaaa caacggaaag 300
   agaaaagcct tcctttgttg gcccttaaac tgagtcaaga tctgaaatgt agagatgatc 360
   tctgacgata cctgtatgtt cttattgtgt aaataaaatt gctggtatga aatgaca 417
<210> 190
   <211> 497
   <212> DNA
   <213> Homo sapiens
<400> 190
   gcactgcggc gctctcccgt cccgcggtgg ttgctgctgc tgccgctgct gctgggcctg 60
   aacgcaggag ctgtcattga ctggcccaca gaggagggca aggaagtatg ggattatgtg 120
   acggtccgca aggatgccta catgttctgg tggctctatt atgccaccaa ctcctgcaag 180
   aacttctcag aactgcccct ggtcatgtgg cttcagggcg gtccaggcgg ttctagcact 240
   ggatttggaa actttgagga aattgggccc cttgacagtg atctcaaacc acggaaaacc 300
   acctggctcc aggctgccag tctcctattt gtggataatc ccgtgggcac tgggttcagt 360
   tatgtgaatg gtagtggtgc ctatgccaag gacctggcta tggtggcttc agacatgatg 420
   gttctcctga agaccttctt cagttgccac aaagaattcc agacagttcc attctacatt 480
   ttctcagagt cctatgg 497
<210> 191
   <211> 175
   <212> DNA
   <213> Homo sapiens
<400> 191
   atgttgaata ttttgcttat taactttgtt tattgtcttc tccctcgatt agaatattag 60
   ctacttgagt acaaggattt gagcctgtta cattcactgc tgaattttag gctcctggaa 120
   gatacccagc attcaataga gaccacacaa taaatatatg tcaaataaaa aaaaa      175
<210> 192
   <211> 526
   <212> DNA
   <213> Homo sapiens
<400> 192
   agtaaacatt attatttttt ttatatttgc aaaggaaaca tatctaatcc ttcctataga 60
   aagaacagta ttgctgtaat tccttttctt ttcttcctca tttcctctgc cccttaaaag 120
   attgaagaaa gagaaacttg tcaactcata tccacgttat ctagcaaagt acataagaat 180
   ctatcactaa gtaatgtatc cttcagaatg tgttggttta ccagtgacac cccatattca 240
   tcacaaaatt aaagcaagaa gtccatagta atttatttgc taatagtgga tttttaatgc 300
   tcagagtttc tgaggtcaaa ttttatcttt tcacttacaa gctctatgat cttaaataat 360
   ttacttaatg tattttggtg tattttcctc aaattaatat tggtgttcaa gactatatct 420
   aattcctctg atcactttga gaaacaaact tttattaaat gtaaggcact tttctatgaa 480
   ttttaaatat aaaaataaat attgttctga ttattactga aaaaaa                526
<210> 193
   <211> 553
   <212> DNA
   <213> Homo sapiens
<220>
   <221> misc_feature
   <222> 290, 300, 411, 441
   <223> n = A,T,C or G
<400> 193
   tccattgtgg tggaattcgc tctctggtaa aggcgtgcag gtgttggccg cggcctctga 60
   gctgggatga gccgtgctcc cggtggaagc aagggagccc agccggagcc atggccagta 120
   cagtggtagc agttggactg accattgctg ctgcaggatt tgcaggccgt tacgttttgc 180
   aagccatgaa gcatatggag cctcaagtaa aacaagtttt tcaaagccta ccaaaatctg 240
   ccttcagtgg tggctattat agaggtgggt ttgaacccaa aatgacaaan cgggaagcan 300
   cattaatact aggtgtaagc cctactgcca ataaagggaa aataagagat gctcatcgac 360
   gaattatgct tttaaatcat cctgacaaag gaggatctcc ttatatagca nccaaaatca 420
   atgaagctaa agatttacta naaggtcaag ctaaaaaatg aagtaaatgt atgatgaatt 480
   ttaagttcgt attagtttat gtatatgagt actaagtttt tataataaaa tgcctcagag 540
   ctacaatttt aaa                                                    553
<210> 194
   <211> 320
   <212> DNA
   <213> Homo sapiens
<400> 194
   cccttcccaa tccatcagta aagaccccat ctgccttgtc catgccgttt cccaacaggg 60
   atgtcacttg atatgagaat ctcaaatctc aatgccttat aagcattcct tcctgtgtcc 120
   attaagactc tgataattgt ctcccctcca taggaatttc tcccaggaaa gaaatatatc 180
   cccatctccg tttcatatca gaactaccgt ccccgatatt cccttcagag agattaaaga 240
   ccagaaaaaa gtgagcctct tcatctgcac ctgtaatagt ttcagttcct attttcttcc 300
   attgacccat atttatacct 320
<210> 195
   <211> 320
   <212> DNA
   <213> Homo sapiens
<220>
   <221> misc_feature
   <222> 203, 218
   <223> n = A,T,C or G
<400> 195
   aagcatgacc tggggaaatg gtcagacctt gtattgtgtt tttggccttg aaagtagcaa 60
   gtgaccagaa tctgccatgg caacaggctt taaaaaagac ccttaaaaag acactgtctc 120
   aactgtggtg ttagcaccag ccagctctct gtacatttgc tagcttgtag ttttctaaga 180
   ctgagtaaac ttcttatttt tanaaagggg aggctggntt gtaactttcc ttgtacttaa 240
   ttgggtaaaa gtcttttcca caaaccacca tctattttgt gaactttgtt agtcatcttt 300
   tatttggtaa attatgaact 320
<210> 196
   <211> 357
   <212> DNA
   <213> Homo sapiens
<220>
   <221> misc_feature
   <222> 36
   <223> n = A,T,C or G
<400> 196
   atataaaata atacgaaact ttaaaaagca ttggantgtc agtatgttga atcagtagtt 60
   tcactttaac tgtaaacaat ttcttaggac accatttggg ctagtttctg tgtaagtgta 120
   aatactacaa aaacttattt atactgttct tatgtcattt gttatattca tagatttata 180
   tgatgatatg acatctggct aaaaagaaat tattgcaaaa ctaaccacta tgtacttttt 240
   tataaatact gtatggacaa aaaatggcat tttttatatt aaattgttta gctctggcaa 300
   aaaaaaaaaa ttttaagagc tggtactaat aaaggattat tatgactgtt aaaaaaa 357
<210> 197
   <211> 565
   <212> DNA
   <213> Homo sapiens
<220>
   <221> misc_feature
   <222> 27
   <223> n = A,T,C or G
<400> 197
   tcagctgagt accatcagga tatttanccc tttaagtgct gttttgggag tagaaaacta 60
   aagcaacaat acttcctctt gacagctttg attggaatgg ggttattaga tcattcacct 120
   tggtcctaca ctttttagga tgcttggtga acataacacc acttataatg aacatccctg 180
   gttcctatat tttgggctat gtgggtagga attgttactt gttactgcag cagcagccct 240
   agaaagtaag cccagggctt cagatctaag ttagtccaaa agctaaatga tttaaagtca 300
   agttgtaatg ctaggcataa gcactctata atacattaaa ttataggccg agcaattagg 360
   gaatgtttct gaaacattaa acttgtattt atgtcactaa aattctaaca caaacttaaa 420
   aaatgtgtct catacatatg ctgtactagg cttcatcatg catttctaaa tttgtgtatg 480
   atttgaatat atgaaagaat ttatacaaga gtgttattta aaattattaa aaataaatgt 540
   atataatttg tacctattgt aaaaa                                       565
<210> 198
   <211> 484
   <212> DNA
   <213> Homo sapiens
<400> 198
   tatgtaagta ttggtgtctg ctttaaaaaa ggagacccag acttcacctg tcctttttaa 60
   acatttgaga acagtgttac tctgagcagt tgggccacct tcaccttatc cgacagctga 120
   ctgttggatg tgtccattgt cgccagtttg gctgttgccc ggacaggaca ggacctccat 180
   tgggcgcagc agcaggtggc aggggtgtgg cttgaggtgg gtggcagcgt ctggtcctcc 240
   tctctggtgc tttctgagag ggtctctaaa gcagagtgtg gttggcctgg gggaaggcag 300
   agcacgtatt tctcccctct agtacctctg catttgtgag tgttccctct ggctttctga 360
   agggcagcag actcttgagt atactgcaga ggacatgctt tatcagtagg tcctgagggc 420
   tccaggggct caactgacca agtaacacag aagttggggt atgtggccta tttgggtcgg 480
   aaac                                                              484
<210> 199
   <211> 429
   <212> DNA
   <213> Homo sapiens
<220>
   <221> misc_feature
   <222> 77, 88, 134, 151, 189, 227, 274, 319
   <223> n = A,T,C or G
<400> 199
   gcttatgttt tttgttttaa cttttgtttt ttaacattta gaatattaca ttttgtatta 60
   tacagtacct ttctcanaca ttttgtanaa ttcatttcgg cagctcacta ggattttgct 120
   gaacattaaa aagngtgata gcgatattag ngccaatcaa atggaaaaaa ggtagtctta 180
   ataaacaana cacaacgttt ttatacaaca tactttaaaa tattaanaaa actccttaat 240
   attgtttcct attaagtatt attctttggg caanattttc tgatgctttt gattttctct 300
   caatttagca tttgctttng gtttttttct ctatttagca ttctgttaag gcacaaaaac 360
   tatgtactgt atgggaaatg ttgtaaatat taccttttcc acattttaaa cagacaactt 420
   tgaatccaa                                                         429
<210> 200
   <211> 279
   <212> DNA
   <213> Homo sapiens
<400> 200
   gcttttttga ggaattacag ggaagctcct ggaattgtac atggatatct ttatccctag 60
   ggggaaatca aggagctggg cacccctaat tctttatgga agtgtttaaa actattttaa 120
   ttttattaca agtattacta gagtagtggt tctactctaa gatttcaaaa gtgcatttaa 180
   aatcatacat gttcccgcct gcaaatatat tgttattttg gtggagaaaa aaatagtata 240
   ttctacataa aaaattaaag atattaacta agaaaaaaa                        279
<210> 201
   <211> 569
   <212> DNA
   <213> Homo sapiens
<400> 201
   taggtcagta tttttagaaa ctcttaatag ctcatactct tgataccaaa agcagccctg 60
   attgttaaag cacacacctg cacaagaagc agtgatggtt gcatttacat ttcctgggtg 120
   cacaaaaaaa aattctcaaa aagcaaggac ttacgctttt tgcaaagcct ttgagaagtt 180
   actggatcat aggaagctta taacaagaat ggaagattct taaataactc actttctttg 240
   gtatccagta acagtagatg ttcaaaatat gtagctgatt aataccagca ttgtgaacgc 300
   tgtacaacct tgtggttatt actaagcaag ttactactag cttctgaaaa gtagcttcat 360
   aattaatgtt atttatacac tgccttccat gacttttact ttgccctaag ctaatctcca 420
   aaatctgaaa tgctactcca atatcagaaa aaaaggggga ggtggaatta tatttcctgt 480
   gattttaaga gtacagagaa tcatgcacat ctctgattag ttcatatatg tctagtgtgt 540
   aataaaagtc aaagatgaac tctcaaaaa                                   569
<210> 202
   <211> 501
   <212> DNA
   <213> Homo sapiens
<400> 202
   attaataggc ttaataattg ttggcaagga tccttttgct ttctttggca tgcaagctcc 60
   tagcatctgg cagtggggcc aagaaaataa ggtttatgca tgtatgatgg ttttcttctt 120
   gagcaacatg attgagaacc agtgtatgtc aacaggtgca tttgagataa ctttaaatga 180
   tgtacctgtg tggtctaagc tggaatctgg tcaccttcca tccatgcaac aacttgttca 240
   aattcttgac aatgaaatga agctcaatgt gcatatggat tcaatcccac accatcgatc 300
   atagcaccac ctatcagcac tgaaaactct tttgcattaa gggatcattg caagagcagc 360
   gtgactgaca ttatgaaggc ctgtactgaa gacagcaagc tgttagtaca gaccagatgc 420
   tttcttggca ggctcgttgt acctcttgga aaacctcaat gcaagatagt gtttcagtgc 480
   tggcatattt tggaattctg c                                           501
<210> 203
   <211> 261
   <212> DNA
   <213> Homo sapiens
<220>
   <221> misc_feature
   <222> 36, 96
   <223> n = A,T,C or G
<400> 203
   gacaagctcc tggtcttgag atgtcttctc gttaangaga tgggcctttt ggaggtaaag 60
   gataaaatga atgagttctg tcatgattca ctattntata acttgcatga cctttactgt 120
   gttagctctt tgaatgttct tgaaatttta gactttcttt gtaaacaaat gatatgtcct 180
   tatcattgta taaaagctgt tatgtgcaac agtgtggaga ttccttgtct gatttaataa 240
   aatacttaaa cactgaaaaa a 261
<210> 204
   <211> 421
   <212> DNA
   <213> Homo sapiens
<400> 204
   agcatctttt ctacaacgtt aaaattgcag aagtagctta tcattaaaaa acaacaacaa 60
   caacaataac aataaatcct aagtgtaaat cagttattct accccctacc aaggatatca 120
   gcctgttttt tccctttttt ctcctgggaa taattgtggg cttcttccca aatttctaca 180
   gcctctttcc tcttctcatg cttgagcttc cctgtttgca cgcatgcgtg tgcaggactg 240
   gcttgtgtgc ttggactcgg ctccaggtgg aagcatgctt tcccttgtta ctgttggaga 300
   aactcaaacc ttcaagccct aggtgtagcc attttgtcaa gtcatcaact gtatttttgt 360
   actggcatta acaaaaaaag aagataaaat attgtaccat taaactttaa taaaacttta 420
   a 421
<210> 205
   <211> 460
   <212> DNA
   <213> Homo sapiens
<400> 205
   tactctcaca atgaaggacc tggaatgaaa aatctgtgtc taaacaagtc ctctttagat 60
   tttagtgcaa atccagagcc agcgtcggtt gcctcgagta attctttcat gggtaccttt 120
   ggaaaagctc tcaggagacc tcacctagat gcctattcaa gctttggaca gccatcagat 180
   tgtcagccaa gagcctttta tttgaaagct cattcttccc cagacttgga ctctgggtca 240
   gaggaagatg ggaaagaaag gacagatttt caggaagaaa atcacatttg tacctttaaa 300
   cagactttag aaaactacag gactccaaat tttcagtctt atgacttgga cacatagact 360
   gaatgagacc aaaggaaaag cttaacatac tacctcaagg tgaactttta tttaaaagag 420
   agagaatctt atgtttttta aatggagtta tgaattttaa 460
<210> 206
   <211> 481
   <212> DNA
   <213> Homo sapiens
<400> 206
   tgtggtggaa ttcgggacgc ccccagaccc tgactttttc ctgcgtgggc cgtctcctcc 60
   tgcggaagca gtgacctctg acccctggtg accttcgctt tgagtgcctt ttgaacgctg 120
   gtcccgcggg acttggtttt ctcaagctct gtctgtccaa agacgctccg gtcgaggtcc 180
   cgcctgccct gggtggatac ttgaacccca gacgcccctc tgtgctgctg tgtccggagg 240
   cggccttccc atctgcctgc ccacccggag ctctttccgc cggcgcaggg tcccaagccc 300
   acctcccgcc ctcagtcctg cggtgtgcgt ctgggcacgt cctgcacaca caatgcaagt 360
   cctggcctcc gcgcccgccc gcccacgcga gccgtacccg ccgccaactc tgttatttat 420
   ggtgtgaccc cctggaggtg ccctcggccc accggggcta tttattgttt aatttatttg 480
   t 481
<210> 207
   <211> 605
   <212> DNA
   <213> Homo sapiens
<400> 207
   accctttttg gattcagggc tcctcacaat taaaatgagt gtaatgaaac aaggtgaaaa 60
   tatagaagca tccctttgta tactgttttg ctacttacag tgtacttggc attgctttat 120
   ctcactggat tctcacggta ggatttctga gatcttaatc taagctccaa agttgtctac 180
   ttttttgatc ctagggtgct ccttttgttt tacagagcag ggtcacttga tttgctagct 240
   ggtggcagaa ttggcaccat tacccaggtc tgactgacca ccagtcagag gcactttatt 300
   tgtatcatga aatgatttga aatcattgta aagcagcgaa gtctgataat gaatgccagc 360
   tttccttgtg ctttgataac aaagactcca aatattctgg agaacctgga taaaagtttg 420
   aagggctaga ttgggatttg aagacaaaat tgtaggaaat cttacatttt tgcaataaca 480
   aacattaatg aaagcaaaac attataaaag taattttaat tcaccacata cttatcaatt 540
   tcttgatgct tccaaatgac atctaccaga tatggttttg tggacatctt tttctgttta 600
   cataa 605
<210> 208
   <211> 655
   <212> DNA
   <213> Homo sapiens
<400> 208
   ggcgttgttc tggattcccg tcgtaactta aagggaaact ttcacaatgt ccggagccct 60
   tgatgtcctg caaatgaagg aggaggatgt ccttaagttc cttgcagcag gaacccactt 120
   aggtggcacc aatcttgact tccagatgga acagtacatc tataaaagga aaagtgatgg 180
   catctatatc ataaatctca agaggacctg ggagaagctt ctgctggcag ctcgtgcaat 240
   tgttgccatt gaaaaccctg ctgatgtcag tgttatatcc tccaggaata ctggccagag 300
   ggctgtgctg aagtttgctg ctgccactgg agccactcca attgctggcc gcttcactcc 360
   tggaaccttc actaaccaga tccaggcagc cttccgggag ccacggcttc ttgtggttac 420
   tgaccccagg gctgaccacc agcctctcac ggaggcatct tatgttaacc tacctaccat 480
   tgcgctgtgt aacacagatt ctcctctgcg ctatgtggac attgccatcc catgcaacaa 540
   caagggagct cactcagtgg gtttgatgtg gtggatgctg gctcgggaag ttctgcgcat 600
   gcgtggcacc atttcccgtg aacacccatg ggaggtcatg cctgatctgt acttc 655
<210> 209
   <211> 621
   <212> DNA
   <213> Homo sapiens
<400> 209
   catttagaac atggttatca tccaagacta ctctaccctg caacattgaa ctcccaagag 60
   caaatccaca ttcctcttga gttctgcagc ttctgtgtaa atagggcagc tgtcgtctat 120
   gccgtagaat cacatgatct gaggaccatt catggaagct gctaaatagc ctagtctggg 180
   gagtcttcca taaagttttg catggagcaa acaaacagga ttaaactagg tttggttcct 240
   tcagccctct aaaagcatag ggcttagcct gcaggcttcc ttgggctttc tctgtgtgtg 300
   tagttttgta aacactatag catctgttaa gatccagtgt ccatggaaac cttcccacat 360
   gccgtgactc tggactatat cagtttttgg aaagcagggt tcctctgcct gctaacaagc 420
   ccacgtggac cagtctgaat gtctttcctt tacacctatg tttttaaata gtcaaacttc 480
   aagaaacaat ctaaacaagt ttctgttgca tatgtgtttg tgaacttgta tttgtattta 540
   gtaggcttct atattgcatt taacttgttt ttgtaactcc tgattcttcc ttttcggata 600
   ctattgatga ataaagaaat t                                           621
<210> 210
   <211> 533
   <212> DNA
   <213> Homo sapiens
<220>
   <221> misc_feature
   <222> 20, 21, 61
   <223> n = A,T,C or G
<400> 210
   cgccttgggg agccggcggn ngagtccggg acgtggagac ccggggtccc ggcagccggg 60
   nggcccgcgg gcccagggtg gggatgcacc gccgcggggt gggagctggc gccatcgcca 120
   agaagaaact tgcagaggcc aagtataagg agcgagggac ggtcttggct gaggaccagc 180
   tagcccagat gtcaaagcag ttggacatgt tcaagaccaa cctggaggaa tttgccagca 240
   aacacaagca ggagatccgg aagaatcctg agttccgtgt gcagttccag gacatgtgtg 300
   caaccattgg cgtggatccg ctggcctctg gaaaaggatt ttggtctgag atgctgggcg 360
   tgggggactt ctattacgaa ctaggtgtcc aaattatcga agtgtgcctg gcgctgaagc 420
   atcggaatgg aggtctgata actttggagg aactacatca acaggtgttg aagggaaggg 480
   gcaagttcgc ccaggatgtc agtcaagatg acctgatcag agccatcaag aaa        533
<210> 211
   <211> 451
   <212> DNA
   <213> Homo sapiens
<400> 211
   ttagcttgag ccgagaacga ggcgagaaag ctggagaccg aggagaccgc ctagagcgga 60
   gtgaacgggg aggggaccgt ggggaccggc ttgatcgtgc gcggacacct gctaccaagc 120
   ggagcttcag caaggaagtg gaggagcgga gtagagaacg gccctcccag cctgaggggc 180
   tgcgcaaggc agctagcctc acggaggatc gggaccgtgg gcgggatgcc gtgaagcgag 240
   aagctgccct acccccagtg agccccctga aggcggctct ctctgaggag gagttagaga 300
   agaaatccaa ggctatcatt gaggaatatc tccatctcaa tgacatgaaa gaggcagtcc 360
   agtgcgtgca ggagctggcc tcaccctcct tgctcttcat ctttgtacgg catggtgtcg 420
   agtctacgct ggagcgcagt gccattgctc g                                451
<210> 212
   <211> 471
   <212> DNA
   <213> Homo sapiens
<220>
   <221> misc_feature
   <222> 54
   <223> n = A,T,C or G
<400> 212
   gtgattattc ttgatcaggg agaagatcat ttagatttgt tttgcattcc ttanaatgga 60
   gggcaacatt ccacagctgc cctggctgtg atgagtgtcc ttgcaggggc cggagtagga 120
   gcactggggt gggggcggaa ttggggttac tcgatgtaag ggattccttg ttgttgtgtt 180
   gagatccagt gcagttgtga tttctgtgga tcccagcttg gttccaggaa ttttgtgtga 240
   ttggcttaaa tccagttttc aatcttcgac agctgggctg gaacgtgaac tcagtagctg 300
   aacctgtctg acccggtcac gttcttggat cctcagaact ctttgctctt gtcggggtgg 360
   gggtgggaac tcacgtgggg agcggtggct gagaaaatgt aaggattctg gaatacatat 420
   tccatgggac tttccttccc tctcctgctt cctcttttcc tgctccctaa c          471
<210> 213
   <211> 511
   <212> DNA
   <213> Homo sapiens
<220>
   <221> misc_feature
   <222> 27, 63, 337, 442
   <223> n = A,T,C or G
<400> 213
   ctaattagaa acttgctgta ctttttnttt tcttttaggg gtcaaggacc ctctttatag 60
   ctnccatttg cctacaataa attattgcag cagtttgcaa tactaaaata ttttttatag 120
   actttatatt tttccttttg ataaagggat gctgcatagt agagttggtg taattaaact 180
   atctcagccg tttccctgct ttcccttctg ctccatatgc ctcattgtcc ttccagggag 240
   ctcttttaat cttaaagttc tacatttcat gctcttagtc aaattctgtt acctttttaa 300
   taactcttcc cactgcatat ttccatcttg aattggnggt tctaaattct gaaactgtag 360
   ttgagataca gctatttaat atttctggga gatgtgcatc cctcttcttt gtggttgccc 420
   aaggttgttt tgcgtaactg anactccttg atatgcttca gagaatttag gcaaacactg 480
   gccatggccg tgggagtact gggagtaaaa t                                511
<210> 214
   <211> 521
   <212> DNA
   <213> Homo sapiens
<400> 214
   agcattgcca aataatccct aattttccac taaaaatata atgaaatgat gttaagcttt 60
   ttgaaaagtt taggttaaac ctactgttgt tagattaatg tatttgttgc ttccctttat 120
   ctggaatgtg gcattagctt ttttatttta accctcttta attcttattc aattccatga 180
   cttaaggttg gagagctaaa cactgggatt tttggataac agactgacag ttttgcataa 240
   ttataatcgg cattgtacat agaaaggata tggctacctt ttgttaaatc tgcactttct 300
   aaatatcaaa aaagggaaat gaagtataaa tcaatttttg tataatctgt ttgaaacatg 360
   agttttattt gcttaatatt agggctttgc cccttttctg taagtctctt gggatcctgt 420
   gtagaagctg ttctcattaa acaccaaaca gttaagtcca ttctctggta ctagctacaa 480
   attcggtttc atattctact taacaattta aataaactga a                    521
<210> 215
   <211> 381
   <212> DNA
   <213> Homo sapiens
<220>
   <221> misc_feature
   <222> 17, 20, 60, 61, 365
   <223> n = A,T,C or G
<400> 215
   gagcggagag cggaccngtn agagccctga gcagccccac cgccgccgcc ggcctagttn 60
   ncatcacacc ccgggaggag ccgcagctgc cgcagccggc cccagtcacc atcaccgcaa 120
   ccatgagcag cgaggccgag acccagcagc cgcccgccgc cccccccgcc gcccccgccc 180
   tcagcgccgc cgacaccaag cccggcacta cgggcagcgg cgcagggagc ggtggcccgg 240
   gcggcctcac atcggcggcg cctgccggcg gggacaagaa ggtcatcgca acgaaggttt 300
   tgggaacagt aaaatggttc aatgtaagga acggatatgg tttcatcaac aggaatgaca 360
   ccaangaaga tgtatttgta c                                          381
<210> 216
   <211> 425
   <212> DNA
   <213> Homo sapiens
<400> 216
   ttactaacta ggtcattcaa ggaagtcaag ttaacttaaa catgtcacct aaatgcactt 60
   gatggtgttg aaatgtccac cttcttaaat ttttaagatg aacttagttc taaagaagat 120
   aacaggccaa tcctgaaggt actccctgtt tgctgcagaa tgtcagatat tttggatgtt 180
   gcataagagt cctatttgcc ccagttaatt caacttttgt ctgcctgttt tgtggactgg 240
   ctggctctgt tagaactctg tccaaaaagt gcatggaata taacttgtaa agcttcccac 300
   aattgacaat atatatgcat gtgtttaaac caaatccaga aagcttaaac aatagagctg 360
   cataatagta tttattaaag aatcacaact gtaaacatga gaataactta aggattctag 420
   tttag                                                            425
<210> 217
   <211> 181
   <212> DNA
   <213> Homo sapiens
<400> 217
   gagaaaccaa atgataggtt gtagagcctg atgactccaa acaaagccat cacccgcatt 60
   cttcctcctt cttctggtgc tacagctcca agggcccttc accttcatgt ctgaaatgga 120
   actttggctt tttcagtgga agaatatgtt gaaggtttca ttttgttcta gaaaaaaaaa 180
   a                                                                 181
<210> 218
   <211> 405
   <212> DNA
   <213> Homo sapiens
<400> 218
   caggccttcc agttcactga caaacatggg gaagtgtgcc cagctggctg gaaacctggc 60
   agtgatacca tcaagcctga tgtccaaaag agcaaagaat atttctccaa gcagaagtga 120
   gcgctgggct gttttagtgc caggctgcgg tgggcagcca tgagaacaaa acctcttctg 180
   tatttttttt ttccattagt aaaacacaag acttcagatt cagccgaatt gtggtgtctt 240
   acaaggcagg cctttcctac agggggtgga gagaccagcc tttcttcctt tggtaggaat 300
   ggcctgagtt ggcgttgtgg gcaggctact ggtttgtatg atgtattagt agagcaaccc 360
   attaatcttt tgtagtttgt attaaacttg aactgagaaa aaaaa                 405
<210> 219
   <211> 216
   <212> DNA
   <213> Homo sapiens
<220>
   <221> misc_feature
   <222> 207, 210
   <223> n = A,T,C or G
<400> 219
   actccaagag ttagggcagc agagtggagc gatttagaaa gaacatttta aaacaatcag 60
   ttaatttacc atgtaaaatt gctgtaaatg ataatgtgta cagattttct gttcaaatat 120
   tcaattgtaa acttcttgtt aagactgtta cgtttctatt gcttttgtat gggatattgc 180
   aaaaataaaa aggaaagaac cctcttnaan aaaaaa                          216
<210> 220
   <211> 380
   <212> DNA
   <213> Homo sapiens
<400> 220
   cttacaaatt gcccccatgt gtaggggaca cagaaccctt tgagaaaact tagatttttg 60
   tctgtacaaa gtctttgcct ttttccttct tcattttttt ccagtacatt aaatttgtca 120
   atttcatctt tgagggaaac tgattagatg ggttgtgttt gtgttctgat ggagaaaaca 180
   gcaccccaag gactcagaag atgattttaa cagttcagaa cagatgtgtg caatattggt 240
   gcatgtaata atgttgagtg gcagtcaaaa gtcatgattt ttatcttagt tcttcattac 300
   tgcattgaaa aggaaaacct gtctgagaaa atgcctgaca gtttaattta aaactatggt 360
   gtaagtcttt gacaaaaaaa                                             380
<210> 221
   <211> 398
   <212> DNA
   <213> Homo sapiens
<400> 221
   ggttagtaag ctgtcgactt tgtaaaaaag ttaaaaatga aaaaaaaagg aaaaatgaat 60
   tgtatattta atgaatgaac atgtacaatt tgccactggg aggaggttcc tttttgttgg 120
   gtgagtctgc aagtgaattt cactgatgtt gatattcatt gtgtgtagtt ttatttcggt 180
   cccagccccg tttcctttta ttttggagct aatgccagct gcgtgtctag ttttgagtgc 240
   agtaaaatag aatcagcaaa tcactcttat ttttcatcct tttccggtat tttttgggtt 300
   gtttctgtgg gagcagtgta caccaactct tcctgtatat tgcctttttg ctggaaaatg 360
   ttgtatgttg aataaaattt tctataaaaa ttaaaaaa 398
<210> 222
   <211> 301
   <212> DNA
   <213> Homo sapiens
<220>
   <221> misc_feature
   <222> 49, 64
   <223> n = A,T,C or G
<400> 222
   ttcgataatt gatctcatgg gctttccctg gaggaaaggt tttttttgnt gtttattttt 60
   taanaacttg aaacttgtaa actgagatgt ctgtagcttt tttgcccatc tgtagtgtat 120
   gtgaagattt caaaacctga gagcactttt tctttgttta gaattatgag aaaggcacta 180
   gatgacttta ggatttgcat ttttcccttt attgcctcat ttcttgtgac gccttgttgg 240
   ggagggaaat ctgtttattt tttcctacaa ataaaaagct aagattctat atcgcaaaaa 300
   a 301
<210> 223
   <211> 200
   <212> DNA
   <213> Homo sapiens
<400> 223
   gtaagtgctt aggaagaaac tttgcaaaca tttaatgagg atacactgtt catttttaaa 60
   attccttcac actgtaattt aatgtgtttt atattctttt gtagtaaaac aacataactc 120
   agatttctac aggagacagt ggttttattt ggattgtctt ctgtaatagg tttcaataaa 180
   gctggatgaa cttaaaaaaa 200
<210> 224
   <211> 385
   <212> DNA
   <213> Homo sapiens
<400> 224
   gaaaggtttg atccggactc aaagaaagca aaggagtgtg agccgccatc tgctggagca 60
   gctgtaactg caagacctgg acaagagatt cgtcagcgaa ctgcagctca aagaaacctt 120
   tctccaacac cagcaagccc taaccagggc cctcctccac aagttccagt atctcctgga 180
   ccaccaaagg acagttctgc ccctggtgga cccccagaaa ggactgttac tccagcccta 240
   tcatcaaatg tgttaccaag acatcttgga tcccctgcta cttcagtgcc tggaatgggt 300
   aaacagagca cttaatgtta tttacagttt atattgtttt ctctggttac caataaaacg 360
   ggccattttc aggtggtaaa aaaaa 385
<210> 225
   <211> 560
   <212> PRT
   <213> Homo sapiens
<400> 225
   Met Glu Cys Leu Tyr Tyr Phe Leu Gly Phe Leu Leu Leu Ala Ala Arg
   1               5                   10                  15
   Leu Pro Leu Asp Ala Ala Lys Arg Phe His Asp Val Leu Gly Asn Glu
               20                  25                  30
   Arg Pro Ser Ala Tyr Met Arg Glu His Asn Gln Leu Asn Gly Trp Ser
           35                  40                  45
   Ser Asp Glu Asn Asp Trp Asn Glu Lys Leu Tyr Pro Val Trp Lys Arg
       50                  55                  60
   Gly Asp Met Arg Trp Lys Asn Ser Trp Lys Gly Gly Arg Val Gln Ala
   65                  70                  75                  80
   Val Leu Thr Ser Asp Ser Pro Ala Leu Val Gly Ser Asn Ile Thr Phe
                   85                  90                  95
   Ala Val Asn Leu Ile Phe Pro Arg Cys Gln Lys Glu Asp Ala Asn Gly
               100                 105                 110
   Asn Ile Val Tyr Glu Lys Asn Cys Arg Asn Glu Ala Gly Leu Ser Ala
           115                 120                 125
   Asp Pro Tyr Val Tyr Asn Trp Thr Ala Trp Ser Glu Asp Ser Asp Gly
       130                 135                 140
   Glu Asn Gly Thr Gly Gln Ser His His Asn Val Phe Pro Asp Gly Lys
   145                 150                 155                 160
   Pro Phe Pro His His Pro Gly Trp Arg Arg Trp Asn Phe Ile Tyr Val
                   165                 170                 175
   Phe His Thr Leu Gly Gln Tyr Phe Gln Lys Leu Gly Arg Cys Ser Val
               180                 185                 190
   Arg Val Ser Val Asn Thr Ala Asn Val Thr Leu Gly Pro Gln Leu Met
           195                 200                 205
   Glu Val Thr Val Tyr Arg Arg His Gly Arg Ala Tyr Val Pro Ile Ala
       210                 215                 220
   Gln Val Lys Asp Val Tyr Val Val Thr Asp Gln Ile Pro Val Phe Val
   225                 230                 235                 240
   Thr Met Phe Gln Lys Asn Asp Arg Asn Ser Ser Asp Glu Thr Phe Leu
                   245                 250                 255
   Lys Asp Leu Pro Ile Met Phe Asp Val Leu Ile His Asp Pro Ser His
               260                 265                 270
   Phe Leu Asn Tyr Ser Thr Ile Asn Tyr Lys Trp Ser Phe Gly Asp Asn
           275                 280                 285
   Thr Gly Leu Phe Val Ser Thr Asn His Thr Val Asn His Thr Tyr Val
       290                 295                 300
   Leu Asn Gly Thr Phe Ser Leu Asn Leu Thr Val Lys Ala Ala Ala Pro
   305                 310                 315                 320
   Gly Pro Cys Pro Pro Pro Pro Pro Pro Pro Arg Pro Ser Lys Pro Thr
                   325                 330                 335
   Pro Ser Leu Gly Pro Ala Gly Asp Asn Pro Leu Glu Leu Ser Arg Ile
               340                 345                 350
   Pro Asp Glu Asn Cys Gln Ile Asn Arg Tyr Gly His Phe Gln Ala Thr
           355                 360                 365
   Ile Thr Ile Val Glu Gly Ile Leu Glu Val Asn Ile Ile Gln Met Thr
       370                 375                 380
   Asp Val Leu Met Pro Val Pro Trp Pro Glu Ser Ser Leu Ile Asp Phe
   385                 390                 395                 400
   Val Val Thr Cys Gln Gly Ser Ile Pro Thr Glu Val Cys Thr Ile Ile
                   405                 410                 415
   Ser Asp Pro Thr Cys Glu Ile Thr Gln Asn Thr Val Cys Ser Pro Val
               420                 425                 430
   Asp Val Asp Glu Met Cys Leu Leu Thr Val Arg Arg Thr Phe Asn Gly
           435                 440                 445
   Ser Gly Thr Tyr Cys Val Asn Leu Thr Leu Gly Asp Asp Thr Ser Leu
       450                 455                 460
   Ala Leu Thr Ser Thr Leu Ile Ser Val Pro Asp Arg Asp Pro Ala Ser
   465                 470                 475                 480
   Pro Leu Arg Met Ala Asn Ser Ala Leu Ile Ser Val Gly Cys Leu Ala
                   485                 490                 495
   Ile Phe Val Thr Val Ile Ser Leu Leu Val Tyr Lys Lys His Lys Glu
               500                 505                 510
   Tyr Asn Pro Ile Glu Asn Ser Pro Gly Asn Val Val Arg Ser Lys Gly
           515                 520                 525
   Leu Ser Val Phe Leu Asn Arg Ala Lys Ala Val Phe Phe Pro Gly Asn
       530                 535                 540
   Gln Glu Lys Asp Pro Leu Leu Lys Asn Gln Glu Phe Lys Gly Val Ser
   545                 550                 555                 560
<210> 226
   <211> 9
   <212> PRT
   <213> Homo sapiens
<400> 226
   Ile Leu Ile Pro Ala Thr Trp Lys Ala
    1               5
<210> 227
   <211> 9
   <212> PRT
   <213> Homo sapiens
<400> 227
   Phe Leu Leu Asn Asp Asn Leu Thr Ala
    1               5
<210> 228
   <211> 9
   <212> PRT
   <213> Homo sapiens
<400> 228
   Leu Leu Gly Asn Cys Leu Pro Thr Val
    1               5
<210> 229
   <211> 10
   <212> PRT
   <213> Homo sapiens
<400> 229
   Lys Leu Leu Gly Asn Cys Leu Pro Thr Val
    1               5                  10
<210> 230
   <211> 10
   <212> PRT
   <213> Homo sapiens
<400> 230
   Arg Leu Thr Gly Gly Leu Lys Phe Phe Val
    1               5                   10
<210> 231
   <211> 9
   <212> PRT
   <213> Homo sapiens
<400> 231
   Ser Leu Gln Ala Leu Lys Val Thr Val
    1               5
<210> 232
   <211> 20
   <212> PRT
   <213> Homo sapiens
<400> 232
   Ala Gly Ala Asp Val Ile Lys Asn Asp Gly Ile Tyr Ser Arg Tyr Phe
    1               5                   10                  15
   Phe Ser Phe Ala
               20
<210> 233
   <211> 21
   <212> PRT
   <213> Homo sapiens
<400> 233
   Phe Phe Ser Phe Ala Ala Asn Gly Arg Tyr Ser Leu Lys Val His Val
    1               5                   10                  15
   Asn His Ser Pro Ser
               20
<210> 234
   <211> 20
   <212> PRT
   <213> Homo sapiens
<400> 234
   Phe Leu Val Thr Trp Gln Ala Ser Gly Pro Pro Glu Ile Ile Leu Phe
   1 5 10 15
   Asp Pro Asp Gly
   20
<210> 235
   <211> 20
   <212> PRT
   <213> Homo sapiens
<400> 235
   Leu Gln Ser Ala Val Ser Asn Ile Ala Gln Ala Pro Leu Phe Ile Pro
   1 5 10 15
   Pro Asn Ser Asp
   20
<210> 236
   <211> 20
   <212> PRT
   <213> Homo sapiens
<400> 236
   Ile Gln Asp Asp Phe Asn Asn Ala Ile Leu Val Asn Thr Ser Lys Arg
   1 5 10 15
   Asn Pro Gln Gln
   20
<210> 237
   <211> 21
   <212> PRT
   <213> Homo sapiens
<400> 237
   Arg Asn Ser Leu Gln Ser Ala Val Ser Asn Ile Ala Gln Ala Pro Leu
   1 5 10 15
   Phe Ile Pro Pro Asn
   20
<210> 238
   <211> 20
   <212> PRT
   <213> Homo sapiens
<400> 238
   Thr His Glu Ser His Arg Ile Tyr Val Ala Ile Arg Ala Met Asp Arg
   1 5 10 15
   Asn Ser Leu Gln
   20
<210> 239
   <211> 20
   <212> PRT
   <213> Homo sapiens
<400> 239
   Arg Asn Pro Gln Gln Ala Gly Ile Arg Glu Ile Phe Thr Phe Ser Pro
   1 5 10 15
   Gln Ile Ser Thr
   20
<210> 240
   <211> 21
   <212> PRT
   <213> Homo sapiens
<400> 240
   Gly Gln Ala Thr Ser Tyr Glu Ile Arg Met Ser Lys Ser Leu Gln Asn
   1 5 10 15
   Ile Gln Asp Asp Phe
   20
<210> 241
   <211> 20
   <212> PRT
   <213> Homo sapiens
<400> 241
   Glu Arg Lys Trp Gly Phe Ser Arg Val Ser Ser Gly Gly Ser Phe Ser
   1 5 10 15
   Val Leu Gly Val
   20
<210> 242
   <211> 20
   <212> PRT
   <213> Homo sapiens
<400> 242
   Gly Ser His Ala Met Tyr Val Pro Gly Tyr Thr Ala Asn Gly Asn Ile
   1 5 10 15
   Gln Met Asn Ala
   20
<210> 243
   <211> 20
   <212> PRT
   <213> Homo sapiens
<400> 243
   Val Asn His Ser Pro Ser Ile Ser Thr Pro Ala His Ser Ile Pro Gly
    1               5                  10                  15
   Ser His Ala Met
               20
<210> 244
   <211> 20
   <212> PRT
   <213> Homo sapiens
<400> 244
   Ala Val Pro Pro Ala Thr Val Glu Ala Phe Val Glu Arg Asp Ser Leu
    1               5                  10                  15
   His Phe Pro His
               20
<210> 245
   <211> 20
   <212> PRT
   <213> Homo sapiens
<400> 245
   Lys Pro Gly His Trp Thr Tyr Thr Leu Asn Asn Thr His His Ser Leu
    1               5                  10                  15
   Gln Ala Leu Lys
               20
<210> 246
   <211> 20
   <212> PRT
   <213> Homo sapiens
<400> 246
   Asn Leu Thr Phe Arg Thr Ala Ser Leu Trp Ile Pro Gly Thr Ala Lys
    1               5                  10                  15
   Pro Gly His Trp
               20
<210> 247
   <211> 20
   <212> PRT
   <213> Homo sapiens
<400> 247
   Leu His Phe Pro His Pro Val Met Ile Tyr Ala Asn Val Lys Gln Gly
   1 5 10 15
   Phe Tyr Pro Ile
   20
<210> 248
   <211> 20
   <212> PRT
   <213> Homo sapiens
<400> 248
   Pro Glu Thr Gly Asp Pro Val Thr Leu Arg Leu Leu Asp Asp Gly Ala
   1 5 10 15
   Gly Ala Asp Val
   20
<210> 249
   <211> 20
   <212> PRT
   <213> Homo sapiens
<400> 249
   Gly Phe Tyr Pro Ile Leu Asn Ala Thr Val Thr Ala Thr Val Glu Pro
   1 5 10 15
   Glu Thr Gly Asp
   20
<210> 250
   <211> 20
   <212> PRT
   <213> Homo sapiens
<400> 250
   Phe Asp Pro Asp Gly Arg Lys Tyr Tyr Thr Asn Asn Phe Ile Thr Asn
   1 5 10 15
   Leu Thr Phe Arg
   20
<210> 251
   <211> 20
   <212> PRT
   <213> Homo sapiens
<400> 251
   Leu Gln Ala Leu Lys Val Thr Val Thr Ser Arg Ala Ser Asn Ser Ala
   1 5 10 15
   Val Pro Pro Ala
   20
<210> 252
   <211> 153
   <212> PRT
   <213> Homo sapiens
<400> 252
   Met Ala Ser Val Arg Val Ala Ala Tyr Phe Glu Asn Phe Leu Ala Ala
    1               5                  10                  15
   Trp Arg Pro Val Lys Ala Ser Asp Gly Asp Tyr Tyr Thr Leu Ala Val
               20                  25                  30
   Pro Met Gly Asp Val Pro Met Asp Gly Ile Ser Val Ala Asp Ile Gly
           35                  40                  45
   Ala Ala Val Ser Ser Ile Phe Asn Ser Pro Glu Glu Phe Leu Gly Lys
       50                  55                  60
   Ala Val Gly Leu Ser Ala Glu Ala Leu Thr Ile Gln Gln Tyr Ala Asp
   65                  70                  75                  80
   Val Leu Ser Lys Ala Leu Gly Lys Glu Val Arg Asp Ala Lys Ile Thr
                   85                  90                  95
   Pro Glu Ala Phe Glu Lys Leu Gly Phe Pro Ala Ala Lys Glu Ile Ala
               100                 105                 110
   Asn Met Cys Arg Phe Tyr Glu Met Lys Pro Asp Arg Asp Val Asn Leu
           115                 120                 125
   Thr His Gln Leu Asn Pro Lys Val Lys Ser Phe Ser Gln Phe Ile Ser
       130                 135                 140
   Glu Asn Gln Gly Ala Phe Lys Gly Met
   145                 150
<210> 253
   <211> 462
   <212> DNA
   <213> Homo sapiens
<400> 253
   atggccagtg tccgcgtggc ggcctacttt gaaaactttc tcgcggcgtg gcggcccgtg 60
   aaagcctctg atggagatta ctacaccttg gctgtaccga tgggagatgt accaatggat 120
   ggtatctctg ttgctgatat tggagcagcc gtctctagca tttttaattc tccagaggaa 180
   tttttaggca aggccgtggg gctcagtgca gaagcactaa caatacagca atatgctgat 240
   gttttgtcca aggctttggg gaaagaagtc cgagatgcaa agattacccc ggaagctttc 300
   gagaagctgg gattccctgc agcaaaggaa atagccaata tgtgtcgttt ctatgaaatg 360
   aagccagacc gagatgtcaa tctcacccac caactaaatc ccaaagtcaa aagcttcagc 420
   cagtttatct cagagaacca gggagccttc aagggcatgt ag                    462
<210> 254
   <211> 8031
   <212> DNA
   <213> Homo sapiens
<400> 254
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg aattaattct tagaaaaact catcgagcat caaatgaaac tgcaatttat 600
   tcatatcagg attatcaata ccatattttt gaaaaagccg tttctgtaat gaaggagaaa 660
   actcaccgag gcagttccat aggatggcaa gatcctggta tcggtctgcg attccgactc 720
   gtccaacatc aatacaacct attaatttcc cctcgtcaaa aataaggtta tcaagtgaga 780
   aatcaccatg agtgacgact gaatccggtg agaatggcaa aagtttatgc atttctttcc 840
   agacttgttc aacaggccag ccattacgct cgtcatcaaa atcactcgca tcaaccaaac 900
   cgttattcat tcgtgattgc gcctgagcga gacgaaatac gcgatcgctg ttaaaaggac 960
   aattacaaac aggaatcgaa tgcaaccggc gcaggaacac tgccagcgca tcaacaatat 1020
   tttcacctga atcaggatat tcttctaata cctggaatgc tgttttcccg gggatcgcag 1080
   tggtgagtaa ccatgcatca tcaggagtac ggataaaatg cttgatggtc ggaagaggca 1140
   taaattccgt cagccagttt agtctgacca tctcatctgt aacatcattg gcaacgctac 1200
   ctttgccatg tttcagaaac aactctggcg catcgggctt cccatacaat cgatagattg 1260
   tcgcacctga ttgcccgaca ttatcgcgag cccatttata cccatataaa tcagcatcca 1320
   tgttggaatt taatcgcggc ctagagcaag acgtttcccg ttgaatatgg ctcataacac 1380
   cccttgtatt actgtttatg taagcagaca gttttattgt tcatgaccaa aatcccttaa 1440
   cgtgagtttt cgttccactg agcgtcagac cccgtagaaa agatcaaagg atcttcttga 1500
   gatccttttt ttctgcgcgt aatctgctgc ttgcaaacaa aaaaaccacc gctaccagcg 1560
   gtggtttgtt tgccggatca agagctacca actctttttc cgaaggtaac tggcttcagc 1620
   agagcgcaga taccaaatac tgtccttcta gtgtagccgt agttaggcca ccacttcaag 1680
   aactctgtag caccgcctac atacctcgct ctgctaatcc tgttaccagt ggctgctgcc 1740
   agtggcgata agtcgtgtct taccgggttg gactcaagac gatagttacc ggataaggcg 1800
   cagcggtcgg gctgaacggg gggttcgtgc acacagccca gcttggagcg aacgacctac 1860
   accgaactga gatacctaca gcgtgagcta tgagaaagcg ccacgcttcc cgaagggaga 1920
   aaggcggaca ggtatccggt aagcggcagg gtcggaacag gagagcgcac gagggagctt 1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatataca tatgcagcat caccaccatc accacggagt 5100
   acagcttcaa gacaatgggt ataatggatt gctcattgca attaatcctc aggtacctga 5160
   gaatcagaac ctcatctcaa acattaagga aatgataact gaagcttcat tttacctatt 5220
   taatgctacc aagagaagag tatttttcag aaatataaag attttaatac ctgccacatg 5280
   gaaagctaat aataacagca aaataaaaca agaatcatat gaaaaggcaa atgtcatagt 5340
   gactgactgg tatggggcac atggagatga tccatacacc ctacaataca gagggtgtgg 5400
   aaaagaggga aaatacattc atttcacacc taatttccta ctgaatgata acttaacagc 5460
   tggctacgga tcacgaggcc gagtgtttgt ccatgaatgg gcccacctcc gttggggtgt 5520
   gttcgatgag tataacaatg acaaaccttt ctacataaat gggcaaaatc aaattaaagt 5580
   gacaaggtgt tcatctgaca tcacaggcat ttttgtgtgt gaaaaaggtc cttgccccca 5640
   agaaaactgt attattagta agctttttaa agaaggatgc acctttatct acaatagcac 5700
   ccaaaatgca actgcatcaa taatgttcat gcaaagttta tcttctgtgg ttgaattttg 5760
   taatgcaagt acccacaacc aagaagcacc aaacctacag aaccagatgt gcagcctcag 5820
   aagtgcatgg gatgtaatca cagactctgc tgactttcac cacagctttc ccatgaacgg 5880
   gactgagctt ccacctcctc ccacattctc gcttgtagag gctggtgaca aagtggtctg 5940
   tttagtgctg gatgtgtcca gcaagatggc agaggctgac agactccttc aactacaaca 6000
   agccgcagaa ttttatttga tgcagattgt tgaaattcat accttcgtgg gcattgccag 6060
   tttcgacagc aaaggagaga tcagagccca gctacaccaa attaacagca atgatgatcg 6120
   aaagttgctg gtttcatatc tgcccaccac tgtatcagct aaaacagaca tcagcatttg 6180
   ttcagggctt aagaaaggat ttgaggtggt tgaaaaactg aatggaaaag cttatggctc 6240
   tgtgatgata ttagtgacca gcggagatga taagcttctt ggcaattgct tacccactgt 6300
   gctcagcagt ggttcaacaa ttcactccat tgccctgggt tcatctgcag ccccaaatct 6360
   ggaggaatta tcacgtctta caggaggttt aaagttcttt gttccagata tatcaaactc 6420
   caatagcatg attgatgctt tcagtagaat ttcctctgga actggagaca ttttccagca 6480
   acatattcag cttgaaagta caggtgaaaa tgtcaaacct caccatcaat tgaaaaacac 6540
   agtgactgtg gataatactg tgggcaacga cactatgttt ctagttacgt ggcaggccag 6600
   tggtcctcct gagattatat tatttgatcc tgatggacga aaatactaca caaataattt 6660
   tatcaccaat ctaacttttc ggacagctag tctttggatt ccaggaacag ctaagcctgg 6720
   gcactggact tacaccctga acaataccca tcattctctg caagccctga aagtgacagt 6780
   gacctctcgc gcctccaact cagctgtgcc cccagccact gtggaagcct ttgtggaaag 6840
   agacagcctc cattttcctc atcctgtgat gatttatgcc aatgtgaaac agggatttta 6900
   tcccattctt aatgccactg tcactgccac agttgagcca gagactggag atcctgttac 6960
   gctgagactc cttgatgatg gagcaggtgc tgatgttata aaaaatgatg gaatttactc 7020
   gaggtatttt ttctcctttg ctgcaaatgg tagatatagc ttgaaagtgc atgtcaatca 7080
   ctctcccagc ataagcaccc cagcccactc tattccaggg agtcatgcta tgtatgtacc 7140
   aggttacaca gcaaacggta atattcagat gaatgctcca aggaaatcag taggcagaaa 7200
   tgaggaggag cgaaagtggg gctttagccg agtcagctca ggaggctcct tttcagtgct 7260
   gggagttcca gctggccccc accctgatgt gtttccacca tgcaaaatta ttgacctgga 7320
   agctgtaaaa gtagaagagg aattgaccct atcttggaca gcacctggag aagactttga 7380
   tcagggccag gctacaagct atgaaataag aatgagtaaa agtctacaga atatccaaga 7440
   tgactttaac aatgctattt tagtaaatac atcaaagcga aatcctcagc aagctggcat 7500
   cagggagata tttacgttct caccccaaat ttccacgaat ggacctgaac atcagccaaa 7560
   tggagaaaca catgaaagcc acagaattta tgttgcaata cgagcaatgg ataggaactc 7620
   cttacagtct gctgtatcta acattgccca ggcgcctctg tttattcccc ccaattctga 7680
   tcctgtacct gccagagatt atcttatatt gaaaggagtt ttaacagcaa tgggtttgat 7740
   aggaatcatt tgccttatta tagttgtgac acatcatact ttaagcagga aaaagagagc 7800
   agacaagaaa gagaatggaa caaaattatt ataatgaatt ctgcagatat ccatcacact 7860
   ggcggccgct cgagcaccac caccaccacc actgagatcc ggctgctaac aaagcccgaa 7920
   aggaagctga gttggctgct gccaccgctg agcaataact agcataaccc cttggggcct 7980
   ctaaacgggt cttgaggggt tttttgctga aaggaggaac tatatccgga t          8031
<210> 255
   <211> 401
   <212> DNA
   <213> Homo sapiens
<220>
   <221> misc_feature
   <222> 9, 67, 247, 275, 277, 397
   <223> n = A,T,C or G
<400> 255
   gtggccagng actagaaggc gaggcgccgc gggaccatgg cggcggcggc ggacgagcgg 60
   agtccanagg acggagaaga cgaggaagag gaggagcagt tggttctggt ggaattatca 120
   ggaattattg attcagactt cctctcaaaa tgtgaaaata aatgcaaggt tttgggcatt 180
   gacactgaga ggcccattct gcaagtggac agctgtgtct ttgctgggga gtatgaagac 240
   actctangga cctgtgttat atttgaagaa aatgntnaac atgctgatac agaaggcaat 300
   aataaaacag tgctaaaata taaatgccat acaatgaaga agctcagcat gacaagaact 360
   ctcctgacag agaagaagga aggagaagaa aacatangtg g                     401
<210> 256
   <211> 401
   <212> DNA
   <213> Homo sapiens
<220>
   <221> misc_feature
   <222> 7, 37, 51, 79, 96, 98, 103, 104, 107, 116, 167, 181, 183, 194, 206, 276, 303, 307, 308, 310, 323, 332, 341, 353, 374, 376
   <223> n = A,T,C or G
<400> 256
   tggtggncct gggatgggga accgcggtgg cttccgngga ggtttcggca ntggcatccg 60
   gggccggggt cgcggccgng gacggggccg gggccnangc cgnnganctc gcggangcaa 120
   ggccgaggat aaggagtgga tgcccgtcac caacttgggc cgcttgncca aggacatgaa 180
   nancaagccc ctgnaggaga tctatntctt cttccctgcc ccattaagga atcaagagat 240
   catttgattt cttcctgggg gcctctctca aggatnaggt ttttgaagat tatgccagtg 300
   canaaannan accccgttgc ccngtccatc tncacccaac ncttccaagg gcnatttttg 360
   tttaggcctc attncngggg ggaaccttaa cccaatttgg g                     401
<210> 257
   <211> 401
   <212> DNA
   <213> Homo sapiens
<220>
   <221> misc_feature
   <222> 382, 387
   <223> n = A,T,C or G
<400> 257
   atgtatgtaa aacacttcat aaaatgtaaa gggctataac aaatatgtta taaagtgatt 60
   ctctcagccc tgaggtatac agaatcattt gcctcagact gctgttggat tttaaaattt 120
   ttaaaatatc tgctaagtaa tttgctatgt cttctcccac actatcaata tgcctgcttc 180
   taacaggctc cccactttct tttaatgtgc tgttatgagc tttggacatg agataaccgt 240
   gcctgttcag agtgtctaca gtaagagctg gacaaactct ggagggacac agtctttgag 300
   acagctcttt tggttgcttt ccacttttct gaaaggttca cagtaacctt ctagataata 360
   gaaactccca gttaaagcct angctancaa ttttttttag t                       401
<210> 258
   <211> 401
   <212> DNA
   <213> Homo sapiens
<400> 258
   ggagcgctag gtcggtgtac gaccgagatt agggtgcgtg ccagctccgg gaggccgcgg 60
   tgaggggccg ggcccaagct gccgacccga gccgatcgtc agggtcgcca gcgcctcagc 120
   tctgtggagg agcagcagta gtcggagggt gcaggatatt agaaatggct actccccagt 180
   caattttcat ctttgcaatc tgcattttaa tgataacaga attaattctg gcctcaaaaa 240
   gctactatga tatcttaggt gtgccaaaat cggcatcaga gcgccaaatc aagaaggcct 300
   ttcacaagtt ggccatgaag taccaccctg acaaaaataa gacccagatg ctgaagcaaa 360
   attcagagag attgcagaag catatgaaac actctcagat g                     401
<210> 259
   <211> 401
   <212> DNA
   <213> Homo sapiens
<400> 259
   attgggtttg gagggaggat gatgacagag gaatgccctt tggccatcac ggttttgatt 60
   ctccagaata ttgtgggttt gatcatcaat gcagtcatgt taggctgcat tttcatgaaa 120
   acagctcagg ctcacagaag ggcagaaact ttgattttca gccgccatgc tgtgattgcc 180
   gtccgaaatg gcaagctgtg cttcatgttc cgagtgggtg acctgaggaa aagcatgatc 240
   attagtgcct ctgtgcgcat ccaggtggtc aagaaaacaa ctacacctga aggggaggtg 300
   gttcctattc accaactgga cattcctgtt gataacccaa tcgagagcaa taacattttt 360
   ctggtggccc ctttgatcat ctgccacgtg attgacaagc g                     401
<210> 260
   <211> 363
   <212> DNA
   <213> Homo sapiens
<220>
   <221> misc_feature
   <222> 7, 9, 19, 41, 63, 73, 106, 111, 113, 116, 119, 156, 158, 162, 187, 247, 288, 289, 290, 292, 298, 299, 300, 340
   <223> n = A,T,C or G
<400> 260
   aggaganang gagggggana tgaataggga tggagaggga natagtggat gagcagggca 60
   canggagagg aancagaaag gagaggcaag acagggagac acacancaca nangangana 120
   caggtggggg ctggggtggg gcatggagag cctttnangt cncccaggcc accctgctct 180
   cgctggnctg ttgaaaccca ctccatggct tcctgccact gcagttgggc ccagggctgg 240
   cttattnctg gaatgcaagt ggctgtggct tggagcctcc cctctggnnn anggaaannn 300
   attgctccct tatctgcttg gaatatctga gtttttccan cccggaaata aaacacacac 360
   aca                                                               363
<210> 261
   <211> 401
   <212> DNA
   <213> Homo sapiens
<220>
   <221> misc_feature
   <222> 114, 152
   <223> n = A,T,C or G
<400> 261
   cggctctccg ccgctctccc ggggtttcgg ggcacttggg tcccacagtc tggtcctgct 60
   tcaccttccc ctgacctgag tagtcgccat ggcacaggtt ctcagaggca ctgngactga 120
   cttccctgga tttgatgagc gggctgatgc anaaactctt cggaaggcta tgaaaggctt 180
   gggcacagat gaggagagca tcctgactct gttgacatcc cgaagtaatg ctcagcgcca 240
   ggaaatctct gcagctttta agactctgtt tggcagggat cttctggatg acctgaaatc 300
   agaactaact ggaaaatttg aaaaattaat tgtggctctg atgaaaccct ctcggcttta 360
   tgatgcttat gaactgaaac atgccttgaa gggagctgga a                      401
<210> 262
   <211> 401
   <212> DNA
   <213> Homo sapiens
<220>
   <221> misc_feature
   <222> 7, 26, 258, 305, 358, 373, 374, 378
   <223> n = A,T,C or G
<400> 262
   agtctanaac atttctaata ttttgngctt tcatatatca aaggagatta tgtgaaacta 60
   tttttaaata ctgtaaagtg acatatagtt ataagatata tttctgtaca gtagagaaag 120
   agtttataac atgaagaata ttgtaccatt atacattttc attctcgatc tcataagaaa 180
   ttcaaaagaa taatgataga ggtgaaaata tgtttacttt ctctaaatca agcctagttg 240
   tcaactcaaa aattatgntg catagtttta ttttgaattt aggttttggg actacttttt 300
   tccancttca atgagaaaat aaaatctaca actcaggagt tactacagaa gttctaanta 360
   tttttttgct aannagcnaa aaatataaac atatgaaaat g                      401
<210> 263
   <211> 401
   <212> DNA
   <213> Homo sapiens
<220>
   <221> misc_feature
   <222> 232, 290, 304, 326, 383
   <223> n = A,T,C or G
<400> 263
   ctgtccgacc aagagaggcc ggccgagccc gaggcttggg cttttgcttt ctggcggagg 60
   gatctgcggc ggtttaggag gcggcgctga tcctgggagg aagaggcagc tacggcggcg 120
   gcggcggtgg cggctagggc ggcggcgaat aaaggggccg ccgccgggtg atgcggtgac 180
   cactgcggca ggcccaggag ctgagtgggc cccggccctc agcccgtccc gncggacccg 240
   ctttcctcaa ctctccatct tctcctgccg accgagatcg ccgaggcggn ctcaggctcc 300
   ctancccctt ccccgtccct tccccncccc cgtccccgcc ccgggggccg ccgccacccg 360
   cctcccacca tggctctgaa ganaatccac aaggaattga a                      401
<210> 264
   <211> 401
   <212> DNA
   <213> Homo sapiens
<400> 264
   aacaccagcc actccaggac ccctgaaggc ctctaccagg tcaccagtgt tctgcgccta 60
   aagccacccc ctggcagaaa cttcagctgt gtgttctgga atactcacgt gagggaactt 120
   actttggcca gcattgacct tcaaagtcag atggaaccca ggacccatcc aacttggctg 180
   cttcacattt tcatcccctc ctgcatcatt gctttcattt tcatagccac agtgatagcc 240
   ctaagaaaac aactctgtca aaagctgtat tcttcaaaag acacaacaaa aagacctgtc 300
   accacaacaa agagggaagt gaacagtgct gtgaatctga acctgtggtc ttgggagcca 360
   gggtgacctg atatgacatc taaagaagct tctggactct g 401
<210> 265
   <211> 271
   <212> DNA
   <213> Homo sapiens
<220>
   <221> misc_feature
   <222> 59
   <223> n = A,T,C or G
<400> 265
   gccacttcct gtggacatgg gcagagcgct gctgccagtt cctggtagcc ttgaccacna 60
   cgctgggggg tctttgtgat ggtcatgggt ctcatttgca cttgggggtg tgggattcaa 120
   gttagaagtt tctagatctg gccgggcgca gtggctcaca cctgtaatcc cagcacttta 180
   ggaggctgag gcaggcggat catgaggtca ggagatcgag accgtcctgg ctaacacagt 240
   gaaaccccgt ctctactaaa aatacaaaaa a 271
<210> 266
   <211> 401
   <212> DNA
   <213> Homo sapiens
<220>
   <221> misc_feature
   <222> 45
   <223> n = A,T,C or G
<400> 266
   attcataaat ttagctgaaa gatactgatt caatttgtat acagngaata taaatgagac 60
   gacagcaaaa ttttcatgaa atgtaaaata tttttatagt ttgttcatac tatatgaggt 120
   tctattttaa atgactttct ggattttaaa aaatttcttt aaatacaatc atttttgtaa 180
   tatttatttt atgcttatga tctagataat tgcagaatat cattttatct gactctgtct 240
   tcataagaga gctgtggccg aattttgaac atctgttata gggagtgatc aaattagaag 300
   gcaatgtgga aaaacaattc tgggaaagat ttctttatat gaagtccctg ccactagcca 360
   gccatcctaa ttgatgaaag ttatctgttc acaggcctgc a 401
<210> 267
   <211> 401
   <212> DNA
   <213> Homo sapiens
<220>
   <221> misc_feature
   <222> 116, 247, 277, 296, 307, 313, 322, 323, 336, 342, 355, 365, 377, 378, 397
   <223> n = A,T,C or G
<400> 267
   gaagaggcat cacctgatcc cggagacctt tggagttaag aggcggcgga agcgagggcc 60
   tgtggagtcg gatcctcttc ggggtgagcc agggtcggcg cgcgcggctg tctcanaact 120
   catgcagctg ttcccgcgag gcctgtttga ggacgcgctg ccgcccatcg tgctgaggag 180
   ccaggtgtac agccttgtgc ctgacaggac cgtggccgac cggcagctga aggagcttca 240
   agagcanggg gagacaaaat cgtccagctg ggcttcnact tggatgccca tggaanttat 300
   tctttcnctt ganggactta cnngggaccc aagaanccct tncaaggggc ccttngtgga 360
   tgggncccga aaccccnnta tttgcccttg ggggggncca a                    401
<210> 268
   <211> 223
   <212> DNA
   <213> Homo sapiens
<400> 268
   tcgccatgtt ggccaggctg gtcttgaact cctgacttta agtgatccac ccgcctcaac 60
   ctcccaaagt gctgggatta caggtgtgag ccaccgcgcc tggcctgata catactttta 120
   gaatcaagta gtcacgcact ttttctgttc atttttctaa aaagtaaata tacaaatgtt 180
   ttgttttttg ttttttttgt ttgtttgttt ctgttttttt ttt                     223
<210> 269
   <211> 401
   <212> DNA
   <213> Homo sapiens
<400> 269
   actatgtaaa ccacattgta ctttttttta ctttggcaac aaatatttat acatacaaga 60
   tgctagttca tttgaatatt tctcccaact tatccaagga tctccagctc taacaaaatg 120
   gtttattttt atttaaatgt caatagttgt tttttaaaat ccaaatcaga ggtgcaggcc 180
   accagttaaa tgccgtctat caggttttgt gccttaagag actacagagt caaagctcat 240
   ttttaaagga gtaggacaaa gttgtcacag gtttttgttg ttgtttttat tgcccccaaa 300
   attacatgtt aatttccatt tatatcaggg attctattta cttgaagact gtgaagttgc 360
   cattttgtct cattgttttc tttgacataa ctaggatcca t                    401
<210> 270
   <211> 401
   <212> DNA
   <213> Homo sapiens
<220>
   <221> misc_feature
   <222> 240, 382
   <223> n = A,T,C or G
<400> 270
   tggctgttga ttcacctcag cactgcttgg tatctgcacc ctacctctct ttagaggctg 60
   ccttgtcaac tgaaaaatgc acctgacttc gagcaagact ctttccttag gttctggatc 120
   tgtttgagcc ccatggcact gagctggaat ctgagggtct tgttccaagg atgtgatgat 180
   gtgggagaat gttctttgaa agagcagaaa tccagtctgc atggaaacag cctgtagagn 240
   agaagtttcc agtgataagt gttcactgtt ctaaggaggt acaccacagc tacctgaatt 300
   ttcccaaaat gagtgcttct gtgcgttaca actggccttt gtacttgact gtgatgactt 360
   tgttttttct tttcaattct anatgaacat gggaaaaaat g                     401
<210> 271
   <211> 329
   <212> DNA
   <213> Homo sapiens
<400> 271
   ccacagcctc caagtcaggt ggggtggagt cccagagctg cacagggttt ggcccaagtt 60
   tctaagggag gcacttcctc ccctcgccca tcagtgccag cccctgctgg ctggtgcctg 120
   agcccctcag acagccccct gccccgcagg cctgccttct cagggacttc tgcggggcct 180
   gaggcaagcc atggagtgag acccaggagc cggacacttc tcaggaaatg gcttttccca 240
   acccccagcc cccacccggt ggttcttcct gttctgtgac tgtgtatagt gccaccacag 300
   cttatggcat ctcattgagg acaaaaaaa                                   329
<210> 272
   <211> 401
   <212> DNA
   <213> Homo sapiens
<220>
   <221> misc_feature
   <222> 1, 7, 12, 21, 61, 62, 66, 72, 78, 88, 90, 92, 98, 117, 119, 128, 130, 134, 142, 144, 151, 159, 162, 164, 168, 169, 177, 184, 185, 188, 194, 202, 204, 209, 213, 218, 223, 231, 260, 272, 299, 300, 306, 321, 322, 323, 331, 335, 336, 338
   <223> n = A,T,C or G
<221> misc_feature
   <222> 341, 342, 343, 345, 346, 351, 358, 360, 362, 363, 387, 390, 392
   <223> n = A,T,C or G
<400> 272
   nggctgntaa cntcggaggt nacttcctgg actatcctgg agaccccctc cgcttccacg 60
   nncatnatat cnctcatngc tgggcccntn angacacnat cccactccaa cacctgngng 120
   atgctggncn cctnggaacc ancntcagaa ngaccctgnt cntntgtnnt ccgcaanctg 180
   aagnnaangc gggntacacc tncntgcant ggnccacnct gcngggaact ntacacacct 240
   acgggatgtg gctgcgccan gagccaagag cntttctgga tgattcccca gcctcttgnn 300
   agggantcta caacattgct nnntaccttt ntccnncngc nnntnntgga ntacaggngn 360
   tnntaacact acatcttttt tactgcnccn tncttggtgg g                     401
<210> 273
   <211> 401
   <212> DNA
   <213> Homo sapiens
<220>
   <221> misc_feature
   <222> 399
   <223> n = A,T,C or G
<400> 273
   cagcaccatg aagatcaaga tcatcgcacc cccagagcgc aagtactcgg tgtggatcgg 60
   tggctccatc ctggcctcac tgtccacctt ccagcagatg tggattagca agcaggagta 120
   cgacgagtcg ggcccctcca tcgtccaccg caaatgcttc taaacggact cagcagatgc 180
   gtagcatttg ctgcatgggt taattgagaa tagaaatttg cccctggcaa atgcacacac 240
   ctcatgctag cctcacgaaa ctggaataag ccttcgaaaa gaaattgtcc ttgaagcttg 300
   tatctgatat cagcactgga ttgtagaact tgttgctgat tttgaccttg tattgaagtt 360
   aactgttccc cttggtatta acgtgtcagg gctgagtgnt c                    401
<210> 274
   <211> 401
   <212> DNA
   <213> Homo sapiens
<400> 274
   ccacccacac ccaccgcgcc ctcgttcgcc tcttctccgg gagccagtcc gcgccaccgc 60
   cgccgcccag gccatcgcca ccctccgcag ccatgtccac caggtccgtg tcctcgtcct 120
   cctaccgcag gatgttcggc ggcccgggca ccgcgagccg gccgagctcc agccggagct 180
   acgtgactac gtccacccgc acctacagcc tgggcagcgc gctgcgcccc agcaccagcc 240
   gcagcctcta cgcctcgtcc ccgggcggcg tgtatgccac gcgctcctct gccgtgcgcc 300
   tgcggagcag cgtgcccggg gtgcggctcc tgcaggactc ggtggacttc tcgctggccg 360
   acgccatcaa caccgagttc aagaacaccc gcaccaacga g                    401
<210> 275
   <211> 401
   <212> DNA
   <213> Homo sapiens
<400> 275
   ccacttccac cactttgtgg agcagtgcct tcagcgcaac ccggatgcca ggtatccctg 60
   ctggcctggg cctgggcttc gggagagcag agggtgctca ggagggtaag gccagggtgt 120
   gaagggactt acctcccaaa ggttctgcag gggaatctgg agctacacac aggagggatc 180
   agctcctggg tgtgtcagag gccagcctgg ggagctctgg ccactgcttc ccatgagctg 240
   agggagaggg agaggggacc cgaggctgag gcataagtgg caggatttcg ggaagctggg 300
   gacacggcag tgatgctgcg gtctctcctc ccctttccct ccaggcccag tgccagcacc 360
   ctcctgaacc actctttctt caagcagatc aagcgacgtg c                    401
<210> 276
   <211> 401
   <212> DNA
   <213> Homo sapiens
<220>
   <221> misc_feature
   <222> 11
   <223> n = A,T,C or G
<400> 276
   tctgatattg ntacccttga gccacctaag ttagaagaaa ttggaaatca agaagttgtc 60
   attgttgaag aagcacagag ttcagaagac tttaacatgg gctcttcctc tagcagccag 120
   tatactttct gtcagccaga aactgtattt tcatctcagc ctagtgatga tgaatcaagt 180
   agtgatgaaa ccagtaatca gcccagtcct gcctttagac gacgccgtgc taggaagaag 240
   accgtttctg cttcagaatc tgaagaccgg ctagttggtg aacaagaaac tgaaccttct 300
   aaggagttga gtaaacgtca gttcagtagt ggtctcaata agtgtgttat acttgctttg 360
   gtgattgcaa tcagcatggg atttggccat ttctatggca c                     401
<210> 277
   <211> 401
   <212> DNA
   <213> Homo sapiens
<220>
   <221> misc_feature
   <222> 227, 333
   <223> n = A,T,C or G
<400> 277
   aactttggca acatatctca gcaaaaacta cagctatgtt attcatgcca aaataaaagc 60
   tgtgcagagg agtggctgca atgaggtcac aacggtggtg gatgtaaaag agatcttcaa 120
   gtcctcatca cccatccctc gaactcaagt cccgctcatt acaaattctt cttgccagtg 180
   tccacacatc ctgccccatc aagatgttct catcatgtgt tacgagnggc gctcaaggat 240
   gatgcttctt gaaaattgct tagttgaaaa atggagagat cagcttagta aaagatccat 300
   acagtgggaa gagaggctgc aggaacagcg ganaacagtt caggacaaga agaaaacagc 360
   cgggcgcacc agtcgtagta atccccccaa accaaaggga a                     401
<210> 278
   <211> 401
   <212> DNA
   <213> Homo sapiens
<220>
   <221> misc_feature
   <222> 322, 354
   <223> n = A,T,C or G
<400> 278
   aatgagtgtg agaccacaaa tgaatgccgg gaggatgaaa tgtgttggaa ttatcatggc 60
   ggcttccgtt gttatccacg aaatccttgt caagatccct acattctaac accagagaac 120
   cgatgtgttt gcccagtctc aaatgccatg tgccgagaac tgccccagtc aatagtctac 180
   aaatacatga gcatccgatc tgataggtct gtgccatcag acatcttcca gatacaggcc 240
   acaactattt atgccaacac catcaatact tttcggatta aatctggaaa tgaaaatgga 300
   gagtctacct acgacaacaa anccctgtaa gtgcaatgct tgtgctcgtg aagncattat 360
   caggaccaag agaacatatc gtggacctgg agatgctgac a                     401
<210> 279
   <211> 401
   <212> DNA
   <213> Homo sapiens
<220>
   <221> misc_feature
   <222> 30, 35, 81, 88, 180, 212, 378, 384, 391
   <223> n = A,T,C or G
<400> 279
   aaattattgc ctctgataca tacctaagtn aacanaacat taatacctaa gtaaacataa 60
   cattacttgg agggttgcag nttctaantg aaactgtatt tgaaactttt aagtatactt 120
   taggaaacaa gcatgaacgg cagtctagaa taccagaaac atctacttgg gtagcttggn 180
   gccattatcc tgtggaatct gatatgtctg gnagcatgtc attgatggga catgaagaca 240
   tctttggaaa tgatgagatt atttcctgtg ttaaaaaaaa aaaaaatctt aaattcctac 300
   aatgtgaaac tgaaactaat aattttgatc ctgatgtatg ggacagcgta tctgtaccag 360
   gctctaaata acaaaagnta gggngacaag nacatgttcc t                    401
<210> 280
   <211> 326
   <212> DNA
   <213> Homo sapiens
<400> 280
   gaagtggaat tgtataattc aattcgataa ttgatctcat gggctttccc tggaggaaag 60
   gttttttttg ttgttttttt tttaagaact tgaaacttgt aaactgagat gtctgtagct 120
   tttttgccca tctgtagtgt atgtgaagat ttcaaaacct gagagcactt tttctttgtt 180
   tagaattatg agaaaggcac tagatgactt taggatttgc atttttccct ttattgcctc 240
   atttcttgtg acgccttgtt ggggagggaa atctgtttat tttttcctac aaataaaaag 300
   ctaagattct atatcgcaaa aaaaaa                                      326
<210> 281
   <211> 374
   <212> DNA
   <213> Homo sapiens
<400> 281
   caacgcgttt gcaaatattc ccctggtagc ctacttcctt acccccgaat attggtaaga 60
   tcgagcaatg gcttcaggac atgggttctc ttctcctgtg atcattcaag tgctcactgc 120
   atgaagactg gcttgtctca gtgtttcaac ctcaccaggg ctgtctcttg gtccacacct 180
   cgctccctgt tagtgccgta tgacagcccc catcaaatga ccttggccaa gtcacggttt 240
   ctctgtggtc aaggttggtt ggctgattgg tggaaagtag ggtggaccaa aggaggccac 300
   gtgagcagtc agcaccagtt ctgcaccagc agcgcctccg tcctagtggg tgttcctgtt 360
   tctcctggcc ctgg                                                   374
<210> 282
   <211> 404
   <212> DNA
   <213> Homo sapiens
<220>
   <221> misc_feature
   <222> 26, 27, 51, 137, 180, 222
   <223> n = A,T,C or G
<400> 282
   agtgtggtgg aattcccgca tcctanncgc cgactcacac aaggcagagt ngccatggag 60
   aaaattccag tgtcagcatt cttgctcctt gtggccctct cctacactct ggccagagat 120
   accacagtca aacctgnagc caaaaaggac acaaaggact ctcgacccaa actgccccan 180
   accctctcca gaggttgggg tgaccaactc atctggactc anacatatga agaagctcta 240
   tataaatcca agacaagcaa caaacccttg atgattattc atcacttgga tgagtgccca 300
   cacagtcaag ctttaaagaa agtgtttgct gaaaataaag aaatccagaa attggcagag 360
   cagtttgtcc tcctcaatct ggtttatgaa acaactgaca aaca 404
<210> 283
   <211> 184
   <212> DNA
   <213> Homo sapiens
<220>
   <221> misc_feature
   <222> 26
   <223> n = A,T,C or G
<400> 283
   agtgtggtgg aattcacttg cttaanttgt gggcaaaaga gaaaaagaag gattgatcag 60
   agcattgtgc aatacagttt cattaactcc ttccctcgct cccccaaaaa tttgaatttt 120
   tttttcaaca ctcttacacc tgttatggaa aatgtcaacc tttgtaagaa aaccaaaata 180
   aaaa 184
<210> 284
   <211> 421
   <212> DNA
   <213> Homo sapiens
<220>
   <221> misc_feature
   <222> 147, 149
   <223> n = A,T,C or G
<400> 284
   ctattaatcc tgccacaata tttttaatta cgtacaaaga tctgacatgt cacccaggga 60
   cccatttcac ccactgctct gtttggccgc cagtcttttg tctctctctt cagcaatggt 120
   gaggcggata ccctttcctc ggggaanana aatccatggt ttgttgccct tgccaataac 180
   aaaaatgttg gaaagtcgag tggcaaagct gttgccattg gcatctttca cgtgaaccac 240
   gtcaaaagat ccagggtgcc tctctctgtt ggtgatcaca ccaattcttc ctaggttagc 300
   acctccagtc accatacaca ggttaccagt gtcgaacttg atgaaatcag taatcttgcc 360
   agtctctaaa tcaatctgaa tggtatcatt caccttgatg aggggatcgg ggtagcggat 420
   g 421
<210> 285
   <211> 361
   <212> DNA
   <213> Homo sapiens
<220>
   <221> misc_feature
   <222> 34, 188
   <223> n = A,T,C or G
<400> 285
   ctgggtggta actctttatt tcattgtccg gaanaaagat gggagtggga acagggtgga 60
   cactgtgcag gcttcagctt ccactccggg caggattcag gctatctggg accgcaggga 120
   ctgccaggtg cacagccctg gctcccgagg caggcaggca aggtgacggg actggaagcc 180
   cttttcanag ccttggagga gctggtccgt ccacaagcaa tgagtgccac tctgcagttt 240
   gcaggggatg gataaacagg gaaacactgt gcattcctca cagccaacag tgtaggtctt 300
   ggtgaagccc cggcgctgag ctaagctcag gctgttccag ggagccacga aactgcaggt 360
   a                                                                 361
<210> 286
   <211> 336
   <212> DNA
   <213> Homo sapiens
<220>
   <221> misc_feature
   <222> 40, 68, 75, 127, 262
   <223> n = A,T,C or G
<400> 286
   tttgagtggc agcgccttta tttgtggggg ccttcaaggn agggtcgtgg ggggcagcgg 60
   ggaggaanag ccganaaact gtgtgaccgg ggcctcaggt ggtgggcatt gggggctcct 120
   cttgcanatg cccattggca tcaccggtgc agccattggt ggcagcgggt accggtcctt 180
   tcttgttcaa catagggtag gtggcagcca cgggtccaac tcgcttgagg ctgggccctg 240
   ggcgctccat tttgtgttcc angagcatgt ggttctgtgg cgggagcccc acgcaggccc 300
   tgaggatgtt ctcgatgcag ctgcgctggc ggaaaa                           336
<210> 287
   <211> 301
   <212> DNA
   <213> Homo sapiens
<220>
   <221> misc_feature
   <222> 15, 33, 44, 53, 76, 83, 107, 117, 154, 166, 192, 194, 207, 215, 241, 246
   <223> n = A,T,C or G
<400> 287
   tgggtaccaa atttntttat ttgaaggaat ggnacaaatc aaanaactta agnggatgtt 60
   ttggtacaac ttatanaaaa ggnaaaggaa accccaacat gcatgcnctg ccttggngac 120
   cagggaagtc accccacggc tatggggaaa ttancccgag gcttancttt cattatcact 180
   gtctcccagg gngngcttgt caaaaanata ttccnccaag ccaaattcgg gcgctcccat 240
   nttgcncaag ttggtcacgt ggtcacccaa ttctttgatg gctttcacct gctcattcag 300
   g                                                                 301
<210> 288
   <211> 358
   <212> DNA
   <213> Homo sapiens
<220>
   <221> misc_feature
   <222> 39, 143, 226
   <223> n = A,T,C or G
<400> 288
   aagtttttaa actttttatt tgcatattaa aaaaattgng cattccaata attaaaatca 60
   tttgaacaaa aaaaaaaatg gcactctgat taaactgcat tacagcctgc aggacacctt 120
   gggccagctt ggttttactc tanatttcac tgtcgtccca ccccacttct tccaccccac 180
   ttcttccttc accaacatgc aagttctttc cttccctgcc agccanatag atagacagat 240
   gggaaaggca ggcgcggcct tcgttgtcag tagttctttg atgtgaaagg ggcagcacag 300
   tcatttaaac ttgatccaac ctctttgcat cttacaaagt taaacagcta aaagaagt   358
<210> 289
   <211> 462
   <212> DNA
   <213> Homo sapiens
<220>
   <221> misc_feature
   <222> 87, 141, 182, 220, 269, 327
   <223> n = A,T,C or G
<400> 289
   ggcatcagaa atgctgttta tttctctgct gctcccaagc tggctggcct ttgcagagga 60
   gcagacaaca gatgcatagt tgggganaaa gggaggacag gttccaggat agagggtgca 120
   ggctgaggga ggaagggtaa naggaaggaa ggccatcctg gatccccaca tttcagtctc 180
   anatgaggac aaagggactc ccaagccccc aaatcatcan aaaacaccaa ggagcaggag 240
   gagcttgagc aggccccagg gagcctcana gccataccag ccactgtcta cttcccatcc 300
   tcctctccca ttccctgtct gcttcanacc acctcccagc taagccccag ctccattccc 360
   ccaatcctgg cccttgccag cttgacagtc acagtgcctg gaattccacc actgaggctt 420
   ctcccagttg gattaggacg tcgccctgtt agcatgctgc cc                   462
<210> 290
   <211> 481
   <212> DNA
   <213> Homo sapiens
<220>
   <221> misc_feature
   <222> 44, 57, 122, 158, 304, 325, 352, 405
   <223> n = A,T,C or G
<400> 290
   tactttccta aactttatta aagaaaaaag caataagcaa tggnggtaaa tctctanaac 60
   atacccaatt ttctgggctt cctcccccga gaatgtgaca ttttgatttc caaacatgcc 120
   anaagtgtat ggttcccaac tgtactaaag taggtganaa gctgaagtcc tcaagtgttc 180
   atcttccaac ttttcccagt ctgtggtctg tctttggatc agcaataatt gcctgaacag 240
   ctactatggc ttcgttgatt tttgtctgta gctctctgag ctcctctatg tgcagcaatc 300
   gcanaatttg agcagcttca ttaanaactg catctcctgt gtcaaaacca anaatatgtt 360
   tgtctaaagc aacaggtaag ccctcttttg tttgatttgc cttancaact gcatcctgtg 420
   tcaggcgctc ctgaaccaaa atccgaattg ccttaagcat taccaggtaa tcatcatgac 480
   g                                                                 481
<210> 291
   <211> 381
   <212> DNA
   <213> Homo sapiens
<220>
   <221> misc_feature
   <222> 79, 166, 187, 208, 219, 315
   <223> n = A,T,C or G
<400> 291
   tcatagtaat gtaaaaccat ttgtttaatt ctaaatcaaa tcactttcac aacagtgaaa 60
   attagtgact ggttaaggng tgccactgta catatcatca ttttctgact ggggtcagga 120
   cctggtccta gtccacaagg gtggcaggag gagggtggag gctaanaaca cagaaaacac 180
   acaaaanaaa ggaaagctgc cttggcanaa ggatgaggng gtgagcttgc cgaaggatgg 240
   tgggaagggg gctccctgtt ggggccgagc caggagtccc aagtcagctc tcctgcctta 300
   cttagctcct ggcanagggt gagtggggac ctacgaggtt caaaatcaaa tggcatttgg 360
   ccagcctggc tttactaaca g                                           381
<210> 292
   <211> 371
   <212> DNA
   <213> Homo sapiens
<220>
   <221> misc_feature
   <222> 32, 55, 72, 151, 189, 292
   <223> n = A,T,C or G
<400> 292
   gaaaaaataa tccgtttaat tgaaaaacct gnaggatact attccactcc cccanatgag 60
   gaggctgagg anaccaaacc cctacatcac ctcgtagcca cttctgatac tcttcacgag 120
   gcagcaggca aagacaattc ccaaaacctc nacaaaagca attccaaggg ctgctgcagc 180
   taccaccanc acatttttcc tcagccagcc cccaatcttc tccacacagc cctccttatg 240
   gatcgccttc tcgttgaaat taatcccaca gcccacagta acattaatgc ancaggagtc 300
   ggggactcgg ttcttcgaca tggaagggat tttctcccaa tctgtgtagt tagcagcccc 360
   acagcactta a                                                      371
<210> 293
   <211> 361
   <212> DNA
   <213> Homo sapiens
<220>
   <221> misc_feature
   <222> 75, 196, 222
   <223> n = A,T,C or G
<400> 293
   gatttaaaag aaaacacttt attgttcagc aattaaaagt tagccaaata tgtatttttc 60
   tccataattt attgngatgt tatcaacatc aagtaaaatg ctcattttca tcatttgctt 120
   ctgttcatgt tttcttgaac acgtcttcaa ttttccttcc aaaatgctgc atgccacact 180
   tgaggtaacg aagcanaagt atttttaaac atgacagcta anaacattca tctacagcaa 240
   cctatatgct caatacatgc cgcgtgatcc tagtagtttt ttcacaacct tctacaagtt 300
   tttggaaaac atctgttatg atgactttca tacaccttca cctcaaaggc tttcttgcac 360
   c                                                                 361
<210> 294
   <211> 391
   <212> DNA
   <213> Homo sapiens
<220>
   <221> misc_feature
   <222> 26, 77, 96, 150, 203, 252, 254, 264, 276
   <223> n = A,T,C or G
<400> 294
   tattttaaag tttaattatg attcanaaaa aatcgagcga ataactttct ctgaaaaaat 60
   atattgactc tgtatanacc acagttattg gggganaagg gctggtaggt taaattatcc 120
   tattttttat tctgaaaatg atattaatan aaagtcccgt ttccagtctg attataaaga 180
   tacatatgcc caaaatggct ganaataaat acaacaggaa atgcaaaagc tgtaaagcta 240
   agggcatgca ananaaaatc tcanaatacc caaagnggca acaaggaacg tttggctgga 300
   atttgaagtt atttcagtca tctttgtctt tggctccatg tttcaggatg cgtgtgaact 360
   cgatgtaatt gaaattcccc tttttatcaa t                                391
<210> 295
   <211> 343
   <212> DNA
   <213> Homo sapiens
<220>
   <221> misc_feature
   <222> 145, 174, 205, 232
   <223> n = A,T,C or G
<400> 295
   ttcttttgtt ttattgataa cagaaactgt gcataattac agatttgatg aggaatctgc 60
   aaataataaa gaatgtgtct actgccagca aaatacaatt attccatgcc ctctcaacat 120
   acaaatatag agttcttcac accanatggc tctggtgtaa caaagccatt ttanatgttt 180
   aattgtgctt ctacaaaacc ttcanagcat gaggtagttt cttttaccta cnatattttc 240
   cacatttcca ttattacact tttagtgagc taaaatcctt ttaacatagc ctgcggatga 300
   tctttcacaa aagccaagcc tcatttacaa agggtttatt tct                   343
<210> 296
   <211> 241
   <212> DNA
   <213> Homo sapiens
<220>
   <221> misc_feature
   <222> 96, 98, 106, 185
   <223> n = A,T,C or G
<400> 296
   ttcttggata ttggttgttt ttgtgaaaaa gtttttgttt ttcttctcag tcaactgaat 60
   tatttctcta ctttgccctc ctgatgccca catgananaa cttaanataa tttctaacag 120
   cttccacttt ggaaaaaaaa aaaacctgtt ttcctcatgg aaccccagga gttgaaagtg 180
   gatanatcgc tctcaaaatc taaggctctg ttcagcttta cattatgtta cctgacgttt 240
   t                                                                 241
<210> 297
   <211> 391
   <212> DNA
   <213> Homo sapiens
<220>
   <221> misc_feature
   <222> 12, 130
   <223> n = A,T,C or G
<400> 297
   gttgtggctg anaatgctgg agatgctcag ttctctccct cacaaggtag gccacaaatt 60
   cttggtggtg ccctcacatc tggggtcttc aggcaccagc catgcctgcc gaggagtgct 120
   gtcaggacan accatgtccg tgctaggccc aggcacagcc caaccactcc tcatccaagt 180
   ctctcccagg tttctggtcc cgatgggcaa ggatgacccc tccagtggct ggtaccccac 240
   catcccacta cccctcacat gctctcactc tccatcaggt ccccaatcct ggcttccctc 300
   ttcacgaact ctcaaagaaa aggaaggata aaacctaaat aaaccagaca gaagcagctc 360
   tggaaaagta caaaaagaca gccagaggtg t                                391
<210> 298
   <211> 321
   <212> DNA
   <213> Homo sapiens
<220>
   <221> misc_feature
   <222> 14, 30, 76, 116, 201, 288, 301
   <223> n = A,T,C or G
<400> 298
   caagccaaac tgtntccagc tttattaaan atactttcca taaacaatca tggtatttca 60
   ggcaggacat gggcanacaa tcgttaacag tatacaacaa ctttcaaact cccttnttca 120
   atggactacc aaaaatcaaa aagccactat aaaacccaat gaagtcttca tctgatgctc 180
   tgaacaggga aagtttaaag ngagggttga catttcacat ttagcatgtt gtttaacaac 240
   ttttcacaag ccgaccctga ctttcaggaa gtgaaatgaa aatggcanaa tttatctgaa 300
   natccacaat ctaaaaatgg a                                           321
<210> 299
   <211> 401
   <212> DNA
   <213> Homo sapiens
<220>
   <221> misc_feature
   <222> 104, 268, 347
   <223> n = A,T,C or G
<400> 299
   tatcataaag agtgttgaag tttatttatt atagcaccat tgagacattt tgaaattgga 60
   attggtaaaa aaataaaaca aaaagcattt gaattgtatt tggnggaaca gcaaaaaaag 120
   agaagtatca tttttctttg tcaaattata ctgtttccaa acattttgga aataaataac 180
   tggaattttg tcggtcactt gcactggttg acaagattag aacaagagga acacatatgg 240
   agttaaattt tttttgttgg gatttcanat agagtttggt ttataaaaag caaacagggc 300
   caacgtccac accaaattct tgatcaggac caccaatgtc atagggngca atatctacaa 360
   taggtagtct cacagccttg cgtgttcgat attcaaagac t                      401
<210> 300
   <211> 188
   <212> DNA
   <213> Homo sapiens
<220>
   <221> misc_feature
   <222> 48
   <223> n = A,T,C or G
<400> 300
   tgaatgcttt gtcatattaa gaaagttaaa gtgcaataat gtttgaanac aataagtggt 60
   ggtgtatctt gtttctaata agataaactt ttttgtcttt gctttatctt attagggagt 120
   tgtatgtcag tgtataaaac atactgtgtg gtataacagg cttaataaat tctttaaaag 180
   gaaaaaaa                                                          188
<210> 301
   <211> 291
   <212> DNA
   <213> Homo sapiens
<400> 301
   aagattttgt tttattttat tatggctaga aagacactgt tatagccaaa atcggcaatg 60
   acactaaaga aatcctctgt gcttttcaat atgcaaatat atttcttcca agagttgccc 120
   tggtgtgact tcaagagttc atgttaactt cttttctgga aacttccttt tcttagttgt 180
   tgtattcttg aagagcctgg gccatgaaga gcttgcctaa gttttgggca gtgaactcct 240
   tgatgttctg gcagtaagtg tttatctggc ctgcaatgag cagcgagtcc a          291
<210> 302
   <211> 341
   <212> DNA
   <213> Homo sapiens
<220>
   <221> misc_feature
   <222> 25
   <223> n = A,T,C or G
<400> 302
   tgatttttca taattttatt aaatnatcac tgggaaaact aatggttcgc gtatcacaca 60
   attacactac aatctgatag gagtggtaaa accagccaat ggaatccagg taaagtacaa 120
   aaacgccacc ttttattgtc ctgtcttatt tctcgggaag gagggttcta ctttacacat 180
   ttcatgagcc agcagtggac ttgagttaca atgtgtaggt tccttgtggt tatagctgca 240
   gaagaagcca tcaaattctt gaggacttga catctctcgg aaagaagcaa actagtggat 300
   cccccgggct gcaggaattc gatatcaagc ttatcgatac c                     341
<210> 303
   <211> 361
   <212> DNA
   <213> Homo sapiens
<220>
   <221> misc_feature
   <222> 15, 27, 92, 124, 127, 183, 198, 244, 320
   <223> n = A,T,C or G
<400> 303
   tgcagacagt aaatnaattt tatttgngtt cacagaacat actaggcgat ctcgacagtc 60
   gctccgtgac agcccaccaa cccccaaccc tntacctcgc agccacccta aaggcgactt 120
   caanaanatg gaaggatctc acggatctca ttcctaatgg tccgccgaag tctcacacag 180
   tanacagacg gagttganat gctggaggat gcagtcacct cctaaactta cgacccacca 240
   ccanacttca tcccagccgg gacgtcctcc cccacccgag tcctccccat ttcttctcct 300
   actttgccgc agttccaggn gtcctgcttc caccagtccc acaaagctca ataaatacca 360
   a                                                                 361
<210> 304
   <211> 301
   <212> DNA
   <213> Homo sapiens
<220>
   <221> misc_feature
   <222> 23, 104, 192
   <223> n = A,T,C or G
<400> 304
   ctctttacaa cagcctttat ttncggccct tgatcctgct cggatgctgg tggaggccct 60
   tagctccgcc cgccaggctc tgtgccgcct ccccgcaggc gcanattcat gaacacggtg 120
   ctcaggggct tgaggccgta ctcccccagc gggagctggt cctccagggg cttcccctcg 180
   aaggtcagcc anaacaggtc gtcctgcaca ccctccagcc cgctcacttg ctgcttcagg 240
   tgggccacgg tctgcgtcag ccgcacctcg taggtgctgc tgcggccctt gttattcctc 300
   a                                                                 301
<210> 305
   <211> 331
   <212> DNA
   <213> Homo sapiens
<220>
   <221> misc_feature
   <222> 3, 36, 60, 193, 223
   <223> n = A,T,C or G
<400> 305
   ganaggctag taacatcagt tttattgggt tggggnggca accatagcct ggctgggggn 60
   ggggctggcc ctcacaggtt gttgagttcc agcagggtct ggtccaaggt ctggtgaatc 120
   tcgacgttct cctccttggc actggccaag gtctcttcta ggtcatcgat ggttttctcc 180
   aactttgcca canacctctc ggcaaactct gctcgggtct cancctcctt cagcttctcc 240
   tccaacagtt tgatctcctc ttcatattta tcttctttgg gggaatactc ctcctctgag 300
   gccatcaggg acttgagggc ctggtccatg g                                331
<210> 306
   <211> 457
   <212> DNA
   <213> Homo sapiens
<400> 306
   aatatgtaaa ggtaataact tttattatat taaagacaat gcaaacgaaa aacagaattg 60
   agcagtgcaa aatttaaagg actgttttgt tctcaaagtt gcaagtttca aagccaaaag 120
   aattatatgt atcaaatata taagtaaaaa aaagttagac tttcaagcct gtaatcccag 180
   cactttggga ggctgaggca ggtggatcac taacattaaa aagacaacat tagattttgt 240
   cgatttatag caattttata aatatataac tttgtcactt ggatcctgaa gcaaaataat 300
   aaagtgaatt tgggattttt gtacttggta aaaagtttaa caccctaaat tcacaactag 360
   tggatccccc gggctgcagg aattcgatat caagcttatc gataccgtcg acctcgaggg 420
   ggggcccggt acccaattcg ccctatagtg agtcgta                          457
<210> 307
   <211> 491
   <212> DNA
   <213> Homo sapiens
<400> 307
   gtgcttggac ggaacccggc gctcgttccc caccccggcc ggccgcccat agccagccct 60
   ccgtcacctc ttcaccgcac cctcggactg ccccaaggcc cccgccgccg ctccagcgcc 120
   gcgcagccac cgccgccgcc gccgcctctc cttagtcgcc gccatgacga ccgcgtccac 180
   ctcgcaggtg cgccagaact accaccagga ctcagaggcc gccatcaacc gccagatcaa 240
   cctggagctc tacgcctcct acgtttacct gtccatgtct tactactttg accgcgatga 300
   tgtggctttg aagaactttg ccaaatactt tcttcaccaa tctcatgagg agagggaaca 360
   tgctgagaaa ctgatgaagc tgcagaacca acgaggtggc cgaatcttcc ttcaggatat 420
   caagaaacca gactgtgatg actgggagag cgggctgaat gcaatggagt gtgcattaca 480
   tttggaaaaa a                                                      491
<210> 308
   <211> 421
   <212> DNA
   <213> Homo sapiens
<400> 308
   ctcagcgctt cttctttctt ggtttgatcc tgactgctgt catggcgtgc cctctggaga 60
   aggccctgga tgtgatggtg tccaccttcc acaagtactc gggcaaagag ggtgacaagt 120
   tcaagctcaa caagtcagaa ctaaaggagc tgctgacccg ggagctgccc agcttcttgg 180
   ggaaaaggac agatgaagct gctttccaga agctgatgag caacttggac agcaacaggg 240
   acaacgaggt ggacttccaa gagtactgtg tcttcctgtc ctgcatcgcc atgatgtgta 300
   acgaattctt tgaaggcttc ccagataagc agcccaggaa gaaatgaaaa ctcctctgat 360
   gtggttgggg ggtctgccag ctggggccct ccctgtcgcc agtgggcact tttttttttc 420
   c 421
<210> 309
   <211> 321
   <212> DNA
   <213> Homo sapiens
<400> 309
   accaaatggc ggatgacgcc ggtgcagcgg gggggcccgg gggccctggt ggccctggga 60
   tggggaaccg cggtggcttc cgcggaggtt tcggcagtgg catccggggc cggggtcgcg 120
   gccgtggacg gggccggggc cgaggccgcg gagctcgcgg aggcaaggcc gaggataagg 180
   agtggatgcc cgtcaccaag ttgggccgct tggtcaagga catgaagatc aagtccctgg 240
   aggagatcta tctcttctcc ctgcccatta aggaatcaga gatcattgat ttcttcctgg 300
   gggcctctct caaggatgag g 321
<210> 310
   <211> 381
   <212> DNA
   <213> Homo sapiens
<400> 310
   ttaaccagcc atattggctc aataaatagc ttcggtaagg agttaatttc cttctagaaa 60
   tcagtgccta tttttcctgg aaactcaatt ttaaatagtc caattccatc tgaagccaag 120
   ctgttgtcat tttcattcgg tgacattctc tcccatgaca cccagaaggg gcagaagaac 180
   cacatttttc atttatagat gtttgcatcc tttgtattaa aattattttg aaggggttgc 240
   ctcattggat ggcttttttt tttttcctcc agggagaagg ggagaaatgt acttggaaat 300
   taatgtatgt ttacatctct ttgcaaattc ctgtacatag agatatattt tttaagtgtg 360
   aatgtaacaa catactgtga a 381
<210> 311
   <211> 538
   <212> DNA
   <213> Homo sapiens
<400> 311
   tttgaattta caccaagaac ttctcaataa aagaaaatca tgaatgctcc acaatttcaa 60
   cataccacaa gagaagttaa tttcttaaca ttgtgttcta tgattatttg taagaccttc 120
   accaagttct gatatctttt aaagacatag ttcaaaattg cttttgaaaa tctgtattct 180
   tgaaaatatc cttgttgtgt attaggtttt taaataccag ctaaaggatt acctcactga 240
   gtcatcagta ccctcctatt cagctcccca agatgatgtg tttttgctta ccctaagaga 300
   ggttttcttc ttatttttag ataattcaag tgcttagata aattatgttt tctttaagtg 360
   tttatggtaa actcttttaa agaaaattta atatgttata gctgaatctt tttggtaact 420
   ttaaatcttt atcatagact ctgtacatat gttcaaatta gctgcttgcc tgatgtgtgt 480
   atcatcggtg ggatgacaga acaaacatat ttatgatcat gaataatgtg ctttgtaa   538
<210> 312
   <211> 176
   <212> DNA
   <213> Homo sapiens
<400> 312
   ggaggagcag ctgagagata gggtcagtga atgcggttca gcctgctacc tctcctgtct 60
   tcatagaacc attgccttag aattattgta tgacacgttt tttgttggtt aagctgtaag 120
   gttttgttct ttgtgaacat gggtattttg aggggagggt ggagggagta gggaag     176
<210> 313
   <211> 396
   <212> DNA
   <213> Homo sapiens
<400> 313
   ccagcacccc caggccctgg gggacctggg ttctcagact gccaaagaag ccttgccatc 60
   tggcgctccc atggctcttg caacatctcc ccttcgtttt tgagggggtc atgccggggg 120
   agccaccagc ccctcactgg gttcggagga gagtcaggaa gggccaagca cgacaaagca 180
   gaaacatcgg atttggggaa cgcgtgtcaa tcccttgtgc cgcagggctg ggcgggagag 240
   actgttctgt tccttgtgta actgtgttgc tgaaagacta cctcgttctt gtcttgatgt 300
   gtcaccgggg caactgcctg ggggcgggga tgggggcagg gtggaagcgg ctccccattt 360
   tataccaaag gtgctacatc tatgtgatgg gtgggg                           396
<210> 314
   <211> 311
   <212> DNA
   <213> Homo sapiens
<400> 314
   cctcaacatc ctcagagagg actggaagcc agtccttacg ataaactcca taatttatgg 60
   cctgcagtat ctcttcttgg agcccaaccc cgaggaccca ctgaacaagg aggccgcaga 120
   ggtcctgcag aacaaccggc ggctgtttga gcagaacgtg cagcgctcca tgcggggtgg 180
   ctacatcggc tccacctact ttgagcgctg cctgaaatag ggttggcgca tacccacccc 240
   cgccacggcc acaagccctg gcatcccctg caaatattta ttgggggcca tgggtagggg 300
   tttggggggc g                                                       311
<210> 315
   <211> 336
   <212> DNA
   <213> Homo sapiens
<400> 315
   tttagaacat ggttatcatc caagactact ctaccctgca acattgaact cccaagagca 60
   aatccacatt cctcttgagt tctgcagctt ctgtgtaaat agggcagctg tcgtctatgc 120
   cgtagaatca catgatctga ggaccattca tggaagctgc taaatagcct agtctgggga 180
   gtcttccata aagttttgca tggagcaaac aaacaggatt aaactaggtt tggttccttc 240
   agccctctaa aagcataggg cttagcctgc aggcttcctt gggctttctc tgtgtgtgta 300
   gttttgtaaa cactatagca tctgttaaga tccagt                           336
<210> 316
   <211> 436
   <212> DNA
   <213> Homo sapiens
<400> 316
   aacatggtct gcgtgcctta agagagacgc ttcctgcaga acaggacctg actacaaaga 60
   atgtttccat tggaattgtt ggtaaagact tggagtttac aatctatgat gatgatgatg 120
   tgtctccatt cctggaaggt cttgaagaaa gaccacagag aaaggcacag cctgctcaac 180
   ctgctgatga acctgcagaa aaggctgatg aaccaatgga acattaagtg ataagccagt 240
   ctatatatgt attatcaaat atgtaagaat acaggcacca catactgatg acaataatct 300
   atactttgaa ccaaaagttg cagagtggtg gaatgctatg ttttaggaat cagtccagat 360
   gtgagttttt tccaagcaac ctcactgaaa cctatataat ggaatacatt tttctttgaa 420
   agggtctgta taatca                                                 436
<210> 317
   <211> 196
   <212> DNA
   <213> Homo sapiens
<400> 317
   tattccttgt gaagatgata tactattttt gttaagcgtg tctgtattta tgtgtgagga 60
   gctgctggct tgcagtgcgc gtgcacgtgg agagctggtg cccggagatt ggacggcctg 120
   atgctccctc ccctgccctg gtccagggaa gctggccgag ggtcctggct cctgaggggc 180
   atctgcccct ccccca                                                196
<210> 318
   <211> 381
   <212> DNA
   <213> Homo sapiens
<220>
   <221> misc_feature
   <222> 8, 9, 102, 122, 167, 162, 193, 235, 253, 265, 266, 290, 321, 378
   <223> n = A,T,C or G
<400> 318
   gacgcttnng ccgtaacgat gatcggagac atcctgctgt tcgggacgtt gctgatgaat 60
   gccggggcgg tgctgaactt taagctgaaa aagaaggaca cncagggctt tggggaggag 120
   tncagggagc ccaacacagg tgacaacatc cgggaattct tgctgancct cagatacttt 180
   cnaatcttca tcnccctgtg gaacatcttc atgatgttct gcatgattgt gctgntcggc 240
   tcttgaatcc cancgatgaa accannaact cactttcccg ggatgccgan tctccattcc 300
   tccattcctg atgacttcaa naatgttttt gaccaaaaaa ccgacaacct tcccagaaag 360
   tccaagctcg tggtgggngg a 381
<210> 319
   <211> 506
   <212> DNA
   <213> Homo sapiens
<400> 319
   ctaagcttta cgaatggggt gacaacttat gataaaaact agagctagtg aattagccta 60
   tttgtaaata cctttgttat aattgatagg atacatcttg gacatggaat tgttaagcca 120
   cctctgagca gtgtatgtca ggacttgttc attaggttgg cagcagaggg gcagaaggaa 180
   ttatacaggt agagatgtat gcagatgtgt ccatatatgt ccatatttac attttgatag 240
   ccattgatgt atgcatctct tggctgtact ataagaacac attaattcaa tggaaataca 300
   ctttgctaat attttaatgg tatagatctg ctaatgaatt ctcttaaaaa catactgtat 360
   tctgttgctg tgtgtttcat tttaaattga gcattaaggg aatgcagcat ttaaatcaga 420
   actctgccaa tgcttttatc tagaggcgtg ttgccatttt tgtcttatat gaaatttctg 480
   tcccaagaaa ggcaggatta catctt 506
<210> 320
   <211> 351
   <212> DNA
   <213> Homo sapiens
<400> 320
   ctgacctgca ggacgaaacc atgaagagcc tgatccttct tgccatcctg gccgccttag 60
   cggtagtaac tttgtgttat gaatcacatg aaagcatgga atcttatgaa cttaatccct 120
   tcattaacag gagaaatgca aataccttca tatcccctca gcagagatgg agagctaaag 180
   tccaagagag gatccgagaa cgctctaagc ctgtccacga gctcaatagg gaagcctgtg 240
   atgactacag actttgcgaa cgctacgcca tggtttatgg atacaatgct gcctataatc 300
   gctacttcag gaagcgccga gggaccaaat gagactgagg gaagaaaaaa a 351
<210> 321
   <211> 421
   <212> DNA
   <213> Homo sapiens
<400> 321
   ctcggaggcg ttcagctgct tcaagatgaa gctgaacatc tccttcccag ccactggctg 60
   ccagaaactc attgaagtgg acgatgaacg caaacttcgt actttctatg agaagcgtat 120
   ggccacagaa gttgctgctg acgctctggg tgaagaatgg aagggttatg tggtccgaat 180
   cagtggtggg aacgacaaac aaggtttccc catgaagcag ggtgtcttga cccatggccg 240
   tgtccgcctg ctactgagta aggggcattc ctgttacaga ccaaggagaa ctggagaaag 300
   aaagagaaaa tcagttcgtg gttgcattgt ggatgcaaat ctgagcgttc tcaacttggt 360
   tattgtaaaa aaaggagaga aggatattcc tggactgact gatactacag tgcctcgccg 420
   c 421
<210> 322
   <211> 521
   <212> DNA
   <213> Homo sapiens
<400> 322
   agcagctctc ctgccacagc tcctcacccc ctgaaaatgt tcgcctgctc caagtttgtc 60
   tccactccct ccttggtcaa gagcacctca cagctgctga gccgtccgct atctgcagtg 120
   gtgctgaaac gaccggagat actgacagat gagagcctca gcagcttggc agtctcatgt 180
   ccccttacct cacttgtctc tagccgcagc ttccaaacca gcgccatttc aagggacatc 240
   gacacagcag ccaagttcat tggagctggg gctgccacag ttggggtggc tggttctggg 300
   gctgggattg gaactgtgtt tgggagcctc atcattggtt atgccaggaa cccttctctg 360
   aagcaacagc tcttctccta cgccattctg ggctttgccc tctcggaggc catggggctc 420
   ttttgtctga tggtagcctt tctcatcctc tttgccatgt gaaggagccg tctccacctc 480
   ccatagttct cccgcgtctg gttggccccg tgtgttcctt t                     521
<210> 323
   <211> 435
   <212> DNA
   <213> Homo sapiens
<400> 323
   ccgaggtcgc acgcgtgaga cttctccgcc gcagacgccg ccgcgatgcg ctacgtcgcc 60
   tcctacctgc tggctgccct agggggcaac tcctccccca gcgccaagga catcaagaag 120
   atcttggaca gcgtgggtat cgaggcggac gacgaccggc tcaacaaggt tatcagtgag 180
   ctgaatggaa aaaacattga agacgtcatt gcccagggta ttggcaagct tgccagtgta 240
   cctgctggtg gggctgtagc cgtctctgct gccccaggct ctgcagcccc tgctgctggt 300
   tctgcccctg ctgcagcaga ggagaagaaa gatgagaaga aggaggagtc tgaagagtca 360
   gatgatgaca tgggatttgg cctttttgat taaattcctg ctcccctgca aataaagcct 420
   ttttacacat ctcaa                                                  435
<210> 324
   <211> 521
   <212> DNA
   <213> Homo sapiens
<400> 324
   aggagatcga ctttcggtgc ccgcaagacc agggctggaa cgccgagatc acgctgcaga 60
   tggtgcagta caagaatcgt caggccatcc tggcggtcaa atccacgcgg cagaagcagc 120
   agcacctggt ccagcagcag cccccctcgc agccgcagcc gcagccgcag ctccagcccc 180
   aaccccagcc tcagcctcag ccgcaacccc agccccaatc acaaccccag cctcagcccc 240
   aacccaagcc tcagccccag cagctccacc cgtatccgca tccacatcca catccacact 300
   ctcatcctca ctcgcaccca caccctcacc cgcacccgca tccgcaccaa ataccgcacc 360
   cacacccaca gccgcactcg cagccgcacg ggcaccggct tctccgcagc acctccaact 420
   ctgcctgaaa ggggcagctc ccgggcaaga caaggttttg aggacttgag gaagtgggac 480
   gagcacattt ctattgtctt cacttggatc aaaagcaaaa c                     521
<210> 325
   <211> 451
   <212> DNA
   <213> Homo sapiens
<400> 325
   attttcattt ccattaacct ggaagctttc atgaatattc tcttctttta aaacatttta 60
   acattattta aacagaaaaa gatgggctct ttctggttag ttgttacatg atagcagaga 120
   tatttttact tagattactt tgggaatgag agattgttgt cttgaactct ggcactgtac 180
   agtgaatgtg tctgtagttg tgttagtttg cattaagcat gtataacatt caagtatgtc 240
   atccaaataa gaggcatata cattgaattg tttttaatcc tctgacaagt tgactcttcg 300
   acccccaccc ccacccaaga cattttaata gtaaatagag agagagagaa gagttaatga 360
   acatgaggta gtgttccact ggcaggatga cttttcaata gctcaaatca atttcagtgc 420
   ctttatcact tgaattatta acttaatttg a                                451
<210> 326
   <211> 421
   <212> DNA
   <213> Homo sapiens
<220>
   <221> misc_feature
   <222> 296
   <223> n = A,T,C or G
<400> 326
   cgcggtcgta agggctgagg atttttggtc cgcacgctcc tgctcctgac tcaccgctgt 60
   tcgctctcgc cgaggaacaa gtcggtcagg aagcccgcgc gcaacagcca tggcttttaa 120
   ggataccgga aaaacacccg tggagccgga ggtggcaatt caccgaattc gaatcaccct 180
   aacaagccgc aacgtaaaat ccttggaaaa ggtgtgtgct gacttgataa gaggcgcaaa 240
   agaaaagaat ctcaaagtga aaggaccagt tcgaatgcct accaagactt tgagantcac 300
   tacaagaaaa actccttgtg gtgaaggttc taagacgtgg gatcgtttcc agatgagaat 360
   tcacaagcga ctcattgact tgcacagtcc ttctgagatt gttaagcaga ttacttccat 420
   c                                                                 421
<210> 327
   <211> 456
   <212> DNA
   <213> Homo sapiens
<400> 327
   atcttgacga ggctgcggtg tctgctgcta ttctccgagc ttcgcaatgc cgcctaagga 60
   cgacaagaag aagaaggacg ctggaaagtc ggccaagaaa gacaaagacc cagtgaacaa 120
   atccgggggc aaggccaaaa agaagaagtg gtccaaaggc aaagttcggg acaagctcaa 180
   taacttagtc ttgtttgaca aagctaccta tgataaactc tgtaaggaag ttcccaacta 240
   taaacttata accccagctg tggtctctga gagactgaag attcgaggct ccctggccag 300
   ggcagccctt caggagctcc ttagtaaagg acttatcaaa ctggtttcaa agcacagagc 360
   tcaagtaatt tacaccagaa ataccaaggg tggagatgct ccagctgctg gtgaagatgc 420
   atgaataggt ccaaccagct gtacatttgg aaaaat                           456
<210> 328
   <211> 471
   <212> DNA
   <213> Homo sapiens
<400> 328
   gtggaagtga catcgtcttt aaaccctgcg tggcaatccc tgacgcaccg ccgtgatgcc 60
   cagggaagac agggcgacct ggaagtccaa ctacttcctt aagatcatcc aactattgga 120
   tgattatccg aaatgtttca ttgtgggagc agacaatgtg ggctccaagc agatgcagca 180
   gatccgcatg tcccttcgcg ggaaggctgt ggtgctgatg ggcaagaaca ccatgatgcg 240
   caaggccatc cgagggcacc tggaaaacaa cccagctctg gagaaactgc tgcctcatat 300
   ccgggggaat gtgggctttg tgttcaccaa ggaggacctc actgagatca gggacatgtt 360
   gctggccaat aaggtgccag ctgctgcccg tgctggtgcc attgccccat gtgaagtcac 420
   tgtgccagcc cagaacactg gtctcgggcc cgagaagacc tcctttttcc a          471
<210> 329
   <211> 278
   <212> DNA
   <213> Homo sapiens
<220>
   <221> misc_feature
   <222> 154, 204
   <223> n = A,T,C or G
<400> 329
   gtttaaactt aagcttggta ccgagctcgg atccactagt ccagtgtggt ggaattctag 60
   aaattgagat gcccccccag gccagcaaat gttccttttt gttcaaagtc tatttttatt 120
   ccttgatatt tttctttttt tttttttttt ttgnggatgg ggacttgtga atttttctaa 180
   aggtgctatt taacatggga gganagcgtg tgcggctcca gcccagcccg ctgctcactt 240
   tccaccctct ctccacctgc ctctggcttc tcaggcct                         278
<210> 330
   <211> 338
   <212> DNA
   <213> Homo sapiens
<400> 330
   ctcaggcttc aacatcgaat acgccgcagg ccccttcgcc ctattcttca tagccgaata 60
   cacaaacatt attataataa acaccctcac cactacaatc ttcctaggaa caacatatga 120
   cgcactctcc cctgaactct acacaacata ttttgtcacc aagaccctac ttctaacctc 180
   cctgttctta tgaattcgaa cagcataccc ccgattccgc tacgaccaac tcatacacct 240
   cctatgaaaa aacttcctac cactcaccct agcattactt atatgatatg tctccatacc 300
   cattacaatc tccagcattc cccctcaaac ctaaaaaa                         338
<210> 331
   <211> 2820
   <212> DNA
   <213> Homo sapiens
<400> 331
   tggcaaaatc ctggagccag aagaaaggac agcagcattg atcaatctta cagctaacat 60
   gttgtacctg gaaaacaatg cccagactca atttagtgag ccacagtaca cgaacctggg 120
   gctcctgaac agcatggacc agcagattcg gaacggctcc tcgtccacca gtccctataa 180
   cacagaccac gcgcagaaca gcgtcacggc gccctcgccc tacgcacagc ccagccccac 240
   cttcgatgct ctctctccat cacccgccat cccctccaac accgactacc caggcccgca 300
   cagttccgac gtgtccttcc agcagtcgag caccgccaag tcggccacct ggacgtattc 360
   cactgaactg aagaaactct actgccaaat tgcaaagaca tgccccatcc agatcaaggt 420
   gatgacccca cctcctcagg gagctgttat ccgcgccatg cctgtctaca aaaaagctga 480
   gcacgtcacg gaggtggtga agcggtgccc caaccatgag ctgagccgtg agttcaacga 540
   gggacagatt gcccctccta gtcatttgat tcgagtagag gggaacagcc atgcccagta 600
   tgtagaagat cccatcacag gaagacagag tgtgctggta ccttatgagc caccccaggt 660
   tggcactgaa ttcacgacag tcttgtacaa tttcatgtgt aacagcagtt gtgttggagg 720
   gatgaaccgc cgtccaattt taatcattgt tactctggaa accagagatg ggcaagtcct 780
   gggccgacgc tgctttgagg cccggatctg tgcttgccca ggaagagaca ggaaggcgga 840
   tgaagatagc atcagaaagc agcaagtttc ggacagtaca aagaacggtg atggtacgaa 900
   gcgcccgttt cgtcagaaca cacatggtat ccagatgaca tccatcaaga aacgaagatc 960
   cccagatgat gaactgttat acttaccagt gaggggccgt gagacttatg aaatgctgtt 1020
   gaagatcaaa gagtccctgg aactcatgca gtaccttcct cagcacacaa ttgaaacgta 1080
   caggcaacag caacagcagc agcaccagca cttacttcag aaacagacct caatacagtc 1140
   tccatcttca tatggtaaca gctccccacc tctgaacaaa atgaacagca tgaacaagct 1200
   gccttctgtg agccagctta tcaaccctca gcagcgcaac gccctcactc ctacaaccat 1260
   tcctgatggc atgggagcca acattcccat gatgggcacc cacatgccaa tggctggaga 1320
   catgaatgga ctcagcccca cccaggcact ccctccccca ctctccatgc catccacctc 1380
   ccactgcaca cccccacctc cgtatcccac agattgcagc attgtcagtt tcttagcgag 1440
   gttgggctgt tcatcatgtc tggactattt cacgacccag gggctgacca ccatctatca 1500
   gattgagcat tactccatgg atgatctggc aagtctgaaa atccctgagc aatttcgaca 1560
   tgcgatctgg aagggcatcc tggaccaccg gcagctccac gaattctcct ccccttctca 1620
   tctcctgcgg accccaagca gtgcctctac agtcagtgtg ggctccagtg agacccgggg 1680
   tgagcgtgtt attgatgctg tgcgattcac cctccgccag accatctctt tcccaccccg 1740
   agatgagtgg aatgacttca actttgacat ggatgctcgc cgcaataagc aacagcgcat 1800
   caaagaggag ggggagtgag cctcaccatg tgagctcttc ctatccctct cctaactgcc 1860
   agccccctaa aagcactcct gcttaatctt caaagccttc tccctagctc ctccccttcc 1920
   tcttgtctga tttcttaggg gaaggagaag taagaggcta cctcttacct aacatctgac 1980
   ctggcatcta attctgattc tggctttaag ccttcaaaac tatagcttgc agaactgtag 2040
   ctgccatggc taggtagaag tgagcaaaaa agagttgggt gtctccttaa gctgcagaga 2100
   tttctcattg acttttataa agcatgttca cccttatagt ctaagactat atatataaat 2160
   gtataaatat acagtataga tttttgggtg gggggcattg agtattgttt aaaatgtaat 2220
   ttaaatgaaa gaaaattgag ttgcacttat tgaccatttt ttaatttact tgttttggat 2280
   ggcttgtcta tactccttcc cttaaggggt atcatgtatg gtgataggta tctagagctt 2340
   aatgctacat gtgagtgcga tgatgtacag attctttcag ttctttggat tctaaataca 2400
   tgccacatca aacctttgag tagatccatt tccattgctt attatgtagg taagactgta 2460
   gatatgtatt cttttctcag tgttggtata ttttatatta ctgacatttc ttctagtgat 2520
   gatggttcac gttggggtga tttaatccag ttataagaag aagttcatgt ccaaacggtc 2580
   ctctttagtt tttggttggg aatgaggaaa attcttaaaa ggcccatagc agccagttca 2640
   aaaacacccg acgtcatgta tttgagcata tcagtaaccc ccttaaattt aatacccaga 2700
   taccttatct tacaatgttg attgggaaaa catttgctgc ccattacaga ggtattaaaa 2760
   ctaaatttca ctactagatt gactaactca aatacacatt tgctactgtt gtaagaattc 2820
<210> 332
   <211> 2270
   <212> DNA
   <213> Homo sapiens
<400> 332
   tcgttgatat caaagacagt tgaaggaaat gaattttgaa acttcacggt gtgccaccct 60
   acagtactgc cctgaccctt acatccagcg tttcgtagaa acccagctca tttctcttgg 120
   aaagaaagtt attaccgatc caccatgtcc cagagcacac agacaaatga attcctcagt 180
   ccagaggttt tccagcatat ctgggatttt ctggaacagc ctatatgttc agttcagccc 240
   attgacttga actttgtgga tgaaccatca gaagatggtg cgacaaacaa gattgagatt 300
   agcatggact gtatccgcat gcaggactcg gacctgagtg accccatgtg gccacagtac 360
   acgaacctgg ggctcctgaa cagcatggac cagcagattc agaacggctc ctcgtccacc 420
   agtccctata acacagacca cgcgcagaac agcgtcacgg cgccctcgcc ctacgcacag 480
   cccagctcca ccttcgatgc tctctctcca tcacccgcca tcccctccaa caccgactac 540
   ccaggcccgc acagtttcga cgtgtccttc cagcagtcga gcaccgccaa gtcggccacc 600
   tggacgtatt ccactgaact gaagaaactc tactgccaaa ttgcaaagac atgccccatc 660
   cagatcaagg tgatgacccc acctcctcag ggagctgtta tccgcgccat gcctgtctac 720
   aaaaaagctg agcacgtcac ggaggtggtg aagcggtgcc ccaaccatga gctgagccgt 780
   gaattcaacg agggacagat tgcccctcct agtcatttga ttcgagtaga ggggaacagc 840
   catgcccagt atgtagaaga tcccatcaca ggaagacaga gtgtgctggt accttatgag 900
   ccaccccagg ttggcactga attcacgaca gtcttgtaca atttcatgtg taacagcagt 960
   tgtgttggag ggatgaaccg ccgtccaatt ttaatcattg ttactctgga aaccagagat 1020
   gggcaagtcc tgggccgacg ctgctttgag gcccggatct gtgcttgccc aggaagagac 1080
   aggaaggcgg atgaagatag catcagaaag cagcaagttt cggacagtac aaagaacggt 1140
   gatggtacga agcgcccgtt tcgtcagaac acacatggta tccagatgac atccatcaag 1200
   aaacgaagat ccccagatga tgaactgtta tacttaccag tgagggggcg tgagacttat 1260
   gaaatgctgt tgaagatcaa agagtccctg gaactcatgc agtaccttcc tcagcacaca 1320
   attgaaacgt acaggcaaca gcaacagcag cagcaccagc acttacttca gaaacagacc 1380
   tcaatacagt ctccatcttc atatggtaac agctccccac ctctgaacaa aatgaacagc 1440
   atgaacaagc tgccttctgt gagccagctt atcaaccctc agcagcgcaa cgccctcact 1500
   cctacaacca ttcctgatgg catgggagcc aacattccca tgatgggcac ccacatgcca 1560
   atggctggag acatgaatgg actcagcccc acccaggcac tccctccccc actctccatg 1620
   ccatccacct cccactgcac acccccacct ccgtatccaa cagattgcag cattgtcggt 1680
   ttcttagcga ggttgggctg ttcatcatgt ctggactatt tcacgaccca ggggctgacc 1740
   accatctatc agattgagca ttactccatg gatgatctgg caagtctgaa aatccctgag 1800
   caatttcgac atgcgatctg gaagggcatc ctggaccacc ggcagctcca cgaattctcc 1860
   tccccttctc atctcctgcg gaccccaagc agtgcctcta cagtcagtgt gggctccagt 1920
   gagacccggg gtgagcgtgt tattgatgct gtgcgattca ccctccgcca gaccatctct 1980
   ttcccacccc gagatgagtg gaatgacttc aactttgaca tggatgctcg ccgcaataag 2040
   caacagcgca tcaaagagga gggggagtga gcctcaccat gtgagctctt cctatccctc 2100
   tcctaactgc cagcccccta aaagcactcc tgcttaatct tcaaagcctt ctccctagct 2160
   cctccccttc ctcttgtctg atttcttagg ggaaggagaa gtaagaggct acctcttacc 2220
   taacatctga cctggcatct aattctgatt ctggctttaa gccttcaaaa            2270
<210> 333
   <211> 2816
   <212> DNA
   <213> Homo sapiens
<400> 333
   tcgttgatat caaagacagt tgaaggaaat gaattttgaa acttcacggt gtgccaccct 60
   acagtactgc cctgaccctt acatccagcg tttcgtagaa acccagctca tttctcttgg 120
   aaagaaagtt attaccgatc caccatgtcc cagagcacac agacaaatga attcctcagt 180
   ccagaggttt tccagcatat ctgggatttt ctggaacagc ctatatgttc agttcagccc 240
   attgacttga actttgtgga tgaaccatca gaagatggtg cgacaaacaa gattgagatt 300
   agcatggact gtatccgcat gcaggactcg gacctgagtg accccatgtg gccacagtac 360
   acgaacctgg ggctcctgaa cagcatggac cagcagattc agaacggctc ctcgtccacc 420
   agtccctata acacagacca cgcgcagaac agcgtcacgg cgccctcgcc ctacgcacag 480
   cccagctcca ccttcgatgc tctctctcca tcacccgcca tcccctccaa caccgactac 540
   ccaggcccgc acagtttcga cgtgtccttc cagcagtcga gcaccgccaa gtcggccacc 600
   tggacgtatt ccactgaact gaagaaactc tactgccaaa ttgcaaagac atgccccatc 660
   cagatcaagg tgatgacccc acctcctcag ggagctgtta tccgcgccat gcctgtctac 720
   aaaaaagctg agcacgtcac ggaggtggtg aagcggtgcc ccaaccatga gctgagccgt 780
   gaattcaacg agggacagat tgcccctcct agtcatttga ttcgagtaga ggggaacagc 840
   catgcccagt atgtagaaga tcccatcaca ggaagacaga gtgtgctggt accttatgag 900
   ccaccccagg ttggcactga attcacgaca gtcttgtaca atttcatgtg taacagcagt 960
   tgtgttggag ggatgaaccg ccgtccaatt ttaatcattg ttactctgga aaccagagat 1020
   gggcaagtcc tgggccgacg ctgctttgag gcccggatct gtgcttgccc aggaagagac 1080
   aggaaggcgg atgaagatag catcagaaag cagcaagttt cggacagtac aaagaacggt 1140
   gatggtacga agcgcccgtt tcgtcagaac acacatggta tccagatgac atccatcaag 1200
   aaacgaagat ccccagatga tgaactgtta tacttaccag tgaggggccg tgagacttat 1260
   gaaatgctgt tgaagatcaa agagtccctg gaactcatgc agtaccttcc tcagcacaca 1320
   attgaaacgt acaggcaaca gcaacagcag cagcaccagc acttacttca gaaacatctc 1380
   ctttcagcct gcttcaggaa tgagcttgtg gagccccgga gagaaactcc aaaacaatct 1440
   gacgtcttct ttagacattc caagccccca aaccgatcag tgtacccata gagccctatc 1500
   tctatatttt aagtgtgtgt gttgtatttc catgtgtata tgtgagtgtg tgtgtgtgta 1560
   tgtgtgtgcg tgtgtatcta gccctcataa acaggacttg aagacacttt ggctcagaga 1620
   cccaactgct caaaggcaca aagccactag tgagagaatc ttttgaaggg actcaaacct 1680
   ttacaagaaa ggatgttttc tgcagatttt gtatccttag accggccatt ggtgggtgag 1740
   gaaccactgt gtttgtctgt gagctttctg ttgtttcctg ggagggaggg gtcaggtggg 1800
   gaaaggggca ttaagatgtt tattggaacc cttttctgtc ttcttctgtt gtttttctaa 1860
   aattcacagg gaagcttttg agcaggtctc aaacttaaga tgtcttttta agaaaaggag 1920
   aaaaaagttg ttattgtctg tgcataagta agttgtaggt gactgagaga ctcagtcaga 1980
   cccttttaat gctggtcatg taataatatt gcaagtagta agaaacgaag gtgtcaagtg 2040
   tactgctggg cagcgaggtg atcattacca aaagtaatca actttgtggg tggagagttc 2100
   tttgtgagaa cttgcattat ttgtgtcctc ccctcatgtg taggtagaac atttcttaat 2160
   gctgtgtacc tgcctctgcc actgtatgtt ggcatctgtt atgctaaagt ttttcttgta 2220
   catgaaaccc tggaagacct actacaaaaa aactgttgtt tggcccccat agcaggtgaa 2280
   ctcattttgt gcttttaata gaaagacaaa tccaccccag taatattgcc cttacgtagt 2340
   tgtttaccat tattcaaagc tcaaaataga atttgaagcc ctctcacaaa atctgtgatt 2400
   aatttgctta attagagctt ctatccctca agcctaccta ccataaaacc agccatatta 2460
   ctgatactgt tcagtgcatt tagccaggag acttacgttt tgagtaagtg agatccaagc 2520
   agacgtgtta aaatcagcac tcctggactg gaaattaaag attgaaaggg tagactactt 2580
   ttcttttttt tactcaaaag tttagagaat ctctgtttct ttccatttta aaaacatatt 2640
   ttaagataat agcataaaga ctttaaaaat gttcctcccc tccatcttcc cacacccagt 2700
   caccagcact gtattttctg tcaccaagac aatgatttct tgttattgag gctgttgctt 2760
   ttgtggatgt gtgattttaa ttttcaataa acttttgcat cttggtttaa aagaaa     2816
<210> 334
   <211> 2082
   <212> DNA
   <213> Homo sapiens
<400> 334
   agatgctaca gcgactgcac acccaggctg tatgatacag cctattgctc ccgggctgca 60
   aacctgtcca gcatgtgatg tggtgggata ctgaattgaa taccgaatac tgtaggcaat 120
   tgtaacacag tggtaagtct ttgtgtatct aaacatagct aaacaccaaa aggtatagta 180
   agaatatggt attataatct tatggaacta tcattgtata tgtggtttgt caaccagaat 240
   gtagttatac agcacaggac tgtgcttatg atgtgccaag cacagctctc agtactaact 300
   cctttaatct tcatatcaac cctaggaggt aacttcttaa gtagattcat attgtaaggg 360
   tctcggggtg ggggggttgg caaaatcctg gagccagaag aaaggacagc agcattgatc 420
   aatcttacag ctaacatgtt gtacctggaa aacaatgccc agactcaatt tagtgagcca 480
   cagtacacga acctggggct cctgaacagc atggaccagc agattcagaa cggctcctcg 540
   tccaccagtc cctataacac agaccacgcg cagaacagcg tcacggcgcc ctcgccctac 600
   gcacagccca gctccacctt cgatgctctc tctccatcac ccgccatccc ctccaacacc 660
   gactacccag gcccgcacag tttcgacgtg tccttccagc agtcgagcac cgccaagtcg 720
   gccacctgga cgtattccac tgaactgaag aaactctact gccaaattgc aaagacatgc 780
   cccatccaga tcaaggtgat gaccccacct cctcagggag ctgttatccg cgccatgcct 840
   gtctacaaaa aagctgagca cgtcacggag gtggtgaagc ggtgccccaa ccatgagctg 900
   agccgtgaat tcaacgaggg acagattgcc cctcctagtc atttgattcg agtagagggg 960
   aacagccatg cccagtatgt agaagatccc atcacaggaa gacagagtgt gctggtacct 1020
   tatgagccac cccaggttgg cactgaattc acgacagtct tgtacaattt catgtgtaac 1080
   agcagttgtg ttggagggat gaaccgccgt ccaattttaa tcattgttac tctggaaacc 1140
   agagatgggc aagtcctggg ccgacgctgc tttgaggccc ggatctgtgc ttgcccagga 1200
   agagacagga aggcggatga agatagcatc agaaagcagc aagtttcgga cagtacaaag 1260
   aacggtgatg gtacgaagcg cccgtctcgt cagaacacac atggtatcca gatgacatcc 1320
   atcaagaaac gaagatcccc agatgatgaa ctgttatact taccagtgag gggccgtgag 1380
   acttatgaaa tgctgttgaa gatcaaagag tccctggaac tcatgcagta ccttcctcag 1440
   cacacaattg aaacgtacag gcaacagcaa cagcagcagc accagcactt acttcagaaa 1500
   cagtgagtgt atcaacgtgt cattttagga ggcatgagtg acggtgactt tatttggatc 1560
   agcaataggg tgattgatga gcaatgtgga acataatggg agatagcaga ttgtcataga 1620
   ttcagatgac ctggtatggc aaccctcttt cagttgcaac cttttttacg tgtcttatta 1680
   taaccttccc ttcagaattc cacttatgtt ctgaaattaa atacaaacca tttctggtga 1740
   attacaaaga aactcacact aacagttctc ttctctatat gcctggtcca tacacactaa 1800
   cagtaagtac acactctatt tggtagtgat gtgtatattt gaaaacatga aatcttttct 1860
   catcccaatg gattgtctta taaatctcct gggatgcaca ctatccactt ttgggaataa 1920
   cactgtagac cagggatagc aaataggctt tactataata taaagtgact tgtttgaatg 1980
   ctgtaatgag aagaattctg agacctagtg catgataatt ggggaaatat ctgggtgcag 2040
   aaggataagg tagcatcatg ttgccgtatt ttagcatctc tg                   2082

<210> 335
   <211> 4849
   <212> DNA
   <213> Homo sapiens
<400> 335
   cgttgatatc aaagacagtt gaaggaaatg aattttgaaa cttcacggtg tgccacccta 60
   cagtactgcc ctgaccctta catccagcgt ttcgtagaaa ccccagctca tttctcttgg 120
   aaagaaagtt attaccgatc caccatgtcc cagagcacac agacaaatga attcctcagt 180
   ccagaggttt tccagcatat ctgggatttt ctggaacagc ctatatgttc agttcagccc 240
   attgacttga actttgtgga tgaaccatca gaagatggtg cgacaaacaa gattgagatt 300
   agcatggact gtatccgcat gcaggactcg gacctgagtg accccatgtg gccacagtac 360
   acgaacctgg ggctcctgaa cagcatggac cagcagattc agaacggctc ctcgtccacc 420
   agtccctata acacagacca cgcgcagaac agcgtcacgg cgccctcgcc ctacgcacag 480
   cccagctcca ccttcgatgc tctctctcca tcacccgcca tcccctccaa caccgactac 540
   ccaggcccgc acagtttcga cgtgtccttc cagcagtcga gcaccgccaa gtcggccacc 600
   tggacgtatt ccactgaact gaagaaactc tactgccaaa ttgcaaagac atgccccatc 660
   cagatcaagg tgatgacccc acctcctcag ggagctgtta tccgcgccat gcctgtctac 720
   aaaaaagctg agcacgtcac ggaggtggtg aagcggtgcc ccaaccatga gctgagccgt 780
   gaattcaacg agggacagat tgcccctcct agtcatttga ttcgagtaga ggggaacagc 840
   catgcccagt atgtagaaga tcccatcaca ggaagacaga gtgtgctggt accttatgag 900
   ccaccccagg ttggcactga attcacgaca gtcttgtaca atttcatgtg taacagcagt 960
   tgtgttggag ggatgaaccg ccgtccaatt ttaatcattg ttactctgga aaccagagat 1020
   gggcaagtcc tgggccgacg ctgctttgag gcccggatct gtgcttgccc aggaagagac 1080
   aggaaggcgg atgaagatag catcagaaag cagcaagttt cggacagtac aaagaacggt 1140
   gatggtacga agcgcccgtt tcgtcagaac acacatggta tccagatgac atccatcaag 1200
   aaacgaagat ccccagatga tgaactgtta tacttaccag tgaggggccg tgagacttat 1260
   gaaatgctgt tgaagatcaa agagtccctg gaactcatgc agtaccttcc tcagcacaca 1320
   attgaaacgt acaggcaaca gcaacagcag cagcaccagc acttacttca gaaacagacc 1380
   tcaatacagt ctccatcttc atatggtaac agctccccac ctctgaacaa aatgaacagc 1440
   atgaacaagc tgccttctgt gagccagctt atcaaccctc agcagcgcaa cgccctcact 1500
   cctacaacca ttcctgatgg catgggagcc aacattccca tgatgggcac ccacatgcca 1560
   atggctggag acatgaatgg actcagcccc acccaggcac tccctccccc actctccatg 1620
   ccatccacct cccagtgcac acccccacct ccgtatccca cagattgcag cattgtcagt 1680
   ttcttagcga ggttgggctg ttcatcatgt ctggactatt tcacgaccca ggggctgacc 1740
   accatctatc agattgagca ttactccatg gatgatctgg caagtctgaa aatccctgag 1800
   caatttcgac atgcgatctg gaagggcatc ctggaccacc ggcagctcca cgaattctcc 1860
   tccccttctc atctcctgcg gaccccaagc agtgcctcta cagtcagtgt gggctccagt 1920
   gagacccggg gtgagcgtgt tattgatgct gtgcgattca ccctccgcca gaccatctct 1980
   ttcccacccc gagatgagtg gaatgacttc aactttgaca tggatgctcg ccgcaataag 2040
   caacagcgca tcaaagagga gggggagtga gcctcaccat gtgagctctt cctatccctc 2100
   tcctaactgc cagcycccta aaagcactcc tgcttaatct tcaaagcctt ctccctagct 2160
   cctccccttc ctcttgtctg atttcttagg ggaaggagaa gtaagaggct acctcttacc 2220
   taacatctga cctggcatct aattctgatt ctggctttaa gccttcaaaa ctatagcttg 2280
   cagaactgta gctgccatgg ctaggtagaa gtgagcaaaa aagagttggg tgtctcctta 2340
   agctgcagag atttctcatt gacttttata aagcatgttc acccttatag tctaagacta 2400
   tatatataaa tgtataaata tacagtatag atttttgggt ggggggcatt gagtattgtt 2460
   taaaatgtaa tttaaatgaa agaaaattga gttgcactta ttgaccattt tttaatttac 2520
   ttgttttgga tggcttgtct atactccttc ccttaagggg tatcatgtat ggtgataggt 2580
   atctagagct taatgctaca tgtgagtgac gatgatgtac agattctttc agttctttgg 2640
   attctaaata catgccacat caaacctttg agtagatcca tttccattgc ttattatgta 2700
   ggtaagactg tagatatgta ttcttttctc agtgttggta tattttatat tactgacatt 2760
   tcttctagtg atgatggttc acgttggggt gatttaatcc agttataaga agaagttcat 2820
   gtccaaacgt cctctttagt ttttggttgg gaatgaggaa aattcttaaa aggcccatag 2880
   cagccagttc aaaaacaccc gacgtcatgt atttgagcat atcagtaacc cccttaaatt 2940
   taataccaga taccttatct tacaatattg attgggaaaa catttgctgc cattacagag 3000
   gtattaaaac taaatttcac tactagattg actaactcaa atacacattt gctactgttg 3060
   taagaattct gattgatttg attgggatga atgccatcta tctagttcta acagtgaagt 3120
   tttactgtct attaatattc agggtaaata ggaatcattc agaaatgttg agtctgtact 3180
   aaacagtaag atatctcaat gaaccataaa ttcaactttg taaaaatctt ttgaagcata 3240
   gataatattg tttggtaaat gtttcttttg tttggtaaat gtttctttta aagaccctcc 3300
   tattctataa aactctgcat gtagaggctt gtttaccttt ctctctctaa ggtttacaat 3360
   aggagtggtg atttgaaaaa tataaaatta tgagattggt tttcctgtgg cataaattgc 3420
   atcactgtat cattttcttt tttaaccggt aagagtttca gtttgttgga aagtaactgt 3480
   gagaacccag tttcccgtcc atctccctta gggactaccc atagacatga aaggtcccca 3540
   cagagcaaga gataagtctt tcatggctgc tgttgcttaa accacttaaa cgaagagttc 3600
   ccttgaaact ttgggaaaac atgttaatga caatattcca gatctttcag aaatataaca 3660
   catttttttg catgcatgca aatgagctct gaaatcttcc catgcattct ggtcaagggc 3720
   tgtcattgca cataagcttc cattttaatt ttaaagtgca aaagggccag cgtggctcta 3780
   aaaggtaatg tgtggattgc ctctgaaaag tgtgtatata ttttgtgtga aattgcatac 3840
   tttgtatttt gattattttt tttttcttct tgggatagtg ggatttccag aaccacactt 3900
   gaaacctttt tttatcgttt ttgtattttc atgaaaatac catttagtaa gaataccaca 3960
   tcaaataaga aataatgcta caattttaag aggggaggga agggaaagtt tttttttatt 4020
   atttttttaa aattttgtat gttaaagaga atgagtcctt gatttcaaag ttttgttgta 4080
   cttaaatggt aataagcact gtaaacttct gcaacaagca tgcagctttg caaacccatt 4140
   aaggggaaga atgaaagctg ttccttggtc ctagtaagaa gacaaactgc ttcccttact 4200
   ttgctgaggg tttgaataaa cctaggactt ccgagctatg tcagtactat tcaggtaaca 4260
   ctagggcctt ggaaattcct gtactgtgtc tcatggattt ggcactagcc aaagcgaggc 4320
   acccttactg gcttacctcc tcatggcagc ctactctcct tgagtgtatg agtagccagg 4380
   gtaaggggta aaaggatagt aagcatagaa accactagaa agtgggctta atggagttct 4440
   tgtggcctca gctcaatgca gttagctgaa gaattgaaaa gtttttgttt ggagacgttt 4500
   ataaacagaa atggaaagca gagttttcat taaatccttt tacctttttt ttttcttggt 4560
   aatcccctaa aataacagta tgtgggatat tgaatgttaa aggatatttt tttttctatt 4620
   atttttataa ttgtacaaaa ttaagcaaat gttaaaagtt ttatatgctt tattaatgtt 4680
   ttcaaaaggt attatacatg tgatacattt tttaagcttc agttgcttgt cttctggtac 4740
   tttctgttat gggcttttgg ggagccagaa gccaatctac aatctctttt tgtttgccag 4800
   gacatgcaat aaaatttaaa aaataaataa aaactaatta agaaataaa 4849
<210> 336
   <211> 1386
   <212> DNA
   <213> Homo sapiens
<400> 336
   atgttgtacc tggaaaacaa tgcccagact caatttagtg agccacagta cacgaacctg 60
   gggctcctga acagcatgga ccagcagatt cagaacggct cctcgtccac cagtccctat 120
   aacacagacc acgcgcagaa cagcgtcacg gcgccctcgc cctacgcaca gcccagctcc 180
   accttcgatg ctctctctcc atcacccgcc atcccctcca acaccgacta cccaggcccg 240
   cacagtttcg acgtgtcctt ccagcagtcg agcaccgcca agtcggccac ctggacgtat 300
   tccactgaac tgaagaaact ctactgccaa attgcaaaga catgccccat ccagatcaag 360
   gtgatgaccc cacctcctca gggagctgtt atccgcgcca tgcctgtcta caaaaaagct 420
   gagcacgtca cggaggtggt gaagcggtgc cccaaccatg agctgagccg tgaattcaac 480
   gagggacaga ttgcccctcc tagtcatttg attcgagtag aggggaacag ccatgcccag 540
   tatgtagaag atcccatcac aggaagacag agtgtgctgg taccttatga gccaccccag 600
   gttggcactg aattcacgac agtcttgtac aatttcatgt gtaacagcag ttgtgttgga 660
   gggatgaacc gccgtccaat tttaatcatt gttactctgg aaaccagaga tgggcaagtc 720
   ctgggccgac gctgctttga ggcccggatc tgtgcttgcc caggaagaga caggaaggcg 780
   gatgaagata gcatcagaaa gcagcaagtt tcggacagta caaagaacgg tgatggtacg 840
   aagcgcccgt ttcgtcagaa cacacatggt atccagatga catccatcaa gaaacgaaga 900
   tccccagatg atgaactgtt atacttacca gtgaggggcc gtgagactta tgaaatgctg 960
   ttgaagatca aagagtccct ggaactcatg cagtaccttc ctcagcacac aattgaaacg 1020
   tacaggcaac agcaacagca gcagcaccag cacttacttc agaaacagac ctcaatacag 1080
   tctccatctt catatggtaa cagctcccca cctctgaaca aaatgaacag catgaacaag 1140
   ctgccttctg tgagccagct tatcaaccct cagcagcgca acgccctcac tcctacaacc 1200
   attcctgatg gcatgggagc caacattccc atgatgggca cccacatgcc aatggctgga 1260
   gacatgaatg gactcagccc cacccaggca ctccctcccc cactctccat gccatccacc 1320
   tcccactgca cacccccacc tccgtatccc acagattgca gcattgtcag gatctggcaa 1380
   gtctga 1386
<210> 337
   <211> 1551
   <212> DNA
   <213> Homo sapiens
<400> 337
   atgtcccaga gcacacagac aaatgaattc ctcagtccag aggttttcca gcatatctgg 60
   gattttctgg aacagcctat atgttcagtt cagcccattg acttgaactt tgtggatgaa 120
   ccatcagaag atggtgcgac aaacaagatt gagattagca tggactgtat ccgcatgcag 180
   gactcggacc tgagtgaccc catgtggcca cagtacacga acctggggct cctgaacagc 240
   atggaccagc agattcagaa cggctcctcg tccaccagtc cctataacac agaccacgcg 300
   cagaacagcg tcacggcgcc ctcgccctac gcacagccca gctccacctt cgatgctctc 360
   tctccatcac ccgccatccc ctccaacacc gactacccag gcccgcacag tttcgacgtg 420
   tccttccagc agtcgagcac cgccaagtcg gccacctgga cgtattccac tgaactgaag 480
   aaactctact gccaaattgc aaagacatgc cccatccaga tcaaggtgat gaccccacct 540
   cctcagggag ctgttatccg cgccatgcct gtctacaaaa aagctgagca cgtcacggag 600
   gtggtgaagc ggtgccccaa ccatgagctg agccgtgaat tcaacgaggg acagattgcc 660
   cctcctagtc atttgattcg agtagagggg aacagccatg cccagtatgt agaagatccc 720
   atcacaggaa gacagagtgt gctggtacct tatgagccac cccaggttgg cactgaattc 780
   acgacagtct tgtacaattt catgtgtaac agcagttgtg ttggagggat gaaccgccgt 840
   ccaattttaa tcattgttac tctggaaacc agagatgggc aagtcctggg ccgacgctgc 900
   tttgaggccc ggatctgtgc ttgcccagga agagacagga aggcggatga agatagcatc 960
   agaaagcagc aagtttcgga cagtacaaag aacggtgatg gtacgaagcg cccgtttcgt 1020
   cagaacacac atggtatcca gatgacatcc atcaagaaac gaagatcccc agatgatgaa 1080
   ctgttatact taccagtgag gggccgtgag acttatgaaa tgctgttgaa gatcaaagag 1140
   tccctggaac tcatgcagta ccttcctcag cacacaattg aaacgtacag gcaacagcaa 1200
   cagcagcagc accagcactt acttcagaaa cagacctcaa tacagtctcc atcttcatat 1260
   ggtaacagct ccccacctct gaacaaaatg aacagcatga acaagctgcc ttctgtgagc 1320
   cagcttatca accctcagca gcgcaacgcc ctcactccta caaccattcc tgatggcatg 1380
   ggagccaaca ttcccatgat gggcacccac atgccaatgg ctggagacat gaatggactc 1440
   agccccaccc aggcactccc tcccccactc tccatgccat ccacctccca ctgcacaccc 1500
   ccacctccgt atcccacaga ttgcagcatt gtcaggatct ggcaagtctg a          1551
<210> 338
   <211> 586
   <212> PRT
   <213> Homo sapiens
<400> 338
<210> 339
   <211> 641
   <212> PRT
   <213> Homo sapiens
<400> 339
<210> 340
   <211> 448
   <212> PRT
   <213> Homo sapiens
<400> 340
   Met Ser Gln Ser Thr Gln Thr Asn Glu Phe Leu Ser Pro Glu Val Phe
    1               5                  10                  15
   Gln His Ile Trp Asp Phe Leu Glu Gln Pro Ile Cys Ser Val Gln Pro
               20                  25                  30
   Ile Asp Leu Asn Phe Val Asp Glu Pro Ser Glu Asp Gly Ala Thr Asn
           35                  40                  45
   Lys Ile Glu Ile Ser Met Asp Cys Ile Arg Met Gln Asp Ser Asp Leu
       50                  55                  60
   Ser Asp Pro Met Trp Pro Gln Tyr Thr Asn Leu Gly Leu Leu Asn Ser
   65                  70                  75                  80
   Met Asp Gln Gln Ile Gln Asn Gly Ser Ser Ser Thr Ser Pro Tyr Asn
                   85                  90                  95
   Thr Asp His Ala Gln Asn Ser Val Thr Ala Pro Ser Pro Tyr Ala Gln
               100                 105                 110
   Pro Ser Ser Thr Phe Asp Ala Leu Ser Pro Ser Pro Ala Ile Pro Ser
           115                 120                 125
   Asn Thr Asp Tyr Pro Gly Pro His Ser Phe Asp Val Ser Phe Gln Gln
       130                 135                 140
   Ser Ser Thr Ala Lys Ser Ala Thr Trp Thr Tyr Ser Thr Glu Leu Lys
   145                 150                 155                 160
   Lys Leu Tyr Cys Gln Ile Ala Lys Thr Cys Pro Ile Gln Ile Lys Val
                   165                 170                 175
   Met Thr Pro Pro Pro Gln Gly Ala Val Ile Arg Ala Met Pro Val Tyr
               180                 185                 190
   Lys Lys Ala Glu His Val Thr Glu Val Val Lys Arg Cys Pro Asn His
           195                 200                 205
   Glu Leu Ser Arg Glu Phe Asn Glu Gly Gln Ile Ala Pro Pro Ser His
       210                 215                 220
   Leu Ile Arg Val Glu Gly Asn Ser His Ala Gln Tyr Val Glu Asp Pro
   225                 230                 235                 240
   Ile Thr Gly Arg Gln Ser Val Leu Val Pro Tyr Glu Pro Pro Gln Val
                   245                 250                 255
   Gly Thr Glu Phe Thr Thr Val Leu Tyr Asn Phe Met Cys Asn Ser Ser
               260                 265                 270
   Cys Val Gly Gly Met Asn Arg Arg Pro Ile Leu Ile Ile Val Thr Leu
           275                 280                 285
   Glu Thr Arg Asp Gly Gln Val Leu Gly Arg Arg Cys Phe Glu Ala Arg
       290                 295                 300
   Ile Cys Ala Cys Pro Gly Arg Asp Arg Lys Ala Asp Glu Asp Ser Ile
   305                 310                 315                 320
   Arg Lys Gln Gln Val Ser Asp Ser Thr Lys Asn Gly Asp Gly Thr Lys
                   325                 330                 335
   Arg Pro Phe Arg Gln Asn Thr His Gly Ile Gln Met Thr Ser Ile Lys
               340                 345                 350
   Lys Arg Arg Ser Pro Asp Asp Glu Leu Leu Tyr Leu Pro Val Arg Gly
           355                 360                 365
   Arg Glu Thr Tyr Glu Met Leu Leu Lys Ile Lys Glu Ser Leu Glu Leu
       370                 375                 380
   Met Gln Tyr Leu Pro Gln His Thr Ile Glu Thr Tyr Arg Gln Gln Gln
   385                 390                 395                 400
   Gln Gln Gln His Gln His Leu Leu Gln Lys His Leu Leu Ser Ala Cys
                   405                 410                 415
   Phe Arg Asn Glu Leu Val Glu Pro Arg Arg Glu Thr Pro Lys Gln Ser
               420                 425                 430
   Asp Val Phe Phe Arg His Ser Lys Pro Pro Asn Arg Ser Val Tyr Pro
           435                 440                 445
<210> 341
   <211> 356
   <212> PRT
   <213> Homo sapiens
<400> 341
<210> 342
   <211> 680
   <212> PRT
   <213> Homo sapiens
<400> 342
   Met Asn Phe Glu Thr Ser Arg Cys Ala Thr Leu Gln Tyr Cys Pro Asp
   1               5                   10                  15
   Pro Tyr Ile Gln Arg Phe Val Glu Thr Pro Ala His Phe Ser Trp Lys
               20                  25                  30
   Glu Ser Tyr Tyr Arg Ser Thr Met Ser Gln Ser Thr Gln Thr Asn Glu
           35                  40                  45
   Phe Leu Ser Pro Glu Val Phe Gln His Ile Trp Asp Phe Leu Glu Gln
       50                  55                  60
   Pro Ile Cys Ser Val Gln Pro Ile Asp Leu Asn Phe Val Asp Glu Pro
   65                  70                  75                  80
   Ser Glu Asp Gly Ala Thr Asn Lys Ile Glu Ile Ser Met Asp Cys Ile
                   85                  90                  95
   Arg Met Gln Asp Ser Asp Leu Ser Asp Pro Met Trp Pro Gln Tyr Thr
               100                 105                 110
   Asn Leu Gly Leu Leu Asn Ser Met Asp Gln Gln Ile Gln Asn Gly Ser
           115                 120                 125
   Ser Ser Thr Ser Pro Tyr Asn Thr Asp His Ala Gln Asn Ser Val Thr
       130                 135                 140
   Ala Pro Ser Pro Tyr Ala Gln Pro Ser Ser Thr Phe Asp Ala Leu Ser
   145                 150                 155                 160
   Pro Ser Pro Ala Ile Pro Ser Asn Thr Asp Tyr Pro Gly Pro His Ser
                   165                 170                 175
   Phe Asp Val Ser Phe Gln Gln Ser Ser Thr Ala Lys Ser Ala Thr Trp
               180                 185                 190
   Thr Tyr Ser Thr Glu Leu Lys Lys Leu Tyr Cys Gln Ile Ala Lys Thr
           195                 200                 205
   Cys Pro Ile Gln Ile Lys Val Met Thr Pro Pro Pro Gln Gly Ala Val
       210                 215                 220
   Ile Arg Ala Met Pro Val Tyr Lys Lys Ala Glu His Val Thr Glu Val
   225                 230                 235                 240
   Val Lys Arg Cys Pro Asn His Glu Leu Ser Arg Glu Phe Asn Glu Gly
                   245                 250                 255
   Gln Ile Ala Pro Pro Ser His Leu Ile Arg Val Glu Gly Asn Ser His
               260                 265                 270
   Ala Gln Tyr Val Glu Asp Pro Ile Thr Gly Arg Gln Ser Val Leu Val
           275                 280                 285
   Pro Tyr Glu Pro Pro Gln Val Gly Thr Glu Phe Thr Thr Val Leu Tyr
       290                 295                 300
   Asn Phe Met Cys Asn Ser Ser Cys Val Gly Gly Met Asn Arg Arg Pro
   305                 310                 315                 320
   Ile Leu Ile Ile Val Thr Leu Glu Thr Arg Asp Gly Gln Val Leu Gly
                   325                 330                 335
   Arg Arg Cys Phe Glu Ala Arg Ile Cys Ala Cys Pro Gly Arg Asp Arg
               340                 345                 350
   Lys Ala Asp Glu Asp Ser Ile Arg Lys Gln Gln Val Ser Asp Ser Thr
           355                 360                 365
   Lys Asn Gly Asp Gly Thr Lys Arg Pro Phe Arg Gln Asn Thr His Gly
       370                 375                 380
   Ile Gln Met Thr Ser Ile Lys Lys Arg Arg Ser Pro Asp Asp Glu Leu
   385                 390                 395                 400
   Leu Tyr Leu Pro Val Arg Gly Arg Glu Thr Tyr Glu Met Leu Leu Lys
                   405                 410                 415
   Ile Lys Glu Ser Leu Glu Leu Met Gln Tyr Leu Pro Gln His Thr Ile
               420                 425                 430
   Glu Thr Tyr Arg Gln Gln Gln Gln Gln Gln His Gln His Leu Leu Gln
           435                 440                 445
   Lys Gln Thr Ser Ile Gln Ser Pro Ser Ser Tyr Gly Asn Ser Ser Pro
       450                 455                 460
   Pro Leu Asn Lys Met Asn Ser Met Asn Lys Leu Pro Ser Val Ser Gln
   465                 470                 475                 480
   Leu Ile Asn Pro Gln Gln Arg Asn Ala Leu Thr Pro Thr Thr Ile Pro
                   485                 490                 495
   Asp Gly Met Gly Ala Asn Ile Pro Met Met Gly Thr His Met Pro Met
               500                 505                 510
   Ala Gly Asp Met Asn Gly Leu Ser Pro Thr Gln Ala Leu Pro Pro Pro
           515                 520                 525
   Leu Ser Met Pro Ser Thr Ser Gln Cys Thr Pro Pro Pro Pro Tyr Pro
       530                 535                 540
   Thr Asp Cys Ser Ile Val Ser Phe Leu Ala Arg Leu Gly Cys Ser Ser
   545                 550                 555                 560
   Cys Leu Asp Tyr Phe Thr Thr Gln Gly Leu Thr Thr Ile Tyr Gln Ile
                   565                 570                 575
   Glu His Tyr Ser Met Asp Asp Leu Ala Ser Leu Lys Ile Pro Glu Gln
               580                 585                 590
   Phe Arg His Ala Ile Trp Lys Gly Ile Leu Asp His Arg Gln Leu His
           595                 600                 605
   Glu Phe Ser Ser Pro Ser His Leu Leu Arg Thr Pro Ser Ser Ala Ser
       610                 615                 620
   Thr Val Ser Val Gly Ser Ser Glu Thr Arg Gly Glu Arg Val Ile Asp
   625                 630                 635                 640
   Ala Val Arg Phe Thr Leu Arg Gln Thr Ile Ser Phe Pro Pro Arg Asp
                   645                 650                 655
   Glu Trp Asn Asp Phe Asn Phe Asp Met Asp Ala Arg Arg Asn Lys Gln
               660                 665                 670
   Gln Arg Ile Lys Glu Glu Gly Glu
           675                 680
<210> 343
   <211> 461
   <212> PRT
   <213> Homo sapiens
<400> 343
   Met Leu Tyr Leu Glu Asn Asn Ala Gln Thr Gln Phe Ser Glu Pro Gln
    1               5                  10                  15
   Tyr Thr Asn Leu Gly Leu Leu Asn Ser Met Asp Gln Gln Ile Gln Asn
               20                  25                  30
   Gly Ser Ser Ser Thr Ser Pro Tyr Asn Thr Asp His Ala Gln Asn Ser
           35                  40                  45
   Val Thr Ala Pro Ser Pro Tyr Ala Gln Pro Ser Ser Thr Phe Asp Ala
       50                  55                  60
   Leu Ser Pro Ser Pro Ala Ile Pro Ser Asn Thr Asp Tyr Pro Gly Pro
   65                  70                  75                  80
   His Ser Phe Asp Val Ser Phe Gln Gln Ser Ser Thr Ala Lys Ser Ala
                   85                  90                  95
   Thr Trp Thr Tyr Ser Thr Glu Leu Lys Lys Leu Tyr Cys Gln Ile Ala
               100                 105                 110
   Lys Thr Cys Pro Ile Gln Ile Lys Val Met Thr Pro Pro Pro Gln Gly
           115                 120                 125
   Ala Val Ile Arg Ala Met Pro Val Tyr Lys Lys Ala Glu His Val Thr
       130                 135                 140
   Glu Val Val Lys Arg Cys Pro Asn His Glu Leu Ser Arg Glu Phe Asn
   145                 150                 155                 160
   Glu Gly Gln Ile Ala Pro Pro Ser His Leu Ile Arg Val Glu Gly Asn
                   165                 170                 175
   Ser His Ala Gln Tyr Val Glu Asp Pro Ile Thr Gly Arg Gln Ser Val
               180                 185                 190
   Leu Val Pro Tyr Glu Pro Pro Gln Val Gly Thr Glu Phe Thr Thr Val
           195                 200                 205
   Leu Tyr Asn Phe Met Cys Asn Ser Ser Cys Val Gly Gly Met Asn Arg
       210                 215                 220
   Arg Pro Ile Leu Ile Ile Val Thr Leu Glu Thr Arg Asp Gly Gln Val
   225                 230                 235                 240
   Leu Gly Arg Arg Cys Phe Glu Ala Arg Ile Cys Ala Cys Pro Gly Arg
                   245                 250                 255
   Asp Arg Lys Ala Asp Glu Asp Ser Ile Arg Lys Gln Gln Val Ser Asp
               260                 265                 270
   Ser Thr Lys Asn Gly Asp Gly Thr Lys Arg Pro Phe Arg Gln Asn Thr
           275                 280                 285
   His Gly Ile Gln Met Thr Ser Ile Lys Lys Arg Arg Ser Pro Asp Asp
       290                 295                 300
   Glu Leu Leu Tyr Leu Pro Val Arg Gly Arg Glu Thr Tyr Glu Met Leu
   305 310 315 320
   Leu Lys Ile Lys Glu Ser Leu Glu Leu Met Gln Tyr Leu Pro Gln His
   325 330 335
   Thr Ile Glu Thr Tyr Arg Gln Gln Gln Gln Gln Gln His Gln His Leu
   340 345 350
   Leu Gln Lys Gln Thr Ser Ile Gln Ser Pro Ser Ser Tyr Gly Asn Ser
   355 360 365
   Ser Pro Pro Leu Asn Lys Met Asn Ser Met Asn Lys Leu Pro Ser Val
   370 375 380
   Ser Gln Leu Ile Asn Pro Gln Gln Arg Asn Ala Leu Thr Pro Thr Thr
   385 390 395 400
   Ile Pro Asp Gly Met Gly Ala Asn Ile Pro Met Met Gly Thr His Met
   405 410 415
   Pro Met Ala Gly Asp Met Asn Gly Leu Ser Pro Thr Gln Ala Leu Pro
   420 425 430
   Pro Pro Leu Ser Met Pro Ser Thr Ser His Cys Thr Pro Pro Pro Pro
   435 440 445
   Tyr Pro Thr Asp Cys Ser Ile Val Arg Ile Trp Gln Val
   450 455 460
<210> 344
   <211> 516
   <212> PRT
   <213> Homo sapiens
<400> 344
   Met Ser Gln Ser Thr Gln Thr Asn Glu Phe Leu Ser Pro Glu Val Phe
    1               5                  10                  15
   Gln His Ile Trp Asp Phe Leu Glu Gln Pro Ile Cys Ser Val Gln Pro
               20                  25                  30
   Ile Asp Leu Asn Phe Val Asp Glu Pro Ser Glu Asp Gly Ala Thr Asn
           35                  40                  45
   Lys Ile Glu Ile Ser Met Asp Cys Ile Arg Met Gln Asp Ser Asp Leu
       50                  55                  60
   Ser Asp Pro Met Trp Pro Gln Tyr Thr Asn Leu Gly Leu Leu Asn Ser
   65                  70                  75                  80
   Met Asp Gln Gln Ile Gln Asn Gly Ser Ser Ser Thr Ser Pro Tyr Asn
                   85                  90                  95
   Thr Asp His Ala Gln Asn Ser Val Thr Ala Pro Ser Pro Tyr Ala Gln
               100                 105                 110
   Pro Ser Ser Thr Phe Asp Ala Leu Ser Pro Ser Pro Ala Ile Pro Ser
           115                 120                 125
   Asn Thr Asp Tyr Pro Gly Pro His Ser Phe Asp Val Ser Phe Gln Gln
       130                 135                 140
   Ser Ser Thr Ala Lys Ser Ala Thr Trp Thr Tyr Ser Thr Glu Leu Lys
   145                 150                 155                 160
   Lys Leu Tyr Cys Gln Ile Ala Lys Thr Cys Pro Ile Gln Ile Lys Val
                   165                 170                 175
   Met Thr Pro Pro Pro Gln Gly Ala Val Ile Arg Ala Met Pro Val Tyr
               180                 185                 190
   Lys Lys Ala Glu His Val Thr Glu Val Val Lys Arg Cys Pro Asn His
           195                 200                 205
   Glu Leu Ser Arg Glu Phe Asn Glu Gly Gln Ile Ala Pro Pro Ser His
       210                 215                 220
   Leu Ile Arg Val Glu Gly Asn Ser His Ala Gln Tyr Val Glu Asp Pro
   225                 230                 235                 240
   Ile Thr Gly Arg Gln Ser Val Leu Val Pro Tyr Glu Pro Pro Gln Val
                   245                 250                 255
   Gly Thr Glu Phe Thr Thr Val Leu Tyr Asn Phe Met Cys Asn Ser Ser
               260                 265                 270
   Cys Val Gly Gly Met Asn Arg Arg Pro Ile Leu Ile Ile Val Thr Leu
           275                 280                 285
   Glu Thr Arg Asp Gly Gln Val Leu Gly Arg Arg Cys Phe Glu Ala Arg
       290                 295                 300
   Ile Cys Ala Cys Pro Gly Arg Asp Arg Lys Ala Asp Glu Asp Ser Ile
   305                 310                 315                 320
   Arg Lys Gln Gln Val Ser Asp Ser Thr Lys Asn Gly Asp Gly Thr Lys
                   325                 330                 335
   Arg Pro Phe Arg Gln Asn Thr His Gly Ile Gln Met Thr Ser Ile Lys
               340                 345                 350
   Lys Arg Arg Ser Pro Asp Asp Glu Leu Leu Tyr Leu Pro Val Arg Gly
           355                 360                 365
   Arg Glu Thr Tyr Glu Met Leu Leu Lys Ile Lys Glu Ser Leu Glu Leu
       370                 375                 380
   Met Gln Tyr Leu Pro Gln His Thr Ile Glu Thr Tyr Arg Gln Gln Gln
   385                 390                 395                 400
   Gln Gln Gln His Gln His Leu Leu Gln Lys Gln Thr Ser Ile Gln Ser
                   405                 410                 415
   Pro Ser Ser Tyr Gly Asn Ser Ser Pro Pro Leu Asn Lys Met Asn Ser
               420                 425                 430
   Met Asn Lys Leu Pro Ser Val Ser Gln Leu Ile Asn Pro Gln Gln Arg
           435                 440                 445
   Asn Ala Leu Thr Pro Thr Thr Ile Pro Asp Gly Met Gly Ala Asn Ile
       450                 455                 460
   Pro Met Met Gly Thr His Met Pro Met Ala Gly Asp Met Asn Gly Leu
   465                 470                 475                 480
   Ser Pro Thr Gln Ala Leu Pro Pro Pro Leu Ser Met Pro Ser Thr Ser
                   485                 490                 495
   His Cys Thr Pro Pro Pro Pro Tyr Pro Thr Asp Cys Ser Ile Val Arg
               500                 505                 510
   Ile Trp Gln Val
           515
<210> 345
   <211> 1800
   <212> DNA
   <213> Homo sapiens
<400> 345
   gcgcctcatt gccactgcag tgactaaagc tgggaagacg ctggtcagtt cacctgcccc 60
   actggttgtt ttttaaacaa attctgatac aggcgacatc ctcactgacc gagcaaagat 120
   tgacattcgt atcatcactg tgcaccattg gcttctaggc actccagtgg ggtaggagaa 180
   ggaggtctga aaccctcgca gagggatctt gccctcattc tttgggtctg aaacactggc 240
   agtcgttgga aacaggactc agggataaac cagcgcaatg gattggggga cgctgcacac 300
   tttcatcggg ggtgtcaaca aacactccac cagcatcggg aaggtgtgga tcacagtcat 360
   ctttattttc cgagtcatga tcctagtggt ggctgcccag gaagtgtggg gtgacgagca 420
   agaggacttc gtctgcaaca cactgcaacc gggatgcaaa aatgtgtgct atgaccactt 480
   tttcccggtg tcccacatcc ggctgtgggc cctccagctg atcttcgtct ccaccccagc 540
   gctgctggtg gccatgcatg tggcctacta caggcacgaa accactcgca agttcaggcg 600
   aggagagaag aggaatgatt tcaaagacat agaggacatt aaaaagcaca aggttcggat 660
   agaggggtcg ctgtggtgga cgtacaccag cagcatcttt ttccgaatca tctttgaagc 720
   agcctttatg tatgtgtttt acttccttta caatgggtac cacctgccct gggtgttgaa 780
   atgtgggatt gacccctgcc ccaaccttgt tgactgcttt atttctaggc caacagagaa 840
   gaccgtgttt accattttta tgatttctgc gtctgtgatt tgcatgctgc ttaacgtggc 900
   agagttgtgc tacctgctgc tgaaagtgtg ttttaggaga tcaaagagag cacagacgca 960
   aaaaaatcac cccaatcatg ccctaaagga gagtaagcag aatgaaatga atgagctgat 1020
   ttcagatagt ggtcaaaatg caatcacagg tttcccaagc taaacatttc aaggtaaaat 1080
   gtagctgcgt cataaggaga cttctgtctt ctccagaagg caataccaac ctgaaagttc 1140
   cttctgtagc ctgaagagtt tgtaaatgac tttcataata aatagacact tgagttaact 1200
   ttttgtagga tacttgctcc attcatacac aacgtaatca aatatgtggt ccatctctga 1260
   aaacaagaga ctgcttgaca aaggagcatt gcagtcactt tgacaggttc cttttaagtg 1320
   gactctctga caaagtgggt actttctgaa aatttatata actgttgttg ataaggaaca 1380
   tttatccagg aattgatacg tttattagga aaagatattt ttataggctt ggatgttttt 1440
   agttccgact ttgaatttat ataaagtatt tttataatga ctggtcttcc ttacctggaa 1500
   aaacatgcga tgttagtttt agaattacac cacaagtatc taaatttcca acttacaaag 1560
   ggtcctatct tgtaaatatt gttttgcatt gtctgttggc aaatttgtga actgtcatga 1620
   tacgcttaag gtgggaaagt gttcattgca caatatattt ttactgcttt ctgaatgtag 1680
   acggaacagt gtggaagcag aaggcttttt taactcatcc gtttggccga tcgttgcaga 1740
   ccactgggag atgtggatgt ggttgcctcc ttttgctcgt ccccgtggct taacccttct 1800
<210> 346
   <211> 261
   <212> PRT
   <213> Homo sapiens
<400> 346
   Met Asp Trp Gly Thr Leu His Thr Phe Ile Gly Gly Val Asn Lys His
    1               5                  10                  15
   Ser Thr Ser Ile Gly Lys Val Trp Ile Thr Val Ile Phe Ile Phe Arg
               20                  25                  30
   Val Met Ile Leu Val Val Ala Ala Gln Glu Val Trp Gly Asp Glu Gln
           35                  40                  45
   Glu Asp Phe Val Cys Asn Thr Leu Gln Pro Gly Cys Lys Asn Val Cys
       50                  55                  60
   Tyr Asp His Phe Phe Pro Val Ser His Ile Arg Leu Trp Ala Leu Gln
   65                  70                  75                  80
   Leu Ile Phe Val Ser Thr Pro Ala Leu Leu Val Ala Met His Val Ala
                   85                  90                  95
   Tyr Tyr Arg His Glu Thr Thr Arg Lys Phe Arg Arg Gly Glu Lys Arg
               100                 105                 110
   Asn Asp Phe Lys Asp Ile Glu Asp Ile Lys Lys His Lys Val Arg Ile
           115                 120                 125
   Glu Gly Ser Leu Trp Trp Thr Tyr Thr Ser Ser Ile Phe Phe Arg Ile
       130                 135                 140
   Ile Phe Glu Ala Ala Phe Met Tyr Val Phe Tyr Phe Leu Tyr Asn Gly
   145                 150                 155                 160
   Tyr His Leu Pro Trp Val Leu Lys Cys Gly Ile Asp Pro Cys Pro Asn
                   165                 170                 175
   Leu Val Asp Cys Phe Ile Ser Arg Pro Thr Glu Lys Thr Val Phe Thr
               180                 185                 190
   Ile Phe Met Ile Ser Ala Ser Val Ile Cys Met Leu Leu Asn Val Ala
           195                 200                 205
   Glu Leu Cys Tyr Leu Leu Leu Lys Val Cys Phe Arg Arg Ser Lys Arg
       210                 215                 220
   Ala Gln Thr Gln Lys Asn His Pro Asn His Ala Leu Lys Glu Ser Lys
   225                 230                 235                 240
   Gln Asn Glu Met Asn Glu Leu Ile Ser Asp Ser Gly Gln Asn Ala Ile
                   245                 250                 255
   Thr Gly Phe Pro Ser
               260
<210> 347
   <211> 1740
   <212> DNA
   <213> Homo sapiens
<400> 347
   atgaacaaac tgtatatcgg aaacctcagc gagaacgccg ccccctcgga cctagaaagt 60
   atcttcaagg acgccaagat cccggtgtcg ggacccttcc tggtgaagac tggctacgcg 120
   ttcgtggact gcccggacga gagctgggcc ctcaaggcca tcgaggcgct ttcaggtaaa 180
   atagaactgc acgggaaacc catagaagtt gagcactcgg tcccaaaaag gcaaaggatt 240
   cggaaacttc agatacgaaa tatcccgcct catttacagt gggaggtgct ggatagttta 300
   ctagtccagt atggagtggt ggagagctgt gagcaagtga acactgactc ggaaactgca 360
   gttgtaaatg taacctattc cagtaaggac caagctagac aagcactaga caaactgaat 420
   ggatttcagt tagagaattt caccttgaaa gtagcctata tccctgatga aacggccgcc 480
   cagcaaaacc ccttgcagca gccccgaggt cgccgggggc ttgggcagag gggctcctca 540
   aggcaggggt ctccaggatc cgtatccaag cagaaaccat gtgatttgcc tctgcgcctg 600
   ctggttccca cccaatttgt tggagccatc ataggaaaag aaggtgccac cattcggaac 660
   atcaccaaac agacccagtc taaaatcgat gtccaccgta aagaaaatgc gggggctgct 720
   gagaagtcga ttactatcct ctctactcct gaaggcacct ctgcggcttg taagtctatt 780
   ctggagatta tgcataagga agctcaagat ataaaattca cagaagagat ccccttgaag 840
   attttagctc ataataactt tgttggacgt cttattggta aagaaggaag aaatcttaaa 900
   aaaattgagc aagacacaga cactaaaatc acgatatctc cattgcagga attgacgctg 960
   tataatccag aacgcactat tacagttaaa ggcaatgttg agacatgtgc caaagctgag 1020
   gaggagatca tgaagaaaat cagggagtct tatgaaaatg atattgcttc tatgaatctt 1080
   caagcacatt taattcctgg attaaatctg aacgccttgg gtctgttccc acccacttca 1140
   gggatgccac ctcccacctc agggccccct tcagccatga ctcctcccta cccgcagttt 1200
   gagcaatcag aaacggagac tgttcatctg tttatcccag ctctatcagt cggtgccatc 1260
   atcggcaagc agggccagca catcaagcag ctttctcgct ttgctggagc ttcaattaag 1320
   attgctccag cggaagcacc agatgctaaa gtgaggatgg tgattatcac tggaccacca 1380
   gaggctcagt tcaaggctca gggaagaatt tatggaaaaa ttaaagaaga aaactttgtt 1440
   agtcctaaag aagaggtgaa acttgaagct catatcagag tgccatcctt tgctgctggc 1500
   agagttattg gaaaaggagg caaaacggtg aatgaacttc agaatttgtc aagtgcagaa 1560
   gttgttgtcc ctcgtgacca gacacctgat gagaatgacc aagtggttgt caaaataact 1620
   ggtcacttct atgcttgcca ggttgcccag agaaaaattc aggaaattct gactcaggta 1680
   aagcagcacc aacaacagaa ggctctgcaa agtggaccac ctcagtcaag acggaagtaa 1740
<210> 348
   <211> 579
   <212> PRT
   <213> Homo sapiens
<400> 348
   Met Asn Lys Leu Tyr Ile Gly Asn Leu Ser Glu Asn Ala Ala Pro Ser
   1               5                   10                  15
   Asp Leu Glu Ser Ile Phe Lys Asp Ala Lys Ile Pro Val Ser Gly Pro
               20                  25                  30
   Phe Leu Val Lys Thr Gly Tyr Ala Phe Val Asp Cys Pro Asp Glu Ser
           35                  40                  45
   Trp Ala Leu Lys Ala Ile Glu Ala Leu Ser Gly Lys Ile Glu Leu His
       50                  55                  60
   Gly Lys Pro Ile Glu Val Glu His Ser Val Pro Lys Arg Gln Arg Ile
   65                  70                  75                  80
   Arg Lys Leu Gln Ile Arg Asn Ile Pro Pro His Leu Gln Trp Glu Val
                   85                  90                  95
   Leu Asp Ser Leu Leu Val Gln Tyr Gly Val Val Glu Ser Cys Glu Gln
                   100                 105                 110
   Val Asn Thr Asp Ser Glu Thr Ala Val Val Asn Val Thr Tyr Ser Ser
               115                 120                 125
   Lys Asp Gln Ala Arg Gln Ala Leu Asp Lys Leu Asn Gly Phe Gln Leu
       130                 135                 140
   Glu Asn Phe Thr Leu Lys Val Ala Tyr Ile Pro Asp Glu Thr Ala Ala
   145                 150                 155                 160
   Gln Gln Asn Pro Leu Gln Gln Pro Arg Gly Arg Arg Gly Leu Gly Gln
                   165                 170                 175
   Arg Gly Ser Ser Arg Gln Gly Ser Pro Gly Ser Val Ser Lys Gln Lys
               180                 185                 190
   Pro Cys Asp Leu Pro Leu Arg Leu Leu Val Pro Thr Gln Phe Val Gly
           195                 200                 205
   Ala Ile Ile Gly Lys Glu Gly Ala Thr Ile Arg Asn Ile Thr Lys Gln
       210                 215                 220
   Thr Gln Ser Lys Ile Asp Val His Arg Lys Glu Asn Ala Gly Ala Ala
   225                 230                 235                 240
   Glu Lys Ser Ile Thr Ile Leu Ser Thr Pro Glu Gly Thr Ser Ala Ala
                   245                 250                 255
   Cys Lys Ser Ile Leu Glu Ile Met His Lys Glu Ala Gln Asp Ile Lys
               260                 265                 270
   Phe Thr Glu Glu Ile Pro Leu Lys Ile Leu Ala His Asn Asn Phe Val
           275                 280                 285
   Gly Arg Leu Ile Gly Lys Glu Gly Arg Asn Leu Lys Lys Ile Glu Gln
       290                 295                 300
   Asp Thr Asp Thr Lys Ile Thr Ile Ser Pro Leu Gln Glu Leu Thr Leu
   305                 310                 315                 320
   Tyr Asn Pro Glu Arg Thr Ile Thr Val Lys Gly Asn Val Glu Thr Cys
                   325                 330                 335
   Ala Lys Ala Glu Glu Glu Ile Met Lys Lys Ile Arg Glu Ser Tyr Glu
               340                 345                 350
   Asn Asp Ile Ala Ser Met Asn Leu Gln Ala His Leu Ile Pro Gly Leu
           355                 360                 365
   Asn Leu Asn Ala Leu Gly Leu Phe Pro Pro Thr Ser Gly Met Pro Pro
       370                 375                 380
   Pro Thr Ser Gly Pro Pro Ser Ala Met Thr Pro Pro Tyr Pro Gln Phe
   385                 390                 395                 400
   Glu Gln Ser Glu Thr Glu Thr Val His Leu Phe Ile Pro Ala Leu Ser
                   405                 410                 415
   Val Gly Ala Ile Ile Gly Lys Gln Gly Gln His Ile Lys Gln Leu Ser
               420                 425                 430
   Arg Phe Ala Gly Ala Ser Ile Lys Ile Ala Pro Ala Glu Ala Pro Asp
           435                 440                 445
   Ala Lys Val Arg Met Val Ile Ile Thr Gly Pro Pro Glu Ala Gln Phe
       450                 455                 460
   Lys Ala Gln Gly Arg Ile Tyr Gly Lys Ile Lys Glu Glu Asn Phe Val
   465                 470                 475                 480
   Ser Pro Lys Glu Glu Val Lys Leu Glu Ala His Ile Arg Val Pro Ser
                   485                 490                 495
   Phe Ala Ala Gly Arg Val Ile Gly Lys Gly Gly Lys Thr Val Asn Glu
               500                 505                 510
   Leu Gln Asn Leu Ser Ser Ala Glu Val Val Val Pro Arg Asp Gln Thr
           515                 520                 525
   Pro Asp Glu Asn Asp Gln Val Val Val Lys Ile Thr Gly His Phe Tyr
       530                 535                 540
   Ala Cys Gln Val Ala Gln Arg Lys Ile Gln Glu Ile Leu Thr Gln Val
   545                 550                 555                 560
   Lys Gln His Gln Gln Gln Lys Ala Leu Gln Ser Gly Pro Pro Gln Ser
   565 570 575
   Arg Arg Lys
<210> 349
   <211> 207
   <212> DNA
   <213> Homo sapiens
<400> 349
   atgtggcagc ccctcttctt caagtggctc ttgtcctgtt gccctgggag ttctcaaatt 60
   gctgcagcag cctccaccca gcctgaggat gacatcaata cacagaggaa gaagagtcag 120
   gaaaagatga gagaagttac agactctcct gggcgacccc gagagcttac cattcctcag 180
   acttcttcac atggtgctaa cagattt                                     207
<210> 350
   <211> 69
   <212> PRT
   <213> Homo sapiens
<400> 350
   Met Trp Gln Pro Leu Phe Phe Lys Trp Leu Leu Ser Cys Cys Pro Gly
    1               5                  10                  15
   Ser Ser Gln Ile Ala Ala Ala Ala Ser Thr Gln Pro Glu Asp Asp Ile
               20                  25                  30
   Asn Thr Gln Arg Lys Lys Ser Gln Glu Lys Met Arg Glu Val Thr Asp
           35                  40                  45
   Ser Pro Gly Arg Pro Arg Glu Leu Thr Ile Pro Gln Thr Ser Ser His
       50                  55                  60
   Gly Ala Asn Arg Phe
   65
<210> 351
   <211> 1012
   <212> DNA
   <213> Homo sapiens
<400> 351
   ccctctagaa ataattttgt ttaactttaa gaaggagata tacatatgca tcaccatcac 60
   catcacacgg ccgcgtccga taacttccag ctgtcccagg gtgggcaggg attcgccatt 120
   ccgatcgggc aggcgatggc gatcgcgggc cagatcaagc ttcccaccgt tcatatcggg 180
   cctaccgcct tcctcggctt gggtgttgtc gacaacaacg gcaacggcgc acgagtccaa 240
   cgcgtggtcg ggagcgctcc ggcggcaagt ctcggcatct ccaccggcga cgtgatcacc 300
   gcggtcgacg gcgctccgat caactcggcc accgcgatgg cggacgcgct taacgggcat 360
   catcccggtg acgtcatctc ggtgacctgg caaaccaagt cgggcggcac gcgtacaggg 420
   aacgtgacat tggccgaggg acccccggcc gaattcatgg attgggggac gctgcacact 480
   ttcatcgggg gtgtcaacaa acactccacc agcatcggga aggtgtggat cacagtcatc 540
   tttattttcc gagtcatgat cctcgtggtg gctgcccagg aagtgtgggg tgacgagcaa 600
   gaggacttcg tctgcaacac actgcaaccg ggatgcaaaa atgtgtgcta tgaccacttt 660
   ttcccggtgt cccacatccg gctgtgggcc ctccagctga tcttcgtctc caccccagcg 720
   ctgctggtgg ccatgcatgt ggcctactac aggcacgaaa ccactcgcaa gttcaggcga 780
   ggagagaaga ggaatgattt caaagacata gaggacatta aaaagcagaa ggttcggata 840
   gaggggtgac tcgagcacca ccaccaccac cactgagatc cggctgctaa caaagcccga 900
   aaggaagctg agttggctgc tgccaccgct gagcaataac tagcataacc ccttggggcc 960
   tctaaacggg tcttgagggg ttttttgctg aaaggaggaa ctatatccgg at        1012
<210> 352
   <211> 267
   <212> PRT
   <213> Homo sapiens
<400> 352
   Met His His His His His His Thr Ala Ala Ser Asp Asn Phe Gln Leu
    1               5                  10                  15
   Ser Gln Gly Gly Gln Gly Phe Ala Ile Pro Ile Gly Gln Ala Met Ala
               20                  25                  30
   Ile Ala Gly Gln Ile Lys Leu Pro Thr Val His Ile Gly Pro Thr Ala
           35                  40                  45
   Phe Leu Gly Leu Gly Val Val Asp Asn Asn Gly Asn Gly Ala Arg Val
       50                  55                  60
   Gln Arg Val Val Gly Ser Ala Pro Ala Ala Ser Leu Gly Ile Ser Thr
   65                  70                  75                  80
   Gly Asp Val Ile Thr Ala Val Asp Gly Ala Pro Ile Asn Ser Ala Thr
                   85                  90                  95
   Ala Met Ala Asp Ala Leu Asn Gly His His Pro Gly Asp Val Ile Ser
               100                 105                 110
   Val Thr Trp Gln Thr Lys Ser Gly Gly Thr Arg Thr Gly Asn Val Thr
           115                 120                 125
   Leu Ala Glu Gly Pro Pro Ala Glu Phe Met Asp Trp Gly Thr Leu His
       130                 135                 140
   Thr Phe Ile Gly Gly Val Asn Lys His Ser Thr Ser Ile Gly Lys Val
   145                 150                 155                 160
   Trp Ile Thr Val Ile Phe Ile Phe Arg Val Met Ile Leu Val Val Ala
                   165                 170                 175
   Ala Gln Glu Val Trp Gly Asp Glu Gln Glu Asp Phe Val Cys Asn Thr
               180                 185                 190
   Leu Gln Pro Gly Cys Lys Asn Val Cys Tyr Asp His Phe Phe Pro Val
           195                 200                 205
   Ser His Ile Arg Leu Trp Ala Leu Gln Leu Ile Phe Val Ser Thr Pro
       210                 215                 220
   Ala Leu Leu Val Ala Met His Val Ala Tyr Tyr Arg His Glu Thr Thr
   225                 230                 235                 240
   Arg Lys Phe Arg Arg Gly Glu Lys Arg Asn Asp Phe Lys Asp Ile Glu
                   245                 250                 255
   Asp Ile Lys Lys Gln Lys Val Arg Ile Glu Gly
               260                 265
<210> 353
   <211> 900
   <212> DNA
   <213> Homo sapiens
<400> 353
   atgcatcacc atcaccatca cacggccgcg tccgataact tccagctgtc ccagggtggg 60
   cagggattcg ccattccgat cgggcaggcg atggcgatcg cgggccagat caagcttccc 120
   accgttcata tcgggcctac cgccttcctc ggcttgggtg ttgtcgacaa caacggcaac 180
   ggcgcacgag tccaacgcgt ggtcgggagc gctccggcgg caagtctcgg catctccacc 240
   ggcgacgtga tcaccgcggt cgacggcgct ccgatcaact cggccaccgc gatggcggac 300
   gcgcttaacg ggcatcatcc cggtgacgtc atctcggtga cctggcaaac caagtcgggc 360
   ggcacgcgta cagggaacgt gacattggcc gagggacccc cggccgaatt ccacgaaacc 420
   actcgcaagt tcaggcgagg agagaagagg aatgatttca aagacataga ggacattaaa 480
   aagcagaagg ttcggataga ggggtcgctg tggtggacgt acaccagcag catctttttc 540
   cgaatcatct ttgaagcagc ctttatgtat gtgttttact tcctttacaa tgggtaccac 600
   ctgccctggg tgttgaaatg tgggattgac ccctgcccca accttgttga ctgctttatt 660
   tctaggccaa cagagaagac cgtgtttacc atttttatga tttctgcgtc tgtgatttgc 720
   atgctgctta acgtggcaga gttgtgctac ctgctgctga aagtgtgttt taggagatca 780
   aagagagcac agacgcaaaa aaatcacccc aatcatgccc taaaggagag taagcagaat 840
   gaaatgaatg agctgatttc agatagtggt caaaatgcaa tcacaggttt cccaagctaa 900
<210> 354
   <211> 299
   <212> PRT
   <213> Homo sapiens
<400> 354
   Met His His His His His His Thr Ala Ala Ser Asp Asn Phe Gln Leu
    1               5                  10                  15
   Ser Gln Gly Gly Gln Gly Phe Ala Ile Pro Ile Gly Gln Ala Met Ala
               20                  25                  30
   Ile Ala Gly Gln Ile Lys Leu Pro Thr Val His Ile Gly Pro Thr Ala
           35                  40                  45
   Phe Leu Gly Leu Gly Val Val Asp Asn Asn Gly Asn Gly Ala Arg Val
       50                  55                  60
   Gln Arg Val Val Gly Ser Ala Pro Ala Ala Ser Leu Gly Ile Ser Thr
   65                  70                  75                  80
   Gly Asp Val Ile Thr Ala Val Asp Gly Ala Pro Ile Asn Ser Ala Thr
                   85                  90                  95
   Ala Met Ala Asp Ala Leu Asn Gly His His Pro Gly Asp Val Ile Ser
               100                 105                 110
   Val Thr Trp Gln Thr Lys Ser Gly Gly Thr Arg Thr Gly Asn Val Thr
           115                 120                 125
   Leu Ala Glu Gly Pro Pro Ala Glu Phe His Glu Thr Thr Arg Lys Phe
       130                 135                 140
   Arg Arg Gly Glu Lys Arg Asn Asp Phe Lys Asp Ile Glu Asp Ile Lys
   145                 150                 155                 160
   Lys Gln Lys Val Arg Ile Glu Gly Ser Leu Trp Trp Thr Tyr Thr Ser
                   165                 170                 175
   Ser Ile Phe Phe Arg Ile Ile Phe Glu Ala Ala Phe Met Tyr Val Phe
               180                 185                 190
   Tyr Phe Leu Tyr Asn Gly Tyr His Leu Pro Trp Val Leu Lys Cys Gly
           195                 200                 205
   Ile Asp Pro Cys Pro Asn Leu Val Asp Cys Phe Ile Ser Arg Pro Thr
       210                 215                 220
   Glu Lys Thr Val Phe Thr Ile Phe Met Ile Ser Ala Ser Val Ile Cys
   225                 230                 235                 240
   Met Leu Leu Asn Val Ala Glu Leu Cys Tyr Leu Leu Leu Lys Val Cys
                   245                 250                 255
   Phe Arg Arg Ser Lys Arg Ala Gln Thr Gln Lys Asn His Pro Asn His
               260                 265                 270
   Ala Leu Lys Glu Ser Lys Gln Asn Glu Met Asn Glu Leu Ile Ser Asp
           275                 280                 285
   Ser Gly Gln Asn Ala Ile Thr Gly Phe Pro Ser
       290                 295
<210> 355
   <211> 24
   <212> DNA
   <213> Artificial Sequence
<220>
   <223> PCR primer
<400> 355
   ggagtacagc ttcaagacaa tggg 24
<210> 356
   <211> 31
   <212> DNA
   <213> Artificial Sequence
<220>
   <223> PCR primer
<400> 356
   ccatgggaat tcattataat aattttgttc c 31
<210> 357
   <211> 920
   <212> PRT
   <213> Homo sapiens
<400> 357
   Met Gln His His His His His His Gly Val Gln Leu Gln Asp Asn Gly
    1               5                  10                  15
   Tyr Asn Gly Leu Leu Ile Ala Ile Asn Pro Gln Val Pro Glu Asn Gln
               20                  25                  30
   Asn Leu Ile Ser Asn Ile Lys Glu Met Ile Thr Glu Ala Ser Phe Tyr
           35                  40                  45
   Leu Phe Asn Ala Thr Lys Arg Arg Val Phe Phe Arg Asn Ile Lys Ile
       50                  55                  60
   Leu Ile Pro Ala Thr Trp Lys Ala Asn Asn Asn Ser Lys Ile Lys Gln
   65                  70                  75                  80
   Glu Ser Tyr Glu Lys Ala Asn Val Ile Val Thr Asp Trp Tyr Gly Ala
                   85                  90                  95
   His Gly Asp Asp Pro Tyr Thr Leu Gln Tyr Arg Gly Cys Gly Lys Glu
               100                 105                 110
   Gly Lys Tyr Ile His Phe Thr Pro Asn Phe Leu Leu Asn Asp Asn Leu
           115                 120                 125
   Thr Ala Gly Tyr Gly Ser Arg Gly Arg Val Phe Val His Glu Trp Ala
       130                 135                 140
   His Leu Arg Trp Gly Val Phe Asp Glu Tyr Asn Asn Asp Lys Pro Phe
   145                 150                 155                 160
   Tyr Ile Asn Gly Gln Asn Gln Ile Lys Val Thr Arg Cys Ser Ser Asp
                   165                 170                 175
   Ile Thr Gly Ile Phe Val Cys Glu Lys Gly Pro Cys Pro Gln Glu Asn
               180                 185                 190
   Cys Ile Ile Ser Lys Leu Phe Lys Glu Gly Cys Thr Phe Ile Tyr Asn
           195                 200                 205
   Ser Thr Gln Asn Ala Thr Ala Ser Ile Met Phe Met Gln Ser Leu Ser
       210                 215                 220
   Ser Val Val Glu Phe Cys Asn Ala Ser Thr His Asn Gln Glu Ala Pro
   225                 230                 235                 240
   Asn Leu Gln Asn Gln Met Cys Ser Leu Arg Ser Ala Trp Asp Val Ile
                   245                 250                 255
   Thr Asp Ser Ala Asp Phe His His Ser Phe Pro Met Asn Gly Thr Glu
               260                 265                 270
   Leu Pro Pro Pro Pro Thr Phe Ser Leu Val Glu Ala Gly Asp Lys Val
           275                 280                 285
   Val Cys Leu Val Leu Asp Val Ser Ser Lys Met Ala Glu Ala Asp Arg
       290                 295                 300
   Leu Leu Gln Leu Gln Gln Ala Ala Glu Phe Tyr Leu Met Gln Ile Val
   305                 310                 315                 320
   Glu Ile His Thr Phe Val Gly Ile Ala Ser Phe Asp Ser Lys Gly Glu
                   325                 330                 335
   Ile Arg Ala Gln Leu His Gln Ile Asn Ser Asn Asp Asp Arg Lys Leu
               340                 345                 350
   Leu Val Ser Tyr Leu Pro Thr Thr Val Ser Ala Lys Thr Asp Ile Ser
           355                 360                 365
   Ile Cys Ser Gly Leu Lys Lys Gly Phe Glu Val Val Glu Lys Leu Asn
       370                 375                 380
   Gly Lys Ala Tyr Gly Ser Val Met Ile Leu Val Thr Ser Gly Asp Asp
   385                 390                 395                 400
   Lys Leu Leu Gly Asn Cys Leu Pro Thr Val Leu Ser Ser Gly Ser Thr
                   405                 410                 415
   Ile His Ser Ile Ala Leu Gly Ser Ser Ala Ala Pro Asn Leu Glu Glu
               420                 425                 430
   Leu Ser Arg Leu Thr Gly Gly Leu Lys Phe Phe Val Pro Asp Ile Ser
           435                 440                 445
   Asn Ser Asn Ser Met Ile Asp Ala Phe Ser Arg Ile Ser Ser Gly Thr
       450                 455                 460
   Gly Asp Ile Phe Gln Gln His Ile Gln Leu Glu Ser Thr Gly Glu Asn
   465                 470                 475                 480
   Val Lys Pro His His Gln Leu Lys Asn Thr Val Thr Val Asp Asn Thr
                   485                 490                 495
   Val Gly Asn Asp Thr Met Phe Leu Val Thr Trp Gln Ala Ser Gly Pro
               500                 505                 510
   Pro Glu Ile Ile Leu Phe Asp Pro Asp Gly Arg Lys Tyr Tyr Thr Asn
           515                 520                 525
   Asn Phe Ile Thr Asn Leu Thr Phe Arg Thr Ala Ser Leu Trp Ile Pro
       530                 535                 540
   Gly Thr Ala Lys Pro Gly His Trp Thr Tyr Thr Leu Asn Asn Thr His
   545                 550                 555                 560
   His Ser Leu Gln Ala Leu Lys Val Thr Val Thr Ser Arg Ala Ser Asn
                   565                 570                 575
   Ser Ala Val Pro Pro Ala Thr Val Glu Ala Phe Val Glu Arg Asp Ser
               580                 585                 590
   Leu His Phe Pro His Pro Val Met Ile Tyr Ala Asn Val Lys Gln Gly
           595                 600                 605
   Phe Tyr Pro Ile Leu Asn Ala Thr Val Thr Ala Thr Val Glu Pro Glu
       610                 615                 620
   Thr Gly Asp Pro Val Thr Leu Arg Leu Leu Asp Asp Gly Ala Gly Ala
   625                 630                 635                 640
   Asp Val Ile Lys Asn Asp Gly Ile Tyr Ser Arg Tyr Phe Phe Ser Phe
                   645                 650                 655
   Ala Ala Asn Gly Arg Tyr Ser Leu Lys Val His Val Asn His Ser Pro
               660                 665                 670
   Ser Ile Ser Thr Pro Ala His Ser Ile Pro Gly Ser His Ala Met Tyr
           675                 680                 685
   Val Pro Gly Tyr Thr Ala Asn Gly Asn Ile Gln Met Asn Ala Pro Arg
       690                 695                 700
   Lys Ser Val Gly Arg Asn Glu Glu Glu Arg Lys Trp Gly Phe Ser Arg
   705                 710                 715                 720
   Val Ser Ser Gly Gly Ser Phe Ser Val Leu Gly Val Pro Ala Gly Pro
                   725                 730                 735
   His Pro Asp Val Phe Pro Pro Cys Lys Ile Ile Asp Leu Glu Ala Val
                   740                 745                 750
   Lys Val Glu Glu Glu Leu Thr Leu Ser Trp Thr Ala Pro Gly Glu Asp
               755                 760                 765
   Phe Asp Gln Gly Gln Ala Thr Ser Tyr Glu Ile Arg Met Ser Lys Ser
       770                 775                 780
   Leu Gln Asn Ile Gln Asp Asp Phe Asn Asn Ala Ile Leu Val Asn Thr
   785                 790                 795                 800
   Ser Lys Arg Asn Pro Gln Gln Ala Gly Ile Arg Glu Ile Phe Thr Phe
                   805                 810                 815
   Ser Pro Gln Ile Ser Thr Asn Gly Pro Glu His Gln Pro Asn Gly Glu
               820                 825                 830
   Thr His Glu Ser His Arg Ile Tyr Val Ala Ile Arg Ala Met Asp Arg
           835                 840                 845
   Asn Ser Leu Gln Ser Ala Val Ser Asn Ile Ala Gln Ala Pro Leu Phe
       850                 855                 860
   Ile Pro Pro Asn Ser Asp Pro Val Pro Ala Arg Asp Tyr Leu Ile Leu
   865                 870                 875                 880
   Lys Gly Val Leu Thr Ala Met Gly Leu Ile Gly Ile Ile Cys Leu Ile
                   885                 890                 895
   Ile Val Val Thr His His Thr Leu Ser Arg Lys Lys Arg Ala Asp Lys
               900                 905                 910
   Lys Glu Asn Gly Thr Lys Leu Leu
           915                 920
<210> 358
   <211> 2773
   <212> DNA
   <213> Homo sapiens
<400> 358
   catatgcagc atcaccacca tcaccacgga gtacagcttc aagacaatgg gtataatgga 60
   ttgctcattg caattaatcc tcaggtacct gagaatcaga acctcatctc aaacattaag 120
   gaaatgataa ctgaagcttc attttaccta tttaatgcta ccaagagaag agtatttttc 180
   agaaatataa agattttaat acctgccaca tggaaagcta ataataacag caaaataaaa 240
   caagaatcat atgaaaaggc aaatgtcata gtgactgact ggtatggggc acatggagat 300
   gatccataca ccctacaata cagagggtgt ggaaaagagg gaaaatacat tcatttcaca 360
   cctaatttcc tactgaatga taacttaaca gctggctacg gatcacgagg ccgagtgttt 420
   gtccatgaat gggcccacct ccgttggggt gtgttcgatg agtataacaa tgacaaacct 480
   ttctacataa atgggcaaaa tcaaattaaa gtgacaaggt gttcatctga catcacaggc 540
   atttttgtgt gtgaaaaagg tccttgcccc caagaaaact gtattattag taagcttttt 600
   aaagaaggat gcacctttat ctacaatagc acccaaaatg caactgcatc aataatgttc 660
   atgcaaagtt tatcttctgt ggttgaattt tgtaatgcaa gtacccacaa ccaagaagca 720
   ccaaacctac agaaccagat gtgcagcctc agaagtgcat gggatgtaat cacagactct 780
   gctgactttc accacagctt tcccatgaac gggactgagc ttccacctcc tcccacattc 840
   tcgcttgtag aggctggtga caaagtggtc tgtttagtgc tggatgtgtc cagcaagatg 900
   gcagaggctg acagactcct tcaactacaa caagccgcag aattttattt gatgcagatt 960
   gttgaaattc ataccttcgt gggcattgcc agtttcgaca gcaaaggaga gatcagagcc 1020
   cagctacacc aaattaacag caatgatgat cgaaagttgc tggtttcata tctgcccacc 1080
   actgtatcag ctaaaacaga catcagcatt tgttcagggc ttaagaaagg atttgaggtg 1140
   gttgaaaaac tgaatggaaa agcttatggc tctgtgatga tattagtgac cagcggagat 1200
   gataagcttc ttggcaattg cttacccact gtgctcagca gtggttcaac aattcactcc 1260
   attgccctgg gttcatctgc agccccaaat ctggaggaat tatcacgtct tacaggaggt 1320
   ttaaagttct ttgttccaga tatatcaaac tccaatagca tgattgatgc tttcagtaga 1380
   atttcctctg gaactggaga cattttccag caacatattc agcttgaaag tacaggtgaa 1440
   aatgtcaaac ctcaccatca attgaaaaac acagtgactg tggataatac tgtgggcaac 1500
   gacactatgt ttctagttac gtggcaggcc agtggtcctc ctgagattat attatttgat 1560
   cctgatggac gaaaatacta cacaaataat tttatcacca atctaacttt tcggacagct 1620
   agtctttgga ttccaggaac agctaagcct gggcactgga cttacaccct gaacaatacc 1680
   catcattctc tgcaagccct gaaagtgaca gtgacctctc gcgcctccaa ctcagctgtg 1740
   cccccagcca ctgtggaagc ctttgtggaa agagacagcc tccattttcc tcatcctgtg 1800
   atgatttatg ccaatgtgaa acagggattt tatcccattc ttaatgccac tgtcactgcc 1860
   acagttgagc cagagactgg agatcctgtt acgctgagac tccttgatga tggagcaggt 1920
   gctgatgtta taaaaaatga tggaatttac tcgaggtatt ttttctcctt tgctgcaaat 1980
   ggtagatata gcttgaaagt gcatgtcaat cactctccca gcataagcac cccagcccac 2040
   tctattccag ggagtcatgc tatgtatgta ccaggttaca cagcaaacgg taatattcag 2100
   atgaatgctc caaggaaatc agtaggcaga aatgaggagg agcgaaagtg gggctttagc 2160
   cgagtcagct caggaggctc cttttcagtg ctgggagttc cagctggccc ccaccctgat 2220
   gtgtttccac catgcaaaat tattgacctg gaagctgtaa aagtagaaga ggaattgacc 2280
   ctatcttgga cagcacctgg agaagacttt gatcagggcc aggctacaag ctatgaaata 2340
   agaatgagta aaagtctaca gaatatccaa gatgacttta acaatgctat tttagtaaat 2400
   acatcaaagc gaaatcctca gcaagctggc atcagggaga tatttacgtt ctcaccccaa 2460
   atttccacga atggacctga acatcagcca aatggagaaa cacatgaaag ccacagaatt 2520
   tatgttgcaa tacgagcaat ggataggaac tccttacagt ctgctgtatc taacattgcc 2580
   caggcgcctc tgtttattcc ccccaattct gatcctgtac ctgccagaga ttatcttata 2640
   ttgaaaggag ttttaacagc aatgggtttg ataggaatca tttgccttat tatagttgtg 2700
   acacatcata ctttaagcag gaaaaagaga gcagacaaga aagagaatgg aacaaaatta 2760
   ttataatgaa ttc                                                    2773
<210> 359
   <211> 25
   <212> DNA
   <213> Artificial Sequence
<220>
   <223> PCR primer
<400> 359
   tggcagcccc tcttcttcaa gtggc 25
<210> 360
   <211> 33
   <212> DNA
   <213> Artificial Sequence
<220>
   <223> PCR primer
<400> 360
   cgccagaatt catcaaacaa atctgttagc acc 33
<210> 361
   <211> 77
   <212> PRT
   <213> Homo sapiens
<400> 361
   Met Gln His His His His His His Trp Gln Pro Leu Phe Phe Lys Trp
   1               5                   10                  15
   Leu Leu Ser Cys Cys Pro Gly Ser Ser Gln Ile Ala Ala Ala Ala Ser
               20                  25                  30
   Thr Gln Pro Glu Asp Asp Ile Asn Thr Gln Arg Lys Lys Ser Gln Glu
           35                  40                  45
   Lys Met Arg Glu Val Thr Asp Ser Pro Gly Arg Pro Arg Glu Leu Thr
       50                  55                  60
   Ile Pro Gln Thr Ser Ser His Gly Ala Asn Arg Phe Val
   65                  70                  75
<210> 362
   <211> 244
   <212> DNA
   <213> Homo sapiens
<400> 362
   catatgcagc atcaccacca tcaccactgg cagcccctct tcttcaagtg gctcttgtcc 60
   tgttgccctg ggagttctca aattgctgca gcagcctcca cccagcctga ggatgacatc 120
   aatacacaga ggaagaagag tcaggaaaag atgagagaag ttacagactc tcctgggcga 180
   ccccgagagc ttaccattcc tcagacttct tcacatggtg ctaacagatt tgtttgatga 240
   attc                                                              244
<210> 363
   <211> 20
   <212> PRT
   <213> Homo sapiens
<400> 363
   Met Trp Gln Pro Leu Phe Phe Lys Trp Leu Leu Ser Cys Cys Pro Gly
   1 5 10 15
   Ser Ser Gln Ile
   20
<210> 364
   <211> 60
   <212> DNA
   <213> Homo sapiens
<400> 364
   atgtggcagc ccctcttctt caagtggctc ttgtcctgtt gccctgggag ttctcaaatt 60
<210> 365
   <211> 20
   <212> PRT
   <213> Homo sapiens
<400> 365
   Gly Ser Ser Gln Ile Ala Ala Ala Ala Ser Thr Gln Pro Glu Asp Asp
   1 5 10 15
   Ile Asn Thr Gln
   20
<210> 366
   <211> 60
   <212> DNA
   <213> Homo sapiens
<400> 366
   gggagttctc aaattgctgc agcagcctcc acccagcctg aggatgacat caatacacag 60
<210> 367
   <211> 20
   <212> PRT
   <213> Homo sapiens
<400> 367
   Lys Pro Gly His Trp Thr Tyr Thr Leu Asn Asn Thr His His Ser Leu
   1 5 10 15
   Gln Ala Leu Lys
   20
<210> 368
   <211> 2343
   <212> DNA
   <213> Homo sapiens
<400> 368
   attccggagc gtttgcggct tcgcttcatg gccgctctcc cgcccctcct gggatctgtg 60
   gggagctggg gagcccgcag cggcccggag ccggagctgg cgagccgagc ggagacctgt 120
   gcgccgcgcc tctgaggcgc agcatgtgaa gcggagacgg catccagtgg ggggcgagcc 180
   tctcagccgg ccgggatggc taccacggcc gagctcttcg aggagccttt tgtggcagat 240
   gaatatattg aacgtcttgt atggagaacc ccaggaggag gctctagagg tggacctgaa 300
   gcttttgatc ctaaaagatt attagaagaa tttgtaaatc atattcagga actccagata 360
   atggatgaaa ggattcagag gaaagtagag aaactagagc aacaatgtca gaaagaagcc 420
   aaggaatttg ccaagaaggt acaagagctg cagaaaagca atcaggttgc cttccaacat 480
   ttccaagaac tagatgagca cattagctat gtagcaacta aagtctgtca ccttggagac 540
   cagttagagg gggtaaacac acccagacaa cgggcagtgg aggctcagaa attgatgaaa 600
   tactttaatg agtttctaga tggagaattg aaatctgatg tttttacaaa ttctgaaaag 660
   ataaaggaag cagcagacat cattcagaag ttgcacctaa ttgcccaaga gttacctttt 720
   gatagatttt cagaagttaa atccaaaatt gcaagtaaat accatgattt agaatgccag 780
   ctgattcagg agtttaccag tgctcaaaga agaggtgaaa tctccagaat gagagaagta 840
   gcagcagttt tacttcattt taagggttat tcccattgtg ttgatgttta tataaagcag 900
   tgccaggagg gtgcttattt gagaaatgat atatttgaag acgctggaat actctgtcaa 960
   agagtgaaca aacaagttgg agatatcttc agtaatccag aaacagtcct ggctaaactt 1020
   attcaaaatg tatttgaaat caaactacag agttttgtga aagagcagtt agaagaatgt 1080
   aggaagtccg atgcagagca atatctcaaa aatctctatg atctgtatac aagaaccacc 1140
   aatctttcca gcaagctgat ggagtttaat ttaggtactg ataaacagac tttcttgtct 1200
   aagcttatca aatccatttt catttcctat ttggagaact atattgaggt ggagactgga 1260
   tatttgaaaa gcagaagtgc tatgatccta cagcgctatt atgattcgaa aaaccatcaa 1320
   aagagatcca ttggcacagg aggtattcaa gatttgaagg aaagaattag acagcgtacc 1380
   aacttaccac ttgggccaag tatcgatact catggggaga cttttctatc ccaagaagtg 1440
   gtggttaatc ttttacaaga aaccaaacaa gcctttgaaa gatgtcatag gctctctgat 1500
   ccttctgact taccaaggaa tgccttcaga atttttacca ttcttgtgga atttttatgt 1560
   attgagcata ttgattatgc tttggaaaca ggacttgctg gaattccctc ttcagattct 1620
   aggaatgcaa atctttattt tttggacgtt gtgcaacagg ccaatactat ttttcatctt 1680
   tttgacaaac agtttaatga tcaccttatg ccactaataa gctcttctcc taagttatct 1740
   gaatgccttc agaagaaaaa agaaataatt gaacaaatgg agatgaaatt ggatactggc 1800
   attgatagga cattaaattg tatgattgga cagatgaagc atattttggc tgcagaacag 1860
   aagaaaacag attttaagcc agaagatgaa aacaatgttt tgattcaata tactaatgcc 1920
   tgtgtaaaag tctgtgctta cgtaagaaaa caagtggaga agattaaaaa ttccatggat 1980
   gggaagaatg tggatacagt tttgatggaa cttggagtac gttttcatcg acttatctat 2040
   gagcatcttc aacaatattc ctacagttgt atgggtggca tgttggccat ttgtgatgta 2100
   gccgaatata ggaagtgtgc caaagacttc aagattccaa tggtattaca tctttttgat 2160
   actctgcatg ctctttgcaa tcttctggta gttgccccag ataatttaaa gcaagtctgc 2220
   tcaggagaac aacttgctaa tctggacaag aatatacttc actccttcgt acaacttcgt 2280
   gctgattata gatctgcccg ccttgctcga cacttcagct gagattgaat ttacaaagga 2340
   att 2343
<210> 369
   <211> 708
   <212> PRT
   <213> Homo sapiens
<400> 369
   Met Ala Thr Thr Ala Glu Leu Phe Glu Glu Pro Phe Val Ala Asp Glu
   1               5                   10                  15
   Tyr Ile Glu Arg Leu Val Trp Arg Thr Pro Gly Gly Gly Ser Arg Gly
               20                  25                  30
   Gly Pro Glu Ala Phe Asp Pro Lys Arg Leu Leu Glu Glu Phe Val Asn
           35                  40                  45
   His Ile Gln Glu Leu Gln Ile Met Asp Glu Arg Ile Gln Arg Lys Val
       50                  55                  60
   Glu Lys Leu Glu Gln Gln Cys Gln Lys Glu Ala Lys Glu Phe Ala Lys
   65                  70                  75                  80
   Lys Val Gln Glu Leu Gln Lys Ser Asn Gln Val Ala Phe Gln His Phe
                   85                  90                  95
   Gln Glu Leu Asp Glu His Ile Ser Tyr Val Ala Thr Lys Val Cys His
               100                 105                 110
   Leu Gly Asp Gln Leu Glu Gly Val Asn Thr Pro Arg Gln Arg Ala Val
           115                 120                 125
   Glu Ala Gln Lys Leu Met Lys Tyr Phe Asn Glu Phe Leu Asp Gly Glu
       130                 135                 140
   Leu Lys Ser Asp Val Phe Thr Asn Ser Glu Lys Ile Lys Glu Ala Ala
   145                 150                 155                 160
   Asp Ile Ile Gln Lys Leu His Leu Ile Ala Gln Glu Leu Pro Phe Asp
                   165                 170                 175
   Arg Phe Ser Glu Val Lys Ser Lys Ile Ala Ser Lys Tyr His Asp Leu
               180                 185                 190
   Glu Cys Gln Leu Ile Gln Glu Phe Thr Ser Ala Gln Arg Arg Gly Glu
           195                 200                 205
   Ile Ser Arg Met Arg Glu Val Ala Ala Val Leu Leu His Phe Lys Gly
       210                 215                 220
   Tyr Ser His Cys Val Asp Val Tyr Ile Lys Gln Cys Gln Glu Gly Ala
   225                 230                 235                 240
   Tyr Leu Arg Asn Asp Ile Phe Glu Asp Ala Gly Ile Leu Cys Gln Arg
                   245                 250                 255
   Val Asn Lys Gln Val Gly Asp Ile Phe Ser Asn Pro Glu Thr Val Leu
               260                 265                 270
   Ala Lys Leu Ile Gln Asn Val Phe Glu Ile Lys Leu Gln Ser Phe Val
           275                 280                 285
   Lys Glu Gln Leu Glu Glu Cys Arg Lys Ser Asp Ala Glu Gln Tyr Leu
       290                 295                 300
   Lys Asn Leu Tyr Asp Leu Tyr Thr Arg Thr Thr Asn Leu Ser Ser Lys
   305                 310                 315                 320
   Leu Met Glu Phe Asn Leu Gly Thr Asp Lys Gln Thr Phe Leu Ser Lys
                   325                 330                 335
   Leu Ile Lys Ser Ile Phe Ile Ser Tyr Leu Glu Asn Tyr Ile Glu Val
               340                 345                 350
   Glu Thr Gly Tyr Leu Lys Ser Arg Ser Ala Met Ile Leu Gln Arg Tyr
           355                 360                 365
   Tyr Asp Ser Lys Asn His Gln Lys Arg Ser Ile Gly Thr Gly Gly Ile
       370                 375                 380
   Gln Asp Leu Lys Glu Arg Ile Arg Gln Arg Thr Asn Leu Pro Leu Gly
   385                 390                 395                 400
   Pro Ser Ile Asp Thr His Gly Glu Thr Phe Leu Ser Gln Glu Val Val
                   405                 410                 415
   Val Asn Leu Leu Gln Glu Thr Lys Gln Ala Phe Glu Arg Cys His Arg
               420                 425                 430
   Leu Ser Asp Pro Ser Asp Leu Pro Arg Asn Ala Phe Arg Ile Phe Thr
           435                 440                 445
   Ile Leu Val Glu Phe Leu Cys Ile Glu His Ile Asp Tyr Ala Leu Glu
       450                 455                 460
   Thr Gly Leu Ala Gly Ile Pro Ser Ser Asp Ser Arg Asn Ala Asn Leu
   465                 470                 475                 480
   Tyr Phe Leu Asp Val Val Gln Gln Ala Asn Thr Ile Phe His Leu Phe
                   485                 490                 495
   Asp Lys Gln Phe Asn Asp His Leu Met Pro Leu Ile Ser Ser Ser Pro
               500                 505                 510
   Lys Leu Ser Glu Cys Leu Gln Lys Lys Lys Glu Ile Ile Glu Gln Met
           515                 520                 525
   Glu Met Lys Leu Asp Thr Gly Ile Asp Arg Thr Leu Asn Cys Met Ile
       530                 535                 540
   Gly Gln Met Lys His Ile Leu Ala Ala Glu Gln Lys Lys Thr Asp Phe
   545                 550                 555                 560
   Lys Pro Glu Asp Glu Asn Asn Val Leu Ile Gln Tyr Thr Asn Ala Cys
                   565                 570                 575
   Val Lys Val Cys Ala Tyr Val Arg Lys Gln Val Glu Lys Ile Lys Asn
               580                 585                 590
   Ser Met Asp Gly Lys Asn Val Asp Thr Val Leu Met Glu Leu Gly Val
           595                 600                 605
   Arg Phe His Arg Leu Ile Tyr Glu His Leu Gln Gln Tyr Ser Tyr Ser
       610                 615                 620
   Cys Met Gly Gly Met Leu Ala Ile Cys Asp Val Ala Glu Tyr Arg Lys
   625                 630                 635                 640
   Cys Ala Lys Asp Phe Lys Ile Pro Met Val Leu His Leu Phe Asp Thr
                   645                 650                 655
   Leu His Ala Leu Cys Asn Leu Leu Val Val Ala Pro Asp Asn Leu Lys
               660                 665                 670
   Gln Val Cys Ser Gly Glu Gln Leu Ala Asn Leu Asp Lys Asn Ile Leu
           675                 680                 685
   His Ser Phe Val Gln Leu Arg Ala Asp Tyr Arg Ser Ala Arg Leu Ala
       690                 695                 700
   Arg His Phe Ser
   705
<210> 370
   <211> 60
   <212> DNA
   <213> Homo sapiens
<400> 370
   gtcaatcact ctcccagcat aagcacccca gcccactcta ttccagggag tcatgctatg 60
<210> 371
   <211> 60
   <212> DNA
   <213> Homo sapiens
<400> 371
   agtagaattt cctctggaac tggagacatt ttccagcaac atattcagct tgaaagtaca 60
<210> 372
   <211> 60
   <212> DNA
   <213> Homo sapiens
<400> 372
   ccagagactg gagatcctgt tacgctgaga ctccttgatg atggagcagg tgctgatgtt 60
<210> 373
   <211> 60
   <212> DNA
   <213> Homo sapiens
<400> 373
   ttacagtctg ctgtatctaa cattgcccag gcgcctctgt ttattccccc caattctgat 60
<210> 374
   <211> 60
   <212> DNA
   <213> Homo sapiens
<400> 374
   gctgtgcccc cagccactgt ggaagccttt gtggaaagag acagcctcca ttttcctcat 60
<210> 375
   <211> 60
   <212> DNA
   <213> Homo sapiens
<400> 375
   aaaaacacag tgactgtgga taatactgtg ggcaacgaca ctatgtttct agttacgtgg 60
<210> 376
   <211> 20
   <212> PRT
   <213> Homo sapiens
<400> 376
   Leu Gln Ser Ala Val Ser Asn Ile Ala Gln Ala Pro Leu Phe Ile Pro
   1               5                   10                  15
   Pro Asn Ser Asp
               20
<210> 377
   <211> 20
   <212> PRT
   <213> Homo sapiens
<400> 377
   Val Asn His Ser Pro Ser Ile Ser Thr Pro Ala His Ser Ile Pro Gly
   1               5                   10                  15
   Ser His Ala Met
               20
<210> 378
   <211> 20
   <212> PRT
   <213> Homo sapiens
<400> 378
   Pro Glu Thr Gly Asp Pro Val Thr Leu Arg Leu Leu Asp Asp Gly Ala
    1               5                  10                  15
   Gly Ala Asp Val
               20
<210> 379
   <211> 20
   <212> PRT
   <213> Homo sapiens
<400> 379
   Ala Val Pro Pro Ala Thr Val Glu Ala Phe Val Glu Arg Asp Ser Leu
    1               5                  10                  15
   His Phe Pro His
               20
<210> 380
   <211> 20
   <212> PRT
   <213> Homo sapiens
<400> 380
   Ser Arg Ile Ser Ser Gly Thr Gly Asp Ile Phe Gln Gln His Ile Gln
    1               5                  10                  15
   Leu Glu Ser Thr
               20
<210> 381
   <211> 20
   <212> PRT
   <213> Homo sapiens
<400> 381
   Lys Asn Thr Val Thr Val Asp Asn Thr Val Gly Asn Asp Thr Met Phe
    1               5                  10                  15
   Leu Val Thr Trp
               20
<210> 382
   <211> 20
   <212> PRT
   <213> Homo sapiens
<400> 382
   Lys Pro Gly His Trp Thr Tyr Thr Leu Asn Asn Thr His His Ser Leu
   1               5                   10                  15
   Gln Ala Leu Lys
               20
<210> 383
   <211> 29
   <212> DNA
   <213> Artificial Sequence
<220>
   <223> PCR primer
<400> 383
   cggcgaattc atggattggg ggacgctgc 29
<210> 384
   <211> 35
   <212> DNA
   <213> Artificial Sequence
<220>
   <223> PCR primer
<400> 384
   cggcctcgag tcacccctct atccgaacct tctgc 35
<210> 385
   <211> 32
   <212> DNA
   <213> Artificial Sequence
<220>
   <223> PCR primer
<400> 385
   cggcgaattc cacgaaccac tcgcaagttc ag 32
<210> 386
   <211> 30
   <212> DNA
   <213> Artificial Sequence
<220>
   <223> PCR primer
<400> 386
   cggctcgagt tagcttgggc ctgtgattgc 30
<210> 387
   <211> 20
   <212> PRT
   <213> Homo sapiens
<400> 387
   Phe Phe Lys Trp Leu Leu Ser Cys Cys Pro Gly Ser Ser Gln Ile Ala
   1 5 10 15
   Ala Ala Ala Ser
   20
<210> 388
   <211> 19
   <212> PRT
   <213> Homo sapiens
<400> 388
   Leu Ser Cys Cys Pro Gly Ser Ser Gln Ile Ala Ala Ala Ser Thr Gln
   1 5 10 15
   Pro Glu Asp
<210> 389
   <211> 20
   <212> PRT
   <213> Homo sapiens
<400> 389
   Ala Ala Ala Ala Ser Thr Gln Pro Glu Asp Asp Ile Asn Thr Gln Arg
   1 5 10 15
   Lys Lys Ser Gln
   20
<210> 390
   <211> 20
   <212> PRT
   <213> Homo sapiens
<400> 390
   Thr Gln Pro Glu Asp Asp Ile Asn Thr Gln Arg Lys Lys Ser Gln Glu
   1 5 10 15
   Lys Met Arg Glu
   20
<210> 391
   <211> 20
   <212> PRT
   <213> Homo sapiens
<400> 391
   Asp Ile Asn Thr Gln Arg Lys Lys Ser Gln Glu Lys Met Arg Glu Val
   1 5 10 15
   Thr Asp Ser Pro
   20
<210> 392
   <211> 20
   <212> PRT
   <213> Homo sapiens
<400> 392
   Arg Lys Lys Ser Gln Glu Lys Met Arg Glu Val Thr Asp Ser Pro Gly
   1 5 10 15
   Arg Pro Arg Glu
   20
<210> 393
   <211> 20
   <212> PRT
   <213> Homo sapiens
<400> 393
   Glu Lys Met Arg Glu Val Thr Asp Ser Pro Gly Arg Pro Arg Glu Leu
   1 5 10 15
   Thr Ile Pro Gln
   20
<210> 394
   <211> 20
   <212> PRT
   <213> Homo sapiens
<400> 394
   Val Thr Asp Ser Pro Gly Arg Pro Arg Glu Leu Thr Ile Pro Gln Thr
   1 5 10 15
   Ser Ser His Gly
   20
<210> 395
   <211> 19
   <212> PRT
   <213> Homo sapiens
<400> 395
   Gly Arg Pro Arg Glu Leu Thr Ile Pro Gln Thr Ser Ser His Gly Ala
   1 5 10 15
   Asn Arg Phe
<210> 396
   <211> 19
   <212> PRT
   <213> Homo sapiens
<400> 396
   Met Asn Lys Leu Tyr Ile Gly Asn Leu Ser Glu Asn Ala Ala Pro Ser
   1 5 10 15
   Asp Leu Glu
<210> 397
   <211> 20
   <212> PRT
   <213> Homo sapiens
<400> 397
   Ser Glu Asn Ala Ala Pro Ser Asp Leu Glu Ser Ile Phe Lys Asp Ala
   1 5 10 15
   Lys Ile Pro Val
   20
<210> 398
   <211> 20
   <212> PRT
   <213> Homo sapiens
<400> 398
   Ser Ile Phe Lys Asp Ala Lys Ile Pro Val Ser Gly Pro Phe Leu Val
   1 5 10 15
   Lys Thr Gly Tyr
   20
<210> 399
   <211> 20
   <212> PRT
   <213> Homo sapiens
<400> 399
   Ser Gly Pro Phe Leu Val Lys Thr Gly Tyr Ala Phe Val Asp Cys Pro
   1 5 10 15
   Asp Glu Ser Trp
   20
<210> 400
   <211> 20
   <212> PRT
   <213> Homo sapiens
<400> 400
   Ala Phe Val Asp Cys Pro Asp Glu Ser Trp Ala Leu Lys Ala Ile Glu
    1               5                  10                  15
   Ala Leu Ser Gly
               20
<210> 401
   <211> 20
   <212> PRT
   <213> Homo sapiens
<400> 401
   Ala Leu Lys Ala Ile Glu Ala Leu Ser Gly Lys Ile Glu Leu His Gly
    1               5                  10                  15
   Lys Pro Ile Glu
               20
<210> 402
   <211> 20
   <212> PRT
   <213> Homo sapiens
<400> 402
   Lys Ile Glu Leu His Gly Lys Pro Ile Glu Val Glu His Ser Val Pro
    1               5                  10                  15
   Lys Arg Gln Arg
               20
<210> 403
   <211> 20
   <212> PRT
   <213> Homo sapiens
<400> 403
   Val Glu His Ser Val Pro Lys Arg Gln Arg Ile Arg Lys Leu Gln Ile
    1               5                  10                  15
   Arg Asn Ile Pro
               20
<210> 404
   <211> 20
   <212> PRT
   <213> Homo sapiens
<400> 404
   Ile Arg Lys Leu Gln Ile Arg Asn Ile Pro Pro His Leu Gln Trp Glu
   1 5 10 15
   Val Leu Asp Ser
   20
<210> 405
   <211> 20
   <212> PRT
   <213> Homo sapiens
<400> 405
   Ala Val Val Asn Val Thr Tyr Ser Ser Lys Asp Gln Ala Arg Gln Ala
   1 5 10 15
   Leu Asp Lys Leu
   20
<210> 406
   <211> 20
   <212> PRT
   <213> Homo sapiens
<400> 406
   Asp Gln Ala Arg Gln Ala Leu Asp Lys Leu Asn Gly Phe Gln Leu Glu
   1 5 10 15
   Asn Phe Thr Leu
   20
<210> 407
   <211> 20
   <212> PRT
   <213> Homo sapiens
<400> 407
   Asn Gly Phe Gln Leu Glu Asn Phe Thr Leu Lys Val Ala Tyr Ile Pro
   1 5 10 15
   Asp Glu Thr Ala
   20
<210> 408
   <211> 20
   <212> PRT
   <213> Homo sapiens
<400> 408
   Lys Val Ala Tyr Ile Pro Asp Glu Thr Ala Ala Gln Gln Asn Pro Leu
   1 5 10 15
   Gln Gln Pro Arg
   20
<210> 409
   <211> 20
   <212> PRT
   <213> Homo sapiens
<400> 409
   Ala Gln Gln Asn Pro Leu Gln Gln Pro Arg Gly Arg Arg Gly Leu Gly
    1               5                  10                  15
   Gln Arg Gly Ser
               20
<210> 410
   <211> 20
   <212> PRT
   <213> Homo sapiens
<400> 410
   Gly Arg Arg Gly Leu Gly Gln Arg Gly Ser Ser Arg Gln Gly Ser Pro
    1               5                  10                  15
   Gly Ser Val Ser
               20
<210> 411
   <211> 20
   <212> PRT
   <213> Homo sapiens
<400> 411
   Ser Arg Gln Gly Ser Pro Gly Ser Val Ser Lys Gln Lys Pro Cys Asp
    1               5                  10                  15
   Leu Pro Leu Arg
               20
<210> 412
   <211> 20
   <212> PRT
   <213> Homo sapiens
<400> 412
   Lys Gln Lys Pro Cys Asp Leu Pro Leu Arg Leu Leu Val Pro Thr Gln
    1               5                  10                  15
   Phe Val Gly Ala
               20
<210> 413
   <211> 20
   <212> PRT
   <213> Homo sapiens
<400> 413
   Leu Leu Val Pro Thr Gln Phe Val Gly Ala Ile Ile Gly Lys Glu Gly
   1 5 10 15
   Ala Thr Ile Arg
   20
<210> 414
   <211> 20
   <212> PRT
   <213> Homo sapiens
<400> 414
   Ile Ile Gly Lys Glu Gly Ala Thr Ile Arg Asn Ile Thr Lys Gln Thr
   1 5 10 15
   Gln Ser Lys Ile
   20
<210> 415
   <211> 20
   <212> PRT
   <213> Homo sapiens
<400> 415
   Asn Ile Thr Lys Gln Thr Gln Ser Lys Ile Asp Val His Arg Lys Glu
   1 5 10 15
   Asn Ala Gly Ala
   20
<210> 416
   <211> 20
   <212> PRT
   <213> Homo sapiens
<400> 416
   Asp Val His Arg Lys Glu Asn Ala Gly Ala Ala Glu Lys Ser Ile Thr
   1 5 10 15
   Ile Leu Ser Thr
   20
<210> 417
   <211> 20
   <212> PRT
   <213> Homo sapiens
<400> 417
   Ala Glu Lys Ser Ile Thr Ile Leu Ser Thr Pro Glu Gly Thr Ser Ala
   1 5 10 15
   Ala Cys Lys Ser
   20
<210> 418
   <211> 20
   <212> PRT
   <213> Homo sapiens
<400> 418
   Pro Glu Gly Thr Ser Ala Ala Cys Lys Ser Ile Leu Glu Ile Met His
    1               5                   10                  15
   Lys Glu Ala Gln
               20
<210> 419
   <211> 20
   <212> PRT
   <213> Homo sapiens
<400> 419
   Ile Leu Glu Ile Met His Lys Glu Ala Gln Asp Ile Lys Phe Thr Glu
    1               5                   10                  15
   Glu Ile Pro Leu
               20
<210> 420
   <211> 455
   <212> DNA
   <213> Homo sapiens
<400> 420
   gaagacatgc ttacttcccc ttcaccttcc ttcatgatgt gggaagagtg ctgcaaccca 60
   gccctagcca acgccgcatg agagggagtg tgccgagggc ttctgagaag gtttctctca 120
   catctagaaa gaagcgctta agatgtggca gcccctcttc ttcaagtggc tcttgtcctg 180
   ttgccctggg agttctcaaa ttgctgcagc agcctccacc cagcctgagg atgacatcaa 240
   tacacagagg aagaagagtc aggaaaagat gagagaagtt acagactctc ctgggcgacc 300
   ccgagagctt accattcctc agacttcttc acatggtgct aacagatttg ttcctaaaag 360
   taaagctcta gaggccgtca aattggcaat agaagccggg ttccaccata ttgattctgc 420
   acatgtttac aataatgagg agcaggttgg actgg                            455
<210> 421
   <211> 24
   <212> DNA
   <213> Artificial Sequence
<220>
   <223> PCR primer
<400> 421
   actagtgtcc gcgtggcggc ctac 24
<210> 422
   <211> 34
   <212> DNA
   <213> Artificial Sequence
<220>
   <223> PCR primer
<400> 422
   catgagaatt catcacatgc ccttgaaggc tccc 34
<210> 423
   <211> 161
   <212> PRT
   <213> Homo sapiens
<400> 423
   Met Gln His His His His His His His Thr Ser Val Arg Val Ala Ala
    1               5                  10                  15
   Tyr Phe Glu Asn Phe Leu Ala Ala Trp Arg Pro Val Lys Ala Ser Asp
               20                  25                  30
   Gly Asp Tyr Tyr Thr Leu Ala Val Pro Met Gly Asp Val Pro Met Asp
           35                  40                  45
   Gly Ile Ser Val Ala Asp Ile Gly Ala Ala Val Ser Ser Ile Phe Asn
       50                  55                  60
   Ser Pro Glu Glu Phe Leu Gly Lys Ala Val Gly Leu Ser Ala Glu Ala
   65                  70                  75                  80
   Leu Thr Ile Gln Gln Tyr Ala Asp Val Leu Ser Lys Ala Leu Gly Lys
                   85                  90                  95
   Glu Val Arg Asp Ala Lys Ile Thr Pro Glu Ala Phe Glu Lys Leu Gly
               100                 105                 110
   Phe Pro Ala Ala Lys Glu Ile Ala Asn Met Cys Arg Phe Tyr Glu Met
           115                 120                 125
   Lys Pro Asp Arg Asp Val Asn Leu Thr His Gln Leu Asn Pro Lys Val
       130                 135                 140
   Lys Ser Phe Ser Gln Phe Ile Ser Glu Asn Gln Gly Ala Phe Lys Gly
   145                 150                 155                 160
   Met
<210> 424
   <211> 489
   <212> DNA
   <213> Homo sapiens
<400> 424
   atgcagcatc accaccatca ccaccacact agtgtccgcg tggcggccta ctttgaaaac 60
   tttctcgcgg cgtggcggcc cgtgaaagcc tctgatggag attactacac cttggctgta 120
   ccgatgggag atgtaccaat ggatggtatc tctgttgctg atattggagc agccgtctct 180
   agcattttta attctccaga ggaattttta ggcaaggccg tggggctcag tgcagaagca 240
   ctaacaatac agcaatatgc tgatgttttg tccaaggctt tggggaaaga agtccgagat 300
   gcaaagatta ccccggaagc tttcgagaag ctgggattcc ctgcagcaaa ggaaatagcc 360
   aatatgtgtc gtttctatga aatgaagcca gaccgagatg tcaatctcac ccaccaacta 420
   aatcccaaag tcaaaagctt cagccagttt atctcagaga accagggagc cttcaagggc 480
   atgtgatga                                                         489
<210> 425
   <211> 32
   <212> DNA
   <213> Artificial Sequence
<220>
   <223> PCR primer
<400> 425
   aacaaactgt atatcggaaa cctcagcgag aa 32
<210> 426
   <211> 33
   <212> DNA
   <213> Artificial Sequence
<220>
   <223> PCR primer
<400> 426
   ccatagaatt cattacttcc gtcttgactg agg 33
<210> 427
   <211> 586
   <212> PRT
   <213> Homo sapiens
<400> 427
   Met Gln His His His His His His Asn Lys Leu Tyr Ile Gly Asn Leu
   1               5                   10                  15
   Ser Glu Asn Ala Ala Pro Ser Asp Leu Glu Ser Ile Phe Lys Asp Ala
               20                  25                  30
   Lys Ile Pro Val Ser Gly Pro Phe Leu Val Lys Thr Gly Tyr Ala Phe
           35                  40                  45
   Val Asp Cys Pro Asp Glu Ser Trp Ala Leu Lys Ala Ile Glu Ala Leu
       50                  55                  60
   Ser Gly Lys Ile Glu Leu His Gly Lys Pro Ile Glu Val Glu His Ser
   65                  70                  75                  80
   Val Pro Lys Arg Gln Arg Ile Arg Lys Leu Gln Ile Arg Asn Ile Pro
                   85                  90                  95
   Pro His Leu Gln Trp Glu Val Leu Asp Ser Leu Leu Val Gln Tyr Gly
               100                 105                 110
   Val Val Glu Ser Cys Glu Gln Val Asn Thr Asp Ser Glu Thr Ala Val
           115                 120                 125
   Val Asn Val Thr Tyr Ser Ser Lys Asp Gln Ala Arg Gln Ala Leu Asp
       130                 135                 140
   Lys Leu Asn Gly Phe Gln Leu Glu Asn Phe Thr Leu Lys Val Ala Tyr
   145                 150                 155                 160
   Ile Pro Asp Glu Thr Ala Ala Gln Gln Asn Pro Leu Gln Gln Pro Arg
                   165                 170                 175
   Gly Arg Arg Gly Leu Gly Gln Arg Gly Ser Ser Arg Gln Gly Ser Pro
               180                 185                 190
   Gly Ser Val Ser Lys Gln Lys Pro Cys Asp Leu Pro Leu Arg Leu Leu
           195                 200                 205
   Val Pro Thr Gln Phe Val Gly Ala Ile Ile Gly Lys Glu Gly Ala Thr
       210                 215                 220
   Ile Arg Asn Ile Thr Lys Gln Thr Gln Ser Lys Ile Asp Val His Arg
   225                 230                 235                 240
   Lys Glu Asn Ala Gly Ala Ala Glu Lys Ser Ile Thr Ile Leu Ser Thr
                   245                 250                 255
   Pro Glu Gly Thr Ser Ala Ala Cys Lys Ser Ile Leu Glu Ile Met His
               260                 265                 270
   Lys Glu Ala Gln Asp Ile Lys Phe Thr Glu Glu Ile Pro Leu Lys Ile
           275                 280                 285
   Leu Ala His Asn Asn Phe Val Gly Arg Leu Ile Gly Lys Glu Gly Arg
       290                 295                 300
   Asn Leu Lys Lys Ile Glu Gln Asp Thr Asp Thr Lys Ile Thr Ile Ser
   305                 310                 315                 320
   Pro Leu Gln Glu Leu Thr Leu Tyr Asn Pro Glu Arg Thr Ile Thr Val
                   325                 330                 335
   Lys Gly Asn Val Glu Thr Cys Ala Lys Ala Glu Glu Glu Ile Met Lys
               340                 345                 350
   Lys Ile Arg Glu Ser Tyr Glu Asn Asp Ile Ala Ser Met Asn Leu Gln
           355                 360                 365
   Ala His Leu Ile Pro Gly Leu Asn Leu Asn Ala Leu Gly Leu Phe Pro
       370                 375                 380
   Pro Thr Ser Gly Met Pro Pro Pro Thr Ser Gly Pro Pro Ser Ala Met
   385                 390                 395                 400
   Thr Pro Pro Tyr Pro Gln Phe Glu Gln Ser Glu Thr Glu Thr Val His
                   405                 410                 415
   Leu Phe Ile Pro Ala Leu Ser Val Gly Ala Ile Ile Gly Lys Gln Gly
               420                 425                 430
   Gln His Ile Lys Gln Leu Ser Arg Phe Ala Gly Ala Ser Ile Lys Ile
           435                 440                 445
   Ala Pro Ala Glu Ala Pro Asp Ala Lys Val Arg Met Val Ile Ile Thr
       450                 455                 460
   Gly Pro Pro Glu Ala Gln Phe Lys Ala Gln Gly Arg Ile Tyr Gly Lys
   465                 470                 475                 480
   Ile Lys Glu Glu Asn Phe Val Ser Pro Lys Glu Glu Val Lys Leu Glu
                   485                 490                 495
   Ala His Ile Arg Val Pro Ser Phe Ala Ala Gly Arg Val Ile Gly Lys
               500                 505                 510
   Gly Gly Lys Thr Val Asn Glu Leu Gln Asn Leu Ser Ser Ala Glu Val
           515                 520                 525
   Val Val Pro Arg Asp Gln Thr Pro Asp Glu Asn Asp Gln Val Val Val
       530                 535                 540
   Lys Ile Thr Gly His Phe Tyr Ala Cys Gln Val Ala Gln Arg Lys Ile
   545                 550                 555                 560
   Gln Glu Ile Leu Thr Gln Val Lys Gln His Gln Gln Gln Lys Ala Leu
                   565                 570                 575
   Gln Ser Gly Pro Pro Gln Ser Arg Arg Lys
               580                 585
<210> 428
   <211> 1764
   <212> DNA
   <213> Homo sapiens
<400> 428
   atgcagcatc accaccatca ccacaacaaa ctgtatatcg gaaacctcag cgagaacgcc 60
   gccccctcgg acctagaaag tatcttcaag gacgccaaga tcccggtgtc gggacccttc 120
   ctggtgaaga ctggctacgc gttcgtggac tgcccggacg agagctgggc cctcaaggcc 180
   atcgaggcgc tttcaggtaa aatagaactg cacgggaaac ccatagaagt tgagcactcg 240
   gtcccaaaaa ggcaaaggat tcggaaactt cagatacgaa atatcccgcc tcatttacag 300
   tgggaggtgc tggatagttt actagtccag tatggagtgg tggagagctg tgagcaagtg 360
   aacactgact cggaaactgc agttgtaaat gtaacctatt ccagtaagga ccaagctaga 420
   caagcactag acaaactgaa tggatttcag ttagagaatt tcaccttgaa agtagcctat 480
   atccctgatg aaacggccgc ccagcaaaac cccttgcagc agccccgagg tcgccggggg 540
   cttgggcaga ggggctcctc aaggcagggg tctccaggat ccgtatccaa gcagaaacca 600
   tgtgatttgc ctctgcgcct gctggttccc acccaatttg ttggagccat cataggaaaa 660
   gaaggtgcca ccattcggaa catcaccaaa cagacccagt ctaaaatcga tgtccaccgt 720
   aaagaaaatg cgggggctgc tgagaagtcg attactatcc tctctactcc tgaaggcacc 780
   tctgcggctt gtaagtctat tctggagatt atgcataagg aagctcaaga tataaaattc 840
   acagaagaga tccccttgaa gattttagct cataataact ttgttggacg tcttattggt 900
   aaagaaggaa gaaatcttaa aaaaattgag caagacacag acactaaaat cacgatatct 960
   ccattgcagg aattgacgct gtataatcca gaacgcacta ttacagttaa aggcaatgtt 1020
   gagacatgtg ccaaagctga ggaggagatc atgaagaaaa tcagggagtc ttatgaaaat 1080
   gatattgctt ctatgaatct tcaagcacat ttaattcctg gattaaatct gaacgccttg 1140
   ggtctgttcc cacccacttc agggatgcca cctcccacct cagggccccc ttcagccatg 1200
   actcctccct acccgcagtt tgagcaatca gaaacggaga ctgttcatct gtttatccca 1260
   gctctatcag tcggtgccat catcggcaag cagggccagc acatcaagca gctttctcgc 1320
   tttgctggag cttcaattaa gattgctcca gcggaagcac cagatgctaa agtgaggatg 1380
   gtgattatca ctggaccacc agaggctcag ttcaaggctc agggaagaat ttatggaaaa 1440
   attaaagaag aaaactttgt tagtcctaaa gaagaggtga aacttgaagc tcatatcaga 1500
   gtgccatcct ttgctgctgg cagagttatt ggaaaaggag gcaaaacggt gaatgaactt 1560
   cagaatttgt caagtgcaga agttgttgtc cctcgtgacc agacacctga tgagaatgac 1620
   caagtggttg tcaaaataac tggtcacttc tatgcttgcc aggttgccca gagaaaaatt 1680
   caggaaattc tgactcaggt aaagcagcac caacaacaga aggctctgca aagtggacca 1740
   cctcagtcaa gacggaagta atga 1764
<210> 429
   <211> 35
   <212> DNA
   <213> Artificial Sequence
<220>
   <223> PCR primer
<400> 429.
   ccatggaatt cattatttca atataagata atctc 35
<210> 430
   <211> 881
   <212> PRT
   <213> Homo sapiens
<400> 430
   Met Gln His His His His His His Gly Val Gln Leu Gln Asp Asn Gly
    1               5                  10                  15
   Tyr Asn Gly Leu Leu Ile Ala Ile Asn Pro Gln Val Pro Glu Asn Gln
               20                  25                  30
   Asn Leu Ile Ser Asn Ile Lys Glu Met Ile Thr Glu Ala Ser Phe Tyr
           35                  40                  45
   Leu Phe Asn Ala Thr Lys Arg Arg Val Phe Phe Arg Asn Ile Lys Ile
       50                  55                  60
   Leu Ile Pro Ala Thr Trp Lys Ala Asn Asn Asn Ser Lys Ile Lys Gln
   65                  70                  75                  80
   Glu Ser Tyr Glu Lys Ala Asn Val Ile Val Thr Asp Trp Tyr Gly Ala
                   85                  90                  95
   His Gly Asp Asp Pro Tyr Thr Leu Gln Tyr Arg Gly Cys Gly Lys Glu
               100                 105                 110
   Gly Lys Tyr Ile His Phe Thr Pro Asn Phe Leu Leu Asn Asp Asn Leu
           115                 120                 125
   Thr Ala Gly Tyr Gly Ser Arg Gly Arg Val Phe Val His Glu Trp Ala
       130                 135                 140
   His Leu Arg Trp Gly Val Phe Asp Glu Tyr Asn Asn Asp Lys Pro Phe
   145                 150                 155                 160
   Tyr Ile Asn Gly Gln Asn Gln Ile Lys Val Thr Arg Cys Ser Ser Asp
                   165                 170                 175
   Ile Thr Gly Ile Phe Val Cys Glu Lys Gly Pro Cys Pro Gln Glu Asn
               180                 185                 190
   Cys Ile Ile Ser Lys Leu Phe Lys Glu Gly Cys Thr Phe Ile Tyr Asn
           195                 200                 205
   Ser Thr Gln Asn Ala Thr Ala Ser Ile Met Phe Met Gln Ser Leu Ser
       210                 215                 220
   Ser Val Val Glu Phe Cys Asn Ala Ser Thr His Asn Gln Glu Ala Pro
   225                 230                 235                 240
   Asn Leu Gln Asn Gln Met Cys Ser Leu Arg Ser Ala Trp Asp Val Ile
                   245                 250                 255
   Thr Asp Ser Ala Asp Phe His His Ser Phe Pro Met Asn Gly Thr Glu
               260                 265                 270
   Leu Pro Pro Pro Pro Thr Phe Ser Leu Val Glu Ala Gly Asp Lys Val
           275                 280                 285
   Val Cys Leu Val Leu Asp Val Ser Ser Lys Met Ala Glu Ala Asp Arg
       290                 295                 300
   Leu Leu Gln Leu Gln Gln Ala Ala Glu Phe Tyr Leu Met Gln Ile Val
   305                 310                 315                 320
   Glu Ile His Thr Phe Val Gly Ile Ala Ser Phe Asp Ser Lys Gly Glu
                   325                 330                 335
   Ile Arg Ala Gln Leu His Gln Ile Asn Ser Asn Asp Asp Arg Lys Leu
               340                 345                 350
   Leu Val Ser Tyr Leu Pro Thr Thr Val Ser Ala Lys Thr Asp Ile Ser
           355                 360                 365
   Ile Cys Ser Gly Leu Lys Lys Gly Phe Glu Val Val Glu Lys Leu Asn
       370                 375                 380
   Gly Lys Ala Tyr Gly Ser Val Met Ile Leu Val Thr Ser Gly Asp Asp
   385                 390                 395                 400
   Lys Leu Leu Gly Asn Cys Leu Pro Thr Val Leu Ser Ser Gly Ser Thr
                   405                 410                 415
   Ile His Ser Ile Ala Leu Gly Ser Ser Ala Ala Pro Asn Leu Glu Glu
               420                 425                 430
   Leu Ser Arg Leu Thr Gly Gly Leu Lys Phe Phe Val Pro Asp Ile Ser
           435                 440                 445
   Asn Ser Asn Ser Met Ile Asp Ala Phe Ser Arg Ile Ser Ser Gly Thr
       450                 455                 460
   Gly Asp Ile Phe Gln Gln His Ile Gln Leu Glu Ser Thr Gly Glu Asn
   465                 470                 475                 480
   Val Lys Pro His His Gln Leu Lys Asn Thr Val Thr Val Asp Asn Thr
                   485                 490                 495
   Val Gly Asn Asp Thr Met Phe Leu Val Thr Trp Gln Ala Ser Gly Pro
               500                 505                 510
   Pro Glu Ile Ile Leu Phe Asp Pro Asp Gly Arg Lys Tyr Tyr Thr Asn
           515                 520                 525
   Asn Phe Ile Thr Asn Leu Thr Phe Arg Thr Ala Ser Leu Trp Ile Pro
       530                 535                 540
   Gly Thr Ala Lys Pro Gly His Trp Thr Tyr Thr Leu Asn Asn Thr His
   545                 550                 555                 560
   His Ser Leu Gln Ala Leu Lys Val Thr Val Thr Ser Arg Ala Ser Asn
                   565                 570                 575
   Ser Ala Val Pro Pro Ala Thr Val Glu Ala Phe Val Glu Arg Asp Ser
               580                 585                 590
   Leu His Phe Pro His Pro Val Met Ile Tyr Ala Asn Val Lys Gln Gly
           595                 600                 605
   Phe Tyr Pro Ile Leu Asn Ala Thr Val Thr Ala Thr Val Glu Pro Glu
       610                 615                 620
   Thr Gly Asp Pro Val Thr Leu Arg Leu Leu Asp Asp Gly Ala Gly Ala
   625                 630                 635                 640
   Asp Val Ile Lys Asn Asp Gly Ile Tyr Ser Arg Tyr Phe Phe Ser Phe
                   645                 650                 655
   Ala Ala Asn Gly Arg Tyr Ser Leu Lys Val His Val Asn His Ser Pro
               660                 665                 670
   Ser Ile Ser Thr Pro Ala His Ser Ile Pro Gly Ser His Ala Met Tyr
           675                 680                 685
   Val Pro Gly Tyr Thr Ala Asn Gly Asn Ile Gln Met Asn Ala Pro Arg
       690                 695                 700
   Lys Ser Val Gly Arg Asn Glu Glu Glu Arg Lys Trp Gly Phe Ser Arg
   705                 710                 715                 720
   Val Ser Ser Gly Gly Ser Phe Ser Val Leu Gly Val Pro Ala Gly Pro
                   725                 730                 735
   His Pro Asp Val Phe Pro Pro Cys Lys Ile Ile Asp Leu Glu Ala Val
               740                 745                 750
   Lys Val Glu Glu Glu Leu Thr Leu Ser Trp Thr Ala Pro Gly Glu Asp
           755                 760                 765
   Phe Asp Gln Gly Gln Ala Thr Ser Tyr Glu Ile Arg Met Ser Lys Ser
       770                 775                 780
   Leu Gln Asn Ile Gln Asp Asp Phe Asn Asn Ala Ile Leu Val Asn Thr
   785                 790                 795                 800
   Ser Lys Arg Asn Pro Gln Gln Ala Gly Ile Arg Glu Ile Phe Thr Phe
                   805                 810                 815
   Ser Pro Gln Ile Ser Thr Asn Gly Pro Glu His Gln Pro Asn Gly Glu
               820                 825                 830
   Thr His Glu Ser His Arg Ile Tyr Val Ala Ile Arg Ala Met Asp Arg
           835                 840                 845
   Asn Ser Leu Gln Ser Ala Val Ser Asn Ile Ala Gln Ala Pro Leu Phe
       850                 855                 860
   Ile Pro Pro Asn Ser Asp Pro Val Pro Ala Arg Asp Tyr Leu Ile Leu
   865                 870                 875                 880
   Lys
<210> 431
   <211> 2646
   <212> DNA
   <213> Homo sapiens
<400> 431
   atgcagcatc accaccatca ccacggagta cagcttcaag acaatgggta taatggattg 60
   ctcattgcaa ttaatcctca ggtacctgag aatcagaacc tcatctcaaa cattaaggaa 120
   atgataactg aagcttcatt ttacctattt aatgctacca agagaagagt atttttcaga 180
   aatataaaga ttttaatacc tgccacatgg aaagctaata ataacagcaa aataaaacaa 240
   gaatcatatg aaaaggcaaa tgtcatagtg actgactggt atggggcaca tggagatgat 300
   ccatacaccc tacaatacag agggtgtgga aaagagggaa aatacattca tttcacacct 360
   aatttcctac tgaatgataa cttaacagct ggctacggat cacgaggccg agtgtttgtc 420
   catgaatggg cccacctccg ttggggtgtg ttcgatgagt ataacaatga caaacctttc 480
   tacataaatg ggcaaaatca aattaaagtg acaaggtgtt catctgacat cacaggcatt 540
   tttgtgtgtg aaaaaggtcc ttgcccccaa gaaaactgta ttattagtaa gctttttaaa 600
   gaaggatgca cctttatcta caatagcacc caaaatgcaa ctgcatcaat aatgttcatg 660
   caaagtttat cttctgtggt tgaattttgt aatgcaagta cccacaacca agaagcacca 720
   aacctacaga accagatgtg cagcctcaga agtgcatggg atgtaatcac agactctgct 780
   gactttcacc acagctttcc catgaacggg actgagcttc cacctcctcc cacattctcg 840
   cttgtagagg ctggtgacaa agtggtctgt ttagtgctgg atgtgtccag caagatggca 900
   gaggctgaca gactccttca actacaacaa gccgcagaat tttatttgat gcagattgtt 960
   gaaattcata ccttcgtggg cattgccagt ttcgacagca aaggagagat cagagcccag 1020
   ctacaccaaa ttaacagcaa tgatgatcga aagttgctgg tttcatatct gcccaccact 1080
   gtatcagcta aaacagacat cagcatttgt tcagggctta agaaaggatt tgaggtggtt 1140
   gaaaaactga atggaaaagc ttatggctct gtgatgatat tagtgaccag cggagatgat 1200
   aagcttcttg gcaattgctt acccactgtg ctcagcagtg gttcaacaat tcactccatt 1260
   gccctgggtt catctgcagc cccaaatctg gaggaattat cacgtcttac aggaggttta 1320
   aagttctttg ttccagatat atcaaactcc aatagcatga ttgatgcttt cagtagaatt 1380
   tcctctggaa ctggagacat tttccagcaa catattcagc ttgaaagtac aggtgaaaat 1440
   gtcaaacctc accatcaatt gaaaaacaca gtgactgtgg ataatactgt gggcaacgac 1500
   actatgtttc tagttacgtg gcaggccagt ggtcctcctg agattatatt atttgatcct 1560
   gatggacgaa aatactacac aaataatttt atcaccaatc taacttttcg gacagctagt 1620
   ctttggattc caggaacagc taagcctggg cactggactt acaccctgaa caatacccat 1680
   cattctctgc aagccctgaa agtgacagtg acctctcgcg cctccaactc agctgtgccc 1740
   ccagccactg tggaagcctt tgtggaaaga gacagcctcc attttcctca tcctgtgatg 1800
   atttatgcca atgtgaaaca gggattttat cccattctta atgccactgt cactgccaca 1860
   gttgagccag agactggaga tcctgttacg ctgagactcc ttgatgatgg agcaggtgct 1920
   gatgttataa aaaatgatgg aatttactcg aggtattttt tctcctttgc tgcaaatggt 1980
   agatatagct tgaaagtgca tgtcaatcac tctcccagca taagcacccc agcccactct 2040
   attccaggga gtcatgctat gtatgtacca ggttacacag caaacggtaa tattcagatg 2100
   aatgctccaa ggaaatcagt aggcagaaat gaggaggagc gaaagtgggg ctttagccga 2160
   gtcagctcag gaggctcctt ttcagtgctg ggagttccag ctggccccca ccctgatgtg 2220
   tttccaccat gcaaaattat tgacctggaa gctgtaaaag tagaagagga attgacccta 2280
   tcttggacag cacctggaga agactttgat cagggccagg ctacaagcta tgaaataaga 2340
   atgagtaaaa gtctacagaa tatccaagat gactttaaca atgctatttt agtaaataca 2400
   tcaaagcgaa atcctcagca agctggcatc agggagatat ttacgttctc accccaaatt 2460
   tccacgaatg gacctgaaca tcagccaaat ggagaaacac atgaaagcca cagaatttat 2520
   gttgcaatac gagcaatgga taggaactcc ttacagtctg ctgtatctaa cattgcccag 2580
   gcgcctctgt ttattccccc caattctgat cctgtacctg ccagagatta tcttatattg 2640
   aaataa                                                            2646
<210> 432
   <211> 36
   <212> DNA
   <213> Artificial Sequence
<220>
   <223> PCR primer
<400> 432
   cgcctgctcg agtcattaat attcatcaga aaatgg 36
<210> 433
   <211> 371
   <212> PRT
   <213> Homo sapiens
<400> 433
<210> 434
   <211> 1119
   <212> DNA
   <213> Homo sapiens
<400> 434
   atgcagcatc accaccatca ccactggcag cccctcttct tcaagtggct cttgtcctgt 60
   tgccctggga gttctcaaat tgctgcagca gcctccaccc agcctgagga tgacatcaat 120
   acacagagga agaagagtca ggaaaagatg agagaagtta cagactctcc tgggcgaccc 180
   cgagagctta ccattcctca gacttcttca catggtgcta acagatttgt tcctaaaagt 240
   aaagctctag aggccgtcaa attggcaata gaagccgggt tccaccatat tgattctgca 300
   catgtttaca ataatgagga gcaggttgga ctggccatcc gaagcaagat tgcagatggc 360
   agtgtgaaga gagaagacat attctacact tcaaagcttt ggagcaattc ccatcgacca 420
   gagttggtcc gaccagcctt ggaaaggtca ctgaaaaatc ttcaattgga ctatgttgac 480
   ctctatctta ttcattttcc agtgtctgta aagccaggtg aggaagtgat cccaaaagat 540
   gaaaatggaa aaatactatt tgacacagtg gatctctgtg ccacatggga ggccatggag 600
   aagtgtaaag atgcaggatt ggccaagtcc atcggggtgt ccaacttcaa ccacaggctg 660
   ctggagatga tcctcaacaa gccagggctc aagtacaagc ctgtctgcaa ccaggtggaa 720
   tgtcatcctt acttcaacca gagaaaactg ctggatttct gcaagtcaaa agacattgtt 780
   ctggttgcct atagtgctct gggatcccat cgagaagaac catgggtgga cccgaactcc 840
   ccggtgctct tggaggaccc agtcctttgt gccttggcaa aaaagcacaa gcgaacccca 900
   gccctgattg ccctgcgcta ccagctgcag cgtggggttg tggtcctggc caagagctac 960
   aatgagcagc gcatcagaca gaacgtgcag gtgtttgaat tccagttgac ttcagaggag 1020
   atgaaagcca tagatggcct aaacagaaat gtgcgatatt tgacccttga tatttttgct 1080
   ggccccccta attatccatt ttctgatgaa tattaatga 1119
<210> 435
   <211> 36
   <212> DNA
   <213> Artificial Sequence
<220>
   <223> Primer
<400> 435
   ggatccgccg ccaccatgac atccattcga gctgta 36
<210> 436
   <211> 27
   <212> DNA
   <213> Artificial Sequence
<220>
   <223> Primer
<400> 436
   gtcgactcag ctggaccaca gccgcag 27
<210> 437
   <211> 37
   <212> DNA
   <213> Artificial Sequence
<220>
   <223> Primer
<400> 437
   ggatccgccg ccaccatgga ctcctggacc ttctgct 37
<210> 438
   <211> 27
   <212> DNA
   <213> Artificial Sequence
<220>
   <223> Primer
<400> 438
   gtcgactcag aaatcctttc tcttgac 27
<210> 439
   <211> 933
   <212> DNA
   <213> Homo sapiens
<400> 439
   atggactcct ggaccttctg ctgtgtgtcc ctttgcatcc tggtagcaaa gcacacagat      60
   gctggagtta tccagtcacc ccggcacgag gtgacagaga tgggacaaga agtgactctg     120
   agatgtaaac caatttcagg acacgactac cttttctggt acagacagac catgatgcgg     180
   ggactggagt tgctcattta ctttaacaac aacgttccga tagatgattc agggatgccc      240
   gaggatcgat tctcagctaa gatgcctaat gcatcattct ccactctgaa gatccagccc     300
   tcagaaccca gggactcagc tgtgtacttc tgtgccagca gtttagttgg agcaaacact     360
   gaagctttct ttggacaagg caccagactc acagttgtag aggacctgaa caaggtgttc     420
   ccacccgagg tcgctgtgtt tgagccatca gaagcagaga tctcccacac ccaaaaggcc     480
   acactggtgt gcctggccac aggcttcttc cctgaccacg tggagctgag ctggtgggtg     540
   aatgggaagg aggtgcacag tggggtcagc acggacccgc agcccctcaa ggagcagccc     600
   gccctcaatg actccagata ctgcctgagc agccgcctga gggtctcggc caccttctgg     660
   cagaaccccc gcaaccactt ccgctgtcaa gtccagttct acgggctctc ggagaatgac     720
   gagtggaccc aggatagggc caaacccgtc acccagatcg tcagcgccga ggcctggggt     780
   agagcagact gtggctttac ctcggtgtcc taccagcaag gggtcctgtc tgccaccatc     840
   ctctatgaga tcctgctagg gaaggccacc ctgtatgctg tgctggtcag cgcccttgtg     900
   ttgatggcca tggtcaagag aaaggatttc tga                                  933
<210> 440
   <211> 822
   <212> DNA
   <213> Homo sapiens
<400> 440
   atgacatcca ttcgagctgt atttatattc ctgtggctgc agctggactt ggtgaatgga      60
   gagaatgtgg agcagcatcc ttcaaccctg agtgtccagg agggagacag cgctgttatc     120
   aagtgtactt attcagacag tgcctcaaac tacttccctt ggtataagca agaacttgga     180
   aaaagacctc agcttattat agacattcgt tcaaatgtgg gcgaaaagaa agaccaacga     240
   attgctgtta cattgaacaa gacagccaaa catttctccc tgcacatcac agagacccaa     300
   cctgaagact cggctgtcta cttctgtgca gcaagtatac tgaacaccgg taaccagttc     360
   tattttggga cagggacaag tttgacggtc attccaaata tccagaaccc tgaccctgcc     420
   gtgtaccagc tgagagactc taaatccagt gacaagtctg tctgcctatt caccgatttt     480
   gattctcaaa caaatgtgtc acaaagtaag gattctgatg tgtatatcac agacaaaact     540
   gtgctagaca tgaggtctat ggacttcaag agcaacagtg ctgtggcctg gagcaacaaa     600
   tctgactttg catgtgcaaa cgccttcaac aacagcatta ttccagaaga caccttcttc     660
   cccagcccag aaagttcctg tgatgtcaag ctggtcgaga aaagctttga aacagatacg     720
   aacctaaact ttcaaaacct gtcagtgatt gggttccgaa tcctcctcct gaaagtggcc     780
   gggtttaatc tgctcatgac gctgcggctg tggtccagct ga                        822
<210> 441
   <211> 2311
   <212> DNA
   <213> Homo sapiens
<400> 441
   gatttaatcc tatgacaaac taagttggtt ctgtcttcac ctgttttggt gaggttgtgt 60
   aagagttggt gtttgctcag gaagagattt aagcatgctt gcttacccag actcagagaa 120
   gtctccctgt tctgtcctag ctatgttcct gtgttgtgtg cattcgtctt ttccagagca 180
   aaccgcccag agtagaagat ggattggggc acgctgcaga cgatcctggg gggtgtgaac 240
   aaacactcca ccagcattgg aaagatctgg ctcaccgtcc tcttcatttt tcgcattatg 300
   atcctcgttg tggctgcaaa ggaggtgtgg ggagatgagc aggccgactt tgtctgcaac 360
   accctgcagc caggctgcaa gaacgtgtgc tacgatcact acttccccat ctcccacatc 420
   cggctatggg ccctgcagct gatcttcgtg tccagcccag cgctcctagt ggccatgcac 480
   gtggcctacc ggagacatga gaagaagagg aagttcatca agggggagat aaagagtgaa 540
   tttaaggaca tcgaggagat caaaacccag aaggtccgca tcgaaggctc cctgtggtgg 600
   acctacacaa gcagcatctt cttccgggtc atcttcgaag ccgccttcat gtacgtcttc 660
   tatgtcatgt acgacggctt ctccatgcag cggctggtga agtgcaacgc ctggccttgt 720
   cccaacactg tggactgctt tgtgtcccgg cccacggaga agactgtctt cacagtgttc 780
   atgattgcag tgtctggaat ttgcatcctg ctgaatgtca ctgaattgtg ttatttgcta 840
   attagatatt gttctgggaa gtcaaaaaag ccagtttaac gcattgccca gttgttagat 900
   taagaaatag acagcatgag agggatgagg caacccgtgc tcagctgtca aggctcagtc 960
   gccagcattt cccaacacaa agattctgac cttaaatgca accatttgaa acccctgtag 1020
   gcctcaggtg aaactccaga tgccacaatg agctctgctc ccctaaagcc tcaaaacaaa 1080
   ggcctaattc tatgcctgtc ttaattttct ttcacttaag ttagttccac tgagacccca 1140
   ggctgttagg ggttattggt gtaaggtact ttcatatttt aaacagagga tatcggcatt 1200
   tgtttctttc tctgaggaca agagaaaaaa gccaggttcc acagaggaca cagagaaggt 1260
   ttgggtgtcc tcctggggtt ctttttgcca actttcccca cgttaaaggt gaacattggt 1320
   tctttcattt gctttggaag ttttaatctc taacagtgga caaagttacc agtgccttaa 1380
   actctgttac actttttgga agtgaaaact ttgtagtatg ataggttatt ttgatgtaaa 1440
   gatgttctgg ataccattat atgttccccc tgtttcagag gctcagattg taatatgtaa 1500
   atggtatgtc attcgctact atgatttaat ttgaaatatg gtcttttggt tatgaatact 1560
   ttgcagcaca gctgagagag gctgtctgtt gtattcattg tggtcatagc acctaacaac 1620
   attgtagcct caatcgagtg agacagacta gaagttccta gttggcttat gatagcaaat 1680
   ggcctcatgt caaatattag atgtaatttt gtgtaagaaa tacagactgg atgtaccacc 1740
   aactactacc tgtaatgaca ggcctgtcca acacatctcc cttttccatg ctgtggtagc 1800
   cagcatcgga aagaacgctg atttaaagag gtgagcttgg gaattttatt gacacagtac 1860
   catttaatgg ggagacaaaa atgggggcca ggggagggag aagtttctgt cgttaaaaac 1920
   gagtttggaa agactggact ctaaattctg ttgattaaag atgagctttg tctaccttca 1980
   aaagtttgtt tggcttaccc ccttcagcct ccaatttttt aagtgaaaat ataactaata 2040
   acatgtgaaa agaatagaag ctaaggttta gataaatatt gagcagatct ataggaagat 2100
   tgaacctgaa tattgccatt atgcttgaca tggtttccaa aaaatggtac tccacatact 2160
   tcagtgaggg taagtatttt cctgttgtca agaatagcat tgtaaaagca ttttgtaata 2220
   ataaagaata gctttaatga tatgcttgta actaaaataa ttttgtaatg tatcaaatac 2280
   atttaaaaca ttaaaatata atctctataa t                                2311
<210> 442
   <211> 226
   <212> PRT
   <213> Homo sapiens
<400> 442
   Met Asp Trp Gly Thr Leu Gln Thr Ile Leu Gly Gly Val Asn Lys His
   5 10 15
   Ser Thr Ser Ile Gly Lys Ile Trp Leu Thr Val Leu Phe Ile Phe Arg
   20 25 30
   Ile Met Ile Leu Val Val Ala Ala Lys Glu Val Trp Gly Asp Glu Gln
   35 40 45
   Ala Asp Phe Val Cys Asn Thr Leu Gln Pro Gly Cys Lys Asn Val Cys
   50 55 60
   Tyr Asp His Tyr Phe Pro Ile Ser His Ile Arg Leu Trp Ala Leu Gln
   65 70 75 80
   Leu Ile Phe Val Ser Ser Pro Ala Leu Leu Val Ala Met His Val Ala
   85 90 95
   Tyr Arg Arg His Glu Lys Lys Arg Lys Phe Ile Lys Gly Glu Ile Lys
   100 105 110
   Ser Glu Phe Lys Asp Ile Glu Glu Ile Lys Thr Gln Lys Val Arg Ile
   115 120 125
   Glu Gly Ser Leu Trp Trp Thr Tyr Thr Ser Ser Ile Phe Phe Arg Val
   130 135 140
   Ile Phe Glu Ala Ala Phe Met Tyr Val Phe Tyr Val Met Tyr Asp Gly
   145 150 155 160
   Phe Ser Met Gln Arg Leu Val Lys Cys Asn Ala Trp Pro Cys Pro Asn
   165 170 175
   Thr Val Asp Cys Phe Val Ser Arg Pro Thr Glu Lys Thr Val Phe Thr
   180 185 190
   Val Phe Met Ile Ala Val Ser Gly Ile Cys Ile Leu Leu Asn Val Thr
   195 200 205
   Glu Leu Cys Tyr Leu Leu Ile Arg Tyr Cys Ser Gly Lys Ser Lys Lys
   210 215 220
   Pro Val
   225
<210> 443
   <211> 23
   <212> PRT
   <213> Homo sapiens
<400> 443
   Val Lys Leu Cys Gly Ile Asp Pro Cys Pro Asn Leu Val Asp Cys Phe
   5 10 15
   Ile Ser Arg Pro Gly Cys Gly
   20
<210> 444
   <211> 36
   <212> DNA
   <213> Artificial Sequence
<220>
   <223> PCR primer
<400> 444
   caatcaggca tgcacaacaa actgtatatc ggaaac 36
<210> 445
   <211> 30
   <212> DNA
   <213> Artificial Sequence
<220>
   <223> PCR primer
<400> 445
   cgtcaagatc ttcattactt ccgtcttgac 30
<210> 446
   <211> 579
   <212> PRT
   <213> Homo sapiens
<400> 446
   Met Asn Lys Leu Tyr Ile Gly Asn Leu Ser Glu Asn Ala Ala Pro Ser
                   5                   10                  15
   Asp Leu Glu Ser Ile Phe Lys Asp Ala Lys Ile Pro Val Ser Gly Pro
               20                  25                  30
   Phe Leu Val Lys Thr Gly Tyr Ala Phe Val Asp Cys Pro Asp Glu Ser
           35                  40                  45
   Trp Ala Leu Lys Ala Ile Glu Ala Leu Ser Gly Lys Ile Glu Leu His
       50                  55                  60
   Gly Lys Pro Ile Glu Val Glu His Ser Val Pro Lys Arg Gln Arg Ile
   65                  70                  75                  80
   Arg Lys Leu Gln Ile Arg Asn Ile Pro Pro His Leu Gln Trp Glu Val
                   85                  90                  95
   Leu Asp Ser Leu Leu Val Gln Tyr Gly Val Val Glu Ser Cys Glu Gln
               100                 105                 110
   Val Asn Thr Asp Ser Glu Thr Ala Val Val Asn Val Thr Tyr Ser Ser
           115                 120                 125
   Lys Asp Gln Ala Arg Gln Ala Leu Asp Lys Leu Asn Gly Phe Gln Leu
       130                 135                 140
   Glu Asn Phe Thr Leu Lys Val Ala Tyr Ile Pro Asp Glu Thr Ala Ala
   145                 150                 155                 160
   Gln Gln Asn Pro Leu Gln Gln Pro Arg Gly Arg Arg Gly Leu Gly Gln
                   165                 170                 175
   Arg Gly Ser Ser Arg Gln Gly Ser Pro Gly Ser Val Ser Lys Gln Lys
               180                 185                 190
   Pro Cys Asp Leu Pro Leu Arg Leu Leu Val Pro Thr Gln Phe Val Gly
           195                 200                 205
   Ala Ile Ile Gly Lys Glu Gly Ala Thr Ile Arg Asn Ile Thr Lys Gln
       210                 215                 220
   Thr Gln Ser Lys Ile Asp Val His Arg Lys Glu Asn Ala Gly Ala Ala
   225                 230                 235                 240
   Glu Lys Ser Ile Thr Ile Leu Ser Thr Pro Glu Gly Thr Ser Ala Ala
   245 250 255
   Cys Lys Ser Ile Leu Glu Ile Met His Lys Glu Ala Gln Asp Ile Lys
   260 265 270
   Phe Thr Glu Glu Ile Pro Leu Lys Ile Leu Ala His Asn Asn Phe Val
   275 280 285
   Gly Arg Leu Ile Gly Lys Glu Gly Arg Asn Leu Lys Lys Ile Glu Gln
   290 295 300
   Asp Thr Asp Thr Lys Ile Thr Ile Ser Pro Leu Gln Glu Leu Thr Leu
   305 310 315 320
   Tyr Asn Pro Glu Arg Thr Ile Thr Val Lys Gly Asn Val Glu Thr Cys
   325 330 335
   Ala Lys Ala Glu Glu Glu Ile Met Lys Lys Ile Arg Glu Ser Tyr Glu
   340 345 350
   Asn Asp Ile Ala Ser Met Asn Leu Gln Ala His Leu Ile Pro Gly Leu
   355 360 365
   Asn Leu Asn Ala Leu Gly Leu Phe Pro Pro Thr Ser Gly Met Pro Pro
   370 375 380
   Pro Thr Ser Gly Pro Pro Ser Ala Met Thr Pro Pro Tyr Pro Gln Phe
   385 390 395 400
   Glu Gln Ser Glu Thr Glu Thr Val His Leu Phe Ile Pro Ala Leu Ser
   405 410 415
   Val Gly Ala Ile Ile Gly Lys Gln Gly Gln His Ile Lys Gln Leu Ser
   420 425 430
   Arg Phe Ala Gly Ala Ser Ile Lys Ile Ala Pro Ala Glu Ala Pro Asp
   435 440 445
   Ala Lys Val Arg Met Val Ile Ile Thr Gly Pro Pro Glu Ala Gln Phe
   450 455 460
   Lys Ala Gln Gly Arg Ile Tyr Gly Lys Ile Lys Glu Glu Asn Phe Val
   465 470 475 480
   Ser Pro Lys Glu Glu Val Lys Leu Glu Ala His Ile Arg Val Pro Ser
   485 490 495
   Phe Ala Ala Gly Arg Val Ile Gly Lys Gly Gly Lys Thr Val Asn Glu
   500 505 510
   Leu Gln Asn Leu Ser Ser Ala Glu Val Val Val Pro Arg Asp Gln Thr
   515 520 525
   Pro Asp Glu Asn Asp Gln Val Val Val Lys Ile Thr Gly His Phe Tyr
   530 535 540
   Ala Cys Gln Val Ala Gln Arg Lys Ile Gln Glu Ile Leu Thr Gln Val
   545 550 555 560
   Lys Gln His Gln Gln Gln Lys Ala Leu Gln Ser Gly Pro Pro Gln Ser
   565 570 575
   Arg Arg Lys
<210> 447
   <211> 1743
   <212> DNA
   <213> Homo sapiens
<400> 447
   atgaacaaac tgtatatcgg aaacctcagc gagaacgccg ccccctcgga cctagaaagt 60
   atcttcaagg acgccaagat cccggtgtcg ggacccttcc tggtgaagac tggctacgcg 120
   ttcgtggact gcccggacga gagctgggcc ctcaaggcca tcgaggcgct ttcaggtaaa 180
   atagaactgc acgggaaacc catagaagtt gagcactcgg tcccaaaaag gcaaaggatt 240
   cggaaacttc agatacgaaa tatcccgcct catttacagt gggaggtgct ggatagttta 300
   ctagtccagt atggagtggt ggagagctgt gagcaagtga acactgactc ggaaactgca 360
   gttgtaaatg taacctattc cagtaaggac caagctagac aagcactaga caaactgaat 420
   ggatttcagt tagagaattt caccttgaaa gtagcctata tccctgatga aacggccgcc 480
   cagcaaaacc ccttgcagca gccccgaggt cgccgggggc ttgggcagag gggctcctca 540
   aggcaggggt ctccaggatc cgtatccaag cagaaaccat gtgatttgcc tctgcgcctg 600
   ctggttccca cccaatttgt tggagccatc ataggaaaag aaggtgccac cattcggaac 660
   atcaccaaac agacccagtc taaaatcgat gtccaccgta aagaaaatgc gggggctgct 720
   gagaagtcga ttactatcct ctctactcct gaaggcacct ctgcggcttg taagtctatt 780
   ctggagatta tgcataagga agctcaagat ataaaattca cagaagagat ccccttgaag 840
   attttagctc ataataactt tgttggacgt cttattggta aagaaggaag aaatcttaaa 900
   aaaattgagc aagacacaga cactaaaatc acgatatctc cattgcagga attgacgctg 960
   tataatccag aacgcactat tacagttaaa ggcaatgttg agacatgtgc caaagctgag 1020
   gaggagatca tgaagaaaat cagggagtct tatgaaaatg atattgcttc tatgaatctt 1080
   caagcacatt taattcctgg attaaatctg aacgccttgg gtctgttccc acccacttca 1140
   gggatgccac ctcccacctc agggccccct tcagccatga ctcctcccta cccgcagttt 1200
   gagcaatcag aaacggagac tgttcatctg tttatcccag ctctatcagt cggtgccatc 1260
   atcggcaagc agggccagca catcaagcag ctttctcgct ttgctggagc ttcaattaag 1320
   attgctccag cggaagcacc agatgctaaa gtgaggatgg tgattatcac tggaccacca 1380
   gaggctcagt tcaaggctca gggaagaatt tatggaaaaa ttaaagaaga aaactttgtt 1440
   agtcctaaag aagaggtgaa acttgaagct catatcagag tgccatcctt tgctgctggc 1500
   agagttattg gaaaaggagg caaaacggtg aatgaacttc agaatttgtc aagtgcagaa 1560
   gttgttgtcc ctcgtgacca gacacctgat gagaatgacc aagtggttgt caaaataact 1620
   ggtcacttct atgcttgcca ggttgcccag agaaaaattc aggaaattct gactcaggta 1680
   aagcagcacc aacaacagaa ggctctgcaa agtggaccac ctcagtcaag acggaagtaa 1740
   tga 1743
<210> 448
   <211> 35
   <212> DNA
   <213> Artificial Sequence
<220>
   <223> PCR primer
<400> 448
   cgtactagca tatgaacaaa ctgtatatcg gaaac 35
<210> 449
   <211> 579
   <212> PRT
   <213> Homo sapiens
<400> 449
   Met Asn Lys Leu Tyr Ile Gly Asn Leu Ser Glu Asn Ala Ala Pro Ser
   5 10 15
   Asp Leu Glu Ser Ile Phe Lys Asp Ala Lys Ile Pro Val Ser Gly Pro
   20 25 30
   Phe Leu Val Lys Thr Gly Tyr Ala Phe Val Asp Cys Pro Asp Glu Ser
   35 40 45
   Trp Ala Leu Lys Ala Ile Glu Ala Leu Ser Gly Lys Ile Glu Leu His
   50 55 60
   Gly Lys Pro Ile Glu Val Glu His Ser Val Pro Lys Arg Gln Arg Ile
   65 70 75 80
   Arg Lys Leu Gln Ile Arg Asn Ile Pro Pro His Leu Gln Trp Glu Val
   85 90 95
   Leu Asp Ser Leu Leu Val Gln Tyr Gly Val Val Glu Ser Cys Glu Gln
   100 105 110
   Val Asn Thr Asp Ser Glu Thr Ala Val Val Asn Val Thr Tyr Ser Ser
   115 120 125
   Lys Asp Gln Ala Arg Gln Ala Leu Asp Lys Leu Asn Gly Phe Gln Leu
   130 135 140
   Glu Asn Phe Thr Leu Lys Val Ala Tyr Ile Pro Asp Glu Thr Ala Ala
   145 150 155 160
   Gln Gln Asn Pro Leu Gln Gln Pro Arg Gly Arg Arg Gly Leu Gly Gln
   165 170 175
   Arg Gly Ser Ser Arg Gln Gly Ser Pro Gly Ser Val Ser Lys Gln Lys
   180 185 190
   Pro Cys Asp Leu Pro Leu Arg Leu Leu Val Pro Thr Gln Phe Val Gly
   195 200 205
   Ala Ile Ile Gly Lys Glu Gly Ala Thr Ile Arg Asn Ile Thr Lys Gln
   210 215 220
   Thr Gln Ser Lys Ile Asp Val His Arg Lys Glu Asn Ala Gly Ala Ala
   225 230 235 240
   Glu Lys Ser Ile Thr Ile Leu Ser Thr Pro Glu Gly Thr Ser Ala Ala
   245 250 255
   Cys Lys Ser Ile Leu Glu Ile Met His Lys Glu Ala Gln Asp Ile Lys
   260 265 270
   Phe Thr Glu Glu Ile Pro Leu Lys Ile Leu Ala His Asn Asn Phe Val
   275 280 285
   Gly Arg Leu Ile Gly Lys Glu Gly Arg Asn Leu Lys Lys Ile Glu Gln
   290 295 300
   Asp Thr Asp Thr Lys Ile Thr Ile Ser Pro Leu Gln Glu Leu Thr Leu
   305 310 315 320
   Tyr Asn Pro Glu Arg Thr Ile Thr Val Lys Gly Asn Val Glu Thr Cys
   325 330 335
   Ala Lys Ala Glu Glu Glu Ile Met Lys Lys Ile Arg Glu Ser Tyr Glu
   340 345 350
   Asn Asp Ile Ala Ser Met Asn Leu Gln Ala His Leu Ile Pro Gly Leu
   355 360 365
   Asn Leu Asn Ala Leu Gly Leu Phe Pro Pro Thr Ser Gly Met Pro Pro
   370 375 380
   Pro Thr Ser Gly Pro Pro Ser Ala Met Thr Pro Pro Tyr Pro Gln Phe
   385 390 395 400
   Glu Gln Ser Glu Thr Glu Thr Val His Leu Phe Ile Pro Ala Leu Ser
   405 410 415
   Val Gly Ala Ile Ile Gly Lys Gln Gly Gln His Ile Lys Gln Leu Ser
   420 425 430
   Arg Phe Ala Gly Ala Ser Ile Lys Ile Ala Pro Ala Glu Ala Pro Asp
   435 440 445
   Ala Lys Val Arg Met Val Ile Ile Thr Gly Pro Pro Glu Ala Gln Phe
   450 455 460
   Lys Ala Gln Gly Arg Ile Tyr Gly Lys Ile Lys Glu Glu Asn Phe Val
   465 470 475 480
   Ser Pro Lys Glu Glu Val Lys Leu Glu Ala His Ile Arg Val Pro Ser
   485 490 495
   Phe Ala Ala Gly Arg Val Ile Gly Lys Gly Gly Lys Thr Val Asn Glu
   500 505 510
   Leu Gln Asn Leu Ser Ser Ala Glu Val Val Val Pro Arg Asp Gln Thr
   515 520 525
   Pro Asp Glu Asn Asp Gln Val Val Val Lys Ile Thr Gly His Phe Tyr
   530 535 540
   Ala Cys Gln Val Ala Gln Arg Lys Ile Gln Glu Ile Leu Thr Gln Val
   545 550 555 560
   Lys Gln His Gln Gln Gln Lys Ala Leu Gln Ser Gly Pro Pro Gln Ser
   565 570 575
   Arg Arg Lys
<210> 450
   <211> 1743
   <212> DNA
   <213> Homo sapiens
<400> 450
   atgaacaaac tgtatatcgg aaacctcagc gagaacgccg ccccctcgga cctagaaagt 60
   atcttcaagg acgccaagat cccggtgtcg ggacccttcc tggtgaagac tggctacgcg 120
   ttcgtggact gcccggacga gagctgggcc ctcaaggcca tcgaggcgct ttcaggtaaa 180
   atagaactgc acgggaaacc catagaagtt gagcactcgg tcccaaaaag gcaaaggatt 240
   cggaaacttc agatacgaaa tatcccgcct catttacagt gggaggtgct ggatagttta 300
   ctagtccagt atggagtggt ggagagctgt gagcaagtga acactgactc ggaaactgca 360
   gttgtaaatg taacctattc cagtaaggac caagctagac aagcactaga caaactgaat 420
   ggatttcagt tagagaattt caccttgaaa gtagcctata tccctgatga aacggccgcc 480
   cagcaaaacc ccttgcagca gccccgaggt cgccgggggc ttgggcagag gggctcctca 540
   aggcaggggt ctccaggatc cgtatccaag cagaaaccat gtgatttgcc tctgcgcctg 600
   ctggttccca cccaatttgt tggagccatc ataggaaaag aaggtgccac cattcggaac 660
   atcaccaaac agacccagtc taaaatcgat gtccaccgta aagaaaatgc gggggctgct 720
   gagaagtcga ttactatcct ctctactcct gaaggcacct ctgcggcttg taagtctatt 780
   ctggagatta tgcataagga agctcaagat ataaaattca cagaagagat ccccttgaag 840
   attttagctc ataataactt tgttggacgt cttattggta aagaaggaag aaatcttaaa 900
   aaaattgagc aagacacaga cactaaaatc acgatatctc cattgcagga attgacgctg 960
   tataatccag aacgcactat tacagttaaa ggcaatgttg agacatgtgc caaagctgag 1020
   gaggagatca tgaagaaaat cagggagtct tatgaaaatg atattgcttc tatgaatctt 1080
   caagcacatt taattcctgg attaaatctg aacgccttgg gtctgttccc acccacttca 1140
   gggatgccac ctcccacctc agggccccct tcagccatga ctcctcccta cccgcagttt 1200
   gagcaatcag aaacggagac tgttcatctg tttatcccag ctctatcagt cggtgccatc 1260
   atcggcaagc agggccagca catcaagcag ctttctcgct ttgctggagc ttcaattaag 1320
   attgctccag cggaagcacc agatgctaaa gtgaggatgg tgattatcac tggaccacca 1380
   gaggctcagt tcaaggctca gggaagaatt tatggaaaaa ttaaagaaga aaactttgtt 1440
   agtcctaaag aagaggtgaa acttgaagct catatcagag tgccatcctt tgctgctggc 1500
   agagttattg gaaaaggagg caaaacggtg aatgaacttc agaatttgtc aagtgcagaa 1560
   gttgttgtcc ctcgtgacca gacacctgat gagaatgacc aagtggttgt caaaataact 1620
   ggtcacttct atgcttgcca ggttgcccag agaaaaattc aggaaattct gactcaggta 1680
   aagcagcacc aacaacagaa ggctctgcaa agtggaccac ctcagtcaag acggaagtaa 1740
   tga                                                               1743
<210> 451
   <211> 25
   <212> PRT
   <213> Homo sapiens
<400> 451
   Leu Gly Lys Glu Val Arg Asp Ala Lys Ile Thr Pro Glu Ala Phe Glu
                   5                   10                  15
   Lys Leu Gly Phe Pro Ala Ala Lys Glu
               20                  25
<210> 452
   <211> 25
   <212> PRT
   <213> Homo sapiens
<400> 452
   Lys Ala Ser Asp Gly Asp Tyr Tyr Thr Leu Ala Val Pro Met Gly Asp
   5 10 15
   Val Pro Met Asp Gly Ile Ser Val Ala
   20 25
<210> 453
   <211> 16
   <212> PRT
   <213> Homo sapiens
<400> 453
   Pro Asp Arg Asp Val Asn Leu Thr His Gln Leu Asn Pro Lys Val Lys
   5 10 15
<210> 454
   <211> 20
   <212> PRT
   <213> Homo sapiens
<400> 454
   Lys Ile Ala Pro Ala Glu Ala Pro Asp Ala Lys Val Arg Met Val Ile
   5 10 15
   Ile Thr Gly Pro
   20
<210> 455
   <211> 20
   <212> PRT
   <213> Homo sapiens
<400> 455
   Pro Asp Glu Thr Ala Ala Gln Gln Asn Pro Leu Gln Gln Pro Arg Gly
   5 10 15
   Arg Arg Gly Leu
   20
<210> 456
   <211> 20
   <212> PRT
   <213> Homo sapiens
<400> 456
   Arg Thr Ile Thr Val Lys Gly Asn Val Glu Thr Cys Ala Lys Ala Glu
   1 5 10 15
   Glu Glu Ile Met
   20
<210> 457
   <211> 20
   <212> PRT
   <213> Homo sapiens
<400> 457
   Ala Phe Val Asp Cys Pro Asp Glu Ser Trp Ala Leu Lys Ala Ile Glu
   1 5 10 15
   Ala Leu Ser Gly
   20
<210> 458
   <211> 20
   <212> PRT
   <213> Homo sapiens
<400> 458
   Ile Arg Lys Leu Gln Ile Arg Asn Ile Pro Pro His Leu Gln Trp Glu
   1 5 10 15
   Val Leu Asp Ser
   20
<210> 459
   <211> 20
   <212> PRT
   <213> Homo sapiens
<400> 459
   Ala Gln Gln Asn Pro Leu Gln Gln Pro Arg Gly Arg Arg Gly Leu Gly
   1 5 10 15
   Gln Arg Gly Ser
   20
<210> 460
   <211> 20
   <212> PRT
   <213> Homo sapiens
<400> 460
   Asp Val His Arg Lys Glu Asn Ala Gly Ala Ala Glu Lys Ser Ile Thr
   5 10 15
   Ile Leu Ser Thr
   20
<210> 461
   <211> 20
   <212> PRT
<213> Homo sapiens
   <400> 461
   Leu Tyr Asn Pro Glu Arg Thr Ile Thr Val Lys Gly Asn Val Glu Thr
   5 10 15
   Cys Ala Lys Ala
   20
<210> 462
   <211> 20
   <212> PRT
   <213> Homo sapiens
<400> 462
   Glu Glu Glu Ile Met Lys Lys Ile Arg Glu Ser Tyr Glu Asn Asp Ile
   5 10 15
   Ala Ser Met Asn
   20
<210> 463
   <211> 20
   <212> PRT
   <213> Homo sapiens
<400> 463
   Leu Asn Ala Leu Gly Leu Phe Pro Pro Thr Ser Gly Met Pro Pro Pro
   5 10 15
   Thr Ser Gly Pro
   20
<210> 464
   <211> 20
   <212> PRT
   <213> Homo sapiens
<400> 464
   Lys Ile Ala Pro Ala Glu Ala Pro Asp Ala Lys Val Arg Met Val Ile
                       5                  10                  15
   Ile Thr Gly Pro
               20
<210> 465
   <211> 18
   <212> PRT
   <213> Homo sapiens
<400> 465
   Thr Gly Tyr Ala Phe Val Asp Cys Pro Asp Glu Ser Trp Ala Leu Lys Ile
                       5                  10                  15
   Glu
<210> 466
   <211> 11
   <212> PRT
   <213> Homo sapiens
<400> 466
   Phe Val Asp Cys Pro Asp Glu Ser Trp Ala Leu
                       5                  10
<210> 467
   <211> 33
   <212> DNA
   <213> Homo sapiens
<400> 467
   ttcgtggact gcccggacga gagctgggcc ctc 33